# EUROPEAN PATENT APPLICATION

(11) **EP 3 995 830 A1**
(43) Date of publication of application: **11.05.2022**
(21) Application number: 20306351.6
(22) Date of filing: 06.11.2020
(51) Int. Cl.: G01N 33/574, C12Q 1/6886

(54) **METHOD OF PROGNOSIS OF AN INDIVIDUAL HAVING MULTIPLE MYELOMA TO BE SENSITIVE TO A TREATMENT**

(71) Applicant: Centre national de la recherche scientifique, 75016 Paris (FR); UNIVERSITE DE MONTPELLIER, 34090 Montpellier (FR); Centre Hospitalier Universitaire de Montpellier, 34295 Montpellier Cedex 05 (FR)
(72) Inventor: MOREAUX, Jérôme, 34090 Montpellier (FR); ALATERRE, Elina, 34790 Grabels (FR); CAVALLI, Giacomo, 34090 Montpellier (FR)
(74) Representative: LLR

(57) **Abstract**

The present invention relates to a method for the *in vitro* prognosis of the likelihood of an individual having multiple myeloma to be sensitive to a therapeutic treatment, comprising the following steps
a) Measuring, in a biological sample of the individual, the level of expression of at least five genes, to obtain a individual value
b) Comparing the individual value obtained in a) with a reference value,
c) Classifying the individual as sensitive to the treatment if the individual value is modified when compared with the reference value.

## Description

The present invention is relating to the prognosis of an individual having multiple myeloma (MM) to be sensitive to a treatment of said condition ; it is also relating to method of prognosis of the outcome of an individual having multiple myeloma. The invention also relates to kits of diagnostic useful for performing said methods.

Multiple myeloma (MM) is a B cell neoplasia characterized by the accumulation of clonal plasma cells in the bone marrow. MM is the second most common blood cancer, and is affecting the plasma cells. In individuals having multiple myeloma, malignant clonal plasma cells accumulate within the bone marrow. These aberrant cells are 'diluting' the normal plasma cells that are devoted to fight infections. The malignant plasma cells also produce abnormal proteins, such as M protein, which may cause solid tumors, damage the kidneys, and impair immune system function. In some cases, the malignant cells may cause a single tumor, called a solitary plasmacytoma, but if multiple tumors are formed, and then the disease is called multiple myeloma.

MM affects approximately 25,000 new individuals per year in the EU, accounts for 10% of hematological malignancies and is the second most common hematological disorder.

MM is a very heterogeneous disease, at the molecular as well as the clinical level. At diagnosis, patients are classified into different subgroups depending on the MMCs molecular discrepancies and on different transcriptome/biomarkers-based scores, each associated to a given outcome.

Despite the survival improvement provided by current treatments, the majority of patients relapse and eventually become resistant to all treatments. The median overall survival of MM patients is 6 years. MM is characterized by a high degree of biological, genetic and intra-clonal heterogeneity. The development of MM is usually accompanied by a series of genetic alterations, such as cytogenetic abnormalities, primary and secondary chromosomal translocations and oncogenic activation.

Chromosomal alterations, including t(4;14), translocation, 17p13 deletion and 1q21 gain correlate with poor disease prognosis. Several mutations also negatively impact on survival, including mutations in CCND1 and genes involved in DNA repair pathways (TP53, ATM, ATR and ZNFHX4).

WO2018/083086 is disclosing a method for the *in vitro* prognosis of individuals having MM, based on a score involving a set of specific genes ; in particular WO2018/083086 is providing a method in vitro predicting the likelihood of such individual to be responsive to a treatment with an inhibitor of EZH2.

However, there is a need for additional therapies since until today, MM is a condition that cannot be cured, and all patients finally relapse. Furthermore, the proposed therapeutic approaches often depend on the patient's age only, and thus are not adapted to an individual patient or even a subgroup of patients. There is therefore a need for prognostic and theranostic biomarkers, to define precisions medicine in MM.

It has now been found in the context of the present invention that 5 specific epigenetics modifications are associated with cancer development and progression and resistance to chemotherapy.

The present invention is therefore relating to a method for the in vitro prognosis of an individual having multiple myeloma to be sensitive to a treatment, comprising the steps of
a) measuring in a biological sample of the individual the level of expression and/ or the presence of at least five genes associated to at least a modified histone selected from H3K4me1, H3K4me3, H3K9me3, H3K27ac and H3K27me3, to obtain an individual value,
b) comparing the individual value obtained in a) with a reference value,
c) classifying the individual as sensitive to the treatment if the individual value is modified when compared with the reference value.
Said genes can be selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 ZNF5188B and from genes mentioned in tables 1 to 4 here-below.

The present invention is also relating to a method for the in vitro prognosis of the outcome of an individual having multiple myeloma comprising the following steps :
a) measuring in a biological sample of the individual the level of expression of at least five genes associated to at least a modified histone selected from H3K4me1, H3K9me3, H3K27ac and H3K27me3
b) calculating a risk score from the expression level obtained in step a
c) comparing the risk score of the individual to a reference value, and if the risk score is higher than the reference value, classifying the individual as having a bad prognosis.
Said genes can be selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B.

The invention is also relating to kits for performing the above methods, said kits comprising means for detecting the presence of at least a protein selected from H3K4me1, H3K4me3, H3K9me3, H3K27ac and H3K27me3 and/or means for measuring the expression level of at least five genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2, ZNF5188B and from genes mentioned in tables 1 to 4 here-below.

The epigenetic control of gene expression plays an essential role in the regulation of cell fate and cell identity. Aberrant changes in key regulatory chromatin features, such as DNA methylation and histone post-translational modifications (PTMs), are involved in MM pathophysiology and drug resistance. Unlike DNA methylation, the histone PTM landscape is more dynamic and constantly evolving. Structural changes in active euchromatin or silenced heterochromatin are controlled by epigenetic enzyme complexes of chromatin writer, reader and eraser. Moreover, epigenetic biomarkers for MM can be used as predictive and prognostic indicators to guide diagnosis and treatment.

Individualizing therapy based on epigenetic biomarkers could increase therapeutic efficacy for MM patients.

Using multiple myeloma cell lines the inventors have now characterized the global epigenetic landscape of human multiple myeloma cell line (HMCL).

The differential analysis of post-translational modifications (PTM) profiles on HMCLs highlighted links between histone modifications and cytogenetic abnormalities.

Super-enhancers (SE) associated with genes involved in the biology of MM, such as MAF, MYC, CCND1, CCND2, TRAF3 or NSD2 have now been identified.

According to one embodiment, the invention is directed to a method for in vitro prognosis of the likelihood of an individual having multiple myeloma to be sensitive to a therapeutic treatment, comprising the following steps:
a) measuring in a biological sample the level of expression of at least five genes selected from the group consisting of BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ,
b) calculating a risk score form the level obtained in step a)
c) comparing the risk score of the individual to a reference value and if the risk score is higher than the reference value, classifying the individual as sensitive to the treatment by a drug which is selected from bromodomain and extra-terminal motif (BET) inhibitors.

Also repressive domains, characterized by the co-localization of the H3K9me3 and H3K27me3 modifications on promoters of genes with potential tumor suppressor function have been identified.

Therefore, according to another embodiment, the invention is directed to a method for in vitro prognosis of the likelihood of an individual having multiple myeloma to be sensitive to a therapeutic treatment, comprising the following steps:
a) Measuring in a biological sample, the level of expression of at least five genes selected from the group consisting of ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B,
b) Calculating a risk score from the expression level obtained in step a)
c) Comparing the risk score of the individual to a reference value, and if the risk score is higher than the reference value, classifying the individual as sensitive to the treatment by a drug modifying the level of H3K9me3 and H3K27me3.

The drug modifying the level of H3K9me3 and H3K27me3 can in particular be selected from SUV39H1 inhibitors, SUV39H2 inhibitors and Polycomb repressive complex (PRC) 2 inhibitors.

The inventors have also identified H3K4me3 active promoters associated with the sensitivity of HMCLs to lenalidomide and histone desacetylase inhibitors (HDACi). From these data, epigenetic biomarkers based on this H3K4me3 PTM predicting lenalidomide and romidepsin HDACi response have been characterized.

Another aspect of the invention is therefore a method for in vitro prognosis of the likelihood of an individual having multiple myeloma to be sensitive to a therapeutic treatment, comprising the following steps :
a) Measuring in a biological sample of said individual the presence and the level of H3K4me3 in at least five genes selected for the genes mentioned in tables 1 and 2,
b) Comparing the level of H3K4me3 associated with at least five genes mentioned in table 1 and the level of H3K4me3 associated with at least five genes mentioned in table 2,
c) Classifying the individual as sensitive to a drug selected from IMiD if the level associated with genes of table 1 is higher than the level associated with genes of table 2.

In a further embodiment, the invention is relating to a method for in vitro prognosis of the likelihood of an individual having multiple myeloma to be sensitive to a therapeutic treatment, comprising the following steps :
a) Measuring in a biological sample the presence and the level of at least five genes selected from tables 3 and 4,
b) Comparing the level of H3K4me3 associated with at least five genes mentioned in table 3 and the level of H3K4me3 associated with at least five genes mentioned in table 4,
c) Classifying the individual as sensitive to a drug selected from histone desacetylase inhibitors (HDACi) if the level associated with genes of table 3 is higher than the level associated with genes of table 4.

### Legends of the figures

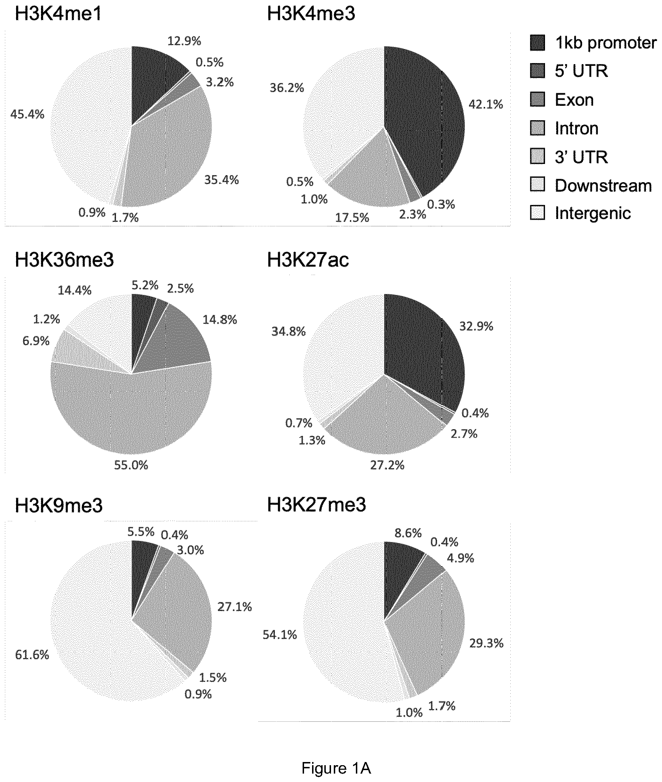

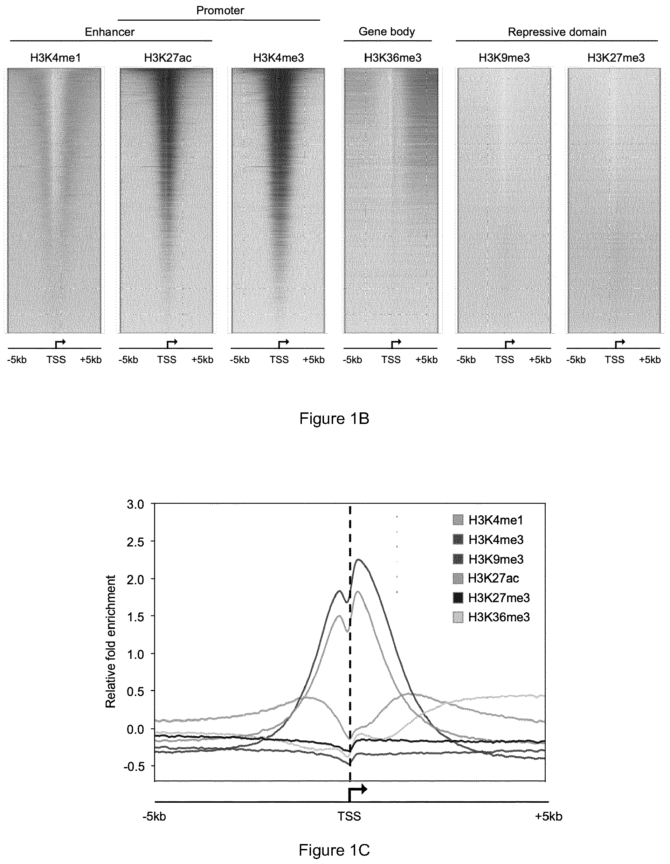

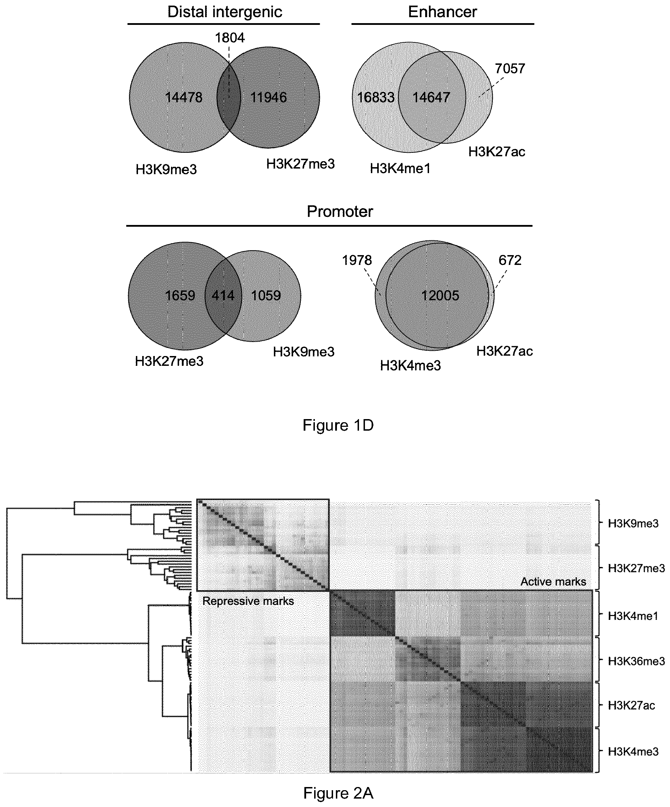

Figure 1: Histone mark enrichment distribution in HMCLs. (A) Pie chart showing the distribution of H3K4me1, H3K4me3, H3K9me3, H3K27ac, H3K27me3 and H3K36me3 peaks in promoter, 5' UTR, exon, intron, 3' UTR, downstream and distal intergenic regions. The peaks were identified by MACS and were annotated using ChIPseeker R package. Results are expressed as the mean percentage distribution of the 16 HMCLs. (B) Heatmap of histone modification peaks binding to the TSS regions (from -5 kb to 5 kb) ranked by histone mark signal in the XG6 cell line. (C) ChIP-seq profiles across 5 kb regions centered on the TSS in the XG6 cell line. The y axis shows the histone ChIP-seq signal normalized using the log2 ratio (histone vs input) normalization. Heatmaps and average profiles of histone mark enrichment were generated by deeptools using all known genes for each individual histone modification. (D) Venn diagram of overlapped region between H3K9me3 and H3K27me3 in intergenic regions and promoters, H3K4me1 and H3K27ac in enhancer regions, and H3K4me1 and H3K27ac in promoters in XG6.

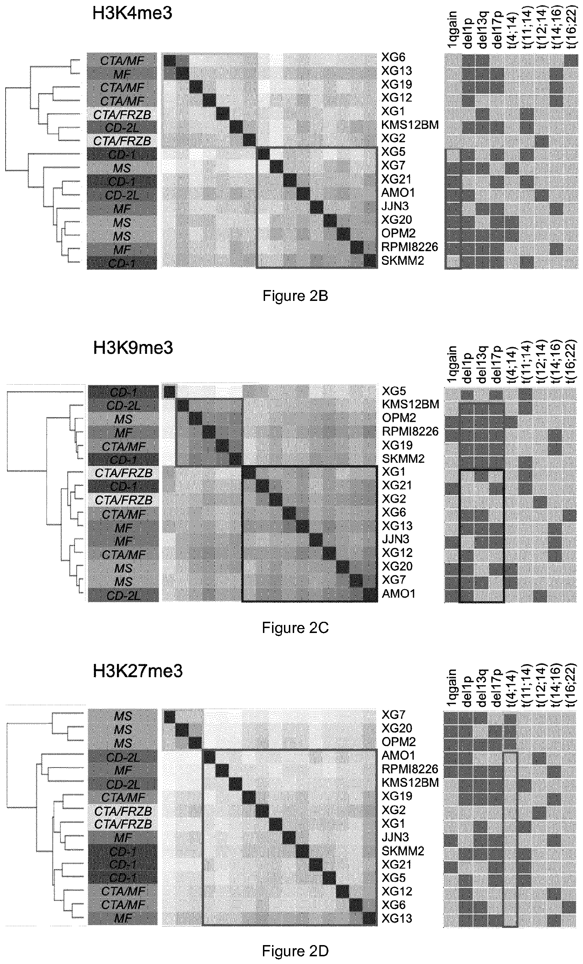

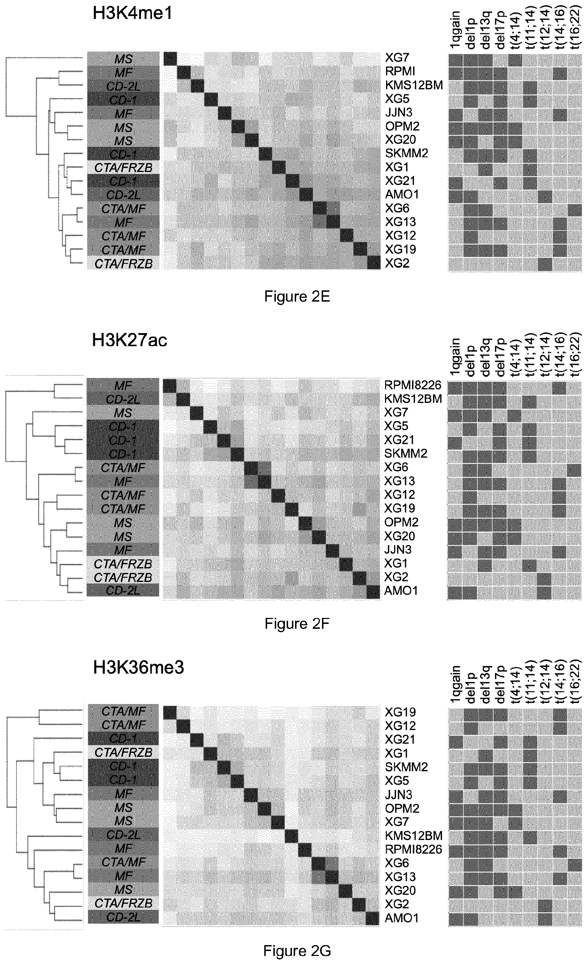

Figure 2: Differential histone enrichment evaluation in 16 HMCLs. Correlation heatmaps of H3K4me1, H3K4me3, H3K9me3, H3K27ac, H3K27me3 and H3K36me3 marks were generated using Diffbind R package. A) Correlation heatmap, using occupancy data (peaks identified by MACS), performed on the 6 histone marks in HMCLs shows distinct separation from each other and between active and repressive histone marks. B) Correlation heatmap, using affinity data (read count), highlights the link between histone modifications and both HMCLs molecular classification and cytogenetic abnormalities (dark box: presence, light box: absence). Yellow, orange and brown boxes encompass interest clusters. CD-1, CD-2L, CTA/MF, CTA/FRZB, MF and MS correspond to the transcriptional classification of HMCLs.

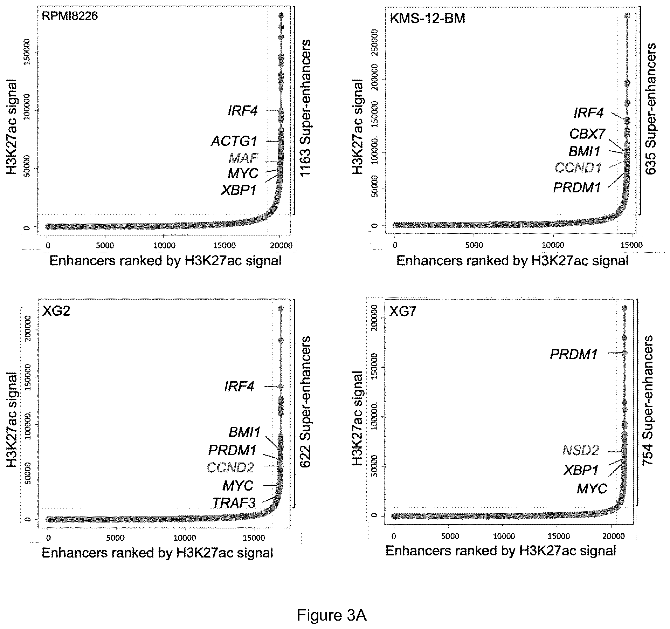

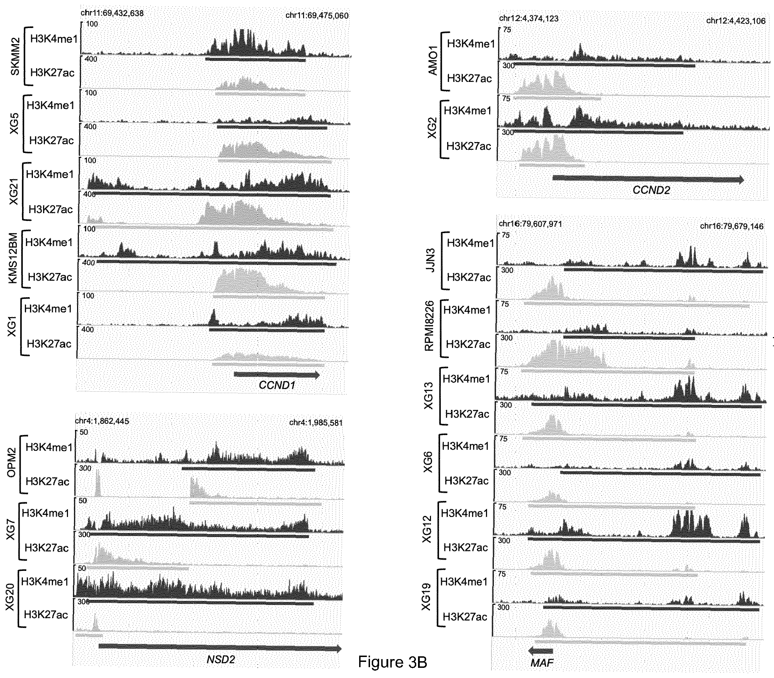

Figure 3: Super-enhancers identified in HMCLs. (A) Total of H3K27ac ChIP-seq signal in units of reads per bin mapped reads in enhancer regions for all enhancers in RPMI8226, KMS-12-BM, XG2 and XG7 cell lines harboring translocation which targets MAF, CCND1, CCND2 and NSD2 gene, respectively. Enhancers are ranked by increasing H3K27ac ChIP-seq signal. (B) Gene tracks of H3H4me1 and H3K27ac ChIP-seq occupancy at super-enhancers near genes involved in MM pathogenesis. Super-enhancers of CCND1, NSD2 and CCND2 overlap the TSS region while MAF enhancer is localized downstream of the gene. The x axis shows the genomic position and H3K27ac and H3K4me1 enhancers-containing regions are depicted with a beige and brown box, respectively. The y axis shows signal coverage of ChIP-seq occupancy in units of reads per bin mapped reads.

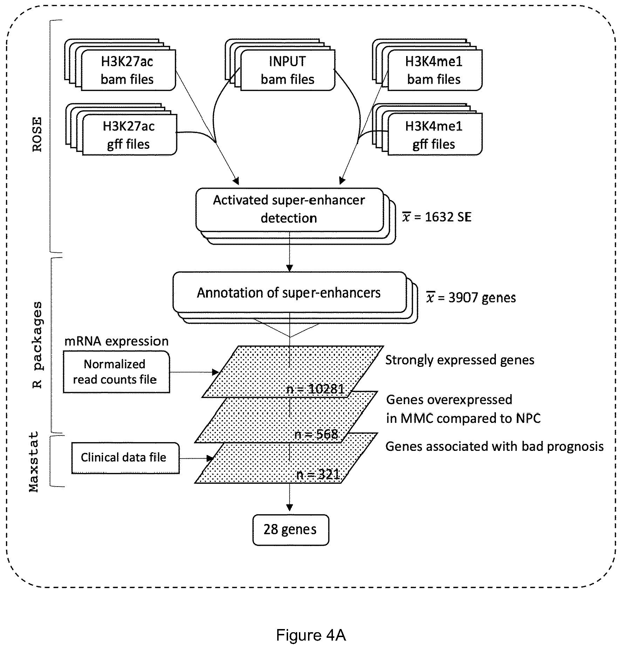

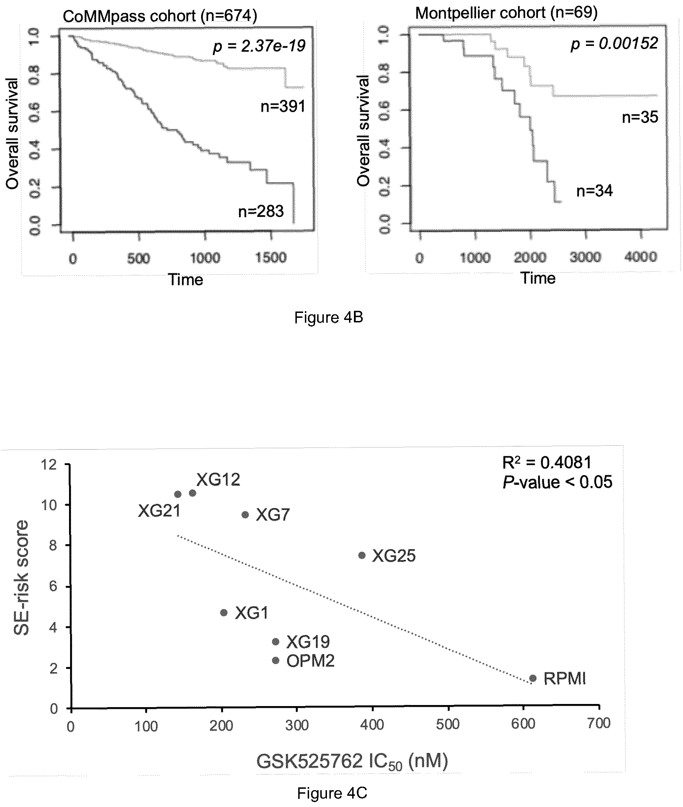

Figure 4: SE-risk score can predict survival in MM patients. (A) SE-risk score pipeline using first ROSE algorithm on ChIP-seq data of HMCLs to find super-enhancers. SE-associated genes are identified using RNA-seq of HMCLs. Genes of interest are then filtered according to their expression in HMCLs, plasma cells of MM patients (MMC) and normal plasma cells (NPC), and their prognostic value in MM patients. (B) Prognostic value of the SE-risk score in MM. Patients of the CoMMpass cohort (n = 674) were ranked according to the increased SE-risk score and a maximum difference in OS was obtained with SE-risk score of -4.0 splitting patients into high-risk (n = 283; red curve) and low-risk (n = 391; green curve) groups. SE-risk score also had a prognostic value in an independent cohort of 69 patients (Montpellier cohort). To compute the SE-risk score in Montpellier cohort, the proportion of patients of the CoMMpass cohort for each gene was applied on the Montpellier cohort to determine gene cut-point. The same gene beta-coefficient and score cut-point was applied to distinguish high-risk (n = 34: red curve) from low-risk (n = 35; green curve) groups. (C) SE-associated gene score correlates with the response to GSK525762 in HMCLs. Linear regression analysis of the SE-associated gene score in function of the IC50 of GSK525762 in 8 HMCLs. Coefficient of determination R² represents the square of the Pearson correlation coefficient (r) (Pearson correlation test).

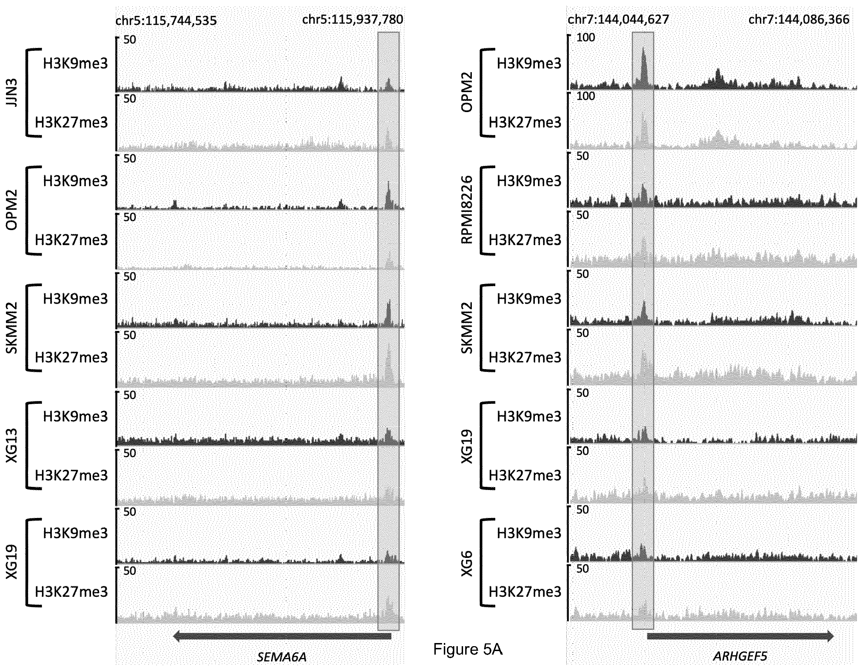

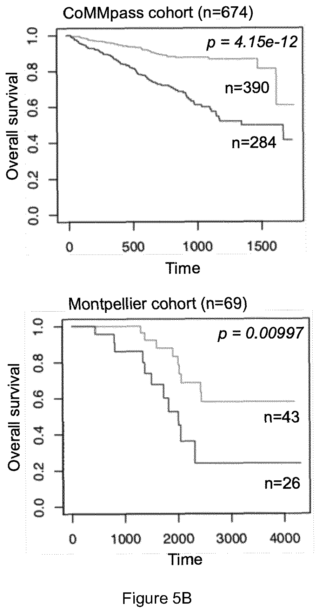

Figure 5: H3K9me3/H3K27me3 score can predict survival in MM patients. (A) Repressive domains characterized by co-localization of H3K9me3 and H3K27me3 enrichment on promoter of SEMA6A and ARHGEF5 gene. The promoter of these genes is the most frequently enriched in H3K9me3 and H3K27me3 modifications in HMCLs. The x axis shows the genomic position and the y axis shows signal coverage of ChIP-seq occupancy in units of reads per bin mapped reads. (B) Prognostic value of the H3K9me3/H3K27me3 score in MM. Patients of the CoMMpass cohort (n = 674) were ranked according to the increased H3K9me3/H3K27me3 score and a maximum difference in OS was obtained with increased H3K9me3/H3K27me3 score of -3.45 splitting patients into high-risk (n = 390; red curve) and low-risk (n = 284; green curve) groups. High-risk group for individual gene composing H3K9me3/H3K27me3 score is associated with low gene expression (negative beta-coefficient) whereas high-risk group for H3K9me3/H3K27me3 score is associated with high score because of the multiplication of the beta-coefficient (negative value) and the weight (+1 or -1 if the signal of MM patient gene is above or below the Maxstat reference value, respectively) of genes. H3K9me3/H3K27me3 score also had a prognostic value in an independent cohort of 69 patients (Montpellier cohort). To compute the H3K9me3/H3K27me3 score in Montpellier cohort, the same method described before was used.

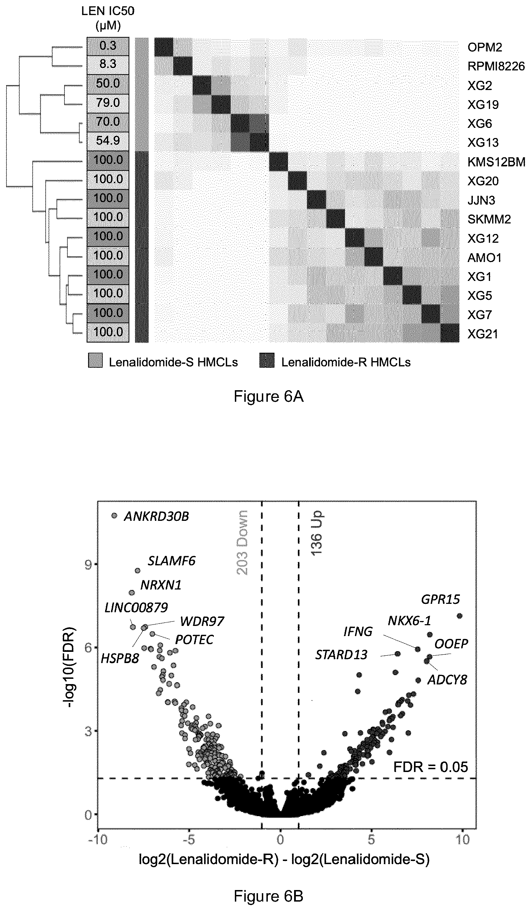

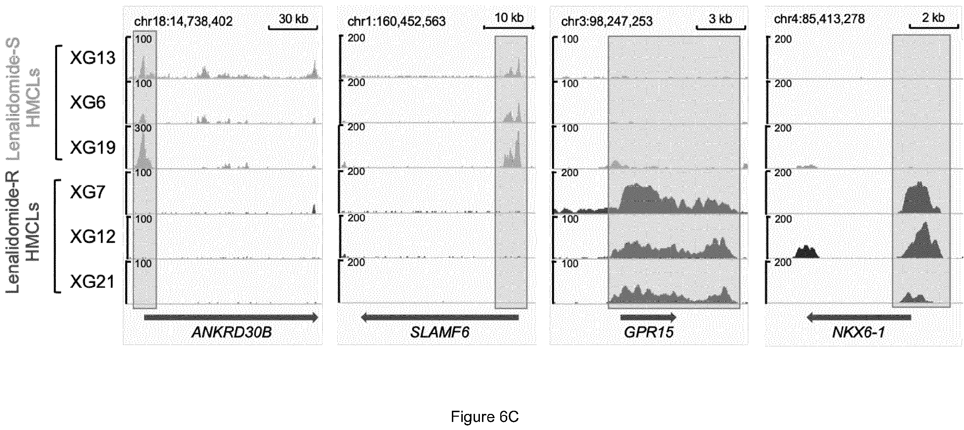

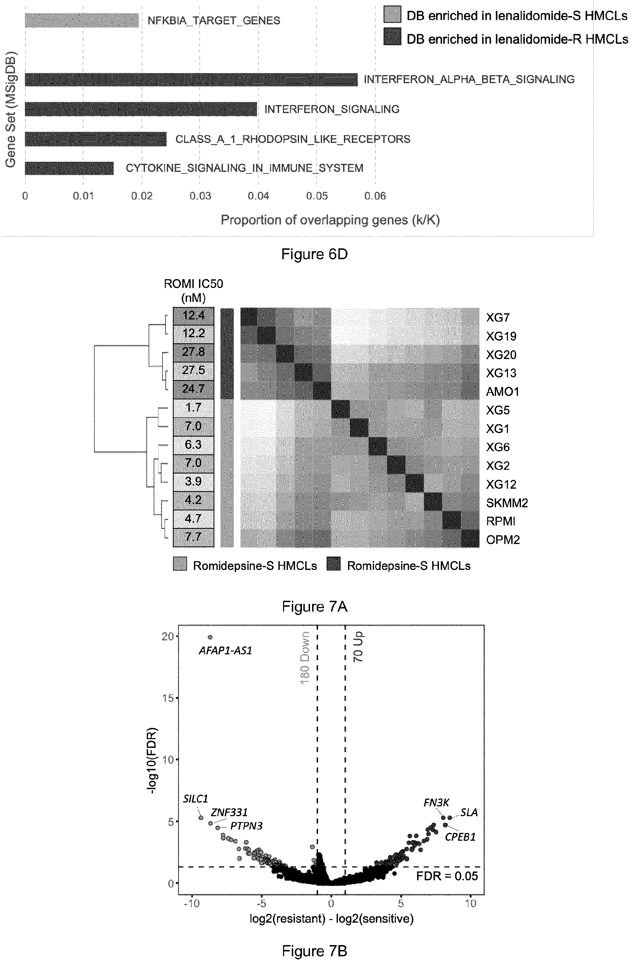

Figure 6: H3K4me3 modification differentially enriched in lenalidomide (LEN) - sensitive and -resistant HMCLs. (A) Correlation heatmap using only the 5903 sites identified as being significantly differentially bound in lenalidomide-resistant compared to lenalidomide-sensitive HMCLs (FDR ≤ 0.05). (B) Volcano plot of differentially bound sites localized on promoters using lenalidomide-resistant vs lenalidomide-sensitive HMCLs contrast. Sites identified as significantly differentially bound in lenalidomide-sensitive and -resistant HMCLs are colored in orange and red, respectively. (C) Gene tracks of H3K4me3 ChIP-seq occupancy. The promoter of ANKRD30B and SLAMF6 genes is the most significantly differentially enriched by H3K4me3 modifications in lenalidomide-sensitive group compared to lenalidomide-resistant group and, conversely, the promoter of GPR15 and NKX6-1 genes is the most significantly differentially enriched by H3K4me3 modifications in lenalidomide-resistant group compared to lenalidomide-sensitive group. The x axis shows the genomic position and the y axis shows signal coverage of ChIP-seq occupancy in units of reads per bin mapped reads. (D) Molecular signature of differentially bound sites localized on gene promoters enriched in lenalidomide-sensitive and -resistant HMCLs was investigated using GSEA database (FDR ≤ 0.05). No overlap was found for gene promoters enriched in lenalidomide-sensitive HMCLs.

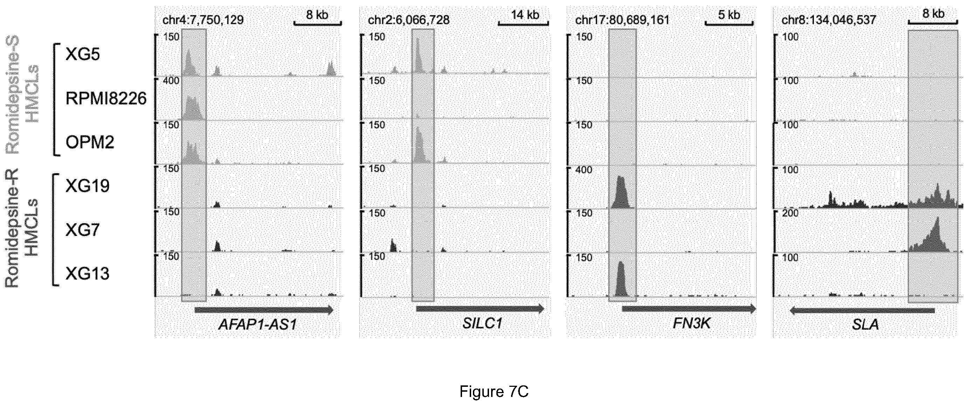

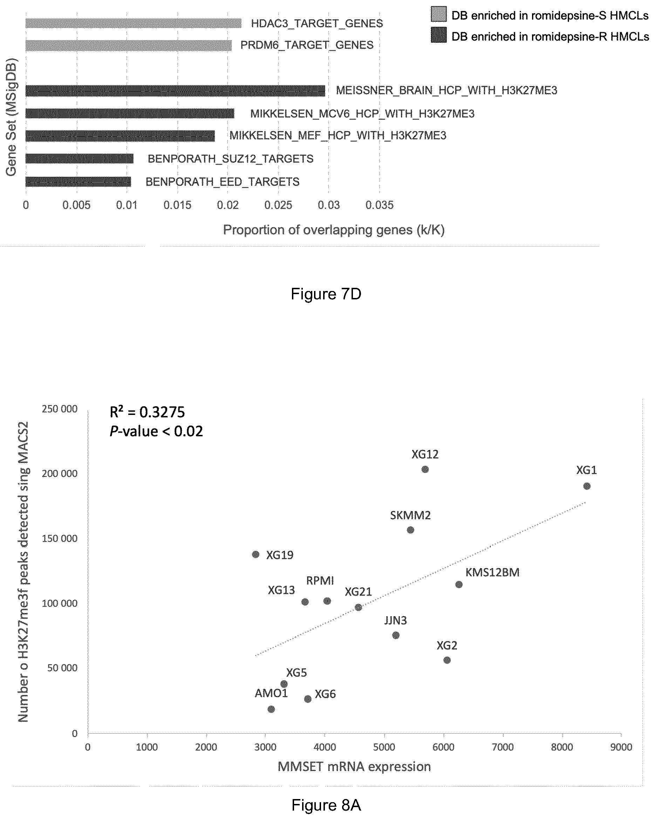

Figure 7: H3K4me3 modification differentially enriched in romidepsin (ROMI)-sensitive and -resistant HMCLs. (A) Correlation heatmap using only the 2098 sites identified as being significantly differentially bound in romidepsin-resistant compared to romidepsin-sensitive HMCLs (FDR ≤ 0.05). (B) Volcano plot of differentially bound sites localized on promoters using romidepsin-resistant vs romidepsin-sensitive HMCLs contrast. Sites identified as significantly differentially bound in romidepsin-sensitive and -resistant HMCLs are colored in orange and red, respectively. (C) Gene tracks of H3K4me3 ChIP-seq occupancy. The promoter of AFAP1-AS1 and SILC1 genes is the most significantly differentially enriched by H3K4me3 modifications in lenalidomide-sensitive group compared to lenalidomide-resistant group and, conversely, the promoter of FN3K and SLA genes is the most significantly differentially enriched by H3K4me3 modifications in lenalidomide-resistant group compared to lenalidomide-sensitive group. The x axis shows the genomic position and the y axis shows signal coverage of ChIP-seq occupancy in units of reads per bin mapped reads. (D) Molecular signature of differentially bound sites localized on gene promoters enriched in romidepsin-sensitive and -resistant HMCLs was investigated using GSEA database (FDR ≤ 0.05).

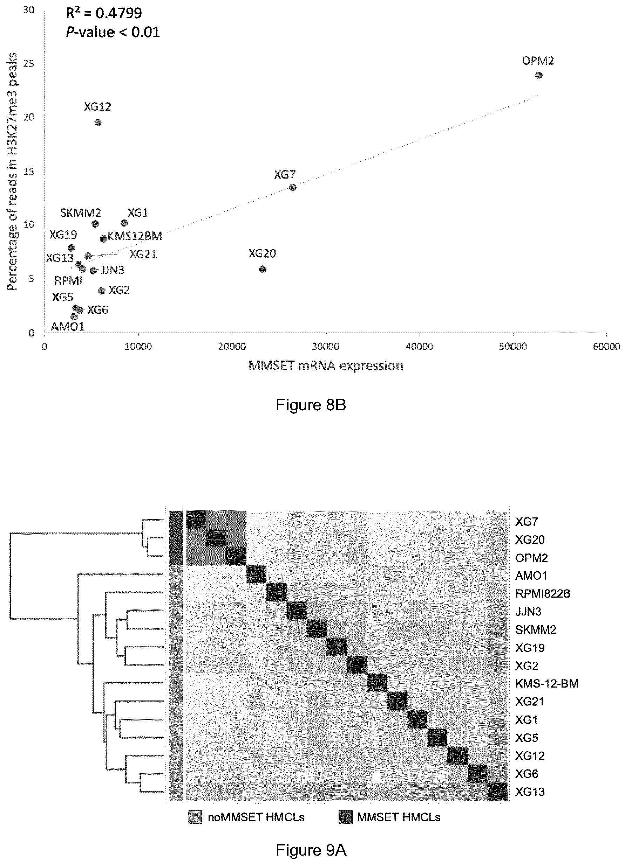

Figure 8 : Correlation between MMSET mRNA expression and H3K27me3 histone modification in HMCLs. (A) Linear regression analysis of the number of H3K27me3 peaks in function of the the MMSET mRNA expression in 13 HMCLs without t(4;14) translocation. (B) Linear regression analysis of the percentage of reads in H3K27me3 peaks (FRiP) in function of the the MMSET mRNA expression in 13 HMCLs without t(4;14) translocation. Coefficient of determination R2 represents the square of the Pearson correlation coefficient (r) (Pearson correlation test).

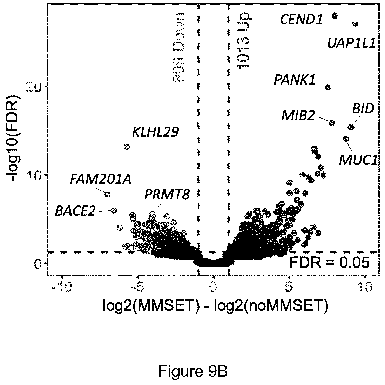

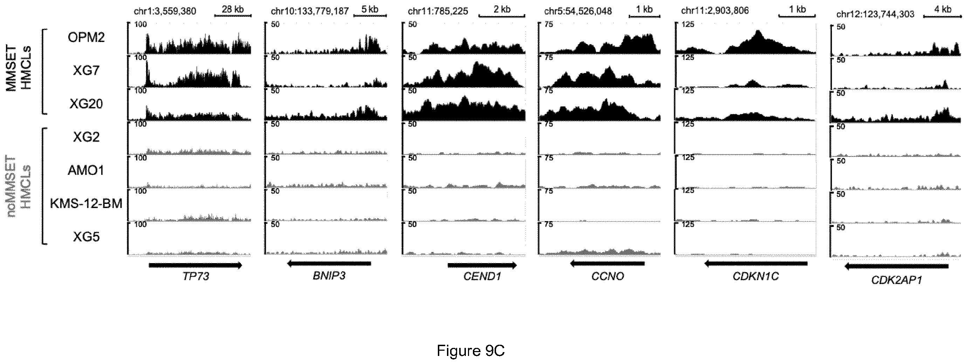

Figure 9 : H3K27me3 modification differentially enriched in HMCLs with and without t(4:14) translocation. (A) Correlation heatmap using only the 45992 sites identified as being significantly differentially bound in MMSET (with t(4;14) translocation) compared to noMMSET (without t(4;14) translocation) HMCLs (FDR ≤ 0.05). (B) Volcano plot of differentially bound sites localized on promoters using MMSET vs noMMSET HMCLs contrast. Sites identified as significantly differentially bound in noMMSET and MMSET HMCLs are colored in orange and red, respectively. (C) Gene tracks of H3K27me3 ChIP-seq occupancy. TP73 and BNIP3 played a role in apoptosis and CEND1, CCNO, CDKN1C and CDK2AP1 were involved in the negative regulation of cell cycle. The x axis shows the genomic position and the y axis shows signal coverage of ChIP-seq occupancy in units of reads per bin mapped reads.

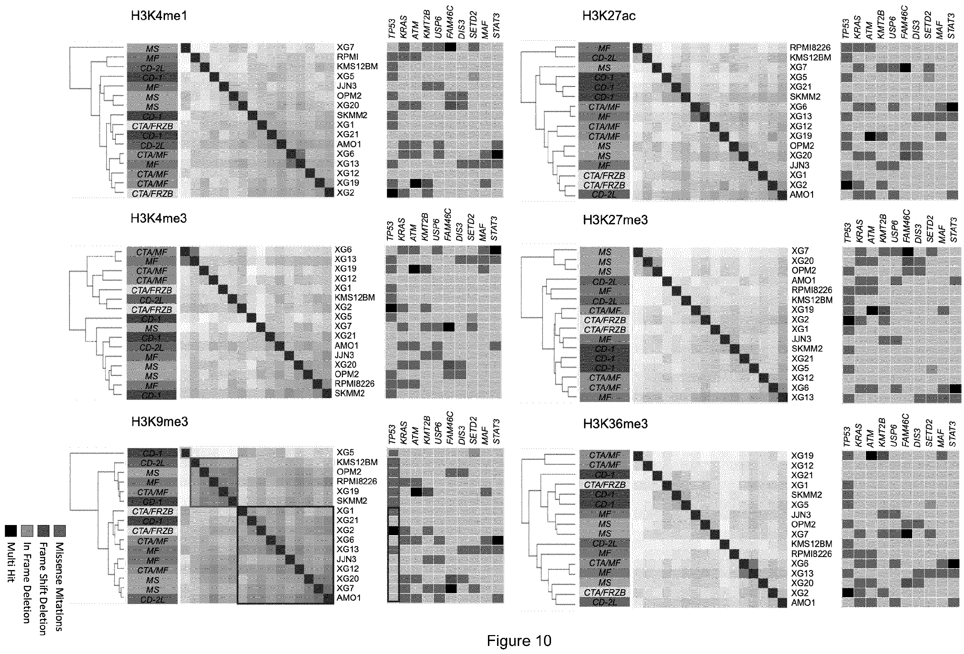

Figure 10 : Link between recurrent mutations and H3K4me1, H3K4me3, H3K9me3, H3K27ac, H3K27me3 and H3K36me3 ChIP-seq profiles in 16 HMCLs. Correlation heatmaps performed using affinity data (read count) suggest a link between H3K9me3 ChIP-seq profile and TP53 mutation. Yellow, orange and brown boxes encompass interest clusters.

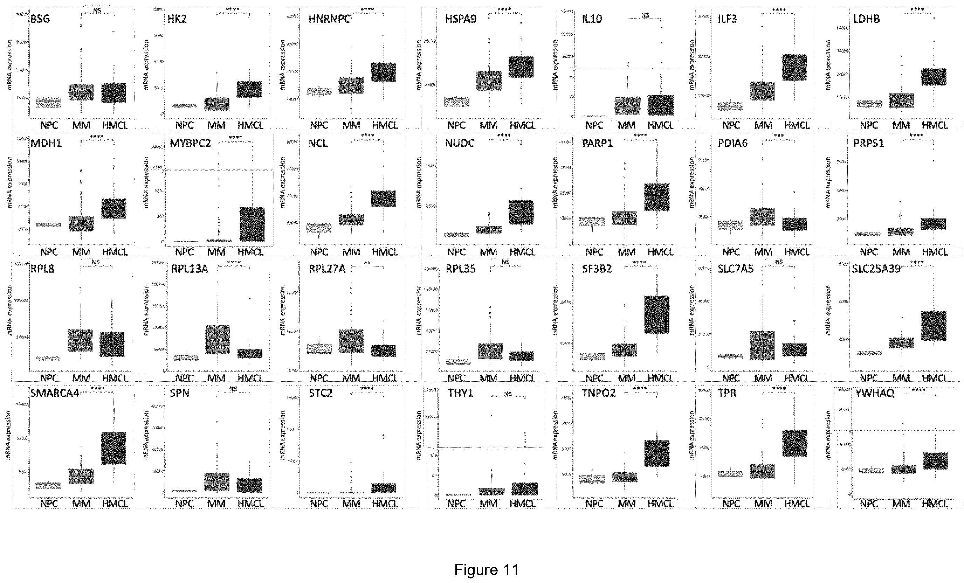

Figure 11 : Boxplots representing mRNA expression of genes composing SE-risk score in normal plasma cells (NPC; n = 3), plasma cells of MM patients (n = 97) and HMCLs (n = 33). Wilcoxon test. NS: non significant, *P-value < 0.05, **P-value < 0.01, ***P-value < 0.001, ****P-value < 0.0001

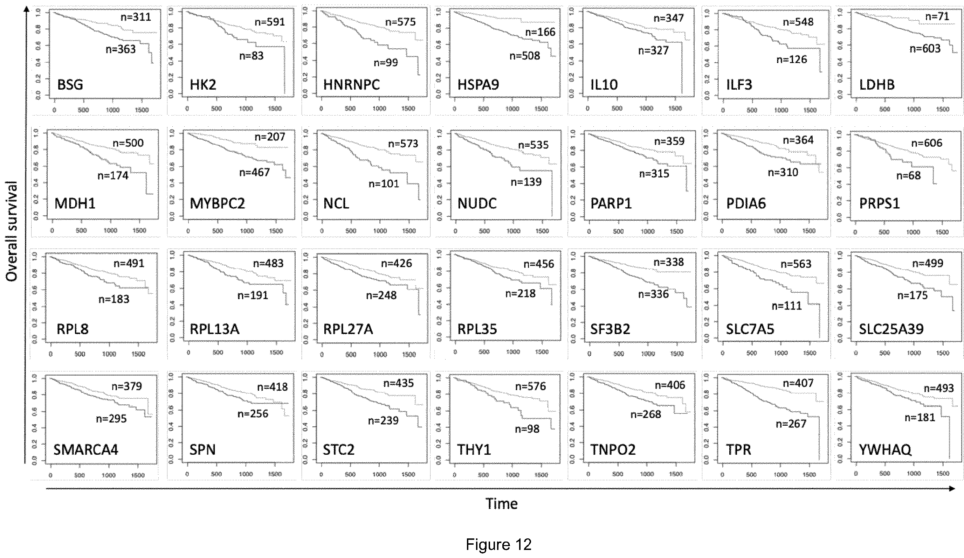

Figure 12 : Kaplan-Meier curves of genes composing SE-risk score in CoMMpass cohort (n = 674). Red curves represent high-risk group associated with high gene expression and green curves correspond to low-risk group associated with low gene expression.

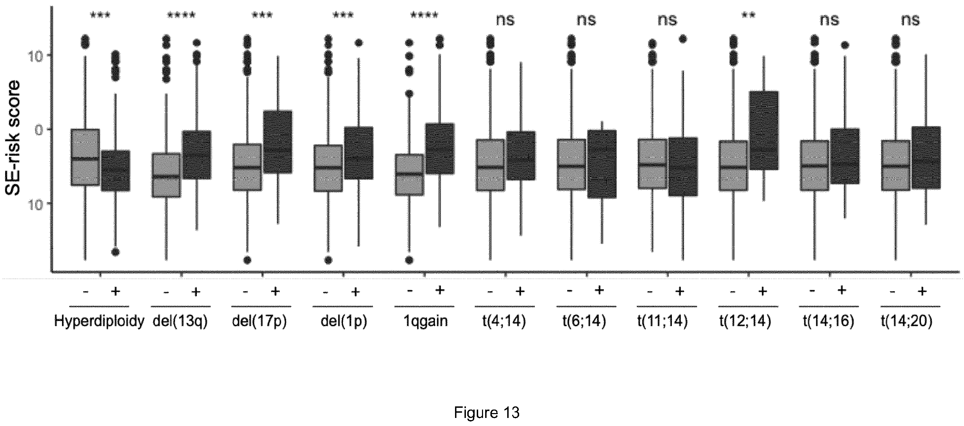

Figure 13 : SE-risk score in cytogenetic abnormality subgroups of the CoMMpass cohort. Cytogenetic abnormality subgroups were composed of hyperdyploid (without: n=240, with: n=331), del(13p) (without: n=307, with: n=266), del(17p) (without: n=523, with: n=50), del(1p) (without: n=455, with: n=118), 1q gain (without: n=378, with: n=195) groups of patients and patients harboring t(4;14) (without: n=515, with: n=73), t(6;14) (without: n=572, with: n=16), t(11;14) (without: n=461, with: n=127), t(12;14) (without: n=564, with: n=24), t(14;16) (without: n=532, with: n=56), t(14;20) (without: n=543, with: n=45) translocations. Wilcoxon test. ns: non significant, *P-value < 0.05, **P-value < 0.01, ***P-value < 0.001, ****P-value < 0.0001.

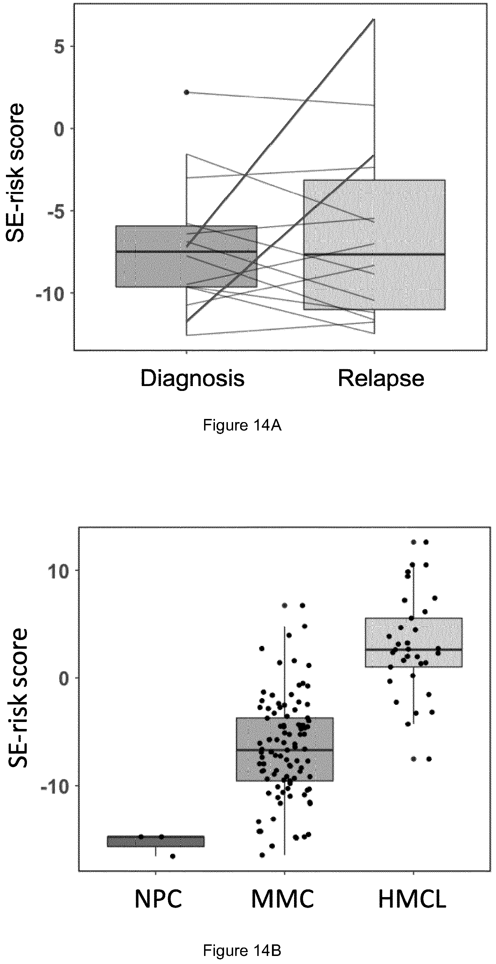

Figure 14 : SE-risk score in normal and tumoral plasma cells. (B) Score evolution in 14 paired diagnosis-relapse samples of MM patients from the Montpellier cohort. Red curves correspond to MM patients with strong increase in the score. (A) The score is calculated in normal plasma cells (NPC; n = 3), plasma cells of MM patients (n = 97) and HMCLs (n = 33).

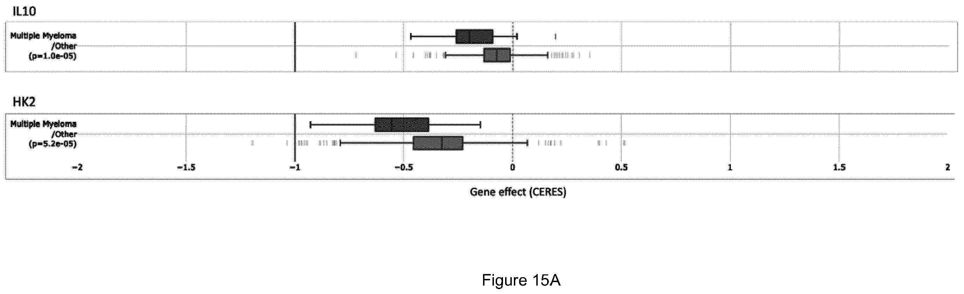

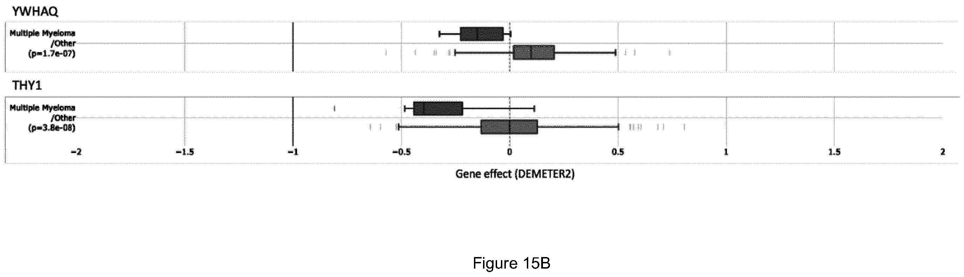

Figure 15 : Snapshots of gene loss-of-function screens from the Cancer Dependency Map project (https://depmap.org). (A) CRISPR/Cas9-based viability assays in MM cell lines (n = 20) and other cancer cell lines (n = 749). (B) RNAi-based viability assays in MM cell lines (n = 12) and other cancer cell lines (n = 534). DEMETER2 and CERES scores were calculated from RNAi-based and CRISPR/Cas9 viability screens, respectively. A lower score means that a gene is more likely to be dependent in a given cell line. A score of 0 is equivalent to a gene that is not essential whereas a score of -1 corresponds to the median of all commun essential genes. Boxplots in pink represent multiple myeloma cell lines and boxplots in grey correspond to other cancer cell lines.

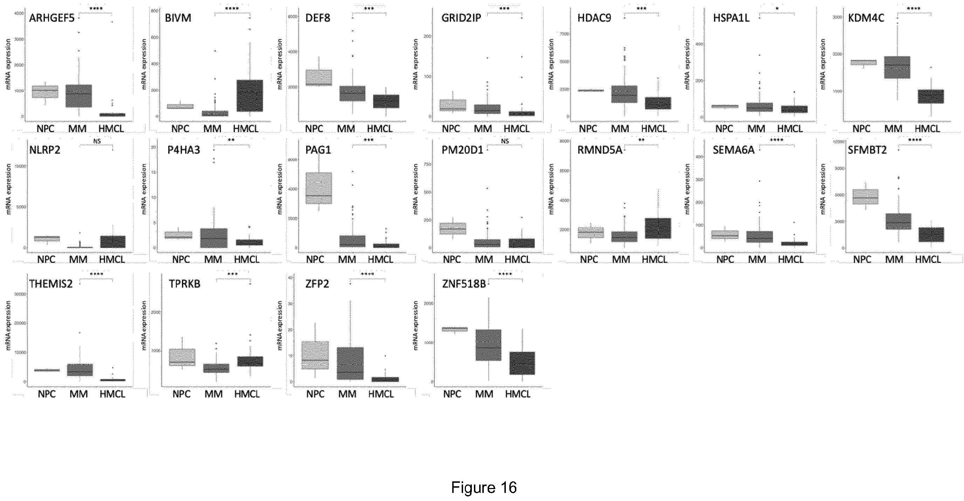

Figure 16 : Boxplots representing mRNA expression of genes composing repressive domain score in normal plasma cells (NPC; n = 3), plasma cells of MM patients (n = 97) and HMCLs (n = 33). Wilcoxon test. NS: non significant, *P-value < 0.05, **P-value < 0.01, ***P-value < 0.001, ****P-value < 0.0001.

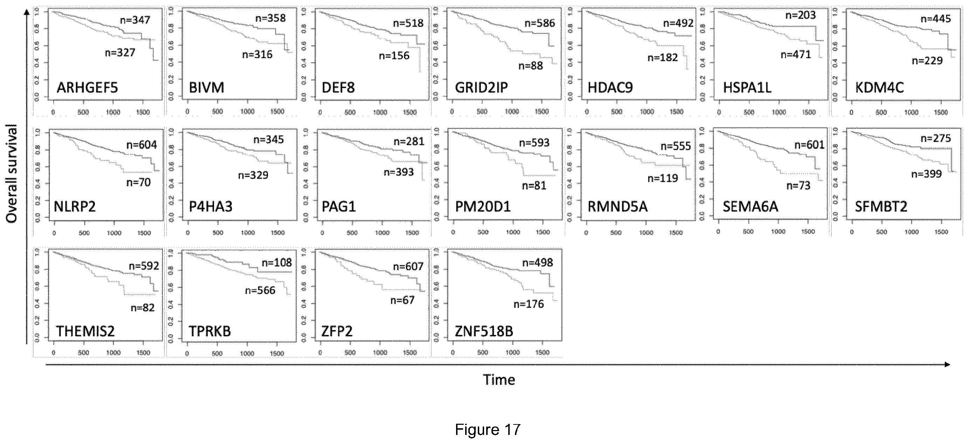

Figure 17 : Kaplan-Meier curves of genes composing repressive domain score in CoMMpass cohort (n = 674). Red curves represent low-risk group associated with high gene expression and green curves correspond to high-risk group associated with low gene expression.

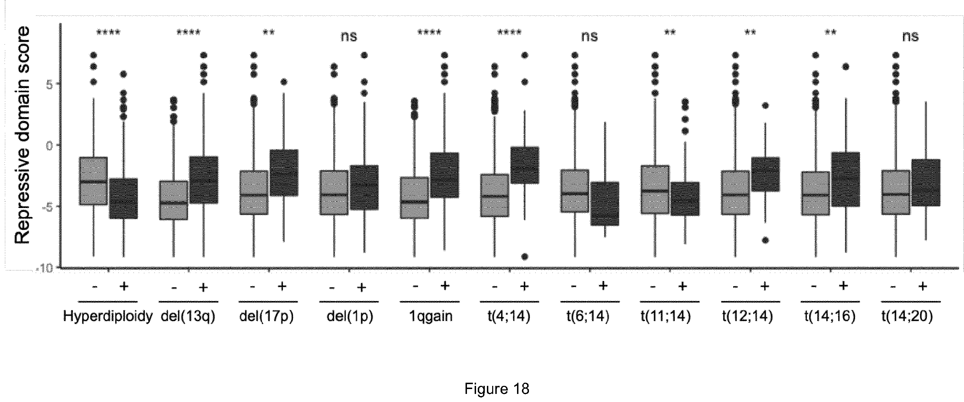

Figure 18: Repressive domain score in cytogenetic abnormality subgroups of the CoMMpass cohort. Cytogenetic abnormality subgroups were composed of hyperdyploid (without: n=240, with: n=331), del(13p) (without: n=307, with: n=266), del(17p) (without: n=523, with: n=50), del(1p) (without: n=455, with: n=118), 1q gain (without: n=378, with: n=195), groups of patients and patients harboring t(4;14) (without: n=515, with: n=73), t(6;14) (without: n=572, with: n=16), t(11;14) (without: n=461, with: n=127), t(12;14) (without: n=564, with: n=24), t(14;16) (without: n=532, with: n=56), t(14;20) (without: n=543, with: n=45) translocations. Wilcoxon test. ns: non significant, *P-value < 0.05, **P-value < 0.01, ***P-value < 0.001, ****P-value < 0.0001.

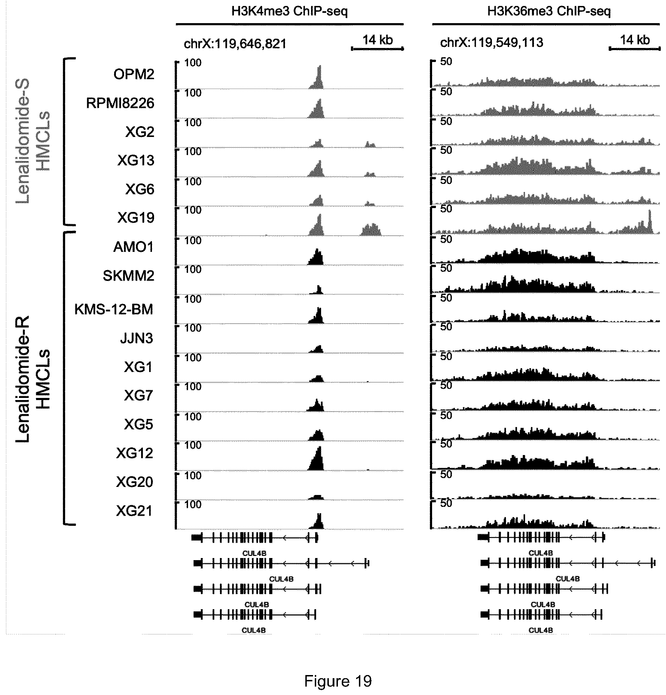

Figure 19 : CUL4B gene tracks of H3K4me3 and H3K36me3 ChIP-seq occupancy in lenalidomide-resistant and -sensitive HMCLs. 4/6 sensitive HMCLs seem to express transcript variant 1 (NM_003588.3) whereas 10/10 resistant HMCLs seem to express transcript variant 2 (NM_001079872.2). The x axis shows the genomic position and the y axis shows signal coverage of ChIP-seq occupancy in units of reads per bin mapped reads.

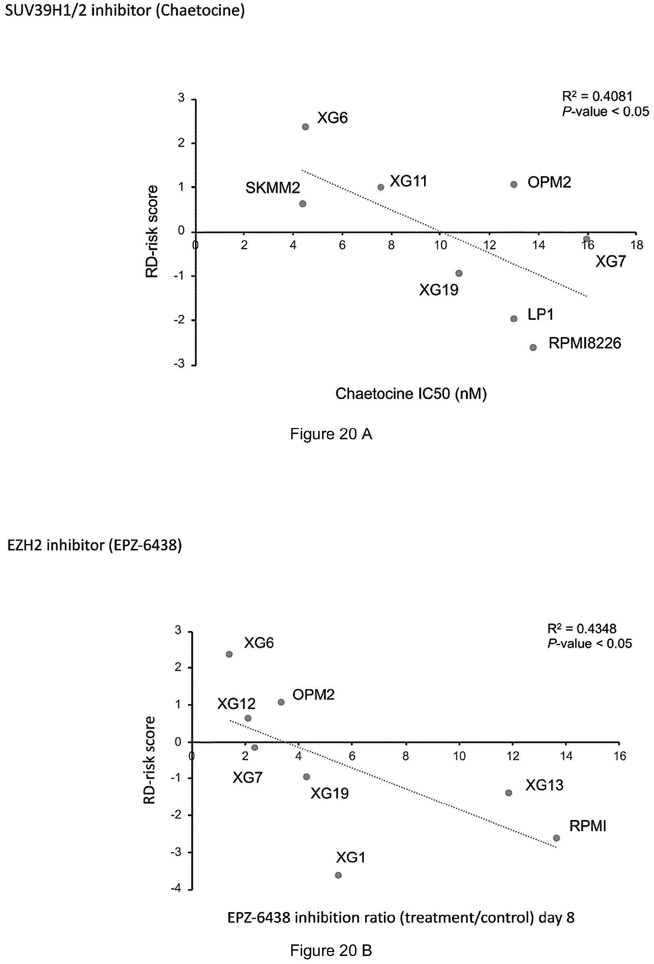

Figure 20 : RD risk score correlates with the response to chaetocine (A) and EPZ-6438 (B) in HMCLs. Linear regression analysis of the RD risk score in function of the IC50 of chaetocine and EPZ-6438 in 8 HMCLs. Coefficient of determination R² represents the square of the Pearson correlation coefficient (r) (Pearson correlation test).

### Detailed description of the invention

The inventors have identified prognostic and theranostic biomarkers in MM, from a comprehensive identification of epigenetic landscape in MM.

They performed chromatin immunoprecipitation sequencing (ChIP-seq) of histone modifications in HCML.

The differential analysis of histone modification profiles on HMCLs highlighted links between histone modifications and cytogenetic abnormalities. The inventors have identified super-enhancers (SE) H3K27ac and H3K4me1, associated with genes involved in the biology of MM. Repressive domains, characterized by co-localization of H3K9me3 and H3K27me3 modifications, on promoters of genes with potential tumor suppressor effect were also identified. Genes of these two subsets associated with prognostic value allow to buil two distinct scores that are able to predict MM patient overall survival.

H3K4me3 sites associated with MM cell sensitivity to IMiD (immunomodulatory drugs) such as lenalidomide and to HDACi (class I histone deacetylase (HDAC) inhibitor) such as romidepsin have also been identified.

The inventors have identified a set of a plurality of genes and/or proteins, which are differentially expressed in individuals having a multiple myeloma as compared to healthy individuals. A score value may be calculated, taking into account the beta coefficient for each gene or protein, based on the Cox statistical model. Said score may be advantageously implemented for prognosis purposes, i.e. to assist physicians classifying individuals between poor prognosis status individuals and good prognosis status individuals.

In particular, the inventors could show that the score value obtained from individuals having a multiple myeloma significantly correlates with the ability of said individuals to respond to a targeted therapeutic treatment. Indeed, individuals with poor prognosis status were prone to respond to a therapeutic treatment, determined according to the specific set of genes associated to at least one epigenetic marker selected from H3K4me1, H3K4me3, H3K9me3, H3K27ac and H3K27me3.

Within the scope of the present invention, the term "individual" refers to a mammal individual, preferably a human individual.

The expression "multiple myeloma individual" refers to an individual having a multiple myeloma.

The expression "multiple myeloma" refers to the multiple myeloma disease such as defined by class C90.0 in accordance with the International Classification of Diseases World Health Organisation Classification (10th revised edition; 2016).

The term "outcome" refers to the survival, the relapse or the death of the individual. The outcome may relate to disease-free survival (DFS), event free survival (EFS) or overall survival (OS), as defined within the state of the art. Illustratively, a "bad outcome" may refer to a disease relapse or death of the individual. Oppositely, a "good outcome" may refer to survival of the individual, with or without relapse episode.

According to an embodiment, the invention is relating to a method for the in vitro prognosis of the likelihood of an individual having MM to be sensitive to a treatment by bromodomain and extra terminal motif (BET) inhibitors, comprising the steps of
a) Measuring in a biological sample, the level of expression of at least five genes selected from the group consisting of BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ,
b) Calculating a risk score from the expression level obtained in step a)
c) Comparing the risk score of the individual to a reference value, and if the risk score is higher than the reference value, classifying the individual as sensitive to the treatment by bromodomain and extra terminal motif (BET) inhibitors.

The inventors have shown that super-enhancers H3K4me3 and/or H3K27ac are associated to 28 key genes contributing to myeloma pathogenesis. Acetylated chromatin, particularly super-enhancer regions, is associated to bromodomain and extra-terminal (BET) proteins to facilitate transcriptional activation. BET inhibitors disrupt the interaction of bromodomains with acetylated histones and leads to loss of enhancer-promoter long-range interactions. The score based on genes associated with super-enhancers allows to identify high-risk MM patients that might benefit from BET inhibitors.

The prognostic value of MYBPC2, NUDC, RPL27A, SF3BS, SLC25A39, TNPO2, TPR, YWHAQ transcriptional deregulation has not been described before. Interestingly, among these genes associated with SE in MM, YWHAQ, IL10, HK2 and THY1 were identified as significant essential MM genes in RNAi or CRISPR screening corroborating epigenetic and transcriptional modifications at a functional level.

Within the scope of the present invention, it has to be understood that the expression level of any combination of 5, 6, 7, 8, 9, 10, 11, 12, 13, 14, 15, 16, 17, 18, 19, 20, 21, 22, 23, 24, 25, 26, 27 and 28 genes and/or proteins encoded by the said 5, 6, 7, 8, 9, 10, 11, 12, 13, 14, 15, 16, 17, 18, 19, 20, 21, 22, 23, 24, 25, 26, 27 and 28 genes, the said genes being selected in a group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is of special interest.

In other words, each of the combination of 5, 6, 7, 8, 9, 10, 11, 12, 13, 14, 15, 16, 17, 18, 19, 20, 21, 22, 23, 24, 25, 26, 27 and 28 genes and/or proteins encoded by the said 5, 6, 7, 8, 9, 10, 11, 12, 13, 14, 15, 16, 17, 18, 19, 20, 21, 22, 23, 24, 25, 26, 27 and 28 genes, the said genes being selected in a group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is explicitly disclosed herein.

In some embodiment, the expression level of at least 10 genes and/ or proteins encoded by the said genes, the said genes being selected in the group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is measured at step a.

In some embodiment, the expression level of at least 15 genes and/ or proteins encoded by the said genes, the said genes being selected in the group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is measured at step a.

In some embodiment, the expression level of at least 20 genes and/ or proteins encoded by the said genes, the said genes being selected in the group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is measured at step a.

In some embodiment, the expression level of at least 25 genes and/ or proteins encoded by the said genes, the said genes being selected in the group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ is measured at step a.

In an embodiment of the invention, the expression level of the 28 genes and/ or proteins encoded by the said 28 genes is measured at step a.

In particular, in the embodiments disclosed here-above, the expression level of at least 5 genes and/ or proteins encoded by said genes, the said genes being selected from HK2, HNRNPC, HSPA9, ILF3, LDHB, MDH1, MYBPC2, NCL, NUDC, PARP1, PDIA6, PRPS1, RPL13A, RPL27A, SF3B2, SLC25A39, SMARCA4, STC2, TNPO2, TPR and YWHAQ is measured at step a.

In some embodiments, the expression level of at least five genes selected from MYBPC2, NUDC, RPL27A, SF3BS, SLC25A39, TNPO2, TPR and YWHAQ is measured at step a).

The individual having MM may originate from the general population of MM individuals, from early stage MM individuals, from intermediary stage MM individuals, from late stage MM individuals, from MM individuals not undergoing therapeutic treatment, from MM individuals not undergoing therapeutic treatment but having experienced at least one previous therapeutic treatment, from MM individuals undergoing therapeutic treatment, from MM individuals experiencing a refractory/relapsing MM, and a combination thereof.

Within the scope of the present invention, a "biological sample" refers to a biological sample obtained, reached, collected or isolated from an individual, in vivo or in situ. Such samples may be, but not limited to, organs, tissues, fractions and cells isolated from an individual. For example, suitable biological samples include but are not limited to a cell culture, a cell line, a tissue biopsy such as a bone marrow aspirate, a biological fluid such as a blood, pleural effusion or a serum sample, and the like.

In certain embodiments, the preferred biological sample includes but is not limited to a blood sample, a tissue biopsy, including a bone marrow aspirate.

In some embodiments, the biological sample may be a crude sample.

In some other embodiments, the biological sample may be purified to various degrees prior to storage, processing, or measurement.

The expression level of the defined set of genes and/or proteins may be measured by the mean of any technique used in the field.

In some embodiments, the expression level of a gene of interest may be measured through the quantification of the level of mRNA expression.

In some embodiments, the level of mRNA expression for each of the genes of interest may be performed using the well-known techniques available in the state of the art.

Illustratively, mRNA may be extracted, for example using lytic enzymes or chemical solutions or extracted by commercially available nucleic-acid-binding resins following the manufacturer's instructions. Extracted mRNA may be subsequently detected by hybridization, such as Northern blot, and/or amplification, such as quantitative or semiquantitative RT-PCR. Other methods of amplification include ligase chain reaction (LCR), transcription-mediated amplification (TMA), strand displacement amplification (SDA) and nucleic acid sequence based amplification (NASBA).

In some embodiments, the level of mRNA expression for each of the genes of interest may be measured by the mean of quantification of the cDNA synthesized from said mRNA, as a template, by one reverse transcriptase.

Methods for determining the quantity of mRNA by microarrays or by RNA sequencing may also be used.

In certain embodiments, complexes between the double-stranded nucleic acids resulting from amplification and fluorescent SYBR^{®} molecules may be obtained and then the fluorescence signal generated by the SYBR^{®} molecules complexed with the said amplified nucleic acids may be measured.

Identification of suitable primers that are specific for each of the genes mRNA consists of a routine work for the one skilled in the art.

In certain embodiments, detection by hybridization may be performed with a detectable label, such as fluorescent probes, radioactive probes, enzymatic reactions or other ligands (e.g. avidin/biotin).

Protein expression level may be measured by well-known techniques including detection and quantification of the protein of interest by the means of any type of ligand molecule that specifically binds thereto, including nucleic acids (for example nucleic acids selected for binding through the well-known SELEX method), antibodies and antibody fragments.

Yet illustratively, antibodies to said given protein of interest may be easily obtained with the conventional techniques, including generation of antibody-producing hybridomas.

Hybridomas prepared by conventional techniques are then screened using standard methods to identify one or more hybridomas which produce an antibody which specifically binds with the biological marker protein or a fragment thereof. The invention also encompasses hybridomas made by this method and antibodies made using such hybridomas. Polyclonal antibodies may be used as well.

Thus, in preferred embodiments, expression of a marker is assessed using for example:
- a radio-labelled antibody, in particular, a radioactive moiety suitable for the invention may for example be selected within the group comprising 3H, 1211, 1231, 99mTc, 14C or 32P;
- a chromophore-labelled or a fluorophore-labelled antibody, wherein a luminescent marker, and in particular a fluorescent marker, suitable for the invention may be any marker commonly used in the field such as fluorescein, BODIPY, fluorescent probes type ALEXA, coumarin and its derivatives, phycoerythrin and its derivatives, or fluorescent proteins such as GFP or the DsRed;
- a polymer-backbone-antibody;
- an enzyme-labelled antibody, said labelling enzyme suitable for the invention may be an alkaline phosphatase, a tyrosinase, a peroxydase, or a glucosidase; for example, suitable avidin-labelled enzyme may be an avidin-Horse Radish Peroxydase (HRP), and a suitable substrate may be AEC, 5-bromo-4-chloro-3-indolyl phosphate (BCIP), nitro blue tetrazolium chloride (NBT);
- an antibody derivative, for example an antibody conjugated with a substrate or with the protein or ligand of a protein-ligand pair, in particular a biotin, a streptavidin or an antibody binding the polyhistidine tag;
- an antibody fragment, for example a single-chain antibody, an isolated antibody hypervariable domain, etc., which binds specifically to a marker protein or a fragment thereof, including a marker protein which has undergone all or a portion of its normal post-translational modification.

In vitro techniques for detection of a biological marker protein include enzyme linked immunosorbent assays (ELISAs), Western blots, immunoprecipitations and immunofluorescence.

The expression level for each combination of genes and/or protein of interest may be associated with a score value.

Illustratively, following the measurement of the expression level of at least 5 or more genes and/or proteins encoded by the said 5 or more genes selected in a group comprising BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ, in a biological sample obtained from an individual having MM (step a) of the method), the computation of a score value may be performed be a method comprising the following steps:
- i) comparing the expression level determined at step a) with a predetermined reference expression level (PREL);
- ii) calculating the score value with the following formula: $Score = {\sum }_{i = 1}^{n} βi \times Ci$ wherein:
   - n represents the number of genes and/or protein which expression level is measured, i.e. n being comprised from 5 to 28,
   - βi represents the regression β coefficient reference value for a given gene or protein, and
   - Ci represents 1 if the expression level of said gene or protein is higher than the predetermined reference level (PREL) or Ci represents -1 if the expression level of the gene or the protein is lower than or equal to the predetermined reference level (PREP).

The predetermined reference level (PREL) is often referred as to "maxstat value" or "maxstat cutpoint".

The regression β coefficient reference value may be easily determined by the skilled man in the art for each gene or protein using the well-known statistical Cox model, which is based on a modelling approach to analyse survival data. The purpose of the model is to simultaneously explore the effects of several variables on survival. When it is used to analyse the survival of patients in a clinical trial, the model allows isolating the effects of the treatment from the effects of other variables.

The Cox model may also be referred as to proportional hazards regression analysis. In particular, this model is a regression analysis of the survival times (or more specifically, the so-called hazard function) with respect to defined variables. The hazard function is the probability that an individual will experience an event, e.g. death, within a small time interval, given that the individual has survived up to the beginning of the interval. It can therefore be interpreted as the risk of dying at time t. The quantity h0 (t) is the baseline or underlying hazard function and corresponds to the probability of dying (or reaching an event) when all the defined variables are zero. The baseline hazard function is analogous to the intercept in ordinary regression (since exp0= 1). The regression coefficient β gives the proportional change that can be expected in the hazard, related to changes in the defined variables. The coefficient β is estimated by a statistical method called maximum likelihood. In survival analysis, the hazard ratio (HR) (Hazard Ratio = exp(β)) is the ratio of the hazard rates corresponding to the conditions described by two sets of defined variables. For example, in a drug study, the treated population may die at twice the rate per unit time as the control population. The hazard ratio would be 2, indicating higher hazard of death from the treatment.

Predetermined reference values, such as PREL or PRV, which are used for comparison purposes may consist of "cut-off' values.

For example, each reference ("cut-off") value PREL for each gene or protein may be determined by carrying out a method comprising the following steps:
a) providing a collection of samples from patients suffering from multiple myeloma;
b) determining the expression level of the relevant gene or protein for each sample contained in the collection provided at step a);
c) ranking the samples according to said expression level;
d) classifying said samples in pairs of subsets of increasing, respectively decreasing, number of members ranked according to their expression level;
e) providing, for each sample provided at step a), information relating to the actual clinical outcome for the corresponding cancer patient (i.e. the duration of the disease-free survival (DFS), or the event free survival (EFS) or the overall survival (OS) or both);
f) for each pair of subsets of tumour tissue samples, obtaining a Kaplan Meier percentage of survival curve;
g) for each pair of subsets of tumour tissue samples calculating the statistical significance (p value) between both subsets;
h) selecting as reference value PREL for the expression level, the value of expression level for which the p value is the smallest.

Illustratively, the expression level of a gene or a protein of interest may be assessed for 100 samples of 100 patients. The 100 samples are ranked according to the expression level of said given gene or protein. Sample 1 may have the highest expression level and sample 100 may have the lowest expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves may be prepared for each of the 99 groups of two subsets. Also for each of the 99 groups, the p value between both subsets was calculated. The reference value PREL is then selected such as the discrimination based on the criterion of the minimum p value is the strongest. In other words, the expression level corresponding to the boundary between both subsets for which the p value is minimum is considered as the reference value. It should be noted that according to the experiments made by the inventors, the reference value PREL is not necessarily the median value of expression levels.

The man skilled in the art also understands that the same technique of assessment of the PRV could be used for obtaining the reference value and thereafter for assessment of the response to the combination treatment of the present invention. However in one embodiment, the reference value PRV is the median value of PRV.

In certain embodiments, the regression β coefficient reference value, the hazard ratio and the reference value PREP for each gene or protein of interest is described in Table A below.

**Table A : SE-risk score cutpoint and β-coefficient**

| ENSEMBL | Gene | cutPoint | beta |
|---|---|---|---|
| ENSG00000113013 | HSPA9 | 95.867 | 1.244 |
| ENSG00000047410 | TPR | 26.936 | 1.060 |
| ENSG00000111716 | LDHB | 118.079 | 0.993 |
| ENSG00000086967 | MYBPC2 | 0.006 | 0.907 |
| ENSG00000115053 | NCL | 258.067 | 0.890 |
| ENSG00000092199 | HNRNPC | 331.738 | 0.828 |
| ENSG00000087365 | SF3B2 | 77.162 | 0.798 |
| ENSG00000090273 | NUDC | 33.706 | 0.754 |
| ENSG00000103257 | SLC7A5 | 98.514 | 0.734 |
| ENSG00000014641 | MDH1 | 132.639 | 0.689 |
| ENSG00000113739 | STC2 | 0.041 | 0.668 |
| ENSG00000147224 | PRPS1 | 0.090 | 0.648 |
| ENSG00000143870 | PDIA6 | 0.011 | 0.641 |
| ENSG00000172270 | BSG | 19.268 | 0.636 |
| ENSG00000129351 | ILF3 | 19.453 | 0.615 |
| ENSG00000013306 | SLC25A39 | 107.200 | 0.609 |
| ENSG00000154096 | THY1 | 0.037 | 0.600 |
| ENSG00000159399 | HK2 | 0.000 | 0.571 |
| ENSG00000142541 | RPL13A | 0.000 | 0.560 |
| ENSG00000105576 | TNPO2 | 22.473 | 0.529 |
| ENSG00000143799 | PARP1 | 0.431 | 0.482 |
| ENSG00000161016 | RPL8 | 26.306 | 0.482 |
| ENSG00000166441 | RPL27A | 5.622 | 0.477 |
| ENSG00000134308 | YWHAQ | 4.000 | 0.470 |
| ENSG00000136634 | IL10 | 15.426 | 0.450 |
| ENSG00000136942 | RPL35 | 9.575 | 0.417 |
| ENSG00000127616 | SMARCA4 | 29.706 | 0.404 |
| ENSG00000197471 | SPN | 1.900 | 0.378 |

In certain embodiments, the score may be generated by a computer program.

The BET inhibitor can in particular be selected from OTX015 (NCT01713582), CPI-0610 (NCT02157636), GSK525762 (NCT01943851), and RO6870810 (NCT03068351).

In another aspect, the invention is directed to a method for the in vitro prognosis of the likelihood of an individual having MM to be sensitive to a therapeutic treatment, comprising the following steps
a) Measuring in a biological sample, the level of expression of at least five genes selected from the group consisting of ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B,
b) Calculating a risk score from the expression level obtained in step a)
c) Comparing the risk score of the individual to a reference value, and if the risk score is higher than the reference value, classifying the individual as sensitive to the treatment by a drug modifying the level of H3K9me3 and/or H3K27me3.

In preferred embodiments, the drug modifying the level of H3K9me3 and/or H3K27me3 is selected from SUV39 H1 inhibitors, SUV39H2 inhibitors and Polycomb repressive complex (PRC) 2 inhibitors.

In a preferred embodiment, PRC2 inhibitors are selected from EZH2 (enhancer of zest homolog 2) inhibitors and EED inhibitors.

The EZH2 inhibitor may in particular be selected in a group comprising CPI-169, EI-1, EPZ-005687, EPZ-6438 (Tazemetostat), GSK-126, GSK-343, GSK-503, DZNep and UNC-1999.

The EED inhibitor may in particular be MAK683.

The SUV39H1 inhibitor can be in particular chaetocin.

The inventors have identified 18 potential suppressor tumor genes enriched in H3K9me3 and H3K27me3 repressive marks on their promoter. The tumor suppressor function of these genes has not yet been described in MM. Some of them are involved in cell proliferation inhibition. NLRP2 gene, coding a member of the nucleotide binding and leucine-rich repeat receptor (NLR), is an inhibitor of NF-kB pathway which play a key role in survival and proliferation of myeloma cells.

From these 18 genes a score was built, identifying high-risk group of MM patients with global low expression of putative suppressor genes associated with H3K9me3 and H3K27me3 marks. These patients could benefit from combined therapeutic targeting of EZH2 and SUV39H1 histone methyltransferases.

In particular, the at least five genes in step a can be selected from ARHGEF5, DEF8, GRID2IP, HDAC9, HSPA1L, KDM4C, P4HA3, PAG1, SEMA6A, SFMBT2, THEMIS2, ZFP2 and ZNF518B.

Within the scope of the present invention, it has to be understood that the expression level of any combination of 5, 6, 7, 8, 9, 10, 11, 12, 13, 14, 15, 16, 17 and 18 genes and/or proteins encoded by the said 5, 6, 7, 8, 9, 10, 11, 12, 13, 14, 15, 16, 17 and 18 genes, the said genes being selected in a group comprising ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B is of special interest.

In other words, each of the combination of 5, 6, 7, 8, 9, 10, 11, 12, 13, 14, 15, 16, 17 and 18 genes and/or proteins encoded by the said 5, 6, 7, 8, 9, 10, 11, 12, 13, 14, 15, 16, 17 and 18 genes, the said genes being selected in a group comprising ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B is explicitly disclosed herein.

In some embodiments, the expression level of at least 10 genes and/ or proteins encoded by the said genes, the said genes being selected in the group comprising ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B is measured at step a).

In some embodiments, the expression level of at least 15 genes and/ or proteins encoded by the said genes, the said genes being selected in the group comprising ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B is measured at step a).

In a particular embodiment, the expression level of the 18 genes and/or proteins encoded by said genes is measured at step a).

The individual having MM may originate from the general population of MM individuals, from early stage MM individuals, from intermediary stage MM individuals, from late stage MM individuals, from MM individuals not undergoing therapeutic treatment, from MM individuals not undergoing therapeutic treatment but having experienced at least one previous therapeutic treatment, from MM individuals undergoing therapeutic treatment, from MM individuals experiencing a refractory/relapsing MM, and a combination thereof.

The expression level of the defined set of genes and/or proteins may be measured by the mean of any technique used in the field, and in particular as disclosed here-above.

Following the measurement of the expression level of at least 5 or more genes and/or proteins encoded by the said 5 or more genes selected in a group comprising ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B, in a biological sample obtained from an individual having MM (step a) of the method), the computation of a score value may be performed be a method comprising the following steps:
- i) comparing the expression level determined at step a) with a predetermined reference expression level (PREL);
- ii) calculating the score value with the following formula: $Score = {\sum }_{i = 1}^{n} βi \times Ci$ wherein:
   - n represents the number of genes and/or protein which expression level is measured, i.e. n being comprised from 5 to 28,
   - βi represents the regression β coefficient reference value for a given gene or protein, and
   - Ci represents 1 if the expression level of said gene or protein is higher than the predetermined reference level (PREL) or Ci represents -1 if the expression level of the gene or the protein is lower than or equal to the predetermined reference level (PREP).
The predetermined reference level (PREL) is often referred as to "maxstat value" or "maxstat cutpoint".

The regression β coefficient reference value may be easily determined by the skilled man in the art for each gene or protein using the well-known statistical Cox model, which is based on a modelling approach to analyse survival data. The purpose of the model is to simultaneously explore the effects of several variables on survival. When it is used to analyse the survival of patients in a clinical trial, the model allows isolating the effects of the treatment from the effects of other variables.

As disclosed above, in certain embodiments, the regression β coefficient reference value, the hazard ratio and the reference value PREP for each 18 gene or protein of interest is described in Table B below.

**Table B : RD-risk score cutpoint and β-coefficient calculated**

| ENSEMBL | Gene | cutPoint | beta |
|---|---|---|---|
| ENSG00000215045 | GRID2IP | 9.787 | -0.858 |
| ENSG00000092421 | SEMA6A | 0.672 | -0.743 |
| ENSG00000144034 | TPRKB | 4.297 | -0.694 |
| ENSG00000198939 | ZFP2 | 0.646 | -0.693 |
| ENSG00000022556 | NLRP2 | 0.006 | -0.659 |
| ENSG00000107077 | KDM4C | 14.529 | -0.607 |
| ENSG00000048052 | HDAC9 | 23.035 | -0.597 |
| ENSG00000134897 | BIVM | 110.776 | -0.564 |
| ENSG00000130775 | THEMIS2 | 2.729 | -0.555 |
| ENSG00000162877 | PM20D1 | 0.038 | -0.526 |
| ENSG00000178163 | ZNF518B | 15.918 | -0.524 |
| ENSG00000204390 | HSPA1L | 161.553 | -0.510 |
| ENSG00000198879 | SFMBT2 | 16.647 | -0.508 |
| ENSG00000140995 | DEF8 | 4.124 | -0.460 |
| ENSG00000153561 | RMND5A | 28.722 | -0.451 |
| ENSG00000076641 | PAG1 | 2.070 | -0.433 |
| ENSG00000050327 | ARHGEF5 | 1.523 | -0.402 |
| ENSG00000149380 | P4HA3 | 54.774 | -0.390 |

A further object if the invention is a method for the in vitro prognosis of an individual having MM to be sensitive to a therapeutic treatment, comprising the following steps :
a) Measuring in a biological sample the presence and the level of H3K4me3 in at least five genes selected from mentioned in Table 1 and Table 2,
b) Classifying the individual having a significant level of H3K4me3 associated to at least five genes selected from Table 1 and/or having no significant level of H3K4me3 associated to at least five genes selected from Table 2 as sensitive to a drug selected from IMiD (Immunomodulator drug).

The method for the *in vitro* prognosis of the likelihood of a individual having multiple myeloma to be sensitive to a therapeutic treatment comprising the following steps
a) Measuring in a biological sample the presence and the level of H3K4me3 in at least five genes selected from genes listed in table 1 and/or table 2,
b) Classifying the individual having a significant level of H3K4me3 of at least five genes selected from the genes listed in table 1 and/or having no significant level of H3K4me3 of at least five genes selected from the genes listed in table 2 as sensitive to a drug which is an IMiD.

By "significant" is intended a value which is higher than a reference value. The reference value can be 0 or can be a threshold previously determined.

On the other hand, individual having a significant level of H3K4me3 of at least five genes selected from the genes listed in table 2 and/or having no significant level of H3K4me3 of at least five genes selected from the genes listed in table 1 will be classified as resistant to a drug which is an IMiD

An immunomodulatory drug, also referred as "IMiD", is. a structural and functional analogue of thalidomide.

Illustratively, IMiD compounds encompass lenalidomide, pomalidomide, and derivative thereof, as well as compounds disclosed by Knight (Semin Oncol. 2005 Aug;32(4 Suppl 5):S24-30).

In certain embodiments, the immunomodulatory agent is selected in a group comprising thalidomide, lenalidomide, pomalidomide and derivatives thereof. In a preferred embodiment, the individual is classified as sensitive to lenalidomide.

The relationship between differential enrichment of H3K4me3 on gene promoters in HMCLs and response to treatment has been analyzed by the inventors. An epigenetic biomarker based on this histone modification has been developed, which predicts lenalidomide and romidepsin responses in HMCLs. IMIDs are known to target the cereblon complex. Cereblon is composed of CUL4, RBX1, DDB1 and CRBN proteins and induces the ubiquitination of B-cell transcription factors IKZF1 and IKZF3 in presence of IMiDs. Among the genes encoding these proteins, a significant difference in H3K4me3 enrichment on the promoter of CUL4B in lenalidomide-sensitive HMCLs compared to resistant HMCLs was observed (Figure 19) .

Interestingly, the presence of the CUL4B splicing variant 1 seemed to be associated with lenalidomide sensitivity whereas the other variant could play a role in lenalidomide resistance.

The genes composing the repressive domain score may in particular be selected from ARHGEF5, BIVM, DEF8, GRID2IP, HDAC9, HSPA11, KDM4C, NLRP2, P4HA3, PAG1, PM20D1, RMND5A, SEMA6A, SFMBT2, THEMIS2, TPRKB, ZFP2 and ZNF518

In particular, an individual sensitive to IMID such as lenalidomide, H3K4me3 will be associated to at least one and preferably all the genes selected from OPM2, RPMI8226, XG2, XG13, XG6 and XG19. No or negligible H3K4me3 will be associated to genes selected from KMS-12-BM, JJN3, XG1, XG7, XG5, XG12, XG20 and XG21.

In yet another aspect, the invention is relating to a method for the in vitro prognosis of an individual having MM to be sensitive to a therapeutic treatment, comprising the following steps
a) Measuring in a biological sample the presence and the level of H3K4me3 in at least five genes mentioned in Table 3 and/or Table 4,
b) Classifying the individual having a significant level of H3K4me3 associated to at least five genes selected from selected from Table 3 and/or having no significant level of H3K4me3 associated to at least five genes selected from Table 4 as sensitive to a drug selected from histone desacetylase inhibitors (HDACi).

On the other hand, individual having a significant level of H3K4me3 of at least five genes selected from the genes listed in table 4 and/or having no significant level of H3K4me3 of at least five genes selected from the genes listed in table 3 will be classified as resistant to a drug selected from histone desacetylase inhibitors.

HDACi may be selected from Panobinostat and romidepsine ; in a preferred embodiment, the individual will be classified as sensitive to romidepsine.

In particular, an individual sensitive to HDACi such as romidepsine, H3K4me3 will be associated to at least one and preferably all the genes selected from XG5, XG1, XG6, XG2, XG12, SKMM2, RPMI8226 and OPM2. No or negligible H3K4me3 will be associated to genes selected from XG19, XG7, XG20, AMO1 and XG13.

The methods for in vitro prognosis of the likelihood of an individual to be sensitive to a therapeutic treatment comprising the measure of the presence and the level of H3K4me3, as disclosed above, will be performed with methods known from the man skilled in the art, and illustrated in the examples below.

Such methods encompass in particular chromatin immunoprecipitation (ChIP) followed by PCR.

ChIP-sequencing will also be used, according to the invention.

The invention is also relating to kits for performing the methods as described above.

The kit is, in one embodiment, comprising means for measuring the expression level of at least 5 genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, CUL4B, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B.

According to another embodiment, the kit is comprising antibodies directed to H3K4me3 and means for measuring the level of genes associated selected from the genes disclosed in tables 1 to 4.

The kits will in particular comprise at least 2 primers or 2 sets of primers and/or at least 2 probes or 2 set of probes.

The invention also encompasses methods of treatment of an individual having MM, wherein after performing the methods for in vitro prognosis of the likelihood of an individual having MM to be sensitive to a therapeutic treatment, if the individual is classified as sensitive to a therapeutic treatment, said therapeutic treatment is then administered to the individual.

The invention is also relating to methods for in vitro prognosis of the outcome of an individual having MM, implementing the above set of genes.

In particular, the invention is relating to a method for the in vitro prognosis of the outcome of an individual having multiple myeloma comprising the following steps :
a) Measuring, in a biological sample of the individual, the level of expression of at least five genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ,
b) Calculating a risk score from the expression level obtained in step a)
c) Comparing the risk score of the individual to a reference value, and if the risk score is higher than the reference value, classifying the individual as having a bad prognosis.

In another embodiment, the invention is relating to a method for the in vitro prognosis of the outcome of an individual having multiple myeloma comprising the following steps
a) Measuring, in a biological sample of the individual, the level of expression of at least five genes selected from ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B,
b) Calculating a risk score from the expression level obtained in step a)
c) Comparing the risk score of the individual to a reference value, and if the risk score is higher than the reference value, classifying the individual as having a bad prognosis.

### Example

### Methods

### Samples

XG human myeloma cell lines were obtained as previously described.16 AMO-1, OPM2 and SKMM2 were purchased from DSMZ (Braunschweig, Germany) and RPMI8226 from ATCC (Manassas, USA). JJN3 was kindly provided by Dr. Van Riet (Brussels, Belgium), and KMS-12-BM by Dr Otsuki (Okayama, Japan). HMCLs characteristics are available in Table 5.

Bone marrow samples were collected after patients' written informed consent in accordance with the Declaration of Helsinki and institutional research board approval from Montpellier University Hospital. Bone marrow were collected from 69 patients at diagnosis and 28 patients at relapse, this cohort was called Montpellier cohort. MM cells of patients were purified using anti-CD138 MACS microbeads (Miltenyi Biotec, Bergisch Gladbach, Germany). The inventorsalso used RNA sequencing data of 674 newly diagnosed MM patients with longitudinal follow-up from the Multiple Myeloma Research Foundation CoMMpass trial (NCT01454297; version IA11a), termed in the following CoMMpass cohort.

Normal plasma cells were generated using a 3-step in vitro model starting from purified memory B cells from 3 different healthy donors as reported (Jourdan M, et al. The Journal of Immunology. 2011).

### HMCLs response to drug treatment

HMCLs were cultured in RPMI-1640 medium (Gibco, Thermo Fisher Scientific, Waltham, USA) supplemented with 10% fetal bovine serum (Eurobio, Les Ulis, France) and IL-6 (Peprotech, Rocky Hill, USA) for XG cell lines. The inventorsevaluated the sensitivity of HMCLs to lenalidomide and romidepsin (Selleckchem, Houston, USA). The IC50 was determined at day 4 using the CellTiter-Glo assay (Promega, Madison, USA), as previously described. The data represent the mean ± standard deviation of three independent experiments that were carried out on sextuplet culture wells (Figure 6A and 7A).

### RNA sequencing

The RNA sequencing (RNA-seq) library preparation was done with 150 ng of input RNA using the Illumina TruSeq Stranded mRNA Library Prep Kit. Paired-end RNA-seq were performed with Illumina NextSeq sequencing instrument (Helixio, Clermont-Ferrand, France). RNA-seq read pairs were mapped to the reference human GRCh37 genome using the STAR aligner (Dobin A, et al. Bioinformatics. 2013). All statistical analyses were performed with the statistics software R (version 3.6.2; https://www.r-project.org), and R packages developed by Bioconductor project (https://www.bioconductor.org (Gentleman RC, et al. Genome Biol. 2004). The expression level of each gene was summarized and normalized using the DESseq R/Bioconductor package (Love MI, et al, Genome Biology. 2014).

### Whole exome sequencing (WES) and variant calling

WES was performed on XG21 cell line as described above.14 WES public data was used for KMS-12-BM. Reads were mapped to the reference human hg19 using the Bowtie 2 aligner version 2.3.2. SAMtools version 1.5 was used to convert SAM files to sorted BAM files. Indel realignment, base quality recalibration and variant calling steps were completed with GATK 3.8-1. ANNOVAR was used to annotate variants. Then, filters described in Vikova et al. (Theranostics. 2019) were applicated.

### HMCLs ChIP-seq

Cells were cross-linked in formaldehyde at a final concentration of 1% for 8 minutes. All experiments reagents were included in the iDeal ChIP-seq kit for histones (Diagenode, Liege, Belgium). Sonication was performed using a Bioruptor Plus sonication devise (Diagenode, Liege, Belgium) under the optimal conditions to shear cross-linked DNA to fragments of 100-300 base pairs in length. ChIP was conducted with the IPStar Compact Automated System (Diagenode) and the iDeal ChIP-seq kit for histones (Diagenode, C01010059). ChIP were performed starting from 1,000,000 cells. Crossed-linked DNA was incubated 13h with H3K4me1 (Diagenode, C15410194), H3K4me3 (Diagenode, C15410003), H3K9me3 (Diagenode, C15410193), H3K27ac (Diagenode, C15410196), H3K27me3 (Diagenode, C15410195) or H3K36me3 (Diagenode, C15410192) antibody and 3h with the beads. After 5min washes, eluates were recovered and reverse cross-linked for 4h at 65°C. Samples were treated for 1h with RNAse at 37°C, prior to DNA purification with the Auto IPure kit v2 (Diagenode, C03010010). Libraries were performed using NEBNext Ultra II DNA Library Prep Kit for Illumina (New England Biolabs). ChIP-seq were performed with Illumina NextSeq500 technology (Helixio, Clermont-Ferrand, France) using the following parameters: single-ended, 50bp, 40 million reads.

Reads were mapped to the human reference genome (hg19) using Bowtie2 (version 2.3.2). Peak-calling was performed using MACS2 (version 2.1.2) (Zhang Y, et al. Genome Biology. 2008) . Peak annotation and differential binding analysis of ChIP-seq peak data were performed using ChIPseeker29 and DiffBind30 R/bioconductor packages, respectively. Heatmaps and average profiles were generated by deeptools (version 3.5.0) Ramírez F, et al. Nucleic Acids Res. 2016). Super-enhancers (SE) were identified using the ROSE (Rank Ordering of Super-Enhancers) algorithm Lovén J, et al. Cell. 2013 based on H3K27ac and H3K4me1 ChIP-seq signal. TSS exclusion zone size was adjusted to 2500 bp to exclude promoter regions. Transcriptionally active genes were assigned to super-enhancers using a simple proximity rule (50 kb window). Scores were built using the inventors' previously published methodology (Moreaux J, et al. Mol Cancer Ther. 2012). Genes composing super-enhancer and repressive domain scores were selected for their prognostic significance using Maxstat R function and the Benjamini Hochberg multiple testing correction. Score values correspond to the sum of the Cox beta-coefficients of each gene, weighted by ± 1 if patient MM cells signal for the gene of interest is above or below the Maxstat reference value of this gene. The statistical significance of differences in overall survival between patients' groups was calculated using the log-rank test and survival curves were plotted using the Kaplan-Meier method. CoMMpass and Montpellier cohorts were used as training and validation cohorts, respectively. Statistical differentially bound regions between drug-sensitive and - resistant HMCLs were identified with DiffBind R/bioconductor package (FDR ≤ 0.05).

### Results

### Epigenetic landscape of HMCLs

ChIP-seq analysis was performed using 16 HMCLs characterized by a molecular and genetic variability, which is representative of the heterogeneity of the disease as shown in table 5).

**Table 5 : Characteristics of HMCLs based on molecular classification**

| HMCLs name | HMCL classificati on | Target genes | Translocati on | del1 p | 1qgai n | del13 q | del17 p |
|---|---|---|---|---|---|---|---|
| AMO-1 | CD-2L | *CCND2* | t(12;14) | + | + | - | - |
| JJN3 | MF | *c-Maf* | t(14;16) | - | + | + | + |
| KMS-12-BM | CD-2L | *CCND1* | t(11;14) | + | - | + | + |
| OPM2 | MS | *MMSET*/*FGF R3* | t(4;14) | + | + | - | + |
| RPMI82 26 | MF | *c-Maf* | t(14;16) | + | + | + | + |
| SKMM2 | CD-1 | *CCND1* | t(11;14) | + | - | + | + |
| XG1 | CTA/FRZB | *CCND1* | t(11;14) | - | - | - | - |
| XG2 | CTA/FRZB | *CCND2* | t(12;14) | - | - | - | - |
| XG5 | CD-1 | *CCND1* | t(11;14) | + | - | - | + |
| XG6 | CTA/M F | *c-Maf* | t(16;22) | + | - | + | - |
| XG7 | MS | *MMSET* | t(4;14) | + | + | + | - |
| XG12 | CTA/M F | *c-Maf* | t(14;16) | + | - | - | - |
| XG13 | MF | *c-Maf* | t(14;16) | + | - | + | + |
| XG19 | CTA/M F | *c-Maf* | t(14;16) | + | - | + | + |
| XG20 | MS | *MMSET* | t(4;14) | + | - | - | |
| XG21 | CD-1 | *CCND1* | t(11;14) | | + | - - | - |

To evaluate the epigenetic landscape of these HMCLs, two repressive marks (H3K9me3 and H3K27me3) and four active marks (H3K4me1, H3K4me3, H3K27ac and H3K36me3) were retained. The annotation of the peaks reveals that the enhancer-related H3K4me1 modification is mainly found in distal intergenic (45.4% ± 2.6%) and intron (35.4% ± 2.4%) regions whereas H3K27ac, a modification known to be associated with both active enhancers and promoters, is mainly found in distal intergenic (34.8% ± 4.0%) and promoter (32.9% ± 4.6%) regions (Figure 1A). H3K4me3 is mainly located at promoters, as expected for a mark of actively transcribed gene promoters, but also in intergenic regions (promoters: 42.1% ± 6.8%; intergenic: 36.2% ± 5.0%). The H3K4me3 and H3K27ac marks exhibit the same profile surrounding the transcription start site (TSS) with a major peak located downstream the TSS (Figure 1B-D). H3K4me1 marks are both located upstream and downstream the TSS (Figure 1B-D). On enhancer regions, the majority of H3K27ac peaks overlapped with H3K4me1 peaks, revealing active enhancers, whereas non-overlapped H3K4me1 peaks were associated with poised enhancers (Figure 1D).34 The H3K36me3 modifications are identified downstream the TSS, on the body of active genes with a distribution of intron and exon regions representing 69.8% of total regions. The repressive marks, H3K9me3 and H3K27me3, respectively detecting HP1- and Polycomb-repressed chromatin, are essentially found in intergenic regions (61.6% ± 6.2% and 54.1% ± 4.3%, respectively), with distinct localization, but also on gene promoters (Figure 1A-D). Moreover, a small fraction of these promoters was enriched by H3K9me3 and H3K27me3 co-localization.

Figure 2A shows the global profile of histone modifications on the 16 HMCLs. The clustering based on peak localizations distinguishes each mark from all others and repressive (H3K9me3 and H3K27me3) from active (H3K4me1, H3K4me3, H3K27ac and H3K36me3) marks. TSSs of actively transcribed genes are marked by H3K4me3 and H3K27ac modifications, which explains the high similarity between H3K4me3 and H3K27 clustering of HMCLs reveals subgroups associated to different cytogenetic abnormalities or HMCLs transcriptional classification. The H3K4me3 analysis splits HMCLs into two subgroups which differ according to the presence or absence of 1q gain (1q12∼q23 region) (Figure 2B). The first one, composed of XG6, XG13, XG19, XG12, XG1, KMS-12-BM and XG2, is associated to the absence of the amplification of 1q (1q12∼q23 region) while the second group (XG5, XG7, XG21, AMO-1, JJN3, XG20, OPM2, RPMI8226 and SKMM2) is largely composed of HMCLs with 1q amplification (7/9). Interestingly, the inventorsfound two chromatin regulatory genes in this region: ANP32E (1q21.2) and SETDB1 (1q21.3). ANP32E has been described as an inhibitor of histone acetyltransferases and is associated with MM progression and poor prognosis in MM patients. SETDB1 is a H3K9 methyltransferase which plays a role in the repression of genes encoding developmental regulators. Promoters of SETDB1-bound genes are co-occupied by H3K9me3 and H3K4me3 while promoter of developmental genes are also enriched in H3K27me3. Differential analysis on H3K9me3 reveals a highly similar cluster, composed of KSM-12-BM, OPM2, RPMI8226, XG19 and SKMM2, characterized by the association of del1p, del13q and del17p cytogenetic abnormalities (Figure 2C). The H3K27me3 analysis distinguishes the group of HMCLs harboring the t(4;14) translocation (XG7, XG20 and OPM2) from other HMCLs (Figure 2D). t(4;14) translocation results in IgH/MMSET hybrid transcripts inducing an ac profiles (Figure 1C and 1D). When each mark is observed independently, the overexpression of MMSET gene. There is a correlation between the number of H3K27me3 peaks and MMSET mRNA expression in the groups of HMCLs without t(4;14) translocation (R2 = 0.33; P-value < 0.02) (Figure 8A) whereas no correlation is observed in t(4;14) cell lines (data not shown). However, the percentage of reads in H3K27me3 peaks is correlated to the MMSET mRNA expression in all HMCLs (R2 = 0.48; P-value < 0.01) (Figure 8B). These results suggest that H3K27me3 localization are very similar in t(4;14) HMCLs and the global enrichment of H3K27me3 increases with the deregulation of MMSET without increasing the total number of H3K27me3 sites on the genome. Differentially bound site analysis revealed around 46000 sites significantly differentially associated to the H3K27me3 modification in t(4;14) HMCLs (MMSET subgroups) compared to HMCLs without t(4;14) translocation (FDR ≤ 0.05) (supplementary Figure 2A). Of these, 1822 sites are located on gene promoters, with 809 gene promoters presenting lower H3K27me3 levels versus 1013 gene promoters were enriched in H3K27me3 in the MMSET subgroup (FDR ≤ 0.05) (Figure 9B and 9C). Among this set of genes, the inventors found genes involved in the induction of apoptosis (TP73 and BNIP3) and the regulation of cell cycle (CEND1, CCNO, CDKN1C and CDK2AP1). The global analysis of H3K4me1, H3K27ac and H3K36me3 modifications do not provide a clear link between cytogenetic abnormalities and histone modifications while H3K27ac and H3K36me3 clustering tends to group HMCLs by HMCL molecular subgroups (Figures 2E-G). The inventors also studied the link between histone modification profiles and frequently mutated genes in HMCLs(Figure 10) . Interestingly, TP53 mutation status appeared linked to a specific H3K9me3 profile.

### SE-risk score predicting survival in MM patients

H3K4me1 and H3K27ac modifications are known to be associated with enhancer regions and, more specifically, active enhancers can be identified by co-occupancy of H3K4me1 and H3K27ac. Super-enhancers consist to a set of large enhancer domains displaying physical proximity (+/- 12.5kb) and are associated with genes that control and define cell identity. The inventors identified 607 to 2510 predicted super-enhancers per HMCL (Table 6).

**Table 6: Super-enhancers and SE-associated genes detected in HMCLs**

| HMCLs name | Activated SE | SE-associated genes (<50kb) | SE-associated genes with strong expression |
|---|---|---|---|
| AMO1 | 994 | 2493 | 96 |
| JJN3 | 1485 | 3202 | 95 |
| KMS-12-BM | 1770 | 5300 | 97 |
| OPM2 | 2211 | 5300 | 163 |
| RPMI8226 | 1187 | 3191 | 106 |
| SKMM2 | 1293 | 3926 | 88 |
| XG1 | 1959 | 4584 | 121 |
| XG12 | 2510 | 5425 | 145 |
| XG13 | 2030 | 4815 | 108 |
| XG19 | 2293 | 5764 | 145 |
| XG2 | 1368 | 3164 | 86 |
| XG20 | 1782 | 3727 | 119 |
| XG21 | 607 | 1540 | 67 |
| XG5 | 631 | 1132 | 34 |
| XG6 | 1643 | 3656 | 112 |
| XG7 | 2352 | 5299 | 153 |

These super-enhancers differed from typical enhancers in both size and H3K4me1 and H3K27ac levels (Figure 3A). Enhancers tend to loop to and associate with adjacent genes in order to activate their transcription. Using a simple proximity rule, all TSSs were assigned to super-enhancers within a 50 kb window. Among the SE-associated genes identified, the inventors found genes such as (MYC, IRF4, PRDM1, XBP1 and CCND2) but also other key MM genes, such as ACTG1, MAF, CCND1, TRAF3 and NSD2 (MMSET). Among them, the inventors found SE-associated genes targeted by MM translocations. Cell lines harboring t(14;16) and t(16;22) translocations involve MAF overexpression, t(4;14) cell lines lead to MMSET deregulation, while cell lines with t(11;14) and t(12;14) are associated with CCND1 and CCND2 overexpression, respectively (Table 5 and Figure 3B).

These SE-associated genes were used to build a score predicting survival in MM patients. First, the inventors selected SE-associated genes with strong expression in HMCLs, overexpressed in plasma cells of MM patients compared to normal plasma cells and associated with shorter overall survival in MM patients using the Maxstat algorithm (Hothorn T et al Computational Statistics & Data Analysis. 2003) (Figure 4A). Twenty-eight genes fulfilled these criteria: BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ (Figure 11 and 12). Then, the prognostic information of these 28 SE-associated genes was combined to build a SE-risk score. This score is defined by the sum of the beta coefficients of the Cox model for each prognostic gene, weighted by ± 1 if the patient plasma cells signal for a given gene is above or below the Maxstat reference value of this gene as previously described Kassambara A, et al. Haematologica. 2012;. Using two independent cohorts, the SE-risk score had a prognostic value when it was used to split patients into two groups using the Maxtstat R function. The score splits patients into a high-risk group (score > -4.0) and a low-risk group (score < -4.0) in the CoMMpass (P-value < 0.0001; high-risk group: 58.0% of patients; low-risk group: 42.0% of patients) and Montpellier (P-value < 0.005; high-risk group: 50.7% of patients; low-risk group: 49.3% of patients) cohorts (Figure 4B). The evolution of the score was evaluated from diagnosis to relapse in 14 MM patients with paired samples. No significant difference between the two groups was identified (Figure 14A) . However, 2 MM patients presented a striking increase in SE-risk score from diagnosis to relapse.

Interestingly, 21 genes (HK2, HNRNPC, HSPA9, ILF3, LDHB, MDH1, MYBPC2, NCL, NUDC, PARP1, PDIA6, PRPS1, RPL13A, RPL27A, SF3B2, SLC25A39, SMARCA4, STC2, TNPO2, TPR and YWHAQ) were significantly overexpressed in HMCLs compared to plasma cells of MM patients (Figure 11), suggesting that genes may play a role in disease progression and independence of MM cells from micro-environment. Moreover, DEMETER2 (McFarland JM, et al. Nat Commun. 2018) and CERES Meyers RM, et al. Nat Genet. 2017) dependency scores calculated from public datasets of RNAi (Tsherniak A, et al. Cell. 2017) and CRISPR/Cas9-based screening (Dependency Map data, Broad Institute, www.depmap.org) indicated that YWHAQ, IL10, HK2 and THY1 are significant essential MM genes (Table 7 and Figure 15).

**Table 7: DEMETER2 and CERES score comparison between MM cell lines and other cancer cell lines. DEMETER2 and CERES scores were calculated from CRISPR-Cas9 and RNAi-based viability assays, respectively**

| Gene | CRISPR | | RNAi | |
|---|---|---|---|---|
| | T-Statistic | P-Value | T-Statistic | P-Value |
| YWHAQ | - | - | -5.2950724 | 1.71E-07 |
| THY1 | - | - | -5.5790531 | 3.78E-08 |
| IL10 | -4.4392273 | 1.04E-05 | - | - |
| HK2 | -4.0703292 | 5.19E-05 | - | - |

Indeed, THY1 knockdown presented the larger DEMETER2 dependency score difference between HMCLs and all other cells. The median score of non-myeloma cell lines was around 0, indicating that gene is not essential in these cells and could be an interesting specific therapeutic target in MM. Altogether, these data show that a signature of genes associated with super-enhancers in MM could be defined allowing the identification of high-risk newly diagnosed MM patients.

### H3K9me3 and H3K27me3 co-localization in promoter can be associated with prognostic value in MM

Gene promoters enriched by both H3K9me3 and H3K27me3 modifications were investigated, to build a score based on genes associated with epigenetic transcriptional repression and identify new potential tumor suppressor genes.

First, H3K9me3 and H3K27me3 overlapping regions on gene promoters were identified for all HMCLs. To identify potential tumor suppressor genes, genes significantly under-expressed in plasma cells of MM patients compared to normal plasma cells were selected, and associated with poor outcome when their expression is low in MM cells of patients. Eighteen genes were identified : ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B (Figure 16 and 17) . The promoters of SEMA6A and ARHGEF5 genes were most frequently enriched by both H3K9me3 and H3K27me3 modifications in HMCLs (Figure 5). The prognostic information from these eighteen genes was combined into a tumor suppressor-based risk score as described above. This score splits patients into two groups in the training cohort (CoMMpass cohort, n=674, P-value < 0.0001): a low-risk group (score < -3.45) of 57.9% MM patients associated with global high expression of these genes and a high-risk group (score > - 3.45) of 42.1% MM patients associated with global low gene expression (Figure 5B). This score also split MM patients into low and high-risk groups (62.3% and 37.7% of patients, respectively) in the validation cohort (Montpellier cohort, n=69, P-value < 0.01). Of the eighteen genes, thirteen genes (ARHGEF5, DEF8, GRID2IP, HDAC9, HSPA1L, KDM4C, P4HA3, PAG1, SEMA6A, SFMBT2, THEMIS2, ZFP2 and ZNF518B) were down-regulated in HMCLs compared to primary MM cells (Figure 16).

### H3K4me3 bound sites linked to lenalidomide and romidepsin resistance in HMCLs

H3K4me3 modification, associated with promoters of actively transcribed genes, was studied in respectively drug resistant and -sensitive HMCLs, to identify epigenetic changes involved in drug resistance. The relationship between the H3K4me3 landscape of HMCLs and their response to conventional drugs including bortezomib, melphalan, dexamethasone, lenalidomide and HDACi was analyzed.

5903 significantly differentially bound H3K4me3 sites between lenalidomide-resistant (AMO1, JJN3, KMS-12-BM, SKMM2, XG1, XG5, XG7, XG12, XG20 and XG21 HMCLs) and lenalidomide-sensitive cell lines (OPM2, RPMI8226, XG2, XG6, XG13 and XG19 HMCLs) (Figure 6A) (FDR ≤ 0.05) were identified. Regarding gene promoters, 203 H3K4me3 differentially bound sites were significantly enriched in lenalidomide-sensitive HMCLs compared to lenalidomide-resistant HMCLs (FDR ≤ 0.05) whereas 136 H3K4me3 differentially bound sites were significantly enriched in the lenalidomide-resistant group compared to the lenalidomide-sensitive group (Figure 6B). Examples of significantly differentially bound regions are presented in Figure 6C. Gene set enrichment analysis revealed that H3K4me3 differentially bound sites enriched in lenalidomide-resistant HMCLs were associated to interferon signaling and cytokine signaling in immune system (Figure 6D). Differentially bound sites enriched in lenalidomide-sensitive HMCLs were significantly associated with an NFKBIA target genes signature.

2098 H3K4me3 significantly differentially bound sites were identified between romidepsin-resistant (AMO1, XG7, XG13, XG19 and XG20) and romidepsin-sensitive (OPM2, RPMI8226, SKMM2, XG1, XG5, XG6 and XG12) cell lines (Figure 7A) (FDR ≤ 0.05) including 250 bound sites on gene promoters (Figure 7B). Romidepsin is a class I histone deacetylase (HDAC) inhibitor currently evaluated in combination with lenalidomide in phase I/II (NCT017755975) in patients with relapse or refractory multiple myeloma. Among the 250 H3K4me3 bound sites located on gene promoters, 180 were significantly enriched in romidepsin-sensitive HMCLs associated with a HDAC3 and PRDM6 targets gene signature (Figure 7D). The inventors found 70 differentially bound sites enriched in romidepsin-resistant group compared to romidepsin-sensitive group. Gene set enrichment analysis revealed a significant association with H3K27me3 and polycomb complex subunit (SUZ12 and EED) target genes.

### Discussion

In this study, the inventors have characterized the landscape of histone modifications in a large collection of HMCLs representative of MM molecular heterogeneity. Some histone modification profiles, such as H3K27me3, were clearly related to cytogenetic abnormalities. In t(4;14) HMCLs, MMSET overexpression results in the genome-wide redistribution of the SET domain protein EZH2 and the associated H3K27me3 mark. Gene expression profiling revealed deregulated genes involved in cell cycle (e.g. CCNE2), apoptosis (e.g. BAX and BCL2) and DNA repair (e.g. ATM and GADD45A). Moreover, the inventors showed that promoter of genes involved in the negative regulation of cell cycle (e.g. CEND1 and CDKN1C) and the induction of apoptosis (e.g. TP73 and BNIP3) were enriched in H3K27me3 mark in t(4;14) HMCLs. Methylation of the BNIP3 promoter was also described and associated with poor overall survival in MM patients. These results confirmed the interplay between MMSET, EZH2 and H3K27me353,55 and revealed new potential therapeutic targets for t(4;14) MM patients associated with poor prognosis. The inventors also observed that TP53 mutation is associated with a H3K9me3 signature. SUV39H1, the histone methyltranferase responsible for the H3K9me3 mark, is a target of p53 repression and p53 target promoters are enriched in the H3K9me3 repressive mark, suggesting that TP53 mutation could modulate H3K9me3 level on these promoters and thus the p53 apoptotic response.

The inventors and others showed that super-enhancers were associated to key genes contributing to myeloma pathogenesis, such as MYC, IRF4, CCND1, NSD2 and MAF. Acetylated chromatin, particularly super-enhancer regions, is associated to bromodomain and extra-terminal (BET) proteins to facilitate transcriptional activation.59 BET inhibitors disrupt the interaction of bromodomains with acetylated histones and leads to loss of enhancer-promoter long-range interactions. The score based on genes associated with super-enhancers built in this study allows to identify high-risk MM patients that might benefit from BET inhibitors. Several BET inhibitors are currently evaluated in phase I/II in MM patients, including OTX015 (NCT01713582), CPI-0610 (NCT02157636), GSK525762 (NCT01943851), and RO6870810 (NCT03068351).60 Moreover, concerning the 28 genes composing the SE-risk score, the prognostic value of 7 genes (BSG, HK2, HSPA9, IL10, PARP1, PDIA6 and SLC7A5) has already been described in MM61-71 while the expression of HNRNPC, ILF3, LDHB, MDH1, NCL, PRPS1, RPL8, RPL13A, RPL35, SMARCA4, SPN, STC2 and THY1 was described to be associated to tumorigenesis, drug resistance and/or poor prognosis in other cancers. Furthermore, the prognostic value of MYBPC2, NUDC, RPL27A, SF3BS, SLC25A39, TNPO2, TPR, YWHAQ transcriptional deregulation has not been described before. Interestingly, among these genes associated with SE in MM, YWHAQ, IL10, HK2 and THY1 were identified as significant essential MM genes in RNAi or CRISPR screening corroborating epigenetic and transcriptional modifications at a functional level.

It is widely believed that H3K9me3 and H3K27me3 do not co-occur at the same loci, but some ChIP-seq data indicate that these two marks can be found together at a subset of developmentally regulated gene in mouse embryonic stem cells, extra-embryonic lineages and human differentiated cells. This dual repression at specialized regulators may point to the importance of maintaining their silencing to confer a selective advantage in tumoral cells. Here, the inventors identified 18 potential suppressor tumor genes enriched in H3K9me3 and H3K27me3 repressive marks on their promoter. To our knowledge, the tumor suppressor function of these genes has not yet been described in MM. However, some of them are involved in cell proliferation inhibition. NLRP2 gene, coding a member of the nucleotide binding and leucine-rich repeat receptor (NLR), is an inhibitor of NF-κB pathway which play a key role in survival and proliferation of myeloma cells. PAG1 encodes for a type III transmembrane adaptor protein and is an inhibitor of Src tyrosine kinases known to promote proliferation in MM. From these 18 genes the inventors built a score identifying high-risk group of MM patients with global low expression of putative suppressor genes associated with H3K9me3 and H3K27me3 marks. These patients could benefit from combined therapeutic targeting of EZH2 and SUV39H1 histone methyltransferases. Moreover, the therapeutic interest of EZH2 inhibitors have already been demonstrated in HMCLs and primary myeloma samples. Concerning SUV39H1, high expression level is associated with a poor prognosis in MM patients and SUV39H1 inhibitor also exhibited anti-MM effects both in HMCLs and primary samples.

Despite the improvement of MM patient survival through the development of novel agents, including new generation of IMIDs or proteasome inhibitors, monoclonal antibodies and HDAC inhibitors, the acquisition of drug resistance is the major limitation of MM therapy. The great majority of MM patients ultimately relapse and the treatment of relapse/refractory MM remains a major challenge. In this study, the inventeorsanalyzed the relationship between differential enrichment of H3K4me3 on gene promoters in HMCLs and response to treatment. The inventors developed an epigenetic biomarker based on this histone modification, which predicts lenalidomide and romidepsin responses in HMCLs. IMIDs are known to target the cereblon complex. Cereblon is composed of CUL4, RBX1, DDB1 and CRBN proteins and induces the ubiquitination of B-cell transcription factors IKZF1 and IKZF3 in presence of IMiDs. Among the genes encoding these proteins, the inventors observed a significant difference in H3K4me3 enrichment on the promoter of CUL4B in lenalidomide-sensitive HMCLs compared to resistant HMCLs (supplementary Figure 10). Interestingly, the presence of the CUL4B splicing variant 1 seemed to be associated with lenalidomide sensitivity whereas the other variant could play a role in lenalidomide resistance. In other studies, loss of CUL4B has been described as conferring resistance to lenalidomide in lymphoma and myeloma cell lines.

This study provides a comprehensive characterization of the MM epigenetic landscape, representing a unique resource for future biological studies which could help to identifying novel critical epigenetic modifications involved in MM progression and drug resistance. Furthermore, risk-scores based on super enhancers and repressive regions together with epigenetic biomarkers of drug response could represent new tools for precision oncology in MM.

### References listing

Moreaux J, Rème T, Leonard W, et al. Development of Gene Expression-Based Score to Predict Sensitivity of Multiple Myeloma Cells to DNA Methylation Inhibitors. Mol Cancer Ther. 2012;11(12):2685-2692.
Vikova V, Jourdan M, Robert N, et al. Comprehensive characterization of the mutational landscape in multiple myeloma cell lines reveals potential drivers and pathways associated with tumor progression and drug resistance. Theranostics. 2019;9(2):540-553.
Moreaux J, Klein B, Bataille R, et al. A high-risk signature for patients with multiple myeloma established from the molecular classification of human myeloma cell lines. Haematologica. 2011;96(4):574-582.
Jourdan M, Caraux A, Caron G, et al. Characterization of a Transitional Preplasmablast Population in the Process of Human B Cell to Plasma Cell Differentiation. The Journal of Immunology. 2011;187(8):3931-3941.
Dobin A, Davis CA, Schlesinger F, et al. STAR: ultrafast universal RNA-seq aligner. Bioinformatics. 2013;29(1):15-21.
Gentleman RC, Carey VJ, Bates DM, et al. Bioconductor: open software development for computational biology and bioinformatics. Genome Biol. 2004;5(10):R80.
Love MI, Huber W, Anders S. Moderated estimation of fold change and dispersion for RNA-seq data with DESeq2. Genome Biology. 2014;15(12):550.
Zhang Y, Liu T, Meyer CA, et al. Model-based Analysis of ChIP-Seq (MACS). Genome Biology. 2008;9(9):R137.
Yu G, Wang L-G, He Q-Y. ChIPseeker: an R/Bioconductor package for ChIP peak annotation, comparison and visualization. Bioinformatics. 2015;31(14):2382-2383.
Ross-Innes CS, Stark R, Teschendorff AE, et al. Differential oestrogen receptor binding is associated with clinical outcome in breast cancer. Nature. 2012;481 (7381):389-393.
Ramírez F, Ryan DP, Grüning B, et al. deepTools2: a next generation web server for deep-sequencing data analysis. Nucleic Acids Res. 2016;44(W1):W160-W165.
Lovén J, Hoke HA, Lin CY, et al. Selective Inhibition of Tumor Oncogenes by Disruption of Super-Enhancers. Cell. 2013;153(2):320-334.
Hothorn T, Lausen B. On the exact distribution of maximally selected rank statistics. Computational Statistics & Data Analysis. 2003;43(2):121-137.
Kassambara A, Hose D, Moreaux J, et al. Genes with a spike expression are clustered in chromosome (sub)bands and spike (sub)bands have a powerful prognostic value in patients with multiple myeloma. Haematologica. 2012;97(4):622-630.
McFarland JM, Ho ZV, Kugener G, et al. Improved estimation of cancer dependencies from large-scale RNAi screens using model-based normalization and data integration. Nat Commun. 2018;9(1):1-13.
Meyers RM, Bryan JG, McFarland JM, et al. Computational correction of copy number effect improves specificity of CRISPR-Cas9 essentiality screens in cancer cells. Nat Genet. 2017;49(12):1779-1784.
Tsherniak A, Vazquez F, Montgomery PG, et al. Defining a Cancer Dependency Map. Cell. 2017;170(3):564-576.e16.
McDonald ER, Weck A de, Schlabach MR, et al. Project DRIVE: A Compendium of Cancer Dependencies and Synthetic Lethal Relationships Uncovered by Large-Scale, Deep RNAi Screening. Cell. 2017;170(3):577-592.e10.

**Table 1: H3K4me3 differentially bound sites significantly enriched in the lenalidomide-sensitive cell lines compared to the lenalidomide-resistant cell lines**

| Seqnames | Start | End | Cone resistant | Cone sensitive | Fold | p.value | FDR | Annotation | Distance to TSS | ENSEMBL | SYMBOL |
|---|---|---|---|---|---|---|---|---|---|---|---|
| chrl 8 | 42279267 | 42286041 | -1,28 | 7,85 | -9,12 | 4,34E-16 | 7,25E-12 | 5'UTR | 18404 | ENSG00000152217 | SETBP1 |
| chrl 8 | 14743460 | 14753575 | -1,28 | 7,83 | -9,11 | 1,59E-15 | 1,78E-11 | Promoter (<=1kb) | 0 | ENSG00000180777 | ANKRD30B |
| chr2 | 107455504 | 107460949 | -0,65 | 8,25 | -8,91 | 7,91E-15 | 7,55E-11 | 5'UTR | 41661 | ENSG00000144057 | ST6GAL2 |
| chr8 | 120952985 | 120956662 | -1,28 | 7,04 | -8,31 | 1,67E-14 | 1,39E-10 | Intron (uc003vow.4/64798, intron 3 of 8) | 67085 | ENSG00000155792 | DEPTOR |
| chr1 | 246376594 | 246384042 | -1,28 | 7,52 | -8,8 | 5,97E-14 | 3,62E-10 | Intron (uc00libk.3/64754, intron 5 of 11) | 196672 | ENSG00000185420 | SMYD3 |
| chr1 | 160489276 | 160493371 | -1,28 | 6,56 | -7,84 | 3,35E-13 | 1,72E-09 | Promoter (<=1kb) | 0 | ENSG00000162739 | SLAMF6 |
| chr7 | 152548575 | 152550574 | -1,28 | 6,1 | -7,37 | 1,20E-12 | 5,35E-09 | Exon (uc003wle.2/57180, exon 10 of 12) | 91741 | ENSG00000133627 | ACTR3B |
| chr2 | 51252500 | 51256267 | -1,28 | 6,87 | -8,15 | 2,65E-12 | 1,06E-08 | Promoter (<= 1 kb) | 65 | ENSG00000179915 | NRXN1 |
| chr12 | 131685455 | 131689593 | -1,28 | 6,89 | -8,17 | 2,98E-12 | 1,06E-08 | Exon (uc001uiw.3/116437, exon 2 of 5) | 35899 | ENSG00000204603 | LINC01257 |
| chr9 | 15865973 | 15873727 | -1,28 | 7,42 | -8,69 | 3,02E-12 | 1,06E-08 | Intron (uc003zmd.3/203238, intron 23 of 25) | 121702 | ENSG00000164989 | CCDC171 |
| chrX | 86853840 | 86856410 | -1,28 | 5,74 | -7,01 | 9,36E-12 | 2,61E-08 | Intron (uc004efa.2/56062, intron 1 of 10) | 81125 | ENSG00000102271 | KLHL4 |
| chr10 | 105343593 | 105346337 | -1,28 | 6,45 | -7,72 | 1,39E-11 | 3,57E-08 | Exon (uc001kxh.3/9148, exon 4 of 6) | 28456 | ENSG00000107954 | NEURL1 |
| chr10 | 48427077 | 48429732 | -1,28 | 6,3 | -7,58 | 1,52E-11 | 3,76E-08 | 3'UTR | 9406 | ENSG00000266524 | GDF10 |
| chr1 1 | 95778808 | 95780096 | -1,28 | 4,14 | -5,42 | 6,15E-11 | 1,37E-07 | Intron (uc001pfw.1/84441, intron 2 of 4) | -121437 | ENSG00000087053 | MTMR2 |
| chr3 | 43457315 | 43459905 | -1,13 | 6,38 | -7,52 | 8,45E-11 | 1,71E-07 | Intron (uc003cmv.3/55129, intron 12 of 12) | -63861 | NA | SNRK-AS1 |
| chr8 | 145163410 | 145165511 | -0,96 | 6,44 | -7,41 | 9,03E-11 | 1,78E-07 | Promoter (<=1kb) | 0 | ENSG00000179698 | WDR97 |
| chr10 | 117707518 | 117714838 | 0,23 | 7,78 | -7,55 | 9,54E-11 | 1,81E-07 | 3'UTR | 316981 | ENSG00000151892 | GFRA1 |
| chr3 | 94653251 | 94660376 | -1,28 | 6,82 | -8,09 | 9,77E-11 | 1,81E-07 | Promoter (<=1kb) | 0 | ENSG00000239589 | LINC00879 |
| chr12 | 119616298 | 119620172 | -1,28 | 6,22 | -7,5 | 1,10E-10 | 1,98E-07 | Promoter (<=1kb) | 0 | ENSG00000152137 | HSPB8 |
| chr1 | 3430843 | 3434252 | -1,28 | 6,85 | -8,13 | 1,26E-10 | 2,21E-07 | Exon (uc001akk.3/1953, exon 3 of 30) | 13760 | ENSG00000162591 | MEGF6 |
| chr18 | 14541185 | 14544942 | -1,28 | 5,75 | -7,02 | 2,03E-10 | 3,22E-07 | Promoter(<=1kb) | 0 | ENSG00000183206 | POTEC |
| chr2 | 1497945 | 1503379 | -1,28 | 6,59 | -7,86 | 2,25E-10 | 3,41E-07 | Exon (uc002qwr.3/7173, exon 12 of 17) | 9577 | ENSG00000115705 | TPO |
| chr22 | 38991713 | 38993759 | -1,28 | 5,87 | -7,15 | 2,33E-10 | 3,41E-07 | Exon (uc011anw.1/646851, exon 15 of 17) | -25512 | ENSG00000100206 | DMC1 |
| chr10 | 116089573 | 116094197 | -1,28 | 6,6 | -7,88 | 2,35E-10 | 3,41E-07 | Exon (uc001lbn.3/84632, exon 3 of 19) | -24868 | ENSG00000169129 | AFAP1L2 |
| chr2 | 181555804 | 181558854 | -1,28 | 5,79 | -7,07 | 5,86E-10 | 7,08E-07 | Exon (uc031rqe.1/uc031rqe.1, exon 1 of 7) | -286258 | ENSG00000170035 | UBE2E3 |
| chr2 | 39186946 | 39188379 | -1,28 | 5,3 | -6,58 | 7,64E-10 | 8,24E-07 | Promoter (<=1kb) | 0 | ENSGO0000269210 | LOC375196 |
| chr10 | 15848042 | 15852318 | -1,28 | 6,29 | -7,57 | 7,91E-10 | 8,39E-07 | Intron (uc001iod.1/80013, intron 10 of 14) | 50201 | ENSG00000148481 | MINDY3 |
| chr2 | 198078332 | 198082514 | -1,28 | 6,19 | -7,47 | 8,12E-10 | 8,48E-07 | Intron (uc021vuj.1/91526, intron 1 of 27) | -15570 | ENSG00000065413 | ANKRD44 |
| chr17 | 26626400 | 26627951 | -1,01 | 5,05 | -6,06 | 8,39E-10 | 8,53E-07 | Intron (uc002has.3/284085, intron 2 of 2) | 6457 | ENSG00000265480 | KRT18P55 |
| chr18 | 37271335 | 37272632 | -1,28 | 5,14 | -6,41 | 8,43E-10 | 8,53E-07 | Intron (uc010xcj.1/647946, intron 2 of 3) | -14594 | ENSG00000283645 | MIR5583-2 |
| chr6 | 25164209 | 25167755 | -1,28 | 6,2 | -7,47 | 1,12E-09 | 1,05E-06 | Promoter (<= 1 kb) | 0 | ENSG00000168405 | CMAHP |
| chr5 | 38288963 | 38292074 | 0,12 | 7,24 | -7,12 | 1,19E-09 | 1,11E-06 | Promoter (<=1kb) | 0 | ENSG00000248730 | EGFLAM-AS4 |
| chr15 | 75112768 | 75114346 | -0,74 | 5,23 | -5,97 | 1,29E-09 | 1,16E-06 | Exon (uc002avt.1/79748, exon 8 of 14) | -3471 | ENSG00000213578 | CPLX3 |
| chr15 | 22009448 | 22030012 | -1,28 | 5,81 | -7,09 | 1,32E-09 | 1,16E-06 | Promoter (<=1kb) | 0 | NA | CXADRP2 |
| chrl7 | 73828097 | 73829508 | -1,28 | 5,52 | -6,8 | 1,49E-09 | 1,28E-06 | 3'UTR | 9496 | ENSG00000092929 | UNC13D |
| chr7 | 117066725 | 117068274 | -1,28 | 5,34 | -6,61 | 1,51E-09 | 1,28E-06 | Promoter (<=1kb) | 0 | ENSG00000154438 | ASZ1 |
| chrX | 109661620 | 109662744 | -1,13 | 4,62 | -5,75 | 1,56E-09 | 1,31E-06 | Promoter (<=1kb) | 0 | ENSG00000243978 | RTL9 |
| chr8 | 102999016 | 103001020 | -1,28 | 5,43 | -6,7 | 1,64E-09 | 1,34E-06 | Intron (uc003ykf.3/83988, intron 3 of 7) | 135542 | ENSG00000104490 | NCALD |
| chr4 | 41120283 | 41123720 | -1,28 | 5,43 | -6,7 | 1,70E-09 | 1,36E-06 | Intron(uc003gvl.3/323,intron 2 of 17) | 92915 | ENSG00000163697 | APBB2 |
| chr6 | 123861774 | 123865832 | -1,28 | 5,82 | -7,1 | 1,80E-09 | 1,41E-06 | Intron (uc003pzj.2/10345, intron 4 of 40) | 92406 | ENSG00000186439 | TRDN |
| chr22 | 30475641 | 30476888 | -1,11 | 4,94 | -6,05 | 2,02E-09 | 1,55E-06 | Promoter (<=1kb) | 0 | ENSG00000176635 | HORMAD2 |
| chr7 | 65698721 | 65700356 | -1,28 | 5,2 | -6,48 | 2,65E-09 | 1,95E-06 | Intron (uc010kzv.2/8460, intron 1 of 4) | 28462 | ENSG00000169902 | TPST1 |
| chr1 | 55464423 | 55466832 | -0,99 | 5,64 | -6,63 | 3,05E-09 | 2,18E-06 | Promoter (<=1kb) | 0 | ENSG00000162399 | BSND |
| chr7 | 69089551 | 69091223 | -1,28 | 5,22 | -6,49 | 3,27E-09 | 2,26E-06 | Intron (uc003tvv.4/26053, intron 1 of 4) | 25646 | ENSG00000158321 | AUTS2 |
| chr4 | 183254945 | 183257357 | -1,28 | 5,18 | -6,46 | 3,35E-09 | 2,28E-06 | Intron (uc010irv.1/55714, intron 2 of 3) | 9846 | ENSG00000218336 | TENM3 |
| chr7 | 69674900 | 69677196 | -1,28 | 4,92 | -6,19 | 3,45E-09 | 2,33E-06 | Intron (uc003tvv.4/26053, intron 4 of 4) | -554052 | ENSG00000158321 | AUTS2 |
| chr21 | 41115745 | 41119124 | -1,28 | 5,21 | -6,49 | 3,73E-09 | 2,47E-06 | Promoter (<=1kb) | 0 | ENSG00000183067 | IGSF5 |
| chr22 | 40524620 | 40527250 | -0,91 | 5,42 | -6,33 | 4,13E-09 | 2,71E-06 | Intron (uc003aym.3/23112, intron 3 of 23) | -46679 | ENSG00000100354 | TNRC6B |
| chr1 1 | 61048281 | 61049570 | -1,28 | 5,19 | -6,46 | 4,95E-09 | 3,09E-06 | Exon (uc001nra.3/220001, exon 7 of 20) | 13218 | ENSG00000167992 | VWCE |
| chrl7 | 1902833 | 1903713 | -1,13 | 4,69 | -5,81 | 5,88E-09 | 3,51E-06 | Intron (uc002ftp.3/146760, intron 1 of 1) | 24465 | ENSG00000185924 | RTN4RL1 |
| chr7 | 33391316 | 33395962 | -1,28 | 5,69 | -6,97 | 6,35E-09 | 3,72E-06 | Promoter (1-2kb) | 1301 | ENSG00000122507 | BBS9 |
| chr12 | 102130793 | 102131681 | -1,28 | 3,62 | -4,89 | 6,64E-09 | 3,79E-06 | Promoter (1-2kb) | 1569 | ENSG00000139351 | SYCP3 |
| chr9 | 36913580 | 36915320 | -1,28 | 5,06 | -6,34 | 7,04E-09 | 3,99E-06 | Intron (uc011lpt.1/5079, intron 4 of 6) | -20052 | ENSG00000265368 | MIR4476 |
| chr22 | 26948462 | 26951122 | -0,65 | 5,2 | -5,85 | 7,95E-09 | 4,34E-06 | Promoter (<= 1 kb) | 167 | ENSG00000221760 | MIR548J |
| chr9 | 103289014 | 103290486 | -1,28 | 5,26 | -6,54 | 8,00E-09 | 4,34E-06 | Intron (uc004bay.2/100526694, intron 5 of 9) | -49850 | ENSG00000170681 | CAVIN4 |
| chr15 | 98325082 | 98326772 | -1,28 | 4,28 | -5,55 | 9,23E-09 | 4,89E-06 | Intron (uc002bug.1/91948, intron 1 of 2) | 90887 | ENSG00000251209 | LINC00923 |
| chr15 | 21144410 | 21148786 | -0,98 | 5,94 | -6,93 | 9,42E-09 | 4,91E-06 | Promoter (<= 1 kb) | 0 | ENSG00000258710 | LINC01193 |
| chr7 | 75188400 | 75189554 | -1,28 | 4,92 | -6,2 | 9,49E-09 | 4,91E-06 | Exon (uc003uds.2/3092, exon 14 of 31) | -30947 | ENSG00000127957 | PMS2P3 |
| chr1 | 236914496 | 236916123 | -1,28 | 5,03 | -6,31 | 9,54E-09 | 4,91E-06 | Exon (uc001hyf.2/88, exon 15 of 21) | 19963 | ENSG00000077522 | ACTN2 |
| chr5 | 64235184 | 64236527 | -1,28 | 4,44 | -5,72 | 1,18E-0 8 | 5,88E-06 | Intron (uc003jtn.1/10283, intron 11 of 13) | 170429 | ENSG00000153015 | CWC27 |
| chr1 | 216135362 | 216139148 | -0,99 | 5,9 | -6,89 | 1,18E-0 8 | 5,88E-06 | Exon (uc001hku.1/7399, exon 37 of 72) | 383019 | ENSG00000136636 | KCTD3 |
| chr21 | 46622446 | 46623278 | -1,07 | 4,62 | -5,69 | 1,27E-08 | 6,13E-06 | Intron (uc002zgq.2/104, intron 9 of 12) | 49923 | ENSG00000197381 | ADARB1 |
| chr1 1 | 62224116 | 62227668 | -0,03 | 6,53 | -6,56 | 1,28E-08 | 6,17E-06 | Intron (uc001ntk.2/79026, intron 5 of 5) | 37609 | ENSG00000149021 | SCGB1A1 |
| chrX | 49159679 | 49161127 | -1,28 | 5,24 | -6,52 | 1,42E-08 | 6,72E-06 | Promoter (<=1kb) | 0 | NA | NA |
| chr10 | 119125400 | 119128931 | -1,28 | 5,52 | -6,8 | 1,43E-08 | 6,73E-06 | Intron (uc001lde.1/118987, intron 1 of 4) | 6006 | ENSG00000165650 | PDZD8 |
| chr19 | 50264189 | 50265603 | -1,28 | 5,13 | -6,41 | 1,51E-08 | 7,01E-06 | Promoter (<= 1 kb) | 912 | ENSG00000126467 | TSKS |
| chr20 | 20247838 | 20249073 | -0,76 | 4,94 | -5,69 | 2,02E-08 | 8,86E-06 | Intron (uc010zse.2/26074, intron 21 of 21) | 41663 | ENSG00000089101 | CFAP61 |
| chr9 | 97877211 | 97879232 | -1,28 | 4,64 | -5,92 | 2,05E-08 | 8,95E-06 | Intron (uc022bkl.1/2176, intron 11 of 14) | -28835 | ENSG00000207617 | MIR3074 |
| chr16 | 356886 | 358462 | -1,02 | 4,36 | -5,38 | 2,18E-08 | 9,36E-06 | Intron (uc021szo.1/55692, intron 1 of 9) | 24271 | ENSG00000185615 | PDIA2 |
| chr17 | 70661660 | 70663990 | -1,28 | 5,3 | -6,58 | 2,25E-08 | 9,58E-06 | Intron (uc002jja.3/201266, intron 7 of 9) | -25049 | ENSG00000227036 | LINC00511 |
| chrX | 119707312 | 119713038 | 1,05 | 7,49 | -6,44 | 2,27E-08 | 9,58E-06 | Promoter(<=1kb) | 0 | ENSG00000158290 | CUL4B |
| chr1 | 9937689 | 9938704 | -1,12 | 4,32 | -5,44 | 2,36E-08 | 9,75E-06 | 5'UTR | 31612 | ENSG00000178585 | CTNNBIP1 |
| chr13 | 67686953 | 67688624 | -1,28 | 4,69 | -5,97 | 2,48E-08 | 9,96E-06 | Intron (uc001vik.3/5101, intron 2 of 4) | 115844 | ENSG00000184226 | PCDH9 |
| chr4 | 3415525 | 3418428 | -1,13 | 4,85 | -5,97 | 2,58E-08 | 1,01E-05 | Promoter(<=1kb) | 569 | ENSG00000159788 | RGS12 |
| chr6 | 7344017 | 7345034 | -1,17 | 4,82 | -5,99 | 2,83E-08 | 1,06E-05 | Intron (uc003mxh.3/285782, intron 7 of 9) | 29330 | ENSG00000164304 | CAGE1 |
| chr22 | 37773019 | 37775864 | -0,1 | 5,55 | -5,65 | 2,86E-08 | 1,06E-05 | Intron (uc003asq.4/114794, intron 2 of 2) | 47641 | ENSG00000166897 | ELFN2 |
| chr12 | 24368577 | 24369865 | -1,28 | 4,71 | -5,99 | 2,87E-08 | 1,06E-05 | Intron (uc001rfx.4/6660, intron 4 of 17) | 3222 | ENSG00000216192 | MIR920 |
| chr19 | 59015509 | 59017748 | -0,06 | 5,54 | -5,6 | 2,87E-08 | 1,06E-05 | Intron (uc002qtc.2/10998, intron 3 of 9) | -4879 | ENSG00000083807 | SLC27A5 |
| chr9 | 129960617 | 129961644 | -0,87 | 4,6 | -5,47 | 2,91E-08 | 1,07E-05 | Exon (uc004bqo.2/9649, exon 14 of 19) | -64297 | ENSG00000136895 | GARNL3 |
| chr17 | 7832223 | 7833447 | -1,28 | 5,22 | -6,49 | 3,11E-08 | 1,13E-05 | Promoter (<= 1 kb) | 0 | ENSG00000170049 | KCNAB3 |
| chr12 | 123559260 | 123561102 | -0,63 | 5,42 | -6,05 | 3,29E-08 | 1,18E-0 5 | Intron (uc001uej.1/57605. intron 1 of 24) | -4829 | ENSG00000251497 | PITPNM2-AS1 |
| chr17 | 8511716 | 8512753 | -0,93 | 4,58 | -5,51 | 3,32E-08 | 1,18E-0 5 | Intron (uc002gll.4/4628, intron 2 of 40) | 21326 | ENSG00000133026 | MYH10 |
| chr6 | 155342015 | 155343524 | -1,28 | 4,22 | -5,5 | 3,34E-08 | 1,19E-05 | Intron (uc003qqb.3/26230, intron 3 of 28) | -67899 | ENSG00000146426 | TIAM2 |
| chr8 | 39837767 | 39839122 | -1,13 | 4,67 | -5,81 | 3,72E-08 | 1,30E-05 | Promoter (1-2kb) | 1696 | ENSG00000188676 | IDO2 |
| chr7 | 51345498 | 51347372 | -1,28 | 4,63 | -5,91 | 3,96E-08 | 1,36E-05 | Intron (uc003tpr.4/23242, intron 1 of 12) | 37143 | ENSG00000106078 | COBL |
| chr2 | 47880785 | 47882405 | -1,28 | 3,85 | -5,12 | 4,28E-08 | 1,42E-05 | Intron (uc002rvz.4/4436, intron 16 of 18) | -83315 | ENSG00000184261 | KCNK12 |
| chr21 | 32862542 | 32864284 | -1,22 | 5,2 | -6,42 | 4,48E-08 | 1,47E-05 | Intron (uc002yow.1/7074, intron 1 of 28) | 67006 | ENSG00000156299 | TIAM1 |
| chr10 | 45406094 | 45408455 | -1,28 | 5,16 | -6,43 | 4,48E-08 | 1,47E-05 | Promoter (<=1kb) | 0 | ENSG00000187783 | TMEM72 |
| chr15 | 26259848 | 26263421 | -1,28 | 5,16 | -6,44 | 4,80E-08 | 1,53E-05 | Exon (uc021sgd.1/100128714, exon 2 of 5) | -97539 | ENSG00000259150 | LINC00929 |
| chr20 | 60029121 | 60030019 | -1,28 | 3,6 | -4,88 | 4,85E-08 | 1,54E-05 | Intron (uc002vbn.2/1002, intron 2 of 15) | -44458 | ENSG00000179242 | CDH4 |
| chr1 | 14127332 | 14129179 | -1,28 | 4,61 | -5,89 | 5,03E-08 | 1,58E-05 | Intron (uc001avg.3/7799, intron 6 of 7) | 51476 | ENSG00000116731 | PRDM2 |
| chr2 | 1322196 | 1323150 | -1,28 | 3,99 | -5,26 | 5,33E-08 | 1,67E-05 | Intron (uc002qwq.3/54221, intron 16 of 16) | -54845 | ENSG00000115705 | TPO |
| chr12 | 96430886 | 96432331 | -0,55 | 5,54 | -6,08 | 5,68E-08 | 1,76E-05 | Promoter (1-2kb) | -1447 | ENSG00000111144 | LTA4H |
| chr6 | 10595011 | 10596991 | -1,28 | 5,07 | -6,34 | 5,91E-08 | 1,80E-05 | Intron (uc010jol.3/2651, intron 3 of 4) | 9018 | ENSG00000111846 | GCNT2 |
| chr3 | 15320247 | 15321858 | -1,28 | 4,41 | -5,69 | 6,04E-08 | 1,82E-05 | Intron (uc003bzp.2/9467, intron 3 of 8) | 24556 | ENSG00000224660 | SH3BP5-AS1 |
| chr17 | 55585823 | 55587115 | -0,98 | 4,4 | -5,38 | 6,08E-08 | 1,82E-05 | Intron (uc010wnm.1/124540, intron 6 of 13) | 246347 | ENSG00000153944 | MSI2 |
| chr1 | 164545376 | 164546615 | -1,28 | 4,19 | -5,47 | 6,21E-08 | 1,85E-05 | Intron (uc001gcs.3/5087, intron 2 of 7) | -12151 | ENSG00000185630 | PBX1 |
| chr14 | 33458403 | 33459537 | -1,11 | 4,46 | -5,57 | 6,25E-08 | 1,85E-05 | Intron (uc001wrs.3/64067, intron 1 of 11) | 49944 | ENSG00000151322 | NPAS3 |
| chr13 | 114610122 | 114612250 | -1,28 | 4,63 | -5,91 | 6,30E-08 | 1,85E-05 | Intron (uc031qnp.1/uc031qnp.1, intron 4 of 7) | 19714 | ENSGO0000260910 | LINC00565 |
| chr1 | 209758744 | 209759586 | -0,14 | 4,91 | -5,05 | 6,50E-08 | 1,90E-05 | Promoter (1-2kb) | 1682 | ENSG00000008118 | CAMK1G |
| chr17 | 17787031 | 17788870 | -1,28 | 5,01 | -6,29 | 6,52E-08 | 1,90E-05 | Exon (uc002gry.4/146691, exon 4 of 14) | -46706 | ENSG00000072310 | SREBF1 |
| chr15 | 99547741 | 99552083 | 0,74 | 6,83 | -6,09 | 6,87E-08 | 2,00E-05 | Promoter (<=1kb) | 0 | ENSG00000183571 | PGPEP1L |
| chr13 | 79169490 | 79171148 | -0,77 | 5,71 | -6,48 | 7,11E-08 | 2,06E-05 | Intron (uc001vku.1/100874222, intron 3 of 5) | 6547 | ENSG00000152192 | POU4F1 |
| chr22 | 40551256 | 40553288 | -1,28 | 4,42 | -5,7 | 7,69E-08 | 2,18E-05 | Exon (uc003aym.3/23112, exon 4 of 24) | -20641 | ENSG00000100354 | TNRC6B |
| chr15 | 42749318 | 42750989 | -1,28 | 4,38 | -5,66 | 8,00E-08 | 2,24E-05 | Promoter (<=1kb) | 0 | ENSG00000103994 | ZNF106 |
| chr2 | 112704332 | 112705800 | -1,28 | 4,03 | -5,31 | 8,12E-08 | 2,27E-05 | Exon (uc002thk.1/10461, exon 4 of 19) | 48141 | ENSG00000153208 | MERTK |
| chr7 | 128452340 | 128453195 | -1,28 | 3,8 | -5,07 | 8,27E-08 | 2,29E-05 | Exon (uc003vnv.2/64753, exon 13 of 18) | 6049 | ENSG00000128596 | CCDC136 |
| chr7 | 143133439 | 143135380 | -1,28 | 4,92 | -6,19 | 8,59E-08 | 2,36E-05 | Intron (uc003wda.4/285965, intron 2 of 4) | -5166 | ENSG00000185899 | TAS2R60 |
| chr7 | 30181652 | 30183435 | -1,28 | 4,64 | -5,92 | 8,64E-08 | 2,37E-05 | Intron (uc011kab.1/222166, intron 1 of 3) | 7100 | ENSG00000180354 | MTURN |
| chr8 | 48664223 | 48665369 | -1,28 | 4,39 | -5,67 | 1,03E-07 | 2,75E-05 | Intron (uc003xqg.1/23514, intron 1 of 1) | -13497 | ENSGO0000221869 | CEBPD |
| chr2 | 216515277 | 216516318 | -1,28 | 4,36 | -5,63 | 1,10E-07 | 2,90E-05 | Intron (uc002vfm.1/646324, intron 5 of 9) | 175808 | ENSG00000235770 | LINC00607 |
| chr22 | 50318174 | 50320460 | -0,86 | 5,41 | -6,26 | 1,22E-07 | 3,16E-05 | 3'UTR | 5891 | ENSG00000184164 | CRELD2 |
| chr5 | 179397097 | 179399688 | -1,28 | 4,39 | -5,67 | 1,27E-07 | 3,22E-05 | Exon (uc003mll.1/55819, exon 6 of 9) | 42709 | ENSG00000198995 | MIR340 |
| chrX | 73064954 | 73072637 | 0,83 | 7,41 | -6,58 | 1,29E-07 | 3,27E-05 | Promoter (<=1kb) | 0 | ENSGO0000229807 | XIST |
| chr3 | 82424436 | 82425921 | -1,28 | 4,77 | -6,05 | 1,42E-07 | 3,52E-05 | Intron (uc003dqh.1/uc003dqh.1, intron 3 of 8) | -613486 | ENSG00000114480 | GBE1 |
| chrX | 130912697 | 130913682 | -1,1 | 4,68 | -5,77 | 1,44E-07 | 3,54E-05 | Intron (uc004ewi.3/286467, intron 6 of 12) | 50989 | ENSG00000213468 | FIRRE |
| chr10 | 108687712 | 108688623 | -1,28 | 3,83 | -5,11 | 1,50E-07 | 3,67E-05 | Intron (uc001kyl.3/114815, intron 2 of 26) | 235843 | ENSG00000108018 | SORCS1 |
| chr8 | 108971240 | 108973062 | -1,28 | 4,19 | -5,46 | 1,67E-07 | 4,02E-05 | Exon (uc003ymq.3/340419, exon 3 of 5) | 121105 | ENSG00000147655 | RSPO2 |
| chr3 | 146180236 | 146181386 | -1,28 | 3,64 | -4,92 | 1,70E-07 | 4,06E-05 | Intron (uc021xfa.1/57047, intron 2 of 9) | 5702 | ENSG00000163746 | PLSCR2 |
| chr7 | 150419146 | 150420059 | -1,28 | 3,88 | -5,16 | 1,75E-07 | 4,15E-05 | 3'UTR | 5501 | ENSG00000213203 | GIMAP1 |
| chr22 | 24145250 | 24146656 | -0,89 | 4,73 | -5,61 | 1,82E-07 | 4,30E-05 | Exon (uc002zya.3/6598, exon 5 of 6) | 11307 | ENSG00000099956 | SMARCB1 |
| chrY | 14529806 | 14533959 | -0,05 | 6,62 | -6,67 | 1,84E-07 | 4,34E-05 | Promoter (<=1kb) | 0 | ENSG00000206159 | GYG2P1 |
| chr3 | 101518292 | 101522783 | -0,89 | 5,27 | -6,16 | 2,09E-07 | 4,86E-05 | Exon (uc003dvn.3/91775, exon 5 of 8) | 20006 | ENSG00000144815 | NXPE3 |
| chr9 | 74304168 | 74305477 | -0,76 | 4,87 | -5,62 | 2,26E-07 | 5,08E-05 | Exon (uc004aik.2/23670, exon 3 of 5) | 7680 | ENSG00000135048 | CEMIP2 |
| chr9 | 96856410 | 96857876 | -1,28 | 4,59 | -5,87 | 2,39E-07 | 5,28E-05 | Exon (uc004auf.2/138639, exon 6 of 10) | 9699 | ENSG00000158079 | PTPDC1 |
| chr16 | 84429568 | 84430996 | -1,28 | 4,14 | -5,42 | 2,48E-07 | 5,42E-05 | Intron (uc002fhx.3/9914, intron 1 of 26) | -7037 | ENSG00000064270 | ATP2C2 |
| chrX | 54168371 | 54169354 | -1,28 | 3,96 | -5,23 | 2,56E-07 | 5,57E-05 | Intron (uc004dsz.4/54954, intron 4 of 15) | 40360 | ENSG00000184083 | FAM120C |
| chr1 | 171233590 | 171234405 | -1,28 | 3,93 | -5,21 | 2,77E-07 | 5,97E-05 | Intron (uc009wvz.3/2326, intron 2 of 8) | 6494 | ENSG00000010932 | FMO1 |
| chr9 | 137259296 | 137260382 | -1,01 | 4,44 | -5,44 | 2,82E-07 | 6,05E-05 | Intron (uc004cfa.1/6256, intron 2 of 2) | -10875 | ENSG00000263897 | MIR4669 |
| chr10 | 3525813 | 3528718 | 1,02 | 6,51 | -5,49 | 3,07E-07 | 6,42E-05 | Intron (uc001igy.1/uc001igy.1, intron 1 of 1) | 298755 | ENSG00000067082 | KLF6 |
| chr22 | 48093948 | 48095760 | -1,28 | 4,65 | -5,93 | 3,09E-07 | 6,44E-05 | Intron (uc003bik.1/uc003bik.1. intron 6 of 8) | -66630 | ENSG00000205634 | LINC00898 |
| chr3 | 135995674 | 135996582 | -1,28 | 3,52 | -4,8 | 3,10E-07 | 6,44E-05 | Intron (uc003eqv.2/5096, intron 5 of 14) | 21511 | ENSG00000 114054 | PCCB |
| chr12 | 25273183 | 25274353 | -1,28 | 3,81 | -5,08 | 3,20E-07 | 6,59E-05 | Intron (uc001rgj.3/55259, intron 12 of 16) | 68002 | ENSG00000118308 | LRMP |
| chr1 | 180930527 | 180932129 | -1,28 | 3,99 | -5,26 | 3,24E-07 | 6,65E-05 | Intron (uc009wxo.1/uc009wxo.1, intron 1 of 1) | 32961 | ENSG00000135835 | KIAA1614 |
| chr3 | 47654666 | 47655690 | -1,28 | 3,89 | -5,17 | 3,3 9E-07 | 6,85E-05 | Intron (uc011bbc.1/6599, intron 11 of 13) | -32936 | ENSG00000 114646 | CSPG5 |
| chr15 | 67794193 | 67794769 | -1,28 | 3,48 | -4,75 | 3,43E-07 | 6,88E-05 | Intron (uc002aqr.2/100506686, intron 2 of 5) | 18542 | ENSG00000259673 | IOCH-AS1 |
| chr12 | 113575582 | 113576357 | -1,28 | 3,25 | -4,53 | 3,70E-07 | 7,29E-05 | Promoter (1-2kb) | -1538 | ENSG00000111344 | RASAL1 |
| chr17 | 73315513 | 73317124 | -0,53 | 5,06 | -5,59 | 3,75E-07 | 7,37E-05 | 3'UTR | -29983 | ENSG00000 125454 | SLC25A19 |
| chr3 | 147105862 | 147107129 | -1,01 | 3,89 | -4,9 | 3,77E-07 | 7,37E-05 | 3'UTR | 3088 | ENSG00000174963 | ZIC4 |
| chr5 | 107481157 | 107482566 | -1,13 | 4,41 | -5,55 | 3,84E-07 | 7,47E-05 | Intron (uc011cvc.2/64839, intron 6 of 8) | 221088 | ENSG00000145743 | FBXL17 |
| chr7 | 51292054 | 51293576 | -1,28 | 4,5 | -5,77 | 4,11E-07 | 7,86E-05 | Intron (uc003tpr.4/23242, intron 1 of 12) | -30768 | ENSG00000106078 | COBL |
| chr1 1 | 111268797 | 111271060 | -1,28 | 4,45 | -5,73 | 4,27E-07 | 8,07E-05 | Intron (uc001plh.1/uc001plh.1. intron 2 of 5) | 17851 | ENSG00000255428 | LOC10013207 8 |
| chr8 | 104760913 | 104762066 | -0,49 | 4,67 | -5,16 | 4,27E-07 | 8,07E-05 | Intron (uc003vlp.3/9699, intron 2 of 23) | -69350 | ENSG00000176406 | RIMS2 |
| chr8 | 113985150 | 113989017 | -1,28 | 4,51 | -5,78 | 4,41E-07 | 8,22E-05 | Exon (uc003ynt.3/114788, exon 8 of 72) | -282871 | ENSG00000164796 | CSMD3 |
| chr10 | 711313 | 712155 | -1,28 | 3,93 | -5,21 | 4,52E-07 | 8,37E-05 | Intron (uc001ifp.3/22982, intron 1 of 36) | 15296 | ENSG00000 180525 | PRR26 |
| chr1 1 | 67407202 | 67408637 | -0,09 | 5,7 | -5,79 | 4,66E-07 | 8,55E-05 | Promoter (<=1kb) | -171 | ENSG00000167800 | TBX10 |
| chr5 | 75604891 | 75605873 | -1,28 | 3,54 | -4,82 | 4,69E-07 | 8,59E-05 | Intron (uc003kei.1/22987, intron 12 of 12) | -93276 | ENSG00000145703 | IQGAP2 |
| chrX | 52895953 | 52897570 | -0,33 | 5,84 | -6,17 | 4,96E-07 | 8,99E-05 | Promoter (<=1kb) | 0 | ENSG00000171402 | XAGE3 |
| chr2 | 165811350 | 165812259 | -1,28 | 3,89 | -5,16 | 5,05E-07 | 9,12E-05 | Promoter (<=1kb) | 0 | ENSG00000169507 | SLC38A11 |
| chr5 | 148809220 | 148811468 | -0,14 | 6,02 | -6,16 | 5,16E-07 | 9,25E-05 | Promoter (<=1kb) | 739 | ENSG00000284182 | MIR143 |
| chrX | 96769655 | 96771750 | -1,28 | 4,38 | -5,65 | 5,16E-07 | 9,25E-05 | Intron (uc004efu.4/1730, intron 26 of 26) | 630748 | ENSG00000204086 | RPA4 |
| chr1 | 171055808 | 171057136 | -1,28 | 3,85 | -5,13 | 5,25E-07 | 9,35E-05 | Promoter (2-3kb) | -2882 | ENSG00000007933 | FMO3 |
| chr16 | 68227130 | 68229145 | -1,06 | 4,13 | -5,19 | 5,39E-07 | 9,53E-05 | Intron (uc010vkm.2/4775, intron 9 of 9) | 37587 | ENSG00000103067 | ESRP2 |
| chr19 | 5307229 | 5308279 | -0,9 | 4,37 | -5,27 | 5,47E-07 | 9,64E-05 | Intron (uc002mbu.1/5802, intron 1 of 30) | -20830 | ENSG00000 105426 | PTPRS |
| chr8 | 58192318 | 58193827 | -1,28 | 4,48 | -5,76 | 5,55E-07 | 9,75E-05 | Promoter (<=1kb) | 216 | ENSG00000215117 | LINC00588 |
| chr12 | 24697764 | 24699564 | -1,28 | 4,39 | -5,67 | 5,60E-07 | 9,83E-05 | Intron (uc001rfx.4/6660, intron 1 of 17) | 15819 | ENSG00000134532 | SOX5 |
| chr4 | 178391510 | 178393168 | -1,28 | 3,87 | -5,15 | 5,83E-07 | 0,000102 | Intron (uc003iux.1/uc003iux.1. intron 2 of 5) | -27853 | ENSG00000038002 | AGA |
| chr9 | 15849994 | 15852698 | -1,28 | 4,38 | -5,66 | 6,00E-07 | 0,000104 | Intron (uc003zmd.3/203238, intron 23 of 25) | 105723 | ENSG00000 164989 | CCDC171 |
| chr3 | 79011476 | 79012684 | -1,28 | 3,54 | -4,82 | 6,12E-07 | 0,000106 | Intron (uc003dqb.2/6091, intron 1 of 28) | 55925 | ENSG00000169855 | ROBO1 |
| ch10 | 50506657 | 50508128 | -1,13 | 4,16 | -5,29 | 6,49E-07 | 0,000112 | Promoter (<=1kb) | 0 | ENSG00000177354 | C1Oorf71 |
| chr15 | 86000440 | 86001656 | -1,28 | 4,33 | -5,61 | 7,04E-07 | 0,000119 | Intron (uc002bls.4/11214, intron 1 of 3) | 76593 | ENSG00000170776 | AKAP13 |
| chr21 | 32623425 | 32624568 | -1,28 | 4,05 | -5,33 | 7,12E-07 | 0,000119 | 5'UTR | 24656 | ENSG00000156299 | TIAM1 |
| chr1 | 7515055 | 7516079 | -1,28 | 3,67 | -4,95 | 7,42E-07 | 0,000123 | Intron (uc001aoi.3/23261, intron 5 of 22) | -215975 | ENSG00000171735 | CAMTA1 |
| chr1 | 68594262 | 68595319 | -1,28 | 3,45 | -4,73 | 7,42E-07 | 0,000123 | Intron (uc001deb.2/100289178, intron 8 of 9) | 53974 | ENSG00000221203 | MIR1262 |
| chr8 | 58964906 | 58966011 | -1,28 | 3,76 | -5,03 | 7,44E-07 | 0,000123 | Intron (uc003xtj.1/90362, intron 3 of 4) | 57793 | ENSG00000169122 | FAM110B |
| chr7 | 73475940 | 73477107 | 0,24 | 5,33 | -5,09 | 7,71E-07 | 0,000127 | Intron (uc003tzn.3/2006, intron 26 of 32) | -21000 | ENSG00000106683 | LIMK1 |
| chr1 | 22136231 | 22137423 | -1,28 | 4,04 | -5,31 | 7,79E-07 | 0,000128 | Promoter (1-2kb) | -1335 | ENSG00000187942 | LDLRAD2 |
| chr22 | 39905030 | 39906666 | -1,28 | 4,25 | -5,53 | 8,03E-07 | 0,000131 | 5'UTR | 6746 | ENSG00000100335 | MIEF1 |
| chr8 | 131277710 | 131278586 | -0,22 | 4,39 | -4,61 | 8,38E-07 | 0,000134 | Intron (uc003vta.2/50807, intron 3 of 29) | 30193 | NA | ASAP1-IT1 |
| chr12 | 24035770 | 24036407 | -1,28 | 3,26 | -4,53 | 8,48E-07 | 0,000135 | Intron (uc001rfw.3/6660, intron 2 of 14) | 66230 | ENSG00000134532 | SOX5 |
| chr8 | 17518296 | 17520177 | -1,02 | 4,34 | -5,36 | 8,67E-07 | 0,000137 | Intron (uc003wxs.3/57509, intron 3 of 9) | 35069 | ENSG00000 129422 | MTUS 1 |
| chr8 | 125183138 | 125184498 | -0,96 | 4,72 | -5,68 | 8,73E-07 | 0,000137 | Exon (uc003vqv.l/uc003vqv.l, exon 1 of 5) | -130115 | ENSG00000181171 | FER1L6-AS1 |
| chr10 | 1307380 | 1308933 | -1,12 | 4,2 | -5,31 | 8,74E-07 | 0,000137 | Intron (uc009xhq.3/105, intron 4 of 9) | -61049 | ENSG00000185736 | ADARB2 |
| chr12 | 64783782 | 64784847 | -1,01 | 3,8 | -4,81 | 8,74E-07 | 0,000137 | Promoter(<=lkb) | 0 | ENSG00000185306 | C12orf56 |
| chr12 | 77171291 | 77172408 | -1,28 | 3,59 | -4,87 | 8,76E-07 | 0,000137 | Intron (ucOOlsvi.1/23390, intron 1 of 7) | 13437 | ENSG00000186908 | ZDHHC17 |
| chr12 | 12147986 | 12149032 | -1,28 | 3,74 | -5,01 | 8,86E-07 | 0,000137 | Intron (uc001raa.l/2120, intron 1 of 1) | -74846 | ENSG00000121380 | BCL2L14 |
| chr18 | 22800834 | 22806748 | 0,5 | 6,72 | -6,23 | 8,87E-07 | 0,000137 | Exon (uc002kvk.2/25925, exon 4 of 8) | 125466 | ENSG00000198795 | ZNF521 |
| chr1 1 | 84734877 | 84736119 | -1,28 | 3,73 | -5,01 | 8,89E-07 | 0,000137 | Intron (uc001pak.2/1740, intron 6 of 27) | -100412 | ENSG00000150672 | DLG2 |
| chr15 | 21939600 | 21940786 | -1,01 | 4,15 | -5,15 | 9,29E-07 | 0,000143 | Promoter(<=lkb) | 0 | NA | LOC646214 |
| chr3 | 77559234 | 77563841 | -0,19 | 5,92 | -6,1 | 9,44E-07 | 0,000144 | Intron (uc021xat.1/6092, intron 5 of 25) | -74061 | ENSG00000185008 | ROBO2 |
| chr7 | 51337189 | 51338458 | -1,28 | 3,71 | -4,98 | 9,63E-07 | 0,000146 | Intron (uc003tpr.4/23242, intron 1 of 12) | 46057 | ENSG00000106078 | COBL |
| chr1 1 | 133026755 | 133028030 | -1,28 | 4,29 | -5,56 | 9,67E-07 | 0,000146 | Intron (uc001qgu.3/4978, intron 1 of 7) | -213092 | ENSG00000183715 | OPCML |
| chr2 | 3642025 | 3643081 | -1,28 | 4,16 | -5,43 | 9,91E-07 | 0,000149 | Promoter(<=lkb) | 0 | ENSG00000 118004 | COLEC11 |
| chr3 | 62709143 | 62710165 | -1,12 | 3,49 | -4,61 | 1,02E-06 | 0,000151 | Intron (uc003dll.2/8618, intron 3 of 29) | -138144 | ENSG00000163618 | CADPS |
| chr8 | 72399092 | 72399735 | -1,28 | 3,6 | -4,88 | 1,02E-06 | 0,000151 | Intron (uc003xyw.3/uc003xyw.3, intron 2 of 3) | -124625 | ENSG00000 104313 | EYA1 |
| chr1 1 | 19487280 | 19488486 | -0,49 | 4,57 | -5,07 | 1,02E-06 | 0,000151 | Intron (uc001mpp.3/89797, intron 1 of 37) | 40290 | ENSG00000255043 | NAV2-AS5 |
| chr15 | 91839659 | 91840550 | -0,75 | 3,98 | -4,73 | 1,04E-06 | 0,000153 | 3'UTR | 196477 | ENSG00000185518 | SV2B |
| chr15 | 52787983 | 52789876 | -1,28 | 4,1 | -5,37 | 1,08E-06 | 0,000158 | Intron (uc002abx.3/4644, intron 1 of 39) | 31371 | ENSG00000197535 | MYO5A |
| chr7 | 111912858 | 111914221 | -1,13 | 4,34 | -5,48 | 1,10E-06 | 0,000159 | Intron (uc003vgc.3/11179, intron 1 of 1) | 66215 | ENSG00000198839 | ZNF277 |
| chr17 | 66863930 | 66865150 | -0,58 | 4,31 | -4,89 | 1,11E-0 6 | 0,000161 | 3'UTR | 86383 | ENSG00000141338 | ABCA8 |
| chr1 1 | 14865392 | 14866836 | -1,28 | 4,15 | -5,43 | 1,15E-06 | 0,000165 | Exon (uc001mln.3/5140, exon 12 of 16) | 42044 | ENSG00000186104 | CYP2R1 |
| chr13 | 48803544 | 48804622 | -1,28 | 3,86 | -5,14 | 1,17E-06 | 0,000167 | Promoter (2-3kb) | -2652 | ENSG00000136156 | ITM2B |
| chr6 | 5111776 | 5113147 | -1,28 | 4,12 | -5,4 | 1,17E-06 | 0,000167 | Intron (uc003mwn.l/uc003mwn.l, intron 3 of 3) | 26056 | ENSG00000219607 | PPP1R3 G |
| chr1 | 7945352 | 7947216 | 0,06 | 5,37 | -5,3 | 1,18E-06 | 0,000167 | Intron (uc001aoq.3/10911, intron 1 of 7) | 26078 | ENSG00000049247 | UTS2 |
| chr18 | 34941107 | 34941887 | -1,28 | 3,59 | -4,86 | 1,25E-06 | 0,000176 | Intron (uc0021ae.2/56853, intron 2 of 12) | -86466 | ENSG00000101489 | CELF4 |
| chr1 1 | 70670543 | 70673331 | -0,99 | 4,55 | -5,54 | 1,27E-06 | 0,000177 | Intron (ucOOloqc.3/22941, intron 8 of 21) | -35564 | ENSG00000171671 | SHANK2-AS3 |
| chrX | 48054992 | 48056351 | -1,28 | 4,15 | -5,42 | 1,29E-06 | 0,000179 | Promoter (<=1kb) | 0 | ENSG00000165583 | SSX5 |
| chrl7 | 47040829 | 47041873 | -1,01 | 3,42 | -4,44 | 1,30E-06 | 0,000181 | Exon (uc002iol.1/2695, exon 3 of 6) | 4082 | ENSG00000159224 | GIP |
| chr3 | 77525095 | 77526509 | -1,13 | 4,32 | -5,46 | 1,37E-06 | 0,000187 | Intron (uc021xat.1/6092, intron 2 of 25) | -111393 | ENSG00000185008 | ROBO2 |
| chr21 | 15939455 | 15940419 | -1,28 | 3,3 | -4,57 | 1,40E-06 | 0,000191 | Intron (uc002vjv.1/64092, intron 2 of 8) | -14104 | ENSG00000223662 | SAMSN1-AS1 |
| chrl 8 | 10790174 | 10791957 | -1,28 | 3,92 | -5,2 | 1,43E-06 | 0,000193 | Promoter (<= 1 kb) | -811 | ENSG00000154864 | PIEZO2 |
| chr18 | 47020105 | 47020655 | -1,28 | 3,56 | -4,84 | 1,45E-06 | 0,000194 | Promoter (1-2kb) | -1170 | ENSG00000265681 | RPL17 |
| chr6 | 168435743 | 168437398 | -0,27 | 4,93 | -5,19 | 1,57E-06 | 0,000207 | Intron (uc003qwk.1/3834, intron 4 of 8) | 17190 | ENSG00000125337 | KIF25 |
| chr21 | 45901230 | 45907032 | 1,06 | 7,86 | -6,8 | 1,67E-06 | 0,000217 | Exon (uc011afp.2/uc011afg.2, exon 1 of 1) | 25837 | ENSG00000160233 | LRRC3 |
| chr6 | 158895911 | 158897714 | -1,28 | 4,03 | -5,31 | 1,73E-06 | 0,000222 | Intron (uc011efo.2/56995, intron 6 of 7) | -59754 | ENSG00000146433 | TMEM181 |
| chr5 | 138346243 | 138347735 | -1,28 | 3,62 | -4,9 | 1,86E-06 | 0,000233 | Intron (uc0031do.3/64374, intron 8 of 10) | 30673 | ENSG00000120725 | SIL1 |
| chr19 | 55447324 | 55452399 | 3,5 | 7,83 | -4,33 | 1,91E-06 | 0,000238 | Exon (uc002qig.4/199713, exon 3 of 11) | 6474 | ENSG00000167634 | NLRP7 |
| chr19 | 18994263 | 18996185 | 0,69 | 6,31 | -5,62 | 1,94E-06 | 0,00024 | Exon (uc002nki.1/2657, exon 3 of 8) | 10768 | ENSG00000223802 | CERS1 |
| chr9 | 35680382 | 35680891 | -1,28 | 2,96 | -4,23 | 1,95E-06 | 0,00024 | Exon (uc003zxo.4/768, exon 10 of 11) | -4519 | ENSG00000137135 | ARHGEF3 9 |
| chr4 | 5276225 | 5276934 | -0,99 | 3,36 | -4,35 | 1,95E-06 | 0,00024 | Intron (uc003gih.1/55351, intron 3 of 11) | 222698 | ENSG00000152953 | STK32B |
| chr4 | 70902239 | 70903709 | -0,26 | 4,46 | -4,72 | 1,96E-06 | 0,00024 | 3'UTR | 8109 | ENSG00000205649 | HTN3 |
| chr5 | 53306403 | 53307228 | -1,13 | 3,37 | -4,5 | 2,04E-06 | 0,000247 | Intron (uc003jpg.1/54622, intron 4 of 4) | -58974 | ENSG00000207627 | MIR581 |
| chr1 1 | 133076104 | 133077079 | -1,28 | 3,72 | -5 | 2,06E-06 | 0,000249 | Intron (uc001qgu.3/4978, intron 1 of 7) | -262441 | ENSG00000183715 | OPCML |
| chr18 | 74091135 | 74093951 | 0,69 | 6,04 | -5,35 | 2,20E-06 | 0,000263 | Promoter(<=1kb) | 0 | ENSG00000101493 | ZNF516 |
| chr1 1 | 129281533 | 129282524 | -1,28 | 3,98 | -5,25 | 2,29E-06 | 0,00027 | Intron (uc001qfc.4/8538.intron 1 of 3) | 35652 | ENSG00000043039 | BARX2 |
| chr9 | 88741914 | 88742811 | -0,89 | 3,44 | -4,34 | 2,30E-06 | 0,00027 | Intron (uc010mqd.1/51280, intron 3 of 6) | -26798 | ENSG00000135052 | GOLM1 |
| chr3 | 150810849 | 150812066 | -1,17 | 3,46 | -4,63 | 2,32E-06 | 0,000271 | Intron (uc003evm.1/11693l, intron 2 of 6) | 6264 | ENSG00000144893 | MED12L |
| chr8 | 70621837 | 70622832 | -1,28 | 3,88 | -5,16 | 2,35E-06 | 0,000273 | Intron (ucOlOlzb.3/81796, intron 3 of 7) | 71998 | ENSG00000137573 | SULF1 |
| chr1 | 151683506 | 151684463 | -1,28 | 3,98 | -5,25 | 2,53E-06 | 0,000288 | Promoter(<=lkb) | 597 | ENSG00000178796 | RIIAD1 |
| chr2 | 218474927 | 218476160 | -1,28 | 3,41 | -4,68 | 2,56E-06 | 0,00029 | Intron (uc002vgo.3/729582, intron 1 of 12) | -9375 | NA | DIRC3 |
| chr3 | 145807928 | 145810114 | -0,87 | 4,47 | -5,34 | 2,59E-06 | 0,000293 | Exon (uc011bnm.1/5352,exon 8 of 20) | -3406 | ENSG00000152952 | PLOD2 |
| chr3 | 12810211 | 12810989 | -1,28 | 2,65 | -3,93 | 2,65E-06 | 0,000298 | Promoter(<=1kb) | 0 | ENSG00000088726 | TMEM40 |
| chr2 | 39235417 | 39237862 | -1,28 | 3,99 | -5,27 | 2,66E-06 | 0,000299 | Exon (uc002rrj.4/6654, exon 8 of 15) | 24589 | ENSG00000115904 | SOS1 |
| chr4 | 103536977 | 103538025 | -1,28 | 3,89 | -5,17 | 2,75E-06 | 0,000305 | 3'UTR | 38106 | ENSG00000109320 | NFKB1 |
| chr16 | 24894815 | 24895979 | 0,16 | 5,03 | -4,87 | 2,78E-06 | 0,000307 | Exon (uc002dms.4/115584, exon 8 of 16) | -22359 | ENSG00000158865 | SLC5A11 |
| chr17 | 65570691 | 65572233 | -1,28 | 3,97 | -5,25 | 2,81E-06 | 0,00031 | Intron (uc002jgb.4/26207, intron 4 of 9) | -141828 | ENSG00000130935 | NOL11 |
| chr14 | 105620716 | 105622960 | 0,76 | 6,47 | -5,71 | 2,93E-06 | 0,00032 | Promoter (2-3kb) | -2976 | ENSG00000184916 | JAG2 |
| chr11 | 134156299 | 134158441 | 0,67 | 6,23 | -5,56 | 2,95E-06 | 0,000321 | Intron (uc010scs.2/112937. intron 6 of 9) | -3576 | ENSG00000166105 | GLB1L3 |
| chr8 | 48571622 | 48572858 | -1,28 | 3,85 | -5,12 | 2,98E-06 | 0,000324 | Intron (uc0101xs.3/23514, intron 6 of 10) | 4972 | ENSG00000 164808 | SPIDR |
| chr9 | 19314718 | 19315428 | -1,28 | 2,85 | -4,13 | 3,06E-06 | 0,00033 | Intron (uc031tcw.1/55667, intron 10 of 31) | -21256 | ENSG00000137145 | DENND4 C |
| chr7 | 93412078 | 93412787 | -1,28 | 3,33 | -4,61 | 3,07E-06 | 0,00033 | Intron (uc003umx.1/2792, intron 1 of 3) | 65838 | ENSG00000265423 | MIR4652 |
| chr7 | 128422387 | 128423942 | 0,46 | 5,29 | -4,83 | 3,09E-06 | 0,000332 | Exon (uc022a1j.1/uc022alj.1, exon 1 of 1) | -6543 | ENSG00000128617 | OPN1 SW |
| chr12 | 25069179 | 25070404 | -1,28 | 3,43 | -4,71 | 3,16E-06 | 0,000337 | Intron(uc001rgd.4/586,intron 1 of 10) | -13170 | ENSG00000060982 | BCAT1 |
| chr17 | 42540874 | 42542174 | -0,79 | 3,82 | -4,6 | 3,22E-06 | 0,000341 | Promoter (<=1kb) | 287 | ENSG00000 186566 | GPATCH8 |
| chr1 | 196977895 | 196979210 | -1,13 | 3,89 | -5,01 | 3,27E-06 | 0,000345 | 3'UTR | 31228 | ENSG00000134389 | CFHR5 |
| chr11 | 8884269 | 8885478 | -0,37 | 4,35 | -4,72 | 3,29E-06 | 0,000346 | Intron (uc001mgv.3/6764. intron 2 of 22) | 7439 | ENSG00000 166444 | ST5 |
| chr8 | 58982434 | 58983683 | -0,86 | 4,23 | -5,08 | 3,30E-06 | 0,000346 | Intron (uc003xtj.1/90362, intron 3 of 4) | 75321 | ENSG00000169122 | FAM110B |
| chr2 | 1051395 | 1053327 | -1,28 | 3,75 | -5,03 | 3,52E-06 | 0,000362 | Intron (uc002qwp.3/54221. intron 1 of 7) | 104841 | ENSG00000172554 | SNTG2 |
| chr15 | 92660012 | 92661226 | -1,28 | 3,81 | -5,09 | 3,53E-06 | 0,000362 | Intron (uc002bqx.2/28232, intron 4 of 9) | 262447 | ENSG00000176463 | SLCO3A1 |
| chr7 | 30185252 | 30186423 | -1,28 | 3,77 | -5,04 | 3,59E-06 | 0,000366 | Exon (uco11kab.1/222166, exon 2 of 4) | 10700 | ENSG00000180354 | MTURN |
| chr7 | 158907592 | 158908856 | -1,28 | 3,46 | -4,74 | 3,69E-06 | 0,000372 | Intron (uc003woh.3/7434, intron 2 of 12) | 28793 | ENSG00000106018 | VIPR2 |
| chr10 | 49666931 | 49668391 | -0,99 | 4,27 | -5,26 | 3,73E-06 | 0,000375 | 5'UTR | -7373 | ENSG00000 128805 | ARHGAP22 |
| chr11 | 103940245 | 103940945 | -1,28 | 3,01 | -4,28 | 3,82E-06 | 0,000383 | Intron (uc001php.3/80310, intron 1 of 6) | 32937 | ENSG00000170967 | DDI1 |
| chr11 | 111233707 | 111234940 | -1,17 | 3,79 | -4,96 | 3,84E-06 | 0,000383 | Intron (uc001plg.4/5450, intron 1 of 4) | 15217 | ENSG00000 110777 | POU2AF1 |
| chr12 | 64375475 | 64376363 | -0,86 | 3,55 | -4,41 | 3,90E-06 | 0,000388 | Promoter (1-2kb) | -1223 | ENSG00000196935 | SRGAP1 |
| chr7 | 139686809 | 139690747 | 0,51 | 6,59 | -6,08 | 3,92E-06 | 0,000389 | Intron (uc003vvh.3/6916, intron 13 of 16) | 72774 | ENSG00000059378 | PARP12 |
| chr5 | 33797428 | 33797850 | -1,28 | 3,28 | -4,56 | 3,97E-06 | 0,000393 | Intron (uc003jia.1/81792, intron 2 of 23) | 94274 | ENSG00000 151388 | ADAMTS12 |
| chr21 | 36561386 | 36562895 | -1,28 | 3,83 | -5,11 | 3,97E-06 | 0,000393 | Intron (uc002vut.1/861, intron 7 of 10) | -139745 | ENSG00000159216 | RUNX1 |
| chr15 | 22155567 | 22159599 | -1,13 | 3,94 | -5,07 | 3,99E-06 | 0,000393 | Intron (uc001yuj.2/uc001yuj.2, intron 11 of 12) | -9765 | NA | NF1P2 |
| chr9 | 139872131 | 139873226 | -0,77 | 4,47 | -5,24 | 4,04E-06 | 0,000396 | Promoter (1-2kb) | 1130 | ENSG00000107317 | PTGDS |
| chr20 | 60025149 | 60025939 | -1,02 | 3,24 | -4,26 | 4,12E-06 | 0,000401 | Intron (uc002vbn.2/1002, intron 2 of 15) | -48538 | ENSG00000179242 | CDH4 |
| chr12 | 6165668 | 6166654 | -0,32 | 4,69 | -5,01 | 4,12E-06 | 0,000402 | Exon (uc001qnn.1/7450. exon 15 of 52) | 67182 | ENSG00000110799 | VWF |
| chr4 | 162705638 | 162706564 | -1,28 | 3,24 | -4,51 | 4,13E-06 | 0,000402 | Intron (uc003iqh.4/56884, intron 4 of 15) | 378622 | ENSG00000168843 | FSTL5 |
| chr21 | 42055365 | 42056645 | -1,28 | 3,67 | -4,94 | 4,15E-06 | 0,000403 | Intron (uc002vvq.1/1826, intron 3 of 32) | -52672 | ENSG00000233756 | DSCAM-IT1 |
| chr4 | 89801224 | 89802634 | -0,55 | 4,1 | -4,65 | 4,16E-06 | 0,000403 | Intron (uc003hse.2/10144, intron 6 of 23) | -56712 | ENSG00000138640 | FAM13A |
| chr15 | 37306833 | 37308335 | -1,28 | 3,74 | -5,02 | 4,36E-06 | 0,000416 | Intron(uc001zjl.3/4212,intron7 of 11) | 82227 | ENSG00000134138 | MEIS2 |
| chr12 | 18420318 | 18421446 | -1,28 | 3,74 | -5,01 | 4,38E-06 | 0,000418 | intron(uc001rdt.3/5288.intronlof31) | 5844 | ENSG00000139144 | PIK3C2G |
| chr1 | 147379899 | 147380619 | -1,18 | 3,41 | -4,6 | 4,40E-06 | 0,000419 | 5'UTR | 4953 | ENSG00000121634 | GJA8 |
| chr2 | 86451558 | 86453159 | -0,49 | 4,51 | -5,01 | 4,44E-06 | 0,000422 | Intron (uc002srh.4/65055, intron 6 of 6) | 25002 | ENSG00000 132313 | MRPL3 5 |
| chr11 | 34661715 | 34662339 | -1,22 | 2,64 | -3,86 | 4,55E-06 | 0,000428 | Intron (uc001mvr.2/26298, intron 1 of 8) | 7704 | ENSG00000135373 | EHF |
| chr6 | 11725764 | 11729659 | 1,94 | 7,32 | -5,39 | 4,67E-06 | 0,000436 | Promoter (<=1kb) | 0 | ENSG00000111863 | ADTRP |
| chr18 | 23751019 | 23751815 | -1,28 | 2,8 | -4,08 | 4,85E-06 | 0,000447 | Intron (uc002kvo.3/143471, intron 4 of 6) | 37203 | ENSG00000154611 | PSMA8 |
| chr10 | 70657400 | 70658392 | -0,86 | 3,46 | -4,32 | 4,96E-06 | 0,000455 | Promoter (2-3kb) | -2642 | ENSG00000107625 | DDX50 |
| chr19 | 33486520 | 33487356 | -1,28 | 3,15 | -4,43 | 4,99E-06 | 0,000455 | Exon (uc002nue.3/85415, exon 8 of 12) | 23372 | ENSG00000 131944 | FAAP24 |
| chr18 | 67562379 | 67563689 | -1,28 | 3,49 | -4,76 | 5,13E-06 | 0,000466 | 5'UTR | 51400 | ENSG00000150637 | CD226 |
| chr9 | 99341308 | 99342583 | -0,87 | 3,94 | -4,81 | 5,23E-06 | 0,000472 | Intron (uc004awj.3/8555. intron 1 of 13) | -12105 | ENSG00000081377 | CDC14B |
| chr1 | 12286101 | 12287392 | -1,13 | 3,29 | -4,41 | 5,27E-06 | 0,000474 | Promoter (2-3kb) | -2721 | ENSG00000048707 | VPS13D |
| chr2 | 39764559 | 39765349 | -1,28 | 3,01 | -4,28 | 5,45E-06 | 0,000484 | Intron (uc002rrq.3/728730, intron 5 of 8) | 23272 | ENSG00000231312 | MAP4K3-DT |
| chr14 | 36607873 | 36608788 | -1,28 | 3,1 | -4,38 | 5,45E-06 | 0,000484 | Intron (uc031qog.1/101101773, intron 1 of 2) | 37069 | NA | PTCSC3 |
| chr11 | 67983657 | 67984663 | -0,65 | 4,25 | -4,9 | 5,51E-06 | 0,000488 | Promoter (2-3kb) | -2589 | ENSG00000 110066 | KMT5B |
| chr9 | 9855412 | 9855968 | -0,98 | 2,96 | -3,94 | 5,57E-06 | 0,000491 | Intron (uc003zkk.3/5789, intron 5 of 45) | 756755 | ENSG00000153707 | PTPRD |
| chr3 | 44937683 | 44938423 | -0,95 | 3,22 | -4,16 | 5,61E-06 | 0,000494 | Exon (uc003cob.3/7047, exon 5 of 6) | 21585 | ENSG00000163810 | TGM4 |
| chr12 | 24441630 | 24442789 | -1,28 | 3,3 | -4,57 | 5,64E-06 | 0,000495 | Intron (ucOOlrfx.4/6660, intron 3 of 17) | 76275 | ENSG00000216192 | MIR920 |
| chr6 | 143946991 | 143948078 | -1,28 | 3,58 | -4,86 | 5,67E-06 | 0,000496 | Intron (uc003qjq.4/9749. intron 1 of 12) | 17674 | ENSG00000112419 | PHACTR2 |
| chr3 | 71591345 | 71592426 | -0,95 | 3,67 | -4,62 | 5,86E-06 | 0,000511 | Promoter (<=1kb) | -105 | ENSG00000221264 | MIR1284 |
| chr11 | 13369659 | 13371383 | -0,98 | 4,1 | -5,08 | 6,18E-06 | 0,00053 | Promoter (<=1kb) | -109 | ENSG00000133794 | ARNTL |
| chr9 | 71837871 | 71838669 | -1,28 | 3,25 | -4,53 | 6,19E-06 | 0,00053 | Intron(uc011lrs.2/9414, intron 6 of 21) | 17793 | ENSG00000119139 | TJP2 |
| chr9 | 15854120 | 15855918 | -1,28 | 3,62 | -4,9 | 6,41E-06 | 0,000545 | Intron (uc003zmd.3/20323 8, intron 23 of 25) | 109849 | ENSG00000 164989 | CCDC171 |
| chr12 | 64708127 | 64708998 | -1,28 | 2,95 | -4,23 | 6,48E-06 | 0,000548 | Intron (uc001srx.3/uc001srx.3, intron 2 of 4) | -20967 | ENSG00000185306 | C12orf56 |
| chr6 | 44240587 | 44241688 | -0,4 | 3,43 | -3,83 | 6,49E-06 | 0,000548 | Promoter (<= 1 kb) | 0 | ENSG00000249481 | SPATS 1 |
| chr1 | 92502601 | 92503671 | -1,28 | 3,3 | -4,58 | 6,63E-06 | 0,000558 | Intron (uc001don.2/253152, intron 2 of 6) | 7068 | ENSG00000 172031 | EPHX4 |
| chr15 | 98360295 | 98361322 | -1,28 | 3,06 | -4,33 | 6,65E-06 | 0,000559 | Intron (uc002bug.1/91948, intron 1 of 2) | 56337 | ENSG00000251209 | LINC00923 |
| chr7 | 141087056 | 141087935 | -1,22 | 3,1 | -4,32 | 6,74E-06 | 0,000564 | Intron (uc003vwg.2/100507421, intron 2 of 3) | -163143 | ENSG00000006530 | AGK |
| chr10 | 117703458 | 117704469 | -1,28 | 3,06 | -4,34 | 6,88E-06 | 0,000572 | 3'UTR | 327350 | ENSG00000151892 | GFRA1 |
| chr10 | 35041951 | 35043699 | -1,28 | 3,32 | -4,6 | 6,90E-06 | 0,000573 | Intron (uc010qej.2/56288, intron 1 of 24) | 60554 | ENSG00000 148498 | PARD3 |
| chr15 | 55792905 | 55793947 | -1,28 | 3,51 | -4,79 | 7,02E-06 | 0,00058 | Promoter (2-3kb) | -2123 | ENSG00000256061 | DNAAF4 |
| chr11 | 33311975 | 33314252 | 0,99 | 5,82 | -4,83 | 7,07E-06 | 0,00058 | Intron (uc031pzm.1/10114, intron 2 of 15) | 32098 | ENSG00000 110422 | HIPK3 |
| chr1 | 65686994 | 65688503 | -1,03 | 3,83 | -4,86 | 7,14E-06 | 0,000583 | Intron (uc001dbv.3/205, intron 4 of 5) | -31645 | ENSG00000 116675 | DNAJC6 |
| chr3 | 187416301 | 187417107 | -0,71 | 3,5 | -4,21 | 7,22E-06 | 0,000588 | Exon (uc003fro.1/344892, exon 2 of 2) | -3047 | ENSG00000228804 | LOC10013163 5 |
| chr1 | 158907396 | 158908270 | -1,28 | 2,99 | -4,27 | 7,26E-06 | 0,00059 | Exon (uc001fta.4/149628,exon 3 of 5) | 6059 | ENSG00000163564 | PYHIN1 |
| chr16 | 19868917 | 19870364 | 0,42 | 5,4 | -4,99 | 7,32E-06 | 0,000593 | 3'UTR | 13804 | ENSG00000167191 | GPRC5B |
| chr1 | 173491006 | 173491598 | -1,28 | 2,51 | -3,79 | 7,35E-06 | 0,000595 | Intron (uc009wwe.2/284525, intron 14 of 20) | 44520 | ENSG00000117592 | PRDX6 |
| chr15 | 33297744 | 33298836 | -1,13 | 3,21 | -4,35 | 7,37E-06 | 0,000595 | Intron (uc001zhf.5/342184, intron 3 of 16) | 61397 | ENSG00000248905 | FMN1 |
| chr3 | 181436995 | 181437761 | -1,22 | 3,08 | -4,31 | 7,54E-06 | 0,000609 | Intron (uc003fkv.4/347689, intron 7 of 7) | 7283 | ENSG00000181449 | SOX2 |
| chr15 | 50372780 | 50373405 | -1,28 | 2,5 | -3,78 | 7,63E-06 | 0,000615 | Intron (uc010ber.3/79895, intron 2 of 28) | 32108 | ENSG00000104043 | ATP8B4 |
| chr1 1 | 115332464 | 115333694 | -1,28 | 3,46 | -4,74 | 7,64E-06 | 0,000615 | Intron (uc001ppf.4/23705, intron 1 of 8) | 41547 | ENSG00000 182985 | CADM1 |
| chrl7 | 7114511 | 7115623 | -0,2 | 4,74 | -4,94 | 7,65E-06 | 0,000615 | Intron (uc010cly.3/1742, intron 1 of 19) | -4821 | ENSG00000072778 | ACADVL |
| chr7 | 106796967 | 106797835 | -1,28 | 2,98 | -4,26 | 7,69E-06 | 0,000616 | Exon (uc003vdx.3/5577, exon 9 of 11) | -11571 | ENSG00000105856 | HBP1 |
| chr10 | 72043294 | 72044247 | -0,4 | 4,43 | -4,83 | 7,83E-06 | 0,000625 | Promoter(<=1kb) | 0 | ENSG00000148734 | NPFFR1 |
| chr2 | 201523653 | 201524908 | -0,76 | 4,28 | -5,04 | 7,84E-06 | 0,000625 | Exon (uc002uvx.3/316, exon 28 of 35) | -35538 | NA | AOX2P |
| chr17 | 43501775 | 43503473 | 0,67 | 6,12 | -5,45 | 7,96E-06 | 0,000633 | Promoter(<=1kb) | 0 | ENSG00000 159314 | ARHGAP27 |
| chr19 | 42546238 | 42546995 | -1,28 | 3,45 | -4,72 | 7,99E-06 | 0,000635 | Promoter(<=1kb) | 58 | ENSG00000105737 | GRIK5 |
| chr15 | 66858050 | 66858659 | -1,13 | 2,24 | -3,38 | 8,11E-06 | 0,000644 | Promoter(<=1kb) | 0 | ENSG00000188501 | LCTL |
| chr3 | 140400930 | 140402013 | -1,28 | 3,46 | -4,74 | 8,34E-06 | 0,000658 | Exon (uc003eto.2/287015, exon 2 of 5) | 4064 | ENSG00000155890 | TRIM42 |
| chr2 | 68518294 | 68518772 | -1,28 | 2,52 | -3,79 | 8,47E-06 | 0,000664 | Intron (uc002sej.4/25927, intron 2 of 2) | 28247 | ENSG00000 119865 | CNRIP1 |
| chr6 | 164091864 | 164094363 | 1,97 | 7,31 | -5,34 | 8,71E-06 | 0,00068 | Exon (uc003quk.l/uc003quk.l, exon 1 of 4) | 256189 | ENSG00000112531 | QKI |
| chr7 | 139462881 | 139464269 | -1,28 | 3,46 | -4,74 | 8,86E-06 | 0,000689 | Intron (uc003vvd.4/28996, intron 1 of 14) | 13424 | ENSG00000064393 | HIPK2 |
| chr6 | 55104563 | 55105504 | -0,81 | 3,31 | -4,12 | 9,07E-06 | 0,000698 | Promoter(<=1kb) | 0 | ENSG00000137252 | HCRTR2 |
| chr5 | 101833089 | 101835031 | -1,28 | 3,39 | -4,66 | 9,25E-06 | 0,000707 | Promoter(<=1kb) | 0 | ENSG00000205359 | SLCO6A1 |
| chr6 | 54055272 | 54056299 | -1,28 | 3,34 | -4,62 | 9,26E-06 | 0,000707 | Intron (uc003pcf.2/90523, intron 10 of 11) | 79027 | ENSG00000146147 | MLIP |
| chr21 | 38582225 | 38583289 | -1,28 | 3,48 | -4,75 | 9,54E-06 | 0,000725 | Promoter (1-2kb) | 1421 | ENSG00000230366 | DSCR9 |
| chr3 | 121948801 | 121949568 | -1,28 | 2,9 | -4,18 | 9,58E-06 | 0,000726 | Intron (uc003eev.4/846, intron 1 of 6) | 45620 | ENSG00000036828 | CASR |
| chr8 | 63648333 | 63649520 | -0,71 | 3,67 | -4,38 | 9,58E-06 | 0,000726 | Intron (ucOlOlvq.1/286183, intron 3 of 5) | -240900 | ENSG00000274956 | NKAIN3 -IT1 |
| chr6 | 152929781 | 152930885 | -1,28 | 3,01 | -4,29 | 9,64E-06 | 0,000728 | Intron (uc003qot.4/23345, intron 2 of 145) | 18804 | ENSG00000 131018 | SYNE1 |
| chr11 | 71541828 | 71542539 | -1,17 | 3,26 | -4,43 | 9,65E-06 | 0,000728 | Promoter (1-2kb) | -1707 | ENSG00000184276 | DEFB108B |
| chr7 | 69626791 | 69627317 | -1,28 | 3,02 | -4,3 | 9,76E-06 | 0,000734 | Intron (uc003tvv.4/26053, intron 4 of 4) | 562886 | ENSG00000158321 | AUTS2 |
| chr3 | 65342060 | 65342854 | -1,13 | 3,58 | -4,7 | 9,79E-06 | 0,000735 | 3'UTR | 44525 | ENSG00000 151276 | MAGI1 |
| chr16 | 84227714 | 84229238 | -0,46 | 3,85 | -4,31 | 1,01E-05 | 0,000756 | Promoter (2-3kb) | 2991 | ENSG00000140955 | ADAD2 |
| chr15 | 85453898 | 85454668 | -1,28 | 3,46 | -4,74 | 1,01E-05 | 0,000751 | Intron (uc010upd.1/9154, intron 8 of 11) | 25985 | ENSG00000 156222 | SLC28A1 |
| chr10 | 18775564 | 18776695 | -1,28 | 2,94 | -4,21 | 1,03E-05 | 0,000765 | Intron (uc001ipr.2/783, intron 3 of 13) | 86051 | ENSG00000165995 | CACNB2 |
| chr3 | 121711394 | 121712611 | -1,28 | 3,01 | -4,28 | 1,03E-05 | 0,000767 | Exon (uc003eeq.3/286676, exon 5 of 6) | 13537 | ENSG00000 145103 | ILDR1 |
| chr8 | 41667391 | 41668533 | -0,46 | 3,81 | -4,26 | 1,04E-05 | 0,000773 | Intron (uc003xom.3/286, intron 1 of 42) | -12251 | ENSG00000029534 | ANK1 |
| chr7 | 36275646 | 36276316 | -1,28 | 3,34 | -4,61 | 1,07E-05 | 0,000784 | Intron (uc003tfa.3/80820, intron 2 of 7) | 82810 | ENSG00000122547 | EEPD1 |
| chr4 | 186006802 | 186007924 | -0,28 | 4,48 | -4,76 | 1,07E-05 | 0,000785 | Exon (uc003ixc.1/uc003ixc.1. exon 6 of 9) | -56493 | ENSG00000151729 | SLC25A4 |
| chr9 | 125145570 | 125146889 | -0,75 | 3,64 | -4,4 | 1,09E-05 | 0,000793 | Exon (uc010mwb.2/5742. exon 8 of 11) | 7969 | ENSG00000095303 | PTGS1 |
| chr5 | 76151482 | 76152871 | -1,01 | 3,14 | -4,16 | 1,09E-05 | 0,000792 | Intron (uc003kep.1/170591. intron 1 of 3) | 5656 | ENSG00000171643 | S100Z |
| chr14 | 57270673 | 57271606 | -1,16 | 3,1 | -4,26 | 1,10E-05 | 0,000799 | Promoter (<=1kb) | 775 | ENSG00000165588 | OTX2 |
| chr7 | 148017734 | 148018652 | -1,28 | 3,38 | -4,66 | 1,11E-0 5 | 0,000802 | Intron (uc003weu.2/26047, intron 21 of 23) | -17901 | ENSG00000174469 | CNTNAP2 |
| chr1 | 214637318 | 214638446 | -0,06 | 4,83 | -4,89 | 1,11E-0 5 | 0,000802 | Promoter (<=1kb) | 0 | ENSG00000152104 | PTPN14 |
| chr7 | 95156888 | 95157740 | -1,28 | 3,45 | -4,72 | 1,12E-0 5 | 0,000806 | 3'UTR | 41675 | ENSG00000005981 | ASB4 |
| chr7 | 124561560 | 124562450 | -1,28 | 3,42 | -4,69 | 1,12E-0 5 | 0,000805 | Intron (uc011koe.2/25913, intron 2 of 18) | 7406 | ENSG00000128513 | POT1 |
| chr3 | 85468034 | 85469125 | -1,28 | 3,4 | -4,68 | 1,19E-0 5 | 0,000847 | Intron (uc003dqj.3/253559, intron 1 of 9) | 33174 | ENSG00000264084 | MIR5688 |
| chr7 | 136962704 | 136963512 | -1,28 | 2,94 | -4,22 | 1,22E-05 | 0,000864 | Intron (uc003vtq.2/5764, intron 1 of 4) | 65034 | ENSG00000105894 | PTN |
| chr10 | 29810876 | 29812111 | 0,26 | 4,93 | -4,66 | 1,22E-05 | 0,000861 | Promoter (<=1kb) | 0 | ENSG00000197321 | SVIL |
| chr10 | 460952 | 462537 | 0,93 | 5,75 | -4,82 | 1,23E-05 | 0,000868 | Promoter(<=1kb) | -902 | ENSG00000 151240 | DIP2C |
| chr2 | 242048584 | 242049595 | -1,28 | 3,26 | -4,54 | 1,23E-05 | 0,000868 | Intron (uc010zol.2/23178, intron 12 of 14) | -6837 | ENSG00000 122085 | MTERF4 |
| chrX | 70127810 | 70128620 | -1,28 | 3,32 | -4,59 | 1,23E-05 | 0,000867 | Promoter(<=1kb) | 0 | ENSG00000 120498 | TEX11 |
| chr20 | 62096561 | 62098064 | -0,36 | 4,04 | -4,39 | 1,31E-05 | 0,000905 | Intron (uc002vev.1/3785, intron 1 of 16) | 5929 | ENSG00000075043 | KCNQ2 |
| chr1 | 154490775 | 154491630 | -1,28 | 3,04 | -4,31 | 1,32E-05 | 0,000909 | Promoter (2-3kb) | -2169 | ENSG00000163239 | TDRD10 |
| chr22 | 29873720 | 29874661 | -1,17 | 3,42 | -4,59 | 1,3 3E-05 | 0,000915 | Promoter (1-2kb) | -1520 | ENSG00000 100285 | NEFH |
| chr22 | 26951167 | 26951936 | -1,12 | 3,01 | -4,13 | 1,35E-0 5 | 0,000921 | Promoter(<=1kb) | 0 | ENSG00000221760 | MIR548J |
| chr10 | 37496844 | 37497887 | -1,28 | 3,36 | -4,64 | 1,36E-05 | 0,000921 | Intron (uc021ppc.1/91074, intron 31 of 34) | 82059 | ENSG00000148513 | ANKRD30A |
| chr10 | 17172882 | 17173429 | -1,12 | 2,69 | -3,81 | 1,36E-05 | 0,000921 | Promoter (1-2kb) | -1066 | ENSG00000107611 | CUBN |
| chr6 | 169928983 | 169931333 | -0,93 | 3,93 | -4,86 | 1,37E-05 | 0,000928 | Intron (uc003qwv.2/253769, intron 13 of 14) | 129579 | ENSG00000 184465 | WDR2 7 |
| chr1 | 51311877 | 51312503 | -1,28 | 3,3 | -4,58 | 1,38E-0 5 | 0,00093 | Intron (uc009vvw.1/1 1124, intron 2 of 15) | 113022 | ENSG00000 185104 | FAF1 |
| chr10 | 55823256 | 55824729 | -0,96 | 3,34 | -4,3 | 1,38E-0 5 | 0,00093 | Intron (uc010qhq.2/65217, intron 19 of 34) | 599321 | ENSG00000150275 | PCDH15 |
| chr14 | 102646230 | 102647637 | -1,02 | 3,19 | -4,21 | 1,42E-05 | 0,00095 | Intron (uc001ykz.3/91833. intron 1 of 2) | 40041 | ENSG00000140153 | WDR20 |
| chr18 | 55862365 | 55863240 | 0,01 | 4,69 | -4,68 | 1,43E-05 | 0,000956 | Promoter(<=1kb) | 0 | ENSG00000049759 | NEDD4L |
| chr4 | 35995734 | 35996470 | -1,28 | 2,86 | -4,13 | 1,49E-05 | 0,000988 | Intron (uc003gso.3/116984, intron 10 of 12) | 78987 | ENSG00000047365 | ARAP2 |
| chr9 | 3369418 | 3370269 | -1,28 | 3,2 | -4,47 | 1,49E-05 | 0,000989 | Intron (uc003zhr.3/5991, intron 3 of 17) | 119159 | ENSG00000080298 | RFX3 |
| chr3 | 177426396 | 177427324 | -1,28 | 3,22 | -4,49 | 1,50E-05 | 0,000994 | Intron (uc031sch.1/100505566, intron 3 of 3) | 266687 | NA | LINC00578 |
| chr15 | 25985362 | 25986562 | -1,28 | 3,15 | -4,43 | 1,52E-05 | 0,001 | Intron (uc010ayu.3/57194, intron 2 of 20) | 107410 | ENSG00000266517 | MIR4715 |
| chr12 | 22440863 | 22443048 | 0,34 | 4,67 | -4,32 | 1,52E-05 | 0,001 | Intron (uc001rfo.4/6489, intron 1 of 4) | 44600 | ENSG00000 111728 | ST8SIA1 |
| chr7 | 75183976 | 75184951 | 0,6 | 4,45 | -3,85 | 1,57E-05 | 0,00103 | Exon (uc003uds.2/3092, exon 19 of 31) | -26523 | ENSG00000127957 | PMS2P3 |
| chr14 | 38679691 | 38681296 | -0,78 | 3,76 | -4,54 | 1,64E-05 | 0,00106 | Promoter (2-3kb) | 2487 | ENSG00000139874 | SSTR1 |
| chr12 | 31324357 | 31326363 | -1,28 | 3,2 | -4,47 | 1,66E-05 | 0,00107 | Intron(uc010sjy.1/uc010sjy.1,intron4 of29) | 68996 | ENSG00000013573 | DDX11 |
| chr12 | 86669090 | 86671208 | -0,13 | 4,54 | -4,67 | 1,69E-05 | 0,00108 | Intron (uc001taj.4/25834, intron 4 of 8) | -19000 | ENSG00000 182050 | MGAT4C |
| chr15 | 69886570 | 69887381 | -1,13 | 3,16 | -4,29 | 1,70E-05 | 0,00108 | Intron (uc002asi.1/uc002asi.1, intron 1 of 1) | 32511 | NA | DRAIC |
| chr10 | 6316895 | 6317960 | -0,69 | 3,87 | -4,56 | 1,73E-05 | 0,00109 | Promoter (1-2kb) | -1690 | ENSG00000215244 | LINC02649 |
| chr11 | 19396706 | 19397519 | -1,12 | 3,02 | -4,14 | 1,73E-05 | 0,00109 | Intron (uc001mpp.3/89797, intron 1 of 37) | 24435 | ENSG00000166833 | NAV2 |
| chr2 | 209048311 | 209049169 | -0,89 | 3,66 | -4,55 | 1,74E-05 | 0,00109 | Intron (uc002vcr.3/389073, intron 3 of 8) | 5604 | ENSG00000188674 | C2orf80 |
| chr5 | 41763086 | 41764135 | -1,28 | 3,04 | -4,32 | 1,74E-05 | 0,00109 | Intron (uc011cpo.2/5019, intron 2 of 5) | 30202 | ENSG00000083720 | OXCT1 |
| chr8 | 43013588 | 43014246 | -1,28 | 2,83 | -4,1 | 1,75E-05 | 0,0011 | Exon (uc003xpx.4/138050, exon 3 of 18) | 17996 | ENSG00000165102 | HGSNAT |
| chr6 | 28599669 | 28604502 | 2,06 | 7,09 | -5,04 | 1,75E-05 | 0,0011 | Exon (uc021ysc.1/uc021vsc.1, exon 1 of 1) | -44557 | ENSG00000232040 | ZBED9 |
| chr17 | 27570876 | 27571784 | -1,28 | 3,29 | -4,56 | 1,77E-05 | 0,00111 | Promoter (2-3kb) | -2091 | ENSG00000108255 | CRYBA1 |
| chr11 | 103304182 | 103305134 | -1,28 | 3,17 | -4,45 | 1,80E-05 | 0,00112 | Intron (uc001phn.1/79659, intron 85 of 89) | 324022 | ENSG00000187240 | DYNC2H1 |
| chr15 | 26234234 | 26235700 | -0,06 | 4,85 | -4,91 | 1,82E-05 | 0,00113 | Intron (uc021sgd.1/100128714, intron 1 of 4) | 86727 | ENSG00000206187 | LINC02346 |
| chr18 | 74709153 | 74710243 | -0,66 | 3,53 | -4,2 | 1,82E-05 | 0,00113 | Intron (uc0021ml.3/4155, intron 2 of 6) | -9241 | ENSG00000197971 | MBP |
| chr1 | 183204283 | 183204982 | -1,28 | 3,15 | -4,42 | 1,85E-05 | 0,00115 | Exon (uc001gpz.4/3918, exon 16 of 22) | 43435 | ENSG00000157064 | NMNAT2 |
| chr1 | 18609173 | 18610499 | -1,02 | 3,58 | -4,6 | 1,86E-05 | 0,00115 | Intron (uc001bau.2/84966, intron 2 of 9) | -77362 | ENSG00000117154 | IGSF21 |
| chr1 | 23107360 | 23108008 | -1,07 | 3,21 | -4,28 | 1,86E-05 | 0,00115 | Exon (uc009vqj.1/2048, exon 2 of 17) | 61350 | ENSG00000265422 | MIR4684 |
| chr11 | 133022654 | 133023667 | -0,91 | 3,54 | -4,45 | 1,86E-05 | 0,00115 | Intron (uc001qgu.3/4978, intron 1 of 7) | -208991 | ENSG00000183715 | OPCML |
| chr19 | 16556356 | 16558013 | -1,28 | 3,17 | -4,45 | 1,88E-05 | 0,00115 | Intron (uc002ndx.4/58513, intron 1 of 23) | -8532 | ENSG00000127527 | EPS15L1 |
| chr3 | 113345528 | 113346160 | -1,28 | 2,65 | -3,93 | 1,88E-05 | 0,00115 | Intron (uc003eak.3/54847, intron 24 of 24) | 21771 | ENSG00000072858 | SIDT1 |
| chr9 | 135042011 | 135042286 | -1,08 | 2,1 | -3,17 | 1,89E-05 | 0,00116 | 5'UTR | 4677 | ENSG00000196358 | NTNG2 |
| chr19 | 19267458 | 19268909 | -1,28 | 3,16 | -4,43 | 1,91E-05 | 0,00116 | Intron (uc002nll.2/100271849, intron 1 of 8) | 12189 | ENSG00000213999 | MEF2B |
| chr12 | 78485205 | 78485989 | -1,13 | 2,88 | -4,01 | 1,92E-05 | 0,00117 | Intron (uc001syo.3/89795, intron 12 of 38) | -25818 | ENSGO0000067798 | NAV3 |
| chr7 | 69111520 | 69112557 | -0,28 | 4,56 | -4,84 | 1,93E-05 | 0,00117 | Intron (uc003tvv.4/26053, intron 1 of 4) | 47615 | ENSG00000158321 | AUTS2 |
| chr1 | 177974391 | 177975298 | -0,65 | 3,78 | -4,43 | 1,94E-05 | 0,00117 | Exon (uc001glm.3/730102, exon 10 of 10) | 16505 | ENSG00000120341 | SEC16B |
| chr7 | 15295513 | 15296422 | -0,65 | 4,02 | -4,67 | 1,96E-05 | 0,00118 | Intron (uc003stb.1/392636, intron 12 of 12) | 305218 | ENSG00000187546 | AGMO |
| chr7 | 14743531 | 14744531 | -0,77 | 3,18 | -3,95 | 1,96E-05 | 0,00118 | Intron (uc003ssz.3/1607, intron 5 of 24) | 136544 | ENSG00000136267 | DGKB |
| chr16 | 6134862 | 6135625 | -1,28 | 2,79 | -4,07 | 2,03E-05 | 0,00121 | Intron (uc002cyr.1/54715, intron 1 of 11) | 65158 | ENSG00000078328 | RBFOX1 |
| chr7 | 111133441 | 111134204 | -1,22 | 2,96 | -4,18 | 2,04E-05 | 0,00121 | Intron (uc003vfq.2/83943, intron 2 of 5) | 68143 | ENSG00000184903 | IMMP2L |
| chrX | 114523398 | 114527710 | 1,24 | 6,64 | -5,4 | 2,04E-05 | 0,00121 | Promoter(<=1kb) | 0 | ENSG00000102021 | LUZP4 |
| chr1 | 38200564 | 38201444 | 0,46 | 4,88 | -4,42 | 2,06E-05 | 0,00122 | Exon (uc001cbu.3/284656, exon 2 of 17) | -3310 | ENSG00000183317 | EPHA10 |
| chr6 | 10517491 | 10518576 | -0,86 | 3,8 | -4,67 | 2,06E-05 | 0,00122 | Promoter (2-3kb) | -2992 | ENSG00000111846 | GCNT2 |
| chr6 | 168466749 | 168468176 | -0,54 | 3,96 | -4,51 | 2,09E-05 | 0,00123 | Promoter(<=1kb) | 0 | ENSG00000153303 | FRMD1 |
| chr6 | 88332833 | 88334163 | -1,28 | 2,86 | -4,13 | 2,10E-05 | 0,00124 | Intron (uc011dzl.2/23595, intron 11 of 14) | 33048 | ENSG00000135336 | ORC3 |
| chr1 | 15487172 | 15488039 | -0,62 | 3,33 | -3,96 | 2,10E-05 | 0,00124 | Intron (uc001avw.4/55092, intron 1 of 3) | 6943 | ENSG00000171729 | TMEM51 |
| chr16 | 70355835 | 70357019 | -0,93 | 3,16 | -4,09 | 2,12E-05 | 0,00124 | Intron (uc031qwx.1/11269, intron 3 of 9) | 22773 | ENSG00000168872 | DDX19A |
| chr3 | 48236064 | 48237680 | 0,13 | 4,57 | -4,44 | 2,12E-05 | 0,00124 | Promoter (<=1kb) | -374 | ENSG00000265483 | MIR4443 |
| chr1 | 217630764 | 217632274 | 0,55 | 5,15 | -4,59 | 2,16E-05 | 0,00126 | Intron (uc001hlf.1/55105, intron 8 of 9) | 48276 | ENSGO0000092978 | GPATCH2 |
| chr11 | 96232881 | 96233848 | -1,28 | 2,68 | -3,96 | 2,16E-05 | 0,00126 | Intron (uc031qdh.1/100874053, intron 1 of 4) | 6193 | NA | JRKL-AS1 |
| chr3 | 123030060 | 123034501 | 0,61 | 5,79 | -5,18 | 2,17E-05 | 0,00126 | Exon (uc021xdd.1/111, exon 12 of 21) | 88907 | ENSG00000173175 | ADCY5 |
| chr10 | 111965296 | 111966157 | -0,84 | 3,48 | -4,32 | 2,19E-05 | 0,00127 | Promoter (1-2kb) | -1206 | ENSG00000119950 | MXI1 |
| chr20 | 5177309 | 5178303 | -1,28 | 3,15 | -4,42 | 2,22E-05 | 0,00128 | 3'UTR | 17846 | ENSG00000101290 | CDS2 |
| chr17 | 63159709 | 63160770 | -1,13 | 3,24 | -4,36 | 2,22E-05 | 0,00128 | Intron (uc021ubw.1/8787, intron 7 of 16) | -24052 | ENSG00000108370 | RGS9 |
| chr14 | 31159097 | 31161054 | 0,02 | 4,84 | -4,81 | 2,22E-05 | 0,00128 | Intron (uc010amd.2/23256, intron 13 of 17) | 67572 | ENSG00000092108 | SCFD1 |
| chr6 | 7820415 | 7821106 | -1,28 | 2,7 | -3,98 | 2,23E-05 | 0,00128 | Intron(uc003mxu.4/654,intron 1 of 6) | 89204 | ENSG00000239264 | TXNDC5 |
| chr10 | 45948355 | 45950852 | -0,42 | 4,57 | -4,99 | 2,24E-05 | 0,00129 | Exon (uc001jcf.3/uc001jcf.3, exon 1 of 1) | 6800 | ENSG00000165406 | MARCH8 |
| chr2 | 215372142 | 215373084 | -1,13 | 3,36 | -4,5 | 2,27E-05 | 0,0013 | Promoter (1-2kb) | -1822 | ENSG00000224257 | VWC2L-IT1 |
| chr5 | 142179914 | 142181234 | -0,71 | 3,73 | -4,44 | 2,31E-05 | 0,00132 | Intron (uc003lmt.3/23092, intron 1 of 22) | 29622 | ENSG00000145819 | ARHGAP26 |
| chr17 | 30885505 | 30886006 | -1,28 | 2,58 | -3,85 | 2,32E-05 | 0,00132 | Intron (uc002hho.1/4642, intron 21 of 21) | 71400 | ENSG00000176749 | CDK5R1 |
| chr6 | 53946019 | 53946656 | -1,12 | 2,68 | -3,8 | 2,33E-05 | 0,00132 | Promoter (1-2kb) | -1680 | ENSG00000146147 | MLIP |
| chr15 | 26182591 | 26187249 | 0,29 | 5,04 | -4,75 | 2,37E-05 | 0,00134 | Intron (uc021sgd.1/100128714, intron 1 of 4) | 35084 | ENSG00000206187 | LINC02346 |
| chr3 | 141496886 | 141497325 | -1,18 | 2,51 | -3,69 | 2,38E-05 | 0,00134 | Promoter(<=1kb) | 0 | ENSG00000114124 | GRK7 |
| chr2 | 64416049 | 64418525 | 0,72 | 5,37 | -4,65 | 2,38E-05 | 0,00134 | Exon (uc021vio.1/150992, exon 5 of 6) | 14094 | ENSG00000230923 | LINC00309 |
| chr17 | 80090967 | 80092512 | -1,06 | 3,16 | -4,22 | 2,39E-05 | 0,00134 | Exon (uc002kdy.3/284001, exon 2 of 5) | 16977 | ENSG00000176155 | CCDC57 |
| chr18 | 10474749 | 10477396 | 0,09 | 5,01 | -4,92 | 2,41E-05 | 0,00135 | Intron (uc002kom.4/147495, intron 3 of 4) | 20124 | ENSG00000154856 | APCDD1 |
| chr10 | 75415041 | 75415911 | -1,02 | 2,97 | -3,99 | 2,41E-05 | 0,00135 | Promoter (<=1kb) | 0 | ENSG00000166317 | SYNPO2L |
| chr9 | 116064811 | 116065581 | -1,13 | 3,34 | -4,46 | 2,45E-05 | 0,00137 | Promoter (<=1kb) | 0 | ENSG00000165188 | RNF183 |
| chr16 | 4238249 | 4239397 | -0,48 | 3,94 | -4,42 | 2,49E-05 | 0,00138 | 3'UTR | 52684 | ENSG00000185739 | SRL |
| chr22 | 44062494 | 44063925 | -1,28 | 3,08 | -4,36 | 2,53E-05 | 0,0014 | Promoter (<=1kb) | 0 | ENSG00000186976 | EFCAB6 |
| chr2 | 152219798 | 152220686 | -1,13 | 2,95 | -4,08 | 2,57E-05 | 0,00141 | Exon (uc002txk.3/7130, exon 2 of 6) | -4162 | ENSG00000264684 | MIR4773-1 |
| chrX | 134303864 | 134305779 | 1,23 | 6,46 | -5,23 | 2,58E-05 | 0,00142 | Promoter (<=1kb) | 0 | ENSG00000169551 | CT55 |
| chr8 | 104800579 | 104802461 | 0,37 | 5,34 | -4,97 | 2,58E-05 | 0,00142 | Intron (uc003ylp.3/9699, intron 3 of 23) | -28955 | ENSG00000176406 | RIMS2 |
| chr18 | 12264057 | 12264947 | -0,75 | 3,44 | -4,19 | 2,64E-05 | 0,00144 | Exon (uc002kqt.4/1149, exon 3 of 5) | 9697 | ENSG00000176194 | CIDEA |
| chr11 | 20671795 | 20672643 | -1,28 | 2,92 | -4,2 | 2,64E-05 | 0,00144 | Intron (uc001mqd.3/9152, intron 14 of 15) | -18474 | ENSG00000165973 | NELL1 |
| chr6 | 65215422 | 65217244 | 0,58 | 5,37 | -4,79 | 2,66E-05 | 0,00145 | Intron (uc011dxt.1/346007, intron 6 of 23) | 314372 | ENSG00000188107 | EYS |
| chr16 | 84696589 | 84697993 | -0,03 | 4,54 | -4,57 | 2,68E-05 | 0,00145 | Exon (uc002fih.3/uc002fih.3, exon 1 of 1) | 14458 | ENSG00000135686 | KLHL36 |
| chr19 | 15083373 | 15084110 | -1,12 | 3,25 | -4,37 | 2,72E-05 | 0,00147 | Promoter (<=1kb) | 0 | ENSG00000105143 | SLC1A6 |
| chr5 | 149131398 | 149132712 | -0,63 | 3,6 | -4,23 | 2,77E-05 | 0,00149 | Intron (uc003lrb.2/133522, intron 1 of 1 0) | -18791 | ENSG00000155846 | PPARGC1B |
| chr22 | 49895753 | 49896896 | -1,28 | 2,76 | -4,03 | 2,78E-05 | 0,0015 | Intron (uc003biq.3/uc003biq.3, intron 1 of 4) | 154294 | ENSG00000188511 | C22orf34 |
| chr21 | 34185291 | 34186319 | -0,34 | 4,13 | -4,47 | 2,82E-05 | 0,00151 | Promoter (<=1kb) | 0 | ENSG00000205929 | C21orf62 |
| chr14 | 23815378 | 23816332 | -0,12 | 3,8 | -3,92 | 2,84E-05 | 0,00152 | 3'UTR | 5328 | ENSG00000092096 | SLC22A17 |
| chr2 | 162683663 | 162684592 | -1,12 | 3,26 | -4,38 | 2,85E-05 | 0,00152 | Intron (uc010fpa.1/57282, intron 6 of 14) | 202818 | ENSG00000144290 | SLC4 A10 |
| chr11 | 65794424 | 65795489 | 0,59 | 4,05 | -3,46 | 2,86E-05 | 0,00153 | Promoter (<=1kb) | -436 | ENSG00000175294 | CATSPER1 |
| chr10 | 117692858 | 117693627 | -1,28 | 2,55 | -3,82 | 2,89E-05 | 0,00154 | Intron (uc001lcg.3/26033, intron 28 of 28) | 338192 | ENSG00000151892 | GFRA1 |
| chr21 | 19693348 | 19694316 | -0,65 | 4,03 | -4,68 | 2,99E-05 | 0,00157 | Intron (uc002ykw.3/5651, intron 16 of 24) | 75156 | ENSG00000154645 | CHODL |
| chr11 | 2356524 | 2358155 | -1,28 | 3,03 | -4,31 | 2,99E-05 | 0,00157 | Intron (uc001lwe.3/uc0011we.3, intron 1 of 2) | 20258 | ENSG0000006420 1 | TSPAN3 2 |
| chr11 | 131451737 | 131452473 | -1,14 | 3,19 | -4,33 | 3,00E-05 | 0,00157 | Intron (uc001qgm.3/50863, intron 1 of 7) | 211366 | ENSG00000182667 | NTM |
| chr1 | 145176170 | 145178197 | -1,28 | 2,75 | -4,03 | 3,01E-05 | 0,00158 | Intron (uc021ott.2/100288142, intron 53 of 131) | -30914 | ENSG00000271425 | NBPF10 |
| chr11 | 65778960 | 65780164 | 0,02 | 4,45 | -4,43 | 3,03E-05 | 0,00159 | Promoter (<=1kb) | 0 | ENSG00000175315 | CST6 |
| chr5 | 139847466 | 139847992 | -1,07 | 2,55 | -3,62 | 3,08E-05 | 0,0016 | Intron (uc003lfo.3/54882, intron 10 of 10) | 8717 | ENSG00000131503 | ANKHD1 |
| chr5 | 87900547 | 87901160 | -1,28 | 2,58 | -3,86 | 3,09E-05 | 0,00161 | Intron (uc011cua.1/645323, intron 3 of 4) | 61597 | ENSG00000284447 | MIR9-2 |
| chr12 | 119613826 | 119614704 | -0,65 | 3,78 | -4,43 | 3,12E-05 | 0,00161 | Promoter (1-2kb) | -1891 | ENSG00000152137 | HSPB8 |
| chr2 | 47882444 | 47883366 | -0,53 | 3,36 | -3,9 | 3,12E-05 | 0,00161 | Intron (uc002rvz.4/4436, intron 16 of 18) | -84974 | ENSG00000184261 | KCNK12 |
| chr19 | 51893588 | 51894706 | -0,37 | 4,09 | -4,46 | 3,14E-05 | 0,00162 | Promoter (<=1kb) | 0 | ENSG00000262874 | C19orf84 |
| chr11 | 111789213 | 111790244 | -0,11 | 4,16 | -4,26 | 3,14E-05 | 0,00162 | 5'UTR | 5753 | ENSG00000170276 | HSPB2 |
| chr5 | 177869483 | 177870038 | -1,22 | 2,63 | -3,85 | 3,15E-05 | 0,00162 | Intron (uc021yiy.1/91522, intron 2 of 28) | 147518 | ENSG00000050767 | COL23A1 |
| chr2 | 214669085 | 214669728 | -1,28 | 2,54 | -3,81 | 3,15E-05 | 0,00162 | Intron (uc010fuz.3/79582, intron 8 of 13) | 519945 | ENSG00000144451 | SPAG16 |
| chr4 | 66438522 | 66439731 | -0,99 | 2,96 | -3,95 | 3,16E-05 | 0,00162 | Intron (uc003hcx.3/2044, intron 3 of 17) | 95475 | ENSG00000145242 | EPHA5 |
| chr18 | 48226821 | 48227494 | -1,28 | 2,98 | -4,26 | 3,20E-05 | 0,00164 | Intron (uc002lev.3/5596, intron 2 of 5) | 118578 | ENSG00000134042 | MRO |
| chr15 | 41987989 | 41988772 | -1,28 | 2,68 | -3,95 | 3,30E-05 | 0,00167 | Exon (uc001zog.1/23269, exon 3 of 9) | 4206 | ENSG00000207766 | MIR626 |
| chr2 | 198071829 | 198076122 | 0,07 | 5,17 | -5,1 | 3,33E-05 | 0,00168 | Intron (uc021vuj.1/91526, intron 1 of 27) | -9067 | ENSG00000065413 | ANKRD44 |
| chr6 | 124187142 | 124187968 | -1,28 | 2,95 | -4,23 | 3,34E-05 | 0,00168 | Intron (uc010kep.1/154215, intron 1 of 2) | 62073 | ENSG00000188580 | NKAIN2 |
| chr8 | 35390561 | 35392336 | 0,43 | 4,81 | -4,38 | 3,38E-05 | 0,00169 | Intron (uc003xjr.2/137970, intron 1 of 16) | -9630 | ENSG00000156687 | UNC5D |
| chr11 | 133032358 | 133033476 | -1,28 | 3,04 | -4,31 | 3,39E-05 | 0,00169 | Intron (uc001qgu.3/4978, intron 1 of 7) | -218695 | ENSG00000183715 | OPCML |
| chr12 | 63275601 | 63276801 | -0,67 | 3,38 | -4,04 | 3,40E-05 | 0,00169 | Intron (uc001srk.3/57460, intron 1 of 9) | 51864 | ENSG00000111110 | PPM1H |
| chr8 | 59952422 | 59953524 | -1,28 | 2,92 | -4,2 | 3,41E-05 | 0,0017 | Intron (uc003xtw.1/9760, intron 1 of 8) | 78243 | ENSG00000198846 | TOX |
| chr18 | 65295242 | 65296054 | -0,93 | 3,09 | -4,02 | 3,42E-05 | 0,0017 | Intron (uc021ulh.1/643542, intron 1 of 4) | -111275 | ENSG00000171451 | DSEL |
| chr7 | 23625746 | 23626287 | -1,28 | 2,89 | -4,16 | 3,47E-05 | 0,00172 | Promoter (<=1kb) | 0 | NA | CLK2P1 |
| chr6 | 34285001 | 34286206 | -1,28 | 2,98 | -4,26 | 3,49E-05 | 0,00172 | Intron (uc003ojl.3/11165, intron 2 of 4) | -68097 | ENSG00000186577 | SMIM29 |
| chr7 | 15718134 | 15719169 | -1,28 | 2,92 | -4,2 | 3,51E-05 | 0,00173 | Exon (uc003ste.3/uc003ste.3, exon 4 of 4) | 7139 | ENSG00000106511 | MEOX2 |
| chr18 | 3148288 | 3149082 | -1,18 | 2,94 | -4,13 | 3,53E-05 | 0,00174 | Intron (uc002klp.3/8736, intron 13 of 37) | 71024 | ENSG00000101605 | MYOM1 |
| chr17 | 79025406 | 79028841 | 1,49 | 6,17 | -4,68 | 3,55E-05 | 0,00174 | 5'UTR | 16459 | ENSG00000175866 | BAIAP2 |
| chrX | 91487408 | 91488126 | -1,21 | 2,79 | -4 | 3,58E-05 | 0,00175 | Intron (uc004efk.2/27328, intron 3 of 6) | 396948 | ENSG00000102290 | PCDH11X |
| chr3 | 76920201 | 76921199 | -1,28 | 3,05 | -4,32 | 3,58E-05 | 0,00175 | Intron (uc021xat.1/6092, intron 1 of 25) | -168095 | ENSG00000185008 | ROBO2 |
| chr2 | 206410611 | 206411306 | -1,22 | 2,61 | -3,83 | 3,60E-05 | 0,00176 | Intron (uc002vao.2/117583, intron 20 of 21) | -135918 | ENSG00000118257 | NRP2 |
| chr16 | 29719981 | 29720865 | -0,64 | 3,65 | -4,29 | 3,60E-05 | 0,00176 | Intron (uc010bzb.2/552900, intron 2 of 4) | 29540 | ENSG00000103485 | QPRT |
| chr1 | 52420026 | 52422087 | -0,02 | 4,77 | -4,79 | 3,63E-05 | 0,00177 | Intron (uc001cth.3/5865, intron 2 of 4) | 34349 | ENSG00000169213 | RAB3B |
| chr18 | 74134882 | 74135917 | -1,28 | 2,65 | -3,93 | 3,65E-05 | 0,00178 | Intron (uc021ulp.1/9658, intron 3 of 7) | -42623 | ENSG00000101493 | ZNF516 |
| chr17 | 19462836 | 19464268 | 0,16 | 4,48 | -4,32 | 3,66E-05 | 0,00178 | Promoter (1-2kb) | 1963 | ENSG00000239149 | SNORA59A |
| chr18 | 72008123 | 72009001 | -1,1 | 3,01 | -4,1 | 3,70E-05 | 0,00179 | Intron (uc002llj.3/644041, intron 8 of 13) | 25013 | ENSG00000206043 | C18orf63 |
| chr7 | 132160083 | 132160823 | -1,28 | 2,9 | -4,18 | 3,70E-05 | 0,00179 | Intron (uc003vra.4/91584, intron 3 of 31) | 100500 | ENSG00000221866 | PLXNA4 |
| chr10 | 108649177 | 108650013 | -1,28 | 2,59 | -3,86 | 3,74E-05 | 0,0018 | Intron (uc001kyl.3/114815, intron 2 of 26) | 274453 | ENSG00000108018 | SORCS1 |
| chr5 | 175592486 | 175595163 | 0,47 | 5,39 | -4,92 | 3,78E-05 | 0,00181 | Exon (uc003mdn.3/643201, exon 16 of 26) | 31135 | ENSG00000248596 | LOC643201 |
| chr12 | 18485077 | 18486215 | -1,28 | 2,93 | -4,2 | 3,91E-05 | 0,00186 | Intron (uc001rdt.3/5288, intron 8 of 31) | 50139 | ENSG00000139144 | PIK3C2G |
| chr7 | 89966956 | 89968514 | 0,48 | 5,31 | -4,83 | 3,92E-05 | 0,00186 | Promoter (1-2kb) | -1176 | ENSG00000105793 | GTPBP10 |
| chr3 | 145816870 | 145818203 | -0,87 | 3,6 | -4,47 | 3,94E-05 | 0,00187 | Intron (uc011bnm.1/5352, intron 7 of 19) | -12348 | ENSG00000152952 | PLOD2 |
| chr11 | 132206334 | 132207364 | -0,86 | 3,51 | -4,37 | 3,98E-05 | 0,00188 | 3'UTR | 425622 | ENSG00000182667 | NTM |
| chr12 | 15496334 | 15496977 | -1,28 | 2,57 | -3,85 | 3,98E-05 | 0,00188 | Intron (uc001rcu.2/5800, intron 1 of 8) | 21143 | ENSG00000151490 | PTPRO |
| chr8 | 70981486 | 70985125 | 1,06 | 6,29 | -5,23 | 4,03E-05 | 0,0019 | Promoter (<=1kb) | 0 | ENSG00000147596 | PRDM14 |
| chr8 | 41517623 | 41518202 | -1,28 | 2,47 | -3,75 | 4,04E-05 | 0,00191 | Promoter (<=1kb) | 0 | ENSG00000274705 | MIR486-1 |
| chr10 | 5540575 | 5541334 | -0,68 | 2,86 | -3,54 | 4,05E-05 | 0,00191 | Promoter (<=1kb) | 199 | ENSG00000178372 | CALML5 |
| chr6 | 25298463 | 25299504 | -0,98 | 3,12 | -4,09 | 4,08E-05 | 0,00192 | Intron (uc031smx.1/55604, intron 2 of 8) | 18807 | ENSG00000079691 | CARMIL1 |
| chr11 | 122545425 | 122546197 | -1,28 | 2,37 | -3,64 | 4,09E-05 | 0,00192 | Intron (uc001pyi.4/84959, intron 1 of 13) | 19027 | ENSG00000154127 | UBASH3B |
| chr3 | 185138430 | 185140143 | 0,27 | 4,59 | -4,33 | 4,13E-05 | 0,00193 | Intron (uc010hyf.3/9175, intron 2 of 14) | 57594 | ENSG00000073803 | MAP3K13 |
| chr1 1 | 74978234 | 74978978 | -0,79 | 2,94 | -3,73 | 4,18E-05 | 0,00195 | Exon (uc001owe.2/408, exon 15 of 16) | 26284 | ENSG00000261594 | TPBGL |
| chr5 | 94078888 | 94080235 | -0,74 | 3,52 | -4,26 | 4,19E-05 | 0,00195 | Intron (uc003kku.2/79772, intron 12 of 15) | 64784 | ENSG00000133302 | SLF1 |
| chr15 | 40186991 | 40187925 | -1,18 | 3 | -4,18 | 4,28E-05 | 0,00198 | Intron (uc001zkj.2/11245, intron 1 of 2) | 25168 | ENSG00000166073 | GPR176 |
| chr2 | 141546397 | 141547229 | -1,28 | 2,95 | -4,22 | 4,29E-05 | 0,00198 | Intron (uc002tvj.1/53353, intron 32 of 90) | 1342041 | ENSG00000168702 | LRP1B |
| chr22 | 50496080 | 50498500 | 1,74 | 6,29 | -4,55 | 4,31E-05 | 0,00199 | 3'UTR | 25281 | ENSG00000100427 | MLC1 |
| chr1 | 7700974 | 7701905 | -1,28 | 2,86 | -4,13 | 4,32E-05 | 0,00199 | Intron (uc001aoi.3/23261, intron 7 of 22) | -30149 | ENSG00000171735 | CAMTA1 |
| chr21 | 10521769 | 10522632 | -1,1 | 3,11 | -4,21 | 4,33E-05 | 0,00199 | Intron (uc011abv.2/uc011abv.2, intron 1 of 11) | 468288 | ENSG00000274391 | TPTE |
| chr18 | 72200944 | 72205201 | 2,78 | 7,3 | -4,52 | 4,34E-05 | 0,002 | Promoter(<=1kb) | 0 | ENSG00000150656 | CNDP1 |
| chr17 | 45352697 | 45353365 | -0,65 | 3,44 | -4,09 | 4,36E-05 | 0,002 | Intron (uc002ili.1/3690, intron 2 of 8) | 21489 | ENSG00000259207 | ITGB3 |
| chr10 | 13544266 | 13546117 | -0,29 | 4,23 | -4,52 | 4,41E-05 | 0,00202 | Promoter(<=1kb) | 0 | ENSG00000165626 | BEND7 |
| chr7 | 124519075 | 124519794 | -1,28 | 2,45 | -3,73 | 4,42E-05 | 0,00202 | Intron (uc011koe.2/25913, intron 6 of 18) | 50062 | ENSG00000128513 | POT1 |
| chr3 | 183768160 | 183769131 | -0,86 | 3,1 | -3,96 | 4,49E-05 | 0,00205 | Promoter (1-2kb) | -1704 | ENSG00000178084 | HTR3C |
| chr3 | 77564240 | 77565450 | -1,28 | 2,88 | -4,16 | 4,52E-05 | 0,00206 | Intron (uc021xat.1/6092, intron 5 of 25) | -72452 | ENSG00000185008 | ROBO2 |
| chr10 | 47665692 | 47666549 | -1,28 | 2,92 | -4,19 | 4,53E-05 | 0,00206 | Intron (uc031pux.1/uc031pux.1, intron 4 of 18) | -80371 | NA | NA |
| chr3 | 46661754 | 46662635 | -1,13 | 3,15 | -4,28 | 4,54E-05 | 0,00206 | Intron (uc003cpw.1/100132146, intron 2 of 2) | 7829 | ENSG00000283473 | FAM240A |
| chr16 | 5012372 | 5012985 | -0,99 | 2,6 | -3,59 | 4,59E-05 | 0,00207 | Intron (uc002cve.2/9717, intron 2 of 15) | 4054 | ENSG00000103184 | SEC14L5 |
| chr7 | 130021288 | 130021904 | -1,28 | 2,78 | -4,06 | 4,65E-05 | 0,00208 | Promoter (<=1kb) | 998 | ENSG00000091704 | CPA1 |
| chr20 | 23371885 | 23372724 | -1,28 | 2,48 | -3,76 | 4,65E-05 | 0,00208 | Intron (uc002wta.3/63908, intron 5 of 10) | 3110 | ENSG00000125814 | NAPB |
| chr3 | 179570814 | 179571497 | -1,22 | 2,08 | -3,31 | 4,66E-05 | 0,00208 | Intron (uc011bqd.2/51555, intron 8 of 14) | 120511 | ENSG00000114757 | PEX5L |
| chr15 | 26059382 | 26061230 | 0,53 | 4,86 | -4,34 | 4,67E-05 | 0,00209 | Intron (uc010ayu.3/57194, intron 1 of 20) | 32742 | ENSG00000266517 | MIR4715 |
| chr1 | 53555456 | 53556869 | 0,52 | 5,17 | -4,65 | 4,67E-05 | 0,00209 | Promoter(<=1kb) | 0 | ENSG00000162383 | SLC1A7 |
| chr19 | 50968985 | 50971400 | 0,4 | 5,21 | -4,82 | 4,73E-05 | 0,0021 | 3'UTR | -8257 | ENSG00000161671 | EMC10 |
| chr2 | 192138614 | 192140104 | -0,09 | 4,17 | -4,26 | 4,80E-05 | 0,00211 | Intron (uc002usq.2/4430, intron 1 of 28) | 10889 | ENSG00000128641 | MYO1B |
| chr7 | 51356711 | 51357925 | -0,6 | 3,66 | -4,26 | 4,84E-05 | 0,00212 | Intron (uc003tpr.4/23242, intron 1 of 12) | 26590 | ENSG00000106078 | COBL |
| chr1 1 | 12765637 | 12767900 | 0,82 | 4,98 | -4,16 | 4,86E-05 | 0,00213 | Intron (uc021qdx.1/7003, intron 2 of 12) | 69668 | ENSG00000187079 | TEAD1 |
| chr20 | 29980814 | 29981435 | -1,28 | 2,47 | -3,75 | 4,87E-05 | 0,00213 | Promoter (2-3kb) | -2362 | ENSG00000180483 | DEFB119 |
| chr7 | 123271688 | 123272446 | -1,28 | 2,45 | -3,72 | 4,90E-05 | 0,00214 | Intron (uc003vku.1/142685, intron 11 of 11) | 22576 | ENSG00000146809 | ASB15 |
| chr3 | 196527508 | 196528203 | -0,85 | 3,09 | -3,93 | 4,91E-05 | 0,00214 | Intron (uc003fwy.4/5062, intron 2 of 14) | 60780 | ENSG00000180370 | PAK2 |
| chr20 | 61839137 | 61840356 | -1,28 | 2,65 | -3,93 | 4,95E-05 | 0,00215 | Promoter (<=1kb) | 0 | ENSG00000149658 | YTHDF1 |
| chr2 | 10902625 | 10903468 | 0 | 4 | -4 | 4,98E-05 | 0,00216 | Intron (uc002ras.3/245973, intron 4 of 13) | 40850 | ENSG00000143882 | ATP6V1C2 |
| chr6 | 126935409 | 126936867 | -0,05 | 4,38 | -4,43 | 4,99E-05 | 0,00216 | Intron (uc003qaq.1/uc003qaq.1, intron 6 of 9) | 274156 | ENSG00000203760 | CENPW |
| chr3 | 38321330 | 38322318 | -1,12 | 3,07 | -4,19 | 5,02E-05 | 0,00217 | Promoter (1-2kb) | -1471 | ENSG00000144671 | SLC22A14 |
| chr2 | 59013277 | 59013913 | -1,02 | 2,79 | -3,81 | 5,03E-05 | 0,00218 | Intron (uc002rzy.3/400955, intron 2 of 13) | -138833 | ENSG00000233723 | LINC01122 |
| chr6 | 168975242 | 168976305 | -0,36 | 3,15 | -3,51 | 5,09E-05 | 0,00219 | Intron (uc003qwr.2/64094, intron 7 of 12) | -41617 | ENSG00000112562 | SMOC2 |
| chr2 | 121572767 | 121573531 | -1,28 | 2,84 | -4,12 | 5,10E-05 | 0,0022 | Intron (uc010yyu.1/2736, intron 2 of 3) | 17900 | ENSG00000074047 | GLI2 |
| chr7 | 116010931 | 116012243 | 1,43 | 5,7 | -4,27 | 5,13E-05 | 0,00221 | Intron (uc003vhv.2/858, intron 7 of 10) | 81025 | ENSG00000105971 | CAV2 |
| chr17 | 60855736 | 60857291 | -0,26 | 4,19 | -4,45 | 5,18E-05 | 0,00222 | Intron (uc010ddr.3/162333, intron 3 of 10) | 26720 | ENSG00000173838 | MARCH10 |
| chr5 | 41445838 | 41446574 | -0,74 | 3,65 | -4,39 | 5,18E-05 | 0,00222 | Intron (uc003jmm.1/345557, intron 1 of 2) | 64156 | ENSG00000182836 | PLCXD3 |
| chr2 | 99004500 | 99004972 | -1,28 | 2 | -3,28 | 5,29E-05 | 0,00225 | Intron (uc002svt.3/1261, intron 5 of 7) | 10749 | ENSG00000144191 | CNGA3 |
| chr15 | 44222807 | 44223464 | -1,28 | 2,77 | -4,05 | 5,35E-05 | 0,00227 | Intron (uc001ztk.1/84978, intron 2 of 15) | -20630 | ENSG00000171877 | FRMD5 |
| chr6 | 129635174 | 129636257 | -0,54 | 3,53 | -4,07 | 5,37E-05 | 0,00228 | Exon (uc003qbn.3/3908, exon 24 of 64) | 395113 | ENSG00000146376 | ARHGAP18 |
| chr9 | 135726475 | 135726836 | -1,09 | 1,9 | -2,99 | 5,37E-05 | 0,00228 | Intron (uc004cbu.1/158067, intron 5 of 12) | -26914 | ENSG00000165698 | SPACA9 |
| chr4 | 41081178 | 41082034 | -1,28 | 2,78 | -4,06 | 5,47E-05 | 0,0023 | Intron (uc003gvl.3/323, intron 3 of 17) | -64763 | ENSG00000163697 | APBB2 |
| chr11 | 127181086 | 127181772 | -1,28 | 2,78 | -4,06 | 5,47E-05 | 0,0023 | Intron (uc001qeh.3/uc001qeh.3, intron 1 of 2) | -307731 | ENSG00000149571 | KIRREL3 |
| chr6 | 35992091 | 35992546 | -1,28 | 2,35 | -3,63 | 5,51E-05 | 0,00232 | Promoter (<=1kb) | 0 | ENSG00000112053 | SLC26A8 |
| chr7 | 69534855 | 69535950 | -0,52 | 3,36 | -3,88 | 5,65E-05 | 0,00236 | Intron (uc003tvv.4/26053, intron 2 of 4) | 470950 | ENSG00000158321 | AUTS2 |
| chr14 | 33119152 | 33120153 | -1,28 | 2,6 | -3,87 | 5,65E-05 | 0,00236 | Intron (uc010aml.3/9472, intron 7 of 9) | -288306 | ENSG00000151322 | NPAS3 |
| chr6 | 24839044 | 24839987 | -0,71 | 3,32 | -4,03 | 5,80E-05 | 0,0024 | 3'UTR | 37596 | ENSG00000111913 | RIPOR2 |
| chr1 | 43751004 | 43751803 | -1,28 | 2,48 | -3,76 | 5,88E-05 | 0,00242 | Promoter (<=1kb) | 0 | ENSG00000253313 | Clorf210 |
| chr3 | 148220698 | 148221501 | -0,86 | 3,09 | -3,95 | 6,03E-05 | 0,00247 | Exon (uc021xfh.1/uc021xfh.1, exon 1 of 1) | -194157 | ENSG00000144891 | AGTR1 |
| chr2 | 214000523 | 214001093 | -1,28 | 2,47 | -3,74 | 6,03E-05 | 0,00247 | Intron (uc002vej.3/22807, intron 2 of 8) | 12275 | ENSG00000030419 | IKZF2 |
| chr4 | 184237043 | 184237817 | -0,64 | 3,75 | -4,39 | 6,05E-05 | 0,00248 | 3'UTR | 4110 | ENSG00000177300 | CLDN22 |
| chr16 | 81294890 | 81295579 | -0,94 | 2,88 | -3,81 | 6,06E-05 | 0,00248 | Intron (uc002fgm.1/53630, intron 3 of 3) | 22594 | ENSG00000135697 | BCO1 |
| chr18 | 14799788 | 14800968 | -1,28 | 2,01 | -3,28 | 6,15E-05 | 0,00251 | Intron (uc010xak.2/374860, intron 21 of 29) | -29197 | ENSG00000265499 | MIR3156-2 |
| chr2 | 99121582 | 99121964 | -1,12 | 1,94 | -3,06 | 6,17E-05 | 0,00251 | Intron (uc010yvj.1/3631, intron 1 of 25) | 60261 | ENSG00000040933 | INPP4A |
| chr17 | 79126072 | 79128101 | 0,6 | 5,04 | -4,44 | 6,17E-05 | 0,00251 | Intron (uc010dia.3/9625, intron 1 of 13) | -11206 | ENSG00000225180 | PVALEF |
| chr2 | 1291262 | 1291829 | -1,28 | 2,35 | -3,63 | 6,20E-05 | 0,00252 | Intron (uc002qwq.3/54221, intron 14 of 16) | -86166 | ENSG00000115705 | TPO |
| chr6 | 151668948 | 151669588 | -1,28 | 2,5 | -3,78 | 6,22E-05 | 0,00252 | Intron (uc011eep.2/9590, intron 3 of 4) | 6127 | ENSG00000131016 | AKAP12 |
| chr18 | 65531403 | 65532126 | -1,13 | 2,72 | -3,84 | 6,23E-05 | 0,00252 | Intron (uc021ulh.1/643542, intron 3 of 4) | -347436 | ENSG00000171451 | DSEL |
| chr2 | 157466293 | 157467156 | -1,28 | 2,35 | -3,63 | 6,24E-05 | 0,00252 | Intron (uc002tze.1/2820, intron 14 of 15) | 98985 | ENSG00000115159 | GPD2 |
| chr7 | 95117288 | 95118010 | -1,28 | 2,8 | -4,07 | 6,25E-05 | 0,00252 | Promoter (2-3kb) | 2075 | ENSG00000005981 | ASB4 |
| chr10 | 3558897 | 3559930 | 0,85 | 4,74 | -3,89 | 6,25E-05 | 0,00252 | Intron (uc001igy.1/uc001igy.1, intron 1 of 1) | 267543 | ENSG00000067082 | KLF6 |
| chr15 | 37348414 | 37349379 | -0,99 | 3,19 | -4,18 | 6,27E-05 | 0,00253 | Intron (uc001zjl.3/4212, intron 6 of 11) | 41183 | ENSG00000134138 | MEIS2 |
| chr14 | 23045942 | 23047171 | -1,17 | 2,85 | -4,02 | 6,30E-05 | 0,00253 | Intron (uc001wgl.2/1603, intron 1 of 2) | 10972 | ENSG00000129562 | DAD1 |
| chr10 | 119119893 | 119125155 | 0,33 | 5,32 | -4,98 | 6,39E-05 | 0,00256 | Intron (uc001lde.1/118987, intron 1 of 4) | 9782 | ENSG00000165650 | PDZD8 |
| chr1 | 48935473 | 48936419 | -1,28 | 2,42 | -3,7 | 6,40E-05 | 0,00256 | Promoter (1-2kb) | 1457 | ENSG00000132122 | SPATA6 |
| chr5 | 41786879 | 41787598 | -0,78 | 2,71 | -3,49 | 6,46E-05 | 0,00258 | Intron (uc011cpo.2/5019, intron 2 of 5) | 6739 | ENSG00000083720 | OXCT1 |
| chr11 | 77759676 | 77760791 | -1,28 | 2,47 | -3,74 | 6,46E-05 | 0,00258 | Intron (uc021qnr.1/100532726, intron 2 of 3) | -14116 | ENSG00000151365 | THRSP |
| chr18 | 21336131 | 21337607 | 0,17 | 4,61 | -4,44 | 6,50E-05 | 0,00259 | Intron (uc010dlv.2/3909, intron 6 of 12) | 66569 | ENSG00000053747 | LAMA3 |
| chr18 | 11023403 | 11025033 | -0,57 | 3,67 | -4,24 | 6,52E-05 | 0,00259 | Intron (uc002kos.2/63895, intron 2 of 51) | 123728 | ENSG00000154864 | PIEZO2 |
| chr11 | 61676254 | 61676921 | -1,13 | 2,71 | -3,84 | 6,55E-05 | 0,0026 | Intron (uc001nso.4/5866, intron 1 of 9) | 8160 | ENSG00000167994 | RAB3IL1 |
| chr17 | 59551689 | 59552582 | -0,99 | 3,07 | -4,06 | 6,55E-05 | 0,0026 | Intron (uc010ddo.3/9496, intron 5 of 8) | 17882 | ENSG00000121075 | TBX4 |
| chr15 | 25957787 | 25960091 | 0,61 | 5,02 | -4,41 | 6,63E-05 | 0,00263 | Exon (uc010ayu.3/57194, exon 10 of 21) | 133881 | ENSG00000266517 | MIR4715 |
| chr11 | 19532673 | 19534618 | 0,08 | 4,42 | -4,34 | 6,74E-05 | 0,00266 | Exon (uc021qet.1/399876, exon 4 of 4) | -3897 | ENSG00000255043 | NAV2-AS5 |
| chr2 | 51055271 | 51056880 | -1,28 | 2,42 | -3,7 | 6,76E-05 | 0,00266 | Intron (uc021vhg.1/9378, intron 5 of 22) | -171719 | ENSG00000179915 | NRXN1 |
| chr10 | 16869441 | 16870427 | -0,37 | 3,79 | -4,16 | 6,80E-05 | 0,00268 | Intron (uc001ioo.3/8029, intron 66 of 66) | -9988 | ENSG00000148484 | RSU1 |
| chr2 | 238773629 | 238775395 | -0,53 | 3,93 | -4,46 | 6,89E-05 | 0,00271 | Intron (uc002vxj.3/10267, intron 1 of 2) | 5442 | ENSG00000132329 | RAMP1 |
| chr11 | 5658042 | 5658790 | -0,73 | 3,47 | -4,2 | 6,90E-05 | 0,00271 | Intron (uc001mbf.3/445372, intron 10 of 13) | 4557 | ENSG00000258659 | TRIM34 |
| chr1 | 26101547 | 26102168 | -1,28 | 2,22 | -3,49 | 6,99E-05 | 0,00273 | Intron (uc001bkm.2/57134, intron 8 of 11) | -24499 | ENSG00000162430 | SELENON |
| chr1 | 75589550 | 75591690 | 0,24 | 4,75 | -4,51 | 7,18E-05 | 0,00279 | Promoter (2-3kb) | -2429 | ENSG00000162624 | LHX8 |
| chr2 | 121637011 | 121638261 | -0,46 | 3,77 | -4,23 | 7,40E-05 | 0,00285 | Intron (uc010yyu.1/2736, intron 2 of 3) | 82144 | ENSG00000074047 | GLI2 |
| chr22 | 26090260 | 26091135 | -1,28 | 2,57 | -3,85 | 7,46E-05 | 0,00287 | Exon (uc010gux.3/157, exon 13 of 16) | -46985 | ENSG00000133454 | MYO18B |
| chr9 | 3888251 | 3888933 | -1,13 | 2,86 | -3,99 | 7,48E-05 | 0,00287 | Intron (uc010mhf.1/169792, intron 4 of 7) | -9713 | ENSG00000237009 | GLIS3-AS1 |
| chr8 | 133772276 | 133773146 | -0,21 | 3,69 | -3,9 | 7,48E-05 | 0,00287 | Promoter (<=1kb) | 0 | ENSG00000165071 | TMEM71 |
| chr3 | 36531344 | 36532116 | -1,28 | 2,16 | -3,43 | 7,52E-05 | 0,00288 | Intron (uc010hgd.1/6769, intron 7 of 9) | 109247 | ENSG00000144681 | STAC |
| chr15 | 52795594 | 52800152 | 0,66 | 5,45 | -4,79 | 7,54E-05 | 0,00289 | Intron (uc002abx.3/4644, intron 1 of 39) | 21095 | ENSG00000197535 | MYO5A |
| chr1 | 223840475 | 223841111 | -1,28 | 2,29 | -3,57 | 7,60E-05 | 0,00291 | Intron (uc009xee.2/388743, intron 2 of 17) | 12325 | ENSG00000203697 | CAPN8 |
| chr17 | 7077404 | 7077991 | -1,14 | 2,46 | -3,61 | 7,68E-05 | 0,00293 | Promoter (2-3kb) | 2404 | ENSG00000141505 | ASGR1 |
| chr2 | 97643249 | 97643794 | -0,62 | 2,92 | -3,54 | 7,71E-05 | 0,00294 | Intron (uc002sxl.4/51252, intron 1 of 16) | 8507 | ENSG00000168754 | FAM178B |
| chr2 | 159134391 | 159135540 | -0,66 | 3,14 | -3,8 | 7,87E-05 | 0,00297 | Intron (uc002tzq.3/130940, intron 9 of 13) | 111229 | ENSG00000227480 | CCDC148-AS1 |
| chr8 | 42571371 | 42572356 | -0,16 | 3,86 | -4,02 | 7,94E-05 | 0,00299 | Intron (uc003xpi.1/1142, intron 3 of 5) | 18809 | ENSG00000147432 | CHRNB3 |
| chr19 | 51684346 | 51686943 | 1,39 | 6,03 | -4,63 | 7,94E-05 | 0,00299 | Exon (uc031rmj.1/uc031rmj.1, exon 1 of 5) | 13761 | ENSG00000171101 | SIGLEC17P |
| chr13 | 67729928 | 67731109 | -0,22 | 4,07 | -4,29 | 8,03E-05 | 0,00301 | Intron (uc001vik.3/5101, intron 2 of 4) | 73359 | ENSG00000184226 | PCDH9 |
| chr3 | 195448567 | 195449321 | -1,13 | 2,5 | -3,63 | 8,09E-05 | 0,00303 | Promoter(<=1kb) | 814 | ENSG00000176945 | MUC20 |
| chr11 | 78078661 | 78079389 | -1,28 | 2,27 | -3,54 | 8,10E-05 | 0,00303 | Intron (uc001ozh.3/9846, intron 1 of 9) | -25735 | ENSG00000033327 | GAB2 |
| chr2 | 181601581 | 181602303 | -1,28 | 2,58 | -3,86 | 8,14E-05 | 0,00304 | Intron (uc031rqe.1/uc031rqe.1, intron 3 of 6) | -242809 | ENSG00000170035 | UBE2E3 |
| chr12 | 87175365 | 87175991 | -1,14 | 2,39 | -3,53 | 8,16E-05 | 0,00304 | Intron (uc001taj.4/25834, intron 1 of 8) | 56690 | ENSG00000182050 | MGAT4C |
| chr15 | 24920706 | 24922168 | -0,55 | 3,37 | -3,92 | 8,30E-05 | 0,00308 | Promoter (<=1kb) | 165 | ENSG00000185823 | NPAP1 |
| chr4 | 68592621 | 68593210 | -1,12 | 2,37 | -3,49 | 8,37E-05 | 0,0031 | Intron (uc003hdl.4/550112, intron 2 of 7) | 25625 | ENSG00000248049 | UBA6-AS1 |
| chr4 | 14864339 | 14864998 | -1,13 | 2,72 | -3,86 | 8,45E-05 | 0,00312 | Intron (uc003gne.3/uc003gne.3, intron 1 of 6) | 131131 | ENSG00000247624 | CPEB2-DT |
| chr3 | 155240582 | 155240976 | -0,98 | 2,31 | -3,29 | 8,50E-05 | 0,00314 | Intron (uc021xgd.1/23007, intron 10 of 23) | 153129 | ENSG00000114805 | PLCH1 |
| chr14 | 89773769 | 89774603 | -1,28 | 2,59 | -3,86 | 8,62E-05 | 0,00316 | Intron (uc001xxn.4/1112, intron 3 of 5) | 108851 | ENSG00000053254 | FOXN3 |
| chr19 | 40367091 | 40368714 | -0,28 | 2,71 | -2,99 | 8,65E-05 | 0,00317 | Exon (uc002omp.4/8857, exon 28 of 36) | -30037 | ENSG00000105202 | FBL |
| chr8 | 10854852 | 10856154 | -0,87 | 3,25 | -4,11 | 8,68E-05 | 0,00317 | Intron (uc003wtk.1/286046, intron 1 of 2) | 36658 | ENSGO0000207600 | MIR598 |
| chr21 | 38879378 | 38880168 | -1,28 | 2,28 | -3,56 | 8,77E-05 | 0,0032 | Intron (uc002vwi.3/1859, intron 12 of 12) | 18291 | ENSG00000157540 | DYRK1A |
| chr17 | 19993049 | 19994031 | -1,28 | 2,31 | -3,59 | 8,79E-05 | 0,0032 | Promoter (2-3kb) | 2714 | ENSG00000128487 | SPECC1 |
| chr7 | 78489666 | 78490852 | -0,36 | 3,89 | -4,25 | 8,80E-05 | 0,0032 | Intron (uc003ugx.3/9863, intron 2 of 21) | -89028 | ENSG00000187391 | MAGI2 |
| chr10 | 71087326 | 71088619 | 0,28 | 4,62 | -4,34 | 8,86E-05 | 0,00322 | Intron (uc009xqc.2/3098, intron 4 of 5) | 8723 | ENSG00000156515 | HK1 |
| chr13 | 91826949 | 91828224 | 1,66 | 6,03 | -4,37 | 8,87E-05 | 0,00322 | Intron (uc031qms.1/100874150, intron 2 of 3) | 35728 | NA | LINC00379 |
| chr20 | 36879208 | 36879784 | -1,28 | 2,32 | -3,6 | 8,97E-05 | 0,00324 | Intron (uc002xhv.1/85449, intron 1 of 13) | 9390 | ENSG00000149633 | KIAA1755 |
| chr19 | 4524725 | 4525287 | -0,96 | 3,13 | -4,09 | 8,97E-05 | 0,00324 | Exon (uc002mas.3/440503, exon 7 of 8) | -7009 | ENSG00000167676 | PLIN4 |
| chr11 | 104833258 | 104833915 | -1,28 | 2,58 | -3,85 | 8,98E-05 | 0,00324 | Intron (uc001pid.1/837, intron 1 of 8) | 5410 | ENSG00000196954 | CASP4 |
| chr6 | 27064223 | 27065482 | 0,15 | 4,69 | -4,54 | 9,01E-05 | 0,00324 | Exon (uc021yow.1/uc021yow.1, exon 1 of 1) | 35093 | ENSG00000124635 | HIST1H2BJ |
| chr10 | 99178213 | 99179363 | 0,11 | 4,15 | -4,04 | 9,05E-05 | 0,00326 | Exon (uc001kng.1/uc001kng.1, exon 4 of 4) | -6664 | ENSG00000171314 | PGAM1 |
| chr14 | 22748878 | 22749706 | -1,28 | 2,59 | -3,86 | 9,26E-05 | 0,00331 | 5'UTR | 308437 | ENSG00000129562 | DAD1 |
| chr6 | 127044946 | 127045410 | -1,28 | 2,09 | -3,36 | 9,36E-05 | 0,00333 | Intron (uc003qaq.1/uc003qaq.1, intron 6 of 9) | 383693 | ENSG00000203760 | CENPW |
| chr14 | 25480699 | 25481298 | -1,28 | 2,12 | -3,4 | 9,40E-05 | 0,00334 | Intron (uc001wpu.3/29091, intron 1 of 5) | 37797 | ENSG00000168952 | STXBP6 |
| chr1 | 169522583 | 169523381 | -0,75 | 2,88 | -3,63 | 9,46E-05 | 0,00335 | Intron (uc001ggg.1/2153, intron 7 of 24) | 32388 | ENSG00000198734 | F5 |
| chr8 | 63594237 | 63595921 | 0,37 | 5,2 | -4,82 | 9,49E-05 | 0,00336 | Intron (uc010lyq.1/286183, intron 3 of 5) | -294499 | ENSG00000274956 | NKAIN3 -IT1 |
| chr8 | 135519420 | 135520662 | -0,93 | 3,16 | -4,09 | 9,66E-05 | 0,00341 | Intron (uc011ljj.2/57623, intron 5 of 5) | 57252 | ENSG00000066827 | ZFAT |
| chr7 | 16834290 | 16835697 | -0,49 | 3,67 | -4,15 | 9,68E-05 | 0,00341 | Exon (uc003str.3/10551, exon 7 of 8) | 9041 | ENSG00000106541 | AGR2 |
| chr2 | 132056786 | 132058686 | -1,1 | 2,46 | -3,56 | 9,76E-05 | 0,00344 | Exon (uc002tsq.1/440910, exon 6 of 6) | 19923 | ENSG00000231431 | LOC440910 |
| chr6 | 96525585 | 96526801 | -0,76 | 3,48 | -4,23 | 9,82E-05 | 0,00345 | Intron (uc003pop.4/10690, intron 1 of 2) | 61740 | ENSG00000172461 | FUT9 |
| chr7 | 139597272 | 139598194 | -1,28 | 2,53 | -3,81 | 9,82E-05 | 0,00345 | Intron (uc003vvh.3/6916, intron 7 of 16) | 68161 | ENSG00000059377 | TBXAS1 |
| chr10 | 90489955 | 90490465 | -1,13 | 2,35 | -3,48 | 9,91E-05 | 0,00348 | Intron (uc010qmv.2/643414, intron 2 of 8) | 5654 | ENSG00000204021 | LIPK |
| chr8 | 105565138 | 105566119 | -0,68 | 3,23 | -3,91 | 9,92E-05 | 0,00348 | Intron (uc003yma.3/29967, intron 1 of 6) | 35133 | ENSG00000147650 | LRP12 |
| chr15 | 51393645 | 51394219 | -1,02 | 2,44 | -3,47 | 9,98E-05 | 0,00349 | Intron (uc001zvy.3/388121, intron 1 of 2) | 3254 | ENSG00000183578 | TNFAIP8L3 |
| chr7 | 93422010 | 93423592 | -0,07 | 4,6 | -4,67 | 0,000101 | 0,00351 | Intron (uc003umx.1/2792, intron 1 of 3) | 75770 | ENSG00000265423 | MIR4652 |
| chr1 | 155441957 | 155442359 | -1,28 | 1,86 | -3,13 | 0,000101 | 0,00351 | Intron (uc001fkt.3/55870, intron 3 of 27) | 38986 | ENSG00000237872 | POU5F1P4 |
| chr16 | 21637787 | 21638600 | -1,28 | 2,34 | -3,61 | 0,000103 | 0,00356 | Intron (uc021tel.1/uc021tel.1, intron 2 of 3) | 25387 | ENSG00000140749 | IGSF6 |
| chr15 | 26038402 | 26039391 | -1,28 | 2,42 | -3,7 | 0,000103 | 0,00356 | Intron (uc010ayu.3/57194, intron 1 of 20) | 54581 | ENSG00000266517 | MIR4715 |
| chr10 | 5566789 | 5567222 | -1,28 | 2,1 | -3,37 | 0,000104 | 0,0036 | Promoter (<=1kb) | 0 | ENSG00000178363 | CALML3 |
| chr20 | 33539292 | 33540038 | -0,05 | 3,72 | -3,77 | 0,000104 | 0,00359 | Promoter (<=1kb) | 0 | ENSG00000100983 | GSS |
| chr13 | 53607848 | 53608661 | -1,28 | 2,56 | -3,83 | 0,000105 | 0,00364 | Exon (uc001vhk.2/10562, exon 2 of 4) | 4972 | ENSG00000102837 | OLFM4 |
| chr19 | 5746064 | 5746829 | -0,98 | 2,87 | -3,85 | 0,000106 | 0,00364 | 5'UTR | 25376 | ENSG00000174898 | CATSPERD |
| chr3 | 158831877 | 158832707 | -0,89 | 2,66 | -3,55 | 0,000106 | 0,00365 | Intron (uc003fco.3/654502, intron 1 of 3) | 44836 | ENSG00000214216 | IQCJ |
| chr2 | 105371666 | 105372385 | -0,79 | 3,36 | -4,15 | 0,000106 | 0,00365 | Promoter (<=1kb) | 0 | ENSG00000234177 | LINC01114 |
| chr4 | 114823404 | 114823887 | -1,28 | 2,11 | -3,39 | 0,000107 | 0,00367 | 3'UTR | 76361 | ENSG00000180801 | ARSJ |
| chr11 | 89400314 | 89400845 | -1,18 | 2,68 | -3,87 | 0,000107 | 0,00367 | Intron (uc001pda.3/219595, intron 2 of 13) | 7849 | ENSG00000134612 | FOLH1B |
| chr12 | 18868101 | 18869077 | -0,54 | 3,52 | -4,07 | 0,000107 | 0,00367 | Promoter (<=1kb) | -396 | ENSG00000139151 | PLCZ1 |
| chr3 | 10266811 | 10268952 | 0,09 | 4,51 | -4,42 | 0,000107 | 0,00367 | Exon (uc003bve.1/3656, exon 10 of 13) | -21225 | ENSG00000157014 | TATDN2 |
| chr2 | 1266407 | 1266946 | -1,17 | 2,41 | -3,57 | 0,000107 | 0,00366 | Intron (uc002qwq.3/54221, intron 13 of 16) | -111049 | ENSG00000115705 | TPO |
| chr19 | 4693775 | 4694225 | -1,28 | 2,48 | -3,76 | 0,000108 | 0,0037 | Intron (uc002mba.3/91039, intron 13 of 21) | 14481 | ENSG00000205790 | DPP9-AS1 |
| chr4 | 159492769 | 159494231 | 0,27 | 4,38 | -4,11 | 0,000108 | 0,00369 | 5' UTR | 49903 | ENSG00000171509 | RXFP1 |
| chr11 | 111262946 | 111264493 | -0,55 | 3,71 | -4,27 | 0,00011 | 0,00372 | Intron (uc001plh.1/uc001plh.1, intron 4 of 5) | -12789 | ENSG00000110777 | POU2AF1 |
| chr18 | 72741335 | 72741931 | -1,28 | 2,49 | -3,77 | 0,00011 | 0,00374 | Intron (uc00211w.2/55628, intron 7 of 7) | 175537 | ENSG00000180011 | ZADH2 |
| chr4 | 113300432 | 113301056 | -0,94 | 2,89 | -3,82 | 0,000111 | 0,00375 | Promoter (2-3kb) | -2498 | ENSG00000073331 | ALPK1 |
| chr19 | 17434316 | 17434941 | -1,28 | 2,42 | -3,7 | 0,000112 | 0,00378 | 3'UTR | 10697 | ENSG00000074855 | ANO8 |
| chr19 | 44308732 | 44309528 | 0,26 | 4,23 | -3,97 | 0,000112 | 0,00377 | Promoter (2-3kb) | -2119 | ENSG00000159871 | LYPD5 |
| chr13 | 67347128 | 67348115 | -0,98 | 2,96 | -3,94 | 0,000112 | 0,00378 | Intron (uc010aei.3/5101, intron 2 of 3) | -51186 | ENSG00000228842 | PCDH9-AS2 |
| chr10 | 64039691 | 64040615 | -1,28 | 2,45 | -3,73 | 0,000112 | 0,00377 | Intron (uc001jly.4/22891, intron 1 of 4) | -11225 | ENSG00000182010 | RTKN2 |
| chr3 | 121342845 | 121343724 | -1,28 | 2,47 | -3,74 | 0,000113 | 0,00379 | Intron (uc003eeg.2/51725, intron 3 of 3) | 30675 | ENSG00000163833 | FBXO40 |
| chr19 | 6476306 | 6476668 | -1,11 | 2,13 | -3,24 | 0,000115 | 0,00383 | Promoter (<=1kb) | -49 | ENSG00000205744 | DENND1C |
| chr3 | 79012737 | 79013210 | -0,99 | 2,12 | -3,11 | 0,000115 | 0,00383 | Intron (uc003dqb.2/6091, intron 1 of 28) | 55399 | ENSG00000169855 | ROBO1 |
| chr19 | 39018188 | 39019936 | 2,01 | 6,15 | -4,14 | 0,000115 | 0,00385 | Exon (uc002oit.3/6261, exon 73 of 106) | 15987 | ENSG00000196218 | RYR1 |
| chr16 | 29956007 | 29956756 | -0,44 | 2,77 | -3,21 | 0,000116 | 0,00387 | Intron (uc002duw.2/124446, intron 1 of 5) | 3801 | ENSG00000149932 | TMEM219 |
| chr5 | 55528924 | 55529572 | -1,28 | 2,32 | -3,6 | 0,000119 | 0,00394 | Promoter (<=1kb) | 0 | ENSG00000164512 | ANKRD55 |
| chr8 | 121517793 | 121518562 | -1,21 | 2,27 | -3,48 | 0,00012 | 0,00396 | Intron (uc003vpc.2/27085, intron 15 of 21) | -60146 | ENSG00000172172 | MRPL13 |
| chrX | 25024679 | 25025819 | -0,6 | 3,54 | -4,13 | 0,000121 | 0,00397 | Exon (uc004dbp.4/170302, exon 4 of 5) | 8246 | ENSG00000004848 | ARX |
| chrX | 135776225 | 135777109 | 0,07 | 3,81 | -3,74 | 0,000121 | 0,00397 | Intron (uc011mwd.2/9459, intron 9 of 21) | 43800 | ENSG00000102245 | CD40LG |
| chr11 | 46015266 | 46017383 | -0,22 | 3,98 | -4,2 | 0,000121 | 0,00397 | Intron (uc001ncb.4/51317, intron 5 of 17) | -22348 | ENSG00000135365 | PHF21A |
| chr6 | 42641538 | 42642987 | -0,63 | 3,59 | -4,22 | 0,000121 | 0,00397 | Promoter (<=1kb) | 563 | ENSG00000024048 | UBR2 |
| chrX | 49353680 | 49355633 | 0,19 | 4,36 | -4,18 | 0,000122 | 0,004 | Promoter (<=1kb) | 0 | ENSG00000205777 | GAGE1 |
| chr12 | 97047866 | 97048526 | -1,28 | 2,51 | -3,79 | 0,000122 | 0,004 | Intron (uc021rcc.1/uc021rcc.1, intron 3 of 26) | -252475 | ENSG00000139350 | NEDD1 |
| chr9 | 35406710 | 35407208 | -1,28 | 2,48 | -3,76 | 0,000123 | 0,00401 | Promoter (<=1kb) | 0 | ENSG00000179766 | ATP8B5P |
| chrl7 | 74673323 | 74674975 | 1,14 | 5,09 | -3,95 | 0,000123 | 0,00401 | 3' UTR | 11326 | ENSG00000182534 | MXRA7 |
| chr7 | 124426105 | 124426522 | -1,13 | 2,38 | -3,51 | 0,000124 | 0,00402 | Intron (uc003vlj.2/154872, intron 2 of 3) | 4342 | ENSG00000275356 | C7orf77 |
| chr17 | 38821065 | 38821580 | -1,28 | 2,45 | -3,73 | 0,000126 | 0,00409 | Promoter (<=1kb) | 0 | ENSG00000213424 | KRT222 |
| chr12 | 87233296 | 87233931 | -1,12 | 2,23 | -3,35 | 0,000126 | 0,00408 | Promoter (<=1kb) | -615 | ENSG00000182050 | MGAT4C |
| chr19 | 33278411 | 33278843 | -1,28 | 2,07 | -3,35 | 0,000126 | 0,00409 | Intron (uc002ntr.4/91646, intron 11 of 12) | 67732 | ENSG00000173809 | TDRD12 |
| chr5 | 16089989 | 16090765 | -1,07 | 2,76 | -3,83 | 0,000127 | 0,00411 | Promoter (<=1kb) | 461 | ENSG00000183654 | MARCH11 |
| chr15 | 93270386 | 93270960 | -1,28 | 2,49 | -3,77 | 0,000128 | 0,00412 | Intron (uc002bs1.4/400451, intron 1 of 3) | 6344 | ENSG00000185442 | FAM174B |
| chr13 | 67768798 | 67769760 | -0,37 | 3,65 | -4,02 | 0,000128 | 0,00413 | Intron (uc001vik.3/5101, intron 2 of 4) | 34708 | ENSG00000184226 | PCDH9 |
| chr1 | 7427260 | 7427990 | -1,22 | 2,51 | -3,73 | 0,000129 | 0,00415 | Intron (uc001aoi.3/23261, intron 5 of 22) | -304064 | ENSG00000171735 | CAMTA1 |
| chr1 | 52144243 | 52144942 | -1,28 | 2,23 | -3,5 | 0,000129 | 0,00414 | Intron (uc001css.4/114883, intron 6 of 25) | -50544 | ENSG00000117859 | OSBPL9 |
| chr16 | 23960438 | 23960843 | -1,28 | 2,12 | -3,4 | 0,000129 | 0,00414 | Intron (uc002dmd.3/5579, intron 2 of 16) | 113138 | ENSG00000166501 | PRKCB |
| chr12 | 6648839 | 6650747 | 0,18 | 4,88 | -4,7 | 0,00013 | 0,00417 | 3' UTR | 4430 | ENSG00000111640 | GAPDH |
| chr2 | 17818301 | 17818954 | -1,28 | 2,41 | -3,69 | 0,000131 | 0,00417 | Intron (uc002rcm.3/7447, intron 2 of 3) | 96494 | ENSG00000163032 | VSNL1 |
| chr5 | 43559699 | 43561585 | 0,2 | 4,61 | -4,41 | 0,000132 | 0,0042 | Promoter (2-3kb) | -2178 | ENSG00000172239 | PAIP1 |
| chr6 | 154507323 | 154508199 | -1,21 | 2,49 | -3,7 | 0,000133 | 0,00422 | Intron (uc003qpt.1/4988, intron 3 of 3) | 26265 | ENSG00000074706 | IPCEF1 |
| chr8 | 133334029 | 133334730 | -1,28 | 2,4 | -3,68 | 0,000133 | 0,00424 | Intron (uc003yti.3/3786, intron 1 of 14) | 124779 | ENSG00000184156 | KCNQ3 |
| chrl7 | 56956668 | 56957388 | -0,46 | 3,08 | -3,54 | 0,000137 | 0,00431 | Intron (uc002iwx.4/22843, intron 1 of 6) | 123438 | ENSG00000175175 | PPM1E |
| chr7 | 33389801 | 33390577 | -1,28 | 2,51 | -3,79 | 0,000138 | 0,00433 | Promoter (<=1kb) | 0 | ENSG00000122507 | BBS9 |
| chr3 | 121920153 | 121921410 | -1,28 | 2,45 | -3,73 | 0,000138 | 0,00433 | Intron (uc003eev.4/846, intron 1 of 6) | 16972 | ENSG00000036828 | CASR |
| chrl7 | 71423855 | 71424623 | -0,87 | 3,02 | -3,88 | 0,000139 | 0,00435 | Intron (uc010dfm.3/54549, intron 12 of 44) | 9338 | ENSG00000069188 | SDK2 |
| chr7 | 127643462 | 127644471 | -1,28 | 2,37 | -3,64 | 0,000139 | 0,00435 | Intron (uc003vmi.3/27044, intron 16 of 23) | 5900 | ENSG00000279078 | SND1-IT1 |
| chr6 | 147109369 | 147110063 | -1,28 | 2,48 | -3,76 | 0,00014 | 0,00437 | Exon (uc003qlp.3/79747, exon 29 of 32) | 14897 | NA | KATNBL1P6 |
| chr14 | 104417801 | 104418479 | -1,28 | 2,43 | -3,71 | 0,000141 | 0,00439 | Intron (uc001yom.4/122402, intron 1 of 35) | -9156 | ENSG00000227729 | RD3L |
| chr5 | 76144873 | 76148431 | 1,03 | 6,04 | -5,01 | 0,000141 | 0,00439 | Promoter (<=1kb) | 0 | ENSG00000171643 | S100Z |
| chr14 | 92574723 | 92575449 | -1,12 | 2,66 | -3,78 | 0,000144 | 0,00445 | Promoter (1-2kb) | -1758 | ENSG00000066427 | ATXN3 |
| chr1 1 | 118893750 | 118894691 | -0,62 | 3,31 | -3,93 | 0,000145 | 0,00446 | 3' UTR | 4096 | NA | NA |
| chrl6 | 10880715 | 10881373 | -0,46 | 3,09 | -3,55 | 0,000147 | 0,00453 | Intron (uc002dad.3/780776, intron 2 of 8) | -11762 | ENSG00000166676 | TVP23A |
| chr4 | 176737560 | 176738418 | -1,12 | 2,57 | -3,7 | 0,000148 | 0,00455 | Intron (uc003iug.4/2823, intron 1 of 7) | -3295 | ENSG00000150625 | GPM6A |
| chr17 | 73732978 | 73733872 | -0,18 | 3,69 | -3,87 | 0,000148 | 0,00456 | 3' UTR | 5085 | ENSG00000132470 | ITGB4 |
| chr4 | 186187994 | 186188698 | -1,28 | 2,33 | -3,61 | 0,000148 | 0,00455 | Exon (uc003ixh.3/83891, exon 5 of 19) | -28283 | ENSG00000109762 | SNX25 |
| chr11 | 126628408 | 126628982 | -1,28 | 2,39 | -3,66 | 0,000149 | 0,00457 | Intron (uc001qea.3/84623, intron 1 of 16) | -181660 | ENSG00000254960 | KIRREL3 -AS2 |
| chr4 | 110308188 | 110308687 | -1,13 | 2,16 | -3,29 | 0,000149 | 0,00457 | Intron (uc003hzi.1/uc003hzi.1, intron 5 of 7) | -46284 | ENSG00000138802 | SEC24B |
| chr4 | 77363481 | 77364084 | -1,28 | 2,23 | -3,5 | 0,000149 | 0,00457 | Intron (uc011cbx.2/57619, intron 1 of 10) | 7228 | ENSG00000138771 | SHROOM3 |
| chr6 | 30984083 | 30985118 | 0,77 | 4,87 | -4,1 | 0,000152 | 0,00464 | Intron (uc021yug.1/100507679, intron 2 of 4) | 10354 | ENSG00000261272 | MUC22 |
| chr5 | 32735008 | 32735459 | -1,08 | 2,37 | -3,44 | 0,000152 | 0,00464 | Intron (uc010iuo.3/4883, intron 2 of 5) | 23570 | ENSG00000113389 | NPR3 |
| chr1 | 161167954 | 161168474 | -1,02 | 2,86 | -3,88 | 0,000153 | 0,00465 | Promoter (<=1kb) | 371 | ENSG00000158859 | ADAMTS4 |
| chr7 | 93406998 | 93407991 | -0,14 | 3,96 | -4,1 | 0,000154 | 0,00466 | Intron (uc003umx.1/2792, intron 1 of 3) | 60758 | ENSG00000265423 | MIR4652 |
| chr2 | 172174874 | 172175758 | -1,28 | 2,12 | -3,39 | 0,000154 | 0,00466 | 3' UTR | -87050 | ENSG00000198586 | TLK1 |
| chr3 | 9952937 | 9954192 | -0,58 | 3,61 | -4,19 | 0,000155 | 0,00469 | Exon (uc003btu.3/132014, exon 12 of 17) | -4566 | ENSG00000163702 | IL17RC |
| chr1 | 245939877 | 245940537 | -1,28 | 2,38 | -3,66 | 0,000155 | 0,00469 | Intron (uc001ibj.3/64754, intron 5 of 6) | 130483 | ENSG00000162849 | KIF26B |
| chr17 | 74440641 | 74441755 | -0,36 | 3,63 | -3,99 | 0,000157 | 0,00474 | Intron (uc002jrm.4/63893, intron 1 of 17) | 7533 | ENSG00000175931 | UBE2O |
| chr7 | 69304802 | 69305585 | -1,07 | 2,72 | -3,79 | 0,000158 | 0,00476 | Intron (uc003tvv.4/26053, intron 1 of 4) | 240897 | ENSG00000158321 | AUTS2 |
| chr12 | 93655483 | 93656404 | -0,71 | 3,34 | -4,05 | 0,000158 | 0,00476 | Intron (uc021rbu.1/643339, intron 2 of 4) | 115108 | NA | LOC643339 |
| chr7 | 37770712 | 37771919 | 0,13 | 4,75 | -4,62 | 0,000159 | 0,00478 | Intron (uc003tfl.3/uc003tfl.3, intron 2 of 3) | -8077 | ENSG00000187037 | GPR141 |
| chr8 | 95987657 | 95988442 | -1,28 | 2,33 | -3,61 | 0,000161 | 0,00482 | Intron (uc003yhi.3/137682, intron 1 of 9) | 17421 | ENSG00000156170 | NDUFAF6 |
| chr6 | 114344140 | 114344812 | -0,35 | 3,26 | -3,61 | 0,000162 | 0,00485 | Intron (uc003pwf.3/uc003pwf.3, intron 4 of 9) | 39229 | ENSG00000249853 | HS3 ST5 |
| chr22 | 36018239 | 36018853 | -1,28 | 2,33 | -3,61 | 0,000164 | 0,00489 | Promoter (<=1kb) | 548 | ENSG00000198125 | MB |
| chr20 | 60000155 | 60000773 | -1,1 | 2,67 | -3,76 | 0,000165 | 0,00491 | Intron (uc002vbn.2/1002, intron 2 of 15) | -73704 | ENSG00000179242 | CDH4 |
| chr18 | 74271051 | 74271717 | -1,16 | 2,5 | -3,66 | 0,000165 | 0,00492 | Exon (uc0021mg.3/284276, exon 3 of 3) | 30439 | ENSG00000266256 | LINC00908 |
| chr21 | 46072870 | 46073504 | -1,28 | 2,34 | -3,62 | 0,000166 | 0,00492 | Promoter (1-2kb) | 1072 | ENSG00000212933 | KRTAP12-4 |
| chr18 | 48329878 | 48330663 | -1,13 | 2,49 | -3,62 | 0,000167 | 0,00494 | Intron (uc010xdn.2/83876, intron 4 of 5) | 15409 | ENSG00000134042 | MRO |
| chr3 | 47857616 | 47858487 | -0,98 | 2,41 | -3,39 | 0,000168 | 0,00497 | Exon (uc003crr.4/22907, exon 4 of 4) | -7989 | ENSG00000132153 | DHX30 |
| chr9 | 132665106 | 132665895 | -0,32 | 3,19 | -3,5 | 0,000169 | 0,00499 | Exon (uc011mbu.1/23048, exon 4 of 8) | 15694 | ENSG00000187239 | FNBP1 |
| chr7 | 24760964 | 24761499 | -0,49 | 2,97 | -3,46 | 0,000169 | 0,00501 | Intron (uc010kus.1/1687, intron 3 of 9) | 35584 | ENSG00000105928 | GSDME |
| chr11 | 99490258 | 99491063 | -0,55 | 3,09 | -3,65 | 0,00017 | 0,00501 | Intron (uc009ywv.2/53942, intron 3 of 15) | 63382 | ENSG00000149972 | CNTN5 |
| chr3 | 15325428 | 15325908 | -1,28 | 1,89 | -3,17 | 0,00017 | 0,00501 | Intron (uc003bzp.2/9467, intron 3 of 8) | 29737 | ENSG00000224660 | SH3BP5-AS1 |
| chr2 | 74497484 | 74498127 | -0,98 | 2,73 | -3,71 | 0,00017 | 0,00501 | Intron (uc002skl.3/57835, intron 13 of 38) | 44025 | ENSG00000188687 | SLC4A5 |
| chr15 | 54459307 | 54460026 | -0,55 | 2,97 | -3,52 | 0,00017 | 0,00501 | Intron (uc021smr.1/440279, intron 3 of 30) | -96317 | ENSG00000137766 | UNC13C |
| chrl | 10707708 | 10708376 | -1,1 | 2,18 | -3,28 | 0,000172 | 0,00507 | 3'UTR | 13079 | ENSG00000130940 | CASZ1 |
| chr6 | 10594063 | 10594815 | -1,12 | 2,62 | -3,74 | 0,000172 | 0,00505 | Intron (uc010jol.3/2651, intron 3 of 4) | 8070 | ENSG00000111846 | GCNT2 |
| chr2 | 58240598 | 58241473 | -1,28 | 2,34 | -3,62 | 0,000172 | 0,00505 | Intron (uc002rzo.2/7444, intron 1 of 15) | -32304 | ENSG00000028116 | VRK2 |
| chrX | 138637256 | 138637885 | -1,28 | 2,05 | -3,33 | 0,000173 | 0,00509 | Intron (uc004fas.1/2158, intron 6 of 7) | 24361 | ENSG00000101981 | F9 |
| chr9 | 15844496 | 15845094 | -1,13 | 2,62 | -3,75 | 0,000174 | 0,0051 | Intron (uc003zmd.3/203238, intron 21 of 25) | 100225 | ENSG00000164989 | CCDC171 |
| chr18 | 33624861 | 33625276 | -1,28 | 1,96 | -3,23 | 0,000176 | 0,00513 | Intron (uc002kze.1/55197, intron 1 of 8) | 22097 | ENSG00000141425 | RPRD1A |
| chr17 | 58585082 | 58586241 | -0,19 | 3,56 | -3,75 | 0,000177 | 0,00516 | Intron (uc002ivs.1/10513, intron 1 of 12) | 17339 | ENSG00000062725 | APPBP2 |
| chr17 | 37295035 | 37295551 | -1,28 | 2,3 | -3,57 | 0,000178 | 0,00518 | Intron (uc002hrg.2/57125, intron 2 of 13) | 12351 | ENSG00000161381 | PLXDC1 |
| chr11 | 70489553 | 70490900 | -0,31 | 3,42 | -3,72 | 0,000178 | 0,00519 | Intron (uc009ysn.1/uc009ysn.1, intron 1 of 1) | 12354 | ENSG0000226627 | SHANK2-AS1 |
| chr18 | 65270634 | 65271797 | -0,16 | 3,56 | -3,72 | 0,000182 | 0,00527 | Intron (uc021ulh.1/643542, intron 1 of 4) | -86667 | ENSG00000171451 | DSEL |
| chr7 | 38508309 | 38508985 | -1,13 | 2,17 | -3,31 | 0,000185 | 0,00534 | Intron (uc003tgu.3/273, intron 7 of 20) | -4609 | ENSG00000078053 | AMPH |
| chr6 | 123861106 | 123861659 | -1,28 | 1,98 | -3,25 | 0,000186 | 0,00536 | Intron (uc003pzj.2/10345, intron 4 of 40) | 96579 | ENSG00000186439 | TRDN |
| chr19 | 49876692 | 49877586 | -1,13 | 2,55 | -3,68 | 0,000188 | 0,0054 | Intron (uc021uxk.1/27120, intron 4 of 4) | 9650 | ENSG00000104901 | DKKL1 |
| chr12 | 52712700 | 52713117 | -0,95 | 2,35 | -3,3 | 0,000188 | 0,0054 | Promoter (2-3kb) | 2065 | ENSG00000170523 | KRT83 |
| chr6 | 25489924 | 25490474 | -1,1 | 2,18 | -3,27 | 0,000189 | 0,00542 | Intron (uc010jpy.3/55604, intron 13 of 36) | -37132 | ENSG00000079691 | CARMIL1 |
| chr4 | 141265559 | 141266532 | -0,43 | 3,47 | -3,9 | 0,000189 | 0,00542 | Promoter (<=1kb) | 944 | ENSG00000153130 | SCOC |
| chrX | 55100995 | 55102776 | 0,79 | 5,08 | -4,3 | 0,00019 | 0,00543 | Promoter (<=1kb) | 0 | ENSG00000238269 | PAGE2B |
| chr15 | 75626027 | 75626609 | -0,58 | 2,96 | -3,54 | 0,000191 | 0,00546 | Promoter (1-2kb) | -1765 | ENSG00000140365 | COMMD4 |
| chr3 | 77595674 | 77596347 | -1,28 | 2,32 | -3,6 | 0,000191 | 0,00546 | Intron (uc021xat.1/6092, intron 7 of 25) | -41555 | ENSG00000185008 | ROBO2 |
| chr10 | 56302754 | 56303344 | -1,1 | 1,82 | -2,92 | 0,000195 | 0,00556 | Intron (uc010qhq.2/65217, intron 2 of 34) | 120706 | ENSG00000150275 | PCDH15 |
| chr6 | 167033815 | 167034758 | -0,81 | 3,11 | -3,92 | 0,000196 | 0,00557 | Intron (uc003qvb.1/6196, intron 1 of 20) | 5968 | ENSG00000071242 | RPS6KA2 |
| chr18 | 77764375 | 77764841 | -1,28 | 1,6 | -2,88 | 0,000196 | 0,00557 | Intron (uc010drg.3/10907, intron 1 of 2) | -15843 | ENSG00000141759 | TXNL4A |
| chr11 | 84335596 | 84336307 | -1,28 | 2,26 | -3,53 | 0,000197 | 0,00559 | Intron (uc001paj.2/1740, intron 1 of 22) | 298158 | ENSG00000150672 | DLG2 |
| chr11 | 34675200 | 34675689 | -1,14 | 2,52 | -3,66 | 0,000197 | 0,00558 | Intron (uc001mvr.2/26298, intron 6 of 8) | 21189 | ENSG00000135373 | EHF |
| chr13 | 113741820 | 113744605 | 1,64 | 6,09 | -4,45 | 0,000197 | 0,00559 | 3' UTR | -15497 | ENSG00000057593 | F7 |
| chr12 | 131477644 | 131480143 | 2,87 | 7,23 | -4,36 | 0,000198 | 0,0056 | Intron (uc001uit.4/283383, intron 8 of 24) | 39192 | ENSG00000111452 | ADGRD1 |
| chr7 | 27154618 | 27155746 | 0,7 | 4,59 | -3,9 | 0,000202 | 0,00571 | Intron (uc011jzl.2/3200, intron 1 of 2) | 3468 | ENSG00000105997 | HOXA3 |
| chr17 | 17409828 | 17410785 | 0,26 | 4,34 | -4,09 | 0,000202 | 0,0057 | Exon (uc010vwx.3/10400, exon 6 of 8) | -10119 | ENSG00000108551 | RASD1 |
| chr2 | 102639920 | 102641003 | 0,12 | 3,97 | -3,85 | 0,000204 | 0,00574 | Exon (uc002tbo.2/7850, exon 7 of 7) | 14943 | ENSG00000115590 | IL1R2 |
| chr2 | 242127512 | 242128566 | 1,06 | 4,98 | -3,92 | 0,000206 | 0,00578 | Promoter (<=1kb) | 0 | ENSG00000146205 | ANO7 |
| chrX | 25033166 | 25034601 | 0,1 | 4,6 | -4,5 | 0,000206 | 0,00579 | Promoter (<=1kb) | 0 | ENSG00000004848 | ARX |
| chr1 | 51376133 | 51376720 | -0,71 | 2,82 | -3,52 | 0,000206 | 0,00578 | Intron (uc009vyw.1/11124, intron 1 of 15) | 48805 | ENSG00000185104 | FAF1 |
| chr8 | 913513 | 917808 | 1,47 | 6,31 | -4,85 | 0,000206 | 0,00579 | Intron (uc003wpj.2/619343, intron 3 of 3) | 80315 | NA | NA |
| chr2 | 148927209 | 148927919 | -1,28 | 2,21 | -3,49 | 0,000206 | 0,00579 | Intron (uc002twm.4/55777, intron 2 of 14) | -148036 | ENSG00000115947 | ORC4 |
| chr15 | 76004725 | 76005786 | 1,53 | 5,58 | -4,05 | 0,000207 | 0,00581 | Promoter (<=1kb) | 0 | ENSG00000173546 | CSPG4 |
| chr3 | 177609335 | 177610624 | -0,29 | 3,6 | -3,89 | 0,000208 | 0,00582 | Intron (uc003fiz.1/uc003fiz.1, intron 1 of 2) | 449626 | NA | LINC00578 |
| chr14 | 22739479 | 22740467 | -0,87 | 2,96 | -3,83 | 0,000209 | 0,00584 | 5' UTR | 317676 | ENSG00000129562 | DAD1 |
| chr21 | 42163271 | 42164072 | -1,28 | 2,23 | -3,51 | 0,000209 | 0,00583 | Intron (uc002yyq.1/1826, intron 1 of 32) | 54967 | ENSG00000171587 | DSCAM |
| chr2 | 17808392 | 17808971 | -1,28 | 2,01 | -3,29 | 0,000211 | 0,00587 | Intron (uc002rcm.3/7447, intron 2 of 3) | 86585 | ENSG00000163032 | VSNL1 |
| chr12 | 131678627 | 131679489 | 0,05 | 3,89 | -3,84 | 0,000212 | 0,00589 | Intron (uc001uiw.3/116437, intron 1 of 4) | 29071 | ENSG00000204603 | LINC01257 |
| chr21 | 37183411 | 37183883 | -1,28 | 2,24 | -3,52 | 0,000212 | 0,0059 | Intron (uc002yut.1/861, intron 4 of 10) | 90398 | ENSG00000211590 | MIR802 |
| chr6 | 65829726 | 65830358 | -1,28 | 2,06 | -3,33 | 0,000214 | 0,00593 | Intron (uc011dxu.1/346007, intron 12 of 42) | -180953 | NA | LOC441155 |
| chr15 | 52779204 | 52780568 | -0,19 | 3,91 | -4,1 | 0,000216 | 0,00597 | Intron (uc002abx.3/4644, intron 1 of 39) | 40679 | ENSG00000197535 | MYO5A |
| chr8 | 17767391 | 17768036 | -1,28 | 2,3 | -3,58 | 0,000218 | 0,00601 | Promoter (<=1kb) | 0 | ENSG00000104760 | FGL1 |
| chr12 | 18551687 | 18552724 | -0,54 | 3,3 | -3,85 | 0,000221 | 0,00607 | Exon (uc001rdt.3/5288, exon 15 of 32) | 116749 | ENSG00000139144 | PIK3 C2G |
| chr1 | 230818546 | 230819505 | -0,93 | 2,82 | -3,75 | 0,000223 | 0,0061 | Exon (uc001htw.3/22796, exon 11 of 18) | 4170 | ENSG00000135775 | COG2 |
| chr19 | 50875899 | 50877191 | 0,37 | 3,63 | -3,26 | 0,000223 | 0,00609 | Promoter (2-3kb) | -2301 | ENSG00000131408 | NR1H2 |
| chr15 | 49842790 | 49843636 | -0,75 | 3,15 | -3,9 | 0,000224 | 0,00611 | Intron (uc001zxl.2/196951, intron 9 of 15) | 69415 | ENSG00000166262 | FAM227B |
| chr3 | 133464772 | 133465521 | -0,02 | 3,75 | -3,77 | 0,000224 | 0,00612 | Promoter (<=1kb) | 0 | ENSG00000091513 | TF |
| chr15 | 54553321 | 54554497 | 0,39 | 4,28 | -3,88 | 0,000224 | 0,00612 | Promoter (1-2kb) | -1846 | ENSG00000137766 | UNC13C |
| chr18 | 67265981 | 67291786 | 3,44 | 9,46 | -6,02 | 0,000225 | 0,00614 | Exon (uc002lkl.3/220164, exon 3 of 8) | 197697 | ENSG00000206052 | DOK6 |
| chr16 | 67318985 | 67319471 | -1,28 | 2,12 | -3,39 | 0,000225 | 0,00613 | Exon (uc002eso.4/25894, exon 12 of 21) | 5493 | ENSG00000196155 | PLEKHG4 |
| chr8 | 19246295 | 19247072 | -0,42 | 2,98 | -3,39 | 0,000225 | 0,00614 | Intron (uc003wzb.3/63898, intron 8 of 9) | 74808 | ENSG00000104611 | SH2D4A |
| chr8 | 73146679 | 73147055 | -1,28 | 1,78 | -3,06 | 0,000226 | 0,00614 | Intron (uc022avu.1/392232, intron 6 of 20) | 16814 | NA | LOC392232 |
| chr10 | 14157799 | 14158491 | -1,28 | 1,92 | -3,2 | 0,000227 | 0,00617 | Intron (uc001ims.3/55691, intron 2 of 24) | -107639 | ENSG00000151474 | FRMD4A |
| chr4 | 122842273 | 122843155 | -0,73 | 2,89 | -3,62 | 0,000228 | 0,00619 | Intron (uc010inr.2/7222, intron 2 of 9) | 11042 | ENSG00000138741 | TRPC3 |
| chr3 | 134742953 | 134743706 | -1,28 | 2,2 | -3,47 | 0,000229 | 0,0062 | Intron (uc010htz.2/2047, intron 4 of 5) | -42668 | ENSG00000154928 | EPHB1 |
| chr1 | 70474657 | 70476434 | 0,97 | 5,34 | -4,37 | 0,000229 | 0,0062 | Promoter (<=1kb) | -765 | ENSG00000226627 | SHANK2-AS1 |
| chr5 | 142511194 | 142512088 | -1,1 | 2,49 | -3,58 | 0,00023 | 0,00621 | Intron (uc003lmt.3/23092, intron 18 of 22) | -262719 | ENSG00000226272 | ARHGAP26-AS1 |
| chr14 | 107122072 | 107123212 | -0,56 | 3,09 | -3,65 | 0,00023 | 0,00622 | Exon (uc031qqx.1/uc031qqx.1, exon 98 of 5065) | 183617 | ENSG00000270816 | LINC00221 |
| chr19 | 43253207 | 43254236 | -0,87 | 2,83 | -3,7 | 0,000232 | 0,00626 | Intron (uc002ouf.3/5671, intron 1 of 7) | -8539 | ENSG00000221826 | PSG3 |
| chr15 | 98415093 | 98419448 | 1,98 | 6,91 | -4,93 | 0,000233 | 0,00628 | Promoter (<=1kb) | 0 | ENSG00000251209 | LINC00923 |
| chr7 | 107357468 | 107358202 | -1,12 | 2,48 | -3,6 | 0,000234 | 0,0063 | 3' UTR | 23691 | ENSG00000091137 | SLC26A4 |
| chr18 | 14781351 | 14782003 | -1,28 | 2,25 | -3,52 | 0,000237 | 0,00635 | Intron (uc010xak.2/374860, intron 11 of 29) | 33112 | ENSG00000180777 | ANKRD30B |
| chr4 | 154177918 | 154179219 | 0,37 | 4,46 | -4,1 | 0,000237 | 0,00634 | Promoter (<=1kb) | 0 | ENSG00000109654 | TRIM2 |
| chrX | 153009524 | 153010441 | -0,45 | 3,06 | -3,51 | 0,000238 | 0,00637 | 3'UTR | 19201 | ENSG00000101986 | ABCD1 |
| chr5 | 139403491 | 139404457 | 0,18 | 4,15 | -3,98 | 0,000239 | 0,00637 | Intron (uc003lev.2/9542, intron 1 of 10) | 18427 | ENSG00000158458 | NRG2 |
| chr4 | 80854701 | 80855307 | -1,28 | 2,15 | -3,43 | 0,00024 | 0,0064 | Intron (uc003hly.4/118429, intron 16 of 16) | 106076 | ENSG00000251321 | PCAT4 |
| chr19 | 7669951 | 7671662 | 1,43 | 5,63 | -4,2 | 0,000241 | 0,0064 | Exon (uc002mgu.4/57662, exon 2 of 19) | -7858 | ENSG00000076826 | CAMSAP3 |
| chr1 | 1755803 | 1756387 | -1,28 | 2,2 | -3,47 | 0,000242 | 0,00642 | Intron (uc001aif.3/2782, intron 3 of 11) | -44295 | ENSG0000000813 0 | NADK |
| chr4 | 169440521 | 169442091 | 1,33 | 5,44 | -4,1 | 0,000243 | 0,00644 | Intron (uc003iru.3/23022, intron 2 of 20) | 22304 | ENSG00000129116 | PALLD |
| chr3 | 177365185 | 177365837 | -1,07 | 2,49 | -3,55 | 0,000245 | 0,00648 | Intron (uc031sch.1/100505566, intron 3 of 3) | 205476 | NA | LINC00578 |
| chr7 | 157793771 | 157794864 | -1,18 | 2,15 | -3,33 | 0,000247 | 0,00652 | Intron (uc011kwa.2/5799, intron 11 of 22) | 146494 | NA | LOC10050658 5 |
| chr6 | 25362216 | 25364121 | 0,09 | 4,32 | -4,22 | 0,000248 | 0,00656 | Intron (uc031smx.1/55604, intron 2 of 8) | 82560 | ENSG00000079691 | CARMIL1 |
| chr16 | 34982828 | 34984579 | 0,43 | 4,34 | -3,91 | 0,000248 | 0,00656 | Promoter (1-2kb) | 1905 | ENSG00000261122 | LINC02167 |
| chr5 | 78202951 | 78203606 | -1,12 | 2,45 | -3,57 | 0,000251 | 0,00661 | Intron (uc003kfq.3/411, intron 4 of 7) | 78160 | ENSG00000113273 | ARSB |
| chr3 | 123518229 | 123519019 | -0,49 | 3,56 | -4,05 | 0,000251 | 0,00661 | Promoter (<=1kb) | 16 | ENSG00000065534 | MYLK |
| chr1 | 61843070 | 61843679 | -1,28 | 1,75 | -3,03 | 0,000252 | 0,00663 | Intron (uc001czy.3/4774, intron 6 of 10) | -26069 | ENSG00000162599 | NFIA |
| chr10 | 22496629 | 22497183 | -0,63 | 2,56 | -3,19 | 0,000253 | 0,00665 | Promoter (1-2kb) | 1729 | ENSG00000223601 | EBLN1 |
| chr2 | 1286242 | 1287162 | -1,28 | 2,17 | -3,44 | 0,000253 | 0,00664 | Intron (uc002qwq.3/54221, intron 14 of 16) | -90833 | ENSG00000115705 | TPO |
| chr17 | 48543017 | 48544296 | -0,65 | 2,97 | -3,62 | 0,000254 | 0,00665 | Promoter (1-2kb) | -1394 | ENSG00000167107 | ACSF2 |
| chr1 | 6291414 | 6293068 | 0,87 | 5,28 | -4,41 | 0,000254 | 0,00665 | Promoter (<=1kb) | 0 | ENSG00000110148 | CCKBR |
| chr7 | 18356284 | 18357001 | -1,28 | 2,2 | -3,48 | 0,000258 | 0,00675 | Intron (uc011jya.2/9734, intron 2 of 12) | 26239 | ENSG00000048052 | HDAC9 |
| chr2 | 166996803 | 166997839 | 0,39 | 4,28 | -3,88 | 0,000258 | 0,00675 | Intron (uc002udp.3/uc002udp.3, intron 2 of 11) | 7803 | ENSG00000144285 | SCN1A |
| chr12 | 15865207 | 15866000 | -1,01 | 2,57 | -3,58 | 0,000259 | 0,00676 | Promoter (<=1kb) | 0 | ENSG00000151491 | EPS8 |
| chr1 | 216132777 | 216133352 | -1,28 | 2,18 | -3,46 | 0,000259 | 0,00678 | Intron (uc001hku.1/7399, intron 37 of 71) | 380434 | ENSG00000136636 | KCTD3 |
| chr1 | 67066681 | 67067334 | -0,74 | 2,64 | -3,38 | 0,000261 | 0,00679 | Intron (uc001dcr.3/84251, intron 1 of 24) | -26789 | ENSG00000264720 | MIR3117 |
| chr3 | 23430598 | 23431416 | -1,22 | 2,22 | -3,44 | 0,000261 | 0,00679 | Intron (uc010hfc.2/7325, intron 3 of 4) | 54972 | ENSG00000264419 | MIR548AC |
| chr10 | 18726968 | 18727639 | -0,75 | 2,82 | -3,57 | 0,000262 | 0,00681 | Intron (uc001ipr.2/783, intron 3 of 13) | 37455 | ENSG00000165995 | CACNB2 |
| chr18 | 692595 | 693075 | -1,28 | 2,14 | -3,41 | 0,000263 | 0,00681 | Exon (uc010wyt.1/55556, exon 6 of 7) | 19442 | ENSG00000132199 | ENOSF1 |
| chr15 | 26089502 | 26091417 | 0,25 | 4,19 | -3,94 | 0,000263 | 0,00681 | Promoter (2-3kb) | 2555 | ENSG00000266517 | MIR4715 |
| chr1 1 | 27098381 | 27099076 | -1,28 | 2,16 | -3,44 | 0,000264 | 0,00683 | Intron (uc001mre.1/8424, intron 4 of 8) | 21438 | ENSG00000129151 | BBOX1 |
| chr22 | 44061796 | 44062347 | -1,28 | 2,14 | -3,41 | 0,000265 | 0,00684 | Promoter (<=1kb) | 805 | ENSG00000186976 | EFCAB6 |
| chr5 | 5061722 | 5062375 | -0,76 | 2,68 | -3,44 | 0,000265 | 0,00684 | Intron (uc003jdg.3/340094, intron 4 of 6) | 27250 | ENSG00000215231 | LINC01020 |
| chr19 | 38987372 | 38988091 | -1,21 | 2,15 | -3,37 | 0,000265 | 0,00684 | Promoter (<=1kb) | 517 | ENSG00000196218 | RYR1 |
| chr8 | 135673194 | 135673707 | -1,28 | 2,18 | -3,46 | 0,000265 | 0,00684 | Intron (uc003vun.3/57623, intron 2 of 16) | 35094 | ENSG00000066827 | ZFAT |
| chr9 | 16867566 | 16868351 | -0,75 | 2,63 | -3,38 | 0,000266 | 0,00684 | Promoter (<=1kb) | 0 | ENSG00000173068 | BNC2 |
| chr1 | 19566641 | 19567254 | -1,28 | 2,14 | -3,42 | 0,000266 | 0,00684 | Exon (uc001bbo.4/23065, exon 7 of 23) | 10799 | ENSG00000127463 | EMC1 |
| chr3 | 134869045 | 134869655 | -1,28 | 2,2 | -3,48 | 0,000266 | 0,00684 | Intron (uc003eqt.3/2047, intron 5 of 15) | 82671 | ENSG00000154928 | EPHB1 |
| chr14 | 42359589 | 42360268 | -1,28 | 1,93 | -3,2 | 0,000267 | 0,00685 | Intron (uc001wvm.3/145581, intron 3 of 5) | 190959 | ENSG00000165379 | LRFN5 |
| chr17 | 7994163 | 7994871 | -0,46 | 3,12 | -3,57 | 0,000267 | 0,00685 | Promoter (2-3kb) | 2789 | ENSG00000264005 | MIR4314 |
| chr4 | 152080834 | 152081681 | 0,3 | 3,93 | -3,63 | 0,000269 | 0,0069 | Intron (uc003imb.2/152503, intron 1 of 12) | 5791 | ENSG00000109686 | SH3D19 |
| chr1 | 18194550 | 18195545 | -0,19 | 3,76 | -3,96 | 0,000272 | 0,00696 | Promoter (<=1kb) | 166 | ENSG00000179817 | MRGPRX4 |
| chr4 | 104643719 | 104644704 | -0,49 | 3,33 | -3,81 | 0,000272 | 0,00697 | Promoter (2-3kb) | -2746 | ENSG00000169836 | TACR3 |
| chr9 | 114369255 | 114370126 | -0,13 | 3,22 | -3,36 | 0,000273 | 0,00699 | Intron (uc022blv.1/652972, intron 2 of 2) | 5707 | ENSG00000204173 | LRRC37A5P |
| chr14 | 65174975 | 65176262 | -0,14 | 3,5 | -3,64 | 0,000274 | 0,007 | Intron (uc001xhn.1/26030, intron 1 of 14) | 3782 | ENSG00000126822 | PLEKHG3 |
| chr2 | 218764001 | 218767032 | 0,91 | 5,43 | -4,52 | 0,000274 | 0,007 | Promoter (1-2kb) | 1504 | ENSG00000079308 | TNS1 |
| chr1 1 | 57314798 | 57315421 | -0,56 | 2,84 | -3,4 | 0,000286 | 0,00723 | Intron (uc021qjh.1/219537, intron 6 of 6) | 4684 | ENSG00000214872 | SMTNL1 |
| chr9 | 15284636 | 15285484 | -0,64 | 2,93 | -3,57 | 0,000286 | 0,00722 | Intron (uc003zlr.2/158219, intron 1 of 19) | -10934 | ENSG00000155158 | TTC39B |
| chr6 | 16651772 | 16652687 | -0,42 | 3,2 | -3,61 | 0,000289 | 0,00726 | Intron (uc003nbt.3/6310, intron 4 of 8) | 97637 | ENSG00000124788 | ATXN1 |
| chr1 | 216139154 | 216139887 | -1,28 | 2,1 | -3,37 | 0,000289 | 0,00726 | Intron (uc001hku.1/7399, intron 36 of 71) | 386811 | ENSG00000136636 | KCTD3 |
| chr12 | 24972724 | 24974442 | -0,19 | 4,01 | -4,19 | 0,000291 | 0,00731 | Intron (uc001rgc.3/586, intron 10 of 10) | 80880 | ENSG00000060982 | BCAT1 |
| chrX | 15563330 | 15564066 | -0,42 | 3,22 | -3,64 | 0,000294 | 0,00736 | Intron (uc004cww.3/660, intron 16 of 18) | 37899 | ENSG00000102010 | BMX |
| chr2 | 66016820 | 66018313 | 1,69 | 6,06 | -4,37 | 0,000294 | 0,00736 | Intron (uc010fcy.1/uc010fcy.1, intron 6 of 9) | -357164 | ENSG00000198369 | SPRED2 |
| chr6 | 35754130 | 35755306 | 0,06 | 4,4 | -4,33 | 3,00E-04 | 0,00745 | Exon (uc003old.4/340204, exon 2 of 3) | 5299 | ENSG00000204140 | CLPSL1 |
| chr15 | 98338531 | 98339031 | -1,21 | 1,93 | -3,15 | 3,00E-04 | 0,00745 | Intron (uc002bug.1/91948, intron 1 of 2) | 78628 | ENSG00000251209 | LINC00923 |
| chr15 | 41912784 | 41914672 | 1,06 | 5,46 | -4,4 | 0,000304 | 0,00755 | Promoter (<=1kb) | 0 | ENSG00000174197 | MGA |
| chr22 | 34253106 | 34254517 | -0,13 | 3,67 | -3,8 | 0,000307 | 0,00758 | Intron (uc003and.4/9215, intron 1 of 15) | 61899 | ENSG00000133424 | LARGE1 |
| chr17 | 79533465 | 79533948 | -1,28 | 2,07 | -3,34 | 0,00031 | 0,00765 | Promoter (<=1kb) | 0 | ENSG00000182446 | NPLOC4 |
| chr16 | 57886043 | 57886639 | -1,17 | 2 | -3,16 | 0,000312 | 0,00769 | Intron (uc002emr.1/3801, intron 1 of 9) | 10094 | ENSG00000140859 | KIFC3 |
| chr3 | 51129419 | 51130254 | -0,61 | 2,57 | -3,18 | 0,000313 | 0,00769 | Intron (uc011bds.2/1795, intron 9 of 52) | -292438 | ENSG00000145050 | MANF |
| chr2 | 63274402 | 63275239 | -0,41 | 3,39 | -3,8 | 0,000315 | 0,00774 | Promoter (<=1kb) | 0 | ENSG00000231609 | LOC10013221 5 |
| chr22 | 31518727 | 31519275 | -0,08 | 3,11 | -3,19 | 0,000316 | 0,00775 | Promoter (<=1kb) | 0 | ENSG00000185133 | INPP5J |
| chr2 | 54525451 | 54526090 | -1,28 | 2,09 | -3,36 | 0,000317 | 0,00775 | Intron (uc002rxq.4/98, intron 3 of 3) | -31981 | ENSG00000177994 | C2orf73 |
| chr2 | 202987325 | 202988270 | -0,62 | 2,83 | -3,45 | 0,000317 | 0,00775 | Intron (uc010zhu.1/100652824, intron 11 of 14) | -12169 | ENSG00000182329 | KIAA2012 |
| chr4 | 164270828 | 164271729 | -0,99 | 2,43 | -3,42 | 0,00032 | 0,0078 | 5' UTR | 5737 | ENSG00000164129 | NPY5R |
| chr1 | 2826234 | 2827228 | -0,36 | 3,36 | -3,73 | 0,000322 | 0,00785 | Intron (uc001lwn.3/3784, intron 15 of 15) | -64035 | ENSG00000237941 | KCNQ1DN |
| chr1 | 183382522 | 183383277 | -1,28 | 2,03 | -3,31 | 0,000323 | 0,00786 | Intron (uc001gqc.2/23057, intron 1 of 10) | 4357 | ENSG00000157064 | NMNAT2 |
| chrX | 152224307 | 152225880 | 1,35 | 5,87 | -4,52 | 0,000325 | 0,00789 | Promoter (<=1kb) | 0 | ENSG00000183837 | PNMA3 |
| chr8 | 113962883 | 113963536 | -1,28 | 2,05 | -3,33 | 0,000327 | 0,00793 | Intron (uc003ynt.3/114788,intron 9 of 71) | -260604 | ENSG00000164796 | CSMD3 |
| chr5 | 171839830 | 171840545 | -0,72 | 2,56 | -3,28 | 0,000328 | 0,00797 | Intron (uc003mbr.3/285590, intron 2 of 12) | 40982 | ENSG00000174705 | SH3PXD2B |
| chr3 | 79380909 | 79381518 | -1,28 | 2,07 | -3,35 | 0,00033 | 0,008 | Intron (uc003dqe.2/6091, intron 2 of 30) | -312300 | ENSG00000169855 | ROBO1 |
| chr7 | 138912734 | 138913729 | 0,24 | 4,28 | -4,04 | 0,00033 | 0,008 | Promoter (2-3kb) | -2502 | ENSG00000157741 | UBN2 |
| chr5 | 177768837 | 177769502 | -1,13 | 1,95 | -3,07 | 0,000331 | 0,008 | Intron (uc021yiy.1/91522, intron 2 of 28) | -71473 | ENSG00000050767 | COL23A1 |
| chr16 | 30154167 | 30154447 | -1,13 | 1,59 | -2,72 | 0,000333 | 0,00804 | Intron (uc010bzb.2/552900, intron 2 of 4) | -19537 | ENSG00000102882 | MAPK3 |
| chr14 | 37438665 | 37441191 | 0,82 | 5,13 | -4,31 | 0,000336 | 0,00808 | Intron (uc001wtz.2/89874, intron 1 of 9) | -17069 | ENSG00000266327 | MIR4503 |
| chr15 | 99572717 | 99573325 | -0,99 | 2,21 | -3,2 | 0,000336 | 0,00808 | Exon (uc021sxj.1/uc021sxj.1, exon 1 of 1) | -21693 | ENSG00000183571 | PGPEP1L |
| chr3 | 162930121 | 162931337 | -0,37 | 3,49 | -3,86 | 0,000337 | 0,00809 | Intron (uc003feg.3/uc003feg.3, intron 5 of 7) | 89752 | ENSG00000241369 | LINC01192 |
| chr1 | 71461772 | 71462966 | 0,11 | 3,84 | -3,74 | 0,000337 | 0,00809 | Intron (uc001dfg.1/5733, intron 2 of 4) | -49223 | NA | ZRANB2-AS1 |
| chr6 | 42145568 | 42146462 | -0,69 | 3,12 | -3,81 | 0,000339 | 0,00813 | Exon (uc003orx.3/2978, exon 4 of 6) | 16232 | ENSG00000112599 | GUCA1B |
| chr1 | 61536297 | 61537386 | -0,05 | 3,1 | -3,16 | 0,000339 | 0,00813 | Exon (uc001nsc.1/745, exon 4 of 27) | -7692 | ENSG00000124920 | MYRF |
| chr9 | 77414822 | 77415466 | -0,89 | 2,61 | -3,5 | 0,000339 | 0,00813 | Exon (uc022bic.1/uc022bic.1, exon 1 of 1) | -23657 | ENSG00000119121 | TRPM6 |
| chr1 | 100192507 | 100193375 | -0,31 | 3,47 | -3,78 | 0,00034 | 0,00814 | Intron (uc001pga.3/53942, intron 21 of 24) | 130649 | ENSG00000149972 | CNTN5 |
| chr15 | 27090885 | 27091279 | -1,28 | 1,79 | -3,06 | 0,000342 | 0,00816 | Intron (uc001zbb.3/2562, intron 1 of 9) | -20587 | ENSG00000186297 | GABRA5 |
| chr7 | 90395785 | 90396588 | -0,58 | 3,27 | -3,85 | 0,000345 | 0,00821 | Intron (uc003ukt.1/5218, intron 7 of 7) | 56609 | ENSG00000058091 | CDK14 |
| chr2 | 1813370 | 1814011 | -1,28 | 1,96 | -3,23 | 0,000345 | 0,00821 | Intron (uc010ewk.3/23040, intron 3 of 6) | 32647 | ENSG00000186487 | MYT1L |
| chr3 | 55722602 | 55723496 | -0,81 | 2,78 | -3,59 | 0,000346 | 0,00824 | Intron (uc003dhq.1/26059, intron 4 of 6) | -29105 | ENSG00000281708 | ERC2-IT1 |
| chrX | 49296284 | 49298367 | -1,28 | 1,73 | -3,01 | 0,000349 | 0,00828 | Promoter (<=1kb) | 0 | ENSG00000227488 | GAGE12D |
| chrl6 | 69806664 | 69807652 | -0,56 | 2,96 | -3,52 | 0,000354 | 0,00838 | Intron (uc031qwu.1/11060, intron 1 of 8) | 10477 | ENSG00000198373 | WWP2 |
| chr1 | 62154348 | 62155198 | -1,16 | 1,98 | -3,14 | 0,000356 | 0,00841 | Intron (uc001nte.4/80150, intron 4 of 6) | 29889 | ENSG00000162174 | ASRGL1 |
| chr1 | 81861763 | 81862372 | -1,28 | 1,99 | -3,26 | 0,000356 | 0,00842 | Intron (uc001ozo.1/uc001ozo.1, intron 3 of 7) | -259885 | ENSG0000026411 0 | MIR4300 |
| chrX | 135771435 | 135772041 | -1,16 | 1,69 | -2,85 | 0,000358 | 0,00845 | Intron (uc011mwd.2/9459, intron 10 of 21) | 39010 | ENSG00000102245 | CD40LG |
| chr12 | 124371079 | 124372299 | 0,45 | 4,21 | -3,76 | 0,000358 | 0,00845 | Exon (uc001uft.4/196385, exon 51 of 78) | -37298 | ENSG00000197653 | DNAH10 |
| chr7 | 78265563 | 78266543 | 0,6 | 4,64 | -4,04 | 0,000359 | 0,00846 | Intron (uc003ugx.3/9863, intron 2 of 21) | 134095 | ENSG00000187391 | MAGI2 |
| chr21 | 32413774 | 32414613 | -0,44 | 2,68 | -3,13 | 0,00036 | 0,00848 | Promoter (2-3kb) | -2979 | ENSG00000206102 | KRTAP19-8 |
| chr15 | 22664672 | 22666063 | -0,9 | 2,16 | -3,05 | 0,00036 | 0,00848 | Intron (uc001yuj.2/uc001yuj.2, intron 4 of 12) | 9178 | ENSG00000276941 | MIR4509-1 |
| chr10 | 33043370 | 33044424 | -0,45 | 2,98 | -3,43 | 0,000361 | 0,00849 | Intron (uc001iwl.1/79741, intron 13 of 22) | 25555 | ENSG00000216937 | CCDC7 |
| chr17 | 26622595 | 26623417 | -0,81 | 2,4 | -3,21 | 0,000363 | 0,00852 | Intron (uc002has.3/284085, intron 2 of 2) | 10991 | ENSG00000265480 | KRT18P55 |
| chr5 | 797907 | 798949 | -1,21 | 2,1 | -3,32 | 0,000363 | 0,00852 | Intron (uc010itc.3/79844, intron 6 of 6) | 24977 | ENSG00000188818 | ZDHHC11 |
| chr2 | 198463820 | 198465223 | 0,34 | 4,21 | -3,86 | 0,000366 | 0,00856 | Intron (uc002uuo.4/130132, intron 7 of 8) | 75361 | ENSG00000162944 | RFTN2 |
| chr2 | 206565171 | 206565946 | -0,74 | 2,66 | -3,4 | 0,000373 | 0,00867 | Intron (uc002vat.3/8828, intron 2 of 8) | 17812 | ENSG00000118257 | NRP2 |
| chr10 | 56535231 | 56535798 | -1,28 | 1,66 | -2,94 | 0,000376 | 0,00874 | Intron (uc010qhq.2/65217, intron 1 of 34) | 25253 | ENSG00000150275 | PCDH15 |
| chr2 | 1203752 | 1204566 | -0,87 | 2,63 | -3,49 | 0,000377 | 0,00876 | Intron (uc002qwq.3/54221, intron 8 of 16) | -173429 | ENSG00000115705 | TPO |
| chr14 | 107113704 | 107114582 | -1,28 | 1,98 | -3,25 | 0,000378 | 0,00878 | Exon (uc031qqx.1/uc031qqx.1, exon 110 of 5065) | 175249 | ENSG00000270816 | LINC00221 |
| chr6 | 65599632 | 65600660 | -0,68 | 2,85 | -3,53 | 0,000381 | 0,00883 | Intron (uc011dxu.1/346007, intron 18 of 42) | -68016 | ENSG00000188107 | EYS |
| chr19 | 17545684 | 17546601 | -0,3 | 3,46 | -3,76 | 0,000381 | 0,00883 | 3' UTR | 12775 | ENSG00000188051 | TMEM221 |
| chr2 | 225810596 | 225812247 | 0,73 | 4,84 | -4,11 | 0,000383 | 0,00886 | Promoter (<=1kb) | 0 | ENSG00000135905 | DOCK10 |
| chr12 | 23274158 | 23274861 | -0,68 | 2,35 | -3,03 | 0,000383 | 0,00886 | Intron (uc001rfu.1/uc001rfu.1, intron 8 of 8) | 462685 | ENSG00000134532 | SOX5 |
| chr21 | 37222850 | 37223270 | -1,13 | 2,01 | -3,15 | 0,000384 | 0,00886 | Intron (uc002yut.1/861, intron 4 of 10) | 129837 | ENSG00000211590 | MIR802 |
| chr4 | 126330544 | 126331240 | -1,28 | 1,98 | -3,25 | 0,000385 | 0,00888 | Intron (uc003ifj.4/79633, intron 4 of 16) | 15453 | ENSG00000196159 | FAT4 |
| chr20 | 61953693 | 61954480 | 0,56 | 3,92 | -3,36 | 0,000386 | 0,00889 | Intron (uc011aau.2/57642, intron 27 of 35) | 16328 | ENSG00000101203 | COL20A1 |
| chrX | 100547283 | 100548071 | -0,86 | 2,73 | -3,59 | 0,000386 | 0,00889 | Promoter (<=1kb) | 0 | ENSG00000102387 | TAF7L |
| chr1 | 47898082 | 47898718 | -1,28 | 1,99 | -3,27 | 0,000387 | 0,0089 | Promoter (1-2kb) | 1595 | ENSG00000237424 | FOXD2-AS1 |
| chr14 | 61547609 | 61548210 | -0,85 | 2,4 | -3,25 | 0,000387 | 0,00891 | Intron (uc001xfh.2/145389, intron 16 of 16) | 99644 | ENSG00000139974 | SLC38A6 |
| chr12 | 124613865 | 124617581 | 1,19 | 5,43 | -4,24 | 0,000389 | 0,00893 | Intron (uc021rfy.1/100533183, intron 2 of 4) | 156103 | ENSG00000179195 | ZNF664 |
| chr2 | 225248011 | 225248526 | -1,28 | 2,01 | -3,29 | 0,000392 | 0,00899 | Intron (uc002vnw.3/79843, intron 2 of 2) | 18185 | ENSG00000124019 | FAM124B |
| chr1 | 15296466 | 15297362 | 0,26 | 3,71 | -3,44 | 0,000402 | 0,00916 | Intron (uc009vog.1/23254, intron 2 of 7) | 9286 | ENSG00000189337 | KAZN |
| chr6 | 70752803 | 70753300 | -1,28 | 1,77 | -3,05 | 0,000405 | 0,00922 | Intron (uc003pfc.1/1310, intron 14 of 50) | 115002 | ENSG00000082293 | COL19A1 |
| chr6 | 117725253 | 117725808 | -0,94 | 2,07 | -3,01 | 0,000405 | 0,00921 | Exon (uc003pxp.1/6098, exon 5 of 43) | 21210 | ENSG0000004 7936 | ROS1 |
| chr15 | 26364764 | 26366210 | 0,88 | 4,72 | -3,83 | 0,000406 | 0,00922 | Intron (uc001zay.3/503519, intron 2 of 3) | 3804 | ENSG00000259150 | LINC00929 |
| chr17 | 53363155 | 53363433 | -1,28 | 1,44 | -2,72 | 0,000407 | 0,00924 | Intron (uc002iug.1/3131, intron 2 of 3) | 18179 | ENSG00000108924 | HLF |
| chr15 | 76460910 | 76461888 | 0,55 | 3,92 | -3,38 | 0,000407 | 0,00924 | Intron (uc002bbq.3/123591, intron 5 of 10) | -21922 | ENSG00000169758 | TMEM266 |
| chr12 | 110603222 | 110604231 | -0,22 | 3,38 | -3,6 | 0,000408 | 0,00924 | Intron (uc001tqg.3/28981. intron 11 of 11) | 38057 | ENSG00000122970 | IFT81 |
| chr15 | 34081902 | 34082550 | -0,98 | 2,51 | -3,49 | 0,00041 | 0,00929 | Intron (uc001zhi.3/6263, intron 67 of 103) | -178539 | ENSG00000 184984 | CHRM5 |
| chr1 | 15898144 | 15899230 | -0,62 | 3,07 | -3,69 | 0,000411 | 0,00929 | 3'UTR | 12123 | ENSG00000116138 | DNAJC16 |
| chr12 | 86701082 | 86701889 | -0,34 | 3,27 | -3,61 | 0,000413 | 0,00933 | Intron (uc001taj.4/25834, intron 4 of 8) | -50992 | ENSG00000 182050 | MGAT4C |
| chr4 | 4217955 | 4218738 | -1,28 | 2,03 | -3,31 | 0,000413 | 0,00933 | Intron (uc003ghp.1/133060, intron 1 of 5) | 9883 | ENSG00000163982 | OTOP1 |
| chr4 | 77410303 | 77411041 | -1,28 | 2 | -3,28 | 0,000413 | 0,00931 | Intron (uc011cbx.2/57619, intron 1 of 10) | 54050 | ENSG00000138771 | SHROOM3 |
| chr3 | 38813843 | 38814363 | -0,66 | 2,57 | -3,23 | 0,000417 | 0,00938 | Intron (uc003ciq.3/6336, intron 3 of 26) | 21138 | ENSG00000 185313 | SCN10A |
| chr10 | 24527465 | 24528582 | -0,6 | 3,24 | -3,84 | 0,00042 | 0,00946 | Promoter (<= 1kb) | 0 | ENSG00000 120549 | KIAA1217 |
| chr15 | 26187359 | 26193002 | 2,22 | 6,73 | -4,51 | 0,000421 | 0,00946 | Intron (uc021sgd.1/100128714. intron 1 of 4) | 39852 | ENSG00000206187 | LINC02346 |
| chr7 | 122753946 | 122754384 | -1,28 | 1,85 | -3,12 | 0,000421 | 0,00946 | 3'UTR | 85641 | ENSG00000081800 | SLC13A1 |
| chr18 | 29340225 | 29341211 | -0,46 | 3,39 | -3,84 | 0,000422 | 0,00947 | Promoter (<= 1kb) | 0 | ENSG00000141437 | SLC25A52 |
| chr | 180312219 | 180313355 | -0,04 | 3,8 | -3,84 | 0,000424 | 0,00949 | Intron (uc001gog.3/84320, intron 6 of 7) | -68403 | ENSG00000230124 | LHX4-AS1 |
| chr2 | 47730703 | 47731560 | -0,1 | 3,13 | -3,23 | 0,000424 | 0,00949 | Intron (uc002rvz.4/4436, intron 15 of 18) | 58016 | ENSG00000095002 | MSH2 |
| chr1 | 77945998 | 77946530 | -1,13 | 1,94 | -3,07 | 0,000429 | 0,00959 | Intron (uc001ozg.3/9846. intron 3 of 9) | 35982 | ENSG00000118369 | USP35 |
| chr7 | 156928988 | 156929293 | -1,28 | 1,87 | -3,15 | 0,000436 | 0,00972 | Promoter (2-3kb) | -2362 | ENSG00000009335 | UBE3C |
| chr2 | 2188063 | 2190229 | 0,86 | 5,31 | -4,45 | 0,000437 | 0,00974 | Intron (uc002qxd.3/23040, intron 2 of 24) | -11303 | ENSG00000186487 | MYT1L |
| chr4 | 172996146 | 172996893 | -0,36 | 2,98 | -3,34 | 0,000438 | 0,00976 | Intron (uc003isv.3/442117, intron 2 of 12) | 261571 | ENSG00000174473 | GALNTL6 |
| chr14 | 102318154 | 102318727 | -1,28 | 1,95 | -3,22 | 0,00044 | 0,00978 | Intron (uc001vkk.3/5527, intron 3 of 14) | -29771 | ENSG00000078304 | PPP2R5 C |
| chr1 | 85986480 | 85987526 | -0,07 | 3,95 | -4,02 | 0,000441 | 0,0098 | Intron (uc001dlc.3/23576. intron 1 of 6) | -55591 | ENSG00000153904 | DDAH1 |
| chr2 | 175192966 | 175193795 | -0,65 | 2,91 | -3,56 | 0,000441 | 0,0098 | Promoter (2-3kb) | 2211 | ENSG00000231453 | LINC01305 |
| chr2 | 98316859 | 98317165 | -1,13 | 1,65 | -2,78 | 0,000442 | 0,0098 | Intron (uc002svc.3/728537, intron 8 of 9) | -12866 | ENSG00000115085 | ZAP70 |
| chr7 | 23495089 | 23495671 | -1,28 | 1,93 | -3,21 | 0,000444 | 0,00983 | Intron (uc003swg.3/10643, intron 2 of 14) | 14324 | ENSG00000136231 | IGF2BP3 |
| chr22 | 25215861 | 25216645 | -1,28 | 1,96 | -3,23 | 0,000444 | 0,00982 | Intron (uc003abf.2/129049, intron 2 of 8) | 13725 | ENSG00000167037 | SGSM1 |
| chr5 | 127835950 | 127836428 | -1,28 | 1,55 | -2,82 | 0,000446 | 0,00984 | Intron (uc003kuu.3/2201, intron 5 of 64) | 37307 | ENSG00000138829 | FBN2 |
| chr1 1 | 132112431 | 132112951 | -1,28 | 1,62 | -2,89 | 0,000446 | 0,00984 | Intron (uc001qgm.3/50863, intron 4 of 7) | 331719 | ENSG00000182667 | NTM |
| chrl 5 | 102198322 | 102198816 | -1,28 | 1,56 | -2,84 | 0,000449 | 0,0099 | Intron (uc002bxl.3/123283, intron 9 of 10) | -5728 | ENSG00000184277 | TM2D3 |
| chr10 | 1206839 | 1207489 | -0,57 | 2,43 | -3 | 0,000451 | 0,00993 | Promoter (1-2kb) | 1131 | ENSG00000229205 | LINC00200 |
| chr7 | 140941925 | 140942510 | -1,28 | 1,95 | -3,23 | 0,000454 | 0,00998 | Intron (uc003vwg.2/100507421, intron 2 of 3) | 167893 | ENSG00000261115 | TMEM178B |
| chr14 | 75906496 | 75907765 | 0,35 | 4,16 | -3,81 | 0,000455 | 0,00998 | Intron (uc010asj.3/122953. intron 2 of 3) | 7659 | ENSG00000 140044 | JDP2 |
| chrX | 47496812 | 47497670 | -0,36 | 3,58 | -3,94 | 0,000459 | 0,0101 | Exon (uc004dik.5/2002, exon 5 of 7) | -7108 | ENSG00000126759 | CFP |
| chr15 | 74643075 | 74643766 | -0,77 | 2,72 | -3,49 | 0,000459 | 0,0101 | Intron (uc002axs.2/1583, intron 1 of 8) | -6742 | ENSG00000140459 | CYP11A1 |
| chr10 | 105036093 | 105038643 | 2,88 | 7 | -4,12 | 0,000461 | 0,0101 | Promoter (<=1kb) | 0 | ENSG00000148798 | INA |
| chr2 | 213378243 | 213378827 | -1,28 | 1,92 | -3,2 | 0,000463 | 0,0101 | Intron (uc002veg.1/2066, intron 1 of 27) | 24525 | ENSG00000178568 | ERBB4 |
| chr8 | 94708141 | 94709121 | 0,25 | 4,23 | -3,98 | 0,000468 | 0,0102 | Intron (uc022avb.1/642924, intron 1 of 10) | 3540 | ENSG00000246662 | LINC00535 |
| chr18 | 33843685 | 33844972 | 0,13 | 4 | -3,87 | 0,000469 | 0,0102 | Intron (uc002kzq.4/55034, intron 13 of 14) | -32730 | ENSG00000134775 | FHOD3 |
| chr1 | 184906536 | 184907208 | -0,93 | 2,55 | -3,48 | 0,000469 | 0,0102 | Intron (uc001gra.4/l 16496, intron 1 of 13) | 36510 | ENSG00000135842 | NIBAN1 |
| chr3 | 71212925 | 71213809 | -0,79 | 2,79 | -3,58 | 0,000473 | 0,0103 | Intron (uc003dol.3/27086, intron 2 of 16) | -32833 | ENSG00000 114861 | FOXP1 |
| chr14 | 87371556 | 87374096 | 1,3 | 5,74 | -4,43 | 0,000473 | 0,0103 | Promoter (<=1kb) | 0 | ENSG00000258804 | LINC01148 |
| chr3 | 145786727 | 145791080 | 1,38 | 6,12 | -4,74 | 0,000475 | 0,0103 | 3'UTR | 13442 | ENSG00000152952 | PLOD2 |
| chr16 | 24999541 | 25000474 | -0,41 | 3,41 | -3,82 | 0,000478 | 0,0104 | Intron (uc002dnb.3/55114, intron 1 of 19) | 26201 | ENSG00000140750 | ARHGAP17 |
| chr13 | 22771918 | 22772456 | -0,85 | 2,33 | -3,17 | 0,000484 | 0,0105 | Intron (uc001uoi.3/uc001uoi.3, intron 1 of 1) | -319619 | ENSG00000226722 | LINC00424 |
| chr15 | 76257020 | 76257974 | 0,86 | 4,66 | -3,8 | 0,000485 | 0,0105 | Intron (uc002bbo.3/145957, intron 3 of 5) | -31549 | NA | NA |
| chr18 | 14784036 | 14785981 | 0,3 | 4,15 | -3,85 | 0,000487 | 0,0105 | Exon (uc010xak.2/374860. exon 13 of 30) | 35797 | ENSG00000180777 | ANKRD30B |
| chr16 | 29040533 | 29040811 | -0,88 | 1,86 | -2,73 | 0,000489 | 0,0105 | Intron(uc010vct.2/uc010vct.2,intron2 of7) | 43836 | ENSG00000213658 | LAT |
| chr12 | 62946374 | 62947082 | -1,28 | 1,97 | -3,25 | 0,000489 | 0,0105 | Exon (uc001sre.3/23041, exon 24 of 35) | -6608 | ENSG00000061987 | MON2 |
| chr17 | 27271760 | 27273220 | 0,43 | 4,33 | -3,9 | 0,000489 | 0,0105 | Exon (uc002hd1.3/uc002hd1.3, exon 2 of 2) | 4069 | ENSG00000109118 | PHF12 |
| chr5 | 87896973 | 87897895 | -0,5 | 2,8 | -3,31 | 0,000493 | 0,0106 | Intron (uc011cua.1/645323, intron 3 of 4) | 64862 | ENSG00000284447 | MIR9-2 |
| chr11 | 132533514 | 132533953 | -1,28 | 1,98 | -3,26 | 0,000495 | 0,0106 | Intron (uc001qgs.3/4978, intron 1 of 6) | 279084 | ENSG00000183715 | OPCML |
| chr2 | 201921755 | 201922354 | -1,13 | 2,01 | -3,14 | 0,000498 | 0,0107 | Intron (uc002uws.4/285172, intron 1 of 11) | 14038 | ENSG00000155744 | FAM126B |
| chr9 | 6574730 | 6575591 | -0,42 | 3,11 | -3,53 | 5,00E-04 | 0,0107 | Intron (uc003zkc.3/2731, intron 15 of 24) | 70101 | ENSG00000178445 | GLDC |
| chr11 | 100589883 | 100591012 | -0,14 | 3,55 | -3,69 | 0,000501 | 0,0107 | Intron (uc001pge.2/143872, intron 1 of 23) | 31476 | ENSG00000165895 | ARHGAP42 |
| chr15 | 42438927 | 42439424 | -0,77 | 2,51 | -3,27 | 0,000506 | 0,0108 | Promoter (1-2kb) | -1047 | ENSG00000168907 | PLA2G4F |
| chr1 | 115181640 | 115182205 | -1,21 | 2 | -3,21 | 0,000509 | 0,0108 | Intron (uc001eez.3/163259, intron 1 of 29) | 30527 | ENSG00000175984 | DENND2C |
| chr3 | 141656554 | 141657609 | -0,25 | 3,52 | -3,77 | 0,00051 | 0,0108 | Exon (uc003euj.1/uc003euj.1, exon 1 of 1) | 61084 | ENSG00000069849 | ATP1B3 |
| chr5 | 109048761 | 109049284 | -1,28 | 1,85 | -3,13 | 0,00051 | 0,0108 | Exon (uc003kou.1/4124, exon 2 of 22) | 23605 | ENSG00000 112893 | MAN2A1 |
| chr10 | 465800 | 466486 | -1,14 | 2,15 | -3,3 | 0,000511 | 0,0108 | Intron (uc001ifp.3/22982, intron 5 of 36) | -5750 | ENSG00000 151240 | DIP2C |
| chr11 | 61256022 | 61256683 | -0,05 | 3,3 | -3,35 | 0,000511 | 0,0109 | Intron (uc001nru.2/220004, intron 11 of 13) | 7437 | ENSG00000162148 | PPP1R32 |
| chr1 | 26104559 | 26105403 | -0,55 | 2,91 | -3,46 | 0,000514 | 0,0109 | Exon (uc001bkm.2/57134, exon 9 of 12) | -21264 | ENSG00000162430 | SELENON |
| chr4 | 176823413 | 176824286 | 0,19 | 3,97 | -3,78 | 0,000517 | 0,011 | Intron (uc003iug.4/2823, intron 1 of 7) | -89148 | ENSG00000 150625 | GPM6A |
| chr15 | 44414619 | 44415305 | -0,44 | 3,11 | -3,54 | 0,000524 | 0,0111 | Intron (uc001ztk.1/84978, intron 1 of 15) | 71339 | ENSG00000171877 | FRMD5 |
| chr3 | 146215851 | 146216315 | -1,13 | 2,06 | -3,18 | 0,000524 | 0,0111 | Promoter (2-3kb) | -2073 | ENSG00000163746 | PLSCR2 |
| chr1 | 87574147 | 87574880 | -1,22 | 1,97 | -3,19 | 0,000525 | 0,0111 | 3'UTR | -20568 | ENSG00000267272 | LINC01140 |
| chr20 | 25476997 | 25478718 | 0,29 | 3,91 | -3,62 | 0,000527 | 0,0111 | Exon (uc002wux.1/22981, exon 10 of 24) | -37385 | ENSG00000101004 | NINL |
| chr3 | 78837027 | 78837584 | -0,99 | 1,92 | -2,91 | 0,000529 | 0,0112 | Intron (uc003dqb.2/6091, intron 2 of 28) | -72620 | ENSG00000169855 | ROBO1 |
| chr12 | 132268619 | 132268963 | -1,28 | 1,85 | -3,13 | 0,000538 | 0,0113 | Intron (uc001uja.2/6433, intron 14 of 17) | -43978 | ENSG00000 198598 | MMP17 |
| chr11 | 66009831 | 66010771 | -0,6 | 2,97 | -3,57 | 0,000539 | 0,0113 | 3'UTR | 11930 | ENSG00000175115 | PACS1 |
| chr16 | 21294875 | 21295730 | 0,34 | 4,17 | -3,84 | 0,000543 | 0,0114 | Intron (uc002dil.3/1428, intron 1 of 8) | -5218 | ENSG00000103316 | CRYM |
| chr1 | 247684580 | 247685271 | -0,8 | 2,39 | -3,2 | 0,000551 | 0,0115 | Intron (uc001ida.4/148823, intron 1 of 6) | 8835 | ENSG00000284824 | GCSAML-AS1 |
| chr19 | 3383590 | 3385407 | 0,44 | 4,57 | -4,13 | 0,000556 | 0,0116 | Intron (uc0021xo.3/4782, intron 2 of 10) | 17025 | ENSG00000141905 | NFIC |
| chr10 | 123913506 | 123915246 | 1,69 | 5,3 | -3,61 | 0,000565 | 0,0117 | Intron (uc0011fv.3/10579, intron 7 of 22) | -7859 | ENSG00000138162 | TACC2 |
| chr13 | 113996476 | 113997499 | 1,09 | 4,12 | -3,03 | 0,000566 | 0,0117 | Intron (uc001vtn.3/79774, intron 5 of 7) | 12297 | ENSG00000139835 | GRTP1 |
| chr1 | 183786560 | 183786965 | -1,28 | 1,84 | -3,12 | 0,000566 | 0,0117 | Intron (uc010pof.1/23179, intron 2 of 11) | 12285 | ENSG00000143344 | RGL1 |
| chr12 | 33012200 | 33012792 | -1,17 | 1,94 | -3,11 | 0,000567 | 0,0117 | Intron (uc001rlj.4/5318, intron 4 of 13) | 36988 | ENSG00000057294 | PKP2 |
| chr1 | 93974328 | 93976066 | 0,38 | 4,54 | -4,16 | 0,000568 | 0,0118 | Intron (uc001dpv.3/54874, intron 2 of 14) | -33598 | ENSG00000137942 | FNBP1L |
| chr10 | 64041157 | 64041728 | -1,16 | 2,07 | -3,22 | 0,00057 | 0,0118 | Intron (uc001jly.4/22891, intron 1 of 4) | -12691 | ENSG00000182010 | RTKN2 |
| chr5 | 73001854 | 73002454 | -1,28 | 1,7 | -2,97 | 0,000571 | 0,0118 | Intron (uc010izf.3/64283. intron 2 of 36) | 21188 | ENSG00000214944 | ARHGEF28 |
| chr5 | 73622281 | 73622806 | -0,43 | 2,36 | -2,79 | 0,000571 | 0,0118 | Intron (uc003kdb.3/uc003kdb.3, intron 2 of 2) | -313042 | ENSG00000049860 | HEXB |
| chr11 | 103907091 | 103908215 | -0,15 | 3,41 | -3,56 | 0,000572 | 0,0118 | Promoter (<=1kb) | 0 | ENSG00000170967 | DDI1 |
| chr5 | 83547856 | 83548384 | -0,58 | 2,6 | -3,18 | 0,000572 | 0,0118 | Intron (uc003kio.1/10085, intron 2 of 10) | 132227 | ENSG00000164176 | EDIL3 |
| chr16 | 15081890 | 15084317 | 0,7 | 5,08 | -4,38 | 0,000575 | 0,0118 | Intron (uc010uzt.2/23042. intron 1 of 22) | 13057 | ENSG00000179889 | PDXDC1 |
| chrX | 114800171 | 114801395 | -0,04 | 3,84 | -3,88 | 0,000577 | 0,0119 | Intron (uc010nqf.3/5358, intron 1 of 5) | 4994 | ENSG00000 102024 | PLS3 |
| chr16 | 85074735 | 85075324 | -1,28 | 1,86 | -3,14 | 0,000583 | 0,012 | Intron (uc010voj.2/9764, intron 1 of 11) | 13325 | ENSG00000135709 | KIAA0513 |
| chr18 | 40374974 | 40375826 | -0,82 | 2,48 | -3,29 | 0,000583 | 0,012 | Intron (uc0021av.4/6014, intron 4 of 4) | 319831 | ENSG00000152214 | RIT2 |
| chr17 | 56249217 | 56249985 | -0,61 | 2,55 | -3,16 | 0,000585 | 0,012 | Promoter (2-3kb) | 2200 | ENSG00000255713 | OR4D2 |
| chr17 | 38072913 | 38073446 | -1,13 | 2,08 | -3,21 | 0,000591 | 0,0121 | Promoter (<=1kb) | 128 | ENSG00000073605 | GSDMB |
| chr15 | 74712190 | 74713702 | 0,16 | 4,03 | -3,86 | 0,000591 | 0,0121 | Intron (uc010ulk.2/8482, intron 1 of 13) | 12299 | ENSG00000138623 | SEMA7A |
| chr1 | 167025971 | 167026944 | 0,15 | 3,79 | -3,63 | 0,000593 | 0,0121 | Intron (uc001gea.1/10223, intron 4 of 6) | 32924 | ENSG00000 143167 | GPA33 |
| chr5 | 64684685 | 64685320 | -0,96 | 2,15 | -3,11 | 0,000596 | 0,0121 | Intron (uc003jto.4/11174, intron 6 of 24) | 84459 | ENSG00000049192 | ADAMTS6 |
| chr1 | 27932660 | 27933166 | -1,13 | 2,04 | -3,17 | 0,000596 | 0,0121 | Promoter (2-3kb) | -2299 | ENSG00000126705 | AHDC1 |
| chrX | 43036135 | 43038568 | 0,13 | 4,18 | -4,05 | 0,000596 | 0,0121 | Exon (uc004dfw.1/uc004dfw.1, exon 1 of 6) | -398649 | ENSG00000 102055 | PPP1R2C |
| chr11 | 12863298 | 12865642 | 1,02 | 4,88 | -3,86 | 0,000596 | 0,0121 | Intron (uc021qdx.1/7003, intron 3 of 12) | 13630 | ENSG00000187079 | TEAD1 |
| chr3 | 77575093 | 77575715 | -1,28 | 1,82 | -3,1 | 0,000598 | 0,0122 | Intron (uc021xat.1/6092, intron 6 of 25) | -62187 | ENSG00000185008 | ROBO2 |
| chr1 | 216130379 | 216132486 | 1,12 | 5,62 | -4,5 | 0,000601 | 0,0122 | Intron (uc001hku.1/7399, intron 37 of 71) | 378036 | ENSG00000136636 | KCTD3 |
| chr6 | 169041473 | 169042074 | -1,28 | 1,54 | -2,81 | 0,000609 | 0,0123 | Intron (uc003qwr.2/64094, intron 9 of 12) | 23551 | ENSG00000 112562 | SMOC2 |
| chr12 | 24718095 | 24718676 | -1,17 | 2,01 | -3,18 | 0,000609 | 0,0123 | Promoter (2-3kb) | -2712 | ENSG00000134532 | SOX5 |
| chr11 | 31993143 | 31993678 | -1,28 | 1,87 | -3,15 | 0,00061 | 0,0124 | Intron (uc021qfp.1/5954, intron 1 of 5) | -118799 | ENSG00000049449 | RCN1 |
| chr2 | 207414996 | 207415667 | -0,86 | 2,25 | -3,11 | 0,000613 | 0,0124 | Intron (uc002vbq.4/8745, intron 9 of 25) | -91475 | ENSG00000235118 | FAM237A |
| chr6 | 149971787 | 149972453 | -0,46 | 2,34 | -2,8 | 0,000617 | 0,0124 | Promoter (1-2kb) | -1847 | ENSG00000 186625 | KATNA1 |
| chr4 | 186273191 | 186274135 | -0,16 | 3,33 | -3,49 | 0,000617 | 0,0125 | Intron (uc003ixh.3/83891, intron 14 of 18) | 26017 | ENSG00000109771 | LRP2BP |
| chr2 | 201913861 | 201914447 | -0,07 | 3,41 | -3,48 | 0,000618 | 0,0125 | Intron (uc002uws.4/285172, intron 1 of 11) | 21945 | ENSG00000155744 | FAM126B |
| chr16 | 15497504 | 15498076 | -1,28 | 1,52 | -2,79 | 0,000618 | 0,0125 | Intron (uc002ddm.2/255027, intron 1 of 2) | 7893 | ENSG00000156968 | MPV17L |
| chr15 | 82382773 | 82383140 | -1,28 | 1,59 | -2,86 | 0,000621 | 0,0125 | Intron (uc002bgr.3/uc002bgr.3, intron 1 of 5) | -44289 | ENSG00000183496 | MEX3B |
| chr14 | 23284726 | 23285072 | -0,89 | 1,88 | -2,77 | 0,000621 | 0,0125 | Promoter (<= 1kb) | 35 | ENSG00000155465 | SLC7A7 |
| chr6 | 41823268 | 41824871 | 0,6 | 4,46 | -3,86 | 0,000623 | 0,0125 | Intron (uc003ori.3/25862, intron 3 of 6) | 38228 | ENSG00000164663 | USP49 |
| chr17 | 34781015 | 34782261 | -1,28 | 1,23 | -2,5 | 0,000625 | 0,0125 | Intron (uc002hll.1/654341. intron 11 of 11) | 23754 | ENSG00000274226 | TBC1D3H |
| chr7 | 51329097 | 51333930 | 1,92 | 6,28 | -4,37 | 0,000626 | 0,0126 | Intron (uc003tpr.4/23242, intron 1 of 12) | 50585 | ENSG00000106078 | COBL |
| chr10 | 45408628 | 45409344 | -0,52 | 2,68 | -3,21 | 0,000631 | 0,0126 | Promoter (1-2kb) | 1864 | ENSG00000187783 | TMEM72 |
| chr6 | 161591096 | 161592061 | -0,87 | 2,44 | -3,3 | 0,000634 | 0,0127 | Intron (uc003qtr.1/56895, intron 2 of 8) | -8082 | ENSG00000279355 | AGPAT4-IT1 |
| chr6 | 18697846 | 18698281 | -1,17 | 1,67 | -2,84 | 0,000634 | 0,0127 | Intron (uc003nct.1/uc003nct.1, intron 6 of 7) | 125831 | ENSG00000207775 | MIR548A1 |
| chr22 | 45257609 | 45259878 | 1,27 | 5,44 | -4,17 | 0,000638 | 0,0127 | 3'UTR | 75272 | ENSG00000241484 | ARHGAP8 |
| chr18 | 66547125 | 66547939 | -0,46 | 2,94 | -3,39 | 0,000641 | 0,0128 | Intron (uc0021kk.2/79839, intron 7 of 9) | 56514 | ENSG00000150636 | CCDC102B |
| chr4 | 89020477 | 89021565 | 0,07 | 3,52 | -3,46 | 0,000647 | 0,0129 | Exon (uc003hrf.3/9429, exon 9 of 13) | 31504 | ENSG00000 118777 | ABCG2 |
| chr17 | 7784708 | 7785780 | -0,17 | 3,01 | -3,18 | 0,000647 | 0,0129 | Promoter (2-3kb) | -2343 | ENSG00000170004 | CHD3 |
| chr16 | 22381724 | 22382037 | -0,5 | 2,04 | -2,54 | 0,00065 | 0,0129 | Intron (uc002dkn.3/1039, intron 1 of 4) | 3901 | ENSG00000140743 | CDR2 |
| chr10 | 73380937 | 73382344 | -0,34 | 3,06 | -3,4 | 0,000651 | 0,0129 | Intron (uc001jrw.4/64072, intron 11 of 12) | -90017 | ENSG00000107736 | CDH23 |
| chr9 | 116392 | 117753 | -0,89 | 2,47 | -3,36 | 0,000652 | 0,0129 | Promoter(<=1kb) | 664 | ENSG00000170122 | FOXD4 |
| chr10 | 3169018 | 3169767 | -0,87 | 2,46 | -3,33 | 0,000653 | 0,0129 | Intron (uc001igp.3/5214, intron 16 of 21) | 13454 | ENSG00000067057 | PFKP |
| chr3 | 141859809 | 141860235 | -1,12 | 1,67 | -2,79 | 0,000655 | 0,013 | Intron (uc003eum.4/7029, intron 1 of 13) | 8151 | ENSG00000 114126 | TFDP2 |
| chr1 | 98313101 | 98314587 | 0,13 | 3,83 | -3,7 | 0,00066 | 0,013 | Intron (uc001drv.3/1806, intron 2 of 22) | 72028 | ENSG00000188641 | DPYD |
| chr17 | 15406248 | 15406827 | 0,03 | 3,84 | -3,81 | 0,000662 | 0,013 | Exon(uc010vvw.2/100533496.exon6of7) | -35323 | ENSG00000239704 | CDRT4 |
| chr10 | 56997773 | 56998213 | -1,16 | 1,64 | -2,8 | 0,000663 | 0,0131 | Intron (uc001jjv.1/65217, intron 2 of 20) | -360537 | ENSG00000249860 | MTRNR2L5 |
| chr12 | 41673827 | 41674903 | -0,04 | 3,69 | -3,73 | 0,000664 | 0,0131 | Intron (uc010skn.2/29951, intron 3 of 9) | 91577 | ENSG00000165966 | PDZRN4 |
| chr13 | 43922735 | 43923232 | -0,75 | 1,94 | -2,69 | 0,000665 | 0,0131 | Intron (uc001uza.4/55068, intron 8 of 16) | 11981 | ENSG00000120658 | ENOX1 |
| chr9 | 14100189 | 14101020 | 0,15 | 3,68 | -3,53 | 0,000665 | 0,0131 | Intron (uc003ztd.3/4781. intron 10 of 10) | 79777 | ENSG00000147862 | NFIB |
| chr15 | 99682663 | 99683361 | -0,27 | 3,03 | -3,3 | 0,000666 | 0,0131 | Intron (uc002bur.3/64927, intron 11 of 12) | 37377 | ENSG00000182253 | SYNM |
| chr1 1 | 81892040 | 81893439 | -0,23 | 3,54 | -3,77 | 0,00067 | 0,0132 | Intron (uc001ozo.1/uc001ozo.1, intron 3 of 7) | -290162 | ENSG0000026411 0 | MIR4300 |
| chr6 | 163520712 | 163521704 | 0,36 | 3,91 | -3,55 | 0,00067 | 0,0132 | Intron (uc003qua.3/135138, intron 5 of 6) | 223801 | ENSGO0000281692 | PACRG-AS1 |
| chr7 | 220004 | 222570 | 0,47 | 4,19 | -3,71 | 0,000672 | 0,0132 | Intron (uc003sip.3/56975, intron 3 of 9) | 27035 | ENSG00000177706 | FAM20C |
| chr6 | 5049863 | 5050761 | -0,31 | 3,44 | -3,75 | 0,000674 | 0,0132 | Intron (uc003mwn.1/uc003mwn.1, intron 2 of 3) | -34959 | ENSG00000219607 | PPP1R3 G |
| chr3 | 186789122 | 186793395 | 2,51 | 7,63 | -5,12 | 0,000675 | 0,0132 | Exon (uc003frb.3/6480, exon 6 of 8) | 49457 | ENSG00000073849 | ST6GAL1 |
| chr7 | 92855480 | 92855853 | -1,02 | 2,11 | -3,14 | 0,000679 | 0,0133 | Promoter(<=1kb) | 0 | ENSG00000188175 | HEPACAM2 |
| chr14 | 53034869 | 53035925 | 0,67 | 4,2 | -3,53 | 0,00069 | 0,0134 | Intron (uc001wzt.4/283554, intron 1 of 7) | 15003 | ENSG00000 180998 | GPR137C |
| chr20 | 55097223 | 55097679 | -0,85 | 2,08 | -2,93 | 0,000691 | 0,0134 | Promoter (2-3kb) | -2106 | ENSG00000124103 | FAM209A |
| chr6 | 39759503 | 39761714 | 2,29 | 6,33 | -4,03 | 0,000692 | 0,0134 | Promoter (<=1kb) | 0 | ENSG00000146122 | DAAM2 |
| chr15 | 21926431 | 21936246 | 2,89 | 8,09 | -5,2 | 0,000692 | 0,0134 | Exon (uc010tzj.1/646214, exon 1 of 1) | 4493 | NA | LOC646214 |
| chr1 | 58862377 | 58862937 | -0,75 | 2,18 | -2,93 | 0,000692 | 0,0134 | Intron (uc001cvt.1/1600, intron 3 of 20) | 142045 | ENSG00000 162600 | OMA1 |
| chr6 | 2733860 | 2734227 | -0,86 | 1,98 | -2,84 | 0,000695 | 0,0135 | Intron (uc003mty.4/340156, intron 2 of 12) | 16927 | ENSG00000145949 | MYLK4 |
| chr1 | 246157353 | 246157892 | -1,28 | 1,8 | -3,07 | 0,000703 | 0,0136 | Intron (uc001ibk.3/64754, intron 5 of 11) | -63228 | ENSG00000 185420 | SMYD3 |
| chr4 | 103534242 | 103534627 | -0,6 | 2,26 | -2,87 | 0,000708 | 0,0136 | Exon (uc011cep.2/4790, exon 23 of 24) | 35371 | ENSG00000109320 | NFKB1 |
| chr20 | 8624170 | 8625105 | -1,28 | 1,78 | -3,06 | 0,000712 | 0,0137 | Intron (uc010zrb.1/23236, intron 4 of 27) | 42789 | ENSG00000182621 | PLCB1 |
| chr9 | 69137303 | 69138613 | -0,44 | 2,17 | -2,61 | 0,000713 | 0,0137 | Intron (uc004aff.4/595135, intron 1 of 8) | 9241 | ENSG00000277778 | PGM5P2 |
| chr2 | 153223199 | 153225087 | 1,02 | 4,69 | -3,67 | 0,000722 | 0,0139 | Intron (uc002tve.3/114793, intron 1 of 25) | 31448 | ENSG00000157827 | FMNL2 |
| chr1 | 61649401 | 61651375 | 1,06 | 5,2 | -4,15 | 0,000724 | 0,0139 | Intron (uc001czv.3/4774, intron 2 of 10) | 101421 | ENSG00000 162599 | NFIA |
| chr4 | 13841970 | 13842648 | -0,69 | 2,31 | -3,01 | 0,000726 | 0,0139 | Intron (uc003gna.1/uc003gna.1, intron 3 of 3) | 212481 | ENSG00000266240 | MIR5091 |
| chr3 | 22049374 | 22050333 | 0,68 | 4,01 | -3,32 | 0,000728 | 0,0139 | Intron (uc010hfb.1/79750, intron 2 of 6) | -256558 | ENSG00000151789 | ZNF385D |
| chr12 | 123269956 | 123271122 | -0,36 | 3,04 | -3,4 | 0,000731 | 0,014 | Promoter (2-3kb) | -2183 | ENSG00000130783 | CCDC62 |
| chr3 | 77571599 | 77572766 | -0,55 | 2,93 | -3,49 | 0,000731 | 0,014 | 5'UTR | -65136 | ENSG00000185008 | ROBO2 |
| chr8 | 12652909 | 12654986 | 1,64 | 5,69 | -4,05 | 0,000733 | 0,014 | Promoter (1-2kb) | 1157 | ENSG00000255494 | LINC00681 |
| chr7 | 102677920 | 102679226 | 0,96 | 5,28 | -4,32 | 0,000734 | 0,014 | Intron (uc003vaq.2/222235, intron 2 of 19) | -12243 | ENSG00000161040 | FBXL13 |
| chr2 | 37319676 | 37320244 | -0,3 | 2,37 | -2,67 | 0,000736 | 0,014 | Intron (uc002rpr.4/253635, intron 4 of 6) | 8082 | ENSG00000152133 | GPATCH11 |
| chr3 | 155410205 | 155410922 | 0,56 | 3,94 | -3,38 | 0,000738 | 0,014 | Intron (uc021xgf.1/23007, intron 1 of 21) | 11075 | ENSG00000 114805 | PLCH1 |
| chr8 | 26100906 | 26102012 | 0,68 | 3,77 | -3,09 | 0,000741 | 0,0141 | Intron (uc003xek.3/5520, intron 7 of 10) | -46995 | ENSG00000221914 | PPP2R2A |
| chr3 | 177237194 | 177239572 | 1,57 | 5,79 | -4,22 | 0,000743 | 0,0141 | Intron (uc031sch.1/100505566, intron 1 of 3) | 77485 | NA | LINC00578 |
| chr15 | 66312157 | 66312556 | -0,85 | 2,12 | -2,96 | 0,000743 | 0,0141 | Intron (uc002apl.2/84465, intron 3 of 20) | -20015 | ENSG00000263512 | MIR4311 |
| chr3 | 132756833 | 132758093 | 0,55 | 3,74 | -3,2 | 0,000743 | 0,0141 | Promoter(<=1kb) | 0 | ENSG00000 144868 | TMEM108 |
| chr1 1 | 1767957 | 1768578 | -1,08 | 2,01 | -3,09 | 0,000745 | 0,0141 | Intron (uc0011tq.2/81532, intron 1 of 4) | 3246 | ENSG00000244242 | IFITM10 |
| chr7 | 23360614 | 23361512 | -1,28 | 1,82 | -3,09 | 0,000759 | 0,0143 | Intron (uc003 swf.3/10643, intron 3 of 7) | 21674 | ENSG00000156928 | MALSU1 |
| chr7 | 51281069 | 51281438 | -1,28 | 1,74 | -3,01 | 0,000768 | 0,0144 | Intron (uc003tpr.4/23242, intron 2 of 12) | -19783 | ENSG00000106078 | COBL |
| chr12 | 95941759 | 95943044 | 0,96 | 4,84 | -3,88 | 0,000771 | 0,0145 | Promoter(<=1kb) | 0 | ENSG00000136014 | USP44 |
| chr11 | 67976091 | 67977817 | 2,53 | 5,39 | -2,85 | 0,000776 | 0,0145 | Promoter (2-3kb) | 2967 | ENSG00000 110066 | KMT5B |
| chr3 | 169539688 | 169540837 | 1,11 | 5,13 | -4,02 | 0,00078 | 0,0146 | Promoter(<=1kb) | 0 | ENSG00000188306 | LRRIQ4 |
| chr4 | 79155053 | 79155692 | -0,75 | 2,21 | -2,96 | 0,000782 | 0,0146 | Intron (uc003hkw.3/80144, intron 2 of 41) | -10695 | ENSG00000138759 | FRAS1 |
| chr22 | 40451742 | 40452657 | -0,68 | 2,43 | -3,11 | 0,000782 | 0,0146 | Intron (uc003avm.3/23112, intron 1 of 23) | 10264 | ENSG00000100354 | TNRC6B |
| chr17 | 10368673 | 10369085 | -1,28 | 1,55 | -2,82 | 0,000788 | 0,0147 | Exon (uc002gmn.3/4622, exon 5 of 40) | 3791 | ENSG00000264424 | MYH4 |
| chr10 | 26465219 | 26465574 | -1,28 | 1,43 | -2,71 | 0,00079 | 0,0147 | Intron (uc001isn.2/53904, intron 30 of 34) | -39662 | ENSG00000136750 | GAD2 |
| chr15 | 64458648 | 64459164 | -0,79 | 2,28 | -3,07 | 0,000796 | 0,0148 | 3'UTR | -3294 | ENSG00000166794 | PPIB |
| chr8 | 96443561 | 96444417 | -0,86 | 2,45 | -3,3 | 0,000799 | 0,0148 | Intron (uc022ayl.1/100616530. intron 2 of 5) | -162099 | ENSG00000156172 | C8orf37 |
| chrX | 152682051 | 152682664 | -1,13 | 1,91 | -3,05 | 0,000802 | 0,0149 | Promoter (1-2kb) | -1117 | ENSG00000 189420 | ZFP92 |
| chr10 | 98886636 | 98887825 | 0,13 | 4,05 | -3,93 | 0,000803 | 0,0149 | Intron (uc001kmw.2/6585, intron 4 of 36) | 24123 | ENSG00000234855 | SLIT1-AS 1 |
| chr11 | 57419517 | 57420906 | 0,95 | 4,85 | -3,9 | 0,000803 | 0,0149 | Promoter (2-3kb) | -2100 | ENSG00000166793 | YPEL4 |
| chr6 | 5630843 | 5631134 | -1,28 | 1,43 | -2,71 | 0,000808 | 0,015 | Intron (uc010jnv.1/10667, intron 6 of 6) | 369259 | ENSG00000 145982 | FARS2 |
| chr4 | 114138665 | 114139018 | -1,17 | 1,48 | -2,65 | 0,00082 | 0,0151 | Intron (uc003ibc.2/287,intron 5 of 30) | -75120 | ENSG00000145362 | ANK2 |
| chr18 | 377083 | 377672 | -1,1 | 1,92 | -3,02 | 0,000821 | 0,0152 | Intron (uc002kkm.3/81035, intron 2 of 9) | -109024 | ENSG0000007913 4 | THOC1 |
| chr12 | 113515081 | 113516101 | 0,26 | 3,75 | -3,49 | 0,00083 | 0,0153 | Exon (uc001tuk.1/1840. exon 2 of 9) | 19419 | ENSG00000135144 | DTX1 |
| chr12 | 63081089 | 63081439 | -1,03 | 1,74 | -2,77 | 0,000834 | 0,0153 | Intron (uc001srk.3/57460, intron 8 of 9) | 83623 | ENSG00000199179 | MIRLET7I |
| chr2 | 222285510 | 222286419 | -0,73 | 2,56 | -3,28 | 0,000835 | 0,0153 | Intron (uc002vmq.3/2043, intron 17 of 17) | 150582 | ENSG00000 116106 | EPHA4 |
| chr16 | 11269900 | 11270397 | -1,28 | 1,75 | -3,03 | 0,000842 | 0,0154 | Intron (uc002dao.3/23274, intron 22 of 22) | 50064 | ENSG00000038532 | CLEC16A |
| chr5 | 122885053 | 122886076 | -0,78 | 2,5 | -3,28 | 0,000846 | 0,0155 | Intron (uc003ktn.4/1456, intron 2 of 12) | 3942 | ENSG00000151292 | CSNK1G3 |
| chr18 | 77034689 | 77036689 | 1,02 | 4,67 | -3,65 | 0,000848 | 0,0155 | Intron (uc0021mv.1/374868, intron 9 of 12) | -67576 | ENSG00000166377 | ATP9B |
| chr9 | 15852794 | 15853459 | -1,28 | 1,68 | -2,95 | 0,000848 | 0,0155 | Intron (uc003zmd.3/203238,intron23 of25) | 108523 | ENSG00000 164989 | CCDC171 |
| chr20 | 45522547 | 45530642 | 3,84 | 8,85 | -5,02 | 0,000851 | 0,0155 | Promoter (<=1kb) | 0 | ENSG00000064655 | EYA2 |
| chr19 | 38943235 | 38943800 | -0,71 | 2,38 | -3,09 | 0,000851 | 0,0155 | Exon (uc002oit.3/6261, exon 13 of 106) | 18895 | ENSG00000196218 | RYR1 |
| chr7 | 17377096 | 17378193 | -0,43 | 3,26 | -3,69 | 0,000852 | 0,0155 | Intron (uc011jxz.1/196, intron 9 of 10) | 38820 | ENSG00000 106546 | AHR |
| chr18 | 44601466 | 44602248 | 0,06 | 3,56 | -3,5 | 0,000853 | 0,0155 | Exon (uc010dnq.1/83473,exon 4 of 7) | -39478 | ENSG00000206181 | ELOA2 |
| chr4 | 80896839 | 80897127 | -1,17 | 1,58 | -2,75 | 0,000854 | 0,0155 | Intron (uc003htv.4/118429. intron 16 of 16) | 95681 | ENSG00000163297 | ANTXR2 |
| chr4 | 187002272 | 187002696 | -1,28 | 1,48 | -2,75 | 0,000854 | 0,0155 | Promoter (<=1kb) | -75 | ENSG00000164342 | TLR3 |
| chr1 | 24434062 | 24435746 | 0,35 | 4,34 | -3,99 | 0,000859 | 0,0156 | Promoter (<= 1kb) | 0 | ENSG00000142661 | MYOM3 |
| chr21 | 17715566 | 17716674 | -0,42 | 3,1 | -3,52 | 0,000861 | 0,0156 | Intron (uc002ykb.2/388815, intron 4 o f 7) | 148867 | NA | MIR99AHG |
| chr19 | 47494611 | 47494983 | -0,77 | 2,14 | -2,91 | 0,00087 | 0,0157 | Intron (uc010ekv.3/2909, intron 4 of 5) | -28118 | ENSG00000 130751 | NPAS1 |
| chr3 | 108087456 | 108089197 | 0,43 | 4,25 | -3,82 | 0,000873 | 0,0158 | Intron (uc003dwy.4/11148, intron 7 of 9) | 66124 | ENSG00000114455 | HHLA2 |
| chr16 | 71995001 | 71995417 | -1,13 | 1,86 | -2,99 | 0,000875 | 0,0158 | Intron (uc010vmm.2/342372, intron 16 of 30) | 38460 | ENSG00000277481 | PKD1L3 |
| chr4 | 190805288 | 190806424 | 0,96 | 3,93 | -2,97 | 0,000889 | 0,0159 | Intron(uc003izq.4/uc003izq.4,intron2 of2) | -55550 | ENSG00000109536 | FRG1 |
| chr2 | 106214160 | 106215930 | 0,59 | 3,99 | -3,39 | 0,000889 | 0,0159 | Intron (uc002tdf.3/285000, intron 2 of 3) | 11086 | NA | LOC285000 |
| chr17 | 37782678 | 37784004 | 2,87 | 6,26 | -3,38 | 0,000891 | 0,0159 | Promoter (<=1kb) | 0 | ENSG00000 131771 | PPP1R1B |
| chr7 | 122020724 | 122021295 | -0,89 | 2,23 | -3,13 | 0,000895 | 0,016 | Intron (uc003vkg.4/93664, intron 17 of 22) | -76159 | ENSG00000128610 | FEZF1 |
| chr16 | 3067910 | 3068867 | -0,55 | 2,94 | -3,49 | 0,000911 | 0,0162 | Promoter (<=1kb) | 0 | ENSG00000184697 | CLDN6 |
| chr14 | 100317359 | 100317848 | -0,75 | 1,94 | -2,69 | 0,000914 | 0,0162 | Exon (uc010avt.1/2009, exon 2 of 11) | 57614 | ENSG00000066629 | EML1 |
| chr1 | 196261436 | 196262216 | -0,53 | 2,79 | -3,32 | 0,000919 | 0,0163 | Intron (uc009wvt.1/343450, intron 15 of 20) | 49163 | ENSG00000162687 | KCNT2 |
| chr13 | 42151616 | 42151838 | -1,28 | 1,21 | -2,49 | 0,00092 | 0,0163 | Intron (uc001uyj.3/23078, intron 42 of 44) | -9085 | ENSG00000284584 | MIR5006 |
| chr19 | 43911641 | 43912884 | 1,1 | 4,77 | -3,67 | 0,00092 | 0,0163 | Intron (uc002owk.3/83639, intron 4 of 8) | -6151 | ENSG00000 131126 | TEX101 |
| chr12 | 111291323 | 111291972 | -0,36 | 2,9 | -3,26 | 0,000923 | 0,0163 | Exon (uc001trv.1/160762. exon 3 of 12) | 6512 | ENSG00000173093 | CCDC63 |
| chr4 | 3391898 | 3392744 | -0,37 | 3,18 | -3,55 | 0,000924 | 0,0163 | Intron (uc003ggu.2/6002, intron 4 of 6) | 3851 | ENSG00000159788 | RGS12 |
| chr12 | 110313151 | 110314417 | 0,96 | 4,13 | -3,18 | 0,000932 | 0,0165 | Intron (uc001tpm.3/51228, intron 1 of 4) | 3876 | ENSG00000139433 | GLTP |
| chr8 | 94478668 | 94479403 | -0,89 | 2,01 | -2,9 | 0,000933 | 0,0165 | Intron (uc022avb.1/642924, intron 7 of 10) | 233258 | ENSG00000246662 | LINC00535 |
| chr8 | 113969295 | 113970579 | 1,19 | 4,76 | -3,57 | 0,000934 | 0,0165 | Intron (uc003ynt.3/114788, intron 8 of 71) | -267016 | ENSG00000164796 | CSMD3 |
| chr12 | 12062206 | 12062851 | -1,28 | 1,63 | -2,91 | 0,000934 | 0,0165 | Intron (uc001raa.1/2120, intron 1 of 1) | 39848 | ENSG00000139083 | ETV6 |
| chr6 | 10585413 | 10589345 | 2,82 | 7,51 | -4,69 | 0,000934 | 0,0165 | Promoter (<=1kb) | 0 | ENSG00000 111846 | GCNT2 |
| chr13 | 33683576 | 33684302 | -0,26 | 2,9 | -3,17 | 0,000934 | 0,0165 | Exon (uc001uuu.3/90627, exon 12 of 14) | 75914 | ENSG00000133121 | STARD13 |
| chr2 | 204335787 | 204336911 | -0,47 | 2,56 | -3,03 | 0,000945 | 0,0166 | Intron (uc002vad.3/65059, intron 4 of 13) | 63147 | ENSG00000 173166 | RAPH1 |
| chr2 | 204369164 | 204369567 | -1,28 | 1,69 | -2,96 | 0,000946 | 0,0166 | Intron (uc002vad.3/65059, intron 1 of 13) | 30491 | ENSG00000 173166 | RAPH1 |
| chr4 | 2368221 | 2368661 | -1,28 | 1,63 | -2,9 | 0,000958 | 0,0168 | Promoter (1-2kb) | -1617 | ENSG00000159733 | ZFYVE28 |
| chr6 | 44083776 | 44085090 | -0,47 | 2,75 | -3,23 | 0,00096 | 0,0168 | 5'UTR | 10101 | ENSG00000180992 | MRPL14 |
| chr19 | 38923739 | 38925901 | 3,62 | 6,54 | -2,92 | 0,000965 | 0,0169 | Promoter (<=1kb) | 0 | ENSG00000196218 | RYR1 |
| chr4 | 40583676 | 40584178 | -0,03 | 3,11 | -3,14 | 0,00097 | 0,0169 | Intron (uc003gvc.2/54502, intron 1 of 6) | 47705 | ENSG00000163694 | RBM47 |
| chr1 | 155096971 | 155097840 | -0,26 | 3,38 | -3,64 | 0,000974 | 0,017 | Promoter (2-3kb) | -2509 | ENSG00000 169242 | EFNA1 |
| chr18 | 74700006 | 74701038 | 0,29 | 3,63 | -3,34 | 0,000975 | 0,017 | Promoter (<=1kb) | -94 | ENSG00000197971 | MBP |
| chr20 | 17498541 | 17499524 | 0,06 | 3,53 | -3,46 | 0,000977 | 0,017 | Intron (uc002wpo.3/631, intron 2 of 7) | 12489 | ENSG00000125864 | BFSP1 |
| chr15 | 26093969 | 26095051 | 0,07 | 3,38 | -3,31 | 0,000978 | 0,017 | Promoter (<=1kb) | 0 | ENSG00000266517 | MIR4715 |
| chr10 | 12675763 | 12677373 | 0,43 | 4,33 | -3,9 | 0,000982 | 0,0171 | Intron (uc001iln.3/57118, intron 2 of 9) | 55011 | ENSG00000263584 | MIR4480 |
| chr15 | 92659282 | 92659680 | -1,28 | 1,64 | -2,92 | 0,000982 | 0,0171 | Intron (uc002bqx.2/28232, intron 4 of 9) | 261717 | ENSG00000176463 | SLCO3A1 |
| chr19 | 41985624 | 41986829 | 0,92 | 4,41 | -3,49 | 0,000984 | 0,0171 | Intron (uc002orb.4/100505495, intron 2 of 3) | 19725 | ENSG00000267107 | PCAT19 |
| chr1 1 | 95769962 | 95771453 | -0,07 | 3,69 | -3,76 | 0,000985 | 0,0171 | Intron (uc001pfw.1/84441, intron 2 of 4) | -112591 | ENSG00000087053 | MTMR2 |
| chr6 | 7171105 | 7172164 | -0,51 | 3,03 | -3,55 | 0,000985 | 0,0171 | Intron (uc021vkv.1/6239, intron 1 of 11) | -9939 | ENSG00000124782 | RREB1 |
| chr5 | 153582212 | 153583131 | -0,25 | 3,05 | -3,29 | 0,000989 | 0,0172 | Intron (uc003lvg.1/55568, intron 1 of 6) | 11917 | ENSG00000164574 | GALNT10 |
| chr2 | 3645858 | 3646375 | -1,28 | 1,62 | -2,9 | 0,00099 | 0,0172 | Intron (uc002qxz.4/78989, intron 1 of 7) | -3121 | ENSG00000 118004 | COLEC11 |
| chr2 | 237297343 | 237298777 | 0,78 | 4,29 | -3,51 | 0,000992 | 0,0172 | Intron (uc002vwb.3/79781, intron 11 of 18) | 113563 | ENSG00000132321 | IQCA1 |
| chr7 | 132125124 | 132125478 | -0,83 | 2,07 | -2,9 | 0,000994 | 0,0172 | Intron (uc003vra.4/91584, intron 3 of 31) | 135845 | ENSG00000221866 | PLXNA4 |
| chr10 | 61946236 | 61946793 | -0,55 | 2,85 | -3,39 | 0,001 | 0,0174 | Exon (uc001jky.3/288, exon 17 of 44) | -14075 | ENSG00000151150 | ANK3 |
| chr7 | 132127834 | 132128376 | -1,02 | 2,07 | -3,09 | 0,001 | 0,0173 | Intron (uc003vra.4/91584, intron 3 of 31) | 132947 | ENSG00000221866 | PLXNA4 |
| chr18 | 14486659 | 14488661 | -0,99 | 1,79 | -2,79 | 0,00101 | 0,0174 | Intron (uc010xai.2/440224, intron 2 of 2) | 10044 | ENSG00000265766 | CXADRP3 |
| chr13 | 94845530 | 94846053 | -1,28 | 1,61 | -2,88 | 0,00101 | 0,0174 | Intron (uc010tig.1/10082, intron 4 of 5) | 285883 | ENSG00000080166 | DCT |
| chr16 | 80666757 | 80667220 | -1,14 | 1,76 | -2,9 | 0,00101 | 0,0175 | Exon (uc002ffs.3/124359,exon3of7) | 91903 | ENSG00000168589 | DYNLRB2 |
| chr3 | 135741624 | 135742070 | -1,28 | 1,67 | -2,95 | 0,00101 | 0,0174 | Promoter (<=1kb) | 47 | ENSG00000073711 | PPP2R3 A |
| chr8 | 735253 | 737049 | 0,53 | 3,83 | -3,3 | 0,00102 | 0,0175 | Intron (uc003wpj.2/619343, intron 1 of 3) | 47666 | NA | NA |
| chr3 | 23643899 | 23645185 | 0 | 3,66 | -3,66 | 0,00102 | 0,0176 | Intron (uc021wtz.1/100616384, intron 1 of 1) | -202199 | ENSG00000170142 | UBE2E1 |
| chr3 | 180502981 | 180503533 | -1,28 | 1,58 | -2,85 | 0,00103 | 0,0177 | Intron (uc003fko.3/uc003fko.3, intron 3 of 13) | -47319 | ENSG00000284862 | CCDC39 |
| chr1 | 228603026 | 228605393 | 2,27 | 6,46 | -4,19 | 0,00103 | 0,0177 | Promoter (<=1kb) | 0 | ENSG00000162931 | TRIM17 |
| chr11 | 76629827 | 76630450 | 0,59 | 3,88 | -3,29 | 0,00104 | 0,0178 | Intron (uc010rsg.1/55331. intron 2 of 8) | 57844 | ENSG00000078124 | ACER3 |
| chr3 | 185406813 | 185407642 | -0,65 | 2,66 | -3,31 | 0,00104 | 0,0178 | 5'UTR | -23398 | ENSG00000163915 | IGF2BP2-AS1 |
| chr4 | 178810909 | 178811959 | 1,05 | 4,54 | -3,49 | 0,00104 | 0,0179 | Intron (uc010iru.3/285501, intron 2 of 5) | 160998 | ENSG00000231171 | LINC01098 |
| chrl 9 | 48784121 | 48784529 | -0,86 | 1,94 | -2,8 | 0,00104 | 0,0179 | Promoter (<= 1kb) | -367 | ENSG00000178150 | ZNF114 |
| chr2 | 47527535 | 47529019 | 0,61 | 4,05 | -3,44 | 0,00105 | 0,0179 | Intron (uc002rvu.1/uc002rvu.1, intron 1 of 2) | -67268 | ENSG00000119888 | EPCAM |
| chr4 | 8130183 | 8130727 | -1,28 | 1,59 | -2,86 | 0,00106 | 0,0181 | Promoter (<=1kb) | -794 | ENSG00000163995 | ABLIM2 |
| chr12 | 6343909 | 6345514 | 1,01 | 4,69 | -3,68 | 0,00106 | 0,0181 | 3'UTR | 34427 | ENSG00000010278 | CD9 |
| chr5 | 142163271 | 142164456 | 0,08 | 3,65 | -3,57 | 0,00107 | 0,0182 | Intron (uc0031mt.3/23092, intron 1 of 22) | 12979 | ENSG00000145819 | ARHGAP26 |
| chr3 | 65364840 | 65365389 | -1,17 | 1,73 | -2,9 | 0,00107 | 0,0182 | 3'UTR | 21990 | ENSG00000151276 | MAGI1 |
| chr1 | 201655010 | 201656471 | 0,57 | 4,13 | -3,56 | 0,00107 | 0,0181 | Intron (uc001gwu.3/89796, intron 1 of 28) | -32165 | ENSG00000264802 | MIR5191 |
| chr22 | 46483760 | 46484377 | -0,84 | 2,14 | -2,98 | 0,00107 | 0,0182 | Promoter (1-2kb) | 1883 | ENSG00000197182 | MIRLET7BHG |
| chr3 | 52304115 | 52307505 | 2,22 | 5,67 | -3,45 | 0,00107 | 0,0182 | Promoter (1-2kb) | -1738 | ENSG00000199150 | MIRLET7 G |
| chr2 | 198737947 | 198738366 | -1,28 | 1,6 | -2,87 | 0,00107 | 0,0182 | Intron (uc002uuv.4/5334, intron 1 of 6) | 68521 | ENSG00000115896 | PLCL1 |
| chr2 | 176996835 | 176997486 | -0,86 | 2,28 | -3,14 | 0,00109 | 0,0184 | Promoter (2-3kb) | 2367 | ENSG00000175879 | HOXD8 |
| chr10 | 100233928 | 100234847 | -0,66 | 2,22 | -2,89 | 0,00109 | 0,0184 | Intron (uc001kpn.2/60495, intron 11 of 11) | -27224 | ENSG00000107521 | HPS1 |
| chr14 | 57271863 | 57272203 | -1,28 | 1,21 | -2,49 | 0,00109 | 0,0185 | Promoter(<=1kb) | 178 | ENSG00000165588 | OTX2 |
| chr19 | 19245425 | 19246275 | 0,55 | 4,09 | -3,55 | 0,00109 | 0,0185 | Exon (uc002nlg.4/54929. exon 2 of 12) | 3035 | ENSG00000064545 | TMEM161A |
| chr14 | 95763271 | 95763745 | -1,13 | 1,83 | -2,96 | 0,0011 | 0,0185 | Intron (uc001vef.2/79789, intron 1 of 12) | 22500 | ENSG00000165959 | CLMN |
| chr2 | 147344678 | 147345371 | -0,63 | 2,58 | -3,21 | 0,0011 | 0,0186 | Promoter(<=1kb) | 53 | NA | PABPC1P2 |
| chr9 | 17761234 | 17761912 | 0,29 | 3,22 | -2,94 | 0,0011 | 0,0185 | 5'UTR | 25533 | ENSG00000107295 | SH3 GL2 |
| chr11 | 31071653 | 31072194 | -0,75 | 2,1 | -2,85 | 0,00111 | 0,0186 | Intron (uc009yjk.1/100506627, intron 8 of 25) | 56313 | NA | NA |
| chr5 | 9408707 | 9410266 | 0,6 | 4,7 | -4,11 | 0,00111 | 0,0186 | Intron (uc003jek.2/9037, intron 2 of 22) | 135967 | ENSG00000112902 | SEMA5A |
| chr1 | 102374243 | 102376553 | 1,13 | 4,83 | -3,7 | 0,00112 | 0,0187 | Intron (uc001dug.2/118427, intron 1 of 5) | 36676 | NA | DNAJA1P5 |
| chr2 | 163580889 | 163581555 | -0,75 | 2,11 | -2,86 | 0,00112 | 0,0187 | Intron (uc002uch.2/90134, intron 2 of 15) | 113702 | ENSG00000184611 | KCNH7 |
| chr3 | 65915835 | 65916826 | -0,87 | 2,29 | -3,15 | 0,00112 | 0,0187 | Intron (uc003dmm.3/9223, intron 1 of 24) | -22485 | ENSG00000151276 | MAGI1 |
| chr1 | 166916471 | 166917441 | 1,55 | 5,02 | -3,47 | 0,00113 | 0,0189 | Intron (uc001gdx.2/387597, intron 3 of 9) | 27120 | ENSG00000143195 | ILDR2 |
| chr1 | 24712803 | 24713412 | -0,2 | 2,97 | -3,16 | 0,00113 | 0,0189 | Intron (uc001bja.3/90529, intron 2 of 7) | 4757 | ENSG0000000 1460 | STPG1 |
| chr2 | 237466969 | 237467314 | -1,28 | 1,53 | -2,81 | 0,00114 | 0,019 | Promoter (2-3kb) | -2110 | ENSG00000144476 | ACKR3 |
| chr3 | 37754970 | 37755444 | -1,28 | 1,6 | -2,88 | 0,00114 | 0,0191 | Intron (uc003chd.3/3680, intron 18 of 27) | -148225 | ENSG00000144677 | CTDSPL |
| chr9 | 116060971 | 116063374 | 1,7 | 5,09 | -3,39 | 0,00114 | 0,019 | Promoter (<= 1kb) | 0 | ENSG00000165188 | RNF183 |
| chr2 | 215386477 | 215387463 | 0,77 | 4,35 | -3,57 | 0,00114 | 0,019 | Intron (uc002vet.2/402117, intron 3 of 3) | 11571 | ENSG00000224257 | VWC2L-IT1 |
| chr6 | 36802409 | 36802973 | -0,71 | 2,25 | -2,96 | 0,00115 | 0,0191 | Promoter (1-2kb) | -1714 | ENSG00000124772 | CPNE5 |
| chr20 | 25128746 | 25130080 | 1,84 | 5,59 | -3,75 | 0,00115 | 0,0191 | Promoter(<=lkb) | 0 | ENSG00000230725 | LOC284798 |
| chr22 | 41909106 | 41909405 | -1,28 | 1,63 | -2,9 | 0,00116 | 0,0193 | Intron (uc003bac.3/50, intron 4 of 17) | 31071 | ENSG00000100413 | POLR3H |
| chr21 | 45299011 | 45300538 | 0,92 | 4,85 | -3,93 | 0,00117 | 0,0194 | Intron (uc002zdv.3/56894, intron 1 of 9) | 13895 | ENSG00000160216 | AGPAT3 |
| chr5 | 42772471 | 42772827 | -1,28 | 1,61 | -2,89 | 0,00117 | 0,0193 | Intron (uc003jmx.3/100129792, intron 4 of 8) | 15551 | ENSG00000198865 | CCDC152 |
| chr3 | 82363264 | 82363877 | -1,28 | 1,66 | -2,94 | 0,00117 | 0,0194 | Intron (uc003dqh.1/uc003dqh.1, intron 3 of 8) | -552314 | ENSG00000114480 | GBE1 |
| chr13 | 100361331 | 100362509 | -0,27 | 3,03 | -3,3 | 0,00117 | 0,0194 | Intron (uc010tix.2/171425, intron 1 of 5) | 66018 | ENSG00000263615 | MIR4306 |
| chr22 | 17228004 | 17230485 | 3,36 | 6,86 | -3,5 | 0,00118 | 0,0195 | Exon (uc002zlu.3/uc002zlu.3, exon 1 of 2) | -71574 | NA | ANKRD62P1-PARP4P3 |
| chr7 | 123251265 | 123251664 | -1,28 | 1,43 | -2,7 | 0,00118 | 0,0194 | Promoter (2-3kb) | 2153 | ENSG00000146809 | ASB15 |
| chr14 | 25075839 | 25076365 | -1,12 | 1,7 | -2,81 | 0,00118 | 0,0195 | Promoter (2-3kb) | 2561 | ENSG00000100450 | GZMH |
| chr10 | 3122696 | 3123318 | -0,41 | 2,64 | -3,06 | 0,00118 | 0,0195 | Intron (uc001igp.3/5214, intron 1 of 21) | 11266 | ENSG00000067057 | PFKP |
| chr12 | 124762020 | 124762764 | 0,57 | 3,2 | -2,63 | 0,00118 | 0,0195 | Intron (uc021rfv.1/100533183, intron 3 of 4) | -10946 | ENSG00000178882 | RFLNA |
| chr18 | 60396084 | 60398504 | 1,66 | 5,62 | -3,96 | 0,00119 | 0,0196 | Intron (uc021ule.1/23239, intron 1 of 16) | 13412 | ENSG00000081913 | PHLPP1 |
| chr2 | 1127992 | 1130131 | -0,19 | 3,47 | -3,66 | 0,00119 | 0,0196 | Intron (uc002qwp.3/54221, intron 5 of 7) | 181438 | ENSG00000172554 | SNTG2 |
| chr10 | 128615684 | 128616017 | -1,05 | 1,65 | -2,7 | 0,0012 | 0,0197 | Intron (uc0011jt.3/1793, intron 1 of 51) | 21661 | ENSG00000150760 | DOCK1 |
| chr1 | 17371689 | 17372949 | 1,31 | 4,86 | -3,54 | 0,0012 | 0,0197 | Intron (uc001bae.3/6390, intron 1 of 7) | 7716 | ENSG00000117118 | SDHB |
| chr18 | 56152459 | 56153347 | -0,4 | 2,45 | -2,85 | 0,00121 | 0,0198 | Intron (uc0021hj.4/115701, intron 12 of 12) | -34075 | ENSG00000207778 | MIR3591 |
| chr21 | 36691834 | 36692624 | -0,2 | 3,21 | -3,41 | 0,00122 | 0,0199 | Intron (uc002vut.1/861. intron 7 of 10) | 260603 | ENSG00000159216 | LOC10050640 3 |
| chr7 | 23800857 | 23801429 | -0,63 | 2,47 | -3,1 | 0,00122 | 0,02 | Intron (uc003sws.5/56164, intron 10 of 23) | -26170 | ENSG00000196335 | STK31 |
| chr16 | 4012465 | 4014135 | 0,79 | 4,81 | -4,02 | 0,00123 | 0,0201 | 3'UTR | -82344 | ENSG00000005339 | CREBBP |
| chr3 | 9142859 | 9143255 | -0,87 | 1,91 | -2,78 | 0,00124 | 0,0202 | Intron (uc003brf.2/9901, intron 3 of 21) | -19888 | ENSG00000196220 | SRGAP3 |
| chr17 | 53240831 | 53241279 | -1,28 | 1,56 | -2,84 | 0,00125 | 0,0203 | 3'UTR | -101042 | ENSG00000108924 | HLF |
| chr3 | 178096610 | 178097727 | 0,24 | 3,84 | -3,6 | 0,00126 | 0,0204 | Intron (uc003fja.1/uc003fja.1,intron 1 of 1) | -39262 | ENSG00000223941 | LINC01014 |
| chr17 | 71397357 | 71398910 | 3,02 | 6,58 | -3,56 | 0,00126 | 0,0204 | Promoter (<=1kb) | 0 | ENSG00000069188 | SDK2 |
| chr19 | 51206567 | 51207579 | -0,22 | 3,15 | -3,37 | 0,00126 | 0,0205 | Exon (uc002psx.1/50944, exon 9 of 23) | 12616 | ENSG00000161681 | SHANK1 |
| chr1 | 149333621 | 149334439 | -1,14 | 1,57 | -2,72 | 0,00127 | 0,0205 | Exon (uc021oxn.1/uc021oxn.1, exon 1 of 1) | -34855 | ENSG00000265531 | FCGR1 CP |
| chr5 | 124067182 | 124067638 | -1,28 | 1,47 | -2,74 | 0,00127 | 0,0205 | Intron (uc003ktq.1/57507, intron 1 of 8) | 13227 | ENSG00000168916 | ZNF608 |
| chr17 | 30809739 | 30810015 | -1,16 | 1,23 | -2,39 | 0,00128 | 0,0206 | 3'UTR | -4090 | ENSG00000176749 | CDK5R1 |
| chr12 | 93401474 | 93401983 | -0,77 | 2,01 | -2,78 | 0,00128 | 0,0207 | Intron (uc021rbu.1/643339, intron 3 of 4) | -78367 | ENSG00000102189 | EEA1 |
| chr1 | 66648591 | 66649086 | -0,57 | 2,3 | -2,87 | 0,00128 | 0,0207 | Intron (uc001ojn.1/5091. intron 1 of 20) | 23715 | ENSG00000173621 | LRFN4 |
| chr2 | 88406899 | 88407583 | -0,46 | 2,19 | -2,65 | 0,00128 | 0,0207 | Intron (uc002ssq.2/150572, intron 2 of 2) | 16368 | ENSG00000115593 | SMYD1 |
| chr7 | 130753752 | 130754089 | -0,81 | 1,87 | -2,69 | 0,00129 | 0,0208 | Intron (uc011kpk.2/378805, intron 1 of 2) | 37700 | ENSG00000231721 | LINC-PINT |
| chr6 | 2856276 | 2859436 | 1,2 | 5,32 | -4,12 | 0,0013 | 0,0209 | Promoter (1-2kb) | -1935 | ENSG00000266750 | MIR4645 |
| chr5 | 58883288 | 58883876 | -0,89 | 2,14 | -3,03 | 0,0013 | 0,0209 | Promoter(<=lkb) | -964 | ENSG00000113448 | PDE4D |
| chr3 | 94838685 | 94839552 | -0,38 | 2,87 | -3,25 | 0,00131 | 0,021 | Intron (uc003drn.3/255025, intron 1 of 3) | 181578 | ENSG00000239589 | LINC00879 |
| chr8 | 119960575 | 119961006 | -0,99 | 1,95 | -2,94 | 0,00131 | 0,021 | Intron (uc003von.4/4982, intron 1 of 4) | 3377 | ENSG00000164761 | TNFRSF11B |
| chr1 | 226384043 | 226384954 | -0,14 | 2,89 | -3,03 | 0,00132 | 0,0212 | Exon (uc031psl.1/uc031psl.1, exon 3 of 3) | -9620 | ENSG00000182827 | ACBD3 |
| chr5 | 49726997 | 49727791 | -0,78 | 1,77 | -2,55 | 0,00132 | 0,0211 | Promoter (2-3kb) | -2475 | ENSG00000170571 | EMB |
| chr1 | 19186773 | 19187175 | -1,28 | 1,54 | -2,82 | 0,00133 | 0,0213 | Intron (uc001mpi.3/54503, intron 13 of 16) | 44943 | ENSG00000129170 | CSRP3 |
| chr8 | 63680834 | 63681231 | -1,28 | 1,5 | -2,78 | 0,00133 | 0,0213 | Intron (uc010lvq.1/286183, intron 4 of 5) | -209189 | ENSG00000274956 | NKAIN3 -IT1 |
| chr10 | 50857168 | 50858221 | 0,96 | 3,96 | -3 | 0,00134 | 0,0214 | Exon (uc001jhv.1/1103, exon 10 of 15) | -29463 | ENSG00000178645 | C10orf53 |
| chr6 | 53674646 | 53676769 | 1,56 | 5,06 | -3,49 | 0,00134 | 0,0214 | Intron (uc003pcd.1/55227, intron 1 of 13) | 15112 | ENSG00000137269 | LRRC1 |
| chr2 | 33444263 | 33444679 | -1,28 | 1,59 | -2,87 | 0,00134 | 0,0214 | Intron (uc021vft.1/4052, intron 8 of 33) | -71872 | ENSG00000049323 | LTBP1 |
| chr1 | 16736008 | 16736404 | -0,98 | 1,8 | -2,77 | 0,00134 | 0,0214 | Exon (uc001ayl.1/374955. exon 7 of 17) | 27515 | ENSG00000187144 | SPATA21 |
| chr7 | 138528261 | 138530557 | 1,76 | 6,01 | -4,25 | 0,00135 | 0,0214 | Exon (uc011kqi.2/57670,exon 10 of 12) | -45320 | ENSG00000105929 | ATP6V0A4 |
| chr17 | 28313451 | 28314022 | -1,01 | 1,68 | -2,7 | 0,00135 | 0,0214 | Intron (uc010wbi.1/374786, intron 3 of 10) | 17642 | ENSG00000176927 | EFCAB5 |
| chr1 | 143879449 | 143880192 | -1,28 | 1,37 | -2,64 | 0,00135 | 0,0214 | Exon (uc021otg.1/uc021otg.1, exon 1 of 1) | 32951 | ENSG00000215784 | FAM72D |
| chr6 | 108496358 | 108497796 | 0,21 | 3,59 | -3,38 | 0,00135 | 0,0215 | Exon (uc003psg.3/7101, exon 4 of 9) | 9143 | ENSG00000112333 | NR2E1 |
| chr3 | 179731785 | 179732744 | 0,86 | 4,32 | -3,45 | 0,00135 | 0,0214 | Intron (uc003fki.2/51555, intron 1 of 14) | 22097 | ENSG00000114757 | PEX5L |
| chr4 | 79228024 | 79229793 | 0,52 | 4,33 | -3,81 | 0,00136 | 0,0215 | Exon (uc003hkw.3/80144, exon 15 of 42) | 20478 | ENSG00000138759 | FRAS1 |
| chr10 | 117669732 | 117670175 | -1,28 | 1,49 | -2,76 | 0,00136 | 0,0215 | Intron (uc0011cg.3/26033, intron 28 of 28) | 361644 | ENSG00000151892 | GFRA1 |
| chr1 | 19496340 | 19497368 | -0,05 | 3,58 | -3,63 | 0,00136 | 0,0216 | Intron (uc001mpp.3/89797, intron 1 of 37) | 31408 | ENSG00000255043 | NAV2-AS5 |
| chr3 | 186941926 | 186942182 | -1,28 | 1,02 | -2,3 | 0,00139 | 0,0219 | Intron (uc003frh.2/5648, intron 13 of 15) | 26652 | ENSG00000175077 | RTP1 |
| chr15 | 38509352 | 38510125 | -0,13 | 2,76 | -2,88 | 0,00141 | 0,0222 | Intron (uc001zjy.3/uc001zjy.3, intron 2 of 3) | -34927 | ENSG00000166068 | SPRED1 |
| chr3 | 79494713 | 79495168 | -1,16 | 1,4 | -2,56 | 0,00143 | 0,0223 | Intron (uc003dqe.2/6091, intron 2 of 30) | 321891 | ENSG00000169855 | ROBO1 |
| chr4 | 15356793 | 15357501 | -0,09 | 3,37 | -3,46 | 0,00144 | 0,0226 | Intron (uc003gno.3/114905, intron 1 of 2) | 15233 | ENSG00000163145 | C1QTNF7 |
| chr1 | 111174096 | 111174766 | -0,5 | 2,74 | -3,24 | 0,00145 | 0,0226 | Promoter (1-2kb) | 1004 | ENSG00000196167 | COLCA1 |
| chr2 | 154982820 | 154983501 | -0,53 | 2,45 | -2,98 | 0,00145 | 0,0226 | Intron (uc002tvr.4/114805, intron 3 of 12) | -13349 | ENSG00000144278 | GALNT13 |
| chr15 | 52623450 | 52624322 | -0,13 | 2,93 | -3,07 | 0,00145 | 0,0226 | Intron (uc010ugd.1/4644, intron 4 of 11) | 8269 | ENSG00000197535 | MYO5A |
| chr5 | 41493090 | 41494467 | 1,61 | 5,12 | -3,51 | 0,00145 | 0,0226 | Intron (uc003jmm.1/345557, intron 1 of 2) | 16263 | ENSG00000182836 | PLCXD3 |
| chr1 | 23762933 | 23764014 | 0 | 3,31 | -3,3 | 0,00146 | 0,0227 | Promoter (<=1kb) | 0 | ENSG00000088280 | ASAP3 |
| chr17 | 57711263 | 57712160 | 0,3 | 3,26 | -2,96 | 0,00146 | 0,0227 | Intron (uc002ixp.3/1213, intron 1 of 30) | 14213 | ENSG00000141367 | CLTC |
| chr5 | 66483141 | 66494571 | 4,87 | 9,44 | -4,57 | 0,00147 | 0,0227 | Promoter (<=1kb) | 0 | ENSG00000134061 | CD180 |
| chr18 | 65394219 | 65395525 | 0,82 | 4,26 | -3,44 | 0,00147 | 0,0227 | Intron (uc021ulh.1/643542, intron 1 of 4) | -210252 | ENSG00000171451 | DSEL |
| chr3 | 51891336 | 51892357 | -1,05 | 1,81 | -2,86 | 0,00147 | 0,0227 | Intron(uc003dbq.5/132141,intron3 of 3) | -3288 | ENSG00000184345 | IQCF2 |
| chr6 | 13068783 | 13069298 | -0,58 | 2,6 | -3,18 | 0,00147 | 0,0227 | Intron (uc011dir.2/221692, intron 5 of 11) | 226520 | ENSG00000215022 | LOC10013035 7 |
| chr5 | 14762534 | 14763502 | 0,36 | 3,65 | -3,29 | 0,00147 | 0,0228 | Intron (uc003jfm.4/56172, intron 2 of 11) | 49731 | NA | LOC10013074 4 |
| chrX | 119185461 | 119186496 | -0,84 | 1,78 | -2,62 | 0,00147 | 0,0227 | Intron (uc004esi.1/uc004esi.1, intron 1 of 4) | 25211 | ENSG00000203989 | RHOXF2B |
| chr13 | 36001642 | 36002549 | -1,28 | 1,45 | -2,72 | 0,0015 | 0,0231 | Intron (uc021ric.1/26960, intron 37 of 56) | -43098 | ENSG00000172915 | NBEA |
| chr18 | 7746793 | 7747581 | -0,41 | 2,75 | -3,16 | 0,0015 | 0,0232 | Intron (uc002knn.4/5797, intron 1 of 30) | 179479 | ENSG00000173482 | PTPRM |
| chr15 | 61238099 | 61239028 | 0,4 | 3,83 | -3,43 | 0,0015 | 0,0232 | Intron (uc002agx.3/6095, intron 1 of 10) | 282474 | ENSG00000069667 | RORA |
| chr12 | 99162134 | 99162777 | -0,49 | 2,32 | -2,81 | 0,00151 | 0,0233 | Intron (uc010svd.2/56899, intron 8 of 9) | 96821 | ENSG00000120868 | APAF1 |
| chr17 | 29032925 | 29033681 | -0,71 | 1,92 | -2,63 | 0,00151 | 0,0232 | Promoter (2-3kb) | -2945 | NA | SUZ12P1 |
| chr15 | 31427793 | 31428470 | -0,32 | 2,72 | -3,04 | 0,00153 | 0,0234 | Intron (uc021sia.1/4308, intron 1 of 26) | 25006 | ENSG00000134160 | TRPM1 |
| chr2 | 230350592 | 230351279 | -0,58 | 2,59 | -3,17 | 0,00154 | 0,0235 | Intron (uc002vpv.3/92737, intron 6 of 12) | 100336 | ENSG00000187957 | DNER |
| chr7 | 128549704 | 128550781 | 1,85 | 5,11 | -3,27 | 0,00154 | 0,0235 | Promoter(<=1kb) | 0 | ENSG00000135253 | KCP |
| chr9 | 139116975 | 139118140 | 0,22 | 3,95 | -3,72 | 0,00154 | 0,0235 | Intron (uc010nbi.2/169714, intron 2 of 11) | 19547 | ENSG00000165661 | QSOX2 |
| chr10 | 105605562 | 105606305 | 0,04 | 2,64 | -2,61 | 0,00154 | 0,0236 | Intron (uc001kxj.1/9644, intron 1 of 13) | 8859 | ENSG00000107957 | SH3PXD2A |
| chr1 1 | 129697991 | 129698675 | -0,02 | 2,85 | -2,88 | 0,00154 | 0,0236 | Intron (uc001qfe.1/120224, intron 1 of 5) | 12250 | ENSG00000151715 | TMEM45B |
| chr13 | 34064737 | 34065061 | -1,18 | 1,38 | -2,57 | 0,00155 | 0,0236 | Intron (uc001uux.3/90627, intron 4 of 17) | -139970 | ENSG00000133121 | STARD13 |
| chr1 | 19180614 | 19181656 | 1 | 4,46 | -3,45 | 0,00155 | 0,0236 | Exon (uc001bba.1/80834, exon 3 of 6) | 4499 | ENSG00000179002 | TAS1R2 |
| chr21 | 36072275 | 36073337 | 0,09 | 3,56 | -3,47 | 0,00157 | 0,0239 | Intron (uc002vuf.1/54102, intron 1 of 5) | 30587 | ENSG00000159212 | CLIC6 |
| chr12 | 102284998 | 102286129 | 0,18 | 3,37 | -3,19 | 0,00157 | 0,0239 | Intron (uc001tix.3/55332, intron 1 of 6) | 13893 | ENSG00000136048 | DRAM1 |
| chr14 | 67269497 | 67270166 | -0,58 | 2,19 | -2,78 | 0,00157 | 0,0239 | Intron (uc001xiw.3/10243, intron 3 of 10) | -19797 | ENSG00000171723 | GPHN |
| chr5 | 35744933 | 35745949 | 0,13 | 3,24 | -3,11 | 0,00157 | 0,0239 | Intron (uc003jjo.3/79925, intron 23 of 36) | -28046 | ENSG00000152582 | SPEF2 |
| chr2 | 144418932 | 144420058 | 0,28 | 3,39 | -3,11 | 0,00158 | 0,024 | Intron (uc002tvm.4/55843, intron 12 of 13) | 225769 | ENSG00000075884 | ARHGAP15 |
| chr9 | 130460829 | 130462211 | 2,36 | 5,86 | -3,49 | 0,00158 | 0,024 | Intron (uc004brm.3/uc004brm.3, intron 1 of 2) | -7060 | ENSG00000160401 | CFAP157 |
| chr19 | 3552653 | 3553540 | 0,05 | 3,37 | -3,32 | 0,00158 | 0,024 | Intron (uc0021xw.3/126321, intron 1 of 10) | 3863 | ENSG00000161091 | MFSD12 |
| chr3 | 49282194 | 49283507 | 0,69 | 4,25 | -3,56 | 0,00158 | 0,024 | 3'UTR | -28046 | ENSG00000264633 | MIR4271 |
| chr1 | 226420416 | 226423934 | 1,94 | 6,02 | -4,09 | 0,00159 | 0,0241 | 3'UTR | 9033 | ENSG00000185155 | MIXL1 |
| chr2 | 183363466 | 183363929 | -1,28 | 1,4 | -2,68 | 0,00159 | 0,0241 | Intron (uc010zfp.2/5136, intron 1 of 15) | 23184 | ENSG00000115252 | PDE1A |
| chr1 | 110254265 | 110255044 | -1,28 | 1,4 | -2,67 | 0,0016 | 0,0242 | Promoter (<= lkb) | 0 | ENSG00000134201 | GSTM5 |
| chr17 | 17029303 | 17030804 | 0,77 | 4,68 | -3,91 | 0,0016 | 0,0241 | Exon (uc002gqu.2/23164, exon 4 of 24) | -8729 | ENSG00000133030 | MPRIP |
| chr13 | 31875939 | 31877466 | 0,04 | 3,42 | -3,38 | 0,00161 | 0,0242 | Intron (uc010aaz.3/145173, intron 12 of 14) | 41404 | ENSG00000187676 | B3GLCT |
| chr20 | 60053136 | 60053744 | -1,07 | 1,8 | -2,87 | 0,00161 | 0,0242 | Intron (uc002vbn.2/1002, intron 2 of 15) | -20733 | ENSG00000179242 | CDH4 |
| chr15 | 52941828 | 52944877 | 2,43 | 6,26 | -3,83 | 0,00162 | 0,0243 | Promoter (<=lkb) | 0 | ENSG00000047346 | FAM214A |
| chr9 | 10449128 | 10449702 | -0,99 | 1,5 | -2,49 | 0,00163 | 0,0245 | Intron (uc003zkk.3/5789, intron 2 of 45) | 163021 | ENSG00000153707 | PTPRD |
| chr9 | 109667736 | 109668800 | -0,26 | 3,14 | -3,4 | 0,00163 | 0,0246 | Intron (uc004bcz.3/58499, intron 1 of 12) | -17614 | ENSG00000148143 | ZNF462 |
| chr15 | 66886599 | 66886935 | -0,62 | 2,02 | -2,63 | 0,00165 | 0,0248 | Intron (uc002aqe.3/uc002aqe.3, intron 1 of 4) | -28282 | ENSG00000188501 | LCTL |
| chr1 | 68772638 | 68774901 | 1,79 | 5,75 | -3,96 | 0,00165 | 0,0248 | 3'UTR | 5949 | ENSG00000172935 | MRGPRF |
| chr1 | 167078457 | 167078803 | -1,28 | 1,41 | -2,68 | 0,00166 | 0,0249 | Intron (uc001geb.1/92235, intron 1 of 4) | 14386 | ENSG00000198842 | DUSP27 |
| chr8 | 91908420 | 91909626 | 0,25 | 3,31 | -3,07 | 0,00166 | 0,0249 | Intron (uc011lgg.2/64168, intron 5 of 12) | -43341 | ENSG00000123119 | NECAB1 |
| chr7 | 78308634 | 78309253 | -0,79 | 2,18 | -2,96 | 0,00168 | 0,0251 | Intron (uc003ugx.3/9863, intron 2 of 21) | 91385 | ENSG00000187391 | MAGI2 |
| chr12 | 110270818 | 110271711 | 1,25 | 4,67 | -3,42 | 0,00168 | 0,025 | Promoter(<=lkb) | 0 | ENSG00000263510 | MIR4497 |
| chr2 | 88260592 | 88261441 | -0,78 | 1,82 | -2,59 | 0,00168 | 0,0251 | Intron (uc021vkn.1/400966, intron 1 of 22) | 23868 | ENSG00000187627 | RGPD1 |
| chr1 | 235759120 | 235760018 | -0,01 | 3,13 | -3,14 | 0,00169 | 0,0252 | Intron (uc001hxe.4/2786, intron 1 of 3) | 53275 | ENSG00000168243 | GNG4 |
| chr10 | 98155882 | 98156291 | -0,28 | 2,5 | -2,78 | 0,00169 | 0,0252 | Intron (uc001kml.2/7093, intron 11 of 20) | -36790 | ENSG00000197430 | OPALIN |
| chrY | 4914262 | 4915520 | -1,28 | 1,35 | -2,63 | 0,00169 | 0,0253 | Intron (uc010nwg.1/83259, intron 3 of 4) | -8611 | ENSG00000099715 | PCDH11Y |
| chr21 | 36875892 | 36876271 | -0,99 | 1,61 | -2,61 | 0,0017 | 0,0253 | Intron (uc002vut.1/861, intron 6 of 10) | 76956 | ENSG00000159216 | LOC10050640 3 |
| chr17 | 17012144 | 17012455 | -1,28 | 1,24 | -2,51 | 0,00171 | 0,0254 | Intron (uc002gqu.2/23164, intron 3 of 23) | -27078 | ENSG00000133030 | MPRIP |
| chr15 | 28106924 | 28107722 | -0,66 | 2,57 | -3,23 | 0,00171 | 0,0254 | Intron (uc001zbh.4/4948, intron21 of 23) | 236736 | ENSG00000104044 | OCA2 |
| chr20 | 55033240 | 55034070 | 1,41 | 5,1 | -3,68 | 0,00172 | 0,0255 | 3'UTR | -9577 | ENSGO0000022277 | RTF2 |
| chr15 | 28200020 | 28200635 | -0,75 | 2,04 | -2,79 | 0,00174 | 0,0258 | Exon (uc001zbh.4/4948, exon 17 of 24) | 143823 | ENSG00000104044 | OCA2 |
| chr14 | 25476566 | 25476914 | -1,28 | 1,23 | -2,51 | 0,00174 | 0,0258 | Intron (uc001wpu.3/29091, intron 1 of 5) | 42181 | ENSG00000168952 | STXBP6 |
| chr17 | 77300878 | 77301431 | -0,25 | 2,48 | -2,73 | 0,00175 | 0,0259 | Intron (uc010dhs.4/146713, intron 3 of 14) | 118771 | ENSG00000167281 | RBFOX3 |
| chr3 | 32011655 | 32012562 | -0,69 | 2,33 | -3,02 | 0,00175 | 0,0258 | Intron (uc011axf.2/114884, intron 1 of 10) | -10704 | ENSG00000197385 | ZNF860 |
| chr4 | 102987079 | 102987878 | -0,49 | 2,72 | -3,21 | 0,00176 | 0,0259 | Intron (uc003hvx.4/55024, intron 17 of 20) | 252096 | ENSG00000153064 | BANK1 |
| chr7 | 111835943 | 111836474 | -0,4 | 2,67 | -3,07 | 0,00176 | 0,0259 | Intron (uc003vfx.3/9732, intron 1 of 51) | 9988 | ENSG00000128512 | DOCK4 |
| chr21 | 38301906 | 38302308 | -1,28 | 1,41 | -2,68 | 0,00176 | 0,0259 | Intron (uc010gnb.3/3141, intron 6 of 11) | 36648 | ENSG00000159267 | HLCS |
| chr3 | 156001808 | 156003420 | 0,13 | 3,42 | -3,29 | 0,00176 | 0,026 | Intron (uc003far.2/7881, intron 1 of 13) | -5356 | ENSG00000169282 | KCNAB1 |
| chr1 | 64738569 | 64740174 | 2,45 | 6,29 | -3,84 | 0,00176 | 0,0259 | Promoter(<=lkb) | 0 | ENSG00000168070 | MAJIN |
| chr12 | 21724672 | 21725147 | -1,1 | 1,67 | -2,77 | 0,00178 | 0,0262 | Intron (uc001rfb.3/2998, intron 4 of 15) | 32634 | ENSG00000111713 | GYS2 |
| chr13 | 84943278 | 84944110 | -0,14 | 3,07 | -3,2 | 0,00178 | 0,0262 | Intron (uc031qmn.1/100874128, intron 3 of 6) | 228541 | NA | LINC00333 |
| chr14 | 106405865 | 106406628 | -0,72 | 2,06 | -2,78 | 0,00179 | 0,0263 | Exon (uc031qqx.1/uc031qqx.1, exon 3479 of 5065) | 22027 | ENSG00000226777 | FAM30A |
| chr21 | 41384875 | 41385457 | -0,68 | 2,37 | -3,05 | 0,00179 | 0,0263 | 3'UTR | 145528 | ENSG00000183036 | PCP4 |
| chr15 | 20487123 | 20488842 | 3,22 | 6,67 | -3,45 | 0,0018 | 0,0264 | Promoter (<= 1kb) | 0 | ENSG00000259156 | CHEK2P2 |
| chr16 | 65584938 | 65585991 | 0,02 | 2,82 | -2,81 | 0,0018 | 0,0264 | Intron (uc010cdp.1/283867, intron 1 of 5) | 19837 | ENSG00000261742 | LINC00922 |
| chr6 | 130573322 | 130574124 | -0,58 | 2,46 | -3,04 | 0,00181 | 0,0266 | Intron (uc003qbx.4/154075, intron 2 of 13) | -29223 | ENSG00000164483 | SAMD3 |
| chr1 | 197564161 | 197564915 | 0,23 | 3,35 | -3,12 | 0,00182 | 0,0267 | Exon (uc010ppf.2/163486, exon 13 of 20) | 62584 | ENSG00000213047 | DENND1B |
| chr7 | 69660912 | 69663369 | 1,02 | 4,87 | -3,85 | 0,00183 | 0,0267 | Intron (uc003tvv.4/26053, intron 4 of 4) | -567879 | ENSG00000158321 | AUTS2 |
| chr2 | 179188245 | 179189290 | 0,18 | 3,29 | -3,11 | 0,00183 | 0,0268 | Exon (uc002ulw.3/114880, exon 4 of 14) | 3274 | ENSG00000079156 | OSBPL6 |
| chr10 | 117638944 | 117639449 | -0,92 | 1,69 | -2,61 | 0,00184 | 0,0269 | Intron (uc001lcg.3/26033, intron 28 of 28) | 392370 | ENSG00000151892 | GFRA1 |
| chr1 | 61639696 | 61640390 | -0,63 | 2,28 | -2,91 | 0,00184 | 0,0269 | Intron (uc001czv.3/4774, intron 2 of 10) | 91716 | ENSG00000162599 | NFIA |
| chr1 | 226107714 | 226109612 | 0,99 | 4,42 | -3,44 | 0,00184 | 0,0269 | Promoter(<=1kb) | 836 | ENSG00000143811 | PYCR2 |
| chr12 | 119548286 | 119548691 | -0,96 | 1,67 | -2,64 | 0,00185 | 0,027 | Intron (uc001txa.2/84530, intron 2 of 12) | -67904 | ENSG00000152137 | HSPB8 |
| chr1 | 243676346 | 243678276 | 1,35 | 5,12 | -3,77 | 0,00185 | 0,027 | Intron (uc001hzz.1/10000, intron 11 of 13) | 166868 | ENSG0000026520 1 | MIR4677 |
| chr7 | 75204556 | 75206070 | 0,85 | 4,62 | -3,77 | 0,00186 | 0,0271 | Intron (uc003uds.2/3092, intron 7 of 30) | -47103 | ENSG00000127957 | PMS2P3 |
| chr1 | 225611780 | 225612072 | -1,13 | 1,58 | -2,71 | 0,00187 | 0,0272 | 5'UTR | 3743 | ENSG00000143815 | LBR |
| chr10 | 12701616 | 12702275 | -1,13 | 1,52 | -2,66 | 0,00187 | 0,0272 | Intron (uc001iln.3/57118, intron 2 of 9) | 80864 | ENSG00000263584 | MIR4480 |
| chr7 | 133983077 | 133984919 | 2,92 | 5,92 | -2,99 | 0,00188 | 0,0273 | 3'UTR | 16908 | ENSG00000205060 | SLC35B4 |
| chr10 | 94822065 | 94823240 | 0,53 | 4,28 | -3,75 | 0,0019 | 0,0276 | Promoter (1-2kb) | 1044 | ENSG00000187553 | CYP26C1 |
| chr3 | 94840621 | 94841544 | 0,12 | 3,23 | -3,11 | 0,0019 | 0,0276 | Intron (uc003drn.3/255025, intron 1 of 3) | 183514 | ENSG00000239589 | LINC00879 |
| chr19 | 435153 | 435620 | -0,79 | 2,04 | -2,83 | 0,0019 | 0,0276 | Intron (uc0021oq.4/25759, intron 7 of 12) | 25376 | ENSG00000129946 | SHC2 |
| chr19 | 49078176 | 49078699 | -0,55 | 2,24 | -2,8 | 0,00192 | 0,0277 | Promoter(<=lkb) | -294 | ENSGO0000088002 | SULT2B1 |
| chr1 | 20091425 | 20092065 | -0,69 | 2,16 | -2,85 | 0,00193 | 0,0279 | Intron (uc001bcn.3/255104, intron 6 of 15) | 33772 | ENSG00000162542 | TMCO4 |
| chr1 | 171059516 | 171061975 | 1,3 | 5,05 | -3,75 | 0,00194 | 0,0279 | Promoter(<=lkb) | 0 | ENSG00000007933 | FMO3 |
| chr3 | 150821363 | 150821872 | -0,85 | 2,16 | -3,01 | 0,00194 | 0,028 | Intron (uc003eym.1/116931, intron 2 of 6) | 16778 | ENSG00000144893 | MED12L |
| chr17 | 79225140 | 79225532 | -1,28 | 1,37 | -2,64 | 0,00194 | 0,028 | Exon (uc002jzv.1/124565, exon 12 of 14) | -3705 | ENSG00000157637 | SLC38A10 |
| chr20 | 5176693 | 5177184 | -0,93 | 1,99 | -2,93 | 0,00195 | 0,028 | 3'UTR | 17230 | ENSG00000101290 | CDS2 |
| chr15 | 20960044 | 20962353 | -0,01 | 2,88 | -2,89 | 0,00195 | 0,028 | Promoter(<=1kb) | 0 | NA | NBEAP1 |
| chr12 | 24209999 | 24210252 | -1,28 | 1,19 | -2,46 | 0,00195 | 0,0281 | Intron (uc001rfx.4/6660, intron 4 of 17) | -106033 | ENSG00000134532 | SOX5 |
| chr20 | 55904332 | 55905376 | 0,25 | 3,75 | -3,5 | 0,00195 | 0,028 | Promoter(<=lkb) | 0 | ENSG00000054796 | SPO11 |
| chr5 | 122430139 | 122431479 | 1,02 | 4,27 | -3,25 | 0,00196 | 0,0281 | Intron (uc003kti.3/93166, intron 2 of 7) | 4444 | ENSG00000061455 | PRDM6 |
| chr5 | 124070738 | 124071520 | 0,13 | 3,17 | -3,04 | 0,00196 | 0,0282 | Intron (uc003ktq.1/57507, intron 1 of 8) | 9345 | ENSG00000168916 | ZNF608 |
| chr1 | 11766994 | 11767623 | -0,63 | 2,09 | -2,72 | 0,00197 | 0,0283 | Intron (uc001asr.1/374946, intron 2 of 6) | 15213 | ENSG00000162490 | DRAXIN |
| chr3 | 42743795 | 42744297 | -0,87 | 1,98 | -2,85 | 0,00197 | 0,0282 | Promoter (<=lkb) | 22 | ENSG00000010282 | HHATL |
| chr17 | 31542538 | 31543271 | -0,62 | 2,33 | -2,95 | 0,00198 | 0,0283 | Intron (uc002hht.3/40, intron 1 of 9) | 76735 | ENSG00000108684 | ASIC2 |
| chr17 | 74415272 | 74417370 | 4,14 | 6,85 | -2,71 | 0,00198 | 0,0283 | Intron (uc002jrm.4/63893, intron 1 of 17) | -19270 | ENSG00000175931 | UBE20 |
| chr7 | 82412633 | 82412954 | -1,14 | 1,34 | -2,48 | 0,00199 | 0,0284 | Intron (uc003uhx.2/27445, intron 22 of 24) | 95824 | ENSG00000186472 | PCLO |
| chrX | 73730528 | 73731496 | 0,26 | 3,08 | -2,83 | 0,00199 | 0,0284 | Intron (uc031tjy.1/6567. intron 1 of 5) | -12698 | ENSG00000147100 | SLC16A2 |
| chr12 | 83439865 | 83440624 | -0,86 | 1,96 | -2,82 | 0,00201 | 0,0286 | Intron (uc001szt.3/160335, intron 9 of 11) | 358931 | ENSG00000179104 | TMTC2 |
| chr5 | 120013363 | 120014138 | 0,39 | 3,31 | -2,92 | 0,00203 | 0,0288 | Intron (uc003ksp.3/51334, intron 2 of 2) | 60531 | ENSG00000184838 | PRR16 |
| chr15 | 78819289 | 78820014 | 0,81 | 3,7 | -2,9 | 0,00204 | 0,0289 | 3'UTR | -12733 | ENSG00000041357 | PSMA4 |
| chr1 | 19433336 | 19434679 | 0,31 | 3,84 | -3,53 | 0,00205 | 0,029 | Intron (uc001mpp.3/89797, intron 1 of 37) | 61065 | ENSG00000166833 | NAV2 |
| chr3 | 38134172 | 38134553 | -1,03 | 1,5 | -2,53 | 0,00206 | 0,0291 | Promoter (<=1kb) | -543 | ENSG00000008226 | DLEC1 |
| chr12 | 40747273 | 40748020 | -0,7 | 2,19 | -2,89 | 0,00206 | 0,0291 | Intron (uc001rmg.4/120892, intron 44 of 50) | 50682 | ENSG00000188906 | LRRK2 |
| chr12 | 33052723 | 33053528 | -0,12 | 3,1 | -3,21 | 0,00207 | 0,0293 | Promoter (2-3kb) | -2943 | ENSG00000057294 | PKP2 |
| chr18 | 14786064 | 14786843 | -0,58 | 2,48 | -3,06 | 0,00208 | 0,0294 | Intron (uc010xak.2/374860, intron 14 of 29) | 37825 | ENSG00000180777 | ANKRD30B |
| chr15 | 22776922 | 22777389 | -1,28 | 1,34 | -2,62 | 0,00208 | 0,0294 | Intron (uc001yuj.2/uc001yuj.2, intron 2 of 12) | 40676 | ENSG00000273976 | GOLGA6L1 |
| chr5 | 52961154 | 52961651 | -1,28 | 1,35 | -2,63 | 0,00208 | 0,0293 | Intron (uc003 jpe.2/4724, intron 4 of 4) | 104689 | ENSG00000164258 | NDUFS4 |
| chr5 | 53272561 | 53273148 | -0,99 | 1,76 | -2,75 | 0,00209 | 0,0295 | Intron (uc003jpg.1/54622, intron 4 of 4) | -25132 | ENSG00000207627 | MIR581 |
| chr12 | 82029812 | 82030404 | -1,14 | 1,2 | -2,34 | 0,00209 | 0,0294 | Intron (uc031qim.1/8499, intron 2 of 29) | -37662 | ENSG00000139220 | PPFIA2 |
| chr17 | 151938 | 152417 | -0,28 | 2,41 | -2,69 | 0,00209 | 0,0295 | Intron (uc010vpv.2/9501, intron 3 of 6) | 24953 | ENSG00000181031 | RPH3AL |
| chr9 | 126784654 | 126785384 | 1,23 | 4,41 | -3,19 | 0,00211 | 0,0296 | Intron (uc004boe.1/9355, intron 4 of 4) | 10765 | ENSG00000106689 | LHX2 |
| chr16 | 78829871 | 78830386 | -0,05 | 2,71 | -2,76 | 0,00211 | 0,0296 | Intron (uc002ffk.3/51741, intron 8 of 8) | 696544 | ENSG00000186153 | WWOX |
| chr1 | 43352404 | 43352830 | -0,49 | 2,12 | -2,61 | 0,00211 | 0,0297 | Exon (uc001cij.1/uc001cij.1, exon 4 of 4) | 37442 | ENSG00000164011 | ZNF691 |
| chr15 | 89138282 | 89139247 | -0,28 | 2,99 | -3,27 | 0,00212 | 0,0298 | Intron (uc002bms.1/uc002bms.1, intron 2 of 2) | -12091 | ENSG00000221630 | MIR1179 |
| chr16 | 21005813 | 21007289 | 0,27 | 3,42 | -3,15 | 0,00213 | 0,0298 | Intron (uc010vbe.2/55567, intron 44 of 61) | -9397 | ENSG00000158486 | DNAH3 |
| chr4 | 14591081 | 14591588 | -1,13 | 1,41 | -2,54 | 0,00214 | 0,0299 | Intron (uc003gne.3/uc003gne.3, intron 4 of 6) | 404541 | ENSG00000247624 | CPEB2-DT |
| chr7 | 16401116 | 16401781 | -0,53 | 2,43 | -2,95 | 0,00215 | 0,03 | Intron (uc010ktx.2/729920, intron 3 of 9) | 59166 | ENSG00000214960 | CRPPA |
| chr10 | 105241936 | 105242554 | 0,09 | 3,22 | -3,13 | 0,00216 | 0,0301 | Promoter (2-3kb) | -2939 | ENSG00000183128 | CALHM3 |
| chr6 | 55398267 | 55399003 | -0,32 | 2,49 | -2,8 | 0,00216 | 0,0301 | Intron (uc003pcn.3/54511, intron 4 of 9) | 45009 | ENSG00000146151 | HMGCLL1 |
| chr19 | 45888597 | 45889473 | 0,11 | 3,34 | -3,23 | 0,00217 | 0,0302 | Exon (uc002pbm.3/10848, exon 2 of 6) | 6952 | ENSG00000104881 | PPP1R13L |
| chr8 | 74649802 | 74652122 | 0,2 | 3,82 | -3,62 | 0,00217 | 0,0302 | Promoter(<=1kb) | 0 | ENSG00000040341 | STAU2 |
| chr14 | 52326838 | 52330052 | 3,94 | 6,66 | -2,72 | 0,0022 | 0,0305 | Promoter(<=1kb) | 0 | ENSG00000186469 | GNG2 |
| chr2 | 39163626 | 39164644 | 0,6 | 3,71 | -3,11 | 0,00222 | 0,0307 | Exon (uc021vgd.1/100271715, exon 7 of 16) | 17122 | ENSG00000214694 | ARHGEF3 3 |
| chr6 | 144367456 | 144369633 | 1,42 | 5,27 | -3,86 | 0,00222 | 0,0307 | Intron (uc003qki.3/5325, intron 1 of 4) | 16102 | ENSG00000118495 | PLAGL1 |
| chr10 | 29839681 | 29840133 | -0,96 | 1,52 | -2,49 | 0,00222 | 0,0308 | Exon (uc031ptq.1/6840, exon 3 of 34) | 3904 | ENSG00000197321 | SVIL |
| chr9 | 135631617 | 135632261 | 0,15 | 3,07 | -2,93 | 0,00223 | 0,0308 | Intron (uc004cbu.1/158067, intron 11 of 12) | -85829 | ENSG00000125485 | DDX31 |
| chr10 | 89925651 | 89926136 | -1,28 | 1,32 | -2,59 | 0,00223 | 0,0308 | Intron (uc010qms.1/55328, intron 5 of 5) | 302456 | ENSG00000171862 | PTEN |
| chr2 | 190550085 | 190551003 | 0,41 | 3,33 | -2,93 | 0,00224 | 0,0309 | Intron (uc002uqu.3/150709, intron 1 of 25) | 8903 | ENSG00000151687 | ANKAR |
| chr3 | 108203823 | 108204526 | -0,54 | 2,37 | -2,91 | 0,00224 | 0,0309 | Exon (uc003dxa.1/22989, exon 12 of 42) | 43643 | ENSG00000144821 | MYH15 |
| chr6 | 26501534 | 26502867 | 2,56 | 6,38 | -3,82 | 0,00225 | 0,031 | Promoter(<=1kb) | 76 | ENSG00000124557 | BTN1A1 |
| chr19 | 46285370 | 46285756 | -1,21 | 1,27 | -2,49 | 0,00225 | 0,031 | Promoter(<=1kb) | 59 | ENSG00000104936 | DMPK |
| chr3 | 99318272 | 99319074 | -0,7 | 2,14 | -2,84 | 0,00226 | 0,031 | Intron (uc021xbq.1/100313938, intron 1 of 1) | -38366 | ENSG00000144810 | COL8A1 |
| chr18 | 55459987 | 55460686 | -0,71 | 2,24 | -2,95 | 0,00228 | 0,0312 | Intron (uc002lgw.3/5205, intron 1 of 27) | 9641 | ENSG00000081923 | ATP8B1 |
| chr9 | 15849507 | 15849837 | -1,28 | 1,29 | -2,56 | 0,00228 | 0,0312 | Intron (uc003zmd.3/203238, intron 23 of 25) | 105236 | ENSG00000164989 | CCDC171 |
| chr1 | 3381590 | 3383801 | 1,71 | 5,41 | -3,69 | 0,00229 | 0,0313 | Promoter(<=lkb) | 0 | ENSG00000130762 | ARHGEF16 |
| chr10 | 56438655 | 56439204 | -1,28 | 1,29 | -2,57 | 0,00229 | 0,0313 | Intron (uc010qhq.2/65217, intron 1 of 34) | -14605 | ENSG00000150275 | PCDH15 |
| chr8 | 41593727 | 41594540 | 0,07 | 3,08 | -3,01 | 0,0023 | 0,0314 | Intron (uc010lxa.1/157848, intron 1 of 2) | -29968 | ENSG00000029534 | ANK1 |
| chrX | 28677390 | 28677753 | -0,95 | 1,75 | -2,7 | 0,0023 | 0,0314 | Intron (uc004dby.2/11141, intron 1 of 10) | 71709 | ENSG00000169306 | IL1RAPL1 |
| chr1 | 180203600 | 180204406 | -0,58 | 2,5 | -3,08 | 0,00231 | 0,0315 | Intron (uc001goe.2/89884, intron 1 of 5) | 4167 | ENSG00000121454 | LHX4 |
| chr19 | 55707592 | 55708426 | 1,54 | 4,74 | -3,21 | 0,00231 | 0,0316 | Exon (uc002qjq.3/5794, exon 10 of 20) | 12448 | ENSG00000080031 | PTPRH |
| chr2 | 87893470 | 87894344 | -1,13 | 1,49 | -2,61 | 0,00232 | 0,0317 | Intron (uc002srs.4/64795, intron 3 of 10) | -34930 | ENSG00000265507 | MIR4435-1 |
| chr2 | 160879963 | 160880509 | -0,4 | 2,43 | -2,83 | 0,00232 | 0,0317 | Intron (uc002ube.2/22925, intron 6 of 29) | 38617 | ENSG00000153246 | PLA2R1 |
| chr17 | 15241310 | 15242130 | -0,17 | 2,76 | -2,93 | 0,00232 | 0,0316 | Promoter (2-3kb) | 2828 | ENSG00000125409 | TEKT3 |
| chr13 | 98828381 | 98830259 | 1,42 | 5,37 | -3,95 | 0,00234 | 0,0319 | Promoter(<=lkb) | 0 | ENSG00000139797 | RNF113B |
| chr4 | 99439434 | 99441432 | 0,72 | 4,09 | -3,37 | 0,00236 | 0,0321 | Intron (uc011cdz.2/10098, intron 1 of 7) | 137368 | ENSG00000168785 | TSPAN5 |
| chr18 | 34823617 | 34824136 | 0 | 3,05 | -3,05 | 0,00237 | 0,0322 | 3'UTR | 28987 | ENSG00000101489 | CELF4 |
| chrX | 17080496 | 17080937 | -1,04 | 1,43 | -2,48 | 0,00237 | 0,0322 | Exon (uc004cxv.1/9185, exon 9 of 18) | 83829 | ENSG00000169891 | REPS2 |
| chr2 | 218807380 | 218809309 | 1,58 | 4,96 | -3,38 | 0,00237 | 0,0322 | Promoter(<=1kb) | 0 | ENSG00000079308 | TNS1 |
| chr16 | 49872423 | 49873001 | 0 | 2,87 | -2,88 | 0,00237 | 0,0321 | Intron (uc031qwe.1/23090, intron 1 of 7) | -15774 | ENSG00000102935 | ZNF423 |
| chr2 | 144139888 | 144140701 | -0,32 | 2,61 | -2,93 | 0,00238 | 0,0323 | Intron (uc010zbl.1/55843, intron 6 of 7) | -52462 | ENSG00000075884 | ARHGAP15 |
| chr19 | 15427098 | 15427941 | -0,73 | 2,22 | -2,95 | 0,00238 | 0,0323 | Intron (uc002nat.3/23476, intron 1 of 11) | 14776 | ENSG00000141867 | BRD4 |
| chr12 | 88399234 | 88400215 | -0,21 | 3,04 | -3,25 | 0,00238 | 0,0323 | Intron (uc001tam.1/160419, intron 3 of 12) | 22931 | ENSG00000165805 | C12orf50 |
| chr15 | 94013083 | 94013818 | -0,29 | 2,97 | -3,26 | 0,00238 | 0,0323 | Intron (uc002bsu.1/uc002bsu.1, intron 2 of 3) | -380640 | ENSG00000182175 | RGMA |
| chr13 | 22242543 | 22251506 | 9,36 | 10,36 | -0,99 | 0,00239 | 0,0324 | Promoter(<=1kb) | 0 | ENSG00000102678 | FGF9 |
| chr1 | 178310388 | 178310748 | -0,87 | 1,86 | -2,73 | 0,00239 | 0,0323 | Promoter(<=1kb) | 0 | ENSG00000075391 | RASAL2 |
| chr19 | 41601447 | 41602493 | 0,69 | 4,2 | -3,51 | 0,0024 | 0,0325 | 3'UTR | 7091 | ENSG00000197838 | CYP2A13 |
| chr7 | 32792701 | 32793139 | -1,28 | 1,28 | -2,55 | 0,0024 | 0,0325 | Exon (uc003tcv.3/uc003tcv.3, exon 3 of 3) | -4759 | NA | LINC00997 |
| chr8 | 74536725 | 74537932 | 1,05 | 4,06 | -3,02 | 0,0024 | 0,0325 | Intron (uc003xzm.3/27067, intron 6 of 14) | 112652 | ENSG00000040341 | STAU2 |
| chr12 | 6097964 | 6098313 | -0,89 | 1,89 | -2,79 | 0,00242 | 0,0326 | Intron (uc001qnn.1/7450, intron 38 of 51) | -42566 | ENSG00000047617 | ANO2 |
| chr7 | 73240278 | 73243000 | 1,98 | 5,32 | -3,34 | 0,00242 | 0,0326 | Promoter (2-3kb) | -2193 | ENSG00000189143 | CLDN4 |
| chr3 | 169644698 | 169644972 | -1,28 | 1,11 | -2,39 | 0,00242 | 0,0326 | Exon (uc003fgd.3/344658, exon 6 of 9) | 11543 | ENSG00000187033 | SAMD7 |
| chr10 | 24205528 | 24206203 | -0,45 | 2,67 | -3,12 | 0,00243 | 0,0327 | Intron (uc001irs.3/56243, intron 1 of 18) | 221853 | ENSG00000120549 | KIAA1217 |
| chr8 | 58129743 | 58131546 | 1,61 | 4,95 | -3,34 | 0,00243 | 0,0327 | Promoter(<=lkb) | -102 | ENSG00000253301 | LINC01606 |
| chr3 | 31983210 | 31984260 | 1,03 | 4,14 | -3,11 | 0,00243 | 0,0327 | Intron (uc011axf.2/114884, intron 1 of 10) | -39006 | ENSG00000197385 | ZNF860 |
| chr12 | 67948803 | 67949875 | 0,26 | 3,15 | -2,89 | 0,00244 | 0,0328 | Intron (uc001stq.1/uc001stq.1,intron2 of2) | -92637 | ENSG00000127334 | DYRK2 |
| chr10 | 45886415 | 45887289 | -0,68 | 2,25 | -2,94 | 0,00245 | 0,0329 | Intron (uc001jce.4/240, intron2 of 13) | 16791 | ENSG00000012779 | ALOX5 |
| chr12 | 78472287 | 78472561 | -1,28 | 1 | -2,27 | 0,00247 | 0,0331 | Intron (uc001syo.3/89795, intron 12 of 38) | -39246 | ENSG00000067798 | NAV3 |
| chr6 | 35941646 | 35942601 | 0,91 | 3,85 | -2,94 | 0,00247 | 0,0331 | Intron (uc003oll.3/116369, intron 8 of 17) | -4047 | ENSG00000112053 | SLC26A8 |
| chr9 | 74774936 | 74775301 | -0,84 | 1,59 | -2,43 | 0,00249 | 0,0333 | Intron (uco11lse.2/9615, intron 1 of 13) | 10669 | ENSG00000119125 | GDA |
| chr7 | 69634222 | 69634543 | -1,28 | 1,26 | -2,53 | 0,0025 | 0,0334 | Intron (uc003tvv.4/26053, intron 4 of 4) | 570317 | ENSG00000158321 | AUTS2 |
| chr7 | 136967768 | 136969337 | 1,59 | 5,22 | -3,63 | 0,0025 | 0,0334 | Intron (uc003vtq.2/5764, intron 1 of 4) | 59209 | ENSG00000105894 | PTN |
| chr9 | 115318139 | 115318871 | -0,21 | 2,45 | -2,66 | 0,00253 | 0,0337 | Intron (uc004bgf.1/158405, intron 1 of 3) | 68891 | ENSG00000165185 | KIAA1958 |
| chr15 | 91432537 | 91433308 | 0,02 | 2,78 | -2,76 | 0,00254 | 0,0337 | Exon (uc002bpv.3/2242, exon 6 of 19) | 4270 | ENSG00000182511 | FES |
| chr3 | 51931171 | 51932282 | 0,09 | 3,11 | -3,02 | 0,00254 | 0,0338 | Intron (uc003dbq.5/132141, intron 2 of 3) | 5104 | ENSG00000173389 | IQCF1 |
| chr15 | 52000509 | 52001724 | 0,86 | 4,47 | -3,61 | 0,00254 | 0,0338 | Intron (uc002abh.3/29106, intron 10 of 11) | 26959 | ENSG00000104112 | SCG3 |
| chr5 | 7271454 | 7272079 | -0,5 | 2,37 | -2,87 | 0,00256 | 0,0339 | Promoter (1-2kb) | -1987 | ENSG00000283468 | MIR4454 |
| chr1 | 217239161 | 217239928 | -0,21 | 3,05 | -3,26 | 0,00257 | 0,0341 | Intron (uc001hkz.2/2104, intron 1 of 7) | 10384 | ENSG00000196482 | ESRRG |
| chr3 | 158100245 | 158100510 | -1,28 | 0,95 | -2,22 | 0,00259 | 0,0343 | Intron (uc011bou.2/51319, intron 5 of 6) | -188443 | ENSG00000178053 | MLF1 |
| chr17 | 16954712 | 16955748 | -0,36 | 2,83 | -3,19 | 0,0026 | 0,0343 | Intron (uc002gqu.2/23164, intron 1 of 23) | 8638 | ENSG00000133030 | MPRIP |
| chr1 | 16072894 | 16073369 | -0,67 | 2,01 | -2,68 | 0,0026 | 0,0344 | Exon (uc001axc.4/388595, exon 5 of 6) | 3907 | ENSG00000162460 | TMEM82 |
| chr7 | 51338558 | 51340030 | 1,07 | 4,59 | -3,52 | 0,00261 | 0,0345 | Intron (uc003tpr.4/23242, intron 1 of 12) | 44485 | ENSG00000106078 | COBL |
| chrX | 130868957 | 130869342 | -1,28 | 1,23 | -2,5 | 0,00261 | 0,0345 | Intron (uc004ewi.3/286467, intron 10 of 12) | 95329 | ENSG00000213468 | FIRRE |
| chr22 | 17915560 | 17916546 | 0,26 | 3,32 | -3,06 | 0,00262 | 0,0346 | Intron (uc010gqv.1/27443, intron 1 of 18) | -40084 | ENSG00000099954 | CECR2 |
| chr14 | 78235000 | 78235294 | -1,28 | 0,81 | -2,08 | 0,00262 | 0,0346 | Intron (uc001xuh.2/283579, intron 1 of 1) | -7503 | ENSG00000100603 | SNW1 |
| chr1 | 38021608 | 38022473 | 0,03 | 2,76 | -2,72 | 0,00264 | 0,0348 | Promoter(<=1kb) | -47 | ENSG00000163879 | DNALI1 |
| chr22 | 24862754 | 24863976 | 0,33 | 3,28 | -2,95 | 0,00265 | 0,0349 | Promoter(<=1kb) | 0 | ENSG00000100024 | UPB1 |
| chr22 | 42795844 | 42796330 | -0,29 | 1,93 | -2,23 | 0,00266 | 0,035 | Intron (uc003bcn.4/150372, intron 3 of 5) | 32071 | ENSG00000235568 | NFAM1 |
| chr17 | 10521406 | 10522761 | 2,05 | 5,38 | -3,33 | 0,00268 | 0,0352 | Intron (uc002gml.1/uc002gml.1, intron 10 of 10) | 37865 | ENSG00000109063 | MYH3 |
| chr7 | 82752934 | 82754834 | 1,92 | 5,79 | -3,87 | 0,00269 | 0,0353 | Intron (uc003uhx.2/27445, intron 3 of 24) | 37363 | ENSG00000186472 | PCLO |
| chr1 | 175115523 | 175116897 | 2,28 | 5,37 | -3,09 | 0,0027 | 0,0353 | 3'UTR | 45052 | ENSG00000235750 | KIAA0040 |
| chr2 | 111528363 | 111529117 | -0,25 | 2,58 | -2,83 | 0,00271 | 0,0354 | Intron (uc010vxk.1/55289, intron 2 of 17) | -27474 | ENSG00000153093 | ACOXL |
| chr3 | 2280167 | 2280941 | -0,66 | 2,31 | -2,97 | 0,00271 | 0,0354 | Promoter (<= 1kb) | 0 | ENSG00000144619 | CNTN4 |
| chr16 | 65608951 | 65611209 | 0,94 | 4,48 | -3,55 | 0,00271 | 0,0354 | Promoter (<= 1kb) | 0 | ENSG00000261742 | LINC00922 |
| chr7 | 6803152 | 6804030 | -1,18 | 1,34 | -2,52 | 0,00271 | 0,0354 | Promoter (<=1kb) | 0 | ENSG00000169402 | RSPH10B2 |
| chr20 | 20432471 | 20433802 | 2,05 | 5,7 | -3,65 | 0,00272 | 0,0355 | Intron (uc002wrv.3/57186, intron 28 of 29) | 75100 | ENSG00000188559 | RALGAPA2 |
| chr1 | 44491460 | 44491957 | -0,86 | 1,89 | -2,75 | 0,00272 | 0,0356 | Intron (uc010okm.2/6536, intron 1 of 11) | 5207 | ENSG00000196517 | SLC6A9 |
| chr7 | 155177626 | 155178594 | 1,36 | 4,59 | -3,23 | 0,00274 | 0,0357 | Intron (uc003wma.1/uc003wma.1, intron 1 of 1) | -72230 | ENSG00000164778 | EN2 |
| chr1 | 155505483 | 155506869 | 0,21 | 3,57 | -3,35 | 0,00275 | 0,0359 | Intron (uc001fkt.3/55870, intron 1 of 27) | -24903 | ENSG00000235919 | ASH1L-AS1 |
| chr17 | 79564446 | 79566223 | 1,24 | 4,46 | -3,21 | 0,00275 | 0,0358 | Intron (uc002kat.4/55666, intron 9 of 16) | -28309 | ENSG00000182446 | NPLOC4 |
| chr1 | 165204446 | 165205503 | 0,54 | 3,76 | -3,22 | 0,00277 | 0,0361 | Intron (uc001gcz.2/4009, intron 4 of 8) | -21929 | ENSG00000162761 | LMX1A |
| chr2 | 201567212 | 201568520 | 0,82 | 3,99 | -3,17 | 0,00279 | 0,0362 | Intron (uc021vur.1/344454, intron 1 of 30) | 6766 | NA | AOX2P |
| chr1 | 231787157 | 231788040 | 0,33 | 3,46 | -3,12 | 0,0028 | 0,0362 | Intron (uc010pwe.2/100303453, intron 7 of 8) | 24596 | ENSG00000162946 | DISC1 |
| chr7 | 129360004 | 129360790 | 0,58 | 3,64 | -3,06 | 0,0028 | 0,0362 | Intron (uc003voz.3/4899, intron 9 of 10) | -35316 | NA | RNA5 SP244 |
| chr2 | 38255741 | 38256127 | -1,14 | 1,39 | -2,53 | 0,00282 | 0,0364 | Intron (uc002rqn.2/151393, intron 10 of 10) | 7357 | NA | RMDN2-AS1 |
| chr3 | 173162732 | 173163412 | -0,56 | 1,98 | -2,54 | 0,00284 | 0,0366 | Intron (uc003fio.1/22871, intron 2 of 6) | 46488 | ENSG00000169760 | NLGN1 |
| chr7 | 38339702 | 38339973 | -1,22 | 1,18 | -2,4 | 0,00285 | 0,0367 | 5'UTR | -8023 | ENSG00000211689 | TARP |
| chr10 | 5696272 | 5698222 | 2,19 | 5,47 | -3,28 | 0,00286 | 0,0368 | Intron (uc001iig.2/79754, intron 1 of 5) | 10336 | ENSG00000196372 | ASB13 |
| chr2 | 18082620 | 18082903 | -0,95 | 1,53 | -2,48 | 0,00286 | 0,0368 | Intron (uc002rcv.3/3790, intron 1 of 2) | 22675 | ENSG00000170745 | KCNS3 |
| chr18 | 13826075 | 13826546 | -1,1 | 1,44 | -2,54 | 0,00287 | 0,0369 | Promoter(<=lkb) | 532 | ENSG00000176136 | MC5R |
| chr5 | 141319988 | 141320388 | -0,62 | 1,82 | -2,44 | 0,00289 | 0,0371 | 3'UTR | 16603 | ENSG00000081791 | DELE1 |
| chr9 | 10171288 | 10171707 | -0,68 | 1,98 | -2,66 | 0,0029 | 0,0371 | Intron (uc003zkk.3/5789, intron 3 of 45) | 441016 | ENSG00000153707 | PTPRD |
| chr2 | 65886736 | 65887729 | 0,54 | 3,57 | -3,03 | 0,0029 | 0,0371 | Intron (uc010fcv.1/uc010fcv.1, intron 4 of 9) | -227080 | ENSG00000198369 | SPRED2 |
| chr2 | 169642782 | 169643356 | -0,87 | 1,8 | -2,67 | 0,00291 | 0,0372 | Promoter(<=lkb) | 0 | ENSG00000227617 | CERS6-AS1 |
| chr4 | 91586374 | 91586828 | -1,28 | 1,29 | -2,57 | 0,00292 | 0,0373 | Intron (uc010ikv.2/401145, intron 7 of 9) | 15703 | ENSG00000184305 | CCSER1 |
| chr1 | 216859954 | 216862161 | 1,16 | 4,78 | -3,62 | 0,00294 | 0,0375 | Intron (uc001hkw.2/2104, intron 1 of 6) | 34653 | ENSG00000196482 | ESRRG |
| chr4 | 3447305 | 3447936 | -0,39 | 2,47 | -2,86 | 0,00296 | 0,0378 | Exon (uc003ghc.3/3083, exon 10 of 14) | 3579 | ENSG00000109758 | HGFAC |
| chr2 | 190521760 | 190525937 | 2,82 | 6,41 | -3,58 | 0,00297 | 0,0378 | Promoter (<=1kb) | -188 | ENSG00000138381 | ASNSD1 |
| chr9 | 115146647 | 115147598 | -0,81 | 1,89 | -2,7 | 0,00297 | 0,0378 | Intron (uc004bga.2/84263, intron 1 of 10) | 4458 | ENSG00000119471 | HSDL2 |
| chr1 | 68384426 | 68385260 | -0,37 | 2,6 | -2,97 | 0,00298 | 0,0379 | Intron (uc001deb.2/100289178, intron 1 of 9) | -85271 | ENSG00000172380 | GNG12 |
| chr1 | 178235606 | 178236485 | 0,34 | 3,55 | -3,21 | 0,00299 | 0,038 | Intron (uc009wxb.2/9462, intron 1 of 4) | -74121 | ENSG00000075391 | RASAL2 |
| chr21 | 37784023 | 37784356 | -1,28 | 1,2 | -2,48 | 0,00301 | 0,0381 | Intron (uc002yvj.3/8208, intron 11 of 13) | 26334 | ENSG00000159259 | CHAF1B |
| chr18 | 46302522 | 46302793 | -1,07 | 1,34 | -2,41 | 0,00301 | 0,0381 | Intron (uc0021dc.3/9811, intron 9 of 11) | 15947 | ENSG00000134030 | CTIF |
| chr17 | 56332074 | 56332476 | -0,65 | 1,95 | -2,6 | 0,00301 | 0,0381 | Promoter (2-3kb) | 2531 | ENSG00000167419 | LPO |
| chr19 | 45655135 | 45657703 | 3,39 | 7,68 | -4,29 | 0,00301 | 0,0381 | 5'UTR | 5705 | ENSG00000179846 | NKPD1 |
| chr10 | 1251860 | 1252832 | -0,29 | 2,79 | -3,07 | 0,00302 | 0,0382 | Intron (uc009xhq.3/105, intron 7 of 9) | -5529 | ENSG00000185736 | ADARB2 |
| chr16 | 4832127 | 4832888 | -0,55 | 2,31 | -2,85 | 0,00302 | 0,0382 | Intron (uc002cxq.3/124404, intron 7 of 9) | -5510 | ENSG00000267795 | SMIM22 |
| chr7 | 148098858 | 148099639 | 0,23 | 3,2 | -2,97 | 0,00303 | 0,0383 | Intron (uc003weu.2/26047, intron 22 of 23) | 62305 | ENSG00000174469 | CNTNAP2 |
| chr2 | 166483705 | 166484242 | -0,91 | 1,66 | -2,57 | 0,00303 | 0,0383 | Intron (uc002udf.3/80034, intron 4 of 6) | 54859 | ENSG00000178662 | CSRNP3 |
| chr2 | 206365488 | 206366228 | 0 | 2,78 | -2,78 | 0,00304 | 0,0384 | Intron (uc002vao.2/117583, intron 20 of 21) | -180996 | ENSG00000 118257 | NRP2 |
| chr12 | 32262896 | 32263686 | -0,63 | 2,01 | -2,64 | 0,00305 | 0,0385 | Promoter (2-3kb) | 2711 | ENSG00000 151746 | BICD1 |
| chr2 | 212449736 | 212450070 | -1,12 | 1,21 | -2,33 | 0,00305 | 0,0385 | Intron (uc002veg.1/2066, intron 19 of 27) | 841014 | ENSG00000221782 | MIR548F2 |
| chr1 | 111158812 | 111159683 | -0,02 | 2,53 | -2,55 | 0,00306 | 0,0386 | Intron (uc009wfv.2/3737, intron 1 of 4) | -9837 | ENSG00000177301 | KCNA2 |
| chr7 | 138759610 | 138759958 | 0,19 | 2,67 | -2,48 | 0,00307 | 0,0387 | Intron (uc003vun.3/56829, intron 6 of 12) | 4055 | ENSG00000105939 | ZC3HAV1 |
| chr4 | 41094091 | 41095434 | 0,48 | 3,53 | -3,05 | 0,00308 | 0,0388 | Intron (uc003gvl.3/323.intron 3 of 17) | -77676 | ENSG00000163697 | APBB2 |
| chr8 | 95446863 | 95450022 | 2,6 | 6,09 | -3,49 | 0,00309 | 0,0388 | Promoter (<=1kb) | 0 | ENSG00000265817 | FSBP |
| chr3 | 183817588 | 183818319 | 0,12 | 2,96 | -2,83 | 0,00311 | 0,039 | Promoter (<=1kb) | 0 | ENSG00000186038 | HTR3E |
| chr14 | 97924510 | 97926015 | 1,78 | 4,59 | -2,81 | 0,00311 | 0,039 | Exon (uc001vfu.3/uc001vfu.3, exon 1 of 3) | 226980 | ENSG00000197176 | LINC02291 |
| chr8 | 135633861 | 135635126 | 0,28 | 3,48 | -3,19 | 0,00311 | 0,039 | Intron (uc003yun.3/57623, intron 4 of 16) | 23547 | ENSG00000248492 | ZFAT-AS1 |
| chr3 | 122043621 | 122044300 | -0,15 | 2,82 | -2,97 | 0,00312 | 0,0391 | Promoter (<=1kb) | 0 | ENSG00000121552 | CSTA |
| chr2 | 212388832 | 212389819 | -0,04 | 3,19 | -3,23 | 0,00312 | 0,0391 | Intron (uc002veg.1/2066, intron 20 of 27) | 901265 | ENSG00000221782 | MIR548F2 |
| chr14 | 89017434 | 89018756 | 0,91 | 4,47 | -3,57 | 0,00312 | 0,0391 | Promoter (<=1kb) | 0 | ENSG00000070778 | PTPN21 |
| chr1 | 27761931 | 27764060 | 1,69 | 5,05 | -3,36 | 0,00315 | 0,0394 | Intron (uc001bof.2/10163, intron 1 of 8) | 42783 | ENSG00000181773 | GPR3 |
| chr16 | 65506682 | 65507203 | 0,33 | 3,1 | -2,77 | 0,00315 | 0,0393 | Intron (uc010cdp.1/283867, intron 1 of 5) | 98625 | ENSG00000261742 | LINC00922 |
| chr1 | 173472323 | 173472814 | -0,22 | 2,85 | -3,07 | 0,00315 | 0,0394 | Exon (uc009wwe.2/284525.exon 20 of 21) | 25837 | ENSG00000117592 | PRDX6 |
| chr4 | 148462288 | 148464271 | 1,85 | 5,16 | -3,31 | 0,00316 | 0,0394 | 3'UTR | 55524 | ENSG00000151617 | EDNRA |
| chr21 | 18940148 | 18940873 | -0,6 | 2,22 | -2,82 | 0,00317 | 0,0396 | 3'UTR | 44395 | ENSG00000 154640 | BTG3 |
| chr9 | 88313113 | 88314293 | 0,74 | 4,11 | -3,37 | 0,00318 | 0,0396 | Intron (uc011ltd.2/23287, intron 1 of 24) | 13188 | ENSG00000135049 | AGTPBP1 |
| chr19 | 40360736 | 40361046 | -0,93 | 1,69 | -2,61 | 0,00318 | 0,0396 | Exon (uc002omp.4/8857, exon 33 of 36) | -23682 | ENSG00000105202 | FBL |
| chr4 | 87620256 | 87620912 | -0,19 | 2,95 | -3,13 | 0,00318 | 0,0396 | Intron (uc003hpv.3/5783, intron 6 of 47) | -68755 | ENSG00000163629 | PTPN13 |
| chr2 | 1249800 | 1250199 | -1,17 | 1,34 | -2,51 | 0,00318 | 0,0396 | Intron (uc002qwq .3/54221, intron 11 of 16) | -127796 | ENSG00000115705 | TPO |
| chr19 | 8646968 | 8647734 | 0,42 | 3,3 | -2,88 | 0,00319 | 0,0396 | Intron (uc002mki.1/81794, intron 12 of 12) | -4637 | ENSG00000142347 | MYO1F |
| chr16 | 24676059 | 24676531 | -0,52 | 2,03 | -2,56 | 0,00322 | 0,0399 | Exon (uc002dml.1/uc002dml.1, exon 2 of 3) | -64518 | ENSG00000090905 | TNRC6A |
| chr12 | 116864897 | 116865625 | 0 | 3,13 | -3,13 | 0,00323 | 0,0401 | Promoter (<=1kb) | 498 | ENSG00000264037 | MIR4472-2 |
| chr16 | 81307520 | 81308218 | -0,17 | 2,45 | -2,63 | 0,00324 | 0,0402 | Intron (uc002fgn.1/53630, intron 7 of 10) | 35224 | ENSG00000135697 | BCOl |
| chr19 | 5990108 | 5991051 | 2,13 | 5,06 | -2,93 | 0,00326 | 0,0404 | Intron (uc031rjb.1/100128568, intron 1 of 3) | 11694 | ENSG00000266983 | LOC10012856 8 |
| chr15 | 90615184 | 90620555 | 3,08 | 6,78 | -3,7 | 0,00327 | 0,0404 | Exon (uc002bov.2/374655, exon 3 of 5) | 23372 | ENSG00000 182054 | IDH2 |
| chr7 | 99797640 | 99798156 | 1,03 | 3,75 | -2,72 | 0,00328 | 0,0405 | Promoter (<=1kb) | -236 | ENSG00000066923 | STAG3 |
| chr12 | 120032571 | 120033611 | 1,66 | 4,64 | -2,97 | 0,00328 | 0,0405 | Promoter (1-2kb) | 1307 | ENSG00000224982 | TMEM23 3 |
| chr1 | 186221294 | 186221898 | -1,28 | 1,11 | -2,39 | 0,00329 | 0,0406 | Intron (uc021pgf.1/100302192, intron 1 of 1) | -43507 | ENSG00000 116690 | PRG4 |
| chr15 | 93203673 | 93204940 | 1,32 | 4,89 | -3,57 | 0,0033 | 0,0406 | Intron (uc002bsl.4/400451, intron 2 of 3) | -4642 | ENSG00000185442 | FAM174B |
| chr8 | 26175205 | 26175838 | -0,63 | 1,75 | -2,38 | 0,00332 | 0,0409 | Intron (uc003xek.3/5520, intron 7 of 10) | 24473 | ENSG00000221914 | PPP2R2A |
| chr17 | 17795518 | 17796075 | -0,8 | 1,83 | -2,62 | 0,00332 | 0,0408 | Intron (uc002gry.4/146691, intron 3 of 13) | -55193 | ENSG00000072310 | SREBF1 |
| chr5 | 10700505 | 10701117 | -0,32 | 2,68 | -3 | 0,00333 | 0,041 | Intron (uc003jez.4/1611, intron 2 of 3) | 60270 | ENSG00000112977 | DAP |
| chr1 | 203594852 | 203599914 | 8,78 | 9,79 | -1,01 | 0,00335 | 0,0412 | Promoter (<=1kb) | 0 | ENSG00000058668 | ATP2B4 |
| chr5 | 54807147 | 54808285 | 0,55 | 4,15 | -3,6 | 0,00335 | 0,0411 | Promoter (2-3kb) | -2393 | ENSG0000026513 5 | MIR5687 |
| chr20 | 17708002 | 17708913 | -0,2 | 2,84 | -3,03 | 0,00336 | 0,0412 | Intron (uc010zrs.1/140836, intron 2 of 2) | 27409 | ENSG00000125888 | BANF2 |
| chr10 | 4713471 | 4714391 | 0,18 | 3,22 | -3,04 | 0,00336 | 0,0413 | Intron (uc001ihe.4/100216001, intron 1 of 3) | 5871 | ENSG00000231298 | MANCR |
| chr11 | 84042921 | 84043831 | -0,42 | 2,55 | -2,97 | 0,00337 | 0,0413 | Intron (uc001paj.2/1740,intron2 of22) | -14539 | ENSG00000150672 | DLG2 |
| chr10 | 35079810 | 35080479 | -0,77 | 2,14 | -2,91 | 0,00337 | 0,0413 | Intron (uc010qej.2/56288, intron 1 of 24) | 23774 | ENSG00000 148498 | PARD3 |
| chrl7 | 78711077 | 78711415 | -1,12 | 1,24 | -2,36 | 0,00337 | 0,0414 | Intron (uc002jys.3/57521, intron 5 of 8) | 192452 | ENSG00000141564 | RPTOR |
| chr10 | 25161884 | 25162543 | -0,14 | 2,46 | -2,6 | 0,00338 | 0,0414 | Intron (uc001ise.1/56952, intron 3 of 8) | 78990 | ENSG00000099256 | PRTFDC1 |
| chr1 | 216883239 | 216884901 | 1,32 | 4,89 | -3,57 | 0,00339 | 0,0415 | Intron (uc001hkw.2/2104, intron 1 of 6) | 11913 | ENSG00000 196482 | ESRRG |
| chr8 | 12668763 | 12669685 | 0,14 | 3,06 | -2,92 | 0,0034 | 0,0416 | Promoter (<=1kb) | 0 | ENSG00000254813 | LOC340357 |
| chr2 | 101656399 | 101657186 | 0,2 | 3,33 | -3,12 | 0,00343 | 0,0418 | Exon (uc002tau.4/11138, exon 4 of 18) | 18815 | ENSG00000204634 | TBC1D8 |
| chr11 | 5580767 | 5581162 | -0,44 | 2,02 | -2,46 | 0,00344 | 0,0419 | Intron (uc001mak.1/3048. intron 4 of 6) | -14014 | ENSG00000181616 | OR52H1 |
| chr1 | 158814563 | 158815547 | 0,9 | 4,1 | -3,2 | 0,00345 | 0,0421 | Exon (uc001fsz.1/4332, exon 5 of 7) | 13395 | ENSG00000163563 | MNDA |
| chr3 | 76705582 | 76706769 | 0,27 | 3,31 | -3,04 | 0,00346 | 0,0421 | Intron (uc021xat.1/6092, intron 1 of 25) | -382525 | ENSG00000185008 | ROBO2 |
| chr15 | 101429583 | 101430375 | 0,19 | 3,01 | -2,82 | 0,00348 | 0,0423 | Intron (uc002bwn.4/220, intron 3 of 12) | 9574 | ENSG00000 184254 | ALDH1A3 |
| chr19 | 7922750 | 7923857 | 1,88 | 5,23 | -3,35 | 0,00348 | 0,0423 | 3'UTR | 6430 | ENSG00000 142459 | EVI5L |
| chr17 | 58090054 | 58090982 | -0,78 | 1,93 | -2,71 | 0,00348 | 0,0423 | Exon (uc021uaw.1/uc021uaw.1, exon 1 of 1) | 5354 | ENSG00000267104 | TBC1D3P1-DHX40P1 |
| chrX | 9895662 | 9896449 | -0,02 | 3,02 | -3,04 | 0,0035 | 0,0425 | Intron (uc004csu.1/357, intron 5 of 9) | 14939 | ENSG00000 146950 | SHROOM2 |
| chr17 | 42331287 | 42331556 | -1,28 | 0,99 | -2,26 | 0,0035 | 0,0425 | Intron (uc021tvc.1/6521, intron 10 of 13) | 8746 | ENSG00000004939 | SLC4A1 |
| ch16 | 30130651 | 30131501 | 1,24 | 4,28 | -3,04 | 0,0035 | 0,0424 | Promoter (<=1kb) | 0 | ENSG00000204610 | TRIM15 |
| chr14 | 106891757 | 106892444 | -0,35 | 2,54 | -2,89 | 0,00351 | 0,0425 | Exon (uc031qqx.1/uc031qqx.1, exon 438 of 5065) | -46011 | ENSG00000270816 | LINC00221 |
| chr7 | 104308987 | 104309800 | -0,57 | 2,06 | -2,63 | 0,00352 | 0,0426 | Intron (uc003vce.3/375612, intron 1 of 2) | 134739 | ENSGO0000226869 | LHFPL3-AS1 |
| chr11 | 118215057 | 118215370 | -1,22 | 0,95 | -2,17 | 0,00355 | 0,0428 | Promoter (<=1kb) | 0 | ENSG00000 160654 | CD3G |
| chr17 | 56021980 | 56027386 | 2,5 | 6,29 | -3,79 | 0,00355 | 0,0428 | Intron (uc002ive.2/404093, intron 1 of 10) | 5298 | ENSG00000180891 | CUEDC1 |
| chr17 | 44940117 | 44940750 | 0,85 | 3,64 | -2,79 | 0,00356 | 0,0429 | Intron (uc002ikw.1/7484, intron 1 of 3) | 11149 | ENSG00000158955 | WNT9B |
| chr8 | 104995870 | 104996722 | 0,02 | 3,24 | -3,21 | 0,00358 | 0,0431 | Intron (uc003vlp.3/9699, intron 16 of 23) | 68147 | ENSG00000176406 | RIMS2 |
| chr4 | 13707068 | 13707794 | -0,17 | 2,48 | -2,65 | 0,00361 | 0,0435 | Intron (uc003gna.1/uc003gna.1, intron 2 of 3) | 77579 | ENSG00000266240 | MIR5091 |
| chr17 | 7643463 | 7644399 | 0,64 | 3,71 | -3,07 | 0,00362 | 0,0435 | Exon (uc002git.3/146754, exon 12 of 14) | -13239 | ENSG00000240480 | RPL29P2 |
| chr10 | 119044904 | 119045234 | -0,99 | 1,54 | -2,53 | 0,00362 | 0,0435 | Exon (uc001lde.1/118987, exon 5 of 5) | 44320 | ENSG00000 165646 | SLC18A2 |
| chr18 | 33846802 | 33847085 | -1,02 | 1,37 | -2,39 | 0,00363 | 0,0436 | Exon (uc002kzq.4/55034, exon 14 of 15) | -30617 | ENSG00000134775 | FHOD3 |
| chr6 | 27673272 | 27674398 | 0,2 | 3,36 | -3,17 | 0,00363 | 0,0435 | Intron (uc021yrb.1/100507173, intron 2 of 3) | 11414 | ENSG00000281706 | LINC01012 |
| chr6 | 35705594 | 35705941 | -0,25 | 2,3 | -2,55 | 0,00365 | 0,0437 | Promoter (<=1kb) | 735 | ENSG00000157343 | ARMC12 |
| chr8 | 143742939 | 143743980 | 0,51 | 3,59 | -3,07 | 0,00365 | 0,0437 | 3'UTR | 7421 | ENSG00000234616 | JRK |
| chr3 | 134976156 | 134977191 | -0,75 | 1,94 | -2,69 | 0,00366 | 0,0438 | Intron (uc003eqt.3/2047, intron 15 of 15) | 189782 | ENSG00000154928 | EPHB1 |
| chrX | 115592714 | 115594520 | 2,14 | 5,53 | -3,39 | 0,00369 | 0,044 | Promoter (<=1kb) | 0 | ENSG000002040 19 | CT83 |
| chr7 | 158260566 | 158261600 | 0,13 | 3,37 | -3,23 | 0,00369 | 0,044 | Intron (uc011kwa.2/5799, intron 2 of 22) | 63905 | ENSG00000207637 | MIR595 |
| chr1 | 3649143 | 3649899 | 0,12 | 3,33 | -3,21 | 0,00369 | 0,044 | 3'UTR | 5101 | ENSG00000078900 | TP73 |
| chr21 | 41404546 | 41406047 | 1,13 | 4,38 | -3,24 | 0,0037 | 0,0442 | Intron (uc002yyq.1/1826, intron 32 of 32) | 165199 | ENSG00000183036 | PCP4 |
| chr1 | 18807689 | 18809962 | 2,45 | 5,91 | -3,46 | 0,00371 | 0,0442 | Promoter (<=1kb) | 265 | ENSG00000179023 | KLHDC7A |
| chr17 | 75429659 | 75430449 | -0,11 | 2,98 | -3,1 | 0,00373 | 0,0443 | Intron (uc002jts.4/10801, intron 3 of 11) | -16164 | ENSG00000 184640 | SEPTIN9 |
| chr8 | 68373744 | 68374216 | -1,02 | 1,46 | -2,48 | 0,00374 | 0,0444 | Intron (uc003xxq.4/57094, intron 8 of 10) | -117832 | ENSG00000066777 | ARFGEF1 |
| chr7 | 48086012 | 48086498 | -0,34 | 2,24 | -2,58 | 0,00379 | 0,0449 | 5'UTR | 10904 | ENSG00000164746 | C7orf57 |
| chr10 | 50339787 | 50340266 | -1,05 | 1,53 | -2,58 | 0,00379 | 0,0449 | Promoter (1-2kb) | 1799 | ENSG00000172538 | FAM170B |
| chr7 | 49852497 | 49853296 | 0,35 | 3,45 | -3,09 | 0,00379 | 0,0449 | Intron (uc003tot.1/375567, intron 3 of 3) | 39240 | ENSG00000188730 | VWC2 |
| chr18 | 11020643 | 11022551 | 1,14 | 4,36 | -3,22 | 0,0038 | 0,0449 | Intron (uc002kos.2/63895, intron 2 of 51) | 126210 | ENSG00000154864 | PIEZO2 |
| chr7 | 20747215 | 20747613 | -1,28 | 1,1 | -2,38 | 0,00382 | 0,0451 | Intron (uc010kuh.3/340273, intron 20 of 27) | -14663 | ENSG00000004846 | ABCB5 |
| chr19 | 59073000 | 59075519 | 3,58 | 7,6 | -4,02 | 0,00383 | 0,0452 | Promoter (2-3kb) | 2447 | ENSG00000267858 | MZF1-AS1 |
| chr9 | 140505991 | 140507204 | 0,25 | 3,25 | -3 | 0,00384 | 0,0452 | Promoter (<=1kb) | -54 | ENSG00000197070 | ARRDC1 |
| chr6 | 24643993 | 24644560 | -0,57 | 2,25 | -2,82 | 0,00384 | 0,0452 | Promoter (1-2kb) | 1689 | ENSG00000137261 | KIAA0319 |
| chrX | 48684241 | 48686520 | 3,12 | 7,04 | -3,92 | 0,00385 | 0,0453 | Promoter (<=1kb) | 0 | ENSG00000187682 | ERAS |
| chr21 | 17951546 | 17952681 | 0,58 | 3,52 | -2,94 | 0,00385 | 0,0453 | Intron(uc002ykb.2/388815, intron 6 of 7) | -9876 | ENSG00000207863 | MIR125B2 |
| chr7 | 100844635 | 100845497 | 0,06 | 2,84 | -2,79 | 0,00386 | 0,0454 | Promoter (<=1kb) | -333 | ENSG00000106384 | MOGAT3 |
| chr7 | 4848557 | 4849583 | 1,61 | 4,83 | -3,22 | 0,00389 | 0,0457 | Intron (uc003sng.1/55698. intron 9 of 15) | -4858 | ENSG00000157927 | RADIL |
| chr21 | 46331005 | 46331946 | -0,13 | 2,72 | -2,85 | 0,00391 | 0,0459 | Promoter (<=1kb) | -264 | ENSG00000 160255 | ITGB2 |
| chr18 | 20812135 | 20812437 | -0,96 | 1,02 | -1,98 | 0,00394 | 0,0462 | Intron (uc002kub.2/91768, intron 4 of 6) | 76346 | ENSG00000134508 | CABLES 1 |
| chr1 | 159157898 | 159158661 | -0,2 | 2,58 | -2,78 | 0,00396 | 0,0463 | Intron (uc009wsx.1/57863, intron 1 of 4) | 13551 | NA | CADM3-AS1 |
| chr9 | 86975822 | 86976833 | -0,08 | 2,88 | -2,96 | 0,00397 | 0,0463 | Intron (uc004anu.2/64078, intron 1 of 18) | 6580 | ENSG00000197506 | SLC28A3 |
| chr8 | 94507465 | 94509450 | 2,52 | 5,74 | -3,22 | 0,00399 | 0,0466 | Intron (uc022avb.1/642924, intron 7 of 10) | 203211 | ENSG00000246662 | LINC00535 |
| chr3 | 85516787 | 85517662 | 0,18 | 3,33 | -3,15 | 0,004 | 0,0467 | Intron (uc003dqj.3/253559, intron 1 of 9) | -36547 | ENSG00000175161 | CADM2 |
| chr7 | 75318845 | 75320098 | 1,04 | 4,46 | -3,42 | 0,004 | 0,0466 | Intron (uc003uds.2/3092, intron 1 of 30) | 48185 | ENSG00000127946 | HIP1 |
| chr7 | 6390052 | 6390828 | 0,37 | 3,06 | -2,69 | 0,00402 | 0,0468 | Promoter (1-2kb) | -1462 | ENSG00000286075 | FAM220A |
| chr1 | 32781361 | 32782254 | 0,02 | 2,95 | -2,93 | 0,00404 | 0,047 | Intron (uc010ohd.1/3065, intron 2 of 5) | -12827 | ENSG00000116478 | HDAC1 |
| chrX | 53283610 | 53284230 | -0,66 | 2,02 | -2,68 | 0,00405 | 0,0471 | Exon (uc004dsc.3/23096, exon 4 of 14) | 26566 | ENSG00000 124313 | IQSEC2 |
| chr15 | 65629206 | 65629615 | -0,36 | 2 | -2,37 | 0,00406 | 0,0471 | Intron (uc002aos.2/9543, intron 2 of 13) | -7271 | ENSG00000174498 | IGDCC3 |
| chr13 | 70607164 | 70607448 | -0,75 | 1,53 | -2,28 | 0,00407 | 0,0473 | Intron (uc001vip.3/57626, intron 1 of 10) | -73897 | NA | ATXN8OS |
| chr15 | 29022552 | 29022911 | -1,28 | 1,09 | -2,36 | 0,00407 | 0,0472 | Intron (uc001zci.1/440253, intron 10 of 10) | -10478 | ENSG00000232431 | LOC10028965 6 |
| chr12 | 7991466 | 7992386 | 0,07 | 3,04 | -2,97 | 0,00409 | 0,0474 | Intron (uc001qtk.3/144195, intron 7 of 15) | 33067 | ENSG00000173262 | SLC2A14 |
| chr6 | 35549011 | 35549686 | 0,12 | 3,05 | -2,93 | 0,00411 | 0,0475 | 3'UTR | -68364 | ENSG00000 112041 | TULP1 |
| chr1 | 113394463 | 113395415 | 0,46 | 3,3 | -2,84 | 0,00412 | 0,0476 | Intron (uc021orw.1/uc021orw.1, intron 1 of 1) | -70557 | NA | AKR7A2P1 |
| chr1 | 3781084 | 3781451 | -1,01 | 1,37 | -2,38 | 0,00414 | 0,0478 | Intron (uc009vln.1/1677, intron 2 of 2) | 7239 | ENSG00000169598 | DFFB |
| chr15 | 90573700 | 90576002 | 2,84 | 5,66 | -2,82 | 0,00414 | 0,0478 | Intron (uc002bov.2/374655, intron 1 of 4) | 28948 | ENSG00000 140548 | ZNF710 |
| chr22 | 25310561 | 25311520 | 0,51 | 3,63 | -3,12 | 0,00415 | 0,0478 | Intron (uc003abg.2/129049, intron 23 of 25) | 23794 | ENSGO0000206069 | TMEM211 |
| chr9 | 74682230 | 74682478 | -0,94 | 1,21 | -2,15 | 0,00416 | 0,0479 | Intron (uc011lsd.2/138240, intron 1 of 3) | 5256 | ENSG00000204669 | C9orf57 |
| chr1 1 | 84731850 | 84734161 | 1,88 | 5,53 | -3,65 | 0,00416 | 0,0479 | Intron (uc001pak.2/1740, intron 6 of 27) | -97385 | ENSG00000150672 | DLG2 |
| chr7 | 32727379 | 32729454 | 1,76 | 5,21 | -3,44 | 0,00416 | 0,0479 | Intron (uc011kai.2/23080, intron 10 of 11) | 29326 | NA | DPY19L1P1 |
| chr11 | 3252563 | 3254067 | 2,23 | 4,84 | -2,62 | 0,00417 | 0,048 | Promoter (<=1kb) | 0 | ENSG00000184350 | MRGPRE |
| chr15 | 20708660 | 20712917 | 1,5 | 4,52 | -3,02 | 0,00418 | 0,048 | Promoter (<=1kb) | 0 | ENSG00000180229 | HERC2P3 |
| chr12 | 109825426 | 109825802 | -0,27 | 2,2 | -2,47 | 0,00419 | 0,0481 | Promoter (<=1kb) | -722 | ENSG00000174527 | MYO1H |
| chr21 | 48017304 | 48018870 | 1,12 | 4,45 | -3,33 | 0,00419 | 0,0481 | 3'UTR | 6165 | ENSG00000160307 | S100B |
| chr10 | 129781834 | 129782727 | 0,68 | 3,49 | -2,81 | 0,00422 | 0,0484 | Promoter (2-3kb) | -2817 | ENSG00000132334 | PTPRE |
| chr2 | 234225581 | 234226317 | -0,29 | 2,41 | -2,7 | 0,00423 | 0,0485 | Promoter (1-2kb) | -1877 | ENSG00000130561 | SAG |
| chr17 | 73091962 | 73092844 | 0,58 | 3,72 | -3,14 | 0,00423 | 0,0485 | Promoter (2-3kb) | 2464 | ENSG00000170190 | SLC16A5 |
| chr2 | 171679521 | 171680534 | 0 | 3,1 | -3,1 | 0,00425 | 0,0487 | Intron (uc002ugh.3/2571, intron 3 of 6) | 6321 | ENSG00000128683 | GAD1 |
| chr1 | 214619929 | 214621004 | 0,76 | 3,86 | -3,09 | 0,00426 | 0,0488 | Intron (uc021piv.1/5784, intron 2 of 16) | 17296 | ENSG00000152104 | PTPN14 |
| chr14 | 94743454 | 94743856 | -0,75 | 1,89 | -2,64 | 0,00426 | 0,0488 | Intron (uc001vcs.1/57718, intron 24 of 24) | 15505 | ENSG00000140093 | SERPINA10 |
| chr22 | 49856184 | 49857334 | 1,31 | 4,41 | -3,1 | 0,00431 | 0,0491 | Intron (uc003biq.3/uc003biq 3, intron 1 of 4) | 193856 | ENSG00000188511 | C22orf34 |
| chr7 | 29705617 | 29706111 | -0,79 | 1,71 | -2,51 | 0,00431 | 0,0492 | Intron (uc003tad.4/646762, intron 1 of 1) | 14333 | ENSG00000212024 | MIR550A3 |
| chr17 | 33522429 | 33523309 | 0 | 2,94 | -2,94 | 0,00432 | 0,0492 | Promoter (1-2kb) | -1017 | ENSG00000164729 | SLC35G3 |
| chr19 | 29023400 | 29026238 | 2,64 | 6,28 | -3,64 | 0,00433 | 0,0493 | Intron (uc002nsa.2/uc002nsa.2, intron 5 of 9) | -429800 | ENSG00000267339 | LINC00906 |
| chr10 | 29777048 | 29777427 | -0,8 | 1,69 | -2,49 | 0,00437 | 0,0496 | Intron (uc010qdw.1/6840, intron 9 of 23) | 8020 | ENSG00000197321 | SVIL |
| chr2 | 189919053 | 189920127 | 0,65 | 3,89 | -3,24 | 0,00438 | 0,0497 | Intron (uc010frx.3/1290, intron 19 of 37) | 30371 | ENSG00000204262 | COL5 A2 |
| chr6 | 129530666 | 129531380 | -0,07 | 2,81 | -2,87 | 0,00438 | 0,0497 | Intron (uc003qbn.3/3908, intron 12 of 63) | 326380 | ENSG00000196569 | LAMA2 |
| chrX | 18806417 | 18806834 | -0,28 | 2,17 | -2,44 | 0,00438 | 0,0497 | Intron (uc004cyp.3/5475, intron 12 of 18) | 58135 | ENSG00000086717 | PPEF1 |
| chr10 | 14584948 | 14585941 | 0,3 | 3,07 | -2,78 | 0,00439 | 0,0497 | Intron (uc001imx.1/83641, intron 1 of 3) | 4686 | ENSG00000065809 | FAM107B |
| chr6 | 54721285 | 54722509 | 0,98 | 3,75 | -2,76 | 0,00441 | 0,0499 | Intron (uc003pck.4/222584, intron 1 of 4) | 9716 | ENSG00000168143 | FAM83B |
| chr4 | 84028875 | 84036420 | 8,02 | 9,22 | -1,2 | 0,00441 | 0,05 | Promoter (<=1kb) | 0 | ENSG00000145287 | PLAC8 |

**Table 2: H3K4me3 differentially bound sites significantly enriched in the lenalidomide-resistant cell lines compared to the lenalidomide-sensitive cell lines**

| Seqnames | Start | End | Conc resistant | Conc sensitive | Fold | p.value | FDR | Annotation | Distance to TSS | ENSEMBL | SYMBOL |
|---|---|---|---|---|---|---|---|---|---|---|---|
| chr5 | 37737438 | 37742232 | 6,99 | -1,55 | 8,55 | 3,87E-14 | 2,87E-10 | Intron (uc003jkv.3/55100, intron 17 of 17) | 92692 | ENSG00000168621 | GDNF |
| chr13 | 41216310 | 41220457 | 6,24 | -1,55 | 7,8 | 2,82E-12 | 1,06E-08 | Intron (uc001uxl.4/2308, intron 1 of 2) | 20277 | ENSG00000150907 | FOXO1 |
| chr2 | 201240190 | 201249220 | 8,09 | -1,55 | 9,64 | 3,37E-12 | 1,12E-08 | Intron (uc002uvn.4/26010, intron 2 of 12) | 66517 | ENSG00000196141 | SPATS2L |
| chr6 | 91064244 | 91067552 | 6,47 | -1,55 | 8,02 | 3,90E-12 | 1,24E-08 | Distal Intergenic | 41783 | ENSG00000266760 | MIR4464 |
| chr3 | 98248685 | 98256597 | 8,28 | -1,55 | 9,83 | 3,11E-11 | 7,15E-08 | Promoter (<=1kb) | 0 | ENSG00000154165 | GPR15 |
| chr4 | 85418502 | 85421233 | 6,65 | -1,55 | 8,2 | 2,34E-10 | 3,41E-07 | Promoter (<=1kb) | 0 | ENSG00000163623 | NKX6-1 |
| chr14 | 91852767 | 91857703 | 5,87 | -1,55 | 7,42 | 3,28E-10 | 4,49E-07 | Intron (c010aty.3/440193, intron 3 of 29) | 25479 | ENSG00000015133 | CCDC88C |
| chr1 | 199035019 | 199040596 | 6,06 | -1,55 | 7,61 | 4,69E-10 | 5,91E-07 | Distal Intergenic | -128461 | NA | MIR181A1HG |
| chr7 | 137676189 | 137678651 | 5,43 | -1,55 | 6,98 | 1,02E-09 | 1,00E-06 | Intron (uc003vtw.3/64764, intron 1 of 11) | 8196 | ENSG00000182158 | CREB3 L2 |
| chr1 | 25954836 | 25961725 | 6,77 | -1,55 | 8,32 | 1,11E-09 | 1,05E-06 | Intron (uc001bkm.2/57134, intron 1 of 11) | 10877 | ENSG00000117643 | MAN1C1 |
| chr12 | 68550355 | 68553981 | 5,97 | -1,55 | 7,53 | 1,31E-09 | 1,16E-06 | Promoter (<=1kb) | 0 | ENSG00000111537 | IFNG |
| chr13 | 33777172 | 33780488 | 7,09 | 0,66 | 6,43 | 2,23E-09 | 1,69E-06 | Promoter (<=1kb) | 0 | ENSG00000133121 | STARD13 |
| chr4 | 46972761 | 46978803 | 6,55 | -1,55 | 8,1 | 2,55E-09 | 1,91E-06 | Exon (uc021xnz.1/2557, exon 6 of 9) | 16719 | ENSG00000109158 | GABRA4 |
| chr6 | 74103672 | 74106171 | 6,64 | -1,55 | 8,19 | 3,05E-09 | 2,18E-06 | Promoter (<=1kb) | 0 | ENSG00000203907 | OOEP |
| chr1 | 199044516 | 199053768 | 6,44 | -1,55 | 7,99 | 3,28E-09 | 2,26E-06 | Distal Intergenic | -137958 | NA | MIR181A1HG |
| chr8 | 89241034 | 89246468 | 6,56 | -1,1 | 7,66 | 3,70E-09 | 2,47E-06 | Intron (uc003yeb.4/4325, intron 1 of 9) | 93249 | ENSG00000156103 | MMP16 |
| chr8 | 132051110 | 132054942 | 6,48 | -1,55 | 8,03 | 4,94E-09 | 3,09E-06 | Promoter (<=1kb) | 0 | ENSG00000155897 | ADCY8 |
| chr10 | 52190349 | 52192012 | 5,07 | -1,55 | 6,62 | 6,03E-09 | 3,57E-06 | Intron (uc001jje.3/259230, intron 6 of 10) | -12409 | ENSG00000198964 | SGMS1 |
| chr19 | 42591908 | 42593835 | 5,78 | -1,38 | 7,17 | 6,51E-09 | 3,75E-06 | 3'UTR | 11618 | ENSG00000105732 | ZNF574 |
| chr4 | 105892645 | 105895402 | 5,18 | -1,55 | 6,73 | 7,25E-09 | 4,07E-06 | Distal Intergenic | -171630 | ENSG00000168769 | TET2 |
| chr11 | 93267554 | 93271685 | 5,97 | -1,55 | 7,53 | 1,26E-08 | 6,13E-06 | Intron (uc001pds.4/56935, intron 1 of 2) | 4861 | ENSG00000166002 | SMCO4 |
| chr8 | 107297698 | 107299450 | 4,59 | -1,55 | 6,15 | 1,35E-08 | 6,46E-06 | Intron (uc003ymf.3/55074, intron 1 of 15) | 15292 | ENSG00000164830 | OXR1 |
| chr11 | 307756 | 312632 | 6,12 | -0,19 | 6,31 | 1,73E-08 | 7,79E-06 | Promoter (<=1kb) | 0 | ENSG00000185201 | IFITM2 |
| chr17 | 44222887 | 44224931 | 4,42 | -1,55 | 5,98 | 1,85E-08 | 8,25E-06 | Intron (uc002ikc.3/284058, intron 2 of 14) | 45235 | ENSG00000120071 | KANSL1 |
| chr4 | 123865929 | 123867767 | 5,57 | -1,55 | 7,12 | 1,93E-08 | 8,55E-06 | Intron (uc003iev.3/166378, intron 8 of 10) | 21704 | ENSG00000145375 | SPATA5 |
| chr2 | 33712563 | 33714847 | 5,16 | -0,76 | 5,92 | 2,08E-08 | 9,02E-06 | Intron (uc002rox.3/25780, intron 2 of 18) | 11242 | ENSG00000152689 | RASGRP3 |
| chr1 | 7885941 | 7891202 | 7,16 | -1,55 | 8,71 | 2,28E-08 | 9,58E-06 | 3'UTR | 21976 | ENSG00000049247 | UTS2 |
| chr14 | 92978754 | 92982455 | 8,57 | 4,26 | 4,31 | 2,30E-08 | 9,62E-06 | Promoter (<=1kb) | 0 | ENSG00000100599 | RIN3 |
| chr17 | 44228036 | 44232100 | 5,72 | -0,04 | 5,76 | 2,41E-08 | 9,81E-06 | Intron (uc002ikc.3/284058, intron 2 of 14) | 38066 | ENSG00000120071 | KANSL1 |
| chr2 | 158332665 | 158338735 | 7 | -1,55 | 8,55 | 2,49E-08 | 9,96E-06 | Intron (uc010zcl.1/9595, intron 3 of 10) | 6738 | ENSG00000115165 | CYTIP |
| chr6 | 161449250 | 161454082 | 5,92 | -1,55 | 7,47 | 2,55E-08 | 1,01E-05 | Intron (uc010kkc.2/4216, intron 1 of 22) | 36428 | ENSG00000085511 | MAP3K4 |
| chr4 | 160098472 | 160102695 | 6,2 | -0,39 | 6,59 | 2,56E-08 | 1,01E-05 | Distal Intergenic | -48426 | ENSG00000264105 | MIR3688-1 |
| chr13 | 25754035 | 25756933 | 6,02 | 0,19 | 5,83 | 2,62E-08 | 1,01E-05 | Exon (uc001uqd.3/uc001uqd.3, exon 1 of 2) | -7730 | ENSG00000165566 | AMER2 |
| chr4 | 71578386 | 71581710 | 5,35 | -1,55 | 6,9 | 2,69E-08 | 1,03E-05 | Intron (uc003hfp.4/22902, intron 1 of 11) | -5986 | ENSG00000018189 | RUFY3 |
| chr18 | 77443134 | 77445544 | 4,78 | -1,55 | 6,34 | 3,54E-08 | 1,25E-05 | Promoter (1-2kb) | 1704 | ENSG00000060069 | CTDP1 |
| chr21 | 30206383 | 30210259 | 6,19 | -1,55 | 7,74 | 4,02E-08 | 1,37E-05 | Distal Intergenic | 47436 | ENSG00000156239 | N6AMT1 |
| chr4 | 42640494 | 42646781 | 6,19 | -1,14 | 7,33 | 4,06E-08 | 1,3 8E-0 5 | Intron (uc003gwr.2/10396, intron 1 of 36) | 12341 | ENSG00000124406 | ATP8A1 |
| chr8 | 125618135 | 125620871 | 5,33 | -1,55 | 6,88 | 4,11E-08 | 1,39E-05 | Intron (uc003yrj.2/9788, intron 3 of 13) | -37073 | ENSG00000170873 | MTSS1 |
| chr1 | 200831208 | 200833094 | 4,9 | -1,55 | 6,45 | 4,22E-08 | 1,41E-05 | Distal Intergenic | -8989 | ENSG00000170128 | GPR25 |
| chr1 | 73847497 | 73852751 | 6,5 | -1,55 | 8,05 | 4,28E-08 | 1,42E-05 | Distal Intergenic | 811120 | ENSG00000162620 | LRRIQ3 |
| chr11 | 66102980 | 66104810 | 6,01 | -1,55 | 7,56 | 4,63E-08 | 1,50E-05 | Promoter (<=1kb) | 0 | ENSG00000174791 | RIN1 |
| chr7 | 142506699 | 142508994 | 5,6 | -1,55 | 7,15 | 4,81E-08 | 1,53E-05 | Distal Intergenic | 26790 | NA | PRSS3P2 |
| chr1 | 73829008 | 73833368 | 6,45 | -1,55 | 8 | 5,39E-08 | 1,68E-05 | Distal Intergenic | 830503 | ENSG00000162620 | LRRIQ3 |
| chr5 | 25188173 | 25193623 | 6,96 | -1,55 | 8,51 | 5,70E-08 | 1,76E-05 | Distal Intergenic | -347481 | ENSG00000248908 | LINC02239 |
| chr10 | 90757606 | 90763128 | 6,08 | -1,06 | 7,14 | 6,02E-08 | 1,82E-05 | Exon (uc010qna.2/355, exon 3 of 10) | -4874 | NA | FAS-AS1 |
| chr4 | 69438031 | 69442287 | 5,71 | -0,77 | 6,48 | 7,54E-08 | 2,14E-05 | Distal Intergenic | -3786 | ENSG00000197888 | UGT2B17 |
| chr10 | 12396965 | 12399113 | 5,58 | -1,55 | 7,13 | 9,12E-08 | 2,49E-05 | Intron (uc001iln.3/57118, intron 1 of 9) | 5382 | ENSG00000183049 | CAMK1D |
| chr6 | 158221815 | 158222928 | 3,98 | -1,55 | 5,53 | 9,57E-08 | 2,59E-05 | Distal Intergenic | -21366 | ENSG00000130340 | SNX9 |
| chrX | 19699651 | 19702575 | 5 | -1,55 | 6,56 | 1,04E-07 | 2,75E-05 | Exon (uc004czl.3/30011, exon 5 of 17) | -10532 | ENSG00000147010 | SH3KBP1 |
| chr4 | 153444044 | 153450032 | 5,81 | -1,55 | 7,36 | 1,14E-07 | 2,99E-05 | Intron (uc003ims.3/55294, intron 1 of 11) | 6153 | ENSG00000109670 | FBXW7 |
| chr1 | 198703042 | 198705375 | 4,4 | -1,55 | 5,96 | 1,23E-07 | 3,16E-05 | Exon (uc010ppg.2/5788, exon 20 of 22) | 94624 | ENSG00000081237 | PTPRC |
| chr3 | 175194222 | 175196368 | 4,6 | -1,55 | 6,15 | 1,27E-07 | 3,22E-05 | Intron (uc003fit.3/254827, intron 9 of 13) | 106893 | ENSG00000264974 | MIR4789 |
| chr20 | 50795574 | 50797388 | 4,62 | -1,55 | 6,17 | 1,30E-07 | 3,28E-05 | Intron (uc002xwk.3/55734, intron 2 of 8) | 11136 | ENSG00000020256 | ZFP64 |
| chr5 | 76005779 | 76008465 | 5,19 | -1,55 | 6,75 | 1,36E-07 | 3,40E-05 | Promoter (<=1kb) | 0 | NA | NCRUPAR |
| chr6 | 45888363 | 45890246 | 4,77 | -1,55 | 6,32 | 1,37E-07 | 3,43E-05 | Exon (uc031sos.1/53405, exon 2 of 5) | 15088 | ENSG00000112782 | CLIC5 |
| chr4 | 46088771 | 46096185 | 6,44 | -1,23 | 7,67 | 1,3 8E-07 | 3,43E-05 | Intron (uc003gxb.3/2565, intron 2 of 8) | 29897 | ENSG00000163285 | GABRG1 |
| chr3 | 165473289 | 165484606 | 7,01 | -1,55 | 8,56 | 1,43E-07 | 3,52E-05 | Distal Intergenic | 70647 | ENSG00000114200 | BCHE |
| chr7 | 153077097 | 153078859 | 4,36 | -1,55 | 5,92 | 1,49E-07 | 3,65E-05 | Distal Intergenic | -505560 | ENSG00000130226 | DPP6 |
| chr17 | 34415215 | 34418778 | 7,62 | 3,4 | 4,23 | 1,53E-07 | 3,71E-0 5 | Promoter (<=1kb) | 0 | ENSG00000277632 | CCL3 |
| chr13 | 62612582 | 62616063 | 6,24 | -0,65 | 6,89 | 1,58E-07 | 3,82E-05 | Distal Intergenic | -610503 | ENSG00000280 165 | PCDH20 |
| chr5 | 16735073 | 16738799 | 5,75 | -1,55 | 7,3 | 1,98E-07 | 4,62E-05 | Promoter (<=1kb) | 0 | ENSG00000145555 | MYO10 |
| chr2 | 7577971 | 7579211 | 4,58 | -0,77 | 5,35 | 2,05E-07 | 4,77E-05 | Intron (uc002qyv.3/100506274, intron 4 of 5) | 16579 | ENSG00000229727 | LOC100506274 |
| chr3 | 43666220 | 43669514 | 5,27 | -1,55 | 6,82 | 2,12E-07 | 4,89E-05 | Promoter (2-3kb) | -2660 | ENSG00000160746 | ANO10 |
| chr4 | 156128594 | 156130346 | 5,49 | -1,55 | 7,04 | 2,25E-07 | 5,07E-05 | Promoter (<=1kb) | 0 | ENSG00000185149 | NPY2R |
| chr5 | 53832735 | 53834418 | 4,92 | -1,55 | 6,47 | 2,30E-07 | 5,12E-05 | Intron (uc011cqg.2/112574, intron 1 of 1) | 19142 | ENSG00000178996 | SNX18 |
| chr6 | 106630610 | 106635868 | 5,89 | -1,55 | 7,45 | 2,31E-07 | 5,12E-05 | 3'UTR | 83873 | ENSG00000057657 | PRDM1 |
| chr5 | 37735464 | 37736936 | 3,68 | -1,55 | 5,24 | 2,41E-07 | 5,30E-05 | Intron (uc003jkv.3/55100, intron 17 of 17) | 97988 | ENSG00000168621 | GDNF |
| chr10 | 100161450 | 100165406 | 4,68 | -1,55 | 6,23 | 2,45E-07 | 5,38E-05 | Intron (uc001kpb.3/84795, intron 4 of 14) | 6228 | ENSG00000 119943 | PYROXD2 |
| chr2 | 188856692 | 188860853 | 5,52 | -1,55 | 7,07 | 2,90E-07 | 6,18E-05 | Distal Intergenic | -295543 | ENSG00000144366 | GULP1 |
| chr4 | 189864027 | 189868374 | 5,53 | -1,55 | 7,08 | 2,97E-07 | 6,30E-05 | Distal Intergenic | 487295 | ENSG00000249378 | LINC01060 |
| chr3 | 153885284 | 153890416 | 5,44 | -1,55 | 6,99 | 3,00E-07 | 6,32E-05 | Intron (uc021xgc.1/26084. intron 6 of 14) | 46135 | ENSG00000114790 | ARHGEF26 |
| chr18 | 33195302 | 33197818 | 4,78 | -1,55 | 6,33 | 3,01E-07 | 6,32E-05 | Intron (uc002kvz.4/2589, intron 1 of 12) | 23601 | ENSG00000283514 | MIR3975 |
| chr5 | 53316492 | 53318990 | 5,27 | -1,55 | 6,82 | 3,27E-07 | 6,68E-05 | Intron (uc003jpg.1/54622, intron 4 of 4) | -69063 | ENSG00000207627 | MIR581 |
| chr6 | 4871252 | 4873710 | 4,79 | -1,55 | 6,34 | 3,45E-07 | 6,90E-05 | Intron (uc003mwi.3/9425, intron 3 of 8) | -16516 | ENSG00000153046 | CDYL |
| chr3 | 98280338 | 98283663 | 4,77 | -1,02 | 5,79 | 3,65E-07 | 7,24E-05 | Distal Intergenic | 28792 | ENSG00000080819 | CPOX |
| chr2 | 21419426 | 21423454 | 5,48 | -1,55 | 7,04 | 3,67E-07 | 7,25E-05 | Distal Intergenic | -152481 | ENSG00000084674 | APOB |
| chr7 | 4922453 | 4923624 | 5,12 | -1,55 | 6,67 | 3,77E-07 | 7,37E-05 | Promoter (<=1kb) | 0 | ENSG00000157927 | RADIL |
| chr7 | 142504662 | 142506581 | 4,57 | -1,55 | 6,12 | 4,09E-07 | 7,86E-05 | Distal Intergenic | 24753 | NA | PRSS3P2 |
| chr12 | 1824302 | 1826212 | 4,58 | -1,55 | 6,14 | 4,24E-07 | 8,07E-05 | Intron (uc001qjm.3/79602, intron 1 of 7) | 24041 | ENSG00000006831 | ADIPOR2 |
| chr9 | 5919445 | 5924159 | 5,32 | -1,55 | 6,87 | 4,27E-07 | 8,07E-05 | 3'UTR | 28536 | ENSG00000120215 | MLANA |
| chr3 | 23872550 | 23873670 | 3,98 | -1,55 | 5,53 | 4,31E-07 | 8,12E-05 | Intron (uc003cch.3/7324, intron 3 of 5) | 20616 | ENSG00000170142 | UBE2E1 |
| chr1 | 239578882 | 239585077 | 5,41 | -1,55 | 6,96 | 4,36E-07 | 8,15E-05 | Intron (uc001hy0.1/1131, intron 1 of 8) | 29017 | ENSG00000133019 | CHRM3 |
| chr11 | 5710353 | 5713536 | 6,37 | -0,61 | 6,98 | 4,41E-07 | 8,22E-05 | Promoter (<=1kb) | 0 | ENSG00000132274 | TRIM22 |
| chr16 | 82685054 | 82690086 | 5,64 | -0,17 | 5,81 | 4,44E-07 | 8,25E-05 | Intron (uc010chh.3/1012, intron 1 of 4) | 24655 | ENSG00000140945 | CDH13 |
| chr9 | 98585314 | 98585923 | 3,23 | -1,55 | 4,79 | 4,60E-07 | 8,50E-05 | Intron (uc004avp.4/100128782, intron 2 of 2) | -51977 | ENSG00000 182150 | ERCC6L2 |
| chr2 | 95962476 | 95964164 | 5,04 | -1,55 | 6,59 | 4,63E-07 | 8,52E-05 | Promoter (<=1kb) | 0 | ENSG00000115041 | KCNIP3 |
| chr17 | 44253885 | 44257523 | 6,03 | 0,24 | 5,79 | 5,19E-07 | 9,27E-05 | Intron (uc002ikc.3/284058, intron 1 of 14) | 12643 | ENSG00000120071 | KANSL1 |
| chr3 | 170999515 | 171003300 | 4,87 | -1,55 | 6,42 | 5,31E-07 | 9,43E-05 | Intron (uc003fhh.2/23043, intron 2 of 32) | 174897 | ENSG00000154310 | TNIK |
| chr4 | 3766894 | 3769189 | 4,94 | -1,55 | 6,5 | 5,35E-07 | 9,48E-05 | Promoter (<=1kb) | 0 | ENSG00000 184160 | ADRA2C |
| chr6 | 117868705 | 117872341 | 6,83 | 0,79 | 6,03 | 5,88E-07 | 0,000103 | 3'UTR | 8287 | ENSG00000164465 | DCBLD1 |
| chr5 | 140207270 | 140208333 | 4,91 | -1,55 | 6,46 | 6,50E-07 | 0,000112 | Promoter (<=1kb) | 0 | ENSG00000081842 | PCDHA6 |
| chr4 | 15772423 | 15774179 | 4,22 | -0,72 | 4,94 | 6,72E-07 | 0,000115 | Distal Intergenic | -5752 | ENSG00000004468 | CD3 8 |
| chr5 | 36172844 | 36174917 | 5,16 | -1,55 | 6,72 | 6,80E-07 | 0,000116 | Intron (uc003jkc.2/6502, intron 7 of 9) | 20699 | ENSG00000145604 | SKP2 |
| chr7 | 76955883 | 76958790 | 5,58 | -1,55 | 7,13 | 6,81E-07 | 0,000116 | Promoter (<=1kb) | 0 | ENSG00000186088 | GSAP |
| chr6 | 79793235 | 79794455 | 4,55 | -1,04 | 5,59 | 7,08E-07 | 0,000119 | Distal Intergenic | -5224 | ENSG00000146247 | PHIP |
| chr3 | 165527782 | 165531035 | 5,25 | -1,55 | 6,8 | 7,08E-07 | 0,000119 | Intron (uc003fem.4/590, intron 2 of 3) | 24218 | ENSG00000114200 | BCHE |
| chr8 | 68417800 | 68419108 | 3,76 | -1,4 | 5,16 | 7,33E-07 | 0,000123 | Exon (uc003xxq.4/57094, exon 6 of 11) | -161888 | ENSG00000066777 | ARFGEF1 |
| chr7 | 40514939 | 40515734 | 3,56 | -1,55 | 5,11 | 7,86E-07 | 0,000129 | Intron (uc003thn.2/79783, intron 11 of 13) | -317322 | ENSG00000175600 | SUGCT |
| chr5 | 7876268 | 7878648 | 4,14 | -1,55 | 5,69 | 8,02E-07 | 0,000131 | Exon (uc010itn.1/4552, exon 4 of 6) | 7051 | ENSG00000124275 | MTRR |
| chr1 | 233734490 | 233735978 | 4,01 | -1,38 | 5,4 | 8,22E-07 | 0,000133 | Distal Intergenic | -13772 | ENSG00000135750 | KCNK1 |
| chr15 | 60864682 | 60868719 | 4,39 | -1,55 | 5,94 | 8,51E-07 | 0,000136 | Intron (uc002agt.4/6095, intron 1 of 9) | 15988 | ENSG00000069667 | RORA |
| chr8 | 1426866 | 1428908 | 4,87 | -1,55 | 6,42 | 8,71E-07 | 0,000137 | Distal Intergenic | -20624 | ENSG00000198010 | DLGAP2 |
| chr2 | 191844177 | 191846411 | 5,46 | -1,55 | 7,01 | 8,86E-07 | 0,000137 | Exon (uc021vue.1/6772, exon 17 of 23) | 31993 | ENSG00000115415 | STAT1 |
| chr1 | 240470945 | 240473641 | 4,58 | -1,55 | 6,13 | 9,46E-07 | 0,000144 | Intron (uc010pvd.2/56776, intron 8 of 17) | 62385 | ENSG00000155816 | FMN2 |
| chr3 | 170972678 | 170975509 | 4,13 | -1,55 | 5,69 | 9,90E-07 | 0,000149 | Intron (uc003fhh.2/23043, intron 2 of 32) | -148130 | ENSG00000207963 | MIR569 |
| chr3 | 58404524 | 58408173 | 5,3 | -1,55 | 6,85 | 1,03E-06 | 0,000152 | 3'UTR | 11393 | ENSG00000168291 | PDHB |
| chr16 | 3138620 | 3140793 | 4,85 | -1,55 | 6,4 | 1,11E-06 | 0,00016 | Promoter (2-3kb) | 2068 | ENSG00000130182 | ZSCAN10 |
| chr3 | 107337010 | 107340608 | 5 | -1,55 | 6,56 | 1,17E-06 | 0,000167 | Intron (uc003dwk.5/56987, intron 2 of 16) | -24136 | ENSG00000114439 | BBX |
| chr11 | 100799645 | 100801158 | 4,11 | -1,55 | 5,67 | 1,20E-06 | 0,000171 | Intron (uc001pge.2/143872, intron 6 of 23) | -29423 | ENSG00000165895 | ARHGAP42 |
| chr8 | 128578070 | 128579342 | 4,08 | -1,01 | 5,09 | 1,22E-06 | 0,000173 | Distal Intergenic | -83686 | ENSG00000246228 | CASC8 |
| chr6 | 16712001 | 16714378 | 4,71 | -1,55 | 6,26 | 1,22E-06 | 0,000173 | Intron (uc003nbt.3/6310, intron 3 of 8) | 35946 | ENSG00000124788 | ATXN1 |
| chr3 | 102844116 | 102848104 | 4,77 | -1,55 | 6,33 | 1,24E-06 | 0,000175 | Distal Intergenic | 690257 | ENSG00000170044 | ZPLD1 |
| chr2 | 158341834 | 158344318 | 5,27 | -1,55 | 6,82 | 1,34E-06 | 0,000184 | Promoter (1-2kb) | 1155 | ENSG00000115165 | CYTIP |
| chr4 | 46923202 | 46925626 | 4,82 | -1,55 | 6,37 | 1,3 8E-06 | 0,000188 | 3'UTR | -11950 | ENSG00000170516 | COX7B2 |
| chr13 | 114539970 | 114542365 | 4,42 | -1,55 | 5,97 | 1,41E-06 | 0,000192 | Promoter (<=1kb) | -953 | ENSG00000183087 | GAS6 |
| chr2 | 178141132 | 178144092 | 4,95 | -1,55 | 6,51 | 1,42E-06 | 0,000192 | Intron (uc002uli.5/4780, intron 4 of 7) | -11273 | ENSG00000116044 | NFE2L2 |
| chr2 | 166247563 | 166248909 | 4,01 | -1,55 | 5,56 | 1,46E-06 | 0,000195 | 3'UTR | 10219 | ENSG00000136531 | SCN2A |
| chr6 | 135409867 | 135411011 | 4,07 | -1,55 | 5,62 | 1,52E-06 | 0,000201 | Distal Intergenic | -33831 | ENSG00000112339 | HBS1L |
| chr1 | 77879228 | 77880549 | 3,6 | -1,55 | 5,15 | 1,57E-06 | 0,000207 | Intron (uc001dhn.3/26289, intron 7 of 13) | 130941 | ENSG00000154027 | AK5 |
| chr8 | 4848528 | 4852824 | 5,79 | 0,03 | 5,76 | 1,59E-06 | 0,000209 | Promoter (<=1kb) | 0 | ENSG00000183117 | CSMD1 |
| chr8 | 138119677 | 138124494 | 5,22 | -1,55 | 6,77 | 1,59E-06 | 0,000209 | Distal Intergenic | 1040965 | ENSG00000147724 | FAM135B |
| chr3 | 47483399 | 47486706 | 4,79 | -1,55 | 6,34 | 1,63E-06 | 0,000213 | 5'UTR | 30739 | ENSG00000114650 | SCAP |
| chr3 | 170964925 | 170967441 | 4,38 | -1,55 | 5,93 | 1,65E-06 | 0,000215 | Intron (uc003fhh.2/23043, intron 2 of 32) | -140377 | ENSG00000207963 | MIR569 |
| chr2 | 169066923 | 169069810 | 4,72 | -1,55 | 6,27 | 1,65E-06 | 0,000215 | Intron (uc002uea.3/27347, intron 1 of 17) | 34295 | ENSG00000 198648 | STK39 |
| chr15 | 85303426 | 85305366 | 4,38 | -1,55 | 5,93 | 1,69E-06 | 0,000219 | Intron (uc002b1d.3/9640, intron 1 of 10) | 11608 | ENSG00000166716 | ZNF592 |
| chr1 1 | 78064708 | 78066686 | 4,73 | -1,55 | 6,28 | 1,70E-06 | 0,00022 | Intron (uc001ozh.3/9846, intron 1 of 9) | -11782 | ENSG00000033327 | GAB2 |
| chr1 | 161751583 | 161752564 | 3,76 | -1,55 | 5,31 | 1,78E-06 | 0,000227 | Exon (uc001gbq.2/22926, exon 3 of 14) | 15549 | ENSG00000 118217 | ATF6 |
| chr2 | 68443557 | 68446772 | 4,58 | -1,55 | 6,13 | 1,79E-06 | 0,000228 | Exon (uc002sei.1/5534, exon 2 of 6) | 32879 | ENSG00000221823 | PPP3R1 |
| chr18 | 41151333 | 41152875 | 3,89 | -1,55 | 5,45 | 1,80E-06 | 0,000228 | Distal Intergenic | -293718 | ENSG00000132872 | SYT4 |
| chr14 | 57457619 | 57459797 | 4,84 | -1,55 | 6,4 | 1,80E-06 | 0,000228 | Distal Intergenic | 177718 | ENSG00000248550 | OTX2-AS1 |
| chr7 | 15416266 | 15418874 | 4,56 | -1,55 | 6,12 | 1,81E-06 | 0,000228 | Intron (uc003stb.1/392636, intron 10 of 12) | 182766 | ENSG00000187546 | AGMO |
| chr8 | 75771149 | 75772456 | 4,81 | -1,55 | 6,37 | 1,81E-06 | 0,000228 | Intron (uc003yak.1/uc003yak.1, intron 2 of 2) | 34128 | ENSG00000137558 | PI15 |
| chr10 | 133108682 | 133111540 | 6,35 | -0,33 | 6,68 | 1,95E-06 | 0,00024 | Promoter (<=1kb) | 0 | ENSG00000176769 | TCERG1L |
| chr12 | 133048383 | 133052456 | 6,86 | 1,4 | 5,46 | 1,96E-06 | 0,00024 | Distal Intergenic | -14701 | ENSG00000112787 | FBRSL1 |
| chrl6 | 23279261 | 23281156 | 4,98 | -1,55 | 6,53 | 1,96E-06 | 0,000241 | Distal Intergenic | -32435 | ENSG00000168447 | SCNN1B |
| chr20 | 47354629 | 47356447 | 4,51 | -1,55 | 6,06 | 2,04E-06 | 0,000247 | Intron (uc002xtw.1/57580, intron 3 of 39) | -81511 | ENSG00000124126 | PREX1 |
| chr2 | 25523924 | 25525469 | 3,98 | -0,75 | 4,73 | 2,06E-06 | 0,000249 | Intron (uc002rgc.4/1788, intron 2 of 22) | 26121 | ENSG00000221445 | MIR1301 |
| chr13 | 74702265 | 74704918 | 4,15 | -1,55 | 5,7 | 2,12E-06 | 0,000255 | Intron (uc001vjf.3/11278, intron 1 of 7) | 3148 | ENSG00000118922 | KLF12 |
| chr4 | 188329009 | 188330775 | 5,09 | -0,86 | 5,95 | 2,19E-06 | 0,000261 | Intron (uc021xvl.1/339975, intron 1 of 4) | 95992 | NA | LOC339975 |
| chr4 | 170911205 | 170913197 | 4,09 | -1,38 | 5,47 | 2,23E-06 | 0,000264 | 3'UTR | 11738 | ENSG00000198948 | MFAP3L |
| chrl6 | 53697057 | 53699442 | 4,61 | -1,55 | 6,17 | 2,28E-06 | 0,000269 | Exon (uc002eho.4/23322, exon 10 of 25) | 38329 | ENSG00000103494 | RPGRIP1L |
| chr19 | 19271058 | 19272647 | 5,11 | -1,55 | 6,66 | 2,32E-06 | 0,000271 | Intron (uc002nll.2/100271849, intron 1 of 8) | 8451 | ENSG00000213999 | MEF2B |
| chr15 | 93956366 | 93958797 | 5,03 | -0,31 | 5,35 | 2,42E-06 | 0,00028 | Exon (uc002bsw.1/uc002bsw.1, exon 1 of 1) | -323923 | ENSG00000182175 | RGMA |
| chr12 | 109218958 | 109221657 | 5,04 | -1,55 | 6,59 | 2,44E-06 | 0,000281 | Promoter (<=1kb) | 0 | ENSG00000084112 | SSH1 |
| chr20 | 47356572 | 47358100 | 4,13 | -1,55 | 5,68 | 2,46E-06 | 0,000283 | Intron (uc002xtw.1/57580, intron 3 of 39) | -83454 | ENSG00000124126 | PREX1 |
| chr7 | 28001063 | 28007283 | 5,04 | -1,55 | 6,59 | 2,49E-06 | 0,000285 | Intron (uc003szm.3/221895, intron 1 of 3) | 24392 | ENSG00000153814 | JAZF1 |
| chr7 | 80465065 | 80467892 | 4,28 | -1,55 | 5,84 | 2,56E-06 | 0,00029 | Intron (uc003uhj.3/10512, intron 2 of 17) | 80775 | ENSG00000075223 | SEMA3 C |
| chr3 | 72445184 | 72446884 | 4,62 | -1,55 | 6,17 | 2,63E-06 | 0,000297 | Intron (uc003dpe.3/23429, intron 1 of 3) | 48890 | ENSG00000163602 | RYBP |
| chr20 | 21501514 | 21503720 | 4,22 | -1,55 | 5,77 | 2,67E-06 | 0,000299 | Distal Intergenic | -6850 | ENSG00000125820 | NKX2-2 |
| chr5 | 140235243 | 140236379 | 4,62 | -1,55 | 6,18 | 2,76E-06 | 0,000306 | Promoter (<=1kb) | 0 | ENSG00000250 120 | PCDHA10 |
| chr2 | 137120472 | 137121596 | 4,21 | -1,55 | 5,76 | 2,82E-06 | 0,00031 | Distal Intergenic | -244747 | ENSG00000 121966 | CXCR4 |
| chr22 | 50714244 | 50715437 | 3,95 | -1,4 | 5,35 | 3,10E-06 | 0,000332 | Exon (uc003bkt.1/23654, exon 14 of 17) | -4904 | ENSG00000185386 | MAPK11 |
| chr15 | 60847732 | 60849465 | 3,77 | -1,55 | 5,33 | 3,21E-06 | 0,000341 | Exon (uc002agv.3/6095, exon 3 of 12) | 35242 | ENSG00000069667 | RORA |
| chr2 | 112186386 | 112188243 | 3,13 | -1,26 | 4,39 | 3,29E-06 | 0,000346 | Exon (uc002thf.4/541471, exon 2 of 2) | 64449 | ENSG00000172965 | MIR4435-2HG |
| chr4 | 167261813 | 167264141 | 4,31 | -1,55 | 5,86 | 3,34E-06 | 0,00035 | Distal Intergenic | 467403 | ENSG00000038295 | TLL1 |
| chr19 | 40871031 | 40872216 | 4,48 | -1,55 | 6,03 | 3,39E-06 | 0,000354 | Promoter (<=1kb) | -110 | ENSG00000105223 | PLD3 |
| chr10 | 11569429 | 11570370 | 3,34 | -1,1 | 4,43 | 3,40E-06 | 0,000355 | Exon (uc001iks.1/9712. exon 2 of 14) | 3904 | ENSG00000148429 | USP6NL |
| chr8 | 93029142 | 93031947 | 4,56 | -1,55 | 6,12 | 3,50E-06 | 0,000362 | Promoter (<=1kb) | 0 | ENSG00000079102 | RUNX1T1 |
| chr9 | 14326676 | 14328259 | 3,55 | -1,55 | 5,1 | 3,52E-06 | 0,000362 | Intron (uc022bdp.1/4781, intron 1 of 8) | -12631 | ENSG00000147862 | NFIB |
| chr5 | 130867284 | 130868542 | 4,22 | -1,55 | 5,77 | 3,52E-06 | 0,000362 | Intron (uc003kvn.2/51735, intron 6 of 27) | -23384 | ENSG00000158987 | RAPGEF6 |
| chr14 | 90146717 | 90148625 | 4,78 | 0,74 | 4,04 | 3,54E-06 | 0,000363 | Distal Intergenic | -61223 | ENSG00000053254 | FOXN3 |
| chr17 | 4261909 | 4263213 | 3,62 | -1,55 | 5,17 | 3,55E-06 | 0,000363 | Intron (uc002fxs.3/7326, intron 1 of 5) | 6756 | ENSG00000132388 | UBE2G1 |
| chr16 | 4161555 | 4164293 | 5,09 | -0,77 | 5,86 | 3,56E-06 | 0,000364 | Promoter (1-2kb) | 1893 | ENSG00000162104 | ADCY9 |
| chr10 | 112752131 | 112753542 | 3,87 | -1,55 | 5,43 | 3,61E-06 | 0,000366 | Intron (uc001kzl.4/8036. intron 3 of 8) | 28248 | ENSG00000108061 | SHOC2 |
| chr4 | 35098234 | 35111954 | 8,66 | 1,49 | 7,17 | 3,74E-06 | 0,000375 | Distal Intergenic | 963503 | ENSG0000004 7365 | ARAP2 |
| chr1 | 190276794 | 190278042 | 3,81 | -1,55 | 5,36 | 3,84E-06 | 0,000383 | Intron (uc010pot.1/339479, intron 2 of 6) | 166900 | ENSG00000162670 | BRINP3 |
| chr7 | 50417618 | 50420136 | 4,37 | -1,55 | 5,92 | 3,84E-06 | 0,000383 | Intron (uc003tov.1/10320, intron 3 of 3) | 50384 | ENSG00000185811 | IKZF1 |
| chr6 | 4867797 | 4869625 | 4,85 | -0,28 | 5,13 | 4,00E-06 | 0,000393 | Intron (uc003mwi.3/9425, intron 3 of 8) | -20601 | ENSG00000153046 | CDYL |
| chr21 | 31460396 | 31462310 | 4,18 | -1,55 | 5,73 | 4,00E-06 | 0,000393 | Distal Intergenic | 76661 | ENSG00000156282 | CLDN17 |
| chr7 | 121923889 | 121925728 | 5,27 | -0,69 | 5,97 | 4,08E-06 | 0,000398 | Distal Intergenic | -17984 | ENSG00000230316 | FEZF1-AS1 |
| chr4 | 26131597 | 26133425 | 3,85 | -1,55 | 5,4 | 4,18E-06 | 0,000405 | Distal Intergenic | -187907 | ENSG00000168214 | RBPJ |
| chr2 | 181971795 | 181972770 | 3,77 | -1,55 | 5,32 | 4,20E-06 | 0,000405 | Distal Intergenic | 125944 | ENSG00000170035 | UBE2E3 |
| chr7 | 50355535 | 50358314 | 4,22 | -1,55 | 5,77 | 4,31E-06 | 0,000414 | Intron (uc003tov.1/10320, intron 1 of 3) | 7218 | ENSG00000185811 | IKZF1 |
| chr2 | 89441682 | 89445464 | 4,72 | -1,26 | 5,98 | 4,41E-06 | 0,000419 | Exon (uc031rol.1/uc031rol.1, exon 42 of 312) | 329798 | ENSG00000265510 | MIR4436A |
| chr17 | 38719138 | 38722030 | 4,43 | -1,55 | 5,98 | 4,50E-06 | 0,000425 | Promoter (<=1kb) | 0 | ENSG00000126353 | CCR7 |
| chr10 | 12400659 | 12401891 | 3,72 | -1,55 | 5,27 | 4,55E-06 | 0,000428 | Intron (uc001iln.3/57118, intron 1 of 9) | 9076 | ENSG00000183049 | CAMK1D |
| chr12 | 123911852 | 123913062 | 3,37 | -1,55 | 4,93 | 4,56E-06 | 0,000429 | Intron (uc001uev.1/196383, intron 2 of 3) | 8202 | ENSG00000150977 | RILPL2 |
| chr1 | 153778120 | 153779415 | 3,8 | -1,55 | 5,35 | 4,59E-06 | 0,000431 | 3'UTR | 30352 | ENSG00000143554 | SLC27A3 |
| chr13 | 41372256 | 41374825 | 4,61 | -1,55 | 6,17 | 4,64E-06 | 0,000435 | 5'UTR | 7344 | NA | TPTE2P5 |
| chr3 | 72475949 | 72476859 | 2,67 | -1,55 | 4,22 | 4,69E-06 | 0,000437 | Intron (uc003dpe.3/23429, intron 1 of 3) | 18915 | ENSG00000163602 | RYBP |
| chr7 | 139424634 | 139427259 | 4,46 | -1,55 | 6,01 | 4,80E-06 | 0,000443 | Intron (uc003vvd.4/28996, intron 1 of 14) | 50434 | ENSG00000064393 | HIPK2 |
| chr8 | 79607261 | 79608303 | 3,3 | -1,55 | 4,85 | 4,82E-06 | 0,000444 | Intron (uc003vbd.3/51101, intron 5 of 8) | 28306 | NA | LOC101241902 |
| chr2 | 120331933 | 120332350 | 2,46 | -1,55 | 4,01 | 4,94E-06 | 0,000453 | Intron (uc010yyq.3/200373, intron 5 of 17) | 29925 | ENSG00000163075 | CFAP221 |
| chr1 | 180355667 | 180357187 | 4,33 | -0,93 | 5,26 | 4,97E-06 | 0,000455 | Intron (uc001gog.3/84320. intron 6 of 7) | 50338 | ENSG00000265435 | MIR3121 |
| chr7 | 40684084 | 40685225 | 3,72 | -1,55 | 5,27 | 5,36E-06 | 0,00048 | Intron (uc003thn.2/79783, intron 12 of 13) | -147831 | ENSG00000175600 | SUGCT |
| chr13 | 41214163 | 41215414 | 3,43 | -1,55 | 4,98 | 5,45E-06 | 0,000484 | Intron (uc00luxl.4/2308, intron 1 of 2) | 25320 | ENSG00000150907 | FOXO1 |
| chr5 | 96430401 | 96431461 | 3,15 | -1,55 | 4,7 | 5,64E-06 | 0,000495 | 3'UTR | 47059 | ENSG00000145721 | LIX1 |
| chr18 | 50276634 | 50278077 | 4,04 | -1,55 | 5,59 | 5,64E-06 | 0,000495 | Promoter (<=1kb) | -347 | ENSG00000187323 | DCC |
| chr16 | 46855276 | 46857370 | 3,97 | -1,55 | 5,52 | 5,65E-06 | 0,000495 | Intron (uc002eek.1/3 88272, intron 2 of 3) | 7704 | ENSG00000155330 | C16orf87 |
| chr9 | 6416735 | 6419416 | 5,01 | 0,3 | 4,71 | 5,73E-06 | 0,000501 | Intron (uc003zjv.3/115426, intron 1 of 15) | 3584 | ENSG00000147854 | UHRF2 |
| chr8 | 77447438 | 77448081 | 3,59 | -1,55 | 5,14 | 5,91E-06 | 0,000513 | Distal Intergenic | -145434 | ENSG00000091656 | ZFHX4 |
| chr3 | 183250914 | 183252470 | 3,66 | -1,55 | 5,22 | 5,98E-06 | 0,000518 | Intron (uc003flr.3/89857, intron 1 of 6) | 21029 | ENSG00000172578 | KLHL6 |
| chr3 | 144013500 | 144015810 | 3,89 | -1,55 | 5,44 | 6,01E-06 | 0,00052 | Distal Intergenic | 321333 | ENSG00000 181744 | DIPK2A |
| chr5 | 150447436 | 150449573 | 3,8 | -1,55 | 5,36 | 6,04E-06 | 0,000521 | Promoter (2-3kb) | -2748 | ENSG00000145901 | TNIP1 |
| chr21 | 32567424 | 32569084 | 3,73 | -1,55 | 5,29 | 6,05E-06 | 0,000521 | Exon (uc01 ladk.1/7074, exon 11 of 26) | 80140 | ENSG00000156299 | TIAM1 |
| chr8 | 62616952 | 62619204 | 4,37 | -1,55 | 5,92 | 6,15E-06 | 0,000528 | Intron (uc003xuj.3/444, intron 1 of 24) | 7995 | ENSG00000198363 | ASPH |
| chr14 | 32902071 | 32905513 | 5,16 | -0,98 | 6,14 | 6,46E-06 | 0,000548 | 5'UTR | 103592 | ENSG00000151320 | AKAP6 |
| chr21 | 36339673 | 36342970 | 4,35 | -1,55 | 5,9 | 6,47E-06 | 0,000548 | Intron (uc010gmu.3/861, intron 2 of 8) | 68753 | NA | RUNX1-IT1 |
| chr9 | 3507520 | 3509731 | 4,48 | -1,55 | 6,03 | 6,71E-06 | 0,000563 | Intron (uc003zhr.3/5991, intron 1 of 17) | 16252 | ENSG00000080298 | RFX3 |
| chr5 | 31769971 | 31771694 | 3,81 | -1,55 | 5,36 | 6,86E-06 | 0,000571 | Intron (uc003jhl.3/23037, intron 1 of 24) | -27302 | ENSG00000133401 | PDZD2 |
| chr12 | 12133607 | 12134951 | 3,78 | -1,55 | 5,33 | 6,93E-06 | 0,000575 | Intron (uc001raa.1/2120, intron 1 of 1) | -88927 | ENSG00000121380 | BCL2L14 |
| chr21 | 20115853 | 20118019 | 4,35 | -1,55 | 5,91 | 6,95E-06 | 0,000575 | Intron (uc002ykx.3/uc002ykx.3, intron 1 of 3) | -339883 | ENSG00000154646 | TMPRSS15 |
| chr14 | 102666922 | 102668872 | 4,21 | -1,55 | 5,76 | 6,96E-06 | 0,000575 | Intron (uc001ykz.3/91833, intron 2 of 2) | 38086 | ENSG00000080823 | MOK |
| chr7 | 124810140 | 124811275 | 3,91 | -1,55 | 5,46 | 7,04E-06 | 0,00058 | Intron (uc003vlq.1/uc003vlq.1, intron 5 of 5) | -240103 | ENSG00000128513 | POT1 |
| chr17 | 32830573 | 32831883 | 3,4 | -1,55 | 4,96 | 7,07E-06 | 0,00058 | Distal Intergenic | 74505 | ENSG00000197322 | C17orf102 |
| chr8 | 41753414 | 41755202 | 5,21 | -0,4 | 5,61 | 7,10E-06 | 0,000581 | Promoter (<=1kb) | 0 | ENSG00000029534 | ANK1 |
| chr1 | 211475867 | 211479139 | 4,55 | -1,55 | 6,1 | 7,10E-06 | 0,000581 | Exon (uc010psv.1/55758, exon 12 of 13) | -6440 | ENSG00000117625 | RCOR3 |
| chr19 | 2525006 | 2526369 | 4,65 | -1,55 | 6,2 | 7,26E-06 | 0,00059 | Intron (uc0021wd.2/2788, intron 3 of 4) | 48652 | ENSG00000099860 | GADD45B |
| chr9 | 73134793 | 73136036 | 3,98 | -1,55 | 5,53 | 7,68E-06 | 0,000616 | Distal Intergenic | -105220 | ENSG00000119138 | KLF9 |
| chr4 | 156710348 | 156711229 | 3,27 | -1,55 | 4,82 | 7,82E-06 | 0,000625 | Exon (uc003ipc.3/2983, exon 5 of 14) | 29840 | ENSG00000061918 | GUCY1B1 |
| chr3 | 175409617 | 175410956 | 4,08 | -1,55 | 5,64 | 7,94E-06 | 0,000632 | Intron (uc003fit.3/254827, intron 11 of 13) | 322288 | ENSG00000264974 | MIR4789 |
| chr4 | 106226924 | 106228366 | 3,75 | -1,55 | 5,31 | 8,21E-06 | 0,00065 | Distal Intergenic | 71870 | ENSG00000168769 | TET2 |
| chr1 | 23079842 | 23081361 | 3,81 | -1,55 | 5,36 | 8,30E-06 | 0,000655 | Intron (uc009vqj.1/2048,intron 1 of 16) | 33832 | ENSG00000265422 | MIR4684 |
| chr20 | 61469185 | 61470045 | 3,54 | -1,17 | 4,7 | 8,39E-06 | 0,000661 | Promoter (1-2kb) | 1414 | ENSG00000092758 | COL9 A3 |
| chr7 | 119718564 | 119720235 | 3,73 | -1,55 | 5,28 | 8,56E-06 | 0,00067 | Distal Intergenic | -193487 | ENSG00000 184408 | KCND2 |
| chr2 | 159069466 | 159071468 | 4,74 | -0,34 | 5,08 | 8,93E-06 | 0,000692 | Intron (uc021vro.1/554201, intron 4 of 4) | 46304 | ENSG00000227480 | CCDC148-AS1 |
| chr3 | 43367949 | 43369684 | 3,92 | -1,55 | 5,47 | 8,98E-06 | 0,000694 | Intron (uc003cms.4/54861, intron 3 of 6) | 23359 | ENSG00000163788 | SNRK |
| chr1 | 227102888 | 227103992 | 4,12 | -1,55 | 5,68 | 9,02E-06 | 0,000696 | Intron (uc00lhqm.1/56997, intron 4 of 19) | 18299 | ENSG00000163050 | COQ8A |
| chr21 | 27944531 | 27945854 | 4,68 | -1,23 | 5,91 | 9,05E-06 | 0,000698 | Promoter (<=1kb) | 0 | ENSG00000166265 | CYYR1 |
| chr13 | 32633477 | 32636717 | 4,9 | -1,02 | 5,92 | 9,06E-06 | 0,000698 | Intron (uc001utx.3/10129, intron 1 of 60) | 28040 | ENSG00000073910 | FRY |
| chr2 | 39135078 | 39136342 | 3,7 | -1,55 | 5,26 | 9,10E-06 | 7,00E-04 | Distal Intergenic | -10162 | ENSG00000214694 | ARHGEF33 |
| chr16 | 29836121 | 29837507 | 3,51 | -1,55 | 5,07 | 9,18E-06 | 0,000705 | Intron (uc010bzh.2/9961, intron 1 of 6) | 4406 | ENSG00000013364 | MVP |
| chr3 | 123255015 | 123256300 | 3,7 | -1,55 | 5,26 | 9,26E-06 | 0,000707 | Intron (uc003egj.2/201562. intron 3 of 6) | 47624 | ENSG00000206527 | HACD2 |
| chr14 | 74162352 | 74164194 | 3,75 | -1,55 | 5,3 | 9,59E-06 | 0,000726 | 3'UTR | 16934 | ENSG00000176903 | PNMA1 |
| chr13 | 36786906 | 36789171 | 5,87 | -0,31 | 6,19 | 9,93E-06 | 0,000744 | Promoter (<=1kb) | 0 | ENSG00000120669 | SOHLH2 |
| chr12 | 29668040 | 29670427 | 4,48 | -1,55 | 6,03 | 1,00E-05 | 0,00075 | Promoter (1-2kb) | 1960 | ENSG00000133687 | TMTC1 |
| chr13 | 78470461 | 78473102 | 4,01 | -1,55 | 5,57 | 1,05E-05 | 0,000774 | 3'UTR | 19864 | ENSG00000136160 | EDNRB |
| chr8 | 89217950 | 89218904 | 3,4 | -1,29 | 4,69 | 1,05E-05 | 0,000776 | Intron (uc003veb.4/4325, intron 1 of 9) | 120813 | ENSG00000156103 | MMP16 |
| chr3 | 67051350 | 67052554 | 3,88 | -1,55 | 5,43 | 1,06E-05 | 0,000782 | Promoter (2-3kb) | 2623 | ENSG00000163376 | KBTBD8 |
| chr5 | 79486808 | 79489590 | 4,92 | -0,65 | 5,57 | 1,07E-05 | 0,000784 | Intron (uc003kgj.3/256987, intron 2 of 12) | 62308 | ENSG00000164300 | SERINC5 |
| chr7 | 13983423 | 13986174 | 4,46 | -1,55 | 6,01 | 1,07E-05 | 0,000784 | Intron (uc021zzt.1/2115,intron2 of 9) | 38971 | ENSG00000006468 | ETV1 |
| chr2 | 145006069 | 145007304 | 3,78 | -1,55 | 5,34 | 1,08E-05 | 0,000785 | Intron (uc002tvp.3/79712, intron 1 of 11) | -11163 | ENSG00000 121964 | GTDC1 |
| chr2 | 33129449 | 33131451 | 3,96 | -1,55 | 5,51 | 1,08E-05 | 0,000785 | Intron (uc002roo.2/285045, intron 4 of 5) | -20743 | NA | NA |
| chr5 | 19488346 | 19489280 | 3,54 | -1,55 | 5,09 | 1,12E-05 | 0,000808 | Intron (uc021xwu.1/1016, intron 11 of 12) | 397101 | ENSG00000145526 | CDH18 |
| chr1 | 83490325 | 83491533 | 3,88 | -1,55 | 5,43 | 1,12E-05 | 0,000808 | Intron (uc001pai.2/1740, intron 10 of 19) | -96857 | ENSG00000150672 | DLG2 |
| chr3 | 72869887 | 72872411 | 4,42 | -0,93 | 5,35 | 1,17E-05 | 0,000839 | Intron (uc003dpf.3/55164, intron 6 of 10) | 25187 | ENSG00000144736 | SHQ1 |
| chr7 | 104751834 | 104753334 | 3,9 | -1,55 | 5,45 | 1,19E-05 | 0,000847 | Promoter (2-3kb) | 2445 | ENSG00000005483 | KMT2E |
| chrl7 | 16593442 | 16594894 | 3,79 | -1,55 | 5,35 | 1,20E-05 | 0,000853 | Promoter (<=1kb) | 0 | ENSG00000170160 | CCDC144A |
| chr1 | 238405209 | 238407466 | 4,27 | -1,55 | 5,82 | 1,20E-05 | 0,000853 | Distal Intergenic | 241851 | ENSG00000215808 | LINC01139 |
| chr3 | 48063242 | 48064875 | 3,92 | -1,55 | 5,47 | 1,21E-0 5 | 0,000858 | Intron (uc003csb.2/4134, intron 1 of 18) | 65894 | ENSG00000047849 | MAP4 |
| chr9 | 138557486 | 138559767 | 5,74 | 0,35 | 5,39 | 1,21E-0 5 | 0,000861 | Promoter (2-3kb) | 2318 | ENSG00000148386 | LCN9 |
| chr22 | 51130343 | 51132876 | 3,96 | -1,55 | 5,51 | 1,23E-05 | 0,000866 | Intron (uc031rvd.1/85358. intron 9 of 21) | -3116 | ENSG00000251322 | SHANK3 |
| chr19 | 8590820 | 8591843 | 4 | -1,55 | 5,55 | 1,23E-05 | 0,000868 | Exon (uc002mkg.3/4542, exon 23 of 28) | -11772 | ENSG00000133250 | ZNF414 |
| chr7 | 154861834 | 154863176 | 3,98 | -1,11 | 5,08 | 1,24E-05 | 0,000873 | Promoter (<=1kb) | 0 | ENSG00000157219 | HTR5A |
| chr5 | 147466622 | 147468866 | 4,14 | -1,55 | 5,7 | 1,27E-05 | 0,000886 | Exon (uc010jgq.1/11005, exon 6 of 12) | 23087 | ENSG00000133710 | SPINK5 |
| chr1 | 119132683 | 119135042 | 3,97 | -1,55 | 5,52 | 1,28E-05 | 0,000889 | Intron (uc001pwe.4/867, intron 2 of 15) | 46571 | ENSG00000076706 | MCAM |
| chr4 | 54456716 | 54458384 | 4,55 | -0,56 | 5,11 | 1,29E-05 | 0,000895 | Promoter (<=1kb) | 0 | ENSG00000072201 | LNX1 |
| chr3 | 165384162 | 165386292 | 4,06 | -1,55 | 5,61 | 1,29E-05 | 0,000896 | Distal Intergenic | 168961 | ENSG00000114200 | BCHE |
| chr2 | 161257984 | 161259265 | 3,12 | -1,55 | 4,67 | 1,34E-05 | 0,000918 | Intron (uc002ubi.4/5937, intron 1 of 14) | 5128 | ENSG00000283734 | MIR4785 |
| chr6 | 135435182 | 135436142 | 3,22 | -1,29 | 4,5 | 1,3 5E-05 | 0,000918 | Distal Intergenic | -59146 | ENSG00000112339 | HBS1L |
| chr1 | 236447244 | 236448662 | 4,53 | 0,62 | 3,91 | 1,36E-05 | 0,000921 | Promoter (1-2kb) | -1905 | ENSG00000086619 | ER01B |
| chr2 | 37851026 | 37852520 | 3,36 | -1,55 | 4,91 | 1,36E-05 | 0,000921 | Distal Intergenic | 46822 | ENSG00000163171 | CDC42EP3 |
| chr7 | 111720129 | 111721547 | 3,56 | -1,55 | 5,11 | 1,36E-05 | 0,000921 | Intron (uc003vfx.3/9732, intron 1 of 51) | 124915 | ENSG00000128512 | DOCK4 |
| chr14 | 81788060 | 81791002 | 5,38 | -0,4 | 5,79 | 1,38E-05 | 0,00093 | Intron (uc010tvu.3/85439, intron 4 of 5) | -42898 | ENSG00000140022 | STON2 |
| chr12 | 127410372 | 127412306 | 4,33 | -1,01 | 5,34 | 1,39E-05 | 0,000934 | Intron (uc001uho.3/uc001uho.3, intron 4 of 6) | -51136 | ENSG00000249873 | LINC02372 |
| chr4 | 81951667 | 81952869 | 3,56 | -1,55 | 5,11 | 1,40E-05 | 0,000937 | Promoter (<=1kb) | 0 | ENSG00000152785 | BMP3 |
| chr16 | 68009449 | 68011483 | 3,52 | -1,55 | 5,07 | 1,46E-05 | 0,000971 | Promoter (2-3kb) | 2969 | ENSG00000141096 | DPEP3 |
| chr12 | 93987396 | 93989649 | 3,95 | -1,55 | 5,51 | 1,49E-05 | 0,000988 | Distal Intergenic | 21094 | ENSG00000120833 | SOCS2 |
| chr19 | 45901433 | 45902448 | 4,15 | -1,55 | 5,7 | 1,55E-05 | 0,00102 | 5'UTR | -5008 | ENSG00000104881 | PPP1R13L |
| chr7 | 50662586 | 50663646 | 3,25 | -1,55 | 4,8 | 1,56E-05 | 0,00102 | Exon (uc003tph.3/2887, exon 15 of 16) | -29432 | ENSG00000132437 | DDC |
| chr17 | 38494888 | 38496718 | 3,91 | -1,55 | 5,46 | 1,56E-05 | 0,00102 | Promoter (<=1kb) | -922 | ENSG00000131759 | RARA |
| chrX | 12988672 | 12990827 | 4,19 | -1,55 | 5,74 | 1,57E-05 | 0,00103 | Promoter (2-3kb) | -2399 | ENSG00000205542 | TMSB4X |
| chr14 | 40868234 | 40870189 | 4,11 | -1,55 | 5,66 | 1,58E-05 | 0,00103 | Distal Intergenic | -966530 | ENSG00000165355 | FBXO3 3 |
| chr3 | 165464384 | 165467844 | 4,37 | -1,55 | 5,92 | 1,58E-05 | 0,00103 | Distal Intergenic | 87409 | ENSG00000114200 | BCHE |
| chr2 | 88124500 | 88125785 | 4,2 | -1,55 | 5,75 | 1,59E-05 | 0,00104 | Promoter (<=1kb) | 0 | ENSG00000187627 | RGPD1 |
| chr13 | 50944515 | 50945454 | 3,43 | -1,55 | 4,98 | 1,60E-05 | 0,00104 | Intron (uc010adt.1/10301. intron 1 of 1) | 198361 | NA | ST13P4 |
| chr2 | 106746857 | 106748387 | 3,94 | -1,55 | 5,49 | 1,63E-05 | 0,00105 | Intron (uc002tdl.3/80146, intron 1 of 9) | 6950 | ENSG00000115652 | UXS1 |
| chr4 | 77687099 | 77687849 | 3,16 | -1,55 | 4,71 | 1,63E-05 | 0,00106 | Intron (uc011cbx.2/57619, intron 9 of 10) | 27227 | ENSG00000138771 | SHROOM3 |
| chr4 | 46125176 | 46127394 | 6,01 | 1,11 | 4,9 | 1,65E-05 | 0,00106 | Promoter (<=1kb) | 0 | ENSG00000163285 | GABRG1 |
| chr15 | 72034723 | 72036394 | 3,82 | -1,55 | 5,37 | 1,66E-05 | 0,00106 | Intron (uc002atb.1/79875, intron 10 of 16) | -13778 | ENSG00000187720 | THSD4 |
| chr4 | 31395138 | 31396673 | 4,2 | -1,55 | 5,76 | 1,66E-05 | 0,00107 | Distal Intergenic | 673101 | ENSG00000169851 | PCDH7 |
| chr10 | 135500283 | 135501434 | 3,87 | -1,55 | 5,42 | 1,70E-05 | 0,00108 | Distal Intergenic | 6497 | NA | DUX4L3 |
| chr5 | 21370107 | 21371319 | 3,55 | -1,55 | 5,1 | 1,71E-0 5 | 0,00109 | Intron (uc011cnn.1/728411, intron 1 of 8) | 28165 | ENSG00000183666 | GUSBP1 |
| chr6 | 46261204 | 46262245 | 3,58 | -1,55 | 5,13 | 1,72E-05 | 0,00109 | Intron (uc003ovb.2/10231, intron 1 of 3) | 31384 | ENSG00000172348 | RCAN2 |
| chr17 | 68308985 | 68311282 | 4,05 | -1,55 | 5,61 | 1,72E-05 | 0,00109 | Distal Intergenic | 143309 | ENSG00000123700 | KCNJ2 |
| chr18 | 33183410 | 33186307 | 4,18 | -1,55 | 5,73 | 1,75E-05 | 0,0011 | Intron (uc002kyz.4/2589,intron 1 of 12) | 11709 | ENSG00000283514 | MIR3975 |
| chr8 | 42149623 | 42150718 | 3,67 | -1,55 | 5,23 | 1,77E-05 | 0,00111 | Intron (uc003xov.3/3551, intron 4 of 10) | 20803 | ENSG00000104365 | IKBKB |
| chr3 | 5469789 | 5470839 | 3,32 | -1,55 | 4,88 | 1,82E-05 | 0,00113 | Distal Intergenic | -177849 | ENSG00000265180 | MIR4790 |
| chr8 | 144809130 | 144810994 | 4,2 | -1,55 | 5,75 | 1,86E-05 | 0,00115 | Promoter (2-3kb) | 2076 | ENSG00000180921 | FAM83H |
| chr1 | 51750793 | 51752094 | 3,54 | -1,55 | 5,09 | 1,87E-05 | 0,00115 | Downstream (<lkb) | 11500 | ENSG00000085831 | TTC39A |
| chr2 | 26016306 | 26018176 | 3,72 | -1,55 | 5,27 | 1,89E-05 | 0,00116 | Intron (uc002rgs.2/55252, intron 4 of 11) | -13128 | ENSG00000143970 | ASXL2 |
| chr1 | 11358693 | 11359916 | 4,07 | -1,4 | 5,47 | 1,89E-05 | 0,00116 | Distal Intergenic | 25438 | ENSG00000120942 | UBIAD1 |
| chr12 | 15240582 | 15241941 | 3,76 | -1,29 | 5,05 | 1,92E-05 | 0,00117 | Distal Intergenic | 114626 | ENSG00000139053 | PDE6H |
| chr5 | 17425261 | 17426996 | 3,97 | -0,55 | 4,52 | 1,93E-05 | 0,00117 | Distal Intergenic | -37842 | ENSG00000250822 | LINC02111 |
| chr18 | 59001044 | 59002214 | 3,47 | -1,55 | 5,03 | 1,93E-05 | 0,00117 | Promoter (<=1kb) | 56 | ENSG00000101542 | CDH20 |
| chr4 | 140991699 | 140992966 | 3,54 | -1,55 | 5,09 | 1,95E-05 | 0,00118 | Intron (uc011chd.1/55534, intron 1 of 3) | 82267 | ENSG00000196782 | MAML3 |
| chr9 | 140651621 | 140652973 | 3,12 | -1,55 | 4,68 | 1,96E-05 | 0,00118 | Exon (uc004coa.3/79813, exon 9 of 16) | -4501 | NA | NA |
| chr5 | 96431513 | 96432648 | 3,2 | -1,55 | 4,75 | 1,96E-05 | 0,00118 | Exon (uc003kmy.4/167410, exon 5 of 6) | 45872 | ENSG00000145721 | LIX1 |
| chr10 | 11649830 | 11650548 | 2,45 | -1,55 | 4 | 1,98E-05 | 0,00119 | Intron (uc001ikt.3/9712, intron 1 of 14) | 3131 | ENSG00000148429 | USP6NL |
| chr4 | 116936370 | 116938221 | 4,47 | -0,79 | 5,26 | 1,98E-05 | 0,00119 | Distal Intergenic | -282660 | ENSG00000284253 | MIR1973 |
| chrl6 | 28699269 | 28701182 | 2,83 | -1,55 | 4,38 | 1,99E-05 | 0,00119 | Promoter (<=1kb) | 0 | ENSG00000 184110 | EIF3 C |
| chr4 | 136042263 | 136045166 | 4,98 | -0,8 | 5,78 | 1,99E-05 | 0,00119 | Distal Intergenic | -919360 | ENSG00000254535 | PABPC4L |
| chr1 | 165614525 | 165615940 | 3,67 | -1,55 | 5,22 | 2,00E-05 | 0,0012 | Intron (uc001pdf.3/4259,intron 1 of 5) | 14415 | ENSG00000143198 | MGST3 |
| chr8 | 67037355 | 67045657 | 8,1 | 1,08 | 7,01 | 2,00E-05 | 0,0012 | Promoter (<=1kb) | 0 | ENSG00000147573 | TRIM55 |
| chr3 | 173459510 | 173461323 | 3,73 | -1,55 | 5,29 | 2,01E-05 | 0,0012 | Intron (uc003fio.1/22871, intron 3 of 6) | 137441 | ENSG00000169760 | NLGN1 |
| chr8 | 49208158 | 49209432 | 3,55 | -0,3 | 3,85 | 2,04E-05 | 0,00121 | Distal Intergenic | 287163 | ENSG00000169139 | UBE2 V2 |
| chr20 | 42338349 | 42341403 | 4,11 | -1,55 | 5,66 | 2,04E-05 | 0,00121 | Exon (uc002xlb.1/4605, exon 10 of 14) | 14239 | ENSG00000 124196 | GTSF1L |
| chr2 | 155628082 | 155629450 | 3,62 | -1,55 | 5,17 | 2,05E-05 | 0,00121 | Intron (uc002tyv.2/3760, intron 2 of 2) | 72989 | ENSG00000162989 | KCNJ3 |
| chr10 | 32606325 | 32607655 | 3,46 | -1,1 | 4,56 | 2,06E-05 | 0,00122 | Intron (uc001iwg.2/80314, intron 1 of 14) | 28491 | ENSG00000120616 | EPC1 |
| chr5 | 118014711 | 118015560 | 3,58 | -0,62 | 4,2 | 2,11E-05 | 0,00124 | Distal Intergenic | -294721 | ENSG00000283498 | MIR1244-2 |
| chr3 | 4940017 | 4941114 | 2,72 | -1,55 | 4,27 | 2,12E-05 | 0,00124 | 3'UTR | 7072 | ENSG00000235831 | BHLHE40-AS1 |
| chr3 | 192548391 | 192549890 | 4,01 | -1,55 | 5,56 | 2,13E-05 | 0,00125 | Intron (uc01 1bsp.2/151963, intron 1 of 1) | 86060 | ENSG00000 180611 | MB21D2 |
| chr4 | 168269844 | 168271060 | 4,05 | -1,1 | 5,15 | 2,16E-05 | 0,00126 | Distal Intergenic | -114103 | ENSG00000196104 | SPOCK3 |
| chr1 | 85966285 | 85968186 | 3,74 | -1,55 | 5,3 | 2,16E-05 | 0,00126 | Promoter (<=1kb) | -371 | ENSG00000074266 | EED |
| chr9 | 90078677 | 90079979 | 3,37 | -1,55 | 4,93 | 2,19E-05 | 0,00127 | Distal Intergenic | -32679 | ENSG00000196730 | DAPK1 |
| chr1 | 313160 | 316948 | 7,73 | 4,19 | 3,54 | 2,20E-05 | 0,00127 | Promoter (<=1kb) | 0 | ENSG00000185885 | IFITM1 |
| chr20 | 5111100 | 5112483 | 3,75 | -1,55 | 5,3 | 2,20E-05 | 0,00127 | Intron (uc002wlr.2/8760, intron 2 of 10) | 3693 | ENSG00000101290 | CDS2 |
| chr7 | 39460037 | 39461822 | 3,88 | -1,55 | 5,43 | 2,23E-05 | 0,00129 | Intron (uc003thb.2/11281, intron 7 of 10) | -14092 | ENSG00000224122 | POU6F2-AS1 |
| chr7 | 27169095 | 27171490 | 7,76 | 2,13 | 5,62 | 2,24E-05 | 0,00129 | Promoter (<=1kb) | 0 | ENSG00000197576 | HOXA4 |
| chr8 | 39818418 | 39819565 | 4,01 | -0,75 | 4,77 | 2,26E-05 | 0,00129 | Intron (uc003xno.1/169355, intron 2 of 10) | -16506 | ENSG00000188676 | IDO2 |
| chr7 | 3228015 | 3229239 | 4,07 | -0,63 | 4,71 | 2,28E-05 | 0,0013 | Distal Intergenic | -111841 | ENSG00000146555 | SDK1 |
| chr1 | 110532339 | 110534256 | 4,34 | -1,11 | 5,45 | 2,28E-05 | 0,0013 | Intron (uc001dyx.3/10768, intron 1 of 16) | 4952 | ENSG00000168710 | AHCYL1 |
| chr3 | 190510808 | 190512281 | 3,32 | -1,55 | 4,87 | 2,30E-05 | 0,00131 | Distal Intergenic | 68184 | ENSG00000205835 | GMNC |
| chr7 | 111827020 | 111828575 | 4,01 | -1,55 | 5,56 | 2,32E-05 | 0,00132 | Intron (uc003vfx.3/9732, intron 1 of 51) | 17887 | ENSG00000128512 | DOCK4 |
| chr4 | 71779761 | 71781562 | 4,16 | -1,55 | 5,71 | 2,36E-05 | 0,00133 | Intron (uc003hfv.2/92597, intron 1 of 3) | 11704 | ENSG00000173542 | MOB1B |
| chr4 | 187760138 | 187761755 | 4,69 | -0,3 | 4,99 | 2,36E-05 | 0,00134 | Distal Intergenic | -112288 | ENSG00000083857 | FAT1 |
| chr1 | 33782648 | 33783702 | 3,51 | -1,55 | 5,06 | 2,37E-05 | 0,00134 | Intron (uc001muv.3/26273, intron 2 of 9) | -7703 | ENSG00000110429 | FBXO3 |
| chr3 | 114172269 | 114173898 | 4,64 | -0,3 | 4,93 | 2,38E-05 | 0,00134 | Intron (uc003ebj.3/26137, intron 2 of 5) | -69780 | ENSG00000 181722 | ZBTB20 |
| chr18 | 13576640 | 13578373 | 5,23 | 0,68 | 4,55 | 2,39E-05 | 0,00134 | Intron (uc002ksb.3/753, intron 4 of 5) | -32740 | ENSG00000263527 | MIR4526 |
| chr5 | 124871757 | 124872572 | 3,14 | -0,98 | 4,12 | 2,40E-05 | 0,00134 | Distal Intergenic | -787257 | ENSG00000168916 | ZNF608 |
| chr2 | 65208906 | 65210220 | 3,78 | -1,55 | 5,33 | 2,42E-05 | 0,00135 | Distal Intergenic | -5359 | ENSG00000115902 | SLC1A4 |
| chr5 | 34587099 | 34588655 | 5,04 | 0,35 | 4,69 | 2,50E-05 | 0,00139 | Distal Intergenic | -67778 | ENSG00000039560 | RAI14 |
| chr1 | 126085619 | 126087308 | 4,21 | -1,02 | 5,23 | 2,50E-05 | 0,00139 | Intron (uc001qdf.3/79607, intron 1 of 8) | 4000 | ENSG00000 197798 | FAM118B |
| chr7 | 17273471 | 17274890 | 5 | 0,59 | 4,41 | 2,52E-05 | 0,0014 | Distal Intergenic | -63386 | ENSG00000106546 | AHR |
| chr18 | 72394051 | 72395301 | 3,73 | -1,55 | 5,28 | 2,53E-05 | 0,0014 | Intron(uc010xfc.2/55628, intron 2 of 3) | 51128 | ENSG00000215421 | ZNF407 |
| chr18 | 12843392 | 12845407 | 3,76 | -1,55 | 5,32 | 2,56E-05 | 0,00141 | Intron (uc002krl.3/5771, intron 2 of 9) | 38927 | ENSG00000175354 | PTPN2 |
| chr3 | 166218354 | 166219526 | 2,72 | -1,55 | 4,28 | 2,58E-05 | 0,00142 | Distal Intergenic | -663101 | ENSG00000114200 | BCHE |
| chr4 | 113039862 | 113040984 | 3,61 | -1,23 | 4,84 | 2,59E-05 | 0,00142 | Distal Intergenic | -25569 | ENSG00000174749 | FAM241A |
| chr2 | 230687215 | 230688847 | 3,64 | -1,01 | 4,66 | 2,60E-05 | 0,00143 | Intron (uc021vxw.1/9320, intron 6 of 39) | 55997 | ENSG00000153827 | TRIP12 |
| chr18 | 34579311 | 34580464 | 3,23 | -1,55 | 4,78 | 2,63E-05 | 0,00144 | Intron (uc002kzz.3/57536, intron 6 of 9) | 40040 | ENSG00000 150477 | KIAA1328 |
| chrX | 5221778 | 5224708 | 5,76 | 0,16 | 5,6 | 2,65E-05 | 0,00144 | Distal Intergenic | 845104 | ENSG00000146938 | NLGN4X |
| chr5 | 116540437 | 116542089 | 3,66 | -1,55 | 5,21 | 2,66E-05 | 0,00145 | Distal Intergenic | -209119 | NA | LINC00992 |
| chr17 | 47747238 | 47749492 | 3,68 | -1,55 | 5,24 | 2,72E-05 | 0,00147 | Intron (uc002ipb.3/8405,intron 2 of 11) | 6033 | ENSG00000121067 | SPOP |
| chr15 | 55127866 | 55129399 | 3,25 | -1,55 | 4,81 | 2,74E-05 | 0,00148 | Distal Intergenic | 226326 | ENSG00000137766 | UNC13C |
| chr9 | 130590319 | 130591241 | 3,92 | -1,55 | 5,47 | 2,78E-05 | 0,0015 | Intron (uc01 1mam.3/2022, intron 3 of 14) | -10983 | ENSG00000106991 | ENG |
| chr9 | 113757823 | 113758846 | 2,89 | -1,38 | 4,27 | 2,85E-05 | 0,00152 | Promoter (2-3kb) | 2900 | ENSG00000198121 | LPAR1 |
| chr18 | 52384520 | 52386920 | 5,15 | 0,57 | 4,59 | 2,88E-05 | 0,00153 | Distal Intergenic | -108788 | ENSG00000041353 | RAB27B |
| chr2 | 238336157 | 238337988 | 3,83 | -1,23 | 5,06 | 2,91E-05 | 0,00154 | Distal Intergenic | -13307 | ENSG00000163359 | COL6A3 |
| chr1 | 119561571 | 119563083 | 3,33 | -1,4 | 4,73 | 2,92E-05 | 0,00154 | Intron (uc001pwu.1/5818, intron 1 of 7) | 36352 | ENSG00000110400 | NECTIN1 |
| chr4 | 96649153 | 96650126 | 3,25 | -1,55 | 4,8 | 2,96E-05 | 0,00156 | Distal Intergenic | -111113 | ENSG00000163114 | PDHA2 |
| chr3 | 71478016 | 71479349 | 3,4 | -1,55 | 4,96 | 2,98E-05 | 0,00157 | Intron (uc003don.3/27086, intron 1 of 21) | 63357 | ENSG00000114861 | FOXP1 |
| chr9 | 82277511 | 82279327 | 4 | -1,55 | 5,56 | 2,98E-05 | 0,00157 | Intron (uc004alc.3/7091, intron 8 of 19) | 87723 | ENSG00000106829 | TLE4 |
| chr4 | 26885786 | 26888100 | 3,94 | -1,55 | 5,49 | 3,04E-05 | 0,00159 | Intron (uc003gsg.4/57620, intron 1 of 11) | 23473 | ENSG00000109689 | STIM2 |
| chr20 | 48509739 | 48511006 | 3,47 | -1,55 | 5,02 | 3,05E-05 | 0,00159 | Distal Intergenic | 19270 | ENSG00000158480 | SPATA2 |
| chr20 | 48150981 | 48152633 | 3,75 | -1,55 | 5,3 | 3,05E-05 | 0,00159 | Intron (uc002xut.3/5740, intron 5 of 9) | 32074 | ENSG00000124212 | PTGIS |
| chr12 | 81879868 | 81881703 | 3,79 | -1,55 | 5,34 | 3,05E-05 | 0,00159 | Intron (uc010sug.2/8499, intron 1 of 29) | 24467 | ENSG00000139220 | PPFIA2 |
| chr6 | 128813916 | 128816155 | 3,67 | -1,55 | 5,22 | 3,06E-05 | 0,00159 | Intron (uc003qbj.3/5796, intron 1 of 29) | 25715 | ENSG00000152894 | PTPRK |
| chr9 | 113759942 | 113761952 | 3,95 | -1,55 | 5,51 | 3,11E-05 | 0,00161 | Promoter (<=1kb) | 0 | ENSG00000198121 | LPAR1 |
| chr6 | 84002441 | 84003691 | 3,51 | -0,71 | 4,22 | 3,13E-05 | 0,00162 | Intron (uc003pjy.3/4199, intron 6 of 13) | -98786 | ENSG00000013375 | PGM3 |
| chr2 | 192868999 | 192870500 | 3,63 | -1,55 | 5,18 | 3,19E-05 | 0,00164 | Intron (uc031rqm.1/23671, intron 5 of 9) | -156993 | ENSG00000168497 | CAVIN2 |
| chr4 | 37246000 | 37247383 | 3,51 | -1,55 | 5,06 | 3,22E-05 | 0,00164 | Promoter (<=1kb) | 0 | ENSG00000174145 | NWD2 |
| chr5 | 26622686 | 26624116 | 4,46 | -0,18 | 4,64 | 3,27E-05 | 0,00166 | Distal Intergenic | 266826 | ENSG00000113100 | CDH9 |
| chr21 | 22340479 | 22342864 | 4,3 | -0,87 | 5,17 | 3,29E-05 | 0,00167 | Distal Intergenic | -27769 | ENSG00000154654 | NCAM2 |
| chr5 | 54357319 | 54358509 | 3,81 | -0,87 | 4,68 | 3,30E-05 | 0,00167 | Distal Intergenic | 37212 | ENSG00000113088 | GZMK |
| chr10 | 23983052 | 23985030 | 4,36 | -0,73 | 5,09 | 3,32E-05 | 0,00168 | Promoter (<=1kb) | 0 | ENSG00000120549 | KIAA1217 |
| chr4 | 26084937 | 26086380 | 3,51 | -1,55 | 5,06 | 3,33E-05 | 0,00168 | Distal Intergenic | 169123 | ENSG00000250317 | SMIM20 |
| chr6 | 4935455 | 4936973 | 3,68 | -1,55 | 5,23 | 3,37E-05 | 0,00169 | 5'UTR | 45229 | ENSG00000153046 | CDYL |
| chr9 | 127053731 | 127055529 | 3,48 | -1,55 | 5,04 | 3,38E-05 | 0,00169 | Promoter (<=1kb) | 0 | ENSG00000 119408 | NEK6 |
| chr2 | 181970785 | 181971671 | 3,04 | -1,55 | 4,59 | 3,39E-05 | 0,00169 | Distal Intergenic | 124934 | ENSG00000170035 | UBE2E3 |
| chr4 | 113810550 | 113811523 | 3,28 | -1,4 | 4,68 | 3,42E-05 | 0,0017 | Intron (uc003ibc.2/287, intron 1 of 30) | 71311 | ENSG00000145362 | ANK2 |
| chr3 | 81518794 | 81520643 | 4,2 | -0,72 | 4,92 | 3,47E-05 | 0,00172 | Distal Intergenic | 272137 | ENSG00000114480 | GBE1 |
| chr6 | 15299046 | 15299840 | 2,51 | -1,55 | 4,07 | 3,51E-05 | 0,00173 | Intron (uc011diu.1/3720,intron 1 of 5) | 49960 | ENSG00000008083 | JARID2 |
| chr5 | 24312182 | 24313581 | 3,81 | -1,55 | 5,36 | 3,52E-05 | 0,00173 | Distal Intergenic | 331504 | ENSG00000040731 | CDH10 |
| chr18 | 23394844 | 23395268 | 2,39 | -1,55 | 3,94 | 3,55E-05 | 0,00174 | Distal Intergenic | 275343 | ENSG00000141380 | SS18 |
| chr11 | 118280535 | 118282326 | 3,85 | -1,55 | 5,41 | 3,58E-05 | 0,00175 | 3'UTR | 7204 | NA | LOC100131626 |
| chr8 | 134645833 | 134647578 | 3,76 | -1,55 | 5,31 | 3,61E-05 | 0,00176 | Distal Intergenic | -61650 | ENSG00000008513 | ST3GAL1 |
| chr18 | 30005338 | 30006761 | 3,9 | -1,55 | 5,45 | 3,71E-0 5 | 0,0018 | Intron (uc002kxk.2/64762, intron 1 of 5) | 43686 | ENSG00000141441 | GAREM1 |
| chr2 | 231623243 | 231624518 | 3,54 | -1,55 | 5,1 | 3,72E-05 | 0,0018 | Intron (uc002vqx.3/51719, intron 1 of 8) | 44980 | ENSG00000135932 | CAB39 |
| chr15 | 84596421 | 84598363 | 4,27 | -0,56 | 4,83 | 3,76E-05 | 0,00181 | Intron (uc010bmt.1/57188, intron 17 of 26) | -150576 | NA | EFL1P1 |
| chr7 | 12422558 | 12423689 | 3,88 | -0,75 | 4,63 | 3,78E-05 | 0,00181 | 5'UTR | 20163 | ENSG00000146530 | VWDE |
| chr21 | 25662443 | 25663418 | 3,24 | -1,17 | 4,41 | 3,84E-05 | 0,00184 | Distal Intergenic | -549446 | ENSG00000226983 | LINC01692 |
| chr7 | 112640342 | 112641129 | 3 | -1,55 | 4,56 | 3,87E-05 | 0,00185 | Distal Intergenic | -60410 | ENSG00000164603 | BMT2 |
| chr3 | 27391098 | 27391964 | 3,4 | -0,84 | 4,23 | 3,89E-05 | 0,00186 | Intron (uc003cdt.2/152110, intron 4 of 24) | 18723 | ENSG00000163491 | NEK10 |
| chr7 | 83810657 | 83812030 | 3,4 | -1,55 | 4,95 | 3,92E-05 | 0,00187 | Intron (uc003uhz.3/10371, intron 1 of 16) | 12187 | ENSG00000075213 | SEMA3 A |
| chr4 | 166924322 | 166926739 | 3,92 | -1,55 | 5,48 | 3,93E-05 | 0,00187 | Exon (uc021xud.1/7092, exon 6 of 10) | 129912 | ENSG00000038295 | TLL1 |
| chr6 | 152070551 | 152072226 | 3,71 | -1,55 | 5,26 | 4,03E-05 | 0,0019 | Intron (uc031spz.1/2099, intron 3 of 3) | -54582 | ENSG00000091831 | ESR1 |
| chr6 | 109441270 | 109442637 | 3,87 | -1,38 | 5,25 | 4,04E-05 | 0,0019 | Intron (uc003psw.4/285753, intron 1 of 6) | -7870 | ENSG00000183137 | CEP57L1 |
| chr9 | 99003299 | 99004177 | 2,64 | -1,55 | 4,19 | 4,08E-05 | 0,00192 | Intron (uc004awa.1/3293, intron 9 of 10) | 60257 | ENSG00000130948 | HSD17B3 |
| chr2 | 165007761 | 165009423 | 3,88 | -1,55 | 5,43 | 4,10E-05 | 0,00192 | Distal Intergenic | -415248 | ENSG00000 182263 | FIGN |
| chr6 | 16727931 | 16728734 | 3,05 | -1,55 | 4,6 | 4,12E-05 | 0,00193 | Intron (uc003nbt.3/6310, intron 3 of 8) | 21590 | ENSG00000124788 | ATXN1 |
| chr11 | 113947278 | 113948879 | 4,66 | -0,17 | 4,83 | 4,13E-05 | 0,00193 | Intron (uc001poo.1/7704, intron 2 of 3) | 15990 | ENSG00000109906 | ZBTB16 |
| chr2 | 68440700 | 68442743 | 4,92 | 0,47 | 4,44 | 4,18E-05 | 0,00195 | Intron (uc002sei.1/5534, intron 2 of 5) | 36908 | ENSG00000221823 | PPP3R1 |
| chr18 | 33172270 | 33173261 | 3,41 | -1,55 | 4,96 | 4,22E-05 | 0,00196 | Promoter (<=1kb) | 569 | ENSG00000283514 | MIR3975 |
| chr13 | 110128704 | 110129369 | 2,74 | -1,55 | 4,29 | 4,29E-05 | 0,00198 | Distal Intergenic | -309053 | ENSG00000236242 | MYO16-AS1 |
| chr13 | 32306591 | 32307627 | 4,04 | -0,26 | 4,3 | 4,29E-05 | 0,00198 | Distal Intergenic | -6052 | ENSG00000133105 | RXFP2 |
| chr4 | 79217968 | 79218746 | 3,42 | -0,71 | 4,13 | 4,31E-05 | 0,00199 | Intron (uc003hkw.3/80144, intron 14 of 41) | 10422 | ENSG00000138759 | FRAS1 |
| chr10 | 78646831 | 78648673 | 4,02 | -1,06 | 5,08 | 4,35E-05 | 0,002 | 3'UTR | 221428 | ENSG00000156113 | KCNMA1 |
| chr8 | 138128822 | 138130231 | 3,34 | -1,55 | 4,89 | 4,36E-05 | 0,002 | Distal Intergenic | 1035228 | ENSG00000147724 | FAM135B |
| chr21 | 25833507 | 25835744 | 3,38 | -1,55 | 4,93 | 4,42E-05 | 0,00202 | Intron (uc002yli.1/uc002yli.1, intron 4 of 5) | -377120 | ENSG00000226983 | LINC01692 |
| chr6 | 155094010 | 155095483 | 3,68 | -1,55 | 5,23 | 4,46E-05 | 0,00204 | Exon (uc003qpz.3/22828, exon 2 of 20) | 39029 | ENSG00000213079 | SCAF8 |
| chr12 | 46949419 | 46950312 | 3,07 | -1,55 | 4,62 | 4,49E-05 | 0,00205 | Distal Intergenic | -182774 | ENSG00000134294 | SLC38A2 |
| chr1 | 153814861 | 153815825 | 3,24 | -0,86 | 4,1 | 4,52E-05 | 0,00206 | Intron (uc001fdb.4/57459, intron 1 of 10) | 67093 | ENSG00000143554 | SLC27A3 |
| chr4 | 94905776 | 94906904 | 2,97 | -1,55 | 4,53 | 4,53E-05 | 0,00206 | Distal Intergenic | 155698 | ENSG00000172238 | ATOH1 |
| chr4 | 147575762 | 147576800 | 4,73 | -0,02 | 4,75 | 4,55E-05 | 0,00206 | Distal Intergenic | 15717 | ENSG00000151615 | POU4F2 |
| chr1 | 224803270 | 224806760 | 6,82 | 0,72 | 6,1 | 4,56E-05 | 0,00206 | Promoter (<=1kb) | 0 | ENSG00000143786 | CNIH3 |
| chr6 | 45454732 | 45456248 | 3,25 | -0,77 | 4,02 | 4,57E-05 | 0,00206 | Intron (uc01 1dvx.2/860, intron 5 of 8) | 64818 | ENSG00000124813 | RUNX2 |
| chr4 | 183416328 | 183418017 | 3,48 | -1,55 | 5,03 | 4,57E-05 | 0,00206 | Intron (uc021xux.1/55714, intron 1 of 2) | 171229 | ENSG00000218336 | TENM3 |
| chr14 | 93047313 | 93048605 | 3,21 | -1,55 | 4,76 | 4,60E-05 | 0,00207 | Intron (uc001vap.3/79890, intron 3 of 9) | 4734 | ENSG00000100599 | RIN3 |
| chr6 | 149352289 | 149353947 | 3,91 | -0,97 | 4,88 | 4,62E-05 | 0,00208 | Intron (uc003qmg.3/10090, intron 7 of 7) | -66469 | NA | UST-AS1 |
| chr4 | 153382763 | 153384012 | 3,23 | -1,55 | 4,79 | 4,67E-05 | 0,00209 | Intron (uc003ims.3/55294, intron 1 of 11) | 26556 | ENSG00000264678 | MIR3140 |
| chr18 | 77797197 | 77799270 | 3,8 | -1,55 | 5,35 | 4,67E-05 | 0,00209 | Promoter (2-3kb) | 2851 | ENSG00000101546 | RBFA |
| chr6 | 52515572 | 52517057 | 3,43 | -1,55 | 4,98 | 4,69E-05 | 0,00209 | Distal Intergenic | -12142 | ENSG00000216775 | LOC730101 |
| chr17 | 55739873 | 55740986 | 3,66 | -1,55 | 5,21 | 4,69E-05 | 0,00209 | Intron (uc010wnm.1/124540, intron 11 of 13) | 186447 | ENSG00000181610 | MRPS23 |
| chr1 1 | 77146734 | 77147600 | 2,92 | -1,55 | 4,47 | 4 ,72E-O 5 | 0,0021 | Intron (uc001ovg.4/5058, intron 1 of 15) | -23737 | ENSG00000149269 | PAK1 |
| chr6 | 72233169 | 72234815 | 3,86 | -0,49 | 4,35 | 4,75E-05 | 0,0021 | Distal Intergenic | -102721 | ENSG00000233237 | LINC00472 |
| chr1 | 145715076 | 145715947 | 3,2 | -1,55 | 4,75 | 4,75E-05 | 0,0021 | Promoter (<=1kb) | 0 | ENSG00000117281 | CD160 |
| chr8 | 117198177 | 117199539 | 3,87 | -1,01 | 4,88 | 4,75E-05 | 0,0021 | Intron (uc031tcc.1/100859921, intron 4 of 13) | 137758 | ENSG00000249917 | LINC00536 |
| chr17 | 17391465 | 17393266 | 3,5 | -1,55 | 5,05 | 4,76E-05 | 0,0021 | Intron (uc002grh.1/55090, intron 1 of 1) | 6443 | ENSG00000108551 | RASD1 |
| chr2 | 237039491 | 237040786 | 3,3 | -1,55 | 4,85 | 4,78E-05 | 0,00211 | 3'UTR | 35866 | ENSG00000168505 | GBX2 |
| chr4 | 31024175 | 31026086 | 3,79 | -1,55 | 5,34 | 4,79E-05 | 0,00211 | Intron (uc011bxx.2/5099, intron 2 of 2) | 302138 | ENSG00000169851 | PCDH7 |
| chr14 | 90107686 | 90108559 | 4,08 | 0,54 | 3,54 | 4,80E-05 | 0,00211 | Distal Intergenic | -22192 | ENSG00000053254 | FOXN3 |
| chr9 | 13443776 | 13445262 | 3,46 | -1,29 | 4,75 | 4,82E-05 | 0,00212 | Distal Intergenic | -12448 | ENSG00000270547 | LINC01235 |
| chr11 | 125859958 | 125861225 | 3,21 | -1,11 | 4,32 | 4,84E-05 | 0,00212 | Intron (uc001qdb.4/50937, intron 5 of 10) | 13115 | ENSG00000064309 | CDON |
| chr3 | 4547792 | 4550590 | 5,44 | 1,03 | 4,41 | 4,85E-05 | 0,00213 | Intron (uc010hbz.3/3708, intron 2 of 9) | 12760 | ENSG00000150995 | ITPR1 |
| chr7 | 47993643 | 47994878 | 3,78 | -1,55 | 5,33 | 4,86E-05 | 0,00213 | Distal Intergenic | -5572 | ENSG00000158683 | PKD1L1 |
| chr1 | 221881109 | 221882283 | 3,23 | -1,55 | 4,78 | 4,87E-05 | 0,00213 | Intron (uc001hmx.2/11221, intron 1 of 2) | 28519 | ENSG00000143507 | DUSP10 |
| chr2 | 44154387 | 44155714 | 3,63 | -1,55 | 5,19 | 4,88E-05 | 0,00213 | Intron (uc002rtr.2/10128, intron 25 of 37) | 67401 | ENSG00000138095 | LRPPRC |
| chr1 | 61428360 | 61429691 | 3,39 | -1,55 | 4,94 | 4,89E-05 | 0,00213 | Exon (uc001czu.3/uc001czu.3, exon 2 of 7) | -113255 | ENSG00000162599 | NFIA |
| chr4 | 148890525 | 148891866 | 3,68 | -0,88 | 4,55 | 4,94E-05 | 0,00215 | Intron (uc003ilf.3/79658, intron 18 of 22) | 23135 | ENSG00000071205 | ARHGAP10 |
| chr8 | 104753373 | 104754505 | 3,3 | -1,55 | 4,85 | 5,00E-05 | 0,00217 | Intron (uc003vlp.3/9699, intron 2 of 23) | -76911 | ENSG00000176406 | RIMS2 |
| chr3 | 190250350 | 190252756 | 3,45 | -1,55 | 5 | 5,01E-05 | 0,00217 | Intron (uc003fsk.3/3556, intron 1 of 8) | 18510 | ENSG00000 196083 | IL1RAP |
| chr3 | 103391239 | 103392188 | 3,24 | -1,55 | 4,79 | 5,04E-05 | 0,00218 | Distal Intergenic | 553917 | ENSG00000208032 | MIR548A3 |
| chr1 | 31971783 | 31972959 | 4,08 | -1,1 | 5,18 | 5,04E-05 | 0,00218 | Promoter (<=1kb) | 0 | ENSG00000260386 | LINC01225 |
| chr8 | 134694265 | 134696963 | 3,72 | -1,55 | 5,28 | 5,04E-05 | 0,00218 | Distal Intergenic | -110082 | ENSG00000008513 | ST3GAL1 |
| chr4 | 97009652 | 97010906 | 3,43 | -1,55 | 4,99 | 5,09E-05 | 0,00219 | Distal Intergenic | 248413 | ENSG00000163114 | PDHA2 |
| chr1 | 246183124 | 246184107 | 3,04 | -1,55 | 4,6 | 5,11E-05 | 0,0022 | Intron (uc001ibk.3/64754, intron 5 of 11) | -88999 | ENSG00000185420 | SMYD3 |
| chr2 | 56447039 | 56448414 | 3,66 | -1,55 | 5,21 | 5,22E-05 | 0,00224 | Intron (uc021vhw.1/114800, intron 3 of 4) | 35781 | ENSG00000055813 | CCDC85A |
| chr17 | 44215715 | 44222723 | 6,87 | 2,44 | 4,43 | 5,24E-05 | 0,00224 | Intron (uc002ikc.3/284058, intron 2 of 14) | 47443 | ENSG00000120071 | KANSL1 |
| chr16 | 81998432 | 81999246 | 3,38 | -1,55 | 4,94 | 5,25E-05 | 0,00224 | Distal Intergenic | 45847 | ENSG00000184860 | SDR42E1 |
| chr2 | 85442339 | 85443231 | 2,95 | -1,55 | 4,5 | 5,36E-05 | 0,00228 | Intron (uc002sov.3/83439, intron 3 of 11) | 81756 | ENSG00000152284 | TCF7L1 |
| chr13 | 20138865 | 20139863 | 4,11 | -0,73 | 4,84 | 5,38E-05 | 0,00228 | Distal Intergenic | -3151 | ENSG00000132958 | TPTE2 |
| chr8 | 2856975 | 2858405 | 3,72 | -1,29 | 5 | 5,41E-05 | 0,00229 | Exon (uc010lrg.3/64478, exon 17 of 32) | 187082 | ENSG00000183117 | CSMD1 |
| chr18 | 76437723 | 76438610 | 2,86 | -1,55 | 4,42 | 5,42E-05 | 0,00229 | Distal Intergenic | -301665 | ENSG00000256463 | SALL3 |
| chr3 | 173806624 | 173809077 | 4,35 | -0,8 | 5,16 | 5,44E-05 | 0,0023 | Intron (uc003fio.1/22871, intron 4 of 6) | 484555 | ENSG00000169760 | NLGN1 |
| chr13 | 106862595 | 106863880 | 3,59 | -1,4 | 4,99 | 5,47E-05 | 0,0023 | Distal Intergenic | -165031 | NA | LINC00460 |
| chr6 | 138225583 | 138226992 | 3,42 | -1,55 | 4,97 | 5,51E-05 | 0,00232 | Distal Intergenic | 33233 | ENSG00000118503 | TNFAIP3 |
| chr2 | 3464934 | 3466239 | 4,64 | -0,39 | 5,02 | 5,54E-05 | 0,00233 | Intron (uc002qxm.1/51112, intron 8 of 11) | 55279 | ENSG00000182551 | ADI1 |
| chr2 | 101184954 | 101187700 | 4,92 | -0,16 | 5,08 | 5,54E-05 | 0,00233 | Exon (uc002tao.2/79031, exon 3 of 6) | 5536 | ENSG00000115539 | PDCL3 |
| chr11 | 110395971 | 110396735 | 2,89 | -1,55 | 4,45 | 5,55E-05 | 0,00233 | Distal Intergenic | 63674 | ENSG00000137727 | ARHGAP20 |
| chr17 | 53702081 | 53703063 | 3,12 | -1,55 | 4,67 | 5,57E-05 | 0,00233 | Distal Intergenic | 97162 | ENSG00000166292 | TMEM100 |
| chr13 | 43294034 | 43295285 | 3,5 | -1,55 | 5,05 | 5,59E-05 | 0,00234 | Distal Intergenic | -60401 | ENSG00000179813 | FAM216B |
| chr10 | 73923795 | 73925459 | 3,71 | -1,55 | 5,26 | 5,59E-05 | 0,00234 | Intron (uc021psn.1/119504, intron 1 of 3) | 37823 | ENSG00000138303 | ASCC1 |
| chr12 | 12627517 | 12629057 | 3,73 | -1,55 | 5,28 | 5,59E-05 | 0,00234 | 3'UTR | 45340 | ENSG00000111266 | DUSP16 |
| chr5 | 26790872 | 26791862 | 3,12 | -1,55 | 4,67 | 5,66E-05 | 0,00236 | Distal Intergenic | 99080 | ENSG00000113100 | CDH9 |
| chr21 | 19942381 | 19943065 | 3,14 | -1,55 | 4,69 | 5,74E-05 | 0,00238 | Exon (uc010glf.2/uc010glf.2. exon 2 of 4) | -166411 | ENSG00000154646 | TMPRSS15 |
| chr9 | 29779239 | 29780524 | 3,89 | -0,55 | 4,44 | 5,75E-05 | 0,00239 | Distal Intergenic | -566241 | ENSG00000174482 | LINGO2 |
| chrX | 133687657 | 133688988 | 4,41 | -0,72 | 5,13 | 5,78E-05 | 0,0024 | Intron (uc004exn.1/100506757, intron 1 of 2) | 3603 | NA | LINC00629 |
| chr21 | 27471089 | 27472199 | 3,34 | -1,55 | 4,89 | 5,81E-05 | 0,0024 | Intron (uc010glk.3/351, intron 2 of 16) | 40509 | ENSG00000142192 | APP |
| chr7 | 115467081 | 115468163 | 3,42 | -1,55 | 4,98 | 5,82E-05 | 0,00241 | Distal Intergenic | 140204 | ENSG00000105967 | TFEC |
| chr11 | 27465225 | 27466825 | 3,26 | -1,55 | 4,81 | 5,84E-05 | 0,00241 | Intron (uc001mrj.4/55366, intron 1 of 17) | 27509 | ENSG00000205213 | LGR4 |
| chr10 | 116299428 | 116302008 | 3,67 | -1,55 | 5,22 | 5,86E-05 | 0,00242 | Intron (uc021pvw.1/3983, intron 6 of 21) | -12743 | ENSG00000099204 | ABLIM1 |
| chr13 | 32454293 | 32455955 | 3,14 | -1,55 | 4,69 | 5,91E-05 | 0,00243 | Intron (uc001utu.3/196549, intron 1 of 3) | 33373 | NA | EEF1DP3 |
| chr12 | 4417117 | 4417679 | 3,03 | -1,55 | 4,58 | 5,93E-05 | 0,00244 | Distal Intergenic | -12680 | ENSG00000078237 | TIGAR |
| chr8 | 48078776 | 48079185 | 2,87 | -1,14 | 4,01 | 5,95E-05 | 0,00244 | Distal Intergenic | -21745 | NA | LOC100287846 |
| chr6 | 42369741 | 42370903 | 3,27 | -1,55 | 4,83 | 6,07E-05 | 0,00249 | Intron (uc003osb.2/55809, intron 1 of 16) | 48096 | ENSG00000124496 | TRERF1 |
| chr4 | 187747053 | 187749013 | 3,52 | -1,55 | 5,07 | 6,09E-05 | 0,00249 | Distal Intergenic | -99203 | ENSG00000083857 | FAT1 |
| chr2 | 232878235 | 232879225 | 3,01 | -1,55 | 4,56 | 6,11E-05 | 0,0025 | Intron (uc002vsm.4/129563, intron 1 of 20) | -9762 | ENSG00000144535 | DIS3L2 |
| chr3 | 144425812 | 144427930 | 3,5 | -1,55 | 5,05 | 6,17E-05 | 0,00251 | Distal Intergenic | 733645 | ENSG00000 181744 | DIPK2A |
| chr4 | 96445577 | 96446476 | 2,9 | -1,55 | 4,46 | 6,23E-05 | 0,00252 | Intron (uc003hto.3/863 3, intron 1 of 15) | 23885 | ENSG00000 182168 | UNC5 C |
| chr1 | 65421830 | 65423210 | 2,97 | -1,4 | 4,37 | 6,26E-05 | 0,00253 | 5'UTR | 8977 | ENSG00000162434 | JAK1 |
| chr7 | 11886471 | 11888104 | 3,67 | -1,55 | 5,22 | 6,28E-05 | 0,00253 | Distal Intergenic | -14647 | ENSG00000005108 | THSD7A |
| chr1 1 | 63456303 | 63457577 | 3,71 | -1,55 | 5,26 | 6,32E-05 | 0,00254 | Intron (uc001nxm.3/10313, intron 1 of 7) | 7381 | ENSG00000133318 | RTN3 |
| chr13 | 110122934 | 110124265 | 3,37 | -1,55 | 4,92 | 6,33E-05 | 0,00254 | Distal Intergenic | -303283 | ENSG00000236242 | MYO16-AS1 |
| chr18 | 76475223 | 76477584 | 4,1 | -0,49 | 4,59 | 6,37E-05 | 0,00256 | Distal Intergenic | -262691 | ENSG00000256463 | SALL3 |
| chr10 | 106398446 | 106402957 | 6,01 | 0,68 | 5,32 | 6,37E-05 | 0,00256 | Promoter (<=1kb) | 0 | ENSG00000156395 | SORCS3 |
| chr21 | 44406286 | 44407323 | 3,88 | -0,46 | 4,34 | 6,47E-05 | 0,00258 | Intron (uc002zcp.1/5316, intron 1 of 9) | 11643 | ENSG00000160199 | PKNOX1 |
| chr1 | 219667495 | 219668201 | 3,07 | -1,55 | 4,62 | 6,49E-05 | 0,00259 | Distal Intergenic | 320303 | ENSG00000143353 | LYPLAL1 |
| chr6 | 14635831 | 14637090 | 3,08 | -1,38 | 4,46 | 6,52E-05 | 0,00259 | Distal Intergenic | 517966 | ENSG00000112149 | CD83 |
| chr11 | 15501042 | 15502145 | 3 | -1,55 | 4,55 | 6,54E-05 | 0,0026 | Distal Intergenic | 330394 | ENSG00000188487 | INSC |
| chr9 | 29792878 | 29795148 | 4,41 | -0,62 | 5,04 | 6,54E-05 | 0,0026 | Distal Intergenic | -579880 | ENSG00000174482 | LINGO2 |
| chr2 | 168438073 | 168439604 | 4,51 | -0,1 | 4,61 | 6,60E-05 | 0,00262 | Distal Intergenic | -235578 | ENSG00000172318 | B3GALT1 |
| chr1 | 233058987 | 233060386 | 3,28 | -1,55 | 4,84 | 6,69E-05 | 0,00265 | Distal Intergenic | -25984 | ENSG00000135778 | NIPCR |
| chr3 | 129204459 | 129206028 | 3,58 | -1,55 | 5,14 | 6,71E-05 | 0,00265 | Promoter (<=1kb) | -321 | ENSG00000163913 | IFT122 |
| chr10 | 72032137 | 72033173 | 2,96 | -1,06 | 4,02 | 6,75E-05 | 0,00266 | Intron (uc021psj.1/64106, intron 1 of 3) | 10277 | ENSG00000148734 | NPFFR1 |
| chr4 | 33865517 | 33866477 | 3,3 | -0,93 | 4,23 | 6,96E-05 | 0,00272 | Distal Intergenic | 2208980 | ENSG0000004 7365 | ARAP2 |
| chr4 | 26133668 | 26134476 | 2,58 | -1,55 | 4,13 | 7,01E-05 | 0,00274 | Distal Intergenic | -186856 | ENSG00000168214 | RBPJ |
| chr3 | 165570086 | 165572001 | 3,69 | -1,55 | 5,24 | 7,04E-05 | 0,00275 | Distal Intergenic | -14833 | ENSG00000114200 | BCHE |
| chr6 | 16471626 | 16472716 | 3,08 | -0,62 | 3,7 | 7,15E-05 | 0,00278 | Intron (uc003nbt.3/6310, intron 7 of 8) | 232815 | ENSG00000137198 | GMPR |
| chr21 | 27489505 | 27491636 | 5,15 | 0,73 | 4,42 | 7,16E-05 | 0,00279 | Intron (uc010glk.3/351, intron 1 of 16) | 21072 | ENSG00000142192 | APP |
| chr6 | 22332641 | 22334356 | 3,58 | -1,55 | 5,13 | 7,21E-05 | 0,0028 | Distal Intergenic | -29559 | ENSG00000 172179 | PRL |
| chr6 | 125139931 | 125140826 | 3,39 | -1,29 | 4,68 | 7,29E-05 | 0,00282 | Intron (uc003pzo.3/154215, intron 6 of 6) | -143036 | ENSG00000146373 | RNF217 |
| chr7 | 87227560 | 87228611 | 2,99 | -1,55 | 4,54 | 7,35E-05 | 0,00284 | Promoter (<=1kb) | 895 | ENSG00000085563 | ABCB1 |
| chr7 | 8838663 | 8839565 | 2,92 | -1,55 | 4,48 | 7,39E-05 | 0,00285 | Distal Intergenic | 365078 | ENSG00000122584 | NXPH1 |
| chr5 | 26801334 | 26802248 | 3,42 | -1,02 | 4,44 | 7,59E-05 | 0,0029 | Distal Intergenic | 88694 | ENSG00000113100 | CDH9 |
| chr10 | 20399364 | 20400067 | 3,41 | -0,86 | 4,27 | 7,71E-05 | 0,00294 | Intron (uc001iqg.1/84898, intron 4 of 13) | -32157 | ENSG00000120594 | PLXDC2 |
| chr17 | 406000 | 407301 | 3,55 | -1,55 | 5,1 | 7,72E-05 | 0,00294 | Distal Intergenic | 18932 | ENSG00000141252 | VPS53 |
| chr5 | 65308421 | 65309012 | 2,41 | -1,55 | 3,97 | 7,7 7E-05 | 0,00295 | Intron (uc003jui.2/55914, intron 5 of 24) | 23958 | ENSG00000112851 | ERBIN |
| chr5 | 118732910 | 118733859 | 3,09 | -1,55 | 4,65 | 7,80E-05 | 0,00296 | Distal Intergenic | 41314 | ENSG00000145779 | TNFAIP8 |
| chr13 | 40600830 | 40601448 | 3,16 | -0,51 | 3,67 | 7,81E-05 | 0,00296 | Distal Intergenic | -154498 | ENSG00000230710 | LINC00332 |
| chr7 | 82013122 | 82014308 | 3,32 | -1,55 | 4,88 | 7,83E-05 | 0,00297 | Intron (uc003uhr.1/781, intron 1 of 38) | 58723 | ENSG00000153956 | CACNA2D1 |
| chr12 | 127101027 | 127102883 | 5,17 | 0,68 | 4,49 | 7,84E-05 | 0,00297 | Distal Intergenic | -118670 | NA | LINC00943 |
| chr20 | 1701923 | 1703307 | 3,32 | -1,38 | 4,7 | 7,92E-05 | 0,00299 | Distal Intergenic | 57085 | ENSG00000232528 | LOC100289473 |
| chr10 | 29935548 | 29937097 | 3,64 | -1,55 | 5,19 | 7,96E-05 | 0,003 | Intron (uc001iuu.1/6840, intron 3 of 35) | -11647 | ENSG00000197321 | SVIL |
| chr1 | 86846187 | 86849236 | 5,14 | 0,15 | 4,99 | 7,98E-05 | 0,003 | 5'UTR | 12789 | ENSG00000 122417 | ODF2L |
| chr10 | 121543687 | 121544681 | 2,91 | -1,55 | 4,46 | 8,05E-05 | 0,00302 | Intron (uc001len.4/22876, intron 3 of 4) | -34088 | ENSG00000198825 | INPP5F |
| chr20 | 21537550 | 21541009 | 6,71 | 2,01 | 4,7 | 8,10E-05 | 0,00303 | Distal Intergenic | -42886 | ENSG00000125820 | NKX2-2 |
| chr2 | 96796543 | 96798034 | 3,78 | -0,29 | 4,07 | 8,11E-05 | 0,00303 | Intron (uc010vui.2/431705, intron 6 of 8) | 6141 | ENSG00000188886 | ASTL |
| chr9 | 140772068 | 140773461 | 3,43 | -1,55 | 4,98 | 8,15E-05 | 0,00304 | Promoter (<=1kb) | 0 | ENSG00000148408 | CACNA1B |
| chr18 | 75447917 | 75449738 | 3,52 | -1,55 | 5,07 | 8,19E-05 | 0,00305 | Distal Intergenic | 485909 | ENSG00000166573 | GALR1 |
| chr1 | 238488608 | 238490134 | 3,59 | -1,55 | 5,14 | 8,25E-05 | 0,00307 | Distal Intergenic | 159183 | ENSG00000215808 | LINC01139 |
| chr8 | 113443041 | 113444085 | 3,82 | 0,08 | 3,75 | 8,29E-05 | 0,00308 | Intron (uc003yns.3/114788, intron 19 of 55) | -93081 | ENSG00000164796 | CSMD3 |
| chr15 | 60944693 | 60945806 | 3,17 | -1,29 | 4,45 | 8,34E-05 | 0,00309 | Intron (uc002agx.3/6095, intron 2 of 10) | -24964 | ENSG00000069667 | RORA |
| chr3 | 165375624 | 165376595 | 3,2 | -1,55 | 4,75 | 8,35E-05 | 0,0031 | Distal Intergenic | 178658 | ENSG00000114200 | BCHE |
| chr4 | 90815126 | 90821094 | 5,99 | 0,97 | 5,02 | 8,35E-05 | 0,0031 | Promoter (<=1kb) | 0 | ENSG00000138722 | MMRN1 |
| chr4 | 187548526 | 187549775 | 3,09 | -1,55 | 4,65 | 8,37E-05 | 0,0031 | Exon (uc003izf.3/2195, exon 8 of 27) | -31546 | ENSG00000083857 | FAT1 |
| chr7 | 30382995 | 30384842 | 3,29 | -1,55 | 4,84 | 8,39E-05 | 0,0031 | Intron (uc003tat.3/223082, intron 2 of 4) | -24824 | NA | DKFZP586I1420 |
| chr2 | 103210375 | 103211687 | 3,46 | -1,55 | 5,01 | 8,51E-05 | 0,00314 | Distal Intergenic | -24479 | ENSG00000115616 | SLC9A2 |
| chr1 | 228929571 | 228930590 | 2,41 | -1,55 | 3,96 | 8,53E-05 | 0,00314 | Distal Intergenic | 58747 | ENSG00000116574 | RHOU |
| chr1 | 196527163 | 196528768 | 3,74 | -0,31 | 4,05 | 8,53E-05 | 0,00314 | Intron (uc001gtd.1/343450, intron 1 of 27) | 22843 | ENSG00000265986 | MIR4735 |
| chr19 | 50435549 | 50436366 | 3,33 | -1,55 | 4,89 | 8,54E-05 | 0,00314 | Promoter (<=1kb) | 0 | ENSG00000283842 | MIR4751 |
| chr11 | 26593355 | 26594678 | 3,21 | -1,38 | 4,59 | 8,59E-05 | 0,00316 | Promoter (<=1kb) | 0 | ENSG00000169550 | MUC15 |
| chr8 | 78031191 | 78031873 | 2,73 | -1,55 | 4,28 | 8,61E-05 | 0,00316 | Distal Intergenic | -117911 | ENSG00000164751 | PEX2 |
| chr9 | 130727158 | 130728608 | 3,57 | -1,55 | 5,12 | 8,61E-05 | 0,00316 | Intron (uc004bsx.2/399665, intron 1 of 10) | -14165 | ENSG00000167106 | FAM102A |
| chr10 | 34628919 | 34630537 | 3,18 | -1,55 | 4,73 | 8,66E-05 | 0,00317 | Intron (uc010qej.2/56288, intron 16 of 24) | 42645 | ENSG00000148498 | PARD3 |
| chr12 | 88811081 | 88812820 | 3,47 | -1,55 | 5,02 | 8,68E-05 | 0,00318 | Distal Intergenic | 99824 | ENSG00000049130 | KITLG |
| chr4 | 69770361 | 69772765 | 4,52 | 0,22 | 4,31 | 8,73E-05 | 0,00319 | Distal Intergenic | 44744 | ENSG00000135220 | UGT2A3 |
| chr4 | 95446448 | 95448152 | 3,3 | -1,55 | 4,85 | 8,76E-05 | 0,0032 | Intron (uc003htf.4/10611, intron 3 of 9) | -57400 | ENSG00000163110 | PDLIM5 |
| chr2 | 242882086 | 242882686 | 3,08 | -1,55 | 4,63 | 8,78E-05 | 0,0032 | Distal Intergenic | 70200 | ENSG00000188011 | RTP5 |
| chr3 | 165563621 | 165565002 | 3,59 | -1,55 | 5,15 | 8,78E-05 | 0,0032 | Distal Intergenic | -8368 | ENSG00000114200 | BCHE |
| chr2 | 141286057 | 141287436 | 3,41 | -1,55 | 4,96 | 8,80E-05 | 0,0032 | Intron (uc002tvj.1/53353, intron 47 of 90) | 1601834 | ENSG00000168702 | LRP1B |
| chr4 | 110798315 | 110799134 | 2,64 | -1,55 | 4,19 | 8,83E-05 | 0,00321 | Distal Intergenic | 25829 | ENSG00000183423 | LRIT3 |
| chr3 | 89670611 | 89672118 | 3,27 | -1,55 | 4,82 | 8,91E-05 | 0,00323 | Distal Intergenic | 513937 | ENSG00000044524 | EPHA3 |
| chr11 | 48034902 | 48035835 | 2,68 | -1,55 | 4,23 | 8,92E-05 | 0,00323 | Intron (uc001ngo.4/5795, intron 1 of 8) | 32792 | ENSG00000149177 | PTPRJ |
| chr14 | 92788997 | 92790841 | 5,59 | 0,82 | 4,76 | 8,92E-05 | 0,00323 | Promoter (<=1kb) | 0 | ENSG00000140090 | SLC24A4 |
| chr8 | 126274436 | 126275551 | 3,18 | -1,55 | 4,74 | 8,95E-05 | 0,00324 | Intron (uc003vrw.2/286053, intron 5 of 7) | -167012 | ENSG00000173334 | TRIB 1 |
| chr17 | 48595723 | 48596786 | 3,31 | -1,55 | 4,86 | 8,97E-05 | 0,00324 | Exon (uc002iqz.3/84073, exon 5 of 21) | 9978 | ENSG00000136449 | MYCBPAP |
| chr1 | 92217288 | 92218042 | 2,81 | -1,55 | 4,37 | 9,10E-05 | 0,00327 | Intron (uc009wde.3/7049, intron 7 of 19) | 109561 | ENSG00000069702 | TGFBR3 |
| chr8 | 79651094 | 79652274 | 3,47 | -1,55 | 5,02 | 9,19E-05 | 0,00329 | Exon (uc003vbg.3/3574, exon 3 of 6) | -14485 | NA | LOC101241902 |
| chr18 | 74819704 | 74821119 | 2,87 | -1,55 | 4,42 | 9,23E-05 | 0,00331 | Intron (uc010xfd.2/4155, intron 1 of 8) | 23655 | ENSG00000197971 | MBP |
| chr13 | 54767769 | 54768682 | 3,21 | -1,55 | 4,76 | 9,24E-05 | 0,00331 | Distal Intergenic | 117501 | ENSG00000283126 | MIR1297 |
| chr2 | 191310972 | 191312375 | 3,52 | -1,55 | 5,08 | 9,25E-05 | 0,00331 | Intron (uc002urz.2/54842, intron 3 of 7) | -21843 | ENSG00000151690 | MFSD6 |
| chr1 1 | 68238853 | 68239710 | 2,93 | -1,55 | 4,49 | 9,30E-05 | 0,00332 | Intron (uc010rqb.1/55291, intron 1 of 6) | 10667 | ENSG00000110075 | PPP6R3 |
| chr17 | 78260234 | 78263391 | 6,3 | 0,98 | 5,32 | 9,31E-05 | 0,00332 | Promoter (<=1kb) | 0 | ENSG00000173821 | RNF213 |
| chr7 | 106527660 | 106529786 | 3,34 | -1,55 | 4,89 | 9,37E-05 | 0,00334 | Intron (uc003vdv.4/5294, intron 10 of 10) | 21736 | ENSG00000105851 | PIK3 CG |
| chr2 | 96205145 | 96206233 | 3 | -1,55 | 4,55 | 9,39E-05 | 0,00334 | Distal Intergenic | -51533 | ENSG00000144015 | TRIM43 |
| chr5 | 147569480 | 147570956 | 3,2 | -1,55 | 4,75 | 9,41E-05 | 0,00334 | Distal Intergenic | -11401 | ENSG00000178172 | SPINK6 |
| chr10 | 54330132 | 54330921 | 3 | -0,86 | 3,86 | 9,53E-05 | 0,00337 | Distal Intergenic | 200539 | ENSG00000165471 | MBL2 |
| chr2 | 21553398 | 21554369 | 3,3 | -1,55 | 4,85 | 9,53E-05 | 0,00337 | Distal Intergenic | -286453 | ENSG00000084674 | APOB |
| chr11 | 111106999 | 111107555 | 2,62 | -1,55 | 4,18 | 9,62E-05 | 0,0034 | Distal Intergenic | -19152 | ENSG00000150750 | Cllorf53 |
| chr5 | 164295506 | 164296961 | 3,27 | -1,55 | 4,83 | 9,64E-05 | 0,0034 | Distal Intergenic | 1351043 | ENSG0000003 8274 | MAT2B |
| chr1 | 239679214 | 239680267 | 3,27 | -1,55 | 4,82 | 9,79E-05 | 0,00344 | Intron (uc001hyo.1/1131, intron 2 of 8) | -112106 | ENSG00000133019 | CHRM3 |
| chr21 | 24868538 | 24869674 | 2,75 | -1,55 | 4,3 | 9,86E-05 | 0,00346 | Distal Intergenic | -111382 | ENSG00000228592 | D21S2088E |
| chr6 | 22025536 | 22027126 | 3,23 | -1,55 | 4,78 | 9,95E-05 | 0,00348 | Intron (uc003ndj.3/401237, intron 7 of 11) | -119757 | NA | CASC15 |
| chr14 | 91435466 | 91436589 | 3,24 | -1,55 | 4,79 | 9,98E-05 | 0,00349 | Intron (uc010twi.1/9252, intron 3 of 16) | 89889 | ENSG00000100784 | RPS6KA5 |
| chr5 | 105493322 | 105494099 | 3,06 | -1,14 | 4,19 | 0,000101 | 0,00351 | Distal Intergenic | 1058147 | ENSG00000232159 | RAB9BP1 |
| chr2 | 76676750 | 76677731 | 2,87 | -1,55 | 4,42 | 0,000101 | 0,00351 | Distal Intergenic | -738639 | ENSG00000005436 | GCFC2 |
| chr9 | 971626 | 972416 | 3,39 | -1,31 | 4,7 | 0,000102 | 0,00353 | Distal Intergenic | -4552 | ENSG00000064218 | DMRT3 |
| chr2 | 232891936 | 232892933 | 3,28 | -0,93 | 4,21 | 0,000102 | 0,00354 | Promoter (2-3kb) | 2949 | ENSG00000144535 | DIS3L2 |
| chr2 | 214402203 | 214403253 | 2,89 | -1,55 | 4,44 | 0,000103 | 0,00356 | Intron (uc010fuz.3/79582, intron 8 of 13) | 253063 | ENSG00000144451 | SPAG16 |
| chrl6 | 23515518 | 23516808 | 2,98 | -1,55 | 4,53 | 0,000105 | 0,00363 | Intron (uc002dlq.3/23062, intron 1 of 16) | 5007 | ENSG00000103365 | GGA2 |
| chr2 | 205920227 | 205921415 | 3,12 | -1,55 | 4,67 | 0,000105 | 0,00363 | Intron (uc010fub.2/117583, intron 4 of 18) | 509711 | ENSG00000116117 | PARD3B |
| chr10 | 121298263 | 121299460 | 3,47 | -1,55 | 5,03 | 0,000106 | 0,00364 | Promoter (2-3kb) | -2218 | ENSG00000148908 | RGS10 |
| chr21 | 22710400 | 22711275 | 3,24 | -1,55 | 4,8 | 0,000106 | 0,00365 | Exon (uc002yld.2/4685, exon 8 of 18) | 52578 | NA | RNU6-67P |
| chr2 | 164512847 | 164513426 | 2,66 | -1,55 | 4,21 | 0,000107 | 0,00367 | Intron (uc002uck.1/55137, intron 2 of 2) | 79087 | ENSG00000 182263 | FIGN |
| chr10 | 104884877 | 104885953 | 2,84 | -1,55 | 4,4 | 0,000107 | 0,00367 | Intron (uc010qqp.2/22978, intron 2 of 16) | 27431 | ENSG00000076685 | NT5C2 |
| chr1 1 | 131762747 | 131763678 | 2,48 | -1,55 | 4,04 | 0,000108 | 0,00369 | Intron (uc001qgm.3/50863, intron 1 of 7) | -15822 | ENSG00000 182667 | NTM |
| chr21 | 22559196 | 22560349 | 3,79 | -0,86 | 4,65 | 0,000109 | 0,00371 | Intron (uc002vld.2/4685, intron 1 of 17) | 39758 | ENSG00000154654 | NCAM2 |
| chr10 | 91087252 | 91090813 | 5,04 | 0,28 | 4,76 | 0,000109 | 0,00371 | Promoter (<=1kb) | 0 | ENSG00000119917 | IFIT3 |
| chr21 | 17386096 | 17387036 | 3,61 | -0,72 | 4,33 | 0,000109 | 0,00372 | Distal Intergenic | -55806 | NA | MIR99 AHG |
| chr11 | 119563098 | 119565123 | 3,36 | -1,02 | 4,38 | 0,000109 | 0,00372 | Intron (uc001pwu.1/5818, intron 1 of 7) | 34312 | ENSG00000110400 | NECTIN1 |
| chr17 | 62192224 | 62196644 | 5,47 | 0,3 | 5,18 | 0,000109 | 0,00372 | Intron (uc002jdz.2/2081, intron 1 of 21) | 10858 | ENSG00000178607 | ERN1 |
| chr20 | 8474984 | 8475791 | 2,86 | -1,55 | 4,42 | 0,00011 | 0,00374 | Intron (uc010zrb.1/23236, intron 3 of 27) | -105590 | ENSG00000 182621 | PLCB1 |
| chr10 | 1161820 | 1163417 | 3,1 | -1,55 | 4,65 | 0,000112 | 0,00377 | Intron (uc031ptc.1/22884, intron 11 of 13) | 41976 | ENSG00000047056 | WDR3 7 |
| chr4 | 48029926 | 48031620 | 3,14 | -1,55 | 4,69 | 0,000112 | 0,00378 | Intron (uc003gxw.3/152519, intron 2 of 5) | 11135 | ENSG00000163293 | NIPAL1 |
| chr3 | 28882892 | 28884217 | 3,41 | -1,55 | 4,97 | 0,000113 | 0,00379 | Distal Intergenic | 266123 | NA | LINC00693 |
| chr15 | 99246048 | 99247114 | 3,08 | -1,55 | 4,64 | 0,000113 | 0,0038 | Intron (uc010urq.2/3480, intron 1 of 10) | 53287 | ENSG00000140443 | IGF1R |
| chr2 | 65833016 | 65834324 | 3,77 | -0,03 | 3,8 | 0,000114 | 0,00381 | Intron (uc010fcy.1/uc010fcy.1, intron 4 of 9) | -173360 | ENSG00000198369 | SPRED2 |
| chr3 | 165538024 | 165539436 | 3,25 | -1,55 | 4,8 | 0,000115 | 0,00383 | Intron (uc003fem.4/590, intron 2 of 3) | 15817 | ENSG00000114200 | BCHE |
| chr8 | 78049897 | 78050515 | 2,57 | -1,55 | 4,13 | 0,000116 | 0,00385 | Distal Intergenic | -136617 | ENSG00000164751 | PEX2 |
| chr22 | 19701404 | 19704218 | 6,95 | 2,6 | 4,34 | 0,000116 | 0,00385 | Promoter (<=1kb) | 0 | ENSG00000284874 | SEPT5-GP1BB |
| chr12 | 39104291 | 39105832 | 3,23 | -1,55 | 4,79 | 0,000116 | 0,00386 | Intron (uc001rlr.1/144402, intron 1 of 7) | 9192 | ENSG00000139117 | CPNE8 |
| chr5 | 90830366 | 90831987 | 3,56 | -1,55 | 5,11 | 0,000116 | 0,00386 | Distal Intergenic | -151217 | ENSG00000113369 | ARRDC3 |
| chr22 | 41612987 | 41614226 | 3,15 | -1,55 | 4,71 | 0,000117 | 0,00388 | Exon (uc010gyi.1/83746, exon 5 of 14) | 11674 | ENSG00000100395 | L3MBTL2 |
| chr4 | 95260202 | 95261160 | 2,9 | -1,38 | 4,29 | 0,000118 | 0,0039 | Promoter (2-3kb) | 2867 | ENSG00000163106 | HPGDS |
| chr1 | 186152637 | 186153824 | 4,35 | 0,3 | 4,05 | 0,000119 | 0,00394 | Intron (uc001grq.1/83872, intron 105 of 106) | 55427 | ENSG00000143341 | HMCN1 |
| chr3 | 72442762 | 72443731 | 2,92 | -1,55 | 4,47 | 0,000119 | 0,00394 | Intron (uc003dpe.3/23429, intron 1 of 3) | 52043 | ENSG00000163602 | RYBP |
| chr21 | 40757615 | 40758572 | 3,05 | -1,55 | 4,6 | 0,00012 | 0,00394 | Promoter (1-2kb) | -1120 | ENSG00000 182093 | WRB |
| chr9 | 103812728 | 103813677 | 2,96 | -1,55 | 4,51 | 0,000121 | 0,00397 | Intron (uc004bbb.3/54886, intron 1 of 7) | 21697 | ENSG00000148123 | PLPPR1 |
| chr12 | 42702746 | 42704260 | 3,35 | -1,55 | 4,9 | 0,000121 | 0,00397 | Downstream (1-2kb) | 15672 | ENSG00000139168 | ZCRB 1 |
| chr17 | 13983430 | 13985451 | 3,42 | -1,55 | 4,97 | 0,000121 | 0,00397 | Intron (uc002gof.4/1352, intron 3 of 6) | -10655 | ENSG00000236088 | COX10-AS1 |
| chr2 | 12641016 | 12642424 | 3,28 | -0,9 | 4,18 | 0,000122 | 0,00398 | Intron (uc002rbu.1/uc002rbu.1, intron 4 of 5) | -214574 | ENSG00000071575 | TRIB2 |
| chr9 | 1574203 | 1575719 | 3,26 | -1,55 | 4,81 | 0,000123 | 0,004 | Distal Intergenic | -439623 | ENSG00000080503 | SMARCA2 |
| chr14 | 93532052 | 93533337 | 2,93 | -1,55 | 4,48 | 0,000123 | 0,00401 | Promoter (<=1kb) | -460 | ENSG00000258730 | ITPK1-AS 1 |
| chr3 | 171847083 | 171848687 | 3,06 | -1,55 | 4,62 | 0,000123 | 0,00401 | Promoter (2-3kb) | -2574 | ENSG00000075420 | FNDC3B |
| chr2 | 43521244 | 43522191 | 2,77 | -1,55 | 4,32 | 0,000124 | 0,00403 | Intron (uc010far.3/63892, intron 16 of 22) | 66894 | ENSG00000279873 | LINC01126 |
| chr7 | 15439069 | 15440019 | 2,76 | -1,55 | 4,32 | 0,000124 | 0,00403 | Intron (uc003stb.1/392636, intron 5 of 12) | 161621 | ENSG00000187546 | AGMO |
| chr16 | 17331320 | 17333132 | 3,01 | -1,55 | 4,56 | 0,000125 | 0,00405 | Intron (uc002dfa.3/64131, intron 3 of 11) | 231606 | ENSG00000103489 | XYLT1 |
| chr5 | 5754926 | 5756765 | 3,52 | -1,26 | 4,78 | 0,000125 | 0,00405 | Distal Intergenic | 332140 | ENSG00000164151 | ICE1 |
| chr14 | 91848440 | 91850417 | 3,65 | -1,14 | 4,79 | 0,000125 | 0,00405 | Intron (uc010aty.3/440193, intron 3 of 29) | 32765 | ENSG00000015133 | CCDC88C |
| chr13 | 95362777 | 95365833 | 6,73 | 1,79 | 4,94 | 0,000125 | 0,00405 | Promoter (<=1kb) | 0 | ENSG00000125285 | SOX21 |
| chr17 | 63831860 | 63832778 | 2,46 | -1,55 | 4,01 | 0,000125 | 0,00406 | Intron (uc010deo.3/201134, intron 13 of 16) | -9199 | ENSG00000154240 | CEP112 |
| chr1 | 189173737 | 189174892 | 3,19 | -1,55 | 4,74 | 0,000126 | 0,00408 | Distal Intergenic | 1270050 | ENSG00000162670 | BRINP3 |
| chr2 | 198565641 | 198566478 | 3,17 | -1,55 | 4,72 | 0,000126 | 0,00409 | Intron (uc002uup.3/uc002uup.3, intron 2 of 3) | -3550 | ENSG00000247626 | mars-02 |
| chr11 | 88087004 | 88088035 | 2,83 | -1,55 | 4,38 | 0,000127 | 0,00411 | Distal Intergenic | -16063 | ENSG00000109861 | CTSC |
| chr6 | 158787053 | 158788312 | 3,12 | -1,55 | 4,67 | 0,000127 | 0,00411 | Intron (uc01 1efo.2/56995, intron 1 of 7) | 53361 | ENSG00000130338 | TULP4 |
| chr10 | 29887373 | 29888580 | 3,29 | -1,55 | 4,84 | 0,000129 | 0,00413 | Promoter (2-3kb) | 2695 | ENSG00000216035 | MIR938 |
| chr6 | 117798930 | 117800704 | 5,24 | 1,55 | 3,7 | 0,000129 | 0,00414 | Intron (uc003pxq.1/57120, intron 4 of 6) | -3116 | ENSG00000164465 | DCBLD1 |
| chr8 | 48947104 | 48948081 | 3,07 | -1,55 | 4,62 | 0,00013 | 0,00417 | Intron (uc003xqm.3/7336, intron 1 of 3) | 26109 | ENSG00000169139 | UBE2 V2 |
| chr7 | 9977734 | 9979271 | 3,31 | -1,55 | 4,86 | 0,00013 | 0,00417 | Distal Intergenic | 303834 | NA | PER4 |
| chr2 | 153022922 | 153024230 | 3,4 | -1,55 | 4,95 | 0,00013 | 0,00417 | Intron (uc002tvc.4/10254, intron 1 of 13) | 8276 | ENSG00000115145 | STAM2 |
| chr9 | 94277282 | 94278278 | 3 | -1,35 | 4,35 | 0,000131 | 0,00417 | Distal Intergenic | -91138 | ENSG00000165030 | NFIL3 |
| chr19 | 8590048 | 8590591 | 2,87 | -1,55 | 4,43 | 0,000131 | 0,0042 | Exon (uc002mkg.3/4542, exon 25 of 28) | -11000 | ENSG00000133250 | ZNF414 |
| chrX | 25085288 | 25086269 | 2,78 | -0,89 | 3,67 | 0,000132 | 0,0042 | Distal Intergenic | -51223 | ENSG00000004848 | ARX |
| chr20 | 44513225 | 44514238 | 2,91 | -1,55 | 4,47 | 0,000132 | 0,0042 | Promoter (1-2kb) | 2000 | ENSG00000149634 | SPATA25 |
| chr8 | 117957279 | 117958892 | 3,29 | -1,55 | 4,84 | 0,000132 | 0,00421 | Distal Intergenic | -3620 | ENSG00000164756 | SLC30A8 |
| chr7 | 103678764 | 103679419 | 2,66 | -1,55 | 4,21 | 0,000133 | 0,00423 | Distal Intergenic | -48801 | ENSG00000189056 | RELN |
| chr3 | 149888530 | 149889468 | 3,01 | -1,23 | 4,24 | 0,000133 | 0,00423 | Distal Intergenic | 199464 | NA | LOC646903 |
| chr6 | 24554280 | 24555586 | 2,92 | -1,55 | 4,47 | 0,000133 | 0,00423 | Exon (uc010jpt.1/9856, exon 15 of 17) | 27869 | ENSG00000137261 | KIAA0319 |
| chr1 | 186375894 | 186377024 | 2,89 | -1,55 | 4,44 | 0,000134 | 0,00425 | Intron (uc021pgg.1/54953, intron 12 of 13) | 6190 | ENSG00000262180 | OCLM |
| chr4 | 6964784 | 6966781 | 4,04 | -0,4 | 4,44 | 0,000134 | 0,00426 | Intron (uc011bwg.2/57533, intron 2 of 13) | -22108 | ENSG00000132405 | TBC1D14 |
| chr12 | 128058266 | 128059691 | 3,59 | -0,75 | 4,34 | 0,000135 | 0,00428 | Distal Intergenic | -306471 | ENSG00000256597 | LINC02393 |
| chr7 | 155452134 | 155453727 | 3,33 | -1,55 | 4,88 | 0,000135 | 0,00428 | Intron (uc003wme.3/155435, intron 1 of 5) | 14931 | ENSG00000 184863 | RBM33 |
| chr6 | 128385085 | 128386599 | 3,24 | -1,55 | 4,79 | 0,000136 | 0,0043 | Exon (uc003qbj.3/5796, exon 13 of 30) | -58696 | ENSG00000152894 | PTPRK |
| chr10 | 97747542 | 97748451 | 2,59 | -1,55 | 4,14 | 0,000136 | 0,00431 | Exon (uc001klk.3/387707, exon 5 of 14) | -11397 | ENSG00000188649 | CC2D2B |
| chr8 | 109566551 | 109567949 | 3,28 | -1,55 | 4,83 | 0,000136 | 0,00431 | Distal Intergenic | 110698 | ENSG00000104412 | EMC2 |
| chr7 | 125107784 | 125109274 | 3,34 | -1,55 | 4,89 | 0,000136 | 0,00431 | Distal Intergenic | -537747 | ENSG00000128513 | POT1 |
| chr1 | 240626044 | 240630869 | 5,36 | 0,71 | 4,65 | 0,000137 | 0,00431 | Intron (uc010pvd.2/56776, intron 16 of 17) | 5250 | ENSG00000155816 | FMN2 |
| chr6 | 112144339 | 112146004 | 3,3 | -1,55 | 4,86 | 0,000137 | 0,00431 | Intron (uc003pvk.3/2534, intron 2 of 13) | -29255 | ENSG00000010810 | FYN |
| chr8 | 61720500 | 61722434 | 4,75 | 0,13 | 4,62 | 0,000138 | 0,00433 | Exon (uc003xue.3/55636, exon 7 of 38) | -6512 | ENSG00000171316 | CHD7 |
| chr7 | 50728412 | 50733482 | 6,71 | 1,84 | 4,87 | 0,000138 | 0,00433 | Intron (uc003tph.3/2887, intron 4 of 15) | 39538 | ENSG00000106070 | GRB10 |
| chr17 | 55539997 | 55541153 | 3,01 | -1,55 | 4,57 | 0,000139 | 0,00435 | Intron (uc010wnm.1/124540, intron 6 of 13) | 200521 | ENSG00000153944 | MSI2 |
| chr7 | 13913717 | 13914911 | 3,3 | -1,55 | 4,85 | 0,000139 | 0,00435 | Distal Intergenic | 110234 | ENSG00000006468 | ETV1 |
| chr17 | 72638047 | 72639103 | 2,54 | -1,55 | 4,09 | 0,00014 | 0,00438 | Distal Intergenic | -18150 | ENSG00000186407 | CD300E |
| chr8 | 103168520 | 103169994 | 3,65 | -0,61 | 4,25 | 0,00014 | 0,00438 | Distal Intergenic | 30860 | ENSG00000266756 | MIR5680 |
| chr5 | 173341612 | 173343906 | 3,19 | -1,55 | 4,74 | 0,000141 | 0,00439 | Intron (uc010jju.2/80315, intron 2 of 6) | 21531 | ENSG00000113742 | CPEB4 |
| chr6 | 121762257 | 121764060 | 3,31 | -1,55 | 4,87 | 0,000141 | 0,00439 | Intron (uc011ebo.1/2697, intron 1 of 2) | 5512 | ENSG00000152661 | GJA1 |
| chr1 | 99912035 | 99912804 | 2,58 | -1,06 | 3,64 | 0,000142 | 0,00442 | Distal Intergenic | 182187 | ENSG00000117600 | PLPPR4 |
| chr11 | 133814517 | 133816179 | 3,8 | -0,02 | 3,82 | 0,000142 | 0,00442 | Promoter (<=1kb) | 8 | ENSG00000080854 | IGSF9B |
| chr11 | 38300007 | 38300708 | 2,87 | -1,55 | 4,42 | 0,000143 | 0,00444 | Distal Intergenic | -1680178 | ENSG00000175097 | RAG2 |
| chr8 | 15787869 | 15789111 | 2,93 | -1,55 | 4,48 | 0,000143 | 0,00444 | Distal Intergenic | 186822 | ENSG00000104723 | TUSC3 |
| chr17 | 87831 | 88878 | 3,02 | -1,55 | 4,57 | 0,000143 | 0,00444 | Intron (uc010vpy.2/9501, intron 5 of 6) | -56411 | ENSG00000272636 | DOC2B |
| chr2 | 88122869 | 88124224 | 3,23 | -1,55 | 4,79 | 0,000143 | 0,00445 | Promoter (1-2kb) | 1062 | ENSG00000187627 | RGPD1 |
| chr20 | 3657480 | 3657732 | 1,91 | -1,55 | 3,46 | 0,000144 | 0,00445 | 3'UTR | -3086 | ENSG00000149451 | ADAM33 |
| chr6 | 31244458 | 31245238 | 2,66 | -1,14 | 3,8 | 0,000144 | 0,00445 | Intron (uc031snl.1/3106, intron 4 of 4) | -4545 | ENSG00000204525 | HLA-C |
| chr21 | 20050532 | 20051868 | 2,79 | -1,55 | 4,34 | 0,000144 | 0,00445 | Intron (uc010plf.2/uc010plf.2, intron 1 of 3) | -274562 | ENSG00000154646 | TMPRSS15 |
| chr16 | 90050058 | 90051414 | 3,27 | -1,55 | 4,82 | 0,000146 | 0,0045 | 3'UTR | 3408 | ENSG00000223959 | AFG3L1P |
| chr12 | 100575168 | 100576184 | 2,9 | -1,55 | 4,45 | 0,000149 | 0,00457 | Distal Intergenic | -7478 | ENSG00000221476 | MIR1827 |
| chr1 | 198349479 | 198350209 | 2,59 | -1,55 | 4,14 | 0,00015 | 0,0046 | Distal Intergenic | 159606 | ENSG00000151418 | ATP6V1G3 |
| chr4 | 108970319 | 108971214 | 2,97 | -1,55 | 4,52 | 0,00015 | 0,0046 | Intron (uc011cfj.1/51176, intron 9 of 9) | 29556 | ENSG00000138795 | LEF1 |
| chr3 | 157845543 | 157846853 | 3,05 | -1,55 | 4,61 | 0,000152 | 0,00463 | Intron (uc01 1bou.2/51319, intron 3 of 6) | 17702 | ENSG00000174891 | RSRC1 |
| chr10 | 38104579 | 38106225 | 3,13 | -1,55 | 4,69 | 0,000152 | 0,00464 | Intron (uc031pue.1/57209, intron 6 of 6) | -20476 | NA | ZNF33BP1 |
| chrX | 8949431 | 8950841 | 3,06 | -0,89 | 3,95 | 0,000153 | 0,00465 | Distal Intergenic | 50275 | ENSG00000177138 | FAM9B |
| chr3 | 187366152 | 187366984 | 3,19 | -0,8 | 3,99 | 0,000153 | 0,00465 | Distal Intergenic | 21217 | ENSG00000157005 | SST |
| chr5 | 105075814 | 105076552 | 2,62 | -1,55 | 4,17 | 0,000153 | 0,00465 | Distal Intergenic | 640639 | ENSG00000232159 | RAB9BP1 |
| chr6 | 52356003 | 52357612 | 3,93 | -0,55 | 4,48 | 0,000153 | 0,00465 | 3'UTR | 13322 | ENSG00000065308 | TRAM2 |
| chr2 | 120619332 | 120620593 | 3,03 | -1,55 | 4,58 | 0,000153 | 0,00465 | Exon (uc002tmf.2/5775, exon 3 of 27) | -19080 | ENSG00000088179 | PTPN4 |
| chr18 | 76485230 | 76486241 | 3,11 | -1,55 | 4,67 | 0,000154 | 0,00468 | Distal Intergenic | -254034 | ENSG00000256463 | SALL3 |
| chr4 | 137603314 | 137609581 | 5,64 | 1,01 | 4,63 | 0,000156 | 0,00472 | Distal Intergenic | 844048 | ENSG00000189184 | PCDH18 |
| chr4 | 181381173 | 181382323 | 3,1 | -1,55 | 4,66 | 0,000157 | 0,00473 | Distal Intergenic | 697979 | ENSG00000248197 | LINC00290 |
| chr19 | 50850304 | 50850942 | 2,33 | -1,55 | 3,88 | 0,00016 | 0,00481 | Promoter (2-3kb) | -2299 | ENSG00000131401 | NAPSB |
| chr3 | 167768714 | 167771107 | 5,23 | 0,27 | 4,96 | 0,000161 | 0,00482 | Intron (uc003ffe.2/27333, intron 1 of 15) | 42310 | ENSG00000173905 | GOLIM4 |
| chrX | 141478455 | 141480947 | 5,63 | 0,77 | 4,86 | 0,000161 | 0,00483 | Distal Intergenic | -185379 | ENSG00000046774 | MAGEC2 |
| chr18 | 60882619 | 60884934 | 7,1 | 2,07 | 5,03 | 0,000162 | 0,00485 | Intron (uc002lit.1/596, intron 2 of 2) | 101679 | ENSG00000171791 | BCL2 |
| chr1 | 182598543 | 182599557 | 2,87 | -1,55 | 4,42 | 0,000162 | 0,00486 | Distal Intergenic | 14268 | ENSG00000261504 | LINC01686 |
| chr3 | 150901118 | 150901932 | 2,67 | -1,55 | 4,23 | 0,000163 | 0,00486 | Intron (uc003eyn.3/116931, intron 10 of 14) | 19056 | ENSG00000174946 | GPR171 |
| chr2 | 204605262 | 204606709 | 4,11 | -0,46 | 4,57 | 0,000163 | 0,00486 | Distal Intergenic | 34064 | ENSG00000178562 | CD28 |
| chr4 | 137554989 | 137556635 | 3,25 | -1,55 | 4,8 | 0,000163 | 0,00487 | Distal Intergenic | 896994 | ENSG00000189184 | PCDH18 |
| chr9 | 126021355 | 126022456 | 3,01 | -1,55 | 4,56 | 0,000164 | 0,00489 | Intron (uc004bns.3/55342, intron 1 of 18) | 8399 | ENSG00000165209 | STRBP |
| chr21 | 22683762 | 22684758 | 3,19 | -1,55 | 4,74 | 0,000165 | 0,00492 | Intron (uc002vld.2/4685, intron 5 of 17) | 25940 | NA | RNU6-67P |
| chr1 | 73824840 | 73826835 | 4,09 | -0,75 | 4,84 | 0,000166 | 0,00493 | Distal Intergenic | 837036 | ENSG00000162620 | LRRIQ3 |
| chr6 | 75307163 | 75308159 | 2,71 | -1,55 | 4,26 | 0,000166 | 0,00494 | Intron (uc003phr.3/uc003phr.3, intron 2 of 3) | 590092 | ENSG00000111799 | COL12A1 |
| chr2 | 111890295 | 111891369 | 2,85 | -1,55 | 4,41 | 0,000167 | 0,00495 | Intron (uc021vmo.1/10018, intron 3 of 5) | 11804 | ENSG00000153094 | BCL2L11 |
| chr20 | 60457180 | 60458066 | 2,57 | -1,55 | 4,13 | 0,000169 | 0,00501 | Intron (uc002vbn.2/1002, intron 7 of 15) | 70652 | ENSG00000284193 | MIR1257 |
| chr13 | 110959134 | 110961470 | 3,69 | -0,63 | 4,32 | 0,000171 | 0,00503 | Promoter (<=1kb) | 0 | ENSG00000187498 | COL4A1 |
| chr1 | 178990783 | 178991590 | 2,8 | -1,55 | 4,35 | 0,000171 | 0,00504 | Distal Intergenic | -3484 | ENSG00000116199 | FAM20B |
| chr8 | 2556929 | 2560589 | 5,26 | 0,19 | 5,07 | 0,000173 | 0,00507 | Intron (uc003wqb.1/uc003wqb.1, intron 2 of 2) | 484898 | ENSG00000183117 | CSMD1 |
| chr4 | 6182903 | 6184009 | 3,03 | -0,93 | 3,96 | 0,000174 | 0,00511 | Intron (uc010idb.1/152789, intron 1 of 20) | 12337 | ENSG00000152969 | JAKMIP1 |
| chr8 | 59711644 | 59712737 | 2,61 | -1,55 | 4,17 | 0,000174 | 0,00511 | Distal Intergenic | -139240 | ENSG00000035681 | NSMAF |
| chr6 | 157990722 | 157991637 | 2,36 | -0,97 | 3,33 | 0,000175 | 0,00512 | Intron (uc003qqs.3/79683, intron 2 of 8) | 27105 | ENSG00000175048 | ZDHHC14 |
| chr17 | 46709926 | 46710914 | 2,51 | -1,55 | 4,06 | 0,000175 | 0,00512 | Promoter (<=1kb) | -5 | ENSG00000210741 | MIR196A1 |
| chr19 | 23901687 | 23903049 | 3,44 | -1,1 | 4,54 | 0,000175 | 0,00512 | Distal Intergenic | -31670 | ENSG00000197372 | ZNF675 |
| chr13 | 35965915 | 35967207 | 3,36 | -1,26 | 4,62 | 0,000175 | 0,00512 | Intron (uc021ric.1/26960, intron 37 of 56) | -78440 | ENSG00000172915 | NBEA |
| chr3 | 60174526 | 60175413 | 2,58 | -1,55 | 4,14 | 0,000175 | 0,00513 | Intron (uc003dkx.4/2272, intron 5 of 9) | 1061720 | ENSG00000189283 | FHIT |
| chr5 | 19656457 | 19657379 | 2,8 | -1,38 | 4,18 | 0,000176 | 0,00513 | Intron (uc021xwu.1/1016, intron 5 of 12) | 229002 | ENSG00000145526 | CDH18 |
| chr12 | 9819703 | 9828418 | 8,08 | 3,32 | 4,76 | 0,000176 | 0,00513 | Promoter (<=1kb) | 0 | ENSG00000069493 | CLEC2D |
| chr2 | 63351272 | 63352202 | 2,57 | -1,55 | 4,13 | 0,000177 | 0,00515 | Intron (uc002sce.3/51057, intron 8 of 8) | -4595 | ENSG00000242412 | DBIL5P2 |
| chr8 | 68482840 | 68484712 | 3,5 | -1,26 | 4,76 | 0,000178 | 0,00518 | Intron (uc003xxq.4/57094, intron 2 of 10) | 173908 | ENSG00000165078 | CPA6 |
| chr1 | 100222989 | 100224879 | 4,84 | 1,17 | 3,67 | 0,000178 | 0,00519 | Intron (uc001dsh.1/391059, intron 1 of 16) | 6470 | ENSG00000156869 | FRRS1 |
| chr2 | 9434506 | 9435196 | 2,74 | -0,93 | 3,67 | 0,000179 | 0,00519 | Intron (uc002qzh.2/8853, intron 2 of 27) | 87612 | ENSG00000151693 | ASAP2 |
| chr12 | 122771768 | 122772408 | 2,43 | -1,4 | 3,83 | 0,000179 | 0,00521 | Intron (uc001ucg.2/6249, intron 21 of 25) | -13051 | ENSG00000130779 | CLIP1 |
| chr1 | 90184347 | 90186047 | 3,73 | -0,61 | 4,34 | 0,00018 | 0,00523 | 3'UTR | 85703 | ENSG00000171488 | LRRC8C |
| chr3 | 73010206 | 73011357 | 3 | -1,31 | 4,32 | 0,000182 | 0,00527 | Intron (uc003dpg.3/727936, intron 5 of 6) | -34762 | ENSG00000163605 | PPP4R2 |
| chr1 | 82300574 | 82301473 | 2,88 | -1,55 | 4,43 | 0,000182 | 0,00527 | Intron (uc001dis.3/23266, intron 5 of 8) | 34492 | ENSG00000117114 | ADGRL2 |
| chr15 | 86161836 | 86162770 | 3,25 | -1,55 | 4,8 | 0,000183 | 0,00528 | Promoter (<=1kb) | -411 | ENSG00000170776 | AKAP13 |
| chr7 | 40539396 | 40540215 | 2,65 | -1,55 | 4,2 | 0,000184 | 0,00531 | Intron (uc003thn.2/79783, intron 12 of 13) | -292841 | ENSG00000175600 | SUGCT |
| chr9 | 24520050 | 24520801 | 2,91 | -1,55 | 4,46 | 0,000184 | 0,00531 | Distal Intergenic | 24873 | ENSG00000205442 | IZUMO3 |
| chr13 | 78659552 | 78660627 | 3,14 | -1,55 | 4,69 | 0,000184 | 0,00531 | Intron (uc001vks.3/100874222, intron 1 of 2) | 30562 | ENSG00000234377 | RNF219-AS1 |
| chr13 | 104411073 | 104412214 | 3,15 | -1,55 | 4,71 | 0,000184 | 0,00532 | Distal Intergenic | -476225 | ENSG00000263741 | MIR548AS |
| chr6 | 157296731 | 157298498 | 6,06 | 1,16 | 4,9 | 0,000185 | 0,00533 | Intron (uc003qqn.3/57492, intron 4 of 19) | 40131 | ENSG00000049618 | ARID1B |
| chr1 | 36947028 | 36948705 | 5,03 | 1,12 | 3,9 | 0,000185 | 0,00534 | Promoter (<=1kb) | 210 | ENSG00000119535 | CSF3R |
| chr4 | 45552690 | 45553442 | 2,59 | -1,55 | 4,14 | 0,000186 | 0,00534 | Distal Intergenic | 572640 | ENSG00000163285 | GABRG1 |
| chr1 | 117906195 | 117907014 | 2,86 | -1,55 | 4,41 | 0,000186 | 0,00535 | Distal Intergenic | -3071 | ENSG00000198162 | MAN1A2 |
| chr9 | 25537715 | 25538657 | 2,86 | -1,55 | 4,42 | 0,000186 | 0,00536 | Distal Intergenic | 140199 | ENSG00000198680 | TUSC1 |
| chr9 | 3730255 | 3731428 | 3,47 | -0,31 | 3,78 | 0,000188 | 0,0054 | Distal Intergenic | -167218 | ENSG00000237009 | GLIS3-AS1 |
| chr8 | 68728825 | 68729719 | 2,81 | -0,87 | 3,68 | 0,000191 | 0,00546 | Distal Intergenic | -70205 | ENSG00000165078 | CPA6 |
| chr2 | 154038520 | 154039064 | 2,46 | -1,55 | 4,02 | 0,000192 | 0,00547 | Distal Intergenic | 296258 | ENSG00000177519 | RPRM |
| chr7 | 7887130 | 7888228 | 2,56 | -1,55 | 4,12 | 0,000192 | 0,00548 | Intron (uc011jxf.2/729852, intron 3 of 3) | -120146 | ENSG00000106415 | GLCCI1 |
| chr15 | 86246006 | 86247092 | 2,96 | -1,55 | 4,52 | 0,000193 | 0,0055 | Intron (uc002btu.2/11214, intron 17 of 36) | 25738 | ENSG00000170776 | AKAP13 |
| chr5 | 19270514 | 19271759 | 2,8 | -1,55 | 4,35 | 0,000193 | 0,00551 | Distal Intergenic | 614622 | ENSG00000145526 | CDH18 |
| chr16 | 17200421 | 17202082 | 3,29 | -1,06 | 4,35 | 0,000195 | 0,00556 | 3'UTR | 362656 | ENSG00000103489 | XYLT1 |
| chr7 | 40514437 | 40514841 | 2,44 | -1,4 | 3,85 | 0,000196 | 0,00557 | Intron (uc003thn.2/79783, intron 11 of 13) | -318215 | ENSG00000175600 | SUGCT |
| chr5 | 81407211 | 81407676 | 2,17 | -1,55 | 3,72 | 0,000197 | 0,00559 | Intron (uc003khq.2/83734, intron 3 of 3) | 139367 | ENSG00000152348 | ATG10 |
| chr2 | 141174228 | 141176011 | 3,19 | -1,55 | 4,74 | 0,000198 | 0,00561 | Intron (uc002tvj.1/53353, intron 66 of 90) | 1519334 | NA | YY1P2 |
| chr7 | 124306734 | 124308182 | 4,01 | -0,1 | 4,11 | 2,00E-04 | 0,00565 | Distal Intergenic | 97897 | ENSG00000170775 | GPR37 |
| chr21 | 21210271 | 21211742 | 3,12 | -1,55 | 4,67 | 2,00E-04 | 0,00566 | Distal Intergenic | 963684 | ENSG00000224924 | LINC00320 |
| chr14 | 80067574 | 80068347 | 2,76 | -1,55 | 4,31 | 0,000202 | 0,0057 | Intron (uc001xum.2/9369, intron 15 of 20) | 321892 | ENSG00000021645 | NRXN3 |
| chr16 | 28332741 | 28335177 | 5,16 | 0,57 | 4,59 | 0,000205 | 0,00577 | 3'UTR | 28901 | ENSG00000188322 | SBK1 |
| chr4 | 30764908 | 30766650 | 3,47 | -0,87 | 4,34 | 0,000205 | 0,00578 | Intron (uc011bxx.2/5099, intron 1 of 2) | 42871 | ENSG00000169851 | PCDH7 |
| chr3 | 181649503 | 181650383 | 2,78 | -1,38 | 4,17 | 0,000206 | 0,00578 | Distal Intergenic | 219791 | ENSG00000181449 | SOX2 |
| chr6 | 144929236 | 144930067 | 2,5 | -1,55 | 4,05 | 0,000206 | 0,00579 | Intron (uc003qkt.3/7402, intron 50 of 73) | 316363 | ENSG00000152818 | UTRN |
| chr3 | 32855873 | 32856499 | 2,46 | -1,55 | 4,01 | 0,000207 | 0,00579 | Distal Intergenic | -3011 | ENSG00000206557 | TRIM71 |
| chr18 | 21002363 | 21003518 | 3,05 | -1,55 | 4,61 | 0,000208 | 0,00582 | Intron (uc010xaq.2/85019, intron 2 of 15) | 14407 | ENSG00000134490 | TMEM241 |
| chr2 | 102372032 | 102373152 | 2,66 | -1,26 | 3,93 | 0,000208 | 0,00583 | Intron (uc002tbc.3/9448, intron 2 of 31) | -34030 | ENSG00000071054 | MAP4K4 |
| chr1 | 190322835 | 190323652 | 2,78 | -1,55 | 4,33 | 0,000208 | 0,00583 | Intron (uc010pot.1/339479, intron 2 of 6) | 121290 | ENSG00000162670 | BRINP3 |
| chr4 | 181481198 | 181482407 | 3,64 | -0,69 | 4,34 | 0,000208 | 0,00583 | Distal Intergenic | 597895 | ENSG00000248197 | LINC00290 |
| chr2 | 175912985 | 175914092 | 2,65 | -1,55 | 4,2 | 0,000209 | 0,00584 | Distal Intergenic | -42878 | ENSG00000128656 | CHN1 |
| chr2 | 238318887 | 238323623 | 7,75 | 1,76 | 5,99 | 0,000211 | 0,00588 | Promoter (<=1kb) | 0 | ENSG00000163359 | COL6A3 |
| chr16 | 73096707 | 73097502 | 2,68 | -1,55 | 4,23 | 0,000213 | 0,00591 | Distal Intergenic | -4173 | ENSG00000140836 | ZFHX3 |
| chr15 | 70778364 | 70779819 | 2,85 | -1,55 | 4,4 | 0,000213 | 0,00591 | Distal Intergenic | 214801 | ENSG00000137831 | UACA |
| chr1 | 210546713 | 210547872 | 2,87 | -1,55 | 4,42 | 0,000214 | 0,00592 | Intron (uc009xcx.3/55733, intron 3 of 11) | 30087 | ENSG00000054392 | HHAT |
| chr14 | 67182882 | 67183982 | 2,63 | -1,55 | 4,18 | 0,000215 | 0,00595 | Intron (uc001xiw.3/10243, intron 2 of 10) | -105981 | ENSG00000171723 | GPHN |
| chr7 | 76588065 | 76591363 | 3,64 | -0,51 | 4,15 | 0,000216 | 0,00596 | Distal Intergenic | -18776 | ENSG00000265479 | DTX2P1-UPK3BP1-PMS2P11 |
| chr11 | 115079682 | 115081236 | 3,03 | -1,55 | 4,59 | 0,000216 | 0,00598 | Exon (uc031qeh.1/23705, exon 9 of 11) | 204231 | ENSG00000182985 | CADM1 |
| chr10 | 8401841 | 8402899 | 2,82 | -1,26 | 4,08 | 0,000217 | 0,006 | Distal Intergenic | 305174 | ENSG00000107485 | GATA3 |
| chr19 | 30520425 | 30521794 | 3,84 | -0,04 | 3,88 | 0,000218 | 0,00601 | Distal Intergenic | 44324 | ENSG00000105176 | URI1 |
| chr7 | 106504627 | 106514849 | 9,64 | 7,26 | 2,38 | 0,000219 | 0,00604 | Promoter (<=1kb) | 0 | ENSG00000105851 | PIK3 CG |
| chr10 | 2990197 | 2991120 | 2,43 | -1,55 | 3,98 | 0,000219 | 0,00604 | Distal Intergenic | -118592 | ENSG00000067057 | PFKP |
| chr5 | 80926484 | 80927524 | 3,1 | -0,75 | 3,85 | 0,00022 | 0,00605 | Intron (uc003kho.4/23635, intron 3 of 16) | 119548 | ENSG00000145687 | SSBP2 |
| chr2 | 65597543 | 65598467 | 2,41 | -1,55 | 3,96 | 0,00022 | 0,00605 | Intron (uc002sdr.4/200734, intron 1 of 5) | -3612 | ENSG00000198369 | SPRED2 |
| chr4 | 153379712 | 153380740 | 2,44 | -1,55 | 3,99 | 0,00022 | 0,00606 | Intron (uc003ims.3/55294, intron 1 of 11) | 29828 | ENSG00000264678 | MIR3140 |
| chr12 | 19847129 | 19848869 | 3,1 | -1,55 | 4,65 | 0,000221 | 0,00607 | Distal Intergenic | 253614 | ENSG00000139154 | AEBP2 |
| chr5 | 7878901 | 7879499 | 2,35 | -1,55 | 3,9 | 0,000222 | 0,00609 | Intron (uc010itn.1/4552,intron 4 of 5) | 9684 | ENSG00000124275 | MTRR |
| chr7 | 11970805 | 11971839 | 3,07 | -1,55 | 4,62 | 0,000222 | 0,00609 | Distal Intergenic | -98981 | ENSG00000005108 | THSD7 A |
| chr16 | 12104243 | 12105412 | 3,14 | -1,55 | 4,7 | 0,000225 | 0,00613 | Intron (uc002dbw.3/92017, intron 3 of 7) | 33641 | ENSG00000048471 | SNX29 |
| chr1 | 73852759 | 73854328 | 3,23 | -1,55 | 4,78 | 0,000225 | 0,00613 | Distal Intergenic | 809543 | ENSG00000162620 | LRRIQ3 |
| chr17 | 75420895 | 75421610 | 2,61 | -1,55 | 4,16 | 0,000225 | 0,00614 | Intron (uc002jts.4/10801, intron 3 of 11) | 19736 | ENSG00000184640 | SEPTIN9 |
| chr7 | 50353806 | 50355477 | 2,81 | -1,55 | 4,36 | 0,000227 | 0,00617 | Intron (uc003tov.1/10320, intron 1 of 3) | 5489 | ENSG00000185811 | IKZF1 |
| chr1 | 74822767 | 74823926 | 3,81 | -0,65 | 4,46 | 0,000227 | 0,00617 | Intron (uc001dgc.2/100526835, intron 13 of 18) | 121696 | ENSG00000116783 | TNNI3K |
| chr3 | 165531310 | 165532637 | 3,16 | -1,55 | 4,71 | 0,000227 | 0,00617 | Intron (uc003fem.4/590, intron 2 of 3) | 22616 | ENSG00000114200 | BCHE |
| chr15 | 85985887 | 85986876 | 3,03 | -0,72 | 3,75 | 0,000228 | 0,00618 | Intron (uc002bls.4/11214, intron 1 of 3) | 62040 | ENSG00000170776 | AKAP13 |
| chr5 | 90561841 | 90562738 | 2,95 | -0,7 | 3,65 | 0,000229 | 0,0062 | Distal Intergenic | -113426 | ENSG00000281357 | ARRDC3-AS1 |
| chr4 | 96520825 | 96521906 | 3,6 | -0,23 | 3,83 | 0,000229 | 0,0062 | Distal Intergenic | -50464 | ENSG00000182168 | UNC5 C |
| chr7 | 145683198 | 145684132 | 3,44 | -0,47 | 3,91 | 0,000229 | 0,0062 | Distal Intergenic | -129321 | ENSG00000174469 | CNTNAP2 |
| chr14 | 82240524 | 82241320 | 2,61 | -1,55 | 4,16 | 0,000229 | 0,0062 | Distal Intergenic | -240319 | ENSG00000071537 | SEL1L |
| chr3 | 80973989 | 80974559 | 2,54 | -1,26 | 3,8 | 0,00023 | 0,00621 | Distal Intergenic | 818221 | ENSG00000114480 | GBE1 |
| chr9 | 35275792 | 35276856 | 2,53 | -1,55 | 4,09 | 0,00023 | 0,00622 | Intron (uc010mkl.1/10497, intron 7 of 12) | -76829 | ENSG00000198722 | UNC13B |
| chr13 | 65980597 | 65981399 | 2,77 | -1,55 | 4,33 | 0,000232 | 0,00626 | Distal Intergenic | -1417902 | ENSG00000228842 | PCDH9-AS2 |
| chr7 | 43742410 | 43743033 | 2,34 | -0,73 | 3,07 | 0,000233 | 0,00628 | Intron (uc003tij.4/55744, intron 1 of 8) | 26107 | ENSG00000106603 | COA1 |
| chr6 | 3333213 | 3334219 | 2,66 | -1,1 | 3,76 | 0,000233 | 0,00628 | Intron (uc003mvn.3/63027, intron 4 of 10) | 74051 | ENSG00000180822 | PSMG4 |
| chrX | 116922378 | 116923824 | 3,51 | -0,77 | 4,28 | 0,000233 | 0,00628 | Distal Intergenic | 158081 | ENSG00000003096 | KLHL13 |
| chr13 | 62611664 | 62612490 | 2,24 | -1,55 | 3,79 | 0,000235 | 0,0063 | Distal Intergenic | -609585 | ENSG00000280 165 | PCDH20 |
| chr2 | 166150443 | 166151887 | 5,02 | 0,74 | 4,28 | 0,000235 | 0,0063 | Promoter (<=1kb) | 102 | ENSG00000136531 | SCN2A |
| chr2 | 187684180 | 187685608 | 4,77 | 0,22 | 4,55 | 0,000235 | 0,0063 | Distal Intergenic | 28289 | ENSG00000163012 | ZSWIM2 |
| chr15 | 57078783 | 57079890 | 3,04 | -0,61 | 3,65 | 0,000236 | 0,00632 | Intron (uc002adw.1/54816, intron 6 of 23) | -52996 | ENSG00000137871 | ZNF280D |
| chr6 | 72153618 | 72154281 | 2,32 | -1,55 | 3,87 | 0,000236 | 0,00632 | Distal Intergenic | -23170 | ENSG00000233237 | LINC00472 |
| chr3 | 82128206 | 82128983 | 2,67 | -1,55 | 4,22 | 0,000236 | 0,00632 | Intron (uc003dqh.1/uc003dqh.1, intron 1 of 8) | -317256 | ENSG00000114480 | GBE1 |
| chr2 | 240217059 | 240217635 | 2,5 | -1,55 | 4,05 | 0,000236 | 0,00633 | Promoter (2-3kb) | 2699 | ENSG00000068024 | HDAC4 |
| chr2 | 173443503 | 173446046 | 5,12 | 0,3 | 4,82 | 0,000236 | 0,00633 | Intron (uc010zdz.1/5163, intron 9 of 11) | 22724 | ENSG00000152256 | PDK1 |
| chr8 | 10973699 | 10975847 | 3,76 | -0,65 | 4,41 | 0,000237 | 0,00635 | Intron (uc003wtk.1/286046, intron 1 of 2) | -80887 | ENSG00000207600 | MIR598 |
| chrX | 81346584 | 81347741 | 2,54 | -1,31 | 3,85 | 0,000239 | 0,00637 | Distal Intergenic | 888669 | ENSG00000131171 | SH3BGRL |
| chr11 | 99556670 | 99557898 | 4,24 | -0,01 | 4,25 | 0,000239 | 0,00637 | Intron (uc009ywv.2/53942, intron 3 of 15) | 129794 | ENSG00000149972 | CNTN5 |
| chr6 | 111622155 | 111624221 | 4,12 | -0,39 | 4,51 | 0,000239 | 0,00637 | Intron (uc003puw.4/5980, intron 2 of 2) | 7092 | ENSG00000009413 | REV3L |
| chr2 | 21332238 | 21333588 | 3,05 | -1,55 | 4,61 | 0,000239 | 0,00638 | Distal Intergenic | -65293 | ENSG00000084674 | APOB |
| chr11 | 3900443 | 3901398 | 2,52 | -1,4 | 3,92 | 0,000242 | 0,00644 | Intron (uc0011yv.2/6786, intron 1 of 11) | 23151 | ENSG00000284525 | MIR4687 |
| chr22 | 39824074 | 39825101 | 2,58 | -1,55 | 4,13 | 0,000243 | 0,00645 | Exon (uc003axr.3/10454, exon 11 of 12) | -3064 | NA | LOC100506472 |
| chr17 | 16637936 | 16640424 | 2,61 | -1,55 | 4,16 | 0,000243 | 0,00645 | Exon (uc002gqk.1/9720, exon 12 of 18) | 22943 | ENSG00000188933 | USP32P1 |
| chr2 | 121044872 | 121045479 | 2,54 | -1,55 | 4,09 | 0,000244 | 0,00647 | Intron (uc010vvs.2/5899, intron 3 of 4) | 34458 | ENSG00000144118 | RALB |
| chr15 | 60831879 | 60832928 | 2,66 | -1,55 | 4,21 | 0,000245 | 0,00649 | Intron (uc002agt.4/6095, intron 1 of 9) | 51779 | ENSG00000069667 | RORA |
| chr3 | 59429372 | 59430700 | 2,99 | -1,55 | 4,54 | 0,000245 | 0,00649 | Distal Intergenic | -393614 | ENSG00000163689 | C3orf67 |
| chr5 | 65121864 | 65122469 | 2,16 | -1,55 | 3,72 | 0,000246 | 0,0065 | 3'UTR | 40262 | ENSG00000123213 | NLN |
| chr3 | 188506116 | 188507074 | 2,57 | -1,55 | 4,12 | 0,000246 | 0,0065 | Intron (uc011bsp.2/4026, intron 7 of 9) | -157929 | ENSG00000188001 | TPRG1 |
| chr2 | 9802903 | 9804030 | 3,86 | 0,17 | 3,69 | 0,000249 | 0,00657 | Distal Intergenic | -31797 | ENSG00000134308 | YWHAQ |
| chr14 | 46182289 | 46183333 | 3,22 | -1,1 | 4,32 | 0,000249 | 0,00657 | Distal Intergenic | -350029 | ENSG00000258700 | LINC00871 |
| chr4 | 96527451 | 96528550 | 2,73 | -1,55 | 4,28 | 0,00025 | 0,00659 | Distal Intergenic | -57090 | ENSG00000182168 | UNC5 C |
| chr17 | 35790271 | 35791881 | 3,02 | -1,55 | 4,57 | 0,000251 | 0,00661 | Intron (uc002hnt.3/6871, intron 4 of 15) | 6662 | ENSG00000276234 | TADA2A |
| chr3 | 165534473 | 165535761 | 3,1 | -1,55 | 4,65 | 0,000252 | 0,00663 | Intron (uc003fem.4/590, intron 2 of 3) | 19492 | ENSG00000114200 | BCHE |
| chr12 | 11753913 | 11755634 | 3,05 | -1,55 | 4,6 | 0,000253 | 0,00664 | Distal Intergenic | -47154 | ENSG00000139083 | ETV6 |
| chr11 | 98281917 | 98283074 | 3,77 | -0,91 | 4,68 | 0,000253 | 0,00664 | Distal Intergenic | -608632 | ENSG00000149972 | CNTN5 |
| chr2 | 120332580 | 120333089 | 2,26 | -1,55 | 3,82 | 0,000257 | 0,00674 | Intron (uc010yyq.3/200373, intron 5 of 17) | 30572 | ENSG00000163075 | CFAP221 |
| chr13 | 65671632 | 65672589 | 2,89 | -1,55 | 4,44 | 0,000258 | 0,00676 | Distal Intergenic | 1360064 | NA | OR7E156P |
| chr14 | 102860777 | 102861832 | 2,95 | -1,55 | 4,5 | 0,000259 | 0,00676 | Intron (uc010txw.2/9895, intron 2 of 5) | 31477 | ENSG00000196663 | TECPR2 |
| chrX | 32384190 | 32385143 | 2,81 | -1,55 | 4,36 | 0,00026 | 0,00679 | Promoter (<=1kb) | -865 | ENSG00000198947 | DMD |
| chr2 | 188686204 | 188687298 | 2,93 | -1,55 | 4,49 | 0,00026 | 0,00679 | Distal Intergenic | -266985 | ENSG00000003436 | TFPI |
| chrX | 30330873 | 30332235 | 3,03 | -1,01 | 4,04 | 0,000262 | 0,00681 | Distal Intergenic | -3378 | ENSG00000169297 | NR0B1 |
| chr9 | 113940525 | 113941296 | 2,6 | -1,55 | 4,16 | 0,000262 | 0,00681 | Distal Intergenic | -138999 | ENSG00000198121 | LPAR1 |
| chr4 | 154125004 | 154126788 | 5,21 | 0,58 | 4,64 | 0,000262 | 0,00681 | Promoter (<=1kb) | 0 | ENSG00000109654 | TRIM2 |
| chr3 | 167599684 | 167600180 | 2,42 | -0,8 | 3,23 | 0,000263 | 0,00683 | Distal Intergenic | -13556 | ENSG00000244227 | LRRC77P |
| chr3 | 170290032 | 170290762 | 2,48 | -1,26 | 3,74 | 0,000263 | 0,00683 | Intron (uc011bpt.1/5010, intron 2 of 3) | 13101 | ENSG00000013293 | SLC7A14 |
| chr4 | 140235798 | 140237644 | 4,14 | -0,4 | 4,54 | 0,000264 | 0,00683 | Intron (uc003ihu.1/80155, intron 1 of 19) | -12093 | ENSG00000109390 | NDUFC1 |
| chr2 | 6121441 | 6122338 | 4,22 | 0,78 | 3,43 | 0,000265 | 0,00684 | Promoter (<=1kb) | 0 | NA | LOC400940 |
| chr11 | 115798354 | 115799362 | 2,46 | -1,55 | 4,01 | 0,000265 | 0,00684 | Distal Intergenic | -167009 | NA | LINC00900 |
| chr1 | 115822371 | 115823880 | 3,68 | -0,49 | 4,18 | 0,000265 | 0,00684 | Distal Intergenic | 56977 | ENSG00000134259 | NGF |
| chr6 | 4800695 | 4806348 | 5,92 | 1,47 | 4,45 | 0,000265 | 0,00684 | Intron (uc003mwi.3/9425, intron 3 of 8) | 24015 | ENSG00000153046 | CDYL |
| chr15 | 64216258 | 64217311 | 3,07 | -1,11 | 4,18 | 0,000266 | 0,00684 | Exon (uc002amr.3/23604, exon 9 of 12) | -90111 | ENSG00000103657 | HERC1 |
| chr19 | 23373536 | 23374939 | 3,57 | -0,65 | 4,22 | 0,000266 | 0,00684 | Distal Intergenic | 58233 | ENSG00000196081 | ZNF724 |
| chr10 | 8172906 | 8174353 | 3,89 | -0,21 | 4,1 | 0,000267 | 0,00687 | Distal Intergenic | 76239 | ENSG00000107485 | GATA3 |
| chr5 | 109604996 | 109605950 | 2,67 | -1,55 | 4,22 | 0,000269 | 0,0069 | Distal Intergenic | 159854 | ENSG00000186952 | TMEM2 3 2 |
| chr11 | 78042725 | 78043485 | 2,74 | -1,55 | 4,29 | 0,000269 | 0,0069 | Intron (uc001ozg.3/9846, intron 1 of 9) | 9441 | ENSG00000033327 | GAB2 |
| chr18 | 58039364 | 58040664 | 3,35 | -0,99 | 4,35 | 0,000269 | 0,0069 | Promoter (<=1kb) | 0 | ENSG00000166603 | MC4R |
| chr1 | 199003257 | 199003764 | 2,35 | -1,23 | 3,59 | 0,000271 | 0,00693 | Distal Intergenic | -96699 | NA | MIR181A1HG |
| chr5 | 118623048 | 118624069 | 3,28 | -0,02 | 3,3 | 0,000272 | 0,00696 | Intron (uc003ksf.1/25816, intron 1 of 2) | 18630 | ENSG00000145779 | TNFAIP8 |
| chr6 | 135408311 | 135409595 | 2,76 | -1,55 | 4,31 | 0,000274 | 0,007 | Distal Intergenic | -32275 | ENSG00000112339 | HBS1L |
| chr15 | 64207562 | 64208786 | 2,84 | -1,55 | 4,4 | 0,000274 | 0,007 | Intron (uc002amr.3/23604, intron 9 of 11) | -81415 | ENSG00000103657 | HERC1 |
| chr7 | 105152638 | 105153796 | 3,44 | -1,1 | 4,53 | 0,000275 | 0,00701 | Intron (uc003vcx.3/54517, intron 1 of 15) | -3671 | ENSG00000091127 | PUS7 |
| chr7 | 123037876 | 123038608 | 2,46 | -1,55 | 4,01 | 0,000275 | 0,00702 | Distal Intergenic | 63062 | ENSG00000164675 | IQUB |
| chr1 | 152115182 | 152116431 | 2,89 | -1,55 | 4,44 | 0,000276 | 0,00703 | Promoter (1-2kb) | 1275 | ENSG00000159450 | TCHH |
| chr16 | 74614101 | 74615648 | 3,89 | -0,24 | 4,12 | 0,000277 | 0,00705 | Intron (uc002fcw.4/2734, intron 1 of 25) | 25394 | ENSG00000090863 | GLG1 |
| chr19 | 19282467 | 19283336 | 2,75 | -1,55 | 4,3 | 0,000278 | 0,00706 | Promoter (1-2kb) | -1369 | ENSG00000213999 | MEF2B |
| chr4 | 87318991 | 87320002 | 2,54 | -1,1 | 3,64 | 0,000279 | 0,00709 | Intron (uc003hpt.3/5602, intron 1 of 13) | -37616 | ENSG00000109339 | MAPK10 |
| chr15 | 43207143 | 43208127 | 3,51 | -0,55 | 4,06 | 0,000281 | 0,00714 | Intron (uc001zqo.2/146057, intron 1 of 14) | 4880 | ENSG00000128881 | TTBK2 |
| chr12 | 102009391 | 102010243 | 2,36 | -1,55 | 3,91 | 0,000282 | 0,00715 | Intron (uc001tif.2/4604, intron 2 of 14) | 20682 | ENSG00000196091 | MYBPC1 |
| chr10 | 63723616 | 63724570 | 2,94 | -1,55 | 4,49 | 0,000283 | 0,00716 | Intron (uc010qil.2/84159, intron 3 of 4) | -11491 | ENSG00000263750 | MIR548AV |
| chr6 | 54546009 | 54546817 | 2,79 | -1,23 | 4,03 | 0,000283 | 0,00717 | Distal Intergenic | -164752 | ENSG00000168143 | FAM83B |
| chr5 | 119438764 | 119439948 | 4,42 | 0,1 | 4,32 | 0,000283 | 0,00717 | Distal Intergenic | -360071 | ENSG00000184838 | PRR16 |
| chr21 | 27567038 | 27567824 | 2,52 | -1,55 | 4,07 | 0,000284 | 0,00719 | Distal Intergenic | -23592 | ENSG00000142192 | APP |
| chr7 | 17450904 | 17452527 | 3,5 | -0,93 | 4,42 | 0,000285 | 0,0072 | Distal Intergenic | 112628 | ENSG00000106546 | AHR |
| chr10 | 134148030 | 134148867 | 3,5 | -1,06 | 4,56 | 0,000285 | 0,0072 | Promoter (<=1kb) | 0 | ENSG00000148814 | LRRC27 |
| chr8 | 93021166 | 93023806 | 4,33 | 0,09 | 4,24 | 0,000286 | 0,00723 | 3'UTR | 6102 | ENSG00000079102 | RUNX1T1 |
| chr6 | 45864392 | 45865617 | 3,65 | -0,46 | 4,11 | 0,000287 | 0,00723 | Downstream (<1kb) | 39717 | ENSG00000112782 | CLIC5 |
| chr8 | 117132492 | 117134137 | 3,66 | -0,62 | 4,29 | 0,000287 | 0,00723 | Intron (uc031tcc.1/100859921, intron 6 of 13) | 203160 | ENSG00000249917 | LINC00536 |
| chr8 | 77897710 | 77898707 | 2,85 | -1,55 | 4,41 | 0,000287 | 0,00723 | 5'UTR | 13817 | ENSG00000164751 | PEX2 |
| chr1 | 16275867 | 16277219 | 2,92 | -1,55 | 4,48 | 0,000288 | 0,00724 | Intron (uc001axl.4/7709, intron 2 of 15) | 25408 | ENSG00000116809 | ZBTB17 |
| chr11 | 114058283 | 114058592 | 1,67 | -1,31 | 2,98 | 0,000288 | 0,00725 | 3'UTR | -69961 | ENSG00000166741 | NNMT |
| chr10 | 28700033 | 28700800 | 2,46 | -1,55 | 4,02 | 0,000292 | 0,00732 | Distal Intergenic | -108038 | ENSG00000150054 | MPP7 |
| chr17 | 28024446 | 28025880 | 3,01 | -1,11 | 4,11 | 0,000294 | 0,00736 | Intron (uc002heo.1/85464, intron 3 of 14) | 62528 | ENSG00000141298 | SSH2 |
| chr3 | 153895677 | 153897230 | 2,95 | -1,55 | 4,5 | 0,000295 | 0,00738 | Intron (uc021xgc.1/26084, intron 6 of 14) | -46344 | ENSG00000114790 | ARHGEF26 |
| chr11 | 39220633 | 39221818 | 3,04 | -1,55 | 4,59 | 0,000295 | 0,00738 | Distal Intergenic | 1092862 | ENSG00000148948 | LRRC4C |
| chr11 | 117149752 | 117150816 | 2,77 | -1,31 | 4,08 | 0,000296 | 0,00739 | Exon (uc001pqt.4/257160, exon 7 of 15) | -11246 | NA | BACE1-AS |
| chr11 | 38779828 | 38781138 | 2,87 | -1,55 | 4,42 | 0,000296 | 0,00739 | Distal Intergenic | 1533542 | ENSG00000148948 | LRRC4C |
| chr4 | 166992177 | 166993391 | 2,93 | -1,55 | 4,49 | 0,000296 | 0,00739 | Intron (uc003irh.2/7092, intron 16 of 20) | 197767 | ENSG00000038295 | TLL1 |
| chr8 | 80931792 | 80933418 | 4,01 | 0,05 | 3,96 | 0,000297 | 0,00741 | Intron (uc003ybp.3/28957, intron 1 of 2) | 9088 | ENSG00000147586 | MRPS28 |
| chr4 | 90756412 | 90759780 | 6,51 | 1,67 | 4,84 | 0,000297 | 0,00741 | Promoter (<=1kb) | 0 | ENSG00000247775 | SNCA-AS1 |
| chr8 | 125380306 | 125381165 | 3,27 | -0,07 | 3,34 | 0,000299 | 0,00743 | Intron (uc010mdl.3/157378, intron 1 of 6) | 3775 | ENSG00000164983 | TMEM65 |
| chr13 | 77893681 | 77894648 | 2,71 | -1,55 | 4,26 | 0,000299 | 0,00743 | Intron (uc010aev.3/23077, intron 1 of 82) | 6529 | ENSG00000005810 | MYCBP2 |
| chr8 | 42034444 | 42036088 | 3,66 | -0,65 | 4,32 | 0,000299 | 0,00743 | Intron (uc0101xf.1/5327, intron 10 of 10) | 14641 | ENSG00000104368 | PLAT |
| chr2 | 215601342 | 215602715 | 2,64 | -1,55 | 4,19 | 0,000305 | 0,00755 | Intron (uc002veu.2/580, intron 9 of 10) | 71713 | ENSG00000138376 | BARD1 |
| chr3 | 175614185 | 175614864 | 2,16 | -1,55 | 3,71 | 0,000305 | 0,00756 | Distal Intergenic | 526856 | ENSG00000264974 | MIR4789 |
| chr6 | 109059084 | 109060550 | 4,53 | 0,45 | 4,08 | 0,000305 | 0,00756 | Distal Intergenic | -12307 | ENSG00000203801 | LINC00222 |
| chr20 | 61607222 | 61610002 | 7,58 | 4,31 | 3,27 | 0,000306 | 0,00756 | Distal Intergenic | 22746 | ENSG00000101194 | SLC17A9 |
| chr5 | 76255498 | 76256295 | 2,78 | -0,78 | 3,56 | 0,000306 | 0,00756 | Intron (uc003ker.3/1393,intron 5 of 6) | 6818 | ENSG00000145708 | CRHBP |
| chr8 | 117313369 | 117314576 | 2,96 | -1,55 | 4,52 | 0,000307 | 0,00758 | Intron (uc031tcc.1/100859921, intron 1 of 13) | 22721 | ENSG00000249917 | LINC00536 |
| chr2 | 17610405 | 17611201 | 2,33 | -1,01 | 3,34 | 0,000308 | 0,00759 | Distal Intergenic | 88505 | ENSG00000214842 | RAD51AP2 |
| chr20 | 18730692 | 18732492 | 3,97 | 0,28 | 3,69 | 0,00031 | 0,00765 | Intron (uc002wrf.4/92675, intron 5 of 5) | 42134 | NA | LINC00652 |
| chr11 | 76587274 | 76588204 | 2,42 | -1,55 | 3,97 | 0,000311 | 0,00765 | Intron (uc010rsg.1/55331, intron 2 of 8) | 15291 | ENSG00000078124 | ACER3 |
| chr6 | 97471648 | 97472475 | 2,31 | -1,55 | 3,86 | 0,000315 | 0,00773 | Intron (uc003poy.3/114792, intron 3 of 6) | 13572 | ENSG00000186231 | KLHL32 |
| chr5 | 140186369 | 140187416 | 4,56 | -0,2 | 4,76 | 0,000315 | 0,00773 | Promoter (<=1kb) | 0 | ENSG00000204967 | PCDHA4 |
| chr4 | 168648436 | 168649086 | 2,81 | -1,4 | 4,21 | 0,000315 | 0,00774 | Distal Intergenic | -364602 | ENSG00000109511 | ANXA10 |
| chr7 | 17342053 | 17343160 | 3,19 | -0,93 | 4,12 | 0,000316 | 0,00775 | Intron (uc011jxz.1/196, intron 1 of 10) | 3777 | ENSG00000106546 | AHR |
| chr21 | 21989274 | 21991969 | 4,84 | 0,65 | 4,18 | 0,000316 | 0,00775 | Distal Intergenic | 183457 | ENSG00000224924 | LINC00320 |
| chr2 | 11672067 | 11673081 | 2,93 | -1,55 | 4,48 | 0,000316 | 0,00775 | Promoter (1-2kb) | -1161 | ENSG00000196208 | GREB1 |
| chr15 | 60843157 | 60844074 | 2,19 | -1,55 | 3,74 | 0,000317 | 0,00776 | Intron (uc002agt.4/6095, intron 1 of 9) | 40633 | ENSG00000069667 | RORA |
| chr9 | 24539321 | 24540421 | 2,82 | -1,55 | 4,38 | 0,000317 | 0,00776 | Downstream (2-3kb) | 5253 | ENSG00000205442 | IZUMO3 |
| chr4 | 148638702 | 148639871 | 2,47 | -1,55 | 4,03 | 0,000319 | 0,00779 | Distal Intergenic | -13582 | ENSG00000071205 | ARHGAP10 |
| chr8 | 134202893 | 134205675 | 4,59 | -0,2 | 4,78 | 0,000319 | 0,0078 | Promoter (<=1kb) | 0 | ENSG00000104415 | CCN4 |
| chr4 | 82326705 | 82327403 | 2,51 | -1,14 | 3,64 | 0,000322 | 0,00785 | Distal Intergenic | 65658 | ENSG00000138670 | RASGEF1B |
| chr1 | 78988176 | 78988727 | 2,37 | -1,38 | 3,76 | 0,000323 | 0,00787 | Intron (uc001dim.3/5737, intron 3 of 3) | 31448 | ENSG00000122420 | PTGFR |
| chr4 | 157724420 | 157725290 | 2,41 | -1,55 | 3,97 | 0,000323 | 0,00787 | Intron (uc011cir.3/56034, intron 3 of 7) | 46278 | ENSG00000145431 | PDGFC |
| chr13 | 99890346 | 99891211 | 2,74 | -1,55 | 4,29 | 0,000326 | 0,00791 | 5'UTR | 19629 | ENSG00000125245 | GPR18 |
| chr17 | 38754368 | 38755749 | 4,09 | 0,38 | 3,71 | 0,00033 | 0,008 | Distal Intergenic | -32632 | ENSG00000126353 | CCR7 |
| chr5 | 20708126 | 20709165 | 3,43 | -0,44 | 3,87 | 0,000331 | 0,008 | Intron (uc003jge.1/uc003jge.1, intron 3 of 11) | -132144 | ENSG00000145526 | CDH18 |
| chr14 | 93556036 | 93557017 | 2,67 | -1,55 | 4,22 | 0,000331 | 0,008 | Intron (uc001ybe.2/3705, intron 2 of 10) | 22239 | ENSG00000258730 | ITPK1-AS 1 |
| chr4 | 22073598 | 22074120 | 2,38 | -1,55 | 3,93 | 0,000331 | 0,00801 | Distal Intergenic | -123224 | ENSG00000185774 | KCNIP4 |
| chr1 | 172113864 | 172115047 | 2,89 | -1,55 | 4,45 | 0,000333 | 0,00805 | Promoter (<=1kb) | 0 | NA | DNM3OS |
| chr6 | 136172455 | 136173433 | 2,65 | -1,38 | 4,03 | 0,000335 | 0,00807 | Promoter (<=1kb) | 0 | ENSG00000171408 | PDE7B |
| chr5 | 171387930 | 171389147 | 3,32 | -0,86 | 4,17 | 0,000335 | 0,00807 | Intron (uc011dey.1/23291, intron 1 of 12) | 34844 | ENSG00000072803 | FBXW11 |
| chr5 | 502161 | 503185 | 2,31 | -1,55 | 3,87 | 0,000336 | 0,00808 | Intron (uc003jbe.2/6550, intron 1 of 16) | 21364 | ENSG00000066230 | SLC9A3 |
| chr12 | 12911464 | 12912493 | 2,8 | -1,55 | 4,35 | 0,000336 | 0,00808 | Intron (uc001rau.4/81575, intron 1 of 1) | -5090 | ENSG00000207983 | MIR613 |
| chr8 | 74037002 | 74037716 | 2,4 | -1,38 | 3,78 | 0,000336 | 0,00809 | Distal Intergenic | -31495 | ENSG00000164764 | SBSPON |
| chr6 | 68028625 | 68029662 | 2,98 | -1,06 | 4,04 | 0,000338 | 0,00811 | Distal Intergenic | -1315970 | ENSG00000135298 | ADGRB3 |
| chr11 | 120039360 | 120040525 | 4,96 | 0,72 | 4,24 | 0,000339 | 0,00813 | Distal Intergenic | -30497 | ENSG00000137699 | TRIM29 |
| chr9 | 11927275 | 11928306 | 3,02 | -1,11 | 4,13 | 0,00034 | 0,00814 | Distal Intergenic | -765080 | ENSG00000107165 | TYRP1 |
| chr18 | 29968905 | 29969971 | 2,6 | -1,55 | 4,16 | 0,000341 | 0,00814 | Intron (uc002kxk.2/64762, intron 2 of 5) | 80476 | ENSG00000141441 | GAREM1 |
| chr1 | 238607834 | 238608855 | 2,87 | -1,55 | 4,42 | 0,000341 | 0,00814 | Distal Intergenic | 40462 | ENSG00000215808 | LINC01139 |
| chr4 | 56471316 | 56472088 | 2,93 | -0,97 | 3,9 | 0,000342 | 0,00816 | Exon (uc003hbc.3/10874, exon 7 of 10) | -12937 | ENSG00000163440 | PDCL2 |
| chr1 1 | 40880326 | 40881500 | 2,92 | -1,11 | 4,03 | 0,000342 | 0,00816 | Intron (uc031pzt.1/57689, intron 2 of 6) | -565646 | ENSG00000148948 | LRRC4C |
| chr22 | 40927810 | 40930745 | 5,27 | 0,92 | 4,35 | 0,000343 | 0,00818 | Intron (uc003ayw.1/57591, intron 3 of 14) | -68372 | ENSG00000196588 | MRTFA |
| chr19 | 35730832 | 35731912 | 2,68 | -1,55 | 4,23 | 0,000343 | 0,00819 | Distal Intergenic | -7647 | ENSG00000105699 | LSR |
| chr8 | 9687792 | 9688582 | 2,8 | -0,97 | 3,77 | 0,000345 | 0,00821 | Distal Intergenic | 72257 | ENSG00000253230 | LINC00599 |
| chr9 | 79908758 | 79909721 | 2,76 | -1,29 | 4,05 | 0,000346 | 0,00824 | Promoter (<=1kb) | 501 | ENSG00000197969 | VPS13A |
| chr8 | 77712186 | 77712978 | 2,84 | -1,55 | 4,39 | 0,000351 | 0,00832 | Intron (uc003vau.2/79776, intron 4 of 10) | 95908 | ENSG00000091656 | ZFHX4 |
| chr9 | 71191953 | 71193238 | 2,58 | -1,55 | 4,13 | 0,000351 | 0,00833 | Distal Intergenic | -36170 | ENSG00000 181778 | TMEM252 |
| chr10 | 126302669 | 126303074 | 2,04 | -1,55 | 3,59 | 0,000352 | 0,00835 | 3'UTR | 8899 | ENSG00000107902 | LHPP |
| chr4 | 30476597 | 30477713 | 4,06 | -0,08 | 4,14 | 0,000353 | 0,00836 | Distal Intergenic | -244317 | ENSG00000169851 | PCDH7 |
| chr5 | 32276251 | 32276978 | 2,21 | -1,55 | 3,76 | 0,000353 | 0,00837 | Exon (uc003jhq.3/54545, exon 2 of 16) | 36136 | ENSG00000 150712 | MTMR12 |
| chr9 | 101881017 | 101881932 | 2,8 | -1,01 | 3,81 | 0,000353 | 0,00837 | Intron (uc004azc.3/7046, intron 1 of 8) | -8266 | ENSG00000106799 | TGFBR1 |
| chr15 | 76796120 | 76797334 | 2,74 | -1,55 | 4,3 | 0,000357 | 0,00843 | Exon (uc010bkr.3/49855, exon 8 of 15) | -149128 | ENSG00000140386 | SCAPER |
| chr1 | 165611126 | 165612117 | 3,95 | 0,19 | 3,76 | 0,000357 | 0,00844 | Intron (uc001gdf.3/4259, intron 1 of 5) | 11016 | ENSG00000143198 | MGST3 |
| chr22 | 43285522 | 43286662 | 2,57 | -1,55 | 4,12 | 0,000358 | 0,00844 | Intron (uc010gzg.3/11252, intron 4 of 10) | -32114 | ENSG00000242247 | ARFGAP3 |
| chr4 | 119746138 | 119748491 | 4,6 | 0,81 | 3,79 | 0,000358 | 0,00845 | Exon (uc003icl.2/9871, exon 3 of 23) | 8835 | ENSG00000150961 | SEC24D |
| chr4 | 175458769 | 175460213 | 4,2 | 0,57 | 3,63 | 0,00036 | 0,00847 | Distal Intergenic | -14720 | ENSG00000164120 | HPGD |
| chr15 | 100710704 | 100711460 | 2,3 | -1,55 | 3,86 | 0,00036 | 0,00847 | Intron (uc002bvv.1/170691, intron 8 of 21) | 167845 | ENSG00000140470 | ADAMTS17 |
| chr10 | 22670234 | 22671299 | 2,76 | -1,55 | 4,31 | 0,000362 | 0,0085 | Intron (uc001iri.3/9576, intron 4 of 10) | -4384 | ENSG00000077327 | SPAG6 |
| chr8 | 80869909 | 80870974 | 3,98 | 0,25 | 3,74 | 0,000363 | 0,00852 | Exon (uc0101zr.3/7163, exon 7 of 8) | 71532 | ENSG00000147586 | MRPS28 |
| chr3 | 191289765 | 191290328 | 2,31 | -1,55 | 3,87 | 0,000363 | 0,00852 | Distal Intergenic | 110813 | ENSG00000253548 | PYDC2 |
| chr4 | 32010144 | 32010912 | 2,57 | -1,55 | 4,12 | 0,000364 | 0,00853 | Distal Intergenic | 1288107 | ENSG00000169851 | PCDH7 |
| chr7 | 40672521 | 40673498 | 2,6 | -1,55 | 4,15 | 0,000364 | 0,00853 | Intron (uc003thn.2/79783, intron 12 of 13) | -159558 | ENSG00000175600 | SUGCT |
| chr3 | 176775382 | 176776499 | 2,43 | -1,55 | 3,98 | 0,000365 | 0,00854 | Intron (uc003fiw.4/79718, intron 3 of 15) | 137767 | ENSG00000177565 | TBL1XR1 |
| chr2 | 232922727 | 232923676 | 3,55 | -0,26 | 3,81 | 0,000367 | 0,00858 | Intron (uc002vsm.4/129563, intron 5 of 20) | 33740 | ENSG00000144535 | DIS3L2 |
| chrX | 147996678 | 147997296 | 2,37 | -1,55 | 3,92 | 0,000367 | 0,00858 | Intron (uc004fcp.3/2334, intron 9 of 20) | 196063 | ENSG00000155966 | AFF2 |
| chrX | 45636347 | 45638961 | 3,85 | -0,06 | 3,92 | 0,000368 | 0,00859 | Distal Intergenic | -29817 | ENSG00000207725 | MIR222 |
| chr8 | 61841420 | 61842496 | 2,7 | -1,55 | 4,25 | 0,000368 | 0,00859 | Distal Intergenic | 37811 | NA | LOC100130298 |
| chr2 | 6078335 | 6078856 | 2,22 | -1,55 | 3,77 | 0,00037 | 0,00862 | Intron (uc002qvk.2/150622, intron 1 of 1) | 5516 | NA | SILC1 |
| chr15 | 23061916 | 23063220 | 2,94 | -1,55 | 4,49 | 0,00037 | 0,00862 | 5'UTR | 23216 | ENSG00000170113 | NIPA1 |
| chr16 | 73097876 | 73100873 | 4,98 | 0,76 | 4,22 | 0,000371 | 0,00863 | Distal Intergenic | -5342 | ENSG00000140836 | ZFHX3 |
| chr19 | 43045813 | 43046774 | 2,75 | -1,55 | 4,31 | 0,000371 | 0,00864 | Intron (uc010eif.2/100996307, intron 2 of 2) | -13152 | ENSG00000079385 | CEACAM1 |
| chr13 | 21900012 | 21901106 | 4,51 | -0,21 | 4,72 | 0,000371 | 0,00864 | Intron (uc001unz.2/650794, intron 2 of 2) | 27748 | NA | MIPEPP3 |
| chr4 | 23912657 | 23913836 | 2,4 | -1,38 | 3,78 | 0,000374 | 0,00869 | Intron (uc031sdx.1/10891, intron 2 of 14) | -20957 | ENSG00000109819 | PPARGC1A |
| chr2 | 12646535 | 12648021 | 3,26 | -0,4 | 3,66 | 0,000378 | 0,00878 | Intron (uc002rbu.1/uc002rbu.1, intron 4 of 5) | -208977 | ENSG00000071575 | TRIB2 |
| chr6 | 31333109 | 31333882 | 2,4 | -1,55 | 3,95 | 0,000379 | 0,0088 | Distal Intergenic | -8120 | ENSG0000023 4745 | HLA-B |
| chr2 | 139276701 | 139283086 | 8,19 | 4,24 | 3,95 | 0,00038 | 0,00881 | Intron (uc002tvh.3/339745, intron 1 of 10) | 17351 | ENSG00000144228 | SPOPL |
| chr5 | 43304517 | 43305620 | 2,58 | -1,38 | 3,97 | 0,000381 | 0,00883 | Intron (uc003jnq.5/3157, intron 1 of 9) | 7994 | ENSG00000112972 | HMGCS1 |
| chr7 | 25919157 | 25920039 | 2,49 | -1,55 | 4,04 | 0,000382 | 0,00883 | Distal Intergenic | -38527 | NA | RNU6-16P |
| chr1 | 222906083 | 222906916 | 2,58 | -0,95 | 3,53 | 0,000384 | 0,00887 | 3'UTR | -3642 | ENSG00000197520 | FAM177B |
| chr11 | 107831863 | 107832830 | 2,61 | -1,55 | 4,16 | 0,000385 | 0,00888 | Exon (uc001pjt.3/54734, exon 2 of 2) | 32586 | ENSG00000179331 | RAB39A |
| chr2 | 238338175 | 238338873 | 2,42 | -1,55 | 3,97 | 0,000386 | 0,00889 | Distal Intergenic | -15325 | ENSG00000163359 | COL6A3 |
| chr11 | 39212969 | 39214700 | 3,73 | -0,46 | 4,18 | 0,000386 | 0,00889 | Distal Intergenic | 1099980 | ENSG00000148948 | LRRC4C |
| chr4 | 22876792 | 22877570 | 2,65 | -1,55 | 4,2 | 0,000386 | 0,00889 | Distal Intergenic | 161080 | ENSG00000249948 | GBA3 |
| chr11 | 120906025 | 120913326 | 7,59 | 2,42 | 5,17 | 0,000387 | 0,0089 | Exon (uc001pxq.3/219899, exon 2 of 8) | 11212 | ENSG00000154114 | TBCEL |
| chr14 | 74220270 | 74222904 | 5,52 | 2,19 | 3,33 | 0,000388 | 0,00891 | Promoter (2-3kb) | -2546 | ENSG00000264741 | MIR4505 |
| chr2 | 191646031 | 191646545 | 2,22 | -1,55 | 3,77 | 0,000389 | 0,00893 | Distal Intergenic | -99002 | ENSG00000115419 | GLS |
| chr3 | 58026747 | 58027460 | 2,2 | -1,55 | 3,75 | 0,00039 | 0,00894 | Intron (uc003djj.2/2317, intron 1 of 45) | 32620 | ENSG00000136068 | FLNB |
| chr1 | 203970182 | 203970776 | 2,38 | -1,55 | 3,94 | 0,00039 | 0,00894 | Distal Intergenic | 39616 | ENSG00000176754 | LINC00303 |
| chr13 | 106996159 | 106997237 | 3,95 | 0,42 | 3,53 | 0,000391 | 0,00898 | Distal Intergenic | -31674 | NA | LINC00460 |
| chr4 | 34271793 | 34272869 | 2,61 | -1,55 | 4,16 | 0,000392 | 0,00898 | Distal Intergenic | 1802588 | ENSG0000004 7365 | ARAP2 |
| chr1 | 191285492 | 191286717 | 2,84 | -1,55 | 4,39 | 0,000392 | 0,00898 | Distal Intergenic | 691472 | ENSG00000231175 | LINC01720 |
| chr6 | 134299493 | 134300510 | 2,58 | -1,55 | 4,13 | 0,000394 | 0,00902 | Intron (uc010kgg.3/9519, intron 1 of 6) | 25192 | ENSG00000028839 | TBPL1 |
| chr3 | 145286908 | 145287831 | 2,6 | -1,55 | 4,15 | 0,000394 | 0,00903 | Distal Intergenic | 516691 | ENSG00000152952 | PLOD2 |
| chr3 | 129312102 | 129312956 | 2,44 | -1,55 | 3,99 | 0,000395 | 0,00903 | Intron (uc003emx.2/23129, intron 1 of 35) | 12626 | ENSG00000004399 | PLXND1 |
| chr14 | 80522192 | 80522721 | 2,52 | -1,55 | 4,07 | 0,000395 | 0,00904 | Distal Intergenic | -155041 | ENSG00000258766 | DIO2-AS1 |
| chr7 | 38055744 | 38056380 | 2,4 | -1,55 | 3,95 | 0,000396 | 0,00905 | Distal Intergenic | 94823 | ENSGO0000086289 | EPDR1 |
| chr15 | 76906739 | 76907596 | 2,42 | -1,55 | 3,98 | 0,000397 | 0,00906 | Intron (uc010bkr.3/49855, intron 6 of 14) | 113482 | ENSG00000140386 | SCAPER |
| chr3 | 33487688 | 33490455 | 5,76 | 0,86 | 4,9 | 0,000397 | 0,00907 | Distal Intergenic | -5791 | ENSG00000153560 | UBP1 |
| chr5 | 144537546 | 144538659 | 3,77 | -0,56 | 4,33 | 0,000401 | 0,00915 | Distal Intergenic | 676240 | ENSG00000186314 | PRELID2 |
| chr1 | 183839860 | 183840731 | 2,6 | -1,55 | 4,15 | 0,000402 | 0,00917 | Intron (uc010pof.1/23179, intron 2 of 11) | 65585 | ENSG00000143344 | RGL1 |
| chr18 | 48802171 | 48803072 | 2,61 | -1,55 | 4,17 | 0,000403 | 0,00918 | Distal Intergenic | -78120 | ENSG00000176624 | MEX3C |
| chr18 | 47390010 | 47390750 | 2,55 | -1,55 | 4,11 | 0,000404 | 0,0092 | Exon (uc002lea.2/4645, exon 8 of 19) | -13833 | ENSG00000167306 | MYO5B |
| chr17 | 18481214 | 18484100 | 3,08 | -0,48 | 3,56 | 0,000405 | 0,00921 | Exon (uc002gua.4/284047, exon 12 of 17) | -35980 | ENSG00000233327 | USP32P2 |
| chr3 | 180996221 | 180996775 | 2,69 | -0,97 | 3,66 | 0,000407 | 0,00924 | Intron (uc003fkv.4/347689, intron 3 of 7) | 103289 | NA | SOX2-OT |
| chr1 | 178069271 | 178070630 | 2,63 | -1,55 | 4,19 | 0,000407 | 0,00924 | Intron (uc009wxb.2/9462, intron 1 of 4) | -6143 | ENSG00000224687 | RASAL2-AS1 |
| chr3 | 165387902 | 165388993 | 2,89 | -1,55 | 4,44 | 0,000407 | 0,00924 | Distal Intergenic | 166260 | ENSG00000114200 | BCHE |
| chr2 | 199236321 | 199237215 | 3 | -0,52 | 3,52 | 0,000408 | 0,00924 | Intron (uc002uux.1/uc002uux.1, intron 1 of 1) | 566895 | ENSG00000115896 | PLCL1 |
| chr18 | 28408466 | 28409238 | 2,52 | -1,55 | 4,08 | 0,000411 | 0,00929 | Distal Intergenic | 213543 | ENSG00000134762 | DSC3 |
| chr12 | 122651048 | 122651951 | 2,72 | -1,55 | 4,28 | 0,000411 | 0,00929 | Promoter (<=1kb) | -315 | ENSG00000158113 | LRRC43 |
| chr12 | 2496762 | 2497326 | 2,43 | -1,55 | 3,98 | 0,000414 | 0,00934 | Intron (uc001qjz.2/775, intron 3 of 46) | -116276 | ENSG00000151067 | CACNA1C |
| chr11 | 77943036 | 77944034 | 2,54 | -1,55 | 4,09 | 0,000414 | 0,00934 | Intron (uc001ozg.3/9846, intron 3 of 9) | 33020 | ENSG00000118369 | USP35 |
| chr14 | 88635069 | 88635597 | 2,23 | -1,55 | 3,78 | 0,000415 | 0,00934 | Distal Intergenic | 101658 | ENSG00000100433 | KCNK10 |
| chr4 | 48501131 | 48501796 | 2,47 | -1,14 | 3,61 | 0,000421 | 0,00946 | 3'UTR | 6833 | ENSG00000075539 | FRYL |
| chr20 | 61089427 | 61090302 | 2,61 | -1,55 | 4,16 | 0,000422 | 0,00947 | Distal Intergenic | -38401 | ENSG00000130700 | GATA5 |
| chr2 | 21825698 | 21826292 | 1,87 | -1,55 | 3,42 | 0,000423 | 0,00949 | Distal Intergenic | 107232 | ENSGO0000229621 | LINC01822 |
| chr1 | 75322978 | 75324538 | 3,05 | -1,01 | 4,05 | 0,000423 | 0,00949 | Distal Intergenic | -123886 | ENSG00000116791 | CRYZ |
| chr3 | 85398208 | 85399179 | 3,2 | -0,8 | 4 | 0,000424 | 0,0095 | Intron (uc003dqj.3/253559, intron 1 of 9) | -35681 | ENSG00000264084 | MIR5688 |
| chr1 | 246527566 | 246528349 | 2,38 | -1,38 | 3,77 | 0,000427 | 0,00954 | Intron (uc001ibk.3/64754, intron 1 of 11) | 52365 | ENSG00000185420 | SMYD3 |
| chr18 | 58136644 | 58137636 | 2,89 | -0,87 | 3,76 | 0,000429 | 0,00959 | Distal Intergenic | -96643 | ENSG00000166603 | MC4R |
| chr6 | 131099819 | 131100774 | 2,72 | -1,55 | 4,28 | 0,000432 | 0,00964 | Distal Intergenic | -47771 | ENSG00000256162 | SMLR1 |
| chr2 | 208368853 | 208370649 | 3,96 | -0,56 | 4,52 | 0,000432 | 0,00964 | Distal Intergenic | -23967 | ENSG00000 118260 | CREB1 |
| chr14 | 100095041 | 100095350 | 1,88 | -1,38 | 3,27 | 0,000434 | 0,00969 | Distal Intergenic | -16130 | ENSG00000 182218 | HHIPL1 |
| chr17 | 68234756 | 68235711 | 2,8 | -1,55 | 4,35 | 0,000435 | 0,0097 | Distal Intergenic | 69080 | ENSG00000123700 | KCNJ2 |
| chr19 | 48085530 | 48086569 | 2,88 | -0,86 | 3,74 | 0,000437 | 0,00974 | Distal Intergenic | -24884 | ENSG00000063169 | BICRA |
| chr4 | 97742056 | 97742819 | 2,6 | -1,55 | 4,16 | 0,000438 | 0,00976 | Distal Intergenic | -545258 | ENSG00000251620 | STPG2-AS1 |
| chr2 | 12643990 | 12644739 | 2,4 | -1,55 | 3,96 | 0,000439 | 0,00977 | Intron (uc002rbu.1/uc002rbu.1, intron 4 of 5) | -212259 | ENSG00000071575 | TRIB2 |
| chr9 | 136727865 | 136728484 | 2,29 | -1,55 | 3,84 | 0,000441 | 0,0098 | Intron (uc004cer.3/7410,intron2 of 26) | -72516 | ENSG00000160293 | VAV2 |
| chr7 | 124129821 | 124130930 | 3,53 | -0,46 | 3,98 | 0,000441 | 0,0098 | Distal Intergenic | 275149 | ENSG00000170775 | GPR37 |
| chr1 | 184133018 | 184134061 | 3,23 | -0,81 | 4,04 | 0,000441 | 0,0098 | Distal Intergenic | 112207 | ENSGO0000198860 | TSEN15 |
| chr14 | 24164028 | 24164947 | 3,65 | -0,4 | 4,05 | 0,000441 | 0,0098 | Distal Intergenic | 58455 | ENSG00000100867 | DHRS2 |
| chr3 | 197018638 | 197020305 | 4,28 | 0,24 | 4,04 | 0,000442 | 0,0098 | Promoter (<=1kb) | 514 | ENSG00000265850 | MIR4797 |
| chr18 | 33204244 | 33204634 | 1,57 | -1,55 | 3,12 | 0,000443 | 0,00982 | Intron (uc002kyz.4/2589, intron 1 of 12) | -29899 | ENSG00000141429 | GALNT1 |
| chr7 | 128984448 | 128985048 | 2,1 | -1,55 | 3,65 | 0,000445 | 0,00984 | Intron (uc003vot.3/23382, intron 1 of 16) | -22916 | ENSG00000158467 | AHCYL2 |
| chr2 | 165218079 | 165218597 | 2,94 | -0,74 | 3,68 | 0,000445 | 0,00984 | Distal Intergenic | 206420 | ENSG00000115290 | GRB14 |
| chr6 | 10418496 | 10420658 | 5,29 | 1,14 | 4,15 | 0,000447 | 0,00986 | Promoter (<=1kb) | 0 | ENSG00000137203 | TFAP2A |
| chr5 | 130823968 | 130824858 | 2,3 | -1,55 | 3,85 | 0,000447 | 0,00987 | Intron (uc003kvn.2/51735, intron 15 of 27) | 19042 | ENSG00000158987 | RAPGEF6 |
| chr6 | 152375151 | 152375890 | 2,63 | -1,55 | 4,18 | 0,000447 | 0,00987 | Intron (uc003qom.4/2099, intron 7 of 9) | 109843 | ENSG00000091831 | ESR1 |
| chr18 | 33304868 | 33305816 | 2,45 | -1,55 | 4,01 | 0,000449 | 0,0099 | Distal Intergenic | 70335 | ENSG00000141429 | GALNT1 |
| chr2 | 165653997 | 165654676 | 2,25 | -1,55 | 3,81 | 0,000451 | 0,00993 | Intron (uc002ucp.3/22837, intron 1 of 13) | 43252 | ENSG00000082438 | COBLL1 |
| chrX | 32392862 | 32393583 | 2,21 | -1,55 | 3,76 | 0,000452 | 0,00995 | Intron (uc004dcw.2/1756, intron 6 of 50) | -9537 | ENSG00000198947 | DMD |
| chr6 | 15341830 | 15342758 | 2,5 | -1,55 | 4,05 | 0,000454 | 0,00998 | Intron (uc011diu.1/3720, intron 1 of 5) | -58331 | ENSG00000008083 | JARID2 |
| chr8 | 98795661 | 98796555 | 2,62 | -1,55 | 4,17 | 0,000456 | 0,01 | Intron (uc003yia.3/55353, intron 1 of 6) | 7852 | ENSG00000104341 | LAPTM4 B |
| chr4 | 27947790 | 27948712 | 2,67 | -1,55 | 4,23 | 0,000457 | 0,01 | Distal Intergenic | -872492 | ENSG00000283275 | MIR4275 |
| chr13 | 81554878 | 81555318 | 2,27 | -1,4 | 3,67 | 0,000458 | 0,01 | Distal Intergenic | -639792 | ENSG00000136158 | SPRY2 |
| chr4 | 148757520 | 148758623 | 3,25 | -0,75 | 4 | 0,000458 | 0,01 | Intron (uc003ile.1/79658, intron 3 of 4) | -44366 | ENSG00000071205 | ARHGAP10 |
| chr16 | 82958012 | 82959051 | 2,29 | -1,55 | 3,84 | 0,000459 | 0,0101 | Intron (uc010chh.3/1012, intron2 of 4) | 297613 | ENSG00000140945 | CDH13 |
| chr3 | 156675139 | 156676019 | 2,38 | -1,55 | 3,93 | 0,000461 | 0,0101 | Intron (uc003fba.1/389170, intron 6 of 13) | 130991 | ENSG00000197980 | LEKR1 |
| chr2 | 182007066 | 182007879 | 2,75 | -1,06 | 3,81 | 0,000462 | 0,0101 | Intron (uc002uns.1/uc002uns.1, intron 1 of 4) | 161215 | ENSG00000170035 | UBE2E3 |
| chr6 | 152284016 | 152284488 | 1,96 | -1,55 | 3,51 | 0,000463 | 0,0101 | Intron (uc003qom.4/2099, intron 6 of 9) | 18708 | ENSG00000091831 | ESR1 |
| chr14 | 91500894 | 91502646 | 3,75 | -0,47 | 4,22 | 0,000463 | 0,0101 | Intron (uc010twi.1/9252, intron 1 of 16) | 23832 | ENSG00000100784 | RPS6KA5 |
| chr6 | 11325093 | 11325915 | 2,72 | -1,55 | 4,27 | 0,000463 | 0,0101 | Intron (uc003mzw.3/4739, intron 1 of 9) | 56666 | ENSG00000111859 | NEDD9 |
| chr4 | 37717606 | 37718413 | 2,16 | -1,55 | 3,71 | 0,000465 | 0,0101 | Distal Intergenic | -29607 | ENSG00000 181826 | RELL1 |
| chr1 | 238222990 | 238223555 | 2,07 | -1,55 | 3,62 | 0,000466 | 0,0102 | Distal Intergenic | -168768 | ENSG00000116996 | ZP4 |
| chr3 | 100530496 | 100531258 | 2,16 | -1,55 | 3,72 | 0,000469 | 0,0102 | Exon (uc003duj.3/25890, exon 6 of 26) | 5081 | ENSG00000154175 | ABI3BP |
| chr1 1 | 1750153 | 1751099 | 2,47 | -1,55 | 4,02 | 0,000469 | 0,0102 | Intron (uc001ltq.2/81532, intron 1 of 4) | 20725 | ENSG00000244242 | IFITM10 |
| chr9 | 77343954 | 77344826 | 2,59 | -1,55 | 4,14 | 0,000469 | 0,0102 | Intron (uc004ajj.1/140803, intron 14 of 15) | 46339 | ENSG00000119121 | TRPM6 |
| chr2 | 51061989 | 51062797 | 3,27 | -0,39 | 3,66 | 0,00047 | 0,0102 | Intron (uc021vhg.1/9378, intron 5 of 22) | -178437 | ENSG00000179915 | NRXN1 |
| chr15 | 57445353 | 57446153 | 2,46 | -1,55 | 4,02 | 0,00047 | 0,0102 | Intron (uc010ugm.1/6938, intron 5 of 18) | -65502 | ENSG00000140262 | TCF12 |
| chr11 | 39056769 | 39057649 | 2,4 | -1,55 | 3,96 | 0,000471 | 0,0102 | Distal Intergenic | 1257031 | ENSGO0000148948 | LRRC4C |
| chr7 | 145770969 | 145771667 | 2,54 | -1,55 | 4,1 | 0,000471 | 0,0102 | Distal Intergenic | -41786 | ENSG00000174469 | CNTNAP2 |
| chr14 | 106234598 | 106242990 | 6,74 | 2,46 | 4,28 | 0,000471 | 0,0102 | 5'UTR | -95435 | NA | ELK2AP |
| chr21 | 35057149 | 35058162 | 3,01 | -0,86 | 3,87 | 0,000473 | 0,0103 | Intron (uc002vsw.3/6453, intron 1 of 29) | -32940 | ENSG00000205726 | ITSN1 |
| chr1 | 240666559 | 240667530 | 2,54 | -1,55 | 4,1 | 0,000474 | 0,0103 | Intron (uc001hys.3/64388, intron 1 of 1) | 45765 | ENSG00000155816 | FMN2 |
| chr12 | 75210080 | 75210646 | 2,4 | -1,55 | 3,95 | 0,000477 | 0,0103 | Distal Intergenic | 278529 | ENSG00000253719 | ATXN7L3B |
| chr15 | 90802999 | 90803637 | 2,33 | -1,26 | 3,59 | 0,000478 | 0,0103 | Intron (uc002bpe.1/440307, intron 8 of 12) | -5258 | ENSG00000182768 | NGRN |
| chr16 | 25838579 | 25840113 | 3,4 | -0,28 | 3,67 | 0,000479 | 0,0104 | Intron (uc002dof.3/9951, intron 1 of 1) | 135232 | ENSG00000182601 | HS3 ST4 |
| chr2 | 188940505 | 188941550 | 2,63 | -1,55 | 4,19 | 0,000481 | 0,0104 | Distal Intergenic | -214846 | ENSG00000144366 | GULP1 |
| chr8 | 126787429 | 126788494 | 2,91 | -0,77 | 3,68 | 0,000484 | 0,0105 | Distal Intergenic | 174947 | ENSG00000245164 | LINC00861 |
| chr14 | 103481046 | 103481851 | 2,59 | -1,55 | 4,14 | 0,000484 | 0,0105 | Intron (uc001ymi. 1/9578, intron 1 of 36) | 41891 | ENSG00000198752 | CDC42BPB |
| chr5 | 34448738 | 34449584 | 2,79 | -1,01 | 3,8 | 0,000485 | 0,0105 | Distal Intergenic | -206849 | ENSG00000039560 | RAI14 |
| chr1 | 184377519 | 184378554 | 2,41 | -1,55 | 3,96 | 0,000485 | 0,0105 | Intron (uc001gqv.1/81563, intron 1 of 5) | 21369 | ENSG00000116667 | Clorf21 |
| chr21 | 28240004 | 28240674 | 2,3 | -1,4 | 3,7 | 0,000488 | 0,0105 | Distal Intergenic | -22276 | ENSG00000154734 | ADAMTS1 |
| chr20 | 8504703 | 8505656 | 2,37 | -1,26 | 3,64 | 0,000489 | 0,0105 | Intron (uc010zrb.1/23236, intron 3 of 27) | -75725 | ENSG00000 182621 | PLCB1 |
| chr5 | 19064677 | 19065581 | 2,51 | -1,55 | 4,06 | 0,00049 | 0,0105 | Distal Intergenic | 820800 | ENSG00000145526 | CDH18 |
| chr6 | 162599863 | 162600842 | 2,8 | -1,55 | 4,35 | 0,000491 | 0,0106 | Intron (uc003qtv.4/5071, intron 2 of 6) | 82955 | ENSG00000185345 | PRKN |
| chr6 | 122892976 | 122893773 | 2,08 | -1,55 | 3,64 | 0,000494 | 0,0106 | Intron (uc003pvz.3/5570, intron 2 of 6) | -37604 | ENSG00000135549 | PKIB |
| chr20 | 49002510 | 49003365 | 2,4 | -1,55 | 3,95 | 0,000494 | 0,0106 | Distal Intergenic | 93253 | ENSG00000203999 | LINC01270 |
| chr6 | 128891329 | 128892179 | 2,81 | -1,26 | 4,07 | 0,000497 | 0,0107 | Distal Intergenic | -49459 | ENSG00000152894 | PTPRK |
| chr13 | 97776098 | 97779585 | 6,38 | 2,01 | 4,37 | 0,000499 | 0,0107 | Distal Intergenic | -14505 | ENSG00000139793 | MBNL2 |
| chr3 | 153850240 | 153852564 | 3,82 | -0,62 | 4,44 | 0,000499 | 0,0107 | Intron (uc021xgc.1/26084, intron 4 of 14) | 11091 | ENSG00000114790 | ARHGEF26 |
| chr19 | 29749440 | 29750284 | 2,08 | -1,55 | 3,64 | 0,000505 | 0,0108 | Distal Intergenic | -45304 | ENSG00000169021 | UQCRFS1 |
| chr2 | 172575317 | 172575792 | 1,83 | -1,55 | 3,38 | 0,000508 | 0,0108 | Intron (uc002uha.2/1781, intron 7 of 17) | -7276 | ENSG00000077380 | DYNC1I2 |
| chrX | 9797968 | 9799134 | 3,86 | 0,38 | 3,48 | 0,000508 | 0,0108 | Intron (uc004csu.1/357, intron 1 of 9) | 43472 | ENSG00000146950 | SHROOM2 |
| chr5 | 129250661 | 129252553 | 4,85 | 0,73 | 4,12 | 0,000509 | 0,0108 | Intron (uc003kvd.3/337876, intron 2 of 2) | 10138 | ENSG00000198108 | CHSY3 |
| chr13 | 91274588 | 91275443 | 2,12 | -1,55 | 3,68 | 0,00051 | 0,0108 | Distal Intergenic | 303408 | ENSG00000231674 | LINC00410 |
| chr7 | 135649158 | 135649951 | 2,27 | -1,55 | 3,82 | 0,000511 | 0,0109 | Intron (uc003vte.4/767558, intron 1 of 3) | 12253 | ENSG00000267697 | LUZP6 |
| chr18 | 76321506 | 76323037 | 3,11 | -0,79 | 3,9 | 0,000512 | 0,0109 | Distal Intergenic | -417238 | ENSG00000256463 | SALL3 |
| chr13 | 109549658 | 109550721 | 2,34 | -1,55 | 3,9 | 0,000513 | 0,0109 | Exon (uc001vqt.1/23026, exon 15 of 35) | 11163 | ENSG00000041515 | MYO16 |
| chr4 | 167723556 | 167724634 | 2,65 | -1,55 | 4,2 | 0,000513 | 0,0109 | Intron (uc021xuf.1/50859, intron 6 of 10) | 430690 | ENSG00000196104 | SPOCK3 |
| chr2 | 166231935 | 166232600 | 2,44 | -1,55 | 3,99 | 0,000517 | 0,011 | Intron (uc002udc.3/6326, intron 22 of 26) | -4744 | ENSG00000136531 | SCN2A |
| chr5 | 25479712 | 25481068 | 2,75 | -1,55 | 4,31 | 0,000518 | 0,011 | Distal Intergenic | -639020 | ENSG00000248908 | LINC02239 |
| chr3 | 71503285 | 71504506 | 3,16 | -0,34 | 3,49 | 0,000519 | 0,011 | Intron (uc003don.3/27086, intron 1 of 21) | 38200 | ENSG00000114861 | FOXP1 |
| chr14 | 93016748 | 93019307 | 5,29 | 1,84 | 3,45 | 0,000521 | 0,011 | Intron (uc001yap.3/79890, intron 1 of 9) | -23272 | ENSG00000100599 | RIN3 |
| chr14 | 97176239 | 97177275 | 3,32 | -0,18 | 3,5 | 0,000521 | 0,011 | Distal Intergenic | -86409 | ENSG00000100749 | VRK1 |
| chr12 | 116525162 | 116526546 | 3,94 | 0,46 | 3,48 | 0,000522 | 0,011 | Intron (uc001tvw.3/23389, intron 4 of 30) | 59913 | ENSGO0000207967 | MIR620 |
| chr1 | 44176985 | 44178222 | 2,28 | -1,55 | 3,84 | 0,000522 | 0,011 | Intron (uc009vwu.1/6487, intron 1 of 7) | 3781 | ENSG00000126091 | ST3 GAL3 |
| chr17 | 25792889 | 25794524 | 4,3 | 0,35 | 3,95 | 0,00053 | 0,0112 | Distal Intergenic | -4512 | ENSG00000141068 | KSR1 |
| chr11 | 40049028 | 40049396 | 2,03 | -1,4 | 3,43 | 0,000531 | 0,0112 | Distal Intergenic | 265284 | ENSG00000148948 | LRRC4C |
| chr3 | 71199814 | 71200332 | 1,94 | -1,55 | 3,49 | 0,000531 | 0,0112 | Intron (uc003dol.3/27086, intron 2 of 16) | -19722 | ENSG00000114861 | FOXP1 |
| chrX | 18558831 | 18559605 | 2,15 | -1,55 | 3,7 | 0,000532 | 0,0112 | Intron (uc004cvm.3/6792, intron 3 of 20) | 33776 | ENSGO0000008086 | CDKL5 |
| chr6 | 19849132 | 19849652 | 2,31 | -1,29 | 3,59 | 0,000533 | 0,0112 | Distal Intergenic | 11531 | ENSG00000172201 | ID4 |
| chr4 | 181505473 | 181506121 | 2,4 | -1,26 | 3,66 | 0,000534 | 0,0112 | Distal Intergenic | 574181 | ENSG00000248197 | LINC00290 |
| chr13 | 71722873 | 71723733 | 2,17 | -1,55 | 3,72 | 0,000535 | 0,0112 | Intron (uc031qmd.1/100885781, intron 2 of 3) | 133600 | ENSG00000226846 | LINC00348 |
| chr2 | 164984359 | 164985862 | 3,18 | -1,02 | 4,2 | 0,000535 | 0,0112 | Distal Intergenic | -391846 | ENSG00000 182263 | FIGN |
| chr4 | 80120117 | 80121012 | 2,6 | -1,55 | 4,15 | 0,000536 | 0,0112 | Intron (uc003hlr.2/100505875, intron 4 of 5) | 3127 | NA | LINC01088 |
| chr1 | 174121427 | 174122657 | 3,76 | -0,4 | 4,16 | 0,000538 | 0,0113 | Distal Intergenic | -5895 | ENSG00000 152061 | RABGAP1L |
| chr10 | 23657098 | 23657908 | 2,47 | -1,55 | 4,02 | 0,000541 | 0,0113 | Distal Intergenic | -23326 | ENSG00000179133 | C10orf67 |
| chr8 | 30487951 | 30488678 | 2,62 | -1,55 | 4,17 | 0,000543 | 0,0114 | Intron (uc003xip.3/2961, intron 3 of 7) | -7439 | ENSG00000253457 | SMIM18 |
| chr7 | 89808309 | 89809806 | 3,21 | -0,75 | 3,96 | 0,000547 | 0,0114 | Intron (uc003ujy.2/261729, intron 1 of 2) | 11405 | ENSG00000157214 | STEAP2 |
| chr4 | 87488512 | 87491058 | 4,45 | 0,14 | 4,31 | 0,000547 | 0,0114 | Intron (uc010ikh.1/5602, intron 1 of 6) | 24205 | ENSG00000109339 | MAPK10 |
| chr10 | 6604776 | 6605438 | 2,66 | -1,01 | 3,67 | 0,000548 | 0,0115 | Intron (uc001ijj.2/5588, intron 1 of 17) | 16816 | ENSG00000065675 | PRKCQ |
| chr6 | 15344117 | 15344930 | 2,27 | -1,55 | 3,83 | 0,00055 | 0,0115 | Intron (uc011diu.1/3720, intron 1 of 5) | -56159 | ENSG00000008083 | JARID2 |
| chr3 | 121812803 | 121814401 | 4,41 | 0,39 | 4,02 | 0,00055 | 0,0115 | Intron (uc003eet.3/942, intron 2 of 6) | 16107 | ENSG00000114013 | CD86 |
| chr1 | 189807801 | 189808830 | 2,57 | -1,55 | 4,12 | 0,00055 | 0,0115 | Distal Intergenic | 636112 | ENSG00000162670 | BRINP3 |
| chr2 | 202015532 | 202016710 | 2,56 | -1,55 | 4,11 | 0,000551 | 0,0115 | Exon (uc002uxh.1/65072, exon 3 of 7) | 5805 | ENSG00000226312 | CFLAR-AS1 |
| chr6 | 53146462 | 53147308 | 2,08 | -1,55 | 3,63 | 0,000555 | 0,0116 | Intron (uc003pbq.1/60481, intron 3 of 7) | 4671 | ENSG00000264056 | MIR5685 |
| chr6 | 67830751 | 67831665 | 2,62 | -1,26 | 3,88 | 0,000556 | 0,0116 | Distal Intergenic | 1332979 | NA | SLC25A51P1 |
| chr4 | 94973865 | 94974985 | 2,51 | -1,55 | 4,06 | 0,000556 | 0,0116 | Distal Intergenic | -153774 | ENSG00000163104 | SMARCAD1 |
| chr1 | 149969053 | 149969686 | 2,19 | -1,55 | 3,75 | 0,000558 | 0,0116 | Intron (uc001etn.3/56957, intron 1 of 11) | 13000 | ENSG00000264522 | OTUD7B |
| chr5 | 147465446 | 147466380 | 2,54 | -1,55 | 4,09 | 0,000559 | 0,0116 | Exon (uc010jgq.1/11005, exon 5 of 12) | 21911 | ENSG00000133710 | SPINK5 |
| chrX | 142524400 | 142525787 | 2,82 | -1,06 | 3,88 | 0,000561 | 0,0117 | Distal Intergenic | 79520 | ENSG00000189252 | SPANXN3 |
| chr2 | 152762371 | 152763296 | 2,37 | -1,55 | 3,92 | 0,000562 | 0,0117 | Intron (uc002txv.3/785, intron 1 of 12) | 67189 | ENSG00000182389 | CACNB4 |
| chr7 | 40349765 | 40351666 | 4,03 | 0,05 | 3,98 | 0,000562 | 0,0117 | Intron (uc003thn.2/79783, intron 8 of 13) | 175190 | ENSG00000175600 | SUGCT |
| chr4 | 30588326 | 30589196 | 2,63 | -1,55 | 4,19 | 0,000562 | 0,0117 | Distal Intergenic | -132834 | ENSG00000169851 | PCDH7 |
| chr2 | 217148084 | 217148373 | 1,6 | -1,55 | 3,15 | 0,000565 | 0,0117 | Exon (uc002vgb.3/57574, exon 2 of 4) | 66472 | ENSG00000260804 | LINC01963 |
| chr5 | 55353972 | 55354909 | 2,18 | -1,55 | 3,73 | 0,000565 | 0,0117 | Distal Intergenic | 57869 | ENSG00000164512 | ANKRD55 |
| chr4 | 80145351 | 80146324 | 2,35 | -1,55 | 3,9 | 0,000566 | 0,0117 | Intron (uc003hlr.2/100505875, intron 4 of 5) | 28361 | NA | LINC01088 |
| chr5 | 168174905 | 168175469 | 2,02 | -1,55 | 3,57 | 0,000568 | 0,0118 | Exon (uc003mab.4/6586, exon 20 of 36) | 19791 | ENSG00000207739 | MIR218-2 |
| chr2 | 64340817 | 64341397 | 2,67 | -0,93 | 3,6 | 0,000568 | 0,0118 | Promoter (1-2kb) | -1334 | ENSG00000197329 | PELI1 |
| chr11 | 90305046 | 90305985 | 2,61 | -1,55 | 4,17 | 0,000569 | 0,0118 | Intron (uc021qoq.1/uc021qoq.1, intron 2 of 6) | -16021 | ENSG00000266703 | MIR4490 |
| chr17 | 74783656 | 74784136 | 2,04 | -1,55 | 3,59 | 0,00057 | 0,0118 | Distal Intergenic | 50073 | ENSG00000092931 | MFSD11 |
| chr13 | 52126047 | 52127059 | 2,56 | -1,55 | 4,12 | 0,00057 | 0,0118 | Promoter (<=1kb) | 0 | ENSG00000266611 | MIR4703 |
| chr2 | 164704782 | 164705337 | 2,35 | -1,55 | 3,91 | 0,000571 | 0,0118 | Distal Intergenic | -112269 | ENSG00000 182263 | FIGN |
| chr9 | 124601883 | 124602821 | 2,32 | -1,55 | 3,87 | 0,000572 | 0,0118 | Intron (uc004blr.3/158135, intron 7 of 7) | 71172 | ENSG00000136848 | DAB2IP |
| chr1 | 186047110 | 186048004 | 2,5 | -1,55 | 4,05 | 0,000572 | 0,0118 | Exon (uc001grq.1/83872, exon 55 of 107) | -49206 | ENSG00000143341 | HMCN1 |
| chr18 | 60009699 | 60010780 | 3,05 | -0,59 | 3,64 | 0,000573 | 0,0118 | Intron (uc002lin.4/8792, intron 1 of 9) | -14743 | ENSG00000141655 | TNFRSF11A |
| chr11 | 82573827 | 82574699 | 3,04 | -0,65 | 3,69 | 0,000578 | 0,0119 | Intron (uc001ozr.3/5547, intron 2 of 9) | 36858 | ENSG00000137509 | PRCP |
| chr7 | 18587650 | 18588472 | 2,56 | -1,55 | 4,11 | 0,000578 | 0,0119 | Exon (uc003sub.4/9734, exon 4 of 4) | 38750 | ENSG00000048052 | HDAC9 |
| chr12 | 123477573 | 123478818 | 4,1 | 0,35 | 3,74 | 0,00058 | 0,0119 | Intron (uc001uej.1/57605, intron 14 of 24) | 11897 | ENSG00000 182196 | ARL6IP4 |
| chr9 | 29438182 | 29439275 | 4,02 | 0,2 | 3,82 | 0,000581 | 0,0119 | Distal Intergenic | -225184 | ENSG00000174482 | LINGO2 |
| chr22 | 17348995 | 17351403 | 2,72 | -1,35 | 4,07 | 0,000582 | 0,0119 | Distal Intergenic | 40631 | ENSGO0000229027 | HSFY1P1 |
| chr2 | 240225645 | 240226886 | 2,53 | -1,55 | 4,08 | 0,000582 | 0,0119 | Promoter (<=1kb) | -271 | ENSG00000265215 | MIR4269 |
| chr1 | 190741467 | 190742079 | 2,21 | -1,55 | 3,76 | 0,000583 | 0,012 | Intron (uc021pgm.2/440704, intron 2 of 4) | 147447 | ENSG00000231175 | LINC01720 |
| chr4 | 168632077 | 168633054 | 2,61 | -1,55 | 4,16 | 0,000584 | 0,012 | Distal Intergenic | -380634 | ENSG00000109511 | ANXA10 |
| chr13 | 104648919 | 104649783 | 2,44 | -1,55 | 4 | 0,000587 | 0,012 | Distal Intergenic | -714071 | ENSG00000263741 | MIR548AS |
| chr21 | 20005390 | 20007036 | 3,84 | 0,06 | 3,77 | 0,000588 | 0,012 | Intron (uc010glf.2/uc010glf.2, intron 1 of 3) | -229420 | ENSG00000154646 | TMPRSS15 |
| chr4 | 141019563 | 141020291 | 2,71 | -0,69 | 3,41 | 0,000594 | 0,0121 | Intron (uc011chd.1/55534, intron 1 of 3) | 54942 | ENSG00000196782 | MAML3 |
| chr5 | 108088446 | 108088937 | 2,36 | -1,1 | 3,45 | 0,000596 | 0,0121 | Intron (uc011cve.1/2241, intron 1 of 5) | 3783 | ENSG00000151422 | FER |
| chr2 | 24800168 | 24801088 | 2,53 | -1,55 | 4,08 | 0,000601 | 0,0122 | Intron (uc002rfi.3/8648, intron 1 of 21) | -6257 | ENSG00000084676 | NCOA1 |
| chr13 | 50033595 | 50034564 | 2,11 | -1,55 | 3,66 | 0,000603 | 0,0123 | 5'UTR | -7436 | ENSG00000136169 | SETDB2 |
| chr5 | 139696014 | 139696904 | 2,49 | -1,55 | 4,04 | 0,000603 | 0,0123 | Distal Intergenic | -13325 | ENSG00000113068 | PFDN1 |
| chr4 | 167944779 | 167945339 | 2,14 | -1,55 | 3,69 | 0,000606 | 0,0123 | Intron (uc021xuf.1/50859, intron 3 of 10) | 209985 | ENSG00000196104 | SPOCK3 |
| chr1 | 244762758 | 244764031 | 4,28 | 0,47 | 3,81 | 0,000606 | 0,0123 | Intron (uc001iak.1/257044, intron 14 of 15) | -52321 | ENSG00000121644 | DESI2 |
| chr7 | 84662119 | 84663052 | 2,27 | -1,55 | 3,82 | 0,000606 | 0,0123 | Intron (uc003uib.3/223117, intron 3 of 9) | 8203 | ENSG00000153993 | SEMA3D |
| chr12 | 55454077 | 55455332 | 3,1 | -0,91 | 4,02 | 0,000606 | 0,0123 | Distal Intergenic | 40348 | ENSG00000123307 | NEUROD4 |
| chr8 | 112673317 | 112674222 | 2,59 | -1,55 | 4,15 | 0,000606 | 0,0123 | Distal Intergenic | 675738 | ENSG00000164796 | CSMD3 |
| chr8 | 62748768 | 62749625 | 2,72 | -1,55 | 4,27 | 0,000606 | 0,0123 | Distal Intergenic | 121421 | ENSG00000264408 | MIR4470 |
| chr1 | 89872900 | 89873860 | 3,01 | -0,94 | 3,94 | 0,000611 | 0,0124 | Promoter (<=1kb) | 0 | ENSG00000225492 | GBP1P1 |
| chr15 | 92690246 | 92690906 | 2,74 | -1,26 | 4 | 0,000611 | 0,0124 | Exon (uc002bqx.2/28232, exon 8 of 10) | -246234 | ENSG00000140557 | ST8SIA2 |
| chr7 | 83813299 | 83814328 | 2,62 | -1,55 | 4,17 | 0,000616 | 0,0124 | Intron (uc003uhz.3/10371, intron 1 of 16) | 9889 | ENSG00000075213 | SEMA3 A |
| chr13 | 44102995 | 44103633 | 2,17 | -1,55 | 3,72 | 0,000618 | 0,0125 | Intron (uc001uza.4/55068, intron 2 of 16) | 99980 | ENSG00000120658 | ENOX1 |
| chr4 | 180124042 | 180124606 | 2,28 | -1,55 | 3,83 | 0,000618 | 0,0125 | Distal Intergenic | 1474131 | ENSG00000231171 | LINC01098 |
| chr3 | 99805550 | 99806282 | 3,43 | -0,11 | 3,53 | 0,00062 | 0,0125 | Intron (uc003dtl.3/84319, intron 1 of 9) | 27067 | ENSG00000168386 | FILIP1L |
| chr5 | 139673928 | 139674935 | 2,52 | -1,55 | 4,08 | 0,000621 | 0,0125 | Intron (uc003lff.1/5201, intron 2 of 3) | 7754 | ENSG00000113068 | PFDN1 |
| chr4 | 163455419 | 163456328 | 2,58 | -1,55 | 4,13 | 0,000621 | 0,0125 | Distal Intergenic | -370233 | ENSG00000168843 | FSTL5 |
| chr8 | 15706820 | 15707460 | 2,17 | -1,55 | 3,72 | 0,000622 | 0,0125 | Distal Intergenic | 105773 | ENSG00000104723 | TUSC3 |
| chr2 | 223344872 | 223345533 | 2,28 | -1,55 | 3,83 | 0,000622 | 0,0125 | Intron (uc010zlo.2/130367, intron 2 of 4) | 55550 | ENSG00000163082 | SGPP2 |
| chr16 | 27265544 | 27266108 | 1,95 | -1,55 | 3,5 | 0,000624 | 0,0125 | Intron (uc002doi.1/197370, intron 2 of 7) | -13418 | ENSG00000245888 | NSMCE1-DT |
| chr11 | 34812843 | 34814268 | 4,47 | 0,72 | 3,75 | 0,000625 | 0,0125 | Distal Intergenic | -123409 | ENSG00000110435 | PDHX |
| chr1 | 113941485 | 113942239 | 2,44 | -1,55 | 3,99 | 0,000626 | 0,0126 | Intron (uc001edh.3/260425, intron 1 of 21) | 8010 | ENSGO0000081026 | MAGI3 |
| chr21 | 19616455 | 19618606 | 6,74 | 1,71 | 5,02 | 0,000626 | 0,0126 | Promoter (<=1kb) | 0 | ENSG00000154645 | CHODL |
| chr4 | 72682203 | 72682822 | 2,56 | -1,06 | 3,62 | 0,00063 | 0,0126 | Distal Intergenic | -10966 | ENSG00000145321 | GC |
| chr2 | 60539846 | 60540428 | 2,04 | -1,55 | 3,6 | 0,000631 | 0,0126 | Distal Intergenic | 74152 | ENSG00000266078 | MIR4432 |
| chr8 | 127512108 | 127512641 | 1,83 | -1,55 | 3,39 | 0,000633 | 0,0127 | Distal Intergenic | 58070 | ENSG00000168672 | LRATD2 |
| chr7 | 80454282 | 80455472 | 2,8 | -0,87 | 3,66 | 0,000633 | 0,0127 | Intron (uc003uhj.3/10512, intron 4 of 17) | 93195 | ENSG00000075223 | SEMA3 C |
| chr7 | 18577268 | 18578244 | 2,39 | -1,55 | 3,94 | 0,000634 | 0,0127 | Intron (uc011jya.2/9734, intron 4 of 12) | 28368 | ENSG00000048052 | HDAC9 |
| chr9 | 123918508 | 123919434 | 2,36 | -1,55 | 3,92 | 0,000635 | 0,0127 | Promoter (<=1kb) | -314 | ENSG00000119397 | CNTRL |
| chr11 | 38198029 | 38199053 | 3,68 | -0,56 | 4,24 | 0,000635 | 0,0127 | Distal Intergenic | -1578200 | ENSG00000175097 | RAG2 |
| chr5 | 34031021 | 34032384 | 2,57 | -1,55 | 4,13 | 0,000638 | 0,0127 | Promoter (1-2kb) | -1908 | ENSG00000082196 | C1QTNF3 |
| chr6 | 146473959 | 146474951 | 3,1 | -0,16 | 3,26 | 0,000641 | 0,0128 | Intron (uc010khu.1/2911, intron 2 of 2) | 123625 | ENSG00000152822 | GRM1 |
| chr17 | 25750848 | 25751413 | 2,87 | -0,54 | 3,4 | 0,000643 | 0,0128 | Exon (uc002gzg.3/440419, exon 8 of 13) | 5817 | ENSG00000266433 | TBC1D3P5 |
| chr1 | 37195842 | 37196739 | 2,89 | -1,29 | 4,18 | 0,000644 | 0,0128 | Distal Intergenic | -246927 | ENSG00000119535 | CSF3R |
| chr7 | 41005745 | 41006508 | 2,38 | -1,55 | 3,93 | 0,000646 | 0,0129 | Distal Intergenic | 172689 | ENSG00000175600 | SUGCT |
| chr3 | 161294572 | 161295205 | 2,25 | -1,55 | 3,81 | 0,000648 | 0,0129 | Distal Intergenic | 79976 | ENSG00000 182447 | OTOL1 |
| chr20 | 10022451 | 10023229 | 2,44 | -0,97 | 3,41 | 0,000649 | 0,0129 | Intron (uc002wno.3/63926, intron 3 of 10) | 6754 | ENSG00000132623 | ANKEF1 |
| chr9 | 12811613 | 12815696 | 5,82 | 1,27 | 4,55 | 0,00065 | 0,0129 | Intron (uc003zkw.3/286343, intron 1 of 1) | 36601 | ENSG00000153714 | LURAP1L |
| chr4 | 96560036 | 96560697 | 2,13 | -1,55 | 3,68 | 0,000651 | 0,0129 | Distal Intergenic | -89675 | ENSG00000 182168 | UNC5 C |
| chr1 | 143949993 | 143951526 | 2,28 | -1,26 | 3,55 | 0,000657 | 0,013 | Intron (uc010oxm.1/uc010oxm.1, intron 1 of 3) | -36850 | ENSG00000215784 | FAM72D |
| chr13 | 33772941 | 33774496 | 3,79 | 0,08 | 3,71 | 0,000657 | 0,013 | Intron (uc001uuv.3/90627, intron 1 of 13) | 5691 | ENSG00000133121 | STARD13 |
| chr4 | 105799867 | 105800835 | 2,46 | -1,14 | 3,59 | 0,000659 | 0,013 | Distal Intergenic | -266197 | ENSG00000168769 | TET2 |
| chr7 | 120272054 | 120272567 | 1,99 | -1,55 | 3,54 | 0,000661 | 0,013 | Intron (uc003vjj.1/3751, intron 1 of 5) | 181647 | ENSG00000106025 | TSPAN12 |
| chr18 | 6004328 | 6004893 | 2,02 | -1,55 | 3,57 | 0,000662 | 0,013 | Intron (uc002kmy.4/91133, intron 14 of 16) | -112225 | ENSG00000206432 | TMEM200C |
| chr8 | 113913481 | 113914099 | 2,26 | -1,55 | 3,81 | 0,000662 | 0,013 | Intron (uc003ynt.3/114788, intron 11 of 71) | -211202 | ENSG00000164796 | CSMD3 |
| chr6 | 157396536 | 157397689 | 3,56 | -0,4 | 3,95 | 0,000663 | 0,013 | Intron (uc003qqn.3/57492, intron 4 of 19) | 139936 | ENSG00000049618 | ARID1B |
| chr4 | 4633066 | 4633865 | 2,59 | -1,02 | 3,61 | 0,000669 | 0,0132 | Intron (uc003gid.3/100507266, intron 2 of 5) | 89208 | ENSG00000247708 | STX18-AS1 |
| chr2 | 97136321 | 97136924 | 2,22 | -1,55 | 3,77 | 0,00067 | 0,0132 | Distal Intergenic | 34170 | ENSG00000163121 | NEURL3 |
| chr22 | 40357511 | 40358373 | 2,59 | -0,28 | 2,86 | 0,000671 | 0,0132 | Intron (uc003avh.2/9402, intron 4 of 7) | 14655 | ENSG00000100351 | GRAP2 |
| chr13 | 93885715 | 93886425 | 2,43 | -1,55 | 3,98 | 0,000672 | 0,0132 | Intron (uc010tig.1/10082, intron 1 of 5) | 6637 | ENSG00000183098 | GPC6 |
| chr8 | 101295284 | 101296082 | 2,15 | -1,55 | 3,7 | 0,000674 | 0,0132 | Intron (uc003vjj.1/25897, intron 3 of 10) | 19405 | ENSG00000034677 | RNF19A |
| chr6 | 153175408 | 153176251 | 2,32 | -1,55 | 3,87 | 0,000674 | 0,0132 | Distal Intergenic | 103476 | ENSG00000146469 | VIP |
| chr7 | 87987205 | 87987784 | 2,85 | -0,5 | 3,35 | 0,000675 | 0,0132 | Distal Intergenic | -50977 | ENSG00000127954 | STEAP4 |
| chr21 | 25303947 | 25305175 | 3,88 | 0,19 | 3,69 | 0,000676 | 0,0132 | Distal Intergenic | -546791 | ENSG00000228592 | D21S2088E |
| chr7 | 116273302 | 116274254 | 2,87 | -1,26 | 4,13 | 0,000678 | 0,0132 | Distal Intergenic | -38205 | ENSG00000105976 | MET |
| chr10 | 68298014 | 68298616 | 2,22 | -1,55 | 3,78 | 0,000678 | 0,0133 | Intron (uc009xpn.1/29119, intron 10 of 17) | -387176 | ENSG00000198739 | LRRTM3 |
| chr8 | 98858950 | 98860661 | 4,21 | 0,39 | 3,82 | 0,000678 | 0,0133 | Intron (uc003via.3/55353, intron 6 of 6) | -20650 | ENSG00000132561 | MATN2 |
| chr3 | 41888768 | 41891210 | 3,85 | -0,16 | 4,01 | 0,000679 | 0,0133 | Intron (uc003ckv.4/54986, intron 17 of 36) | 112251 | ENSG00000168038 | ULK4 |
| chr5 | 90657464 | 90657896 | 2 | -1,55 | 3,55 | 0,000681 | 0,0133 | Distal Intergenic | -18268 | ENSG00000281357 | ARRDC3-AS1 |
| chr6 | 98915711 | 98916216 | 1,7 | -1,55 | 3,25 | 0,000682 | 0,0133 | Distal Intergenic | -366364 | ENSG00000184486 | POU3F2 |
| chr15 | 80360512 | 80361204 | 2,43 | -1,55 | 3,98 | 0,000682 | 0,0133 | Intron (uc002bfe.2/54469, intron 1 of 6) | -3699 | ENSG00000086666 | ZFAND6 |
| chr13 | 32504817 | 32505589 | 2,62 | -0,8 | 3,42 | 0,000683 | 0,0133 | Intron (uc001utu.3/196549, intron 1 of 3) | 83897 | NA | EEF1DP3 |
| chr1 | 206879597 | 206880647 | 2,35 | -1,55 | 3,91 | 0,000683 | 0,0133 | Intron (uc001hel.2/9261, intron 1 of 9) | 21232 | ENSG00000162889 | MAPKAPK2 |
| chr14 | 94643181 | 94644472 | 2,48 | -1,55 | 4,03 | 0,000683 | 0,0133 | Promoter (2-3kb) | 2532 | ENSG00000119698 | PPP4R4 |
| chr4 | 133268330 | 133268918 | 2,55 | -1,55 | 4,1 | 0,000684 | 0,0133 | Distal Intergenic | -801552 | ENSG00000138650 | PCDH10 |
| chr5 | 172660521 | 172662661 | 6,87 | 2,75 | 4,12 | 0,00069 | 0,0134 | Promoter (<=1kb) | 0 | ENSG00000183072 | NKX2-5 |
| chr9 | 123652014 | 123655054 | 5,48 | 1,33 | 4,15 | 0,000691 | 0,0134 | Distal Intergenic | -12408 | ENSG00000119403 | PHF19 |
| chr7 | 115494256 | 115494833 | 1,97 | -1,55 | 3,52 | 0,000696 | 0,0135 | Distal Intergenic | 113534 | ENSG00000105967 | TFEC |
| chr2 | 82890254 | 82890929 | 2,06 | -1,55 | 3,61 | 0,000698 | 0,0135 | Distal Intergenic | -192998 | NA | DHFRP3 |
| chr20 | 21147451 | 21148197 | 1,95 | -1,55 | 3,5 | 0,000699 | 0,0135 | Intron (uc002wsb.3/55857, intron 6 of 13) | 4939 | ENSG00000088970 | KIZ |
| chr7 | 12177883 | 12178998 | 2,85 | -0,93 | 3,78 | 0,000701 | 0,0135 | Distal Intergenic | -71850 | ENSG00000106460 | TMEM106B |
| chr2 | 196706681 | 196708311 | 4,33 | 0,55 | 3,78 | 0,000701 | 0,0135 | Intron (uc002utj.4/56171, intron 47 of 64) | -46117 | ENSG00000118997 | DNAH7 |
| chr4 | 17877654 | 17878243 | 2,17 | -1,55 | 3,73 | 0,000703 | 0,0136 | Intron (uc021xmq.1/254251, intron 4 of 4) | 65218 | ENSG00000109805 | NCAPG |
| chr5 | 140613850 | 140614445 | 2,27 | -1,55 | 3,82 | 0,000703 | 0,0136 | Promoter (<=1kb) | 0 | ENSG00000146001 | PCDHB18P |
| chr12 | 34477712 | 34480141 | 4,79 | 0,53 | 4,26 | 0,000706 | 0,0136 | Distal Intergenic | 302496 | ENSG00000139133 | ALG10 |
| chr1 | 193562123 | 193562581 | 2,05 | -1,55 | 3,61 | 0,000707 | 0,0136 | Distal Intergenic | -406380 | ENSG00000162630 | B3GALT2 |
| chrX | 108684039 | 108684979 | 2,41 | -1,31 | 3,73 | 0,000714 | 0,0137 | Exon (uc011msq.2/2986, exon 6 of 21) | 34271 | ENSG00000101890 | GUCY2F |
| chrX | 32338932 | 32339655 | 2,26 | -1,55 | 3,81 | 0,000714 | 0,0137 | Intron (uc004dcw.2/1756, intron 13 of 50) | -33114 | ENSG00000198947 | DMD |
| chr21 | 23775411 | 23776077 | 2,33 | -1,55 | 3,88 | 0,000714 | 0,0137 | Distal Intergenic | 304475 | ENSG00000184856 | LINC00308 |
| chr18 | 5793789 | 5794462 | 2,16 | -1,55 | 3,71 | 0,000716 | 0,0138 | Intron (uc002kmw.3/645355, intron 4 of 4) | 44971 | ENSG00000261738 | MIR3976HG |
| chr2 | 220385884 | 220386391 | 2,18 | -0,91 | 3,1 | 0,000719 | 0,0138 | Intron (uc010fwi.2/55515, intron 1 of 4) | 4026 | ENSG00000072182 | ASIC4 |
| chr1 | 181463806 | 181464771 | 2,53 | -1,55 | 4,08 | 0,000722 | 0,0139 | Intron (uc001gow.3/777, intron 1 of 46) | 11120 | ENSG00000198216 | CACNA1E |
| chr21 | 42833030 | 42833646 | 2,02 | -1,38 | 3,4 | 0,000727 | 0,0139 | Downstream (2-3kb) | 35052 | ENSG00000157601 | MX1 |
| chr7 | 64760057 | 64760664 | 2,33 | -1,55 | 3,88 | 0,000727 | 0,0139 | Distal Intergenic | -78104 | ENSG00000146757 | ZNF92 |
| chr5 | 133331315 | 133332194 | 2,12 | -1,55 | 3,67 | 0,000728 | 0,0139 | Intron (uc003kvp.2/7416, intron 1 of 8) | 8463 | ENSG00000213585 | VDAC1 |
| chr2 | 64341452 | 64342206 | 2,86 | -0,62 | 3,48 | 0,000729 | 0,014 | Promoter (1-2kb) | -1969 | ENSG00000197329 | PELI1 |
| chr5 | 145355645 | 145356759 | 2,63 | -1,26 | 3,88 | 0,000729 | 0,014 | Intron (uc0031nt.3/153769, intron 2 of 9) | 38259 | ENSG00000156463 | SH3 RF2 |
| chr17 | 66768152 | 66768618 | 2,56 | -0,91 | 3,47 | 0,000732 | 0,014 | Distal Intergenic | -171057 | ENSG00000108950 | FAM20A |
| chr5 | 156647036 | 156647693 | 1,85 | -1,55 | 3,41 | 0,000733 | 0,014 | Intron (uc0031wo.1/3702, intron 5 of 16) | 39129 | ENSG00000113263 | ITK |
| chr10 | 11570443 | 11571044 | 2,14 | -1,55 | 3,69 | 0,000734 | 0,014 | Intron (uc001iks.1/9712, intron 1 of 13) | 3230 | ENSG00000148429 | USP6NL |
| chr4 | 167892897 | 167893837 | 2,31 | -1,55 | 3,87 | 0,000734 | 0,014 | Intron (uc021xuf.1/50859, intron 4 of 10) | 261487 | ENSG00000196104 | SPOCK3 |
| chr19 | 42594001 | 42597058 | 7,34 | 4,03 | 3,31 | 0,000735 | 0,014 | 3'UTR | 13711 | ENSG00000105732 | ZNF574 |
| chr21 | 46549848 | 46551101 | 4,27 | 0,89 | 3,38 | 0,000735 | 0,014 | Intron (uc002zgp.2/104, intron 5 of 9) | -21422 | ENSG00000197381 | ADARB1 |
| chr4 | 59501455 | 59502203 | 2,66 | -1,23 | 3,89 | 0,000737 | 0,014 | Distal Intergenic | -1524904 | ENSG00000163453 | IGFBP7 |
| chr2 | 147794094 | 147794988 | 2,81 | -1,23 | 4,05 | 0,000737 | 0,014 | Distal Intergenic | 449469 | NA | PABPC1P2 |
| chr7 | 91084370 | 91086468 | 5,74 | 1,68 | 4,06 | 0,000737 | 0,014 | Distal Intergenic | 190587 | ENSG00000157240 | FZD1 |
| chr10 | 78641375 | 78642397 | 2,42 | -1,55 | 3,97 | 0,000741 | 0,0141 | Intron (uc001jxj.2/3778, intron 28 of 28) | 227704 | ENSG00000156113 | KCNMA1 |
| chr8 | 97349675 | 97351104 | 4 | -0,06 | 4,06 | 0,000741 | 0,0141 | Distal Intergenic | 75508 | ENSG00000156471 | PTDSS1 |
| chr13 | 66204646 | 66206642 | 3,54 | -0,57 | 4,11 | 0,000741 | 0,0141 | Distal Intergenic | -1192659 | ENSG00000228842 | PCDH9-AS2 |
| chr1 | 242406096 | 242407457 | 3,94 | 0,55 | 3,39 | 0,000743 | 0,0141 | Intron (uc001hzl.4/200150, intron 4 of 9) | 205327 | ENSG00000180287 | PLD5 |
| chr2 | 53952791 | 53953565 | 2,49 | -1,26 | 3,75 | 0,000743 | 0,0141 | Intron (uc021vhl.1/51130, intron 4 of 8) | -25119 | ENSG00000115239 | ASB3 |
| chrX | 22704089 | 22704627 | 1,95 | -1,29 | 3,24 | 0,000746 | 0,0141 | Intron (uc031tgz.1/100873065, intron 4 of 5) | -313451 | ENSG00000184735 | DDX53 |
| chr5 | 2944507 | 2945025 | 2,36 | -1,55 | 3,91 | 0,000746 | 0,0141 | Distal Intergenic | 192245 | ENSG00000186493 | C5orf38 |
| chr3 | 141211370 | 141212210 | 2,13 | -1,55 | 3,68 | 0,000747 | 0,0141 | Intron (uc003etz.1/5922, intron 1 of 23) | 5444 | ENSG00000155903 | RASA2 |
| chr5 | 27661025 | 27661735 | 2,25 | -1,55 | 3,8 | 0,000747 | 0,0141 | Distal Intergenic | 188626 | ENSG00000250337 | PURPL |
| chr13 | 39770915 | 39771885 | 2,41 | -1,55 | 3,96 | 0,000747 | 0,0141 | Distal Intergenic | 158467 | ENSG00000188811 | NHLRC3 |
| chr12 | 83551781 | 83552864 | 2,48 | -1,26 | 3,74 | 0,000748 | 0,0141 | Distal Intergenic | 470847 | ENSG00000179104 | TMTC2 |
| chr17 | 80245618 | 80246724 | 3,37 | -0,86 | 4,24 | 0,000749 | 0,0142 | Distal Intergenic | -14024 | ENSG00000141551 | CSNK1D |
| chr4 | 2392536 | 2393165 | 2,22 | -1,55 | 3,77 | 0,000751 | 0,0142 | Intron (uc003gex.2/57732, intron 1 of 12) | -25932 | ENSG00000159733 | ZFYVE28 |
| chr10 | 112610771 | 112611255 | 1,89 | -1,55 | 3,44 | 0,000752 | 0,0142 | Distal Intergenic | 19407 | NA | PDCD4-AS1 |
| chr3 | 89783287 | 89784045 | 2,5 | -1,55 | 4,06 | 0,000752 | 0,0142 | Distal Intergenic | 626613 | ENSG00000044524 | EPHA3 |
| chr2 | 7421186 | 7422071 | 2,2 | -1,55 | 3,76 | 0,000753 | 0,0142 | Distal Intergenic | -139321 | ENSG00000229727 | LOC100506274 |
| chr7 | 114100588 | 114101260 | 2,32 | -1,55 | 3,87 | 0,000753 | 0,0142 | Intron (uc003vgt.2/93986, intron 4 of 11) | 34031 | ENSG00000128573 | FOXP2 |
| chr1 | 194356768 | 194357684 | 2,4 | -1,55 | 3,95 | 0,000759 | 0,0143 | Distal Intergenic | -1201025 | ENSG00000162630 | B3GALT2 |
| chr20 | 14905286 | 14906521 | 2,58 | -1,55 | 4,13 | 0,000762 | 0,0144 | Intron (uc002wot.3/140733, intron 5 of 16) | 3640 | ENSG00000235914 | MACROD2-AS1 |
| chr7 | 36603609 | 36604750 | 3,19 | -0,63 | 3,82 | 0,000764 | 0,0144 | Intron (uc003tfh.4/313. intron 13 of 20) | 34976 | ENSG00000230539 | AOAH-IT1 |
| chr8 | 115400094 | 115400962 | 2,46 | -1,55 | 4,01 | 0,000764 | 0,0144 | Distal Intergenic | -950852 | ENSG00000164796 | CSMD3 |
| chr20 | 20469019 | 20470842 | 4,51 | 1,04 | 3,47 | 0,000765 | 0,0144 | Intron (uc002wrv.3/57186, intron 27 of 29) | 38060 | ENSG00000188559 | RALGAPA2 |
| chr11 | 15662616 | 15663363 | 3,09 | -0,54 | 3,63 | 0,000765 | 0,0144 | Distal Intergenic | 491968 | ENSG00000188487 | INSC |
| chr4 | 97763158 | 97763987 | 2,38 | -1,55 | 3,93 | 0,000765 | 0,0144 | Distal Intergenic | -524090 | ENSG00000251620 | STPG2-AS1 |
| chr4 | 36129391 | 36130497 | 2,58 | -1,55 | 4,13 | 0,000765 | 0,0144 | Exon (uc003psq.2/116984,exon 21 of 33) | -53934 | ENSG0000004 7365 | ARAP2 |
| chr10 | 15224782 | 15226051 | 2,84 | -1,26 | 4,09 | 0,000766 | 0,0144 | Distal Intergenic | -14087 | ENSG00000152465 | NMT2 |
| chr13 | 33762368 | 33764259 | 4,4 | 1,36 | 3,05 | 0,000767 | 0,0144 | Promoter (2-3kb) | -2152 | ENSG00000133121 | STARD13 |
| chr19 | 50638000 | 50638784 | 2,11 | -1,55 | 3,66 | 0,000767 | 0,0144 | Promoter (<=1kb) | -879 | NA | SNAR-B2 |
| chr7 | 33273207 | 33273864 | 3,17 | -0,65 | 3,82 | 0,000768 | 0,0144 | Intron (uc003tdn.1/27241, intron 5 of 22) | 75957 | ENSG00000122507 | BBS9 |
| chr6 | 13681965 | 13682468 | 1,91 | -1,55 | 3,46 | 0,000772 | 0,0145 | Intron (uc003nba.3/10048, intron 1 of 11) | 14660 | ENSG00000010017 | RANBP9 |
| chr2 | 188774664 | 188775227 | 2,15 | -1,55 | 3,7 | 0,000772 | 0,0145 | Distal Intergenic | -355445 | ENSG00000003436 | TFPI |
| chr1 | 184425065 | 184426055 | 2,81 | -0,97 | 3,78 | 0,000772 | 0,0145 | Intron (uc001gqv.1/81563, intron 1 of 5) | 68915 | ENSG00000116667 | Clorf21 |
| chr4 | 181933922 | 181934900 | 3,05 | -0,75 | 3,8 | 0,000772 | 0,0145 | Distal Intergenic | 145402 | ENSG00000248197 | LINC00290 |
| chr12 | 25216619 | 25217280 | 2,04 | -1,55 | 3,59 | 0,000777 | 0,0145 | 5'UTR | 11438 | ENSG00000118308 | LRMP |
| chr20 | 33642159 | 33644098 | 4,63 | 0,81 | 3,82 | 0,000777 | 0,0145 | Exon (uc002xbk.3/26133, exon 5 of 19) | -19048 | ENSG00000100991 | TRPC4AP |
| chr18 | 5394923 | 5396183 | 4,01 | 0,41 | 3,6 | 0,000779 | 0,0146 | Exon (uc002kms.1/23136, exon 6 of 9) | 23693 | ENSG00000082397 | EPB41L3 |
| chr9 | 31278927 | 31280040 | 3,33 | -0,62 | 3,95 | 0,000779 | 0,0146 | Distal Intergenic | -870475 | NA | LINC01242 |
| chr9 | 98486410 | 98486925 | 2,08 | -1,1 | 3,18 | 0,000782 | 0,0146 | Distal Intergenic | -150975 | ENSG00000182150 | ERCC6L2 |
| chrX | 33357326 | 33358055 | 2,24 | -1,55 | 3,79 | 0,000782 | 0,0146 | Promoter (<=1kb) | 0 | ENSG00000198947 | DMD |
| chr2 | 221989483 | 221989946 | 1,97 | -1,55 | 3,52 | 0,000783 | 0,0146 | Distal Intergenic | 447055 | ENSG00000116106 | EPHA4 |
| chr2 | 14794588 | 14795592 | 2,89 | -0,8 | 3,7 | 0,000783 | 0,0146 | Distal Intergenic | 21778 | ENSG00000162981 | LRATD1 |
| chr3 | 175484357 | 175485007 | 2,96 | -0,59 | 3,55 | 0,000785 | 0,0146 | Intron (uc003fit.3/254827, intron 13 of 13) | 397028 | ENSG00000264974 | MIR4789 |
| chr11 | 48194598 | 48195113 | 2,05 | -1,55 | 3,6 | 0,000786 | 0,0147 | Distal Intergenic | 37355 | ENSG00000149177 | PTPRJ |
| chr7 | 36211327 | 36212132 | 2,34 | -1,55 | 3,9 | 0,000786 | 0,0147 | Intron (uc003tfa.3/80820, intron 2 of 7) | 18491 | ENSG00000122547 | EEPD1 |
| chr13 | 104547019 | 104548229 | 2,47 | -1,55 | 4,03 | 0,000787 | 0,0147 | Distal Intergenic | -612171 | ENSG00000263741 | MIR548AS |
| chr6 | 123034881 | 123035748 | 2,37 | -1,55 | 3,92 | 0,000796 | 0,0148 | Intron (uc003pvz.3/5570, intron 5 of 6) | -3165 | ENSG00000135549 | PKIB |
| chr2 | 21669257 | 21670172 | 2,57 | -1,55 | 4,12 | 0,000798 | 0,0148 | Distal Intergenic | 263352 | ENSGO0000229621 | LINC01822 |
| chr10 | 18272348 | 18273053 | 2,21 | -1,55 | 3,76 | 0,000799 | 0,0148 | Intron (uc001ipn.2/221074, intron 6 of 11) | 26438 | ENSG00000226083 | SLC39A12-AS1 |
| chr18 | 27845066 | 27845842 | 2,34 | -1,55 | 3,89 | 0,000803 | 0,0149 | Distal Intergenic | -33034 | ENSG00000283218 | MIR302F |
| chr2 | 139536751 | 139538677 | 4,92 | 0,43 | 4,49 | 0,000803 | 0,0149 | Promoter (<=1kb) | 0 | ENSG00000144227 | NXPH2 |
| chr10 | 104885953 | 104886545 | 1,98 | -1,55 | 3,53 | 0,000804 | 0,0149 | Intron (uc010qqp.2/22978, intron 2 of 16) | 26839 | ENSG00000076685 | NT5C2 |
| chr12 | 99377488 | 99378713 | 2,34 | -1,55 | 3,89 | 0,000808 | 0,015 | Intron (uc001tgd.2/56899, intron 3 of 10) | -88814 | ENSG00000185046 | ANKS1B |
| chr3 | 183638921 | 183639489 | 2,63 | -1,29 | 3,92 | 0,000809 | 0,015 | 3'UTR | -36228 | ENSG00000175193 | PARL |
| chr7 | 25703645 | 25704375 | 2,19 | -1,55 | 3,74 | 0,000813 | 0,015 | Distal Intergenic | 176255 | NA | RNU6-16P |
| chr22 | 38500851 | 38501352 | 1,8 | -1,55 | 3,35 | 0,000814 | 0,0151 | Intron (uc003auw.3/80115, intron 4 of 13) | 5324 | ENSG00000128298 | BAIAP2L2 |
| chr17 | 54450267 | 54451189 | 2,4 | -1,26 | 3,65 | 0,000815 | 0,0151 | Intron (uc002iun.1/162282, intron 6 of 16) | 219431 | ENSG00000153930 | ANKFN1 |
| chr14 | 71964382 | 71965668 | 3,28 | -0,54 | 3,83 | 0,000816 | 0,0151 | Intron (uc001xmr.1/26037, intron 1 of 3) | 9804 | NA | LOC145474 |
| chr12 | 95594062 | 95594761 | 2,24 | -1,55 | 3,79 | 0,000817 | 0,0151 | Intron (uc001tdp.4/55785, intron 2 of 20) | 16479 | ENSG00000180263 | FGD6 |
| chr5 | 22460374 | 22461063 | 2,89 | -0,69 | 3,58 | 0,000818 | 0,0151 | Intron (uc003jgk.2/1010, intron 2 of 14) | -246465 | ENSG00000154162 | CDH12 |
| chr1 | 181366854 | 181368168 | 3,48 | -0,46 | 3,93 | 0,000818 | 0,0151 | Distal Intergenic | -84518 | ENSG00000198216 | CACNA1E |
| chr15 | 77018729 | 77019251 | 2,16 | -1,55 | 3,71 | 0,000822 | 0,0152 | Promoter (1-2kb) | 1827 | ENSG00000140386 | SCAPER |
| chr17 | 31296892 | 31297391 | 1,84 | -1,55 | 3,39 | 0,000823 | 0,0152 | Distal Intergenic | -21491 | ENSG00000141316 | SPACA3 |
| chr2 | 203520684 | 203521235 | 1,87 | -1,55 | 3,43 | 0,000827 | 0,0152 | Intron (uc010zhw.2/150864, intron 1 of 2) | 20783 | ENSG00000138439 | FAM117B |
| chr8 | 84042811 | 84043493 | 2,98 | -0,7 | 3,68 | 0,000829 | 0,0153 | Distal Intergenic | -1051960 | ENSG00000184672 | RALYL |
| chr19 | 44203483 | 44204005 | 2,43 | -1,55 | 3,99 | 0,000829 | 0,0153 | Distal Intergenic | -16209 | ENSG00000124449 | IRGC |
| chr10 | 101743866 | 101744813 | 2,91 | -0,75 | 3,66 | 0,00083 | 0,0153 | Intron (uc001kqj.2/23268, intron 1 of 16) | 24863 | ENSG00000107554 | DNMBP |
| chr5 | 21701310 | 21702101 | 2,97 | -1,01 | 3,98 | 0,000831 | 0,0153 | Intron (uc003jgj.3/uc003jgj.3, intron 2 of 3) | 241721 | ENSG00000183666 | GUSBP1 |
| chr13 | 67607264 | 67608195 | 2,83 | -0,72 | 3,55 | 0,000832 | 0,0153 | Intron (uc001vik.3/5101, intron 2 of 4) | 55743 | ENSG00000225263 | PCDH9-AS3 |
| chr9 | 33130414 | 33131288 | 2,33 | -1,55 | 3,89 | 0,000835 | 0,0153 | Intron (uc003zsg.2/2683, intron 2 of 5) | 36068 | ENSG00000086062 | B4GALT1 |
| chr3 | 197534982 | 197536227 | 2,38 | -1,55 | 3,94 | 0,000835 | 0,0153 | Intron (uc003fyj.1/84859, intron 1 of 18) | 16837 | ENSG00000186001 | LRCH3 |
| chr8 | 60121872 | 60125427 | 4,78 | 0,61 | 4,18 | 0,000835 | 0,0153 | Distal Intergenic | -90105 | ENSG00000198846 | TOX |
| chr14 | 89893815 | 89898045 | 8,04 | 6,34 | 1,7 | 0,000836 | 0,0153 | Intron (uc001xxo.4/1112, intron 1 of 6) | 10117 | ENSG00000258920 | FOXN3-AS1 |
| chr7 | 125359576 | 125360378 | 2,2 | -1,55 | 3,76 | 0,000837 | 0,0154 | Distal Intergenic | -789539 | ENSG00000128513 | POT1 |
| chr13 | 59069365 | 59069843 | 2,19 | -1,35 | 3,54 | 0,000838 | 0,0154 | Distal Intergenic | 828578 | ENSG00000118946 | PCDH17 |
| chr10 | 54643880 | 54644595 | 2,18 | -0,98 | 3,17 | 0,00084 | 0,0154 | Distal Intergenic | -112420 | ENSG00000165471 | MBL2 |
| chr3 | 153462712 | 153465937 | 5,08 | 0,76 | 4,32 | 0,00084 | 0,0154 | Distal Intergenic | 260428 | ENSG00000237787 | C3orf79 |
| chrX | 42295427 | 42297757 | 4,71 | 0,44 | 4,27 | 0,000843 | 0,0154 | Distal Intergenic | 339729 | ENSG00000102055 | PPP1R2C |
| chr17 | 46709506 | 46709846 | 1,88 | -1,26 | 3,14 | 0,000846 | 0,0155 | Promoter (<=1kb) | 75 | ENSG00000210741 | MIR196A1 |
| chr7 | 20277581 | 20280516 | 5,07 | 1,67 | 3,41 | 0,000847 | 0,0155 | Distal Intergenic | -20568 | ENSG00000183742 | MACC1 |
| chr7 | 77669416 | 77670048 | 2,32 | -1,55 | 3,88 | 0,000848 | 0,0155 | Intron (uc003ugx.3/9863, intron 21 of 21) | 131306 | ENSG00000006576 | PHTF2 |
| chr3 | 41721105 | 41723219 | 4,31 | -0,06 | 4,37 | 0,000848 | 0,0155 | Exon (uc003ckv.4/54986, exon 29 of 37) | 280242 | ENSG00000168038 | ULK4 |
| chr18 | 26541787 | 26542295 | 2,44 | -1,55 | 3,99 | 0,000849 | 0,0155 | Distal Intergenic | -784342 | ENSG00000170558 | CDH2 |
| chr5 | 133330067 | 133330724 | 2,25 | -1,11 | 3,36 | 0,000851 | 0,0155 | Intron (uc003kvp.2/7416, intron 1 of 8) | 9933 | ENSG00000213585 | VDAC1 |
| chr2 | 198290596 | 198291532 | 2,21 | -1,55 | 3,77 | 0,000851 | 0,0155 | Intron (uc002uue.3/23451, intron 1 of 24) | 8239 | ENSG00000115524 | SF3B1 |
| chr11 | 115789845 | 115790737 | 2,3 | -1,55 | 3,85 | 0,000854 | 0,0155 | Distal Intergenic | -158500 | NA | LINC00900 |
| chr14 | 48064869 | 48065477 | 1,87 | -1,55 | 3,43 | 0,000856 | 0,0156 | Intron (uc001wwj.4/161357, intron 1 of 16) | 30251 | ENSG00000139915 | MDGA2 |
| chr2 | 169416889 | 169418955 | 4,11 | 0,1 | 4,01 | 0,000856 | 0,0156 | Exon (uc002ueb.2/253782, exon 3 of 10) | -20498 | ENSG00000265694 | MIR4774 |
| chr4 | 78513933 | 78514688 | 2,13 | -1,55 | 3,68 | 0,000859 | 0,0156 | Intron (uc003hkr.3/10563, intron 1 of 4) | 81026 | ENSG00000156234 | CXCL13 |
| chr14 | 97347738 | 97348827 | 2,85 | -0,72 | 3,57 | 0,00086 | 0,0156 | 3'UTR | 84054 | ENSG00000100749 | VRK1 |
| chr2 | 223291506 | 223293507 | 3,6 | -0,28 | 3,87 | 0,000862 | 0,0156 | Promoter (2-3kb) | 2184 | ENSG00000163082 | SGPP2 |
| chr2 | 85146613 | 85147929 | 3,41 | -0,63 | 4,05 | 0,000866 | 0,0157 | Distal Intergenic | 13850 | ENSG00000034510 | TMSB10 |
| chr5 | 91315194 | 91315969 | 2,82 | -0,94 | 3,76 | 0,000867 | 0,0157 | Distal Intergenic | -636045 | ENSG00000113369 | ARRDC3 |
| chr1 | 238416790 | 238417683 | 2,31 | -1,55 | 3,87 | 0,00087 | 0,0157 | Distal Intergenic | 231634 | ENSG00000215808 | LINC01139 |
| chr5 | 19796302 | 19797133 | 2,34 | -1,55 | 3,89 | 0,00087 | 0,0157 | Intron (uc021xwu.1/1016, intron 3 of 12) | 89248 | ENSG00000145526 | CDH18 |
| chr3 | 158756108 | 158756841 | 1,93 | -1,55 | 3,48 | 0,000872 | 0,0158 | Distal Intergenic | -30200 | ENSG00000283154 | IQCJ-SCFHP1 |
| chr17 | 54427810 | 54430108 | 5,09 | 0,76 | 4,33 | 0,000874 | 0,0158 | Exon (uc002iun.1/162282, exon 4 of 17) | 196974 | ENSG00000153930 | ANKFN1 |
| chr3 | 46339597 | 46341832 | 4,3 | 0,58 | 3,72 | 0,000876 | 0,0158 | Distal Intergenic | -53403 | ENSG00000121807 | CCR2 |
| chr3 | 89314817 | 89315782 | 3,91 | -0,18 | 4,09 | 0,000876 | 0,0158 | Intron (uc003dqx.1/2042, intron 3 of 6) | 158143 | ENSG00000044524 | EPHA3 |
| chr21 | 43648402 | 43649273 | 3,34 | 0,43 | 2,9 | 0,000877 | 0,0158 | Intron (uc002zam.3/9619, intron 4 of 16) | 8394 | ENSG00000160179 | ABCG1 |
| chr7 | 13950320 | 13951237 | 2,74 | -1,01 | 3,75 | 0,000878 | 0,0158 | Exon (uc021zzt.1/2115, exon 6 of 10) | 73908 | ENSG00000006468 | ETV1 |
| chr16 | 50045401 | 50046825 | 3,08 | -0,86 | 3,94 | 0,000878 | 0,0158 | Distal Intergenic | -12364 | ENSG00000205423 | CNEP1R1 |
| chr4 | 30786316 | 30787056 | 2,27 | -1,55 | 3,82 | 0,000879 | 0,0158 | Intron (uc011bxx.2/5099, intron 1 of 2) | 64279 | ENSG00000169851 | PCDH7 |
| chr11 | 86304136 | 86304859 | 2,66 | -0,97 | 3,63 | 0,000882 | 0,0159 | Intron (uc001pbz.3/10873, intron 1 of 13) | 78381 | ENSG00000151376 | ME3 |
| chr7 | 129338302 | 129339031 | 2,45 | -1,55 | 4 | 0,000885 | 0,0159 | Intron (uc003voz.3/4899, intron 5 of 10) | -57075 | NA | RNA5SP244 |
| chr6 | 35146666 | 35147682 | 3,09 | -0,55 | 3,64 | 0,000886 | 0,0159 | Distal Intergenic | -34508 | ENSG00000146197 | SCUBE3 |
| chr1 | 183846377 | 183846935 | 2,35 | -1,55 | 3,9 | 0,000886 | 0,0159 | Intron (uc010pof.1/23179, intron 2 of 11) | 68817 | ENSG00000198756 | COLGALT2 |
| chr4 | 132628869 | 132629609 | 2,9 | -0,25 | 3,15 | 0,000888 | 0,0159 | Distal Intergenic | -1440861 | ENSG00000138650 | PCDH10 |
| chr2 | 8892431 | 8893161 | 2,17 | -1,55 | 3,72 | 0,000888 | 0,0159 | Promoter (1-2kb) | -1966 | ENSG00000134313 | KIDINS220 |
| chr20 | 33059591 | 33060717 | 2,33 | -1,55 | 3,88 | 0,000888 | 0,0159 | Intron (uc002xak.3/83737, intron 16 of 24) | 5461 | ENSG00000207997 | MIR644A |
| chr11 | 23986237 | 23987767 | 2,79 | -1,26 | 4,05 | 0,000888 | 0,0159 | Distal Intergenic | -530749 | ENSG00000187398 | LUZP2 |
| chr4 | 186542494 | 186543337 | 3,41 | -0,28 | 3,68 | 0,000889 | 0,0159 | Intron (uc011cku.2/8470, intron 7 of 14) | 34786 | ENSG00000154556 | SORBS2 |
| chr3 | 165468117 | 165470141 | 4,31 | -0,06 | 4,37 | 0,00089 | 0,0159 | Distal Intergenic | 85112 | ENSG00000114200 | BCHE |
| chr15 | 51172180 | 51172743 | 1,76 | -1,55 | 3,31 | 0,000891 | 0,0159 | Distal Intergenic | -28126 | ENSG00000081014 | AP4E1 |
| chr4 | 69923071 | 69924018 | 3,68 | 0,17 | 3,52 | 0,000891 | 0,0159 | Distal Intergenic | -36958 | ENSG00000109181 | UGT2B10 |
| chr1 | 110692869 | 110693955 | 4,7 | 0,56 | 4,13 | 0,000891 | 0,0159 | Promoter (<=1kb) | 0 | ENSG00000197106 | SLC6A17 |
| chr9 | 34754243 | 34754760 | 2,07 | -1,06 | 3,13 | 0,000892 | 0,0159 | Distal Intergenic | -24708 | ENSG00000205108 | FAM205A |
| chr10 | 55442286 | 55442797 | 2,04 | -1,55 | 3,59 | 0,000895 | 0,016 | Distal Intergenic | -910826 | ENSG00000165471 | MBL2 |
| chr8 | 126285092 | 126286387 | 3,37 | -0,26 | 3,63 | 0,000895 | 0,016 | Intron (uc003vrw.2/286053, intron 5 of 7) | -156176 | ENSG00000173334 | TRIB 1 |
| chr4 | 97924839 | 97925654 | 2,72 | -1,01 | 3,72 | 0,000895 | 0,016 | Distal Intergenic | -362423 | ENSG00000251620 | STPG2-AS1 |
| chr19 | 41974940 | 41975799 | 2,22 | -1,55 | 3,77 | 0,000895 | 0,016 | Intron (uc002orb.4/100505495, intron 3 of 3) | 25877 | ENSG00000204978 | ERICH4 |
| chr3 | 171859294 | 171860210 | 2,27 | -1,55 | 3,82 | 0,000897 | 0,016 | Intron (uc003fhx.3/64778, intron 3 of 3) | 8033 | ENSG00000075420 | FNDC3B |
| chr4 | 190689108 | 190690630 | 4,48 | 0,1 | 4,38 | 0,000901 | 0,0161 | Distal Intergenic | -171344 | ENSG00000109536 | FRG1 |
| chr20 | 8374659 | 8375400 | 1,92 | -1,55 | 3,47 | 0,000904 | 0,0161 | Intron (uc010zrb.1/23236, intron 3 of 27) | -205981 | ENSG00000182621 | PLCB1 |
| chr19 | 41476553 | 41477100 | 1,86 | -1,55 | 3,41 | 0,000905 | 0,0161 | Intron (uc002opo.3/1549, intron 1 of 8) | -20104 | ENSG00000197408 | CYP2B6 |
| chr2 | 142567738 | 142568247 | 1,91 | -1,55 | 3,46 | 0,000906 | 0,0161 | Exon (uc002tvj.1/53353, exon 2 of 91) | 321023 | ENSG00000168702 | LRP1B |
| chr9 | 98336255 | 98337500 | 4,4 | 1,42 | 2,98 | 0,000907 | 0,0161 | Distal Intergenic | -57008 | ENSG00000185920 | PTCH1 |
| chr10 | 112009542 | 112010354 | 2,06 | -1,55 | 3,61 | 0,000907 | 0,0161 | Intron (uc001kyy.3/4601, intron 3 of 5) | 22301 | ENSG00000119950 | MXI1 |
| chr5 | 24949313 | 24949899 | 2,33 | -1,55 | 3,88 | 0,00091 | 0,0162 | Distal Intergenic | -108621 | ENSG00000248908 | LINC02239 |
| chr8 | 88556302 | 88556844 | 2,46 | -1,55 | 4,01 | 0,00091 | 0,0162 | Distal Intergenic | 329452 | ENSG00000176566 | DCAF4L2 |
| chr1 | 201088393 | 201088833 | 1,97 | -1,38 | 3,36 | 0,000911 | 0,0162 | Distal Intergenic | -3893 | ENSG00000232237 | ASCL5 |
| chr2 | 144602562 | 144603103 | 2 | -1,55 | 3,55 | 0,000912 | 0,0162 | Distal Intergenic | 366043 | ENSG00000121964 | GTDC1 |
| chr2 | 152827929 | 152828754 | 2,52 | -1,4 | 3,92 | 0,000914 | 0,0162 | Promoter (1-2kb) | 1731 | ENSG00000182389 | CACNB4 |
| chr7 | 122870163 | 122871091 | 3,44 | -0,36 | 3,8 | 0,000916 | 0,0162 | Distal Intergenic | -30138 | ENSG00000081800 | SLC13 A1 |
| chr19 | 54464133 | 54466867 | 5,81 | 1,7 | 4,11 | 0,000917 | 0,0163 | Promoter (<=1kb) | 0 | ENSG00000142408 | CACNG8 |
| chr16 | 69477979 | 69479012 | 2,7 | -0,87 | 3,56 | 0,000921 | 0,0163 | Intron (uc002exf.3/80777, intron 1 of 2) | 19481 | ENSG00000103018 | CYB5B |
| chr15 | 60841778 | 60842613 | 2,25 | -1,55 | 3,81 | 0,000922 | 0,0163 | Intron (uc002agt.4/6095, intron 1 of 9) | 42094 | ENSG00000069667 | RORA |
| chr3 | 176778179 | 176780050 | 3,47 | -0,26 | 3,74 | 0,000924 | 0,0163 | Intron (uc003fiw.4/79718, intron 3 of 15) | 134216 | ENSG00000177565 | TBL1XR1 |
| chr2 | 17579368 | 17579936 | 2,04 | -1,55 | 3,59 | 0,000925 | 0,0164 | Distal Intergenic | 119770 | ENSG00000214842 | RAD51AP2 |
| chr1 | 12493568 | 12494389 | 2,48 | -0,67 | 3,15 | 0,000927 | 0,0164 | Intron (uc001atv.3/55187, intron 65 of 69) | 23684 | ENSG00000048707 | VPS13D |
| chr6 | 4826001 | 4826805 | 2,25 | -1,55 | 3,8 | 0,000933 | 0,0165 | Intron (uc003mwi.3/9425, intron 3 of 8) | -9527 | ENSG00000153046 | CDYL |
| chr1 | 75539297 | 75541530 | 3,97 | -0,21 | 4,18 | 0,000933 | 0,0165 | Distal Intergenic | -52589 | ENSG00000162624 | LHX8 |
| chr4 | 87052006 | 87052641 | 2,21 | -1,55 | 3,76 | 0,000938 | 0,0165 | Intron (uc003hpw.3/uc003hpw.3, intron 3 of 3) | -23200 | ENSG00000109339 | MAPK10 |
| chr4 | 168543676 | 168544380 | 2,46 | -1,26 | 3,72 | 0,000939 | 0,0165 | Distal Intergenic | -387935 | ENSG00000196104 | SPOCK3 |
| chr11 | 121520934 | 121522390 | 3,56 | -0,31 | 3,87 | 0,000942 | 0,0166 | Distal Intergenic | 59806 | ENSG00000137642 | SORL1 |
| chr3 | 121373536 | 121374165 | 1,86 | -1,55 | 3,41 | 0,000944 | 0,0166 | Intron (uc003eeh.4/3059, intron 3 of 13) | 5626 | ENSG00000180353 | HCLS1 |
| chr2 | 194780751 | 194781956 | 2,34 | -1,55 | 3,89 | 0,000945 | 0,0166 | Distal Intergenic | 1166180 | ENSG00000227418 | PCGEM1 |
| chr4 | 31581129 | 31581859 | 2,34 | -1,55 | 3,9 | 0,000946 | 0,0166 | Distal Intergenic | 859092 | ENSG00000169851 | PCDH7 |
| chr10 | 64216634 | 64216998 | 1,41 | -1,55 | 2,96 | 0,000947 | 0,0166 | Intron (uc001jmb.4/22891, intron 3 of 4) | -63209 | ENSG00000138311 | ZNF365 |
| chr4 | 168012918 | 168013612 | 2,11 | -1,55 | 3,66 | 0,000948 | 0,0167 | Intron (uc021xuf.1/50859, intron 1 of 10) | 141712 | ENSG00000196104 | SPOCK3 |
| chr4 | 174881537 | 174882435 | 2,12 | -1,55 | 3,68 | 0,000948 | 0,0167 | Distal Intergenic | -322393 | ENSG00000164118 | CEP44 |
| chr1 | 92646698 | 92647962 | 3,11 | -0,6 | 3,71 | 0,000951 | 0,0167 | Exon (uc010otd.2/23285, exon 8 of 9) | 12283 | ENSG00000189195 | KIAA1107 |
| chr11 | 99552980 | 99553693 | 2,32 | -1,55 | 3,88 | 0,000951 | 0,0167 | Intron (uc009ywv.2/53942, intron 3 of 15) | 126104 | ENSG00000149972 | CNTN5 |
| chr2 | 183748406 | 183749113 | 2,27 | -1,55 | 3,82 | 0,000955 | 0,0167 | Distal Intergenic | -16908 | ENSGO0000162998 | FRZB |
| chr6 | 4928920 | 4929507 | 2,09 | -1,55 | 3,64 | 0,000957 | 0,0168 | Intron (uc003mwi.3/9425, intron 4 of 8) | 38694 | ENSG00000153046 | CDYL |
| chr10 | 78957190 | 78957996 | 2,67 | -0,51 | 3,17 | 0,00096 | 0,0168 | Intron (uc001jxj.2/3778, intron 3 of 28) | -13900 | ENSG00000156113 | KCNMA1 |
| chr5 | 164627969 | 164629365 | 2,67 | -1,55 | 4,22 | 0,000963 | 0,0168 | Distal Intergenic | 1683506 | ENSG0000003 8274 | MAT2B |
| chr14 | 63977531 | 63978277 | 2,28 | -1,23 | 3,51 | 0,000966 | 0,0169 | Promoter (2-3kb) | -2575 | ENSG00000154001 | PPP2R5E |
| chr11 | 75619909 | 75620430 | 1,87 | -1,55 | 3,42 | 0,000967 | 0,0169 | Intron (uc001oxc.3/7405, intron 5 of 14) | -21474 | ENSG00000198382 | UVRAG |
| chr6 | 72112996 | 72113542 | 1,99 | -1,55 | 3,54 | 0,00097 | 0,0169 | Promoter (<=1kb) | 0 | ENSG00000207827 | MIR30A |
| chr5 | 157252224 | 157253272 | 2,74 | -1,11 | 3,85 | 0,00097 | 0,0169 | Intron (uc003lxi.2/9685, intron 1 of 11) | -29071 | ENSG00000113282 | CLINT1 |
| chr4 | 46925760 | 46927039 | 2,29 | -1,55 | 3,84 | 0,000972 | 0,017 | 3'UTR | -14508 | ENSG00000170516 | COX7B2 |
| chr8 | 110848439 | 110849444 | 2,31 | -1,55 | 3,86 | 0,000973 | 0,017 | Distal Intergenic | 137977 | ENSG00000164794 | KCNV1 |
| chr7 | 115112916 | 115113895 | 2,32 | -1,55 | 3,87 | 0,00098 | 0,0171 | Distal Intergenic | 494472 | ENSG00000105967 | TFEC |
| chr2 | 102238817 | 102239718 | 3,11 | -0,26 | 3,37 | 0,000981 | 0,0171 | Distal Intergenic | -74447 | ENSG00000071054 | MAP4K4 |
| chr4 | 31026287 | 31027122 | 2,35 | -1,55 | 3,9 | 0,000981 | 0,0171 | Intron (uc011bxx.2/5099, intron 2 of 2) | 304250 | ENSG00000169851 | PCDH7 |
| chr13 | 107118637 | 107119427 | 2,33 | -1,12 | 3,46 | 0,000983 | 0,0171 | Distal Intergenic | 67961 | ENSG00000125266 | EFNB2 |
| chr2 | 192647074 | 192647492 | 2,56 | -0,75 | 3,31 | 0,000984 | 0,0171 | Distal Intergenic | 64514 | ENSG00000168497 | CAVIN2 |
| chr18 | 27665992 | 27666766 | 1,94 | -1,55 | 3,49 | 0,000986 | 0,0171 | Distal Intergenic | -212110 | ENSG00000283218 | MIR302F |
| chr5 | 26237236 | 26237946 | 2,54 | -1,26 | 3,8 | 0,000989 | 0,0172 | Distal Intergenic | 652996 | ENSG00000113100 | CDH9 |
| chr3 | 6132745 | 6133459 | 2,22 | -1,55 | 3,77 | 0,00099 | 0,0172 | Distal Intergenic | -769343 | ENSG00000196277 | GRM7 |
| chr4 | 48263165 | 48263804 | 2,05 | -1,55 | 3,6 | 0,000994 | 0,0172 | Intron (uc003gxz.3/7006, intron 1 of 17) | 8010 | ENSG00000135605 | TEC |
| chr6 | 52740850 | 52741373 | 2,26 | -0,8 | 3,06 | 0,001 | 0,0173 | Distal Intergenic | -29957 | ENSG00000182793 | GSTA5 |
| chr5 | 125570418 | 125570784 | 2,1 | -1,01 | 3,11 | 0,001 | 0,0173 | Distal Intergenic | -125004 | ENSG00000155324 | GRAMD2B |
| chr8 | 56841575 | 56842365 | 1,89 | -1,55 | 3,44 | 0,001 | 0,0174 | Intron (uc003xsk.4/4067, intron 1 of 12) | 49189 | ENSG00000254087 | LYN |
| chr1 | 171255247 | 171255762 | 1,84 | -1,55 | 3,39 | 0,00101 | 0,0174 | Distal Intergenic | -27724 | ENSG00000076258 | FMO4 |
| chr18 | 72654280 | 72654775 | 2,05 | -1,55 | 3,6 | 0,00101 | 0,0174 | Intron (uc002llw.2/55628, intron 7 of 7) | 262693 | ENSG00000180011 | ZADH2 |
| chr5 | 93010350 | 93011642 | 3,52 | -0,13 | 3,65 | 0,00101 | 0,0174 | Intron (uc010jbd.3/83989, intron 10 of 10) | -53856 | ENSG00000284336 | MIR2277 |
| chr4 | 189888283 | 189889293 | 2,36 | -1,55 | 3,91 | 0,00101 | 0,0174 | Distal Intergenic | 511551 | ENSG00000249378 | LINC01060 |
| chr3 | 29977956 | 29978802 | 2,27 | -1,55 | 3,83 | 0,00101 | 0,0175 | Intron (uc003cek.3/27303, intron 11 of 12) | 655153 | ENSG00000144642 | RBMS3 |
| chr5 | 90656758 | 90657210 | 1,73 | -1,55 | 3,28 | 0,00102 | 0,0175 | Distal Intergenic | -18954 | ENSG00000281357 | ARRDC3-AS1 |
| chr11 | 47376544 | 47377260 | 2,06 | -1,55 | 3,61 | 0,00102 | 0,0175 | Promoter (<=1kb) | 0 | ENSG00000165915 | SLC39A13 |
| chr6 | 98832565 | 98833384 | 2,36 | -1,55 | 3,92 | 0,00102 | 0,0175 | Distal Intergenic | 360158 | ENSG00000238367 | MIR2113 |
| chr2 | 183958777 | 183959403 | 2,1 | -1,55 | 3,65 | 0,00102 | 0,0176 | Intron (uc002upd.3/142679, intron 3 of 3) | 15490 | ENSG00000162999 | DUSP19 |
| chr6 | 105554058 | 105554633 | 2,25 | -1,55 | 3,8 | 0,00103 | 0,0177 | Intron (uc003pqw.3/11149, intron 7 of 7) | 29588 | ENSG00000112276 | BVES |
| chr11 | 75619253 | 75619685 | 1,83 | -1,55 | 3,38 | 0,00104 | 0,0178 | Intron (uc001oxc.3/7405, intron 5 of 14) | -22219 | ENSG00000198382 | UVRAG |
| chr16 | 87490404 | 87492859 | 3,95 | 0,1 | 3,85 | 0,00104 | 0,0178 | Intron (uc002fjz.1/23174, intron 2 of 12) | 32601 | ENSG00000140948 | ZCCHC14 |
| chr10 | 21483141 | 21484017 | 2,31 | -1,55 | 3,87 | 0,00104 | 0,0179 | Distal Intergenic | 19910 | ENSG00000231920 | NEBL-AS1 |
| chr18 | 45651068 | 45651706 | 1,73 | -1,55 | 3,29 | 0,00105 | 0,0179 | Intron (uc002ldb.3/201501, intron 1 of 2) | 11974 | ENSG00000184828 | ZBTB7C |
| chr10 | 112533333 | 112533876 | 2,1 | -1,55 | 3,66 | 0,00105 | 0,0179 | Intron (uc001kzf.2/282996, intron 1 of 13) | 96786 | NA | PDCD4-AS1 |
| chr22 | 18428053 | 18429540 | 3,13 | -0,58 | 3,7 | 0,00105 | 0,0179 | Intron (uc002zng.4/57553, intron 1 of 31) | 34187 | ENSG00000207780 | MIR648 |
| chr13 | 97991924 | 97993045 | 2,72 | -1,11 | 3,83 | 0,00105 | 0,0179 | Intron (uc031qmz.1/10150, intron 4 of 7) | -6250 | ENSG00000139793 | MBNL2 |
| chr11 | 27903397 | 27904187 | 2,1 | -1,55 | 3,65 | 0,00105 | 0,018 | Distal Intergenic | -159792 | ENSG00000176697 | BDNF |
| chr5 | 153939074 | 153940074 | 2,15 | -1,55 | 3,71 | 0,00105 | 0,018 | Distal Intergenic | 35558 | ENSG00000264760 | MIR3141 |
| chr16 | 18107482 | 18108544 | 2,45 | -1,38 | 3,84 | 0,00105 | 0,018 | Distal Intergenic | 387584 | ENSG00000266291 | MIR3180-3 |
| chr2 | 222945252 | 222946090 | 2,29 | -1,55 | 3,85 | 0,00105 | 0,018 | Distal Intergenic | -216776 | ENSG00000163081 | CCDC140 |
| chr11 | 69450472 | 69469775 | 11,15 | 9 | 2,15 | 0,00106 | 0,018 | Promoter (<=1kb) | 0 | ENSG00000110092 | CCND1 |
| chr4 | 87464650 | 87466899 | 4 | 0,31 | 3,69 | 0,00106 | 0,018 | Intron (uc010ikh.1/5602, intron 1 of 6) | 48364 | ENSG00000109339 | MAPK10 |
| chr3 | 165536084 | 165537032 | 2,4 | -1,55 | 3,95 | 0,00106 | 0,018 | Intron (uc003fem.4/590, intron 2 of 3) | 18221 | ENSG00000114200 | BCHE |
| chr15 | 75400410 | 75402255 | 3,97 | 0,37 | 3,6 | 0,00106 | 0,0181 | Distal Intergenic | 84483 | ENSG00000138621 | PPCDC |
| chr3 | 173391489 | 173392161 | 2,13 | -1,55 | 3,68 | 0,00107 | 0,0181 | Intron (uc003fio.1/22871, intron 3 of 6) | 69420 | ENSG00000169760 | NLGN1 |
| chr7 | 106526535 | 106527057 | 2,18 | -1,55 | 3,73 | 0,00107 | 0,0182 | Exon (uc003vdv.4/5294, exon 10 of 11) | 20611 | ENSG00000105851 | PIK3 CG |
| chr3 | 89268912 | 89271260 | 4,55 | 0,19 | 4,36 | 0,00107 | 0,0182 | Intron (uc003dqx.1/2042, intron 3 of 6) | 112238 | ENSG00000044524 | EPHA3 |
| chr5 | 75994909 | 75995508 | 2,05 | -1,55 | 3,6 | 0,00107 | 0,0183 | Intron (uc003kek.3/10788, intron 33 of 35) | -12720 | NA | NCRUPAR |
| chr17 | 57564569 | 57565197 | 3,26 | -0,04 | 3,29 | 0,00108 | 0,0183 | Distal Intergenic | -77689 | ENSG00000108406 | DHX40 |
| chr4 | 156640813 | 156641410 | 2,12 | -1,55 | 3,67 | 0,00108 | 0,0183 | Intron (uc010iqc.3/2982, intron 8 of 8) | -38716 | ENSG00000061918 | GUCY1B1 |
| chr14 | 93048830 | 93049564 | 2,25 | -1,55 | 3,8 | 0,00108 | 0,0183 | Intron (uc001vap.3/79890, intron 3 of 9) | 6251 | ENSG00000100599 | RIN3 |
| chr14 | 75301822 | 75302487 | 2,17 | -1,38 | 3,56 | 0,00108 | 0,0184 | 3'UTR | 25739 | ENSG00000119596 | YLPM1 |
| chr12 | 8796785 | 8797629 | 2,35 | -0,86 | 3,2 | 0,00109 | 0,0184 | Downstream (<1kb) | 17804 | ENSG00000197614 | MFAP5 |
| chr5 | 117299787 | 117300664 | 2,48 | -1,26 | 3,74 | 0,00109 | 0,0184 | Distal Intergenic | 548579 | NA | LINC00992 |
| chr12 | 43072719 | 43073682 | 3,17 | -0,18 | 3,35 | 0,00109 | 0,0185 | Distal Intergenic | -89147 | ENSG00000139174 | PRICKLE1 |
| chr1 | 112899266 | 112899910 | 2,03 | -1,55 | 3,58 | 0,00109 | 0,0185 | Distal Intergenic | -38890 | ENSG00000143079 | CTTNBP2NL |
| chr9 | 12697186 | 12698351 | 2,75 | -0,95 | 3,7 | 0,00109 | 0,0185 | Intron (uc003zkv.4/7306, intron 3 of 7) | 3800 | ENSG00000107165 | TYRP1 |
| chr5 | 65249584 | 65250152 | 2,29 | -1,55 | 3,84 | 0,00109 | 0,0185 | Intron (uc003jui.2/55914, intron 1 of 24) | -8183 | NA | LOC100303749 |
| chr17 | 6353853 | 6355889 | 3,86 | -0,08 | 3,94 | 0,00109 | 0,0185 | 3'UTR | 6118 | ENSG00000129195 | PIMREG |
| chr11 | 2643311 | 2643930 | 2,13 | -1,14 | 3,27 | 0,0011 | 0,0185 | Exon (uc001lwp.3/10984, exon 1 of 1) | 77298 | ENSG00000269821 | KCNQ1OT1 |
| chr4 | 80447971 | 80448834 | 2,36 | -1,29 | 3,65 | 0,0011 | 0,0185 | Intron (uc003hlv.3/100506035, intron 3 of 3) | 34224 | ENSG00000250334 | LINC00989 |
| chr5 | 27826212 | 27826958 | 2,26 | -1,55 | 3,81 | 0,0011 | 0,0185 | Distal Intergenic | 353813 | ENSG00000250337 | PURPL |
| chr21 | 40140205 | 40140817 | 2,5 | -0,86 | 3,36 | 0,0011 | 0,0186 | Promoter (<=1kb) | 81 | ENSG00000223806 | LINC00114 |
| chr16 | 62512982 | 62513736 | 2,12 | -1,55 | 3,67 | 0,0011 | 0,0186 | Distal Intergenic | -442243 | ENSG00000150394 | CDH8 |
| chr11 | 38777678 | 38778354 | 2,7 | -0,97 | 3,68 | 0,00111 | 0,0186 | Distal Intergenic | 1536326 | ENSG00000148948 | LRRC4C |
| chr4 | 181263924 | 181264622 | 2,55 | -0,48 | 3,03 | 0,00111 | 0,0187 | Distal Intergenic | 815680 | ENSG00000248197 | LINC00290 |
| chr4 | 47671028 | 47671342 | 1,58 | -1,55 | 3,13 | 0,00111 | 0,0187 | Intron (uc011bzf.2/10699, intron 9 of 20) | 120935 | ENSG00000145244 | CORIN |
| chr11 | 99396790 | 99397446 | 1,86 | -1,55 | 3,42 | 0,00111 | 0,0187 | Intron (uc009ywv.2/53942, intron 2 of 15) | -29430 | ENSG00000149972 | CNTN5 |
| chr8 | 117023858 | 117024544 | 2,75 | -0,8 | 3,56 | 0,00111 | 0,0187 | Intron (uc031tcc.1/100859921, intron 7 of 13) | 312753 | ENSG00000249917 | LINC00536 |
| chr7 | 23413026 | 23413594 | 1,83 | -1,55 | 3,38 | 0,00112 | 0,0188 | Intron (uc003swg.3/10643, intron 3 of 14) | -25673 | ENSG00000136231 | IGF2BP3 |
| chr19 | 48589415 | 48589939 | 2,43 | -1,01 | 3,45 | 0,00112 | 0,0188 | Intron (uc002phw.3/8605, intron 7 of 14) | 18769 | ENSG00000105499 | PLA2G4C |
| chr8 | 91243244 | 91243670 | 1,93 | -1,55 | 3,49 | 0,00113 | 0,0189 | Intron (uc031tbq.1/100874052, intron 1 of 3) | 9528 | ENSG00000253394 | LINC00534 |
| chr4 | 87215864 | 87216572 | 2,27 | -1,23 | 3,5 | 0,00113 | 0,0189 | Intron (uc010ikg.3/5602, intron 2 of 13) | 63451 | ENSG00000109339 | MAPK10 |
| chr14 | 72030309 | 72031010 | 2,06 | -1,55 | 3,61 | 0,00113 | 0,0189 | Intron (uc001xmr.1/26037, intron 3 of 3) | -21988 | ENSG00000197555 | SIPA1L1 |
| chr8 | 116895283 | 116896172 | 2,65 | -1,14 | 3,79 | 0,00113 | 0,0189 | Distal Intergenic | -214055 | ENSG00000104447 | TRPS1 |
| chr9 | 14325937 | 14326497 | 2,28 | -0,93 | 3,21 | 0,00114 | 0,019 | Intron (uc022bdp.1/4781, intron 1 of 8) | -11892 | ENSG00000147862 | NFIB |
| chr6 | 32631083 | 32635622 | 7,04 | 3,76 | 3,28 | 0,00114 | 0,019 | Promoter (<=1kb) | 0 | ENSG00000179344 | HLA-DQB1 |
| chr13 | 106839539 | 106840150 | 2,7 | -0,77 | 3,47 | 0,00114 | 0,019 | Distal Intergenic | -188761 | NA | LINC00460 |
| chr2 | 207321168 | 207321903 | 2,01 | -1,55 | 3,56 | 0,00114 | 0,019 | Intron (uc002vbq.4/8745, intron 2 of 25) | 11137 | ENSG00000114948 | ADAM23 |
| chr7 | 29310880 | 29311252 | 1,64 | -1,55 | 3,19 | 0,00114 | 0,0191 | Intron (uc011jzs.2/1124,intron 2 of 13) | 73523 | ENSG00000106069 | CHN2 |
| chr7 | 51920859 | 51921389 | 1,8 | -1,55 | 3,35 | 0,00114 | 0,0191 | Distal Intergenic | -536344 | ENSG00000106078 | COBL |
| chr4 | 105830037 | 105830718 | 2,37 | -1,55 | 3,92 | 0,00114 | 0,0191 | Distal Intergenic | -236314 | ENSG00000168769 | TET2 |
| chrX | 108947477 | 108947907 | 1,82 | -1,01 | 2,83 | 0,00115 | 0,0191 | Intron (uc004eoi.2/2182, intron 1 of 16) | 28714 | ENSG00000068366 | ACSL4 |
| chr4 | 116926127 | 116926561 | 1,92 | -1,38 | 3,31 | 0,00115 | 0,0191 | Distal Intergenic | -294320 | ENSG00000284253 | MIR1973 |
| chrX | 84110413 | 84111243 | 2,17 | -1,55 | 3,72 | 0,00115 | 0,0191 | Distal Intergenic | -77914 | ENSG00000229547 | UBE2DNL |
| chr22 | 26443803 | 26444534 | 2,19 | -1,55 | 3,74 | 0,00115 | 0,0191 | Distal Intergenic | 21392 | ENSG00000133454 | MYO18B |
| chr7 | 122715776 | 122716408 | 2,06 | -1,55 | 3,61 | 0,00115 | 0,0192 | Distal Intergenic | -80022 | ENSG00000128519 | TAS2R16 |
| chr3 | 72475242 | 72475946 | 2,36 | -0,59 | 2,95 | 0,00116 | 0,0192 | Intron (uc003dpe.3/23429, intron 1 of 3) | 19828 | ENSG00000163602 | RYBP |
| chr5 | 173845854 | 173846867 | 4,31 | 0,62 | 3,69 | 0,00116 | 0,0192 | Distal Intergenic | -304708 | ENSG00000120149 | MSX2 |
| chr8 | 128784810 | 128785615 | 4,19 | 0,08 | 4,1 | 0,00116 | 0,0192 | Distal Intergenic | -21188 | ENSG00000249859 | PVT1 |
| chr4 | 129434901 | 129435565 | 2,5 | -0,5 | 3 | 0,00116 | 0,0193 | Intron (uc003igh.1/uc003igh.1, intron 7 of 8) | -224917 | ENSG00000164040 | PGRMC2 |
| chr3 | 45906386 | 45906822 | 1,63 | -1,55 | 3,18 | 0,00116 | 0,0193 | Intron (uc003cov.2/54585, intron 2 of 11) | -21174 | ENSG00000173585 | CCR9 |
| chr20 | 43656993 | 43657671 | 1,93 | -1,55 | 3,48 | 0,00116 | 0,0193 | Intron (uc002xnb.3/6789, intron 10 of 10) | 61873 | ENSG00000101109 | STK4 |
| chr22 | 28288739 | 28289754 | 2,24 | -1,55 | 3,79 | 0,00116 | 0,0193 | Intron (uc011akh.2/23760, intron 7 of 11) | 25433 | ENSG00000180957 | PITPNB |
| chr2 | 184860461 | 184871380 | 8,74 | 3,94 | 4,8 | 0,00116 | 0,0193 | Distal Intergenic | -591713 | ENSG00000170396 | ZNF804A |
| chr5 | 111744912 | 111745543 | 1,91 | -1,55 | 3,46 | 0,00117 | 0,0193 | Intron (uc003kpv.1/64097, intron 1 of 22) | 9467 | ENSG00000129595 | EPB41L4A |
| chr3 | 186847851 | 186848975 | 2,19 | -1,55 | 3,75 | 0,00117 | 0,0193 | Intron (uc003fre.1/116832, intron 1 of 2) | 8288 | ENSG00000163923 | RPL39L |
| chr6 | 76122458 | 76123349 | 2,01 | -1,55 | 3,56 | 0,00117 | 0,0194 | Intron (uc003phy.1/27145, intron 2 of 6) | -49739 | ENSG00000118407 | FILIP1 |
| chr3 | 32138875 | 32140176 | 2,26 | -1,55 | 3,81 | 0,00117 | 0,0194 | Distal Intergenic | -7827 | ENSG00000152642 | GPD1L |
| chr10 | 28015523 | 28016202 | 2,44 | -1,26 | 3,7 | 0,00118 | 0,0194 | Intron (uc001itx.4/283078, intron 5 of 6) | 18576 | ENSG00000150051 | MKX |
| chr1 | 16291801 | 16293475 | 4,17 | 0,41 | 3,76 | 0,00118 | 0,0194 | Intron (uc001axl.4/7709, intron 2 of 15) | 9152 | ENSG00000116809 | ZBTB17 |
| chr2 | 175353958 | 175355761 | 5,71 | 2,92 | 2,79 | 0,00118 | 0,0195 | Promoter (2-3kb) | -2142 | ENSG00000163328 | GPR155 |
| chr7 | 3236572 | 3237237 | 2,38 | -0,61 | 2,99 | 0,00118 | 0,0195 | Distal Intergenic | -103843 | ENSG00000146555 | SDK1 |
| chr13 | 31340994 | 31341853 | 1,73 | -1,55 | 3,28 | 0,00118 | 0,0195 | Distal Intergenic | 31349 | ENSG00000132965 | ALOX5 AP |
| chr4 | 156034131 | 156034976 | 2,82 | -0,8 | 3,62 | 0,00118 | 0,0195 | Distal Intergenic | -94805 | ENSG00000185149 | NPY2R |
| chr4 | 72214131 | 72214984 | 2,17 | -1,55 | 3,72 | 0,00118 | 0,0195 | Intron (uc003hfy.3/8671, intron 4 of 25) | 9361 | ENSG00000080493 | SLC4A4 |
| chr3 | 31294107 | 31294992 | 2,2 | -1,55 | 3,75 | 0,00118 | 0,0195 | Distal Intergenic | -279499 | ENSG00000163527 | STT3B |
| chr3 | 165935945 | 165937033 | 2,26 | -1,55 | 3,81 | 0,00118 | 0,0195 | Distal Intergenic | -380692 | ENSG00000114200 | BCHE |
| chr3 | 70560122 | 70560803 | 2,34 | -1,55 | 3,89 | 0,00118 | 0,0195 | Distal Intergenic | 529880 | ENSG00000114861 | FOXP1 |
| chr16 | 87106941 | 87109223 | 5,44 | 2,08 | 3,36 | 0,00119 | 0,0195 | Distal Intergenic | 241803 | ENSG00000260456 | C16orf95 |
| chr1 | 195305736 | 195306238 | 1,93 | -1,55 | 3,48 | 0,00119 | 0,0195 | Distal Intergenic | 1005141 | ENSG00000162687 | KCNT2 |
| chr8 | 22696651 | 22697085 | 2,11 | -0,7 | 2,82 | 0,00119 | 0,0196 | Intron (uc003xcn.1/157310, intron 3 of 6) | 88336 | ENSG00000134020 | PEBP4 |
| chr2 | 184894603 | 184895271 | 2,52 | -1,26 | 3,78 | 0,00119 | 0,0196 | Distal Intergenic | -567822 | ENSG00000170396 | ZNF804A |
| chr6 | 31538900 | 31540952 | 6,68 | 3,29 | 3,4 | 0,0012 | 0,0197 | Promoter (<=1kb) | 0 | ENSGO0000226979 | LTA |
| chr11 | 39240031 | 39240766 | 2,16 | -1,55 | 3,71 | 0,0012 | 0,0197 | Distal Intergenic | 1073914 | ENSG00000148948 | LRRC4C |
| chr6 | 67938234 | 67938815 | 2,21 | -1,55 | 3,76 | 0,0012 | 0,0197 | Distal Intergenic | -1406817 | ENSG00000135298 | ADGRB3 |
| chr7 | 84965988 | 84967133 | 2,92 | -0,87 | 3,78 | 0,0012 | 0,0197 | Distal Intergenic | -149817 | ENSG00000153993 | SEMA3D |
| chr3 | 16464938 | 16466454 | 3,48 | -0,54 | 4,02 | 0,0012 | 0,0197 | Intron (uc010hes.3/23180, intron 2 of 8) | 57918 | ENSG00000131378 | RFTN1 |
| chr16 | 82768994 | 82769988 | 2,09 | -1,01 | 3,1 | 0,00121 | 0,0198 | Intron (uc010chh.3/1012, intron 1 of 4) | 108595 | ENSG00000140945 | CDH13 |
| chr1 1 | 47081821 | 47082507 | 2,19 | -1,31 | 3,5 | 0,00121 | 0,0198 | Intron (uc001ndp.4/79096, intron 3 of 8) | 112884 | ENSG00000149182 | ARFGAP2 |
| chr13 | 31348559 | 31349054 | 2,21 | -1,14 | 3,34 | 0,00122 | 0,0199 | Distal Intergenic | -28289 | ENSG00000237879 | LINC00398 |
| chr8 | 83090062 | 83090878 | 2,78 | -1,01 | 3,8 | 0,00122 | 0,0199 | Distal Intergenic | -335541 | ENSG00000104497 | SNX16 |
| chr6 | 68269659 | 68270282 | 2,66 | -0,65 | 3,31 | 0,00122 | 0,02 | Distal Intergenic | -1075350 | ENSG00000135298 | ADGRB3 |
| chr4 | 112125932 | 112126531 | 1,81 | -1,55 | 3,36 | 0,00122 | 0,02 | Distal Intergenic | -562653 | ENSG00000164093 | PITX2 |
| chr4 | 96517913 | 96518593 | 1,97 | -1,55 | 3,52 | 0,00122 | 0,02 | Distal Intergenic | -47552 | ENSG00000182168 | UNC5 C |
| chr19 | 6947782 | 6948484 | 2 | -1,4 | 3,4 | 0,00123 | 0,02 | Distal Intergenic | 37938 | ENSG00000268758 | ADGRE4 P |
| chr2 | 7693032 | 7693668 | 2,08 | -1,55 | 3,64 | 0,00123 | 0,02 | Distal Intergenic | 131640 | ENSG00000229727 | LOC100506274 |
| chr15 | 94816087 | 94816972 | 2,17 | -1,55 | 3,72 | 0,00123 | 0,02 | Intron (uc002btg.4/55784, intron 1 of 7) | -24458 | ENSG00000140563 | MCTP2 |
| chr1 1 | 99209955 | 99211508 | 4,51 | 0,41 | 4,1 | 0,00123 | 0,02 | Intron (uc009ywv.2/53942, intron 2 of 15) | -215368 | ENSG00000149972 | CNTN5 |
| chr4 | 98297079 | 98297555 | 1,66 | -1,55 | 3,21 | 0,00123 | 0,0201 | Intron (uc031sgo.1/101410545, intron 1 of 5) | 9002 | ENSG00000251620 | STPG2-AS1 |
| chr4 | 143487961 | 143488707 | 1,96 | -1,55 | 3,51 | 0,00123 | 0,0201 | Intron (uc003iiw.4/8821, intron 2 of 25) | -52513 | ENSG00000109452 | INPP4B |
| chr10 | 52186086 | 52186527 | 1,46 | -1,55 | 3,01 | 0,00124 | 0,0202 | Intron (uc001jje.3/259230, intron 6 of 10) | -8146 | ENSG00000198964 | SGMS1 |
| chr6 | 156982885 | 156983616 | 2,95 | -0,62 | 3,57 | 0,00124 | 0,0202 | Distal Intergenic | -115448 | ENSG00000049618 | ARID1B |
| chr1 | 178065951 | 178068684 | 4,9 | 0,6 | 4,3 | 0,00124 | 0,0202 | Promoter (2-3kb) | -2823 | ENSG00000224687 | RASAL2-AS1 |
| chr8 | 119398311 | 119398787 | 2,03 | -1,11 | 3,14 | 0,00125 | 0,0203 | Intron (uc010mda.1/401474, intron 3 of 4) | -234453 | ENSG00000281641 | SAMD12-AS1 |
| chr5 | 177663975 | 177664371 | 1,94 | -1,55 | 3,49 | 0,00125 | 0,0203 | Downstream (<1kb) | -4172 | ENSG00000175309 | PHYKPL |
| chr6 | 152022381 | 152025743 | 5,5 | 1,78 | 3,73 | 0,00126 | 0,0204 | 5'UTR | 10750 | ENSG00000091831 | ESR1 |
| chr16 | 75353937 | 75354413 | 1,72 | -1,55 | 3,27 | 0,00126 | 0,0205 | Intron (uc002fdv.3/10428, intron 5 of 6) | -51986 | ENSG00000050820 | BCAR1 |
| chr4 | 59963762 | 59964355 | 1,87 | -1,55 | 3,42 | 0,00127 | 0,0205 | Distal Intergenic | -1987211 | ENSG00000163453 | IGFBP7 |
| chr1 | 219756376 | 219756993 | 2,59 | -0,85 | 3,44 | 0,00127 | 0,0205 | Distal Intergenic | -289626 | ENSG00000199377 | RNU5F-1 |
| chr3 | 173278778 | 173279353 | 2,19 | -1,55 | 3,74 | 0,00127 | 0,0205 | Intron (uc003fio.1/22871, intron 2 of 6) | -23124 | ENSG00000169760 | NLGN1 |
| chr8 | 111477993 | 111478646 | 2,23 | -1,55 | 3,79 | 0,00127 | 0,0205 | Distal Intergenic | -489917 | ENSG00000164794 | KCNV1 |
| chr13 | 33768760 | 33770180 | 3,84 | -0,06 | 3,9 | 0,00127 | 0,0205 | Intron (uc001uuv.3/90627, intron 1 of 13) | -8544 | ENSG00000133121 | STARD13 |
| chr5 | 163201534 | 163202249 | 2,08 | -1,55 | 3,64 | 0,00127 | 0,0206 | Distal Intergenic | 257071 | ENSG0000003 8274 | MAT2B |
| chr15 | 77157366 | 77158648 | 3,75 | 0,05 | 3,69 | 0,00127 | 0,0206 | Intron (uc002bbv.3/49855, intron 1 of 30) | -3081 | ENSG00000140386 | SCAPER |
| chr10 | 30911150 | 30912447 | 2,22 | -1,55 | 3,78 | 0,00127 | 0,0206 | Intron (uc001ivk.3/119180, intron 3 of 4) | 6200 | ENSG00000151033 | LYZL2 |
| chr17 | 18506694 | 18510932 | 4,75 | 0,86 | 3,88 | 0,00127 | 0,0206 | 3'UTR | 17998 | ENSG00000154874 | CCDC144B |
| chr17 | 27090476 | 27090857 | 1,9 | -1,55 | 3,45 | 0,00128 | 0,0207 | Intron (uc002hct.1/55731, intron 2 of 2) | 19453 | ENSG00000076604 | TRAF4 |
| chr13 | 49974967 | 49975629 | 2,17 | -1,38 | 3,55 | 0,00128 | 0,0207 | Promoter (<=1kb) | 106 | ENSG00000102547 | CAB39L |
| chr5 | 165162558 | 165163074 | 2,4 | -1,38 | 3,78 | 0,00128 | 0,0207 | Distal Intergenic | -1548769 | ENSG00000145934 | TENM2 |
| chr11 | 56550585 | 56551144 | 3,41 | 0,28 | 3,13 | 0,00129 | 0,0207 | Distal Intergenic | -39298 | ENSG00000172457 | OR9G4 |
| chr4 | 153556937 | 153557516 | 2,03 | -1,55 | 3,58 | 0,00129 | 0,0207 | Intron (uc003imw.2/201799, intron 6 of 6) | 43801 | ENSG00000170006 | TMEM154 |
| chr4 | 138560279 | 138560972 | 1,83 | -1,55 | 3,38 | 0,00129 | 0,0208 | Distal Intergenic | -106631 | ENSG00000189184 | PCDH18 |
| chr4 | 187774418 | 187775342 | 2,19 | -1,55 | 3,74 | 0,0013 | 0,0209 | Distal Intergenic | -126568 | ENSG00000083857 | FAT1 |
| chr16 | 76827655 | 76828654 | 3,16 | -0,62 | 3,78 | 0,0013 | 0,0209 | Distal Intergenic | -74179 | ENSG00000266426 | MIR4719 |
| chrX | 9971866 | 9972543 | 1,89 | -1,55 | 3,44 | 0,00131 | 0,021 | Distal Intergenic | -11252 | ENSG00000047644 | WWC3 |
| chr5 | 65099512 | 65100003 | 1,99 | -1,55 | 3,55 | 0,00131 | 0,021 | Intron (uc003juf.3/57486, intron 9 of 12) | 17910 | ENSG00000123213 | NLN |
| chr9 | 114238199 | 114238829 | 2,06 | -1,55 | 3,61 | 0,00131 | 0,0211 | Intron (uc004bfe.1/23392, intron 3 of 50) | 7013 | ENSG00000136813 | ECPAS |
| chr6 | 69193881 | 69194356 | 1,79 | -1,55 | 3,34 | 0,00132 | 0,0212 | Distal Intergenic | -151276 | ENSG00000135298 | ADGRB3 |
| chr17 | 34325029 | 34325657 | 2,26 | -1,17 | 3,43 | 0,00132 | 0,0212 | Exon (uc010wcs.2/348249, exon 3 of 8) | 3427 | NA | CCL15-CCL14 |
| chr8 | 117098031 | 117099063 | 2,78 | -0,75 | 3,53 | 0,00132 | 0,0212 | Intron (uc031tcc.1/100859921, intron 7 of 13) | 238234 | ENSG00000249917 | LINC00536 |
| chr7 | 55253416 | 55254176 | 2,89 | -0,69 | 3,59 | 0,00132 | 0,0212 | Promoter (2-3kb) | 2466 | ENSG00000224057 | EGFR-AS1 |
| chr20 | 16271024 | 16271959 | 2,2 | -1,55 | 3,75 | 0,00132 | 0,0212 | Intron (uc002wpe.1/55614, intron 6 of 6) | 88849 | ENSG00000089177 | KIF16B |
| chr17 | 1296558 | 1297673 | 3,16 | -0,1 | 3,26 | 0,00133 | 0,0212 | Intron (uc002fsj.3/7531, intron 1 of 5) | 5883 | ENSG00000108953 | YWHAE |
| chr12 | 9795426 | 9795798 | 1,79 | -1,55 | 3,35 | 0,00133 | 0,0212 | Promoter (<=1kb) | 0 | ENSG00000256594 | LOC374443 |
| chr7 | 125210711 | 125211277 | 2,21 | -1,55 | 3,76 | 0,00133 | 0,0212 | Distal Intergenic | -640674 | ENSG00000128513 | POT1 |
| chr4 | 25554311 | 25555003 | 2,23 | -1,55 | 3,78 | 0,00133 | 0,0213 | Distal Intergenic | -102432 | ENSG00000157765 | SLC34A2 |
| chr2 | 144998402 | 144998812 | 1,92 | -1,14 | 3,06 | 0,00134 | 0,0213 | Intron (uc002tvp.3/79712, intron 1 of 11) | -3496 | ENSG00000121964 | GTDC1 |
| chr1 | 211482752 | 211483496 | 2,08 | -1,55 | 3,63 | 0,00134 | 0,0213 | Promoter (2-3kb) | -2083 | ENSG00000117625 | RCOR3 |
| chr6 | 167461149 | 167461784 | 2,09 | -1,55 | 3,65 | 0,00134 | 0,0214 | Intron (uc003qvl.3/1235, intron 10 of 12) | 48333 | ENSG00000112486 | CCR6 |
| chr18 | 47138463 | 47139322 | 2,1 | -1,55 | 3,66 | 0,00134 | 0,0214 | Distal Intergenic | 50036 | ENSG00000101670 | LIPG |
| chr3 | 165532997 | 165533561 | 2,15 | -1,55 | 3,71 | 0,00134 | 0,0214 | Intron (uc003fem.4/590, intron 2 of 3) | 21692 | ENSG00000114200 | BCHE |
| chr2 | 64809075 | 64811477 | 5,46 | 2,14 | 3,33 | 0,00135 | 0,0214 | Intron (uc002scz.3/54812, intron 8 of 9) | -22969 | ENSG00000233694 | LINC02579 |
| chr2 | 206640945 | 206641397 | 1,81 | -1,55 | 3,36 | 0,00135 | 0,0214 | 3'UTR | 93586 | ENSG00000118257 | NRP2 |
| chr3 | 89120777 | 89121383 | 2,2 | -1,26 | 3,46 | 0,00135 | 0,0214 | Distal Intergenic | -35291 | ENSG00000044524 | EPHA3 |
| chr13 | 51920163 | 51920958 | 2 | -1,55 | 3,56 | 0,00135 | 0,0214 | Promoter (1-2kb) | 1817 | ENSG00000266072 | MIR5693 |
| chr9 | 25025615 | 25026276 | 2,51 | -1,31 | 3,82 | 0,00135 | 0,0215 | Distal Intergenic | -479941 | ENSG00000205442 | IZUMO3 |
| chr1 | 100249559 | 100251186 | 4,77 | 1,89 | 2,88 | 0,00136 | 0,0215 | Distal Intergenic | -18210 | ENSG00000156869 | FRRS1 |
| chr14 | 53592749 | 53594215 | 4,69 | 1,57 | 3,12 | 0,00136 | 0,0215 | Intron (uc001xah.3/80821, intron 1 of 11) | 25831 | ENSG00000100523 | DDHD1 |
| chr8 | 54260046 | 54260647 | 2,24 | -1,55 | 3,8 | 0,00136 | 0,0215 | Distal Intergenic | -95852 | ENSG00000082556 | OPRK1 |
| chr12 | 111999841 | 112000987 | 3,8 | 0,46 | 3,35 | 0,00136 | 0,0216 | Intron (uc001tsh.3/6311, intron 1 of 25) | 35307 | ENSG00000204842 | ATXN2 |
| chr17 | 27760521 | 27761286 | 2,37 | -1,4 | 3,77 | 0,00136 | 0,0216 | Intron (uc002hdz.2/57551, intron 1 of 19) | 42578 | ENSG00000160551 | TAOK1 |
| chr21 | 24489685 | 24490274 | 2,01 | -1,26 | 3,27 | 0,00137 | 0,0217 | Distal Intergenic | 266882 | ENSG00000228592 | D21S2088E |
| chr1 | 166352421 | 166353784 | 3,4 | -0,56 | 3,96 | 0,00137 | 0,0217 | Distal Intergenic | -216463 | ENSG00000188859 | FAM78B |
| chr1 1 | 112876671 | 112878780 | 4,96 | 1,11 | 3,86 | 0,00138 | 0,0217 | Intron (uc001pno.3/4684, intron 1 of 8) | 44702 | ENSG00000149294 | NCAM1 |
| chr2 | 12003289 | 12004264 | 3,67 | 0,5 | 3,17 | 0,00138 | 0,0218 | Distal Intergenic | -26177 | ENSG00000265172 | MIR4262 |
| chr4 | 28031509 | 28032354 | 2,15 | -1,55 | 3,7 | 0,00138 | 0,0218 | Distal Intergenic | -788850 | ENSG00000283275 | MIR4275 |
| chr17 | 68154618 | 68155390 | 2,19 | -1,55 | 3,75 | 0,00138 | 0,0218 | Distal Intergenic | -9424 | ENSG00000123700 | KCNJ2 |
| chr1 | 187277351 | 187278328 | 3,65 | -0,4 | 4,05 | 0,00138 | 0,0218 | Distal Intergenic | 479319 | ENSG00000116711 | PLA2G4A |
| chr17 | 69463689 | 69464340 | 1,85 | -1,55 | 3,41 | 0,00139 | 0,0219 | Distal Intergenic | -652821 | ENSG00000125398 | SOX9 |
| chr4 | 23908568 | 23909216 | 1,89 | -1,55 | 3,44 | 0,00139 | 0,0219 | Intron (uc031sdx.1/10891, intron 2 of 14) | -16868 | ENSG00000109819 | PPARGC1A |
| chr1 | 227177939 | 227178372 | 1,92 | -1,55 | 3,47 | 0,00139 | 0,0219 | 3'UTR | 7344 | ENSG00000163050 | COQ8A |
| chr14 | 59995221 | 59996416 | 2,92 | -0,77 | 3,69 | 0,00139 | 0,0219 | Intron (uc021rtw.1/729665, intron 14 of 19) | 41309 | ENSG00000050130 | JKAMP |
| chr11 | 125929207 | 125929623 | 2,84 | -0,94 | 3,78 | 0,00139 | 0,0219 | Promoter (2-3kb) | 2587 | ENSG00000064309 | CDON |
| chr4 | 31670397 | 31671109 | 2,14 | -1,55 | 3,69 | 0,0014 | 0,0219 | Distal Intergenic | 948360 | ENSG00000169851 | PCDH7 |
| chr1 | 230357857 | 230358580 | 2,07 | -1,55 | 3,63 | 0,0014 | 0,022 | Intron (uc010pvv.1/2590, intron 3 of 15) | -39036 | ENSG00000143641 | GALNT2 |
| chr3 | 160108411 | 160109066 | 1,74 | -1,55 | 3,29 | 0,00141 | 0,0221 | Intron (uc003fda.3/57560, intron 3 of 18) | -5977 | ENSG00000068885 | IFT80 |
| chr12 | 83516066 | 83517090 | 2,04 | -1,55 | 3,59 | 0,00141 | 0,0221 | Intron (uc001szt.3/160335, intron 11 of 11) | 435132 | ENSG00000179104 | TMTC2 |
| chr12 | 81418630 | 81419296 | 2,12 | -1,55 | 3,67 | 0,00141 | 0,0221 | Distal Intergenic | -52513 | ENSG00000111058 | ACSS3 |
| chr2 | 240180251 | 240182448 | 3,67 | -0,03 | 3,7 | 0,00141 | 0,0221 | Intron (uc002vvk.4/9759, intron 2 of 26) | 37886 | ENSG00000068024 | HDAC4 |
| chr3 | 144176826 | 144177333 | 1,88 | -1,55 | 3,44 | 0,00141 | 0,0222 | Distal Intergenic | 484659 | ENSG00000181744 | DIPK2A |
| chr7 | 82916148 | 82916840 | 1,97 | -1,55 | 3,52 | 0,00142 | 0,0222 | Distal Intergenic | -123951 | ENSG00000186472 | PCLO |
| chr2 | 18248327 | 18250314 | 3,88 | 0,02 | 3,85 | 0,00142 | 0,0222 | Distal Intergenic | 188382 | ENSG00000170745 | KCNS3 |
| chr5 | 162623003 | 162627926 | 6,41 | 1,9 | 4,51 | 0,00142 | 0,0223 | Distal Intergenic | -236651 | ENSG00000113328 | CCNG1 |
| chr10 | 115491735 | 115492291 | 1,73 | -1,55 | 3,28 | 0,00143 | 0,0224 | Distal Intergenic | -18922 | ENSG00000148735 | PLEKHS1 |
| chr12 | 74876943 | 74877759 | 2,2 | -1,26 | 3,46 | 0,00143 | 0,0224 | Distal Intergenic | -53792 | ENSG00000253719 | ATXN7L3B |
| chr19 | 19648890 | 19649562 | 2,02 | -1,55 | 3,57 | 0,00143 | 0,0224 | Promoter (<=1kb) | 0 | ENSG00000160161 | CILP2 |
| chr13 | 45548554 | 45549300 | 2,2 | -1,55 | 3,75 | 0,00143 | 0,0224 | Intron (uc001uzp.2/26747, intron 5 of 9) | 14313 | ENSG00000083635 | NUFIP1 |
| chr6 | 21384439 | 21385168 | 2,44 | -0,8 | 3,25 | 0,00144 | 0,0225 | Distal Intergenic | -208804 | ENSG00000124766 | SOX4 |
| chr10 | 80875781 | 80877232 | 3,61 | 0,41 | 3,2 | 0,00144 | 0,0226 | Intron (uc001kaf.2/57178, intron 1 of 24) | -22207 | ENSG00000108175 | ZMIZ1 |
| chr7 | 128950937 | 128952024 | 2,03 | -1,55 | 3,59 | 0,00144 | 0,0226 | Intron (uc003vot.3/23382, intron 1 of 16) | -55940 | ENSG00000158467 | AHCYL2 |
| chr7 | 42964442 | 42965673 | 2,97 | -0,77 | 3,74 | 0,00144 | 0,0226 | Intron (uc010kxr.3/79020, intron 2 of 6) | 6132 | ENSG00000106588 | PSMA2 |
| chr2 | 140446924 | 140447644 | 1,97 | -1,55 | 3,52 | 0,00145 | 0,0226 | Distal Intergenic | 792030 | NA | YY1P2 |
| chr4 | 12611034 | 12611646 | 2,4 | -1,26 | 3,66 | 0,00145 | 0,0226 | Distal Intergenic | 874343 | ENSG00000157869 | RAB28 |
| chr21 | 19373150 | 19374092 | 2,5 | -1,23 | 3,73 | 0,00145 | 0,0226 | Intron (uc002vkr.3/140578, intron 2 of 6) | 83613 | ENSG00000154645 | CHODL |
| chr5 | 166405384 | 166407036 | 5,07 | 0,92 | 4,15 | 0,00145 | 0,0226 | Distal Intergenic | -304807 | ENSG00000145934 | TENM2 |
| chr3 | 180538171 | 180539979 | 4,17 | 0,25 | 3,92 | 0,00145 | 0,0227 | Intron (uc003fko.3/uc003fko.3, intron 2 of 13) | -82509 | ENSG00000284862 | CCDC39 |
| chr15 | 95847097 | 95848140 | 3,45 | 0,38 | 3,07 | 0,00146 | 0,0227 | Intron (uc002btm.3/400456, intron 1 of 3) | 22189 | ENSG00000248441 | LINC01197 |
| chr20 | 53789257 | 53789640 | 1,76 | -1,55 | 3,31 | 0,00146 | 0,0227 | Distal Intergenic | 696991 | ENSG00000101134 | DOK5 |
| chr5 | 93652802 | 93653417 | 1,92 | -1,55 | 3,47 | 0,00146 | 0,0227 | Intron (uc003kkn.3/285600, intron 4 of 4) | 78797 | ENSG00000185261 | KIAA0825 |
| chrl7 | 17615453 | 17616389 | 1,92 | -1,55 | 3,47 | 0,00146 | 0,0227 | Intron (uc002grm.3/10743, intron 1 of 5) | 30666 | ENSG00000108557 | RAI1 |
| chr13 | 91466108 | 91466803 | 2,14 | -1,38 | 3,52 | 0,00146 | 0,0227 | Distal Intergenic | 112048 | ENSG00000231674 | LINC00410 |
| chr4 | 31667666 | 31668249 | 2,04 | -1,55 | 3,59 | 0,00146 | 0,0227 | Distal Intergenic | 945629 | ENSG00000169851 | PCDH7 |
| chrl7 | 35749031 | 35749697 | 2,66 | -0,5 | 3,16 | 0,00147 | 0,0227 | 3'UTR | 16046 | ENSG00000278505 | C17orf78 |
| chr2 | 36374210 | 36375410 | 3,81 | 0,39 | 3,42 | 0,00147 | 0,0227 | Distal Intergenic | 207303 | NA | CRIM1-DT |
| chr2 | 198492829 | 198493888 | 2,81 | -0,65 | 3,47 | 0,00147 | 0,0227 | Intron (uc002uuo.4/130132, intron 5 of 8) | 46696 | ENSG00000162944 | RFTN2 |
| chr8 | 37012714 | 37013314 | 2,64 | -0,35 | 2,98 | 0,00147 | 0,0228 | Distal Intergenic | 370872 | ENSG00000215262 | KCNU1 |
| chr10 | 76747892 | 76748550 | 1,8 | -1,55 | 3,36 | 0,00147 | 0,0228 | Intron (uc001jwm.2/23522, intron 12 of 17) | 69722 | ENSG00000188716 | DUPD1 |
| chr3 | 181918426 | 181919110 | 1,88 | -1,55 | 3,43 | 0,00147 | 0,0228 | Distal Intergenic | 285040 | ENSG00000241098 | LINC01994 |
| chr18 | 30037099 | 30037885 | 1,94 | -1,55 | 3,49 | 0,00147 | 0,0228 | Intron (uc002kxk.2/64762, intron 1 of 5) | 12562 | ENSG00000141441 | GAREM1 |
| chr4 | 168471010 | 168471648 | 2,13 | -1,55 | 3,68 | 0,00147 | 0,0228 | Distal Intergenic | -315269 | ENSG00000196104 | SPOCK3 |
| chr3 | 164920575 | 164921302 | 2,21 | -1,55 | 3,76 | 0,00147 | 0,0228 | Distal Intergenic | -6106 | ENSG00000121871 | SLITRK3 |
| chr4 | 155919106 | 155920052 | 1,97 | -1,55 | 3,53 | 0,00148 | 0,0228 | Distal Intergenic | -209729 | ENSG00000185149 | NPY2R |
| chr12 | 63544583 | 63546507 | 4,64 | 0,82 | 3,83 | 0,00148 | 0,0228 | Promoter (<=1kb) | 83 | ENSG00000166148 | AVPR1A |
| chr2 | 214968321 | 214969044 | 1,79 | -1,55 | 3,34 | 0,00148 | 0,0229 | Intron (uc010fuz.3/79582, intron 11 of 13) | -307417 | ENSG00000174453 | VWC2L |
| chr3 | 145615700 | 145617515 | 3,67 | -0,18 | 3,85 | 0,00148 | 0,0229 | Distal Intergenic | 187007 | ENSG00000152952 | PLOD2 |
| chr6 | 45454269 | 45454597 | 1,58 | -1,35 | 2,93 | 0,00149 | 0,0229 | Intron (uc01 1dvx.2/860, intron 5 of 8) | 64355 | ENSG00000124813 | RUNX2 |
| chr11 | 18350595 | 18351344 | 2,03 | -1,55 | 3,58 | 0,0015 | 0,0231 | Intron (uc001moh.2/2965, intron 1 of 14) | 6779 | ENSG00000110768 | GTF2H1 |
| chr3 | 16734722 | 16735367 | 2,29 | -1,1 | 3,38 | 0,0015 | 0,0232 | Distal Intergenic | -87716 | ENSG00000092345 | DAZL |
| chr14 | 72058816 | 72059479 | 1,96 | -1,11 | 3,07 | 0,00151 | 0,0232 | Intron (uc001xms.3/26037, intron 2 of 21) | 4528 | ENSG00000197555 | SIPA1L1 |
| chr14 | 81162220 | 81163037 | 1,82 | -1,55 | 3,37 | 0,00151 | 0,0232 | Intron (uc010asz.3/145508, intron 8 of 14) | 139644 | ENSG00000100629 | CEP128 |
| chr1 | 192561549 | 192562393 | 2,56 | -0,86 | 3,42 | 0,00151 | 0,0232 | Distal Intergenic | 16692 | ENSG00000090 104 | RGS1 |
| chr21 | 38747837 | 38749046 | 3,37 | -0,16 | 3,53 | 0,00151 | 0,0232 | Intron (uc002vwg.1/1859, intron 1 of 2) | 7978 | ENSG00000157540 | DYRK1A |
| chr4 | 188927553 | 188927918 | 1,55 | -1,55 | 3,1 | 0,00151 | 0,0233 | Distal Intergenic | 10628 | ENSG00000179059 | ZFP42 |
| chr17 | 3586377 | 3586785 | 2,02 | -1,55 | 3,58 | 0,00151 | 0,0233 | Intron (uc002fwd.3/100533970, intron 9 of 14) | 8511 | ENSG00000083454 | P2RX5 |
| chr3 | 63884956 | 63885846 | 3,14 | -0,46 | 3,6 | 0,00151 | 0,0233 | Promoter (<=1kb) | 881 | ENSG00000163635 | ATXN7 |
| chr5 | 13990886 | 13991535 | 2,23 | -1,55 | 3,78 | 0,00151 | 0,0233 | Distal Intergenic | -46297 | ENSG00000039139 | DNAH5 |
| chr8 | 78059863 | 78060418 | 2,38 | -1,06 | 3,44 | 0,00152 | 0,0234 | Distal Intergenic | -146583 | ENSG00000164751 | PEX2 |
| chr9 | 105206746 | 105208665 | 3,83 | 0,29 | 3,55 | 0,00152 | 0,0234 | Distal Intergenic | -548928 | ENSG00000155833 | CYLC2 |
| chr12 | 59993463 | 59994458 | 2,06 | -1,55 | 3,61 | 0,00152 | 0,0234 | Intron (uc001sqs.4/9194, intron 1 of 5) | 3642 | ENSG00000118596 | SLC16A7 |
| chr13 | 40395756 | 40396935 | 3,57 | 0,24 | 3,32 | 0,00153 | 0,0234 | Distal Intergenic | -157484 | ENSG00000264171 | MIR4305 |
| chr12 | 63908088 | 63908546 | 1,85 | -1,55 | 3,41 | 0,00153 | 0,0234 | Distal Intergenic | 69292 | ENSG00000177990 | DPY19L2 |
| chr3 | 82151936 | 82152463 | 2,23 | -1,23 | 3,47 | 0,00153 | 0,0234 | Intron (uc003dqh.1/uc003dqh.1, intron 1 of 8) | -340986 | ENSG00000114480 | GBE1 |
| chr6 | 22728101 | 22728707 | 1,98 | -1,55 | 3,53 | 0,00153 | 0,0234 | Distal Intergenic | 158423 | ENSG00000112273 | HDGFL1 |
| chr18 | 5868422 | 5869067 | 2,06 | -1,55 | 3,62 | 0,00153 | 0,0234 | Distal Intergenic | 23036 | ENSG00000206432 | TMEM200C |
| chr20 | 44633461 | 44634121 | 1,99 | -1,55 | 3,54 | 0,00153 | 0,0235 | Distal Intergenic | -3426 | ENSG00000100985 | MMP9 |
| chr8 | 96374197 | 96374881 | 2,1 | -1,55 | 3,65 | 0,00153 | 0,0235 | Intron (uc022ayl.1/100616530, intron 2 of 5) | -92735 | ENSG00000156172 | C8orf37 |
| chr3 | 45694813 | 45695372 | 1,83 | -1,55 | 3,38 | 0,00154 | 0,0235 | Intron (uc003coq.3/8994, intron 2 of 7) | 35002 | NA | LIMD1-AS1 |
| chr14 | 92332725 | 92333995 | 3,24 | -0,37 | 3,61 | 0,00154 | 0,0235 | Promoter (<=1kb) | 0 | ENSG00000165929 | TC2N |
| chr3 | 82340202 | 82347012 | 7,16 | 2,56 | 4,6 | 0,00154 | 0,0235 | Intron (uc003dqh.1/uc003dqh.1, intron 3 of 8) | -529252 | ENSG00000114480 | GBE1 |
| chr13 | 24368208 | 24369468 | 3,31 | 0,09 | 3,21 | 0,00154 | 0,0236 | Intron (uc001uox.4/4285, intron 16 of 18) | -93560 | ENSG00000205861 | PCOTH |
| chr6 | 149367876 | 149368591 | 2,34 | -0,56 | 2,9 | 0,00155 | 0,0236 | Intron (uc003qmg.3/10090, intron 7 of 7) | -82056 | NA | UST-AS1 |
| chr18 | 74570813 | 74571569 | 1,65 | -1,55 | 3,2 | 0,00155 | 0,0236 | Intron (uc0021mi.3/7776, intron 3 of 30) | 9658 | ENSG00000130856 | ZNF236 |
| chr4 | 30123614 | 30124179 | 1,82 | -1,55 | 3,37 | 0,00155 | 0,0236 | Distal Intergenic | -597851 | ENSG00000169851 | PCDH7 |
| chr22 | 22211490 | 22212636 | 3,53 | -0,27 | 3,8 | 0,00155 | 0,0236 | Intron (uc002zvn.3/5594, intron 1 of 8) | 9334 | ENSG00000100030 | MAPK1 |
| chr22 | 26142392 | 26143598 | 2,69 | -1,14 | 3,83 | 0,00155 | 0,0236 | Intron (uc003abz.1/84700, intron 1 of 43) | 4272 | ENSG00000133454 | MYO18B |
| chr6 | 164115503 | 164117396 | 4,14 | 0,28 | 3,86 | 0,00155 | 0,0237 | Intron (uc003quk.1/uc003quk.1, intron 1 of 3) | 279828 | ENSG00000112531 | QKI |
| chr5 | 96292835 | 96293893 | 2,97 | -0,65 | 3,62 | 0,00156 | 0,0237 | Promoter (<=1kb) | -263 | ENSG00000113441 | LNPEP |
| chr1 | 51265989 | 51266591 | 1,79 | -1,55 | 3,34 | 0,00157 | 0,0239 | Intron (uc009vyw.1/1 1124, intron 3 of 15) | 158934 | ENSG00000185104 | FAF1 |
| chr6 | 151706104 | 151706907 | 2,11 | -1,29 | 3,39 | 0,00157 | 0,0239 | Intron (uc003qoh.3/57621, intron 1 of 2) | 5770 | ENSG00000181472 | ZBTB2 |
| chr6 | 4969587 | 4970347 | 1,9 | -1,55 | 3,45 | 0,00157 | 0,0239 | Distal Intergenic | 33924 | ENSG00000124787 | RPP40 |
| chr17 | 62183134 | 62183964 | 2,58 | -0,73 | 3,31 | 0,00158 | 0,024 | Intron (uc002jdz.2/2081,intron 1 of 21) | 23538 | ENSG00000178607 | ERN1 |
| chr5 | 5871581 | 5872156 | 1,81 | -1,55 | 3,36 | 0,00158 | 0,024 | Distal Intergenic | 448795 | ENSG00000164151 | ICE1 |
| chr7 | 83051615 | 83052332 | 1,89 | -1,55 | 3,45 | 0,00158 | 0,024 | Intron (uc022agv.1/9723, intron 4 of 16) | 218415 | ENSG00000170381 | SEMA3E |
| chrX | 76264145 | 76267574 | 5,19 | 1,29 | 3,89 | 0,00158 | 0,024 | Distal Intergenic | -124360 | ENSG00000283242 | MIR384 |
| chr3 | 196965368 | 196966041 | 2,34 | -0,86 | 3,2 | 0,00159 | 0,0241 | Intron (uc01 1bud.2/1739, intron 1 of 20) | 43675 | ENSG00000075711 | DLG1 |
| chr4 | 43474191 | 43474727 | 2,2 | -1,01 | 3,21 | 0,00159 | 0,0241 | Distal Intergenic | 578908 | ENSG00000215203 | GRXCR1 |
| chr7 | 41874608 | 41875382 | 2,88 | -0,37 | 3,25 | 0,00159 | 0,0241 | Distal Intergenic | -131902 | ENSG00000122641 | INHBA |
| chr12 | 77197781 | 77198613 | 1,98 | -1,29 | 3,27 | 0,00159 | 0,0241 | Intron (uc001svi.1/23390, intron 2 of 7) | 39927 | ENSG00000186908 | ZDHHC17 |
| chr9 | 117453380 | 117454037 | 1,82 | -1,55 | 3,38 | 0,00159 | 0,0241 | Distal Intergenic | -9011 | ENSG00000230601 | TEX48 |
| chr6 | 70545283 | 70545813 | 1,98 | -1,55 | 3,53 | 0,00159 | 0,0241 | Distal Intergenic | -30635 | ENSG00000082293 | COL19A1 |
| chr7 | 138802924 | 138804339 | 3,32 | -0,24 | 3,56 | 0,00159 | 0,0241 | Distal Intergenic | -8459 | ENSG00000105939 | ZC3HAV1 |
| chr17 | 70000635 | 70001644 | 3,19 | -0,39 | 3,58 | 0,00159 | 0,0241 | Distal Intergenic | -115517 | ENSG00000125398 | SOX9 |
| chr18 | 38487329 | 38488228 | 2,17 | -1,55 | 3,73 | 0,00159 | 0,0241 | Distal Intergenic | 612333 | ENSG00000267313 | KC6 |
| chr6 | 156079647 | 156080400 | 1,88 | -1,55 | 3,44 | 0,0016 | 0,0241 | Distal Intergenic | -302610 | ENSG00000074771 | NOX3 |
| chr8 | 78220030 | 78220547 | 2,11 | -1,29 | 3,39 | 0,0016 | 0,0242 | Distal Intergenic | -306750 | ENSG00000164751 | PEX2 |
| chr14 | 92725853 | 92726556 | 1,8 | -1,55 | 3,36 | 0,00161 | 0,0242 | Distal Intergenic | -62369 | ENSG00000140090 | SLC24A4 |
| chr10 | 55086360 | 55087229 | 1,87 | -1,55 | 3,43 | 0,00161 | 0,0242 | Distal Intergenic | -554900 | ENSG00000165471 | MBL2 |
| chr3 | 114817971 | 114820132 | 5,83 | 2,14 | 3,69 | 0,00161 | 0,0242 | Intron (uc003ebn.3/26137, intron 1 of 9) | -27710 | ENSG00000181722 | ZBTB20 |
| chr5 | 120481373 | 120481941 | 1,93 | -1,55 | 3,48 | 0,00161 | 0,0243 | Distal Intergenic | 528541 | ENSG00000184838 | PRR16 |
| chr9 | 26510229 | 26510963 | 2,24 | -1,01 | 3,25 | 0,00162 | 0,0244 | Distal Intergenic | 381863 | ENSG00000120159 | CAAP1 |
| chr10 | 92753176 | 92753764 | 2,48 | -0,4 | 2,88 | 0,00163 | 0,0245 | Distal Intergenic | -51801 | NA | LINC00502 |
| chr3 | 145676938 | 145678344 | 4,09 | 0,8 | 3,29 | 0,00163 | 0,0245 | Distal Intergenic | 126178 | ENSG00000152952 | PLOD2 |
| chr4 | 27737405 | 27738334 | 2,34 | -1,14 | 3,48 | 0,00163 | 0,0245 | Distal Intergenic | 732856 | ENSG00000109689 | STIM2 |
| chr2 | 41653389 | 41654298 | 2,63 | -0,87 | 3,5 | 0,00164 | 0,0246 | Distal Intergenic | -450397 | ENSG00000214691 | LINC01913 |
| chr3 | 17039570 | 17040132 | 1,86 | -1,23 | 3,09 | 0,00165 | 0,0247 | Intron (uc010het.1/23228, intron 1 of 2) | 64882 | ENSG00000283885 | MIR3714 |
| chr1 1 | 114038023 | 114038691 | 3,39 | 0,63 | 2,76 | 0,00165 | 0,0248 | Intron (uc001poo.1/7704, intron 3 of 3) | -89862 | ENSG00000166741 | NNMT |
| chr1 | 156066505 | 156067169 | 3,45 | 0,62 | 2,84 | 0,00165 | 0,0248 | Intron (uc001fnf.1/4000, intron 3 of 12) | 14136 | ENSG00000160789 | LMNA |
| chr2 | 239606461 | 239607416 | 2,03 | -1,55 | 3,58 | 0,00165 | 0,0248 | Distal Intergenic | -142321 | ENSG00000225493 | LINC01107 |
| chr4 | 45352323 | 45352937 | 1,86 | -1,55 | 3,41 | 0,00166 | 0,0249 | Distal Intergenic | -623672 | ENSG00000163281 | GNPDA2 |
| chr6 | 72352107 | 72352710 | 1,87 | -1,55 | 3,42 | 0,00166 | 0,0249 | Distal Intergenic | -221659 | ENSG00000233237 | LINC00472 |
| chr8 | 10920941 | 10922240 | 2,84 | -0,69 | 3,54 | 0,00166 | 0,0249 | Intron (uc003wtk.1/286046, intron 1 of 2) | -28129 | ENSG00000207600 | MIR598 |
| chr12 | 25103419 | 25104147 | 2,04 | -1,55 | 3,59 | 0,00166 | 0,0249 | Promoter (1-2kb) | -1026 | ENSG00000060982 | BCAT1 |
| chr2 | 163568877 | 163569882 | 2,74 | -0,87 | 3,6 | 0,00166 | 0,0249 | Intron (uc002uch.2/90134, intron 2 of 15) | 125375 | ENSG00000184611 | KCNH7 |
| chr2 | 155756584 | 155757126 | 2,1 | -1,29 | 3,38 | 0,00167 | 0,0249 | Distal Intergenic | 201491 | ENSG00000162989 | KCNJ3 |
| chr1 | 209235574 | 209236312 | 2,13 | -1,55 | 3,68 | 0,00167 | 0,0249 | Distal Intergenic | -365856 | ENSG00000230937 | MIR205HG |
| chr20 | 61516994 | 61517712 | 3,34 | 0,55 | 2,79 | 0,00167 | 0,025 | Intron (uc002vdr.2/11083, intron 15 of 15) | -23879 | ENSG00000101190 | TCFL5 |
| chr7 | 76757397 | 76758348 | 2,16 | -1,04 | 3,2 | 0,00167 | 0,025 | Intron (uc003uga.3/57639, intron 1 of 18) | 5463 | ENSG00000135205 | CCDC146 |
| chr6 | 84346160 | 84347197 | 2,87 | -0,7 | 3,57 | 0,00168 | 0,025 | Intron (uc011dzd.3/9892, intron 7 of 27) | 70479 | ENSG00000065609 | SNAP91 |
| chr8 | 137759185 | 137760449 | 3 | -0,75 | 3,75 | 0,00168 | 0,025 | Distal Intergenic | 1165064 | ENSG00000131773 | KHDRB S3 |
| chr17 | 39683741 | 39684680 | 2,12 | -1,17 | 3,29 | 0,00168 | 0,0251 | Promoter (<=1kb) | 0 | ENSG00000171345 | KRT19 |
| chr12 | 66460267 | 66460973 | 2,35 | -0,77 | 3,12 | 0,00169 | 0,0252 | Distal Intergenic | 63560 | ENSG00000139233 | LLPH |
| chr5 | 39167046 | 39167481 | 1,63 | -1,55 | 3,18 | 0,0017 | 0,0253 | Intron (uc003jls.3/2533, intron 1 of 16) | 35648 | ENSG00000082074 | FYB1 |
| chr2 | 109200641 | 109200974 | 1,63 | -1,55 | 3,19 | 0,0017 | 0,0253 | Intron (uc002tel.3/3987, intron 1 of 9) | -3783 | ENSG00000169756 | LIMS1 |
| chr4 | 137260095 | 137260732 | 1,89 | -1,55 | 3,45 | 0,0017 | 0,0253 | Distal Intergenic | 1192897 | ENSG00000189184 | PCDH18 |
| chr2 | 140214760 | 140215939 | 2,86 | -0,63 | 3,49 | 0,0017 | 0,0253 | Distal Intergenic | 559866 | NA | YY1P2 |
| chr9 | 107512069 | 107513709 | 4,79 | 1,3 | 3,5 | 0,00171 | 0,0254 | Promoter (2-3kb) | 2100 | ENSG00000136783 | NIPSNAP3A |
| chr4 | 93382376 | 93383073 | 2,02 | -1,26 | 3,28 | 0,00172 | 0,0255 | Intron (uc010ikx.3/2895, intron 1 of 5) | 156826 | ENSG00000152208 | GRID2 |
| chr2 | 177741050 | 177741621 | 1,84 | -1,55 | 3,39 | 0,00172 | 0,0255 | Distal Intergenic | -238748 | NA | LINC01116 |
| chr1 | 70406201 | 70407242 | 3,38 | 0,29 | 3,09 | 0,00172 | 0,0256 | Intron (uc001dep.3/57554, intron 6 of 24) | 21196 | ENSG00000229359 | PIN1P1 |
| chr1 1 | 85834363 | 85835118 | 3,03 | -0,17 | 3,2 | 0,00173 | 0,0256 | Distal Intergenic | -53440 | ENSG00000073921 | PICALM |
| chr10 | 22510257 | 22511240 | 2,65 | -0,25 | 2,9 | 0,00173 | 0,0257 | Distal Intergenic | -11345 | ENSG00000223601 | EBLN1 |
| chr6 | 127719185 | 127720209 | 3,24 | -0,16 | 3,4 | 0,00173 | 0,0257 | Distal Intergenic | 48021 | ENSG00000189367 | KIAA0408 |
| chr1 1 | 37899727 | 37900891 | 2,89 | -0,86 | 3,74 | 0,00174 | 0,0257 | Distal Intergenic | -1279898 | ENSG00000175097 | RAG2 |
| chr5 | 117318163 | 117318749 | 2,06 | -1,26 | 3,31 | 0,00174 | 0,0258 | Distal Intergenic | 566955 | NA | LINC00992 |
| chr8 | 138161296 | 138161751 | 2,56 | -0,81 | 3,37 | 0,00174 | 0,0258 | Distal Intergenic | 1003708 | ENSG00000147724 | FAM135B |
| chr4 | 131427212 | 131427760 | 2 | -1,26 | 3,26 | 0,00175 | 0,0258 | Distal Intergenic | 1409930 | ENSG00000151470 | C4orf33 |
| chr16 | 76617119 | 76617986 | 2,49 | -1,01 | 3,5 | 0,00175 | 0,0258 | Distal Intergenic | 267042 | ENSG00000152910 | CNTNAP4 |
| chr9 | 25615403 | 25615968 | 1,85 | -1,26 | 3,1 | 0,00175 | 0,0259 | Distal Intergenic | 62888 | ENSG00000198680 | TUSC1 |
| chr1 1 | 83186615 | 83187353 | 1,78 | -1,55 | 3,33 | 0,00175 | 0,0259 | Intron (uc010rsw.1/1740, intron 5 of 10) | -189201 | ENSG00000137500 | CCDC90B |
| chr12 | 96537120 | 96537651 | 1,78 | -1,55 | 3,34 | 0,00175 | 0,0259 | Distal Intergenic | -50556 | ENSG00000111145 | ELK3 |
| chr2 | 59246821 | 59247279 | 1,85 | -1,55 | 3,4 | 0,00175 | 0,0259 | Exon (uc002rzv.3/400955, exon 9 of 14) | 94075 | ENSG00000233723 | LINC01122 |
| chr10 | 27118770 | 27120377 | 3,62 | -0,04 | 3,66 | 0,00175 | 0,0259 | Intron (uc001isx.3/10006, intron 1 of 11) | 29639 | ENSG00000136754 | ABI1 |
| chr21 | 46838068 | 46838419 | 1,52 | -1,55 | 3,08 | 0,00176 | 0,0259 | Intron (uc002zhg.3/80781, intron 2 of 41) | 6536 | ENSG00000183535 | COL18A1-AS1 |
| chr6 | 165469033 | 165469791 | 2,18 | -1,26 | 3,43 | 0,00177 | 0,0261 | Distal Intergenic | 252551 | ENSG00000112539 | C6orf118 |
| chr2 | 194026588 | 194027097 | 2,52 | -0,91 | 3,44 | 0,00177 | 0,0261 | Distal Intergenic | 412017 | ENSG00000227418 | PCGEM1 |
| chr5 | 144436164 | 144436578 | 1,65 | -1,55 | 3,21 | 0,00178 | 0,0261 | Distal Intergenic | 778321 | ENSG00000186314 | PRELID2 |
| chr17 | 46708862 | 46709385 | 1,67 | -1,55 | 3,22 | 0,00179 | 0,0263 | Promoter (<=1kb) | 536 | ENSG00000210741 | MIR196A1 |
| chr2 | 103620650 | 103621564 | 2,3 | -0,93 | 3,23 | 0,00179 | 0,0263 | Distal Intergenic | 206425 | ENSG00000 170417 | TMEM182 |
| chr6 | 149102333 | 149102909 | 1,47 | -1,55 | 3,02 | 0,0018 | 0,0264 | Intron (uc003qmg.3/10090, intron 1 of 7) | 34062 | ENSG00000 111962 | UST |
| chr5 | 90606413 | 90610747 | 7,11 | 3,86 | 3,25 | 0,0018 | 0,0264 | Exon (uc003kjy.1/uc003kjy.1, exon 1 of 3) | -65417 | ENSG00000281357 | ARRDC3-AS1 |
| chr7 | 114997407 | 114997971 | 1,86 | -1,55 | 3,41 | 0,00181 | 0,0265 | Distal Intergenic | 434498 | ENSG00000135272 | MDFIC |
| chr18 | 27064935 | 27065575 | 1,99 | -1,55 | 3,54 | 0,00181 | 0,0265 | Distal Intergenic | -813301 | ENSG00000283218 | MIR302F |
| chr2 | 80308122 | 80308871 | 2,56 | -1,1 | 3,66 | 0,00181 | 0,0265 | Intron (uc010yse.2/1496, intron 11 of 21) | 222616 | ENSG00000162951 | LRRTM1 |
| chr4 | 75230011 | 75231913 | 5,63 | 1,48 | 4,15 | 0,00181 | 0,0265 | Promoter (<=1kb) | 0 | ENSG00000124882 | EREG |
| chr17 | 20819524 | 20820114 | 2,66 | -0,3 | 2,96 | 0,00182 | 0,0266 | Distal Intergenic | -20071 | ENSG00000205212 | CCDC144NL |
| chr2 | 6125899 | 6126580 | 1,89 | -1,55 | 3,45 | 0,00182 | 0,0266 | Exon (uc010yir.1/400940, exon 3 of 3) | 3789 | NA | LOC400940 |
| chr6 | 72105593 | 72106053 | 1,62 | -1,55 | 3,17 | 0,00183 | 0,0267 | Distal Intergenic | 7271 | ENSG00000207827 | MIR30A |
| chr1 | 186394955 | 186395366 | 1,56 | -1,55 | 3,11 | 0,00183 | 0,0268 | Intron (uc021pgf.1/100302192, intron 1 of 1) | 22490 | ENSG00000116703 | PDC |
| chr4 | 149463937 | 149464504 | 1,78 | -1,55 | 3,33 | 0,00184 | 0,0268 | Distal Intergenic | -100265 | ENSG00000 151623 | NR3C2 |
| chr9 | 20608503 | 20608981 | 1,68 | -1,55 | 3,24 | 0,00184 | 0,0269 | Intron (uc011lne.1/4300, intron 1 of 9) | 12179 | ENSG00000 171843 | MLLT3 |
| chrX | 129309818 | 129311304 | 3,7 | 0,3 | 3,4 | 0,00184 | 0,0269 | Intron (uc004ev1.3/9363,intron 1 of 1) | 4045 | ENSG00000134594 | RAB33A |
| chr14 | 54809653 | 54810399 | 1,99 | -1,55 | 3,55 | 0,00184 | 0,0269 | Distal Intergenic | -53274 | ENSG00000100526 | CDKN3 |
| chr17 | 15264323 | 15266119 | 3,8 | -0,09 | 3,89 | 0,00185 | 0,0269 | Distal Intergenic | -19365 | ENSG00000125409 | TEKT3 |
| chr8 | 132869275 | 132869876 | 1,63 | -1,4 | 3,03 | 0,00185 | 0,027 | Distal Intergenic | -46480 | ENSG00000132294 | EFR3 A |
| chr20 | 58675677 | 58677726 | 4,4 | 1,2 | 3,21 | 0,00186 | 0,0271 | Distal Intergenic | -35822 | ENSG00000228340 | MIR646HG |
| chrX | 12461160 | 12462036 | 1,76 | -1,55 | 3,31 | 0,00186 | 0,0271 | Intron (uc004cuz.2/9758, intron 1 of 16) | 304575 | ENSG00000169933 | FRMPD4 |
| chr2 | 112235676 | 112241843 | 5,77 | 2,08 | 3,69 | 0,00186 | 0,0271 | Intron (uc002the.2/541471, intron 1 of 5) | 10849 | ENSG00000172965 | MIR4435-2HG |
| chr4 | 115534418 | 115535009 | 1,53 | -1,55 | 3,08 | 0,00187 | 0,0272 | Intron (uc003ibs.2/7368, intron 1 of 5) | -8514 | ENSG00000174607 | UGT8 |
| chr3 | 5470929 | 5471420 | 1,62 | -1,55 | 3,18 | 0,00187 | 0,0272 | Distal Intergenic | -178989 | ENSG00000265180 | MIR4790 |
| chr7 | 12677636 | 12678301 | 3,07 | -0,11 | 3,18 | 0,00187 | 0,0272 | Intron (uc003ssn.4/85477, intron 10 of 15) | -48151 | ENSG00000122644 | ARL4A |
| chr13 | 105813145 | 105814037 | 3,09 | -0,24 | 3,33 | 0,00187 | 0,0272 | Distal Intergenic | -304179 | ENSG00000182346 | DAOA |
| chr15 | 100709897 | 100710494 | 2,13 | -1,29 | 3,42 | 0,00187 | 0,0272 | Intron (uc002bvv.1/170691, intron 8 of 21) | 168811 | ENSG00000140470 | ADAMTS17 |
| chr18 | 36161718 | 36162105 | 2,02 | -1,55 | 3,57 | 0,00187 | 0,0272 | Distal Intergenic | 758497 | ENSG00000267374 | MIR924HG |
| chr8 | 89480821 | 89481474 | 2,67 | -0,97 | 3,64 | 0,00187 | 0,0272 | Distal Intergenic | -141104 | ENSG00000156103 | MMP16 |
| chr5 | 123918940 | 123919611 | 2,29 | -0,62 | 2,92 | 0,00188 | 0,0273 | Distal Intergenic | 60743 | ENSG00000168916 | ZNF608 |
| chr9 | 15810325 | 15811039 | 2,96 | -0,4 | 3,36 | 0,00188 | 0,0273 | Intron (uc003zmd.3/203238, intron 21 of 25) | 66054 | ENSG00000164989 | CCDC171 |
| chr2 | 141260533 | 141261230 | 2,41 | -1,01 | 3,42 | 0,00188 | 0,0273 | Exon (uc002tvj.1/53353, exon 54 of 91) | 1605639 | NA | YY1P2 |
| chr7 | 137438864 | 137439356 | 1,39 | -1,55 | 2,94 | 0,00189 | 0,0274 | Intron (uc003vtt.3/9162, intron 1 of 33) | 92253 | ENSG00000157680 | DGKI |
| chr4 | 99467978 | 99468608 | 1,91 | -1,55 | 3,46 | 0,00189 | 0,0274 | Intron (uc01 1cdz.2/10098, intron 1 of 7) | 110192 | ENSG00000168785 | TSPAN5 |
| chr3 | 18832827 | 18833351 | 1,73 | -1,55 | 3,29 | 0,0019 | 0,0275 | Distal Intergenic | -352562 | ENSG00000182568 | SATB1 |
| chr7 | 21710793 | 21711379 | 2,1 | -1,55 | 3,65 | 0,0019 | 0,0275 | Intron (uc031swp.1/8701, intron 30 of 81) | 127960 | ENSG00000105877 | DNAH11 |
| chr1 | 192567788 | 192568219 | 1,9 | -1,08 | 2,98 | 0,0019 | 0,0276 | Distal Intergenic | 22931 | ENSG00000090104 | RGS1 |
| chr20 | 45817559 | 45818210 | 2,03 | -1,55 | 3,58 | 0,0019 | 0,0276 | Distal Intergenic | 21950 | ENSG00000264901 | MIR3616 |
| chr8 | 112392362 | 112392990 | 2,31 | -1,55 | 3,86 | 0,0019 | 0,0276 | Distal Intergenic | 956970 | ENSG00000164796 | CSMD3 |
| chr9 | 25799651 | 25800249 | 1,85 | -1,55 | 3,4 | 0,00191 | 0,0276 | Intron (uc003zpy.3/uc003zpy.3, intron 5 of 5) | -120795 | ENSGO0000198680 | TUSC1 |
| chr13 | 104630670 | 104631483 | 1,95 | -1,55 | 3,5 | 0,00191 | 0,0276 | Distal Intergenic | -695822 | ENSG00000263741 | MIR548AS |
| chr15 | 61349219 | 61349973 | 1,92 | -1,55 | 3,47 | 0,00191 | 0,0277 | Intron (uc002agx.3/6095, intron 1 of 10) | 171529 | ENSG00000069667 | RORA |
| chr1 1 | 15583378 | 15584169 | 2,82 | -0,66 | 3,49 | 0,00192 | 0,0277 | Distal Intergenic | 412730 | ENSG00000188487 | INSC |
| chr1 1 | 122802453 | 122802974 | 1,75 | -1,55 | 3,3 | 0,00192 | 0,0278 | Intron (uc001pvm.4/79864, intron 4 of 8) | 49217 | ENSG00000109944 | JHY |
| chr1 | 234406599 | 234407243 | 2,12 | -1,23 | 3,35 | 0,00192 | 0,0278 | Intron (uc001hvy.1/148641, intron 3 of 7) | -34970 | ENSG00000264377 | MIR4671 |
| chr1 1 | 111504815 | 111505558 | 1,81 | -1,55 | 3,36 | 0,00192 | 0,0278 | Intron (uc001p1t.3/23235, intron 3 of 14) | 31645 | ENSG00000170145 | SIK2 |
| chr4 | 116551931 | 116552460 | 1,82 | -1,55 | 3,37 | 0,00192 | 0,0278 | Distal Intergenic | -516899 | ENSG00000138653 | NDST4 |
| chr10 | 73550789 | 73551211 | 1,84 | -1,55 | 3,39 | 0,00192 | 0,0278 | Exon (uc001jrx.4/64072, exon 45 of 68) | -4317 | ENSG00000107736 | CDH23 |
| chr3 | 55619885 | 55620365 | 1,77 | -1,55 | 3,32 | 0,00193 | 0,0278 | Intron (uc003dho.1/26059, intron 2 of 2) | 24573 | ENSG00000187672 | ERC2 |
| chr18 | 40695355 | 40695903 | 1,88 | -1,55 | 3,43 | 0,00193 | 0,0278 | Promoter (<=1kb) | 0 | ENSG00000152214 | RIT2 |
| chr5 | 41891275 | 41892149 | 1,94 | -1,55 | 3,5 | 0,00193 | 0,0278 | Distal Intergenic | -12321 | ENSG00000205765 | C5orf51 |
| chr7 | 6157486 | 6158033 | 2,05 | -1,55 | 3,6 | 0,00193 | 0,0279 | Promoter (1-2kb) | 1060 | ENSG00000106346 | USP42 |
| chr1 1 | 37929143 | 37930226 | 3,25 | -0,47 | 3,72 | 0,00193 | 0,0279 | Distal Intergenic | -1309314 | ENSG00000175097 | RAG2 |
| chr10 | 19042294 | 19042759 | 1,89 | -1,55 | 3,44 | 0,00195 | 0,0281 | Distal Intergenic | 93981 | ENSG00000165997 | ARL5B |
| chr9 | 29175330 | 29175974 | 2,18 | -1,12 | 3,3 | 0,00196 | 0,0281 | Intron (uc003zqu.2/158038, intron 1 of 6) | 37024 | ENSG00000174482 | LINGO2 |
| chr13 | 41627388 | 41628220 | 1,86 | -1,55 | 3,42 | 0,00197 | 0,0282 | Distal Intergenic | -7477 | ENSG00000120688 | WBP4 |
| chr7 | 135715797 | 135716546 | 1,9 | -1,55 | 3,45 | 0,00197 | 0,0282 | Distal Intergenic | -53593 | ENSGO0000267697 | LUZP6 |
| chr1 1 | 112869228 | 112869958 | 2,59 | -0,47 | 3,06 | 0,00198 | 0,0283 | Intron (uc001pno.3/4684, intron 1 of 8) | 37259 | ENSG00000149294 | NCAM1 |
| chr4 | 102248899 | 102250040 | 3,88 | 0,76 | 3,12 | 0,00198 | 0,0283 | Intron (uc003hvs.2/5530, intron 1 of 12) | 18588 | ENSG00000138814 | PPP3 CA |
| chr16 | 56227640 | 56228819 | 3,95 | 0,6 | 3,35 | 0,00198 | 0,0283 | Promoter (<=1kb) | 0 | ENSG00000261439 | DKFZP434H168 |
| chr6 | 121843592 | 121844146 | 1,84 | -1,55 | 3,39 | 0,00198 | 0,0283 | Distal Intergenic | 86847 | ENSG00000 152661 | GJA1 |
| chr1 | 211805655 | 211806895 | 3,03 | -0,4 | 3,43 | 0,00198 | 0,0283 | Distal Intergenic | 36854 | ENSG00000117650 | NEK2 |
| chr7 | 120509177 | 120509923 | 1,96 | -1,55 | 3,51 | 0,00198 | 0,0283 | Distal Intergenic | -11000 | ENSG00000106025 | TSPAN12 |
| chr7 | 9140166 | 9145355 | 5,55 | 1,34 | 4,2 | 0,00198 | 0,0283 | Intron (uc021zzl.1/uc021zzl.1, intron 3 of 3) | -528545 | NA | PER4 |
| chr5 | 89844027 | 89844483 | 1,86 | -1,26 | 3,11 | 0,00199 | 0,0284 | Distal Intergenic | -10134 | ENSG00000 164199 | ADGRV1 |
| chrX | 133845801 | 133846339 | 1,66 | -1,55 | 3,21 | 0,00199 | 0,0284 | Distal Intergenic | -53288 | ENSG00000170965 | PLAC1 |
| chr10 | 90923616 | 90924035 | 1,8 | -1,55 | 3,35 | 0,00199 | 0,0284 | Distal Intergenic | 43036 | ENSG00000138135 | CH25H |
| chr2 | 44352101 | 44353549 | 3,35 | -0,31 | 3,67 | 0,00199 | 0,0284 | Distal Intergenic | -42451 | ENSG00000138032 | PPM1B |
| chr2 | 103616037 | 103616803 | 3,09 | 0,11 | 2,98 | 0,002 | 0,0285 | Distal Intergenic | 201812 | ENSG00000 170417 | TMEM182 |
| chr6 | 110631873 | 110632502 | 1,67 | -1,55 | 3,22 | 0,002 | 0,0285 | Intron (uc010kdu.1/728464, intron 3 of 4) | 46973 | ENSG00000053328 | METTL24 |
| chr13 | 99974739 | 99975403 | 1,82 | -1,55 | 3,37 | 0,002 | 0,0285 | Intron (uc001voa.4/337867, intron 6 of 8) | 14529 | ENSG00000134882 | UBAC2 |
| chr12 | 75154138 | 75155112 | 3,07 | -0,18 | 3,26 | 0,00201 | 0,0286 | Distal Intergenic | 222587 | ENSG00000253719 | ATXN7L3B |
| chr5 | 161923179 | 161924516 | 3,08 | -0,23 | 3,31 | 0,00201 | 0,0286 | Distal Intergenic | 428531 | ENSG00000113327 | GABRG2 |
| chr1 | 197999779 | 198000271 | 1,81 | -1,55 | 3,36 | 0,00201 | 0,0286 | Distal Intergenic | 103058 | ENSG00000143355 | LHX9 |
| chr15 | 68482093 | 68483036 | 3,18 | 0,14 | 3,04 | 0,00202 | 0,0287 | Downstream (<lkb) | 15412 | ENSG00000129007 | CALML4 |
| chr5 | 123218319 | 123218956 | 1,91 | -1,55 | 3,46 | 0,00202 | 0,0287 | Distal Intergenic | 337208 | ENSG00000151292 | CSNK1G3 |
| chr10 | 133979456 | 133981108 | 4,37 | 0,87 | 3,5 | 0,00202 | 0,0287 | Promoter (<=1kb) | -349 | ENSG00000188385 | JAKMIP3 |
| chr3 | 164983665 | 164984384 | 2,09 | -1,55 | 3,64 | 0,00202 | 0,0287 | Distal Intergenic | -69196 | ENSG00000121871 | SLITRK3 |
| chr9 | 12695462 | 12696303 | 1,98 | -1,55 | 3,53 | 0,00203 | 0,0288 | Promoter (2-3kb) | 2076 | ENSG00000107165 | TYRP1 |
| chr5 | 23117794 | 23118289 | 2,06 | -1,55 | 3,61 | 0,00203 | 0,0288 | Distal Intergenic | -264063 | ENSG00000154162 | CDH12 |
| chr13 | 109814077 | 109814507 | 1,9 | -0,98 | 2,88 | 0,00205 | 0,029 | Intron (uc001vqt.1/23026, intron 32 of 34) | 5144 | ENSG00000236242 | MYO16-AS1 |
| chr3 | 30618973 | 30620049 | 3,02 | -0,4 | 3,42 | 0,00205 | 0,029 | Distal Intergenic | -27945 | ENSG00000163513 | TGFBR2 |
| chr8 | 98667638 | 98668299 | 1,97 | -1,55 | 3,52 | 0,00205 | 0,029 | Intron (uc003yhz.3/92140, intron 1 of 11) | 11231 | ENSG00000147649 | MTDH |
| chr8 | 22271403 | 22271936 | 1,82 | -1,55 | 3,38 | 0,00205 | 0,0291 | Intron (uc003xbp.4/23516, intron 4 of 8) | -26547 | ENSG00000120910 | PPP3 CC |
| chr12 | 78722507 | 78722908 | 1,64 | -1,55 | 3,19 | 0,00206 | 0,0291 | Intron (uc001syq.1/uc001syq.1, intron 1 of 1) | 210700 | ENSG00000067798 | NAV3 |
| chr1 1 | 115809381 | 115811092 | 4,87 | 1,59 | 3,27 | 0,00206 | 0,0291 | Distal Intergenic | -178036 | NA | LINC00900 |
| chr16 | 3561777 | 3562362 | 2,33 | -1,11 | 3,44 | 0,00206 | 0,0291 | Promoter (1-2kb) | 1883 | ENSG00000103351 | CLUAP1 |
| chr4 | 102227157 | 102228448 | 4,15 | 1,2 | 2,95 | 0,00206 | 0,0292 | Intron (uc003hvs.2/5530, intron 1 of 12) | 40180 | ENSG00000138814 | PPP3 CA |
| chr1 | 182595065 | 182595432 | 1,84 | -1,35 | 3,19 | 0,00206 | 0,0292 | Distal Intergenic | 10790 | ENSG00000261504 | LINC01686 |
| chr17 | 21342053 | 21342778 | 1,73 | -1,55 | 3,28 | 0,00206 | 0,0292 | Distal Intergenic | 33605 | ENSG00000 184185 | KCNJ12 |
| chr18 | 41644130 | 41644540 | 1,7 | -1,55 | 3,25 | 0,00207 | 0,0293 | Distal Intergenic | -615598 | ENSG00000152217 | SETBP1 |
| chr1 | 9135773 | 9138259 | 3,81 | 0,23 | 3,58 | 0,00207 | 0,0293 | Intron (uc021ofv.1/6518, intron 2 of 4) | -5886 | ENSG00000142583 | SLC2A5 |
| chr5 | 159252437 | 159253581 | 3,25 | -0,4 | 3,64 | 0,00207 | 0,0293 | Distal Intergenic | -90159 | ENSG00000170214 | ADRA1B |
| chr4 | 166848873 | 166849557 | 1,7 | -1,55 | 3,25 | 0,00208 | 0,0293 | Intron (uc021xud.1/7092, intron 1 of 9) | 54463 | ENSG00000038295 | TLL1 |
| chr3 | 65163905 | 65164587 | 2,07 | -1,55 | 3,62 | 0,00208 | 0,0293 | Distal Intergenic | 222792 | ENSG00000151276 | MAGI1 |
| chr15 | 39845994 | 39847634 | 4,09 | 1,08 | 3 | 0,00208 | 0,0294 | Distal Intergenic | -25646 | ENSG00000137801 | THBS1 |
| chr1 1 | 134509146 | 134509923 | 1,8 | -1,55 | 3,36 | 0,00208 | 0,0294 | Distal Intergenic | 202770 | NA | LOC283177 |
| chr15 | 96075357 | 96076060 | 3,37 | -0,02 | 3,39 | 0,0021 | 0,0295 | Distal Intergenic | 99035 | ENSG00000259134 | LINC00924 |
| chrX | 69772972 | 69774734 | 3,5 | 0,32 | 3,18 | 0,0021 | 0,0296 | Exon (uc004dvk.3/56159, exon 15 of 19) | 98026 | ENSG00000082458 | DLG3 |
| chr7 | 140602364 | 140602967 | 1,74 | -1,55 | 3,29 | 0,0021 | 0,0296 | Intron (uc003vwc.4/673, intron 1 of 17) | 21597 | ENSG00000157764 | BRAF |
| chr17 | 51728368 | 51728978 | 2,16 | -1,26 | 3,41 | 0,0021 | 0,0296 | Distal Intergenic | -171261 | ENSG00000141200 | KIF2B |
| chr2 | 97413717 | 97415017 | 2,66 | -0,77 | 3,43 | 0,0021 | 0,0296 | Distal Intergenic | -7904 | ENSG00000114988 | LMAN2L |
| chr8 | 39116254 | 39117009 | 2,67 | -0,8 | 3,47 | 0,0021 | 0,0296 | Intron (uc003xmt.4/203102, intron 19 of 24) | 37800 | ENSG00000197140 | ADAM32 |
| chr4 | 31398112 | 31399062 | 1,95 | -1,55 | 3,51 | 0,00211 | 0,0296 | Distal Intergenic | 676075 | ENSG00000169851 | PCDH7 |
| chr17 | 21427338 | 21427809 | 1,7 | -1,55 | 3,25 | 0,00212 | 0,0298 | Distal Intergenic | 27132 | ENSG00000212719 | LINC02693 |
| chr5 | 140201187 | 140201802 | 2,01 | -1,26 | 3,26 | 0,00212 | 0,0298 | Promoter (<=1kb) | 0 | ENSG00000204965 | PCDHA5 |
| chr8 | 100668146 | 100668933 | 2,71 | -0,72 | 3,43 | 0,00212 | 0,0298 | Intron (uc003yiv.4/157680, intron 34 of 61) | -119057 | ENSG00000216069 | MIR875 |
| chr3 | 102824397 | 102825141 | 1,91 | -1,55 | 3,47 | 0,00213 | 0,0298 | Distal Intergenic | 670538 | ENSG00000170044 | ZPLD1 |
| chr1 | 73714702 | 73715375 | 2,46 | -1,1 | 3,56 | 0,00213 | 0,0298 | Distal Intergenic | 948496 | ENSG00000162620 | LRRIQ3 |
| chr8 | 89304374 | 89304986 | 2,02 | -1,55 | 3,57 | 0,00213 | 0,0298 | Intron (uc003veb.4/4325, intron 1 of 9) | 34731 | ENSG00000156103 | MMP16 |
| chr1 | 238951075 | 238952044 | 2,05 | -1,55 | 3,6 | 0,00213 | 0,0298 | Distal Intergenic | -301758 | ENSG00000215808 | LINC01139 |
| chr4 | 130263686 | 130265772 | 4,3 | 0,65 | 3,65 | 0,00213 | 0,0298 | Distal Intergenic | 246404 | ENSG00000151470 | C4orf33 |
| chr4 | 30435863 | 30436930 | 3,27 | -0,46 | 3,73 | 0,00213 | 0,0298 | Distal Intergenic | -285100 | ENSG00000169851 | PCDH7 |
| chr2 | 141804735 | 141805775 | 3,19 | -0,36 | 3,55 | 0,00213 | 0,0299 | Intron (uc002tvj.1/53353, intron 11 of 90) | 1083495 | ENSG00000168702 | LRP1B |
| chr12 | 131417133 | 131417796 | 1,63 | -1,29 | 2,92 | 0,00214 | 0,0299 | Distal Intergenic | -20656 | ENSG00000111452 | ADGRD1 |
| chr14 | 36324721 | 36326418 | 4,6 | 1,17 | 3,43 | 0,00214 | 0,0299 | Intron (uc010tpx.1/84312, intron 5 of 6) | 29124 | ENSG00000100916 | BRMS1L |
| chr1 | 174932824 | 174940122 | 7,75 | 4,11 | 3,63 | 0,00214 | 0,0299 | Promoter (<=1kb) | 0 | ENSG00000 152061 | RABGAP1L |
| chr1 1 | 22475913 | 22476749 | 3,23 | -0,24 | 3,47 | 0,00215 | 0,03 | Distal Intergenic | 116246 | ENSG00000091664 | SLC17A6 |
| chr12 | 127331525 | 127332158 | 2,01 | -1,06 | 3,07 | 0,00215 | 0,0301 | Distal Intergenic | 27078 | ENSG00000249873 | LINC02372 |
| chr2 | 9017133 | 9017690 | 1,57 | -1,55 | 3,12 | 0,00216 | 0,0301 | Exon (uc002qzg.1/129642, exon 7 of 13) | -39378 | ENSG00000134313 | KIDINS220 |
| chr5 | 20223241 | 20223620 | 1,62 | -1,55 | 3,17 | 0,00216 | 0,0301 | Intron (uc003jgc.3/1016, intron 2 of 14) | -234888 | ENSG00000145526 | CDH18 |
| chr7 | 77756407 | 77756850 | 1,72 | -1,55 | 3,27 | 0,00216 | 0,0301 | Exon (uc003ugx.3/9863, exon 19 of 22) | 218297 | ENSG00000006576 | PHTF2 |
| chr3 | 87200329 | 87201204 | 3,04 | -0,56 | 3,6 | 0,00216 | 0,0301 | Distal Intergenic | 74223 | ENSG00000264119 | MIR4795 |
| chr7 | 144755860 | 144756351 | 1,88 | -1,55 | 3,43 | 0,00217 | 0,0302 | Distal Intergenic | -222714 | ENSG00000 196511 | TPK1 |
| chr5 | 180560299 | 180562030 | 4,93 | 1,5 | 3,43 | 0,00217 | 0,0302 | Distal Intergenic | -7995 | ENSG00000185372 | OR2V1 |
| chr1 | 190349902 | 190352131 | 4,69 | 1,53 | 3,17 | 0,00218 | 0,0303 | Intron (uc010pot.1/339479, intron 2 of 6) | 92811 | ENSG00000162670 | BRINP3 |
| chr2 | 9678204 | 9678751 | 1,7 | -1,55 | 3,25 | 0,00218 | 0,0303 | Intron (uc002qzu.3/6868, intron 2 of 18) | -14727 | ENSG00000151694 | ADAM17 |
| chr2 | 185644819 | 185646003 | 3,5 | 0,14 | 3,36 | 0,00218 | 0,0303 | Intron (uc002uph.3/91752, intron 1 of 3) | 181726 | ENSG00000170396 | ZNF804A |
| chr8 | 89025987 | 89029886 | 6,53 | 1,59 | 4,94 | 0,00218 | 0,0303 | Distal Intergenic | -139691 | ENSG00000176566 | DCAF4L2 |
| chr21 | 23793161 | 23794011 | 3,17 | -0,39 | 3,57 | 0,00219 | 0,0304 | Distal Intergenic | 322225 | ENSG00000184856 | LINC00308 |
| chr17 | 50891714 | 50892158 | 1,58 | -1,55 | 3,14 | 0,0022 | 0,0305 | Distal Intergenic | -170722 | ENSG0000022 7011 | C17orf112 |
| chr2 | 74517003 | 74517615 | 1,9 | -1,35 | 3,24 | 0,0022 | 0,0306 | Intron (uc002skl.3/57835, intron 12 of 38) | 24537 | ENSG00000188687 | SLC4A5 |
| chr1 | 89642457 | 89643259 | 2,3 | -1,17 | 3,47 | 0,0022 | 0,0306 | Promoter (<=1kb) | -734 | ENSG00000213512 | GBP7 |
| chr5 | 59836527 | 59838438 | 4,49 | 1,11 | 3,38 | 0,00221 | 0,0306 | Intron (uc003jsg.2/25859, intron 3 of 3) | -52602 | ENSG00000113448 | PDE4D |
| chr10 | 15280303 | 15280685 | 1,55 | -1,55 | 3,1 | 0,00221 | 0,0307 | Intron (uc001iob.3/221061, intron 5 of 7) | -69608 | ENSG00000152465 | NMT2 |
| chr6 | 80460157 | 80460865 | 2,27 | -1,1 | 3,37 | 0,00221 | 0,0307 | Distal Intergenic | 8521 | ENSG00000252316 | RNY4 |
| chr13 | 75947144 | 75947989 | 2,51 | -0,8 | 3,31 | 0,00222 | 0,0307 | Intron (uc001vjl.1/9882, intron 1 of 20) | -56405 | ENSG00000136111 | TBC1D4 |
| chr9 | 26331032 | 26331839 | 2,35 | -1,06 | 3,41 | 0,00223 | 0,0308 | Distal Intergenic | 264359 | ENSG00000276759 | LOC100506422 |
| chr4 | 189029203 | 189029909 | 2,12 | -1,38 | 3,51 | 0,00223 | 0,0308 | Promoter (<=1kb) | 513 | ENSG00000179046 | TRIML2 |
| chr14 | 22932924 | 22933988 | 3,94 | 1,3 | 2,64 | 0,00224 | 0,0309 | 3'UTR | 124155 | ENSG00000129562 | DAD1 |
| chr7 | 38211369 | 38211738 | 1,78 | -1,35 | 3,12 | 0,00224 | 0,0309 | Distal Intergenic | -6070 | ENSG00000010270 | STARD3NL |
| chr2 | 142336607 | 142337334 | 1,89 | -1,55 | 3,44 | 0,00224 | 0,0309 | Intron (uc002tvj.1/53353, intron 2 of 90) | 551936 | ENSG00000168702 | LRP1B |
| chr5 | 92930616 | 92931997 | 2,65 | -0,81 | 3,47 | 0,00224 | 0,0309 | Distal Intergenic | 9886 | ENSG00000175745 | NR2F1 |
| chr6 | 91167975 | 91168771 | 2,28 | -1,23 | 3,51 | 0,00224 | 0,0309 | Distal Intergenic | 78166 | ENSG00000135341 | MAP3K7 |
| chr1 | 120173974 | 120174718 | 3,25 | -0,54 | 3,79 | 0,00224 | 0,0309 | Intron (uc001ehv.1/90874, intron 1 of 2) | 15672 | ENSG00000143067 | ZNF697 |
| chr2 | 142221798 | 142222663 | 2,12 | -1,38 | 3,5 | 0,00225 | 0,031 | Intron (uc002tvj.1/53353, intron 3 of 90) | 666607 | ENSG00000168702 | LRP1B |
| chr5 | 20636024 | 20636853 | 2,41 | -1,17 | 3,57 | 0,00225 | 0,031 | Intron (uc003jge.1/uc003jge.1, intron 1 of 11) | -60042 | ENSG00000145526 | CDH18 |
| chr1 | 157622626 | 157623081 | 2,09 | -0,84 | 2,94 | 0,00226 | 0,031 | Distal Intergenic | 44722 | ENSG00000160856 | FCRL3 |
| chr18 | 60649676 | 60650136 | 4,57 | 1,31 | 3,25 | 0,00226 | 0,031 | Distal Intergenic | 267004 | ENSG00000081913 | PHLPP1 |
| chr3 | 84468677 | 84469195 | 1,85 | -1,55 | 3,41 | 0,00226 | 0,031 | Distal Intergenic | 449531 | ENSG00000242641 | LINC00971 |
| chr8 | 76060892 | 76061486 | 2,61 | -0,8 | 3,42 | 0,00226 | 0,031 | Distal Intergenic | 148491 | ENSG00000121005 | CRISPLD1 |
| chr1 | 28593039 | 28593812 | 2,07 | -1,55 | 3,62 | 0,00226 | 0,031 | Intron (uc001bps.3/83667, intron 1 of 9) | 7076 | ENSG00000130766 | SESN2 |
| chr6 | 142987786 | 142988123 | 1,41 | -1,38 | 2,8 | 0,00226 | 0,0311 | Distal Intergenic | -28760 | ENSG00000236366 | LOC153910 |
| chr17 | 17751868 | 17752459 | 1,62 | -1,55 | 3,17 | 0,00226 | 0,0311 | Exon (uc002grv.4/146691, exon 13 of 14) | -11543 | ENSG00000072310 | SREBF1 |
| chr8 | 25501872 | 25502512 | 1,81 | -1,55 | 3,37 | 0,00226 | 0,0311 | Intron (uc003xek.3/5520, intron 7 of 10) | 176139 | ENSG00000 184661 | CDCA2 |
| chr5 | 28192562 | 28193417 | 4,45 | 1,51 | 2,94 | 0,00227 | 0,0312 | Distal Intergenic | 720163 | ENSG00000250337 | PURPL |
| chr5 | 90139192 | 90139756 | 1,65 | -1,55 | 3,2 | 0,00227 | 0,0312 | Intron (uc003kjt.4/84059, intron 78 of 89) | -55323 | ENSG00000 164199 | ADGRV1 |
| chr7 | 41477956 | 41478543 | 1,88 | -1,55 | 3,43 | 0,00227 | 0,0312 | Distal Intergenic | -254971 | ENSG00000224116 | INHBA-AS1 |
| chr19 | 5291648 | 5294269 | 5,95 | 1,95 | 3,99 | 0,00227 | 0,0312 | Intron (uc002mbu.1/5802, intron 1 of 30) | -5249 | ENSG00000105426 | PTPRS |
| chr8 | 134683260 | 134684478 | 4 | 0,76 | 3,24 | 0,00228 | 0,0312 | Distal Intergenic | -99077 | ENSG00000008513 | ST3GAL1 |
| chr3 | 176657127 | 176657491 | 1,78 | -1,26 | 3,03 | 0,00229 | 0,0313 | Distal Intergenic | 256775 | ENSG00000177565 | TBL1XR1 |
| chr1 | 92324806 | 92325685 | 3,22 | 0,13 | 3,09 | 0,00229 | 0,0313 | Promoter (1-2kb) | 1918 | ENSGO0000069702 | TGFBR3 |
| chr5 | 40484429 | 40485947 | 3,84 | 0,35 | 3,5 | 0,00229 | 0,0313 | Distal Intergenic | -194085 | ENSG00000171522 | PTGER4 |
| chr4 | 32604246 | 32604755 | 1,8 | -1,55 | 3,36 | 0,0023 | 0,0314 | Distal Intergenic | 1882209 | ENSG00000169851 | PCDH7 |
| chr8 | 48343790 | 48344609 | 1,85 | -1,55 | 3,4 | 0,00231 | 0,0315 | Intron (uc011lcz.2/23514, intron 6 of 7) | -8276 | ENSG00000164808 | SPIDR |
| chr22 | 19156625 | 19157699 | 5,19 | 1,75 | 3,44 | 0,00232 | 0,0316 | Distal Intergenic | 8319 | ENSG00000100075 | SLC25A1 |
| chr4 | 131466285 | 131466808 | 1,79 | -1,55 | 3,34 | 0,00233 | 0,0317 | Distal Intergenic | 1449003 | ENSG00000151470 | C4orf33 |
| chr12 | 122429703 | 122430339 | 1,84 | -1,55 | 3,4 | 0,00233 | 0,0317 | Intron (uc009zxk.3/144406, intron 19 of 21) | -29522 | ENSG00000110987 | BCL7A |
| chr7 | 116651516 | 116651979 | 1,75 | -1,55 | 3,3 | 0,00233 | 0,0318 | Promoter (2-3kb) | -2974 | ENSG00000004866 | ST7 |
| chr5 | 7779138 | 7779550 | 1,41 | -1,55 | 2,96 | 0,00235 | 0,032 | Promoter (2-3kb) | -2736 | ENSG00000078295 | ADCY2 |
| chrX | 142085929 | 142087263 | 3,96 | 0,85 | 3,11 | 0,00235 | 0,032 | Distal Intergenic | -26441 | ENSG00000189326 | SPANXN4 |
| chr4 | 117162747 | 117163237 | 1,7 | -1,55 | 3,25 | 0,00235 | 0,032 | Distal Intergenic | -57644 | ENSG00000284253 | MIR1973 |
| chr22 | 31198258 | 31199474 | 4,01 | 1,17 | 2,83 | 0,00236 | 0,0321 | Intron (uc011ala.1/23762, intron 3 of 14) | -19029 | ENSG00000 184792 | OSBP2 |
| chr7 | 121384688 | 121385415 | 2,14 | -1,1 | 3,23 | 0,00236 | 0,0321 | Distal Intergenic | -127744 | ENSG00000106278 | PTPRZ1 |
| chr9 | 102476088 | 102476728 | 1,82 | -1,55 | 3,37 | 0,00236 | 0,0321 | Intron (uc004bad.1/uc004bad.1, intron 3 of 6) | -107409 | ENSG00000119508 | NR4A3 |
| chr3 | 164987482 | 164988282 | 2,15 | -1,55 | 3,7 | 0,00236 | 0,0321 | Distal Intergenic | -73013 | ENSG00000121871 | SLITRK3 |
| chr4 | 88328703 | 88329303 | 1,74 | -1,06 | 2,8 | 0,00237 | 0,0321 | Distal Intergenic | -14425 | ENSG00000170502 | NUDT9 |
| chr9 | 71160079 | 71163854 | 5,09 | 0,95 | 4,14 | 0,00237 | 0,0321 | Distal Intergenic | -4296 | ENSG00000 181778 | TMEM252 |
| chrX | 5217952 | 5218903 | 1,94 | -1,55 | 3,5 | 0,00239 | 0,0323 | Distal Intergenic | 850909 | ENSG00000146938 | NLGN4X |
| chr1 1 | 9081805 | 9082284 | 1,89 | -0,97 | 2,86 | 0,00239 | 0,0324 | Exon (uc001mhi.2/57758, exon 8 of 22) | -9548 | ENSG00000175356 | SCUBE2 |
| chr7 | 53924929 | 53925381 | 1,38 | -1,55 | 2,93 | 0,0024 | 0,0324 | Distal Intergenic | -45305 | ENSG00000205628 | LINC01446 |
| chr20 | 10903091 | 10904078 | 2,05 | -1,26 | 3,31 | 0,0024 | 0,0324 | Distal Intergenic | -248397 | ENSG00000101384 | JAG1 |
| chr1 | 168697380 | 168698409 | 3,21 | 0,45 | 2,76 | 0,00241 | 0,0325 | Promoter (<=1kb) | 33 | ENSG00000143196 | DPT |
| chr4 | 168332467 | 168332981 | 1,73 | -1,55 | 3,29 | 0,00241 | 0,0325 | Distal Intergenic | -176726 | ENSG00000196104 | SPOCK3 |
| chr3 | 20102446 | 20103210 | 2,67 | -0,62 | 3,3 | 0,00241 | 0,0325 | Intron (uc003cbq.3/8850, intron 1 of 17) | 20922 | ENSG00000114166 | KAT2B |
| chr13 | 104651998 | 104652769 | 1,89 | -1,55 | 3,44 | 0,00241 | 0,0326 | Distal Intergenic | -717150 | ENSG00000263741 | MIR548AS |
| chr1 | 190796130 | 190796419 | 1,44 | -1,55 | 2,99 | 0,00242 | 0,0326 | Distal Intergenic | 202110 | ENSG00000231175 | LINC01720 |
| chr1 | 75862386 | 75863108 | 2,6 | -0,59 | 3,19 | 0,00242 | 0,0326 | Intron (uc001dgr.2/204962, intron 3 of 25) | 213691 | ENSG00000137968 | SLC44A5 |
| chr3 | 5656917 | 5657696 | 3,34 | -0,13 | 3,48 | 0,00242 | 0,0326 | Distal Intergenic | -364977 | ENSG00000265180 | MIR4790 |
| chr21 | 15314909 | 15315411 | 1,45 | -1,38 | 2,84 | 0,00243 | 0,0327 | Downstream (<1kb) | 37354 | ENSG00000215559 | ANKRD20A11P |
| chr6 | 80686485 | 80687131 | 2,42 | -0,72 | 3,14 | 0,00243 | 0,0327 | Distal Intergenic | -27191 | ENSG00000112742 | TTK |
| chr4 | 168797710 | 168798926 | 3,96 | 0,34 | 3,62 | 0,00243 | 0,0327 | Distal Intergenic | -214762 | ENSG00000109511 | ANXA10 |
| chr8 | 105502922 | 105504496 | 3,21 | -0,46 | 3,67 | 0,00243 | 0,0327 | 5'UTR | 3874 | ENSG00000147650 | LRP12 |
| chr9 | 12783873 | 12786348 | 5,04 | 1,33 | 3,7 | 0,00243 | 0,0327 | Intron (uc003zkw.3/286343, intron 1 of 1) | 8861 | ENSG00000153714 | LURAP1L |
| chr12 | 46964763 | 46965693 | 2,52 | -0,56 | 3,08 | 0,00244 | 0,0327 | Distal Intergenic | -198118 | ENSG00000134294 | SLC38A2 |
| chr4 | 25845123 | 25846005 | 3,85 | 0,9 | 2,95 | 0,00244 | 0,0328 | Intron (uc003gru.4/23231, intron 2 of 23) | 18605 | ENSG00000091490 | SEL1L3 |
| chr18 | 36001765 | 36002368 | 2,07 | -1,23 | 3,3 | 0,00244 | 0,0328 | Distal Intergenic | 764667 | ENSG00000266530 | MIR4318 |
| chr8 | 79990959 | 79991715 | 1,88 | -1,55 | 3,43 | 0,00244 | 0,0328 | Distal Intergenic | -273201 | ENSG00000104432 | IL7 |
| chr4 | 31415285 | 31416253 | 1,92 | -1,55 | 3,47 | 0,00244 | 0,0328 | Distal Intergenic | 693248 | ENSG00000169851 | PCDH7 |
| chr10 | 62579820 | 62580251 | 1,55 | -1,55 | 3,11 | 0,00245 | 0,0328 | Distal Intergenic | 41529 | ENSG00000170312 | CDK1 |
| chr9 | 77350485 | 77351381 | 3,71 | 0,51 | 3,2 | 0,00245 | 0,0328 | Intron (uc004ajj.1/140803, intron 13 of 15) | 39784 | ENSG00000119121 | TRPM6 |
| chr7 | 125211460 | 125212312 | 2,16 | -1,55 | 3,71 | 0,00245 | 0,0328 | Distal Intergenic | -641423 | ENSG00000128513 | POT1 |
| chr4 | 153384094 | 153384415 | 1,5 | -1,55 | 3,05 | 0,00247 | 0,0331 | Intron (uc003ims.3/55294, intron 1 of 11) | 26153 | ENSG00000264678 | MIR3140 |
| chr1 | 74928849 | 74929276 | 1,64 | -1,55 | 3,19 | 0,00247 | 0,0331 | Exon (uc001dgd.3/100526835, exon 23 of 24) | 171262 | ENSG00000178965 | ERICH3 |
| chr21 | 39807132 | 39807808 | 2,14 | -1,1 | 3,24 | 0,00247 | 0,0331 | Intron (uc021wjd.1/2078, intron 4 of 11) | -31501 | ENSG00000157554 | ERG |
| chrX | 133305709 | 133307934 | 5,86 | 2,16 | 3,7 | 0,00247 | 0,0331 | Promoter (1-2kb) | -1401 | ENSG00000284157 | MIR106A |
| chr9 | 82771440 | 82775349 | 5,82 | 2,11 | 3,72 | 0,00247 | 0,0331 | Distal Intergenic | 581652 | ENSG00000106829 | TLE4 |
| chr1 1 | 113957518 | 113958397 | 1,66 | -1,55 | 3,21 | 0,00248 | 0,0332 | Intron (uc001poo.1/7704, intron 2 of 3) | 26230 | ENSG00000109906 | ZBTB16 |
| chr7 | 114472707 | 114473570 | 2,62 | -0,62 | 3,24 | 0,00248 | 0,0332 | Distal Intergenic | -88639 | ENSG00000135272 | MDFIC |
| chr16 | 48570315 | 48571029 | 1,74 | -1,55 | 3,3 | 0,00248 | 0,0332 | Downstream (1-2kb) | 73091 | ENSG00000102921 | N4BP1 |
| chr1 1 | 185421 | 186436 | 1,91 | -1,55 | 3,46 | 0,00248 | 0,0332 | Distal Intergenic | -6644 | ENSG00000188076 | SCGB1C1 |
| chr9 | 114800323 | 114800819 | 1,7 | -1,01 | 2,71 | 0,00249 | 0,0333 | Intron (uc022bma.1/100422990, intron 2 of 2) | 105943 | ENSG00000266315 | MIR4668 |
| chr18 | 59514262 | 59515090 | 1,69 | -1,4 | 3,09 | 0,00249 | 0,0333 | Intron (uc0021ih.1/220441, intron 1 of 1) | 45214 | ENSG00000176641 | RNF152 |
| chr7 | 83753281 | 83753749 | 1,74 | -1,55 | 3,29 | 0,00249 | 0,0333 | Intron (uc003uhz.3/10371, intron 3 of 16) | 70468 | ENSG00000075213 | SEMA3 A |
| chr3 | 175779505 | 175780884 | 2,52 | -0,8 | 3,32 | 0,0025 | 0,0334 | Distal Intergenic | 692176 | ENSG00000264974 | MIR4789 |
| chr5 | 29792963 | 29793896 | 4 | 0,48 | 3,52 | 0,0025 | 0,0334 | Distal Intergenic | 865986 | NA | LSP1P3 |
| chr2 | 234266830 | 234267380 | 1,73 | -1,55 | 3,29 | 0,00251 | 0,0335 | Intron (uc002vui.1/8527, intron 1 of 29) | 3677 | ENSG00000077044 | DGKD |
| chr5 | 39659387 | 39660076 | 2,17 | -0,77 | 2,94 | 0,00252 | 0,0336 | Distal Intergenic | -234052 | ENSG00000153071 | DAB2 |
| chr8 | 115399475 | 115400030 | 1,74 | -1,55 | 3,3 | 0,00252 | 0,0336 | Distal Intergenic | -950233 | ENSG00000164796 | CSMD3 |
| chr4 | 166907063 | 166911181 | 4,45 | 0,47 | 3,97 | 0,00252 | 0,0336 | 5'UTR | 112653 | ENSG00000038295 | TLL1 |
| chr7 | 43741900 | 43742358 | 1,44 | -1,55 | 2,99 | 0,00253 | 0,0337 | Intron (uc003tij.4/55744, intron 1 of 8) | 26782 | ENSG00000106603 | COA1 |
| chr19 | 49013590 | 49015827 | 6,83 | 3,67 | 3,16 | 0,00253 | 0,0337 | Promoter (<=1kb) | 619 | ENSG00000142235 | LMTK3 |
| chr13 | 68838660 | 68839230 | 1,85 | -1,55 | 3,4 | 0,00253 | 0,0337 | Distal Intergenic | 620227 | ENSG00000281778 | LINC00550 |
| chr5 | 165161801 | 165162520 | 2,83 | -0,62 | 3,45 | 0,00253 | 0,0337 | Distal Intergenic | -1549323 | ENSG00000145934 | TENM2 |
| chr9 | 30471634 | 30472327 | 1,93 | -1,55 | 3,48 | 0,00253 | 0,0337 | Distal Intergenic | -63182 | NA | LINC01242 |
| chr7 | 71821357 | 71822356 | 2,61 | -0,72 | 3,33 | 0,00254 | 0,0338 | Intron (uc003twb.4/83698, intron 2 of 6) | -19149 | ENSG00000183166 | CALN1 |
| chr7 | 44498864 | 44501084 | 5,15 | 2,27 | 2,88 | 0,00255 | 0,0338 | Intron (uc010kye.3/23386, intron 1 of 6) | 19977 | ENSG00000015676 | NUDCD3 |
| chr17 | 25624054 | 25624605 | 1,53 | -1,55 | 3,08 | 0,00255 | 0,0338 | Promoter (2-3kb) | 2948 | ENSG00000109046 | WSB1 |
| chr4 | 137077414 | 137078410 | 2,79 | -0,77 | 3,56 | 0,00255 | 0,0339 | Distal Intergenic | 1375219 | ENSG00000189184 | PCDH18 |
| chr12 | 103875884 | 103876565 | 2,72 | -0,24 | 2,96 | 0,00256 | 0,0339 | Intron (uc001tjs.3/374470, intron 1 of 10) | 13181 | ENSG00000179088 | C12orf42 |
| chr10 | 79284032 | 79284806 | 1,87 | -1,38 | 3,25 | 0,00256 | 0,0339 | Intron (uc001jxj.2/3778, intron 1 of 28) | 112771 | ENSG00000156113 | KCNMA1 |
| chr2 | 87773867 | 87774426 | 1,25 | -1,31 | 2,56 | 0,00256 | 0,034 | Intron (uc002srs.4/64795, intron 3 of 10) | 18893 | ENSG00000222041 | CYTOR |
| chr1 1 | 67557004 | 67558348 | 1,54 | -1,55 | 3,1 | 0,00256 | 0,034 | Exon (uc021qmk.1/uc021qmk.1, exon 1 of 1) | 14459 | ENSG00000160172 | FAM86C2P |
| chr1 | 163379528 | 163380235 | 1,66 | -1,4 | 3,06 | 0,00257 | 0,034 | Distal Intergenic | 87805 | ENSG00000143228 | NUF2 |
| chr1 1 | 40029231 | 40029745 | 1,83 | -1,55 | 3,38 | 0,00257 | 0,034 | Distal Intergenic | 284935 | ENSG00000148948 | LRRC4C |
| chr8 | 145002009 | 145005441 | 6,21 | 2,1 | 4,1 | 0,00257 | 0,0341 | Exon (uc003zab.1/5339, exon 20 of 32) | 8317 | ENSG00000178209 | PLEC |
| chr5 | 101926835 | 101927569 | 2,31 | -0,62 | 2,93 | 0,00259 | 0,0342 | Distal Intergenic | -92115 | ENSG00000205359 | SLCO6A1 |
| chr7 | 54318941 | 54319452 | 1,7 | -1,38 | 3,09 | 0,00259 | 0,0343 | Distal Intergenic | -48827 | NA | NA |
| chr14 | 32907443 | 32908834 | 3,53 | 0,57 | 2,96 | 0,0026 | 0,0343 | Intron (uc010aml.3/9472, intron 2 of 9) | 108964 | ENSG00000151320 | AKAP6 |
| chr15 | 65653921 | 65654509 | 1,74 | -1,55 | 3,29 | 0,0026 | 0,0343 | Intron (uc002aos.2/9543, intron2 of 13) | -5211 | ENSG00000174498 | IGDCC3 |
| chr2 | 21711203 | 21712628 | 4,03 | 1,1 | 2,93 | 0,0026 | 0,0344 | Distal Intergenic | 220896 | ENSGO0000229621 | LINC01822 |
| chr4 | 161471958 | 161472638 | 1,72 | -1,55 | 3,27 | 0,00261 | 0,0345 | Distal Intergenic | 1282960 | ENSG00000109756 | RAPGEF2 |
| chr7 | 127411951 | 127412411 | 1,7 | -1,11 | 2,8 | 0,00262 | 0,0346 | Intron (uc003vmi.3/27044, intron 10 of 23) | 53301 | ENSG00000 197157 | SND1 |
| chr1 1 | 97874765 | 97875304 | 1,55 | -1,55 | 3,1 | 0,00263 | 0,0347 | Distal Intergenic | -1016402 | ENSG00000149972 | CNTN5 |
| chr3 | 161368053 | 161368456 | 1,56 | -1,55 | 3,12 | 0,00263 | 0,0347 | Distal Intergenic | 153457 | ENSG00000 182447 | OTOL1 |
| chr10 | 49544401 | 49545923 | 3,45 | -0,04 | 3,49 | 0,00263 | 0,0347 | Intron (uc001jgn.3/5599, intron 1 of 11) | 29615 | ENSG00000107643 | MAPK8 |
| chr1 1 | 40302374 | 40303082 | 2,01 | -1,55 | 3,57 | 0,00263 | 0,0347 | Intron (uc001mxb.2/57689, intron 1 of 2) | 11598 | ENSG00000148948 | LRRC4C |
| chr1 1 | 118318168 | 118320063 | 4,8 | 1,47 | 3,33 | 0,00264 | 0,0348 | Intron (uc001psz.1/4297, intron2 of 3) | 10963 | ENSG00000118058 | KMT2A |
| chr10 | 79401204 | 79401714 | 1,56 | -1,55 | 3,12 | 0,00265 | 0,0348 | Distal Intergenic | -3627 | ENSG00000156113 | KCNMA1 |
| chr6 | 21190850 | 21191696 | 2,26 | -1,14 | 3,39 | 0,00265 | 0,0349 | Intron (uc003ndd.2/54901, intron 13 of 15) | 125571 | ENSG00000145996 | CDKAL1 |
| chr8 | 75250744 | 75252079 | 3,76 | 0,08 | 3,67 | 0,00265 | 0,0349 | Distal Intergenic | -10539 | ENSG00000104381 | GDAP1 |
| chr2 | 147329836 | 147330355 | 1,64 | -1,38 | 3,02 | 0,00266 | 0,035 | Distal Intergenic | -14270 | NA | PABPC1P2 |
| chr13 | 23648759 | 23649333 | 1,59 | -1,55 | 3,14 | 0,00266 | 0,035 | Distal Intergenic | -105727 | ENSG00000102683 | SGCG |
| chr3 | 166146462 | 166147124 | 2,03 | -1,55 | 3,59 | 0,00266 | 0,035 | Distal Intergenic | -591209 | ENSG00000114200 | BCHE |
| chr1 | 117420451 | 117422524 | 4,39 | 0,72 | 3,67 | 0,00266 | 0,035 | Distal Intergenic | -30165 | ENSG00000134247 | PTGFRN |
| chr1 | 146549690 | 146552793 | 5,3 | 1,56 | 3,75 | 0,00266 | 0,035 | Exon (uc021ovj.1/uc021ovj.1, exon 1 of 1) | 33135 | NA | NBPF13P |
| chr6 | 107423716 | 107424265 | 1,68 | -1,26 | 2,95 | 0,00267 | 0,0351 | Intron (uc003prs.2/57673, intron 2 of 4) | 11371 | ENSG00000178409 | BEND3 |
| chr3 | 99591260 | 99591881 | 2,3 | -1,06 | 3,36 | 0,00267 | 0,0351 | Intron (uc003dtk.2/84319, intron 1 of 4) | 3165 | ENSG00000168386 | FILIP1L |
| chr9 | 139934828 | 139936176 | 3,05 | -0,4 | 3,45 | 0,00267 | 0,0351 | Promoter (2-3kb) | 2368 | ENSG00000107281 | NPDC1 |
| chr18 | 57007713 | 57010155 | 4,01 | 0,24 | 3,78 | 0,00268 | 0,0351 | Intron (uc0021hz.3/3998, intron 8 of 12) | 16353 | ENSG00000074695 | LMAN1 |
| chr5 | 117228000 | 117228652 | 1,68 | -1,55 | 3,23 | 0,00268 | 0,0352 | Distal Intergenic | 476792 | NA | LINC00992 |
| chr12 | 113007719 | 113010151 | 5,45 | 1,37 | 4,09 | 0,00269 | 0,0352 | Promoter (2-3kb) | -2750 | ENSGO0000089169 | RPH3 A |
| chr13 | 32525250 | 32525799 | 1,55 | -1,55 | 3,1 | 0,00269 | 0,0353 | Exon (uc001utv.3/uc001utv.3, exon 1 of 1) | -79638 | ENSG00000073910 | FRY |
| chr4 | 93319572 | 93320669 | 2,8 | -0,47 | 3,27 | 0,00269 | 0,0353 | Intron (uc010ikx.3/2895, intron 1 of 5) | 94022 | ENSG00000152208 | GRID2 |
| chrX | 141385900 | 141386870 | 2,92 | -0,31 | 3,23 | 0,0027 | 0,0353 | Distal Intergenic | -92824 | ENSG00000046774 | MAGEC2 |
| chr12 | 78367027 | 78367650 | 1,85 | -1,55 | 3,4 | 0,0027 | 0,0353 | Intron (uc001sv0.3/89795, intron 5 of 38) | -62993 | ENSG00000067798 | NAV3 |
| chr2 | 226636212 | 226636729 | 1,45 | -1,55 | 3 | 0,0027 | 0,0354 | Distal Intergenic | 8051 | ENSG00000264539 | MIR548AR |
| chr7 | 82135889 | 82136572 | 2 | -1,55 | 3,56 | 0,00271 | 0,0354 | Distal Intergenic | -62858 | ENSG00000153956 | CACNA2D1 |
| chrl6 | 56977520 | 56978202 | 1,77 | -1,55 | 3,32 | 0,00272 | 0,0355 | 3'UTR | 7657 | ENSG00000051108 | HERPUD1 |
| chr8 | 145725126 | 145726996 | 5,95 | 1,72 | 4,22 | 0,00272 | 0,0355 | Promoter (2-3kb) | -2469 | ENSG00000167701 | GPT |
| chr10 | 112011506 | 112011808 | 1,51 | -1,55 | 3,07 | 0,00272 | 0,0356 | Intron (uc001kyy.3/4601, intron 3 of 5) | 24265 | ENSG00000119950 | MXI1 |
| chr1 1 | 16926911 | 16927721 | 2,06 | -1,17 | 3,22 | 0,00273 | 0,0356 | Intron (uc010rcu.1/144100, intron 3 of 26) | -78774 | ENSG00000166689 | PLEKHA7 |
| chr1 1 | 63376194 | 63377804 | 4,18 | 0,57 | 3,62 | 0,00273 | 0,0356 | Intron (uc001nxh.2/11145, intron 1 of 3) | 4105 | ENSG00000176485 | PLAAT3 |
| chr4 | 41292141 | 41292943 | 2,85 | -0,28 | 3,13 | 0,00275 | 0,0358 | Distal Intergenic | 33243 | ENSG00000154277 | UCHL1 |
| chr3 | 56070367 | 56070868 | 1,84 | -1,55 | 3,39 | 0,00275 | 0,0358 | Intron (uc003dhr.1/26059, intron 7 of 16) | 44144 | ENSG00000187672 | ERC2 |
| chr2 | 35209188 | 35210053 | 1,98 | -1,55 | 3,53 | 0,00275 | 0,0358 | Distal Intergenic | -1255904 | ENSG00000239649 | MYADML |
| chr8 | 71612560 | 71613857 | 1,74 | -1,55 | 3,29 | 0,00276 | 0,0359 | Intron (uc003xvq.3/389668, intron 3 of 4) | 30960 | ENSG00000221947 | XKR9 |
| chr3 | 24719220 | 24720037 | 1,75 | -1,55 | 3,3 | 0,00276 | 0,0359 | Distal Intergenic | -156294 | NA | NA |
| chr5 | 125035986 | 125036438 | 1,69 | -1,55 | 3,24 | 0,00277 | 0,036 | Distal Intergenic | -659350 | ENSG00000155324 | GRAMD2B |
| chr13 | 79966734 | 79968851 | 4,99 | 1,3 | 3,7 | 0,00277 | 0,0361 | Intron (uc00 1vky.2/64062, intron 1 of 20) | 11072 | ENSG00000139746 | RBM26 |
| chr3 | 144940455 | 144940904 | 1,54 | -1,55 | 3,1 | 0,00278 | 0,0361 | Distal Intergenic | 863618 | ENSG00000152952 | PLOD2 |
| chr1 1 | 36375398 | 36376719 | 3,32 | 0,06 | 3,26 | 0,00278 | 0,0361 | Intron (uc001mwo.4/79899, intron 1 of 8) | -20816 | ENSG00000135362 | PRR5L |
| chr4 | 52917261 | 52918673 | 4,94 | 1,48 | 3,46 | 0,00278 | 0,0362 | Promoter (<=1kb) | 0 | ENSG00000163071 | SPATA18 |
| chr3 | 183095305 | 183095675 | 1,99 | -1,01 | 3 | 0,00279 | 0,0362 | Intron (uc003fli.1/23101, intron 3 of 29) | 22661 | ENSG00000053524 | MCF2L2 |
| chr2 | 232891125 | 232891754 | 3,14 | 0,09 | 3,04 | 0,00279 | 0,0362 | Promoter (2-3kb) | 2138 | ENSG00000144535 | DIS3L2 |
| chrX | 17566993 | 17568559 | 4,11 | 0,75 | 3,36 | 0,00279 | 0,0362 | Intron(uc004cxx.3/4810,intron 1 of 7) | -84854 | ENSG00000188158 | NHS |
| chr2 | 56456632 | 56457733 | 2,72 | -0,69 | 3,41 | 0,00279 | 0,0362 | Intron (uc021vhw.1/114800, intron 3 of 4) | 45374 | ENSG00000055813 | CCDC85A |
| chr4 | 167647723 | 167648564 | 1,9 | -1,55 | 3,45 | 0,00279 | 0,0362 | Distal Intergenic | 506760 | ENSG00000196104 | SPOCK3 |
| chr2 | 183794953 | 183795918 | 1,99 | -1,55 | 3,54 | 0,00279 | 0,0362 | Exon (uc002upb.4/10787, exon 28 of 32) | -63455 | ENSG00000162998 | FRZB |
| chr4 | 105314778 | 105316758 | 4,51 | 0,94 | 3,57 | 0,00279 | 0,0362 | Distal Intergenic | 99300 | ENSG00000168772 | CXXC4 |
| chr17 | 57903813 | 57908521 | 7,21 | 3,35 | 3,86 | 0,00279 | 0,0362 | Intron (uc002ixu.4/81671, intron 10 of 11) | -10106 | ENSG00000284190 | MIR21 |
| chr4 | 69990755 | 69991575 | 2,47 | -0,39 | 2,86 | 0,0028 | 0,0363 | Distal Intergenic | 28562 | ENSG00000171234 | UGT2B7 |
| chr3 | 143995521 | 143995919 | 1,72 | -1,38 | 3,1 | 0,0028 | 0,0363 | Distal Intergenic | 303354 | ENSG00000 181744 | DIPK2A |
| chr3 | 170188350 | 170188770 | 1,43 | -1,55 | 2,98 | 0,00281 | 0,0364 | Intron(uc011bpt.1/5010,intron 2 of 3) | 49322 | ENSG00000013297 | CLDN11 |
| chr18 | 70705482 | 70705862 | 1,53 | -1,55 | 3,08 | 0,00281 | 0,0364 | Distal Intergenic | -170672 | ENSG00000166342 | NETO1 |
| chr13 | 68708163 | 68709770 | 4,58 | 1,46 | 3,12 | 0,00281 | 0,0364 | Distal Intergenic | 749687 | ENSG00000281778 | LINC00550 |
| chr4 | 130889178 | 130889921 | 1,69 | -1,55 | 3,24 | 0,00281 | 0,0364 | Distal Intergenic | 871896 | ENSG00000151470 | C4orf3 3 |
| chr10 | 65625295 | 65626787 | 4 | 0,7 | 3,3 | 0,00281 | 0,0364 | Distal Intergenic | 344172 | ENSG00000165476 | REEP3 |
| chr3 | 95641753 | 95642477 | 2,18 | -1,14 | 3,32 | 0,00281 | 0,0364 | Distal Intergenic | -239716 | ENSG00000244681 | MTHFD2P1 |
| chr21 | 47788454 | 47789045 | 2,08 | -1,14 | 3,22 | 0,00282 | 0,0365 | Intron (uc002zji.4/5116, intron 15 of 46) | 34143 | ENSG00000160299 | PCNT |
| chr9 | 14097827 | 14098471 | 2,11 | -0,91 | 3,02 | 0,00283 | 0,0366 | Intron (uc003z1d.3/4781, intron 10 of 10) | 82326 | ENSG00000147862 | NFIB |
| chr21 | 26590511 | 26590981 | 1,65 | -1,55 | 3,21 | 0,00283 | 0,0366 | Distal Intergenic | 213032 | ENSG00000185433 | LINC00158 |
| chr7 | 11671989 | 11672612 | 1,78 | -1,55 | 3,33 | 0,00283 | 0,0366 | Intron (uc021zzn.1/221981, intron 2 of 26) | 199212 | ENSG00000005108 | THSD7A |
| chr14 | 102904472 | 102904829 | 2,2 | -0,86 | 3,06 | 0,00284 | 0,0366 | Exon (uc010aw1.3/9895, exon 10 of 17) | 70325 | ENSG00000156381 | ANKRD9 |
| chr1 | 92162867 | 92163699 | 2,87 | -0,47 | 3,34 | 0,00284 | 0,0366 | Exon (uc009wde.3/7049, exon 18 of 20) | 163904 | ENSG00000069702 | TGFBR3 |
| chr2 | 153361946 | 153362563 | 1,61 | -1,55 | 3,16 | 0,00285 | 0,0367 | Intron (uc002tve.3/114793, intron 1 of 25) | -113460 | ENSG00000157827 | FMNL2 |
| chr5 | 26852741 | 26853533 | 2,15 | -1,38 | 3,53 | 0,00285 | 0,0367 | Distal Intergenic | 37409 | ENSG00000113100 | CDH9 |
| chr15 | 60507778 | 60508509 | 1,73 | -1,55 | 3,28 | 0,00287 | 0,0368 | Distal Intergenic | 181676 | ENSG00000182718 | ANXA2 |
| chr7 | 114705975 | 114706568 | 1,53 | -1,55 | 3,08 | 0,00287 | 0,0369 | Distal Intergenic | 143066 | ENSG00000135272 | MDFIC |
| chr2 | 181972940 | 181973294 | 1,54 | -1,55 | 3,09 | 0,00287 | 0,0369 | Distal Intergenic | 127089 | ENSG00000170035 | UBE2E3 |
| chr6 | 45403383 | 45403841 | 1,82 | -1,55 | 3,37 | 0,00287 | 0,0369 | Intron (uc01 1dvx.2/860, intron 4 of 8) | 13469 | ENSG00000124813 | RUNX2 |
| chr8 | 48515186 | 48515848 | 1,85 | -1,55 | 3,41 | 0,00287 | 0,0369 | Intron (uc0101xs.3/23514, intron 6 of 10) | -50802 | ENSG00000164808 | SPIDR |
| chr1 1 | 23970084 | 23970920 | 2,06 | -1,55 | 3,61 | 0,00287 | 0,0369 | Distal Intergenic | -547596 | ENSG00000187398 | LUZP2 |
| chr1 | 89636473 | 89636888 | 1,59 | -1,55 | 3,14 | 0,00288 | 0,0369 | Intron (uc001dna.2/388646, intron 2 of 10) | 4835 | ENSG00000213512 | GBP7 |
| chr5 | 55444492 | 55445583 | 2,77 | -0,42 | 3,19 | 0,00288 | 0,0369 | Intron (uc003jqu.3/79722, intron 6 of 11) | -31714 | ENSG00000164512 | ANKRD55 |
| chr3 | 152456085 | 152456936 | 2,13 | -0,75 | 2,88 | 0,00288 | 0,037 | Distal Intergenic | -95800 | ENSG00000169860 | P2RY1 |
| chr7 | 63151291 | 63152409 | 1,54 | -1,55 | 3,09 | 0,00288 | 0,037 | Distal Intergenic | 69823 | ENSG00000264426 | MIR4283-1 |
| chr2 | 139880085 | 139881059 | 2,81 | -0,56 | 3,37 | 0,00288 | 0,037 | Distal Intergenic | 225191 | NA | YY1P2 |
| chr1 1 | 11358035 | 11358924 | 2,78 | -0,2 | 2,97 | 0,00289 | 0,0371 | Intron (uc001mjo.2/374378, intron 7 of 10) | 15980 | ENSG00000254598 | CSNK2A3 |
| chr8 | 106146068 | 106147110 | 2,62 | -0,56 | 3,18 | 0,00289 | 0,0371 | Distal Intergenic | -184037 | ENSG00000169946 | ZFPM2 |
| chr15 | 54442161 | 54442447 | 1,28 | -1,55 | 2,83 | 0,0029 | 0,0371 | Intron (uc021smr.1/440279. intron 3 of 30) | -113896 | ENSG00000137766 | UNC13C |
| chr1 | 182595490 | 182595978 | 1,52 | -1,55 | 3,07 | 0,0029 | 0,0371 | Distal Intergenic | 11215 | ENSG00000261504 | LINC01686 |
| chr17 | 51695236 | 51696271 | 3,75 | 0,44 | 3,31 | 0,0029 | 0,0371 | Distal Intergenic | -203968 | ENSG00000141200 | KIF2B |
| chr17 | 14026624 | 14031612 | 5,97 | 2,12 | 3,86 | 0,0029 | 0,0371 | Intron (uc002gof.4/1352, intron 4 of 6) | -53849 | ENSG00000236088 | COX10-AS1 |
| chr7 | 119625555 | 119626365 | 1,67 | -1,55 | 3,22 | 0,00291 | 0,0373 | Distal Intergenic | -287357 | ENSG00000 184408 | KCND2 |
| chrX | 34405970 | 34406457 | 1,93 | -1,23 | 3,17 | 0,00292 | 0,0373 | Distal Intergenic | -255523 | ENSG00000185448 | FAM47A |
| chr9 | 89794596 | 89795232 | 1,72 | -1,55 | 3,27 | 0,00292 | 0,0373 | Distal Intergenic | 31037 | ENSG00000204446 | C9orf170 |
| chr5 | 109039489 | 109041091 | 3,34 | -0,31 | 3,65 | 0,00292 | 0,0373 | Intron (uc003kou.1/4124, intron 1 of 21) | 14333 | ENSG00000112893 | MAN2A1 |
| chr18 | 13610303 | 13615842 | 7,97 | 6,43 | 1,53 | 0,00292 | 0,0374 | Promoter (<=1kb) | 0 | ENSG00000263527 | MIR4526 |
| chr17 | 74944470 | 74945006 | 1,75 | -1,55 | 3,3 | 0,00293 | 0,0374 | Promoter (2-3kb) | 2138 | ENSG00000167889 | MGAT5B |
| chr2 | 229462623 | 229463251 | 2,08 | -1,55 | 3,63 | 0,00293 | 0,0374 | Distal Intergenic | -416262 | ENSG00000153820 | SPHKAP |
| chr8 | 83169915 | 83170971 | 3,78 | 0,73 | 3,04 | 0,00294 | 0,0375 | Distal Intergenic | -415394 | ENSG00000104497 | SNX16 |
| chr10 | 34569372 | 34569833 | 1,71 | -1,55 | 3,27 | 0,00294 | 0,0375 | Intron (uc010qej.2/56288, intron 21 of 24) | 103349 | ENSG00000148498 | PARD3 |
| chr1 1 | 110219702 | 110220607 | 3,28 | -0,04 | 3,32 | 0,00294 | 0,0375 | Intron (uc001pkv.1/uc001pkv.1, intron 3 of 5) | -52265 | ENSG00000137710 | RDX |
| chr4 | 86860497 | 86861352 | 2,74 | -0,57 | 3,31 | 0,00295 | 0,0377 | Intron (uc003hpj.3/83478, intron 4 of 5) | 9071 | ENSG00000138639 | ARHGAP24 |
| chr7 | 7432722 | 7433224 | 1,77 | -1,1 | 2,86 | 0,00296 | 0,0377 | Intron (uc003src.1/340267, intron 27 of 34) | 43869 | ENSG00000215018 | COL28A1 |
| chr17 | 34409289 | 34410337 | 3,73 | 1,12 | 2,61 | 0,00297 | 0,0378 | Distal Intergenic | 7169 | ENSG00000277632 | CCL3 |
| chr10 | 101736024 | 101736648 | 1,7 | -1,55 | 3,26 | 0,00297 | 0,0378 | Intron (uc001kqj.2/23268, intron 1 of 16) | 33028 | ENSG00000107554 | DNMBP |
| chr10 | 54789721 | 54790311 | 2,52 | -0,24 | 2,77 | 0,00298 | 0,0379 | Distal Intergenic | -258261 | ENSG00000165471 | MBL2 |
| chr7 | 118620498 | 118621446 | 3,63 | 0,14 | 3,49 | 0,00298 | 0,0379 | Distal Intergenic | 755786 | ENSG00000106013 | ANKRD7 |
| chr10 | 121426661 | 121428344 | 4,06 | 0,34 | 3,72 | 0,00298 | 0,0379 | Intron (uc001lel.3/9531, intron 1 of 3) | 15779 | ENSG00000151929 | BAG3 |
| chr2 | 64765991 | 64766632 | 1,64 | -1,55 | 3,19 | 0,00299 | 0,038 | Intron (uc002scz.3/54812, intron 1 of 9) | -11945 | ENSG00000 119844 | AFTPH |
| chr6 | 41340400 | 41341929 | 4,3 | 0,78 | 3,52 | 0,00299 | 0,038 | Distal Intergenic | 36872 | ENSG00000096264 | NCR2 |
| chr1 1 | 133990377 | 133991656 | 3,86 | 0,79 | 3,07 | 0,003 | 0,0381 | Intron (uc001qhb.3/83700, intron 1 of 8) | 51557 | ENSG00000166086 | JAM3 |
| chr6 | 148778435 | 148779224 | 1,72 | -1,55 | 3,27 | 0,003 | 0,0381 | Intron (uc003qme.1/23328, intron 4 of 19) | -50676 | ENSG00000 111961 | SASH1 |
| chr4 | 182659284 | 182659945 | 1,8 | -1,55 | 3,35 | 0,003 | 0,0381 | Distal Intergenic | -405195 | ENSG00000218336 | TENM3 |
| chr6 | 135449714 | 135450145 | 1,4 | -1,55 | 2,95 | 0,00301 | 0,0381 | Distal Intergenic | -52308 | ENSG00000118513 | MYB |
| chr5 | 169998869 | 170000147 | 3,44 | 0,49 | 2,95 | 0,00301 | 0,0381 | Intron (uc003map.3/30820, intron 1 of 7) | 67821 | ENSG00000182132 | KCNIP1 |
| chr7 | 2288393 | 2289546 | 3,53 | 0,54 | 2,99 | 0,00301 | 0,0381 | Exon (uc003slp.1/4521, exon 4 of 5) | 5853 | ENSG00000106268 | NUDT1 |
| chr5 | 116515811 | 116516479 | 2,3 | -0,86 | 3,15 | 0,00301 | 0,0381 | Distal Intergenic | -234729 | NA | LINC00992 |
| chr1 1 | 8498828 | 8499580 | 1,72 | -1,55 | 3,27 | 0,00301 | 0,0381 | Promoter (1-2kb) | -1457 | ENSG00000130413 | STK33 |
| chr1 | 241007075 | 241007666 | 2,11 | -0,69 | 2,8 | 0,00303 | 0,0383 | Intron (uc010pyh.2/6000, intron 9 of 17) | 24486 | ENSG00000182901 | RGS7 |
| chr12 | 31341163 | 31341914 | 1,68 | -1,55 | 3,24 | 0,00304 | 0,0384 | Intron (uc010sjy.1/uc010sjy.1, intron 3 of 29) | 85802 | ENSG00000013573 | DDX11 |
| chr1 | 15427081 | 15427652 | 2,22 | -0,83 | 3,04 | 0,00305 | 0,0385 | Promoter (<=1kb) | -52 | ENSG00000189337 | KAZN |
| chr9 | 5944934 | 5945533 | 1,56 | -1,55 | 3,11 | 0,00305 | 0,0385 | Exon (uc003zjq.4/158358, exon 5 of 8) | 8562 | ENSG00000183354 | KIAA2026 |
| chr1 | 104684166 | 104684728 | 2,05 | -1,12 | 3,17 | 0,00305 | 0,0385 | Distal Intergenic | 68521 | ENSG00000215869 | LOC100129138 |
| chr2 | 148084936 | 148085797 | 3,21 | -0,18 | 3,39 | 0,00306 | 0,0386 | Distal Intergenic | -516289 | ENSG00000121989 | ACVR2A |
| chr2 | 85869243 | 85869898 | 1,99 | -1,01 | 3 | 0,00307 | 0,0386 | Intron (uc002sqb.4/10713, intron 10 of 12) | 18760 | ENSG00000168878 | SFTPB |
| chrX | 108971428 | 108972428 | 2,38 | -0,62 | 3,01 | 0,00307 | 0,0386 | Intron (uc004eoi.2/2182, intron 1 of 16) | 4193 | ENSG00000068366 | ACSL4 |
| chr1 1 | 58359498 | 58360387 | 2,74 | -0,55 | 3,29 | 0,00307 | 0,0387 | Intron (uc001nmx.4/80829, intron 2 of 10) | 12911 | ENSG00000 186660 | ZFP91 |
| chr1 | 77902472 | 77904672 | 4,03 | 0,49 | 3,53 | 0,00307 | 0,0387 | Intron (uc001dhn.3/26289, intron 8 of 13) | 154185 | ENSG00000154027 | AK5 |
| chr4 | 150563453 | 150564204 | 2,17 | -1,01 | 3,18 | 0,00308 | 0,0388 | Intron (uc003ill.3/uc003ill.3, intron 9 of 16) | -435222 | ENSG00000170390 | DCLK2 |
| chr1 | 197182964 | 197183692 | 3,01 | -0,28 | 3,29 | 0,00308 | 0,0388 | Intron (uc010poz.2/23418, intron 1 of 14) | 12372 | ENSG00000134376 | CRB1 |
| chr3 | 69590851 | 69592076 | 3,12 | 0,09 | 3,03 | 0,00309 | 0,0388 | Promoter (<=1kb) | 0 | ENSG00000114541 | FRMD4B |
| chr7 | 28010266 | 28012342 | 4,02 | 0,49 | 3,53 | 0,00309 | 0,0388 | Intron (uc003szm.3/221895, intron 1 of 3) | 19333 | ENSG00000153814 | JAZF1 |
| chr3 | 19231999 | 19232533 | 1,81 | -1,06 | 2,87 | 0,00309 | 0,0389 | Intron (uc011awe.1/131096, intron 1 of 8) | 41982 | ENSG00000183960 | KCNH8 |
| chr5 | 31721086 | 31721617 | 1,41 | -1,55 | 2,96 | 0,00309 | 0,0389 | Intron (uc003jhl.3/23037, intron 1 of 24) | -77379 | ENSG00000133401 | PDZD2 |
| chr5 | 82583958 | 82584350 | 1,33 | -1,55 | 2,89 | 0,0031 | 0,0389 | Intron(uc003kia.1/7518.intron 7 of 7) | 81838 | ENSG00000152422 | XRCC4 |
| chr4 | 82430717 | 82431159 | 1,46 | -1,55 | 3,01 | 0,0031 | 0,0389 | Distal Intergenic | -37656 | ENSG00000138670 | RASGEF1B |
| chr1 | 199910402 | 199910777 | 1,48 | -1,55 | 3,04 | 0,0031 | 0,0389 | Distal Intergenic | -85953 | ENSG00000116833 | NR5 A2 |
| chr1 | 189956465 | 189956984 | 1,7 | -1,55 | 3,25 | 0,0031 | 0,0389 | Distal Intergenic | 487958 | ENSG00000162670 | BRINP3 |
| chr1 1 | 34929968 | 34930529 | 1,52 | -1,55 | 3,07 | 0,00311 | 0,039 | Intron (uc010reo.1/51074, intron 1 of 8) | -7148 | ENSG00000110435 | PDHX |
| chr3 | 36440799 | 36441580 | 1,85 | -1,29 | 3,13 | 0,00311 | 0,039 | Intron (uc010hgd.1/6769. intron 1 of 9) | 18702 | ENSG00000144681 | STAC |
| chr18 | 74812970 | 74814644 | 4,86 | 2,33 | 2,53 | 0,00312 | 0,0391 | Intron (uc010xfd.2/4155, intron 2 of 8) | 30130 | ENSG00000197971 | MBP |
| chr14 | 94275257 | 94275782 | 1,56 | -1,55 | 3,11 | 0,00312 | 0,0391 | Distal Intergenic | -20491 | ENSG00000175785 | PRIMA1 |
| chr2 | 189041921 | 189042472 | 1,74 | -1,55 | 3,29 | 0,00312 | 0,0391 | Distal Intergenic | -113924 | ENSG00000144366 | GULP1 |
| chr4 | 105831636 | 105832075 | 1,87 | -1,55 | 3,42 | 0,00313 | 0,0391 | Distal Intergenic | -234957 | ENSG00000168769 | TET2 |
| chr3 | 372067 | 372768 | 1,82 | -1,55 | 3,38 | 0,00313 | 0,0392 | Intron (uc003bot.3/10752, intron 5 of 27) | 10701 | ENSG00000134121 | CHL1 |
| chr14 | 59527945 | 59528627 | 2,6 | -0,26 | 2,85 | 0,00314 | 0,0393 | Distal Intergenic | -126754 | ENSG00000100592 | DAAM1 |
| chrX | 2810392 | 2811535 | 3,11 | -0,02 | 3,13 | 0,00314 | 0,0393 | Distal Intergenic | 35881 | ENSG00000006756 | ARSD |
| chr3 | 166145466 | 166145866 | 1,83 | -1,55 | 3,38 | 0,00314 | 0,0393 | Distal Intergenic | -590213 | ENSG00000114200 | BCHE |
| chr18 | 13641588 | 13642110 | 1,96 | -1,55 | 3,51 | 0,00314 | 0,0393 | Promoter (<=1kb) | 0 | ENSG00000168675 | LDLRAD4 |
| chr8 | 12035550 | 12039299 | 3,8 | 0,42 | 3,37 | 0,00315 | 0,0394 | Promoter (<=1kb) | -774 | NA | FAM90A2P |
| chr8 | 106200002 | 106200750 | 2,39 | -0,75 | 3,14 | 0,00316 | 0,0394 | Distal Intergenic | -130397 | ENSG00000169946 | ZFPM2 |
| chr2 | 229423915 | 229424658 | 2,07 | -1,4 | 3,47 | 0,00316 | 0,0394 | Distal Intergenic | -377554 | ENSG00000153820 | SPHKAP |
| chr8 | 134564469 | 134564949 | 2,46 | -0,72 | 3,18 | 0,00316 | 0,0395 | Intron (uc003vuk.2/6482, intron 1 of 9) | 19234 | ENSG00000008513 | ST3GAL1 |
| chr15 | 54599661 | 54600311 | 1,94 | -1,38 | 3,33 | 0,00316 | 0,0395 | Intron (uc021smr.1/440279, intron 11 of 30) | 43318 | ENSG00000137766 | UNC13C |
| chr12 | 104880342 | 104880919 | 1,71 | -1,26 | 2,97 | 0,00317 | 0,0395 | Intron (uc001tky.3/50515, intron 1 of 2) | 29650 | ENSG00000171310 | CHST11 |
| chr2 | 38823500 | 38824226 | 2,23 | -0,79 | 3,02 | 0,00317 | 0,0396 | Intron (uc021vgb.1/92906, intron 2 of 13) | 5952 | ENSG00000143889 | HNRNPLL |
| chr3 | 174723093 | 174723524 | 1,43 | -1,55 | 2,99 | 0,00318 | 0,0396 | Intron (uc003fit.3/254827, intron 1 of 13) | 109508 | ENSG00000230292 | NAALADL2 -AS3 |
| chr4 | 105887500 | 105887964 | 1,44 | -1,55 | 3 | 0,00318 | 0,0396 | Distal Intergenic | -179068 | ENSG00000168769 | TET2 |
| chr10 | 75807782 | 75808216 | 1,47 | -1,55 | 3,03 | 0,00318 | 0,0396 | Intron (uc009xrr.3/7414, intron 4 of 22) | 49910 | ENSG00000035403 | VCL |
| chr10 | 5484946 | 5486467 | 4,56 | 1,33 | 3,23 | 0,00318 | 0,0396 | Promoter (2-3kb) | -2047 | ENSG00000173848 | NET1 |
| chr2 | 51091785 | 51092846 | 3,06 | -0,34 | 3,4 | 0,00318 | 0,0396 | Intron (uc021vhg.1/9378, intron 5 of 22) | 163486 | ENSG00000179915 | NRXN1 |
| chr13 | 104535335 | 104537116 | 3,47 | -0,21 | 3,68 | 0,00318 | 0,0396 | Distal Intergenic | -600487 | ENSG00000263741 | MIR548AS |
| chr1 1 | 76087683 | 76088803 | 3,71 | 0,71 | 3 | 0,00319 | 0,0396 | Intron (uc001oxh.1/5612, intron 1 of 4) | 3077 | ENSG00000137492 | THAP12 |
| chr4 | 4385007 | 4385615 | 1,63 | -1,55 | 3,18 | 0,00319 | 0,0396 | Promoter (1-2kb) | -1969 | ENSG00000168824 | NSG1 |
| chrl7 | 29579801 | 29580414 | 1,74 | -1,55 | 3,3 | 0,00319 | 0,0396 | 3'UTR | -11833 | ENSG00000 196712 | NF1 |
| chr3 | 144633853 | 144634538 | 3,41 | 0,43 | 2,99 | 0,00319 | 0,0397 | Distal Intergenic | 941686 | ENSG00000 181744 | DIPK2A |
| chrl6 | 73069677 | 73071761 | 5,12 | 2,14 | 2,98 | 0,0032 | 0,0397 | Intron (uc002fck.3/463, intron 1 of 9) | 10513 | ENSG00000140836 | ZFHX3 |
| chr17 | 39832108 | 39833795 | 3,26 | -0,33 | 3,59 | 0,0032 | 0,0397 | Intron (uc010wfs.2/3728, intron 4 of 8) | -11332 | ENSG00000173812 | EIF1 |
| chr13 | 31998115 | 31998476 | 1,75 | -1,01 | 2,77 | 0,00321 | 0,0398 | Distal Intergenic | 163580 | ENSG00000187676 | B3GLCT |
| chr5 | 120166870 | 120168132 | 3,21 | -0,17 | 3,38 | 0,00321 | 0,0398 | Distal Intergenic | 214038 | ENSG00000184838 | PRR16 |
| chr7 | 53924345 | 53924739 | 1,44 | -1,55 | 2,99 | 0,00321 | 0,0399 | Distal Intergenic | -44721 | ENSG00000205628 | LINC01446 |
| chr3 | 171851488 | 171852722 | 3,7 | 0,8 | 2,9 | 0,00322 | 0,0399 | Promoter (<=1kb) | 227 | ENSG00000075420 | FNDC3B |
| chr4 | 186738386 | 186739128 | 2,54 | -0,2 | 2,74 | 0,00322 | 0,04 | Intron (uc003iyl.3/8470, intron 1 of 20) | -4976 | ENSG00000154556 | SORBS2 |
| chr1 1 | 39289054 | 39289872 | 1,83 | -1,55 | 3,39 | 0,00323 | 0,04 | Distal Intergenic | 1024808 | ENSG00000148948 | LRRC4C |
| chr3 | 72318742 | 72319205 | 1,44 | -1,55 | 2,99 | 0,00323 | 0,0401 | Distal Intergenic | 118334 | ENSG00000243083 | LINC00870 |
| chr6 | 15358033 | 15358646 | 1,46 | -1,55 | 3,01 | 0,00323 | 0,0401 | Intron (uc011diu.1/3720, intron 1 of 5) | -42443 | ENSG00000008083 | JARID2 |
| chr21 | 22699299 | 22699841 | 1,86 | -1,55 | 3,41 | 0,00323 | 0,0401 | Intron (uc002vld.2/4685, intron 6 of 17) | 41477 | NA | RNU6-67P |
| chr4 | 32560641 | 32561019 | 1,54 | -1,55 | 3,09 | 0,00324 | 0,0401 | Distal Intergenic | 1838604 | ENSG00000169851 | PCDH7 |
| chr16 | 11118295 | 11118999 | 2,22 | -0,62 | 2,84 | 0,00324 | 0,0402 | Exon (uc002dan.4/23274, exon 12 of 21) | 79950 | ENSG00000038532 | CLEC16A |
| chr8 | 51933503 | 51934051 | 1,58 | -1,55 | 3,13 | 0,00325 | 0,0402 | Distal Intergenic | 388070 | ENSG00000147485 | PXDNL |
| chr6 | 141572785 | 141574923 | 3,45 | -0,18 | 3,64 | 0,00325 | 0,0402 | Distal Intergenic | 567834 | ENSG00000264390 | MIR4465 |
| chr6 | 68122675 | 68123263 | 1,7 | -1,55 | 3,25 | 0,00325 | 0,0403 | Distal Intergenic | -1222369 | ENSG00000135298 | ADGRB3 |
| chr1 | 104060091 | 104060824 | 2,98 | -0,16 | 3,14 | 0,00326 | 0,0403 | Distal Intergenic | -7754 | ENSG00000185946 | RNPC3 |
| chr5 | 108259680 | 108261706 | 6,95 | 4,14 | 2,81 | 0,00328 | 0,0405 | Promoter (<=1kb) | 0 | ENSG00000151422 | FER |
| chr20 | 14812253 | 14812989 | 2,02 | -1,55 | 3,57 | 0,00328 | 0,0405 | Intron (uc002wot.3/140733, intron 5 of 16) | 97172 | ENSG00000235914 | MACROD2-AS1 |
| chr12 | 126845659 | 126848285 | 4,78 | 1,2 | 3,58 | 0,00328 | 0,0405 | Distal Intergenic | -78742 | ENSG00000214043 | LINC02347 |
| chr7 | 130579178 | 130579744 | 1,59 | -1,55 | 3,14 | 0,00328 | 0,0406 | Intron (uc022alq.1/646329, intron 1 of 2) | -16880 | ENSG00000283797 | MIR29B1 |
| chr9 | 131945891 | 131946266 | 1,59 | -1,31 | 2,9 | 0,00329 | 0,0406 | Distal Intergenic | -5351 | ENSG00000188483 | IER5L |
| chr5 | 88080823 | 88082767 | 3,89 | 0,16 | 3,73 | 0,00329 | 0,0406 | Intron (uc003kji.2/4208, intron 2 of 8) | 36977 | ENSG00000081189 | MEF2C |
| chr20 | 16294947 | 16299559 | 5,35 | 1,32 | 4,03 | 0,00329 | 0,0406 | Intron (uc002wpe.1/55614, intron 5 of 6) | 61249 | ENSG00000089177 | KIF16B |
| chrX | 45620993 | 45631801 | 7,9 | 3,18 | 4,72 | 0,00329 | 0,0406 | Distal Intergenic | -14463 | ENSG00000207725 | MIR222 |
| chr3 | 40226528 | 40227298 | 2,65 | -0,1 | 2,75 | 0,0033 | 0,0406 | Intron (uc003cka.3/25924, intron 9 of 16) | 85026 | ENSG00000170011 | MYRIP |
| chr8 | 110489553 | 110490057 | 1,93 | -1,02 | 2,95 | 0,00331 | 0,0407 | Exon (uc003yne.3/93035, exon 53 of78) | -61872 | ENSG00000147654 | EBAG9 |
| chr15 | 31648644 | 31649204 | 1,84 | -1,17 | 3,01 | 0,00331 | 0,0407 | Intron (uc001zfo.3/51621, intron 1 of 1) | 29561 | ENSG00000169926 | KLF13 |
| chr18 | 40969110 | 40969603 | 1,55 | -1,55 | 3,1 | 0,00333 | 0,0409 | Distal Intergenic | -111495 | ENSG00000132872 | SYT4 |
| chr1 | 226911527 | 226912202 | 1,62 | -1,55 | 3,17 | 0,00333 | 0,0409 | Intron (uc010pvo.2/3707, intron 2 of 7) | 14674 | ENSG00000143772 | ITPKB |
| chr1 | 198148986 | 198149417 | 1,64 | -1,55 | 3,19 | 0,00333 | 0,041 | Intron (uc001gun.4/140609, intron 1 of 9) | 22858 | ENSG00000151414 | NEK7 |
| chr12 | 102821102 | 102821655 | 1,64 | -1,55 | 3,19 | 0,00334 | 0,041 | Intron (uc001tjn.2/3479, intron 2 of 3) | 50774 | ENSG00000017427 | IGF1 |
| chr9 | 12729039 | 12729624 | 1,7 | -1,55 | 3,25 | 0,00334 | 0,0411 | Distal Intergenic | 35653 | ENSG00000107165 | TYRP1 |
| chr1 | 195489436 | 195490062 | 1,72 | -1,55 | 3,28 | 0,00334 | 0,0411 | Distal Intergenic | 821317 | ENSG00000162687 | KCNT2 |
| chr1 | 50575936 | 50577109 | 2,72 | -0,56 | 3,29 | 0,00334 | 0,0411 | Promoter (<=1kb) | 457 | ENSG00000162374 | ELAVL4 |
| chr16 | 21812060 | 21813750 | 1,74 | -1,55 | 3,3 | 0,00335 | 0,0411 | Exon (uc010vbl.1/730092, exon 9 of 12) | 16745 | NA | RRN3P1 |
| chr13 | 31308981 | 31313497 | 6,48 | 2,95 | 3,53 | 0,00335 | 0,0412 | Promoter (<=1kb) | 0 | ENSG00000132965 | ALOX5 AP |
| chr5 | 151158475 | 151159362 | 2,16 | -0,93 | 3,09 | 0,00336 | 0,0412 | Intron (uc010jhy.1/10146, intron 1 of 3) | 6999 | ENSG00000145907 | G3BP1 |
| chr14 | 92336001 | 92336987 | 3,01 | -0,12 | 3,13 | 0,00336 | 0,0412 | Promoter (2-3kb) | -2121 | ENSG00000165929 | TC2N |
| chr2 | 105593066 | 105593484 | 1,84 | -1,17 | 3 | 0,00337 | 0,0413 | Distal Intergenic | -60999 | ENSG00000135972 | MRPS9 |
| chr16 | 49399861 | 49400869 | 2,77 | -0,26 | 3,03 | 0,00338 | 0,0414 | Distal Intergenic | -6939 | ENSG00000 166152 | C16orf78 |
| chr2 | 6063374 | 6065422 | 4,67 | 1,53 | 3,13 | 0,00339 | 0,0415 | Distal Intergenic | -7397 | NA | SILC1 |
| chr4 | 175562512 | 175563238 | 1,71 | -1,55 | 3,26 | 0,00339 | 0,0415 | 3'UTR | -118463 | ENSG00000164120 | HPGD |
| chr4 | 11067623 | 11069365 | 4,67 | 1,37 | 3,29 | 0,00339 | 0,0415 | Distal Intergenic | -301086 | ENSG00000207716 | MIR572 |
| chr3 | 165090027 | 165090733 | 1,99 | -1,55 | 3,54 | 0,00339 | 0,0415 | Distal Intergenic | -175558 | ENSG00000121871 | SLITRK3 |
| chr18 | 12857101 | 12857499 | 1,41 | -1,38 | 2,79 | 0,0034 | 0,0416 | Intron (uc002krl.3/5771, intron 2 of 9) | 26835 | ENSG00000175354 | PTPN2 |
| chr3 | 160530265 | 160530937 | 1,65 | -1,55 | 3,2 | 0,0034 | 0,0416 | Intron (uc003fdr.3/151742, intron 1 of 3) | -29064 | ENSG00000163590 | PPM1L |
| chr12 | 105113868 | 105115110 | 4,67 | 0,69 | 3,98 | 0,0034 | 0,0416 | Intron (uc001tky.3/50515, intron 2 of 2) | 128457 | ENSG00000264295 | MIR3922 |
| chr13 | 65674029 | 65674452 | 1,36 | -1,55 | 2,92 | 0,00341 | 0,0417 | Distal Intergenic | 1362461 | NA | OR7E156P |
| chr12 | 22967184 | 22967712 | 1,51 | -1,55 | 3,06 | 0,00341 | 0,0417 | Distal Intergenic | 189108 | ENSG00000139163 | ETNK1 |
| chr7 | 80502403 | 80503010 | 1,51 | -1,55 | 3,07 | 0,00341 | 0,0417 | Intron (uc003uhj.3/10512, intron 2 of 17) | 45657 | ENSG00000075223 | SEMA3 C |
| chr3 | 71504677 | 71505815 | 2,65 | -0,52 | 3,16 | 0,00341 | 0,0417 | Intron (uc003don.3/27086, intron 1 of 21) | 36891 | ENSG00000114861 | FOXP1 |
| chr6 | 35633505 | 35634044 | 1,67 | -1,55 | 3,22 | 0,00341 | 0,0417 | Promoter (<=1kb) | -939 | ENSG00000265527 | MIR5690 |
| chr15 | 47247394 | 47248469 | 3,54 | 0,32 | 3,23 | 0,00341 | 0,0417 | Distal Intergenic | -227934 | ENSG00000137872 | SEMA6D |
| chr2 | 78899901 | 78900258 | 1,37 | -1,55 | 2,92 | 0,00342 | 0,0418 | Distal Intergenic | -352554 | ENSG00000143954 | REG3 G |
| chr5 | 28614677 | 28615282 | 1,68 | -1,55 | 3,24 | 0,00343 | 0,0419 | Distal Intergenic | -311695 | NA | LSP1P3 |
| chr4 | 21852076 | 21852994 | 1,77 | -1,55 | 3,33 | 0,00343 | 0,0419 | Promoter (1-2kb) | 1817 | ENSG00000280650 | KCNIP4 -IT1 |
| chr2 | 231447343 | 231447843 | 1,59 | -1,55 | 3,14 | 0,00344 | 0,0419 | Distal Intergenic | 78442 | ENSG00000067066 | SP100 |
| chr3 | 19030953 | 19031542 | 1,66 | -1,55 | 3,21 | 0,00344 | 0,0419 | Distal Intergenic | -158475 | ENSG00000183960 | KCNH8 |
| chr8 | 132285038 | 132285762 | 2,44 | -0,86 | 3,3 | 0,00344 | 0,0419 | Distal Intergenic | -232203 | ENSG00000155897 | ADCY8 |
| chr9 | 14479085 | 14479859 | 1,87 | -1,38 | 3,25 | 0,00345 | 0,0421 | Distal Intergenic | -80103 | ENSG00000147862 | NFIB |
| chr9 | 110636844 | 110637512 | 1,68 | -1,55 | 3,23 | 0,00346 | 0,0421 | Distal Intergenic | -384797 | ENSG00000136826 | KLF4 |
| chr7 | 137822314 | 137823075 | 1,65 | -1,55 | 3,21 | 0,00346 | 0,0422 | Distal Intergenic | 13810 | ENSG00000284028 | MIR4468 |
| chr7 | 85100920 | 85101542 | 1,6 | -1,55 | 3,15 | 0,00347 | 0,0422 | Distal Intergenic | -284749 | ENSG00000153993 | SEMA3D |
| chr15 | 45633378 | 45634221 | 1,68 | -1,55 | 3,24 | 0,00347 | 0,0422 | Distal Intergenic | 36521 | ENSG00000171766 | GATM |
| chr5 | 108193741 | 108194101 | 1,08 | -1,55 | 2,63 | 0,00348 | 0,0423 | Intron (uc0 11cve.1/2241, intron 4 of 5) | -66206 | ENSG00000151422 | FER |
| chr21 | 23893149 | 23893555 | 1,52 | -1,55 | 3,07 | 0,00348 | 0,0423 | Distal Intergenic | 422213 | ENSG00000184856 | LINC00308 |
| chr1 | 54144415 | 54145995 | 3,82 | 0,39 | 3,43 | 0,00348 | 0,0423 | Intron (uc001cvr.1/148979, intron 1 of 9) | 53882 | ENSG00000174332 | GLIS1 |
| chr20 | 54319349 | 54320053 | 1,97 | -1,55 | 3,53 | 0,00349 | 0,0424 | Distal Intergenic | 260475 | ENSG00000054803 | CBLN4 |
| chr4 | 77253989 | 77254256 | 1,27 | -1,38 | 2,65 | 0,0035 | 0,0424 | Intron (uc003hkb.4/339965, intron 18 of 23) | 26810 | ENSG00000 118804 | STBD1 |
| chr9 | 755960 | 756467 | 1,46 | -1,35 | 2,81 | 0,0035 | 0,0424 | Distal Intergenic | 21212 | ENSG00000107104 | KANK1 |
| chr4 | 117619036 | 117619593 | 2,3 | -0,91 | 3,21 | 0,0035 | 0,0425 | Distal Intergenic | 387143 | ENSG00000174599 | TRAM1L1 |
| chr21 | 37063855 | 37064262 | 1,35 | -1,55 | 2,91 | 0,00351 | 0,0425 | Intron (uc002yut.1/861, intron 4 of 10) | -28751 | ENSG00000211590 | MIR802 |
| chr5 | 154528554 | 154529150 | 1,5 | -1,55 | 3,05 | 0,00351 | 0,0425 | Distal Intergenic | 135294 | ENSG00000226650 | KIF4B |
| chr9 | 106574506 | 106575051 | 1,7 | -1,29 | 2,99 | 0,00352 | 0,0426 | Distal Intergenic | -281490 | ENSG00000136824 | SMC2 |
| chr2 | 47968851 | 47969394 | 1,46 | -1,55 | 3,02 | 0,00352 | 0,0427 | Distal Intergenic | -40827 | ENSG00000116062 | MSH6 |
| chr15 | 55768269 | 55768573 | 1,51 | -1,01 | 2,52 | 0,00353 | 0,0427 | Intron (uc002acy.3/100533483, intron 3 of 15) | 22209 | ENSG00000261771 | DNAAF4 -CCPG1 |
| chr1 1 | 95989839 | 95990385 | 2,1 | -0,8 | 2,9 | 0,00353 | 0,0427 | Intron (uc001pfw.1/84441, intron 1 of 4) | -84217 | ENSG00000266192 | MIR1260B |
| chr10 | 90600782 | 90602153 | 3,54 | 0,17 | 3,36 | 0,00353 | 0,0427 | Intron (uc001kfj.4/118932, intron 1 of 5) | 9579 | ENSG00000152766 | ANKRD22 |
| chr10 | 126303150 | 126303600 | 1,18 | -1,55 | 2,73 | 0,00354 | 0,0427 | Downstream (2-3kb) | 9380 | ENSG00000107902 | LHPP |
| chr6 | 72138568 | 72139134 | 2,03 | -1,29 | 3,31 | 0,00354 | 0,0427 | Distal Intergenic | -8120 | ENSG00000233237 | LINC00472 |
| chr3 | 169148360 | 169149499 | 2,57 | -0,46 | 3,03 | 0,00354 | 0,0428 | Intron (uc011bpj.1/2122,intron 1 of 16) | -49048 | ENSG00000085276 | MECOM |
| chr4 | 148902697 | 148903092 | 1,44 | -1,55 | 2,99 | 0,00355 | 0,0428 | Intron (uc003ilf.3/79658, intron 18 of 22) | 35307 | ENSG00000071205 | ARHGAP10 |
| chr1 1 | 20633100 | 20633523 | 1,56 | -1,55 | 3,11 | 0,00355 | 0,0428 | Intron (uc001mqd.3/9152, intron 5 of 15) | 12154 | ENSG00000165970 | SLC6A5 |
| chr7 | 146867466 | 146868705 | 3,98 | 0,34 | 3,64 | 0,00358 | 0,0431 | Intron (uc003weu.2/26047, intron 8 of 23) | 206508 | ENSG00000221442 | MIR548F4 |
| chr3 | 102133967 | 102134898 | 3,81 | 1,19 | 2,62 | 0,0036 | 0,0434 | Intron (uc003dvs.1/131368, intron 7 of 17) | -18961 | ENSG00000170044 | ZPLD1 |
| chr3 | 114574533 | 114575124 | 1,57 | -1,55 | 3,12 | 0,00361 | 0,0435 | Intron (uc003ebm.3/26137, intron 4 of 11) | -96409 | ENSG00000 181722 | ZBTB20 |
| chr6 | 67600733 | 67601780 | 4,67 | 2,22 | 2,45 | 0,00362 | 0,0435 | Distal Intergenic | 1102961 | NA | SLC25A51P1 |
| chr5 | 108194417 | 108195171 | 2,1 | -0,91 | 3,01 | 0,00362 | 0,0435 | Intron (uc011cve.1/2241, intron 4 of 5) | -65136 | ENSG00000151422 | FER |
| chr7 | 123249081 | 123249948 | 2,45 | -0,87 | 3,31 | 0,00362 | 0,0435 | Promoter (<=1kb) | 0 | ENSG00000146809 | ASB15 |
| chr21 | 21201259 | 21202905 | 3,36 | -0,06 | 3,42 | 0,00362 | 0,0435 | Distal Intergenic | 972521 | ENSG00000224924 | LINC00320 |
| chr8 | 109480196 | 109481513 | 4,52 | 0,98 | 3,54 | 0,00362 | 0,0435 | Intron (uc003ymw.1/9694, intron 5 of 10) | 24343 | ENSG00000104412 | EMC2 |
| chr22 | 19096567 | 19098050 | 4,35 | 1,6 | 2,75 | 0,00363 | 0,0435 | intron (uc002zoq.1/9993, intron 1 of 9) | 11917 | ENSG00000070413 | DGCR2 |
| chr20 | 30624489 | 30624724 | 1,21 | -1,55 | 2,76 | 0,00363 | 0,0435 | Distal Intergenic | 14311 | ENSG00000101331 | CCM2L |
| chr2 | 2984364 | 2984986 | 1,94 | -0,97 | 2,92 | 0,00363 | 0,0436 | Intron (uc002qxh.1/uc002qxh.1, intron 3 of 6) | 338459 | ENSG00000032389 | EIPR1 |
| chr5 | 98937504 | 98938284 | 2,51 | -0,62 | 3,13 | 0,00364 | 0,0436 | Distal Intergenic | 672666 | NA | LOC100289230 |
| chr6 | 8810025 | 8811316 | 3,15 | 0 | 3,15 | 0,00364 | 0,0436 | Distal Intergenic | 157583 | NA | HULC |
| chr6 | 80044391 | 80044906 | 1,75 | -1,55 | 3,31 | 0,00364 | 0,0436 | Distal Intergenic | -99936 | ENSG00000 118418 | HMGN3 |
| chr3 | 111447997 | 111448641 | 1,6 | -1,55 | 3,16 | 0,00364 | 0,0437 | Promoter (2-3kb) | -2686 | ENSG00000144824 | PHLDB2 |
| chr4 | 96924628 | 96925556 | 2,55 | -0,8 | 3,35 | 0,00364 | 0,0437 | Distal Intergenic | 163389 | ENSG00000163114 | PDHA2 |
| chr1 | 88052845 | 88054077 | 3,13 | 0,41 | 2,72 | 0,00365 | 0,0437 | Distal Intergenic | -215507 | ENSGO00002272 90 | LINC01364 |
| chr21 | 27608675 | 27609491 | 3,84 | 0,83 | 3,01 | 0,00365 | 0,0437 | Distal Intergenic | -65229 | ENSG00000142192 | APP |
| chr16 | 23517629 | 23518586 | 2,22 | -0,93 | 3,15 | 0,00365 | 0,0437 | Intron (uc002dlq.3/23062, intron 1 of 16) | 3229 | ENSG00000103365 | GGA2 |
| chr7 | 154989948 | 154990750 | 1,72 | -1,55 | 3,27 | 0,00365 | 0,0437 | Distal Intergenic | -98736 | ENSG00000186480 | INSIG1 |
| chr7 | 121676002 | 121676287 | 1,27 | -1,55 | 2,82 | 0,00366 | 0,0438 | Intron (uc003vjy.3/5803,intron 17 of 29) | 97589 | ENSG00000008311 | AASS |
| chr4 | 170922679 | 170923152 | 1,55 | -1,55 | 3,1 | 0,00366 | 0,0438 | Promoter (1-2kb) | 1783 | ENSG00000198948 | MFAP3L |
| chr3 | 50611053 | 50611722 | 1,71 | -1,55 | 3,26 | 0,00366 | 0,0438 | Promoter (2-3kb) | -2595 | ENSG00000088543 | C3orf18 |
| chr21 | 26242114 | 26242786 | 1,89 | -1,26 | 3,15 | 0,00367 | 0,0438 | Intron (uc031rvb.1/339622, intron 2 of 3) | 29250 | ENSG00000226983 | LINC01692 |
| chr2 | 212456566 | 212457478 | 2,28 | -1,14 | 3,42 | 0,00367 | 0,0438 | Intron (uc002veg.1/2066, intron 19 of 27) | 833606 | ENSG00000221782 | MIR548F2 |
| chr20 | 54723307 | 54723773 | 1,42 | -1,55 | 2,98 | 0,00367 | 0,0439 | Distal Intergenic | -100015 | ENSG00000124089 | MC3R |
| chr7 | 85341419 | 85342027 | 1,67 | -1,55 | 3,22 | 0,00367 | 0,0439 | Distal Intergenic | -525248 | ENSG00000153993 | SEMA3D |
| chr1 | 233779180 | 233779695 | 1,86 | -1,55 | 3,41 | 0,00368 | 0,044 | Intron (uc010pxo.1/3775, intron 1 of 2) | 13827 | ENSG00000135750 | KCNK1 |
| chr12 | 9854763 | 9855197 | 1,44 | -1,55 | 3 | 0,00369 | 0,044 | Distal Intergenic | 30698 | ENSG00000184293 | CLECL1 |
| chr5 | 39570345 | 39570711 | 2,38 | -0,48 | 2,86 | 0,0037 | 0,0442 | Distal Intergenic | -145010 | ENSG00000153071 | DAB2 |
| chr20 | 53725065 | 53725711 | 2 | -1,1 | 3,1 | 0,0037 | 0,0442 | Distal Intergenic | 632799 | ENSG00000101134 | DOK5 |
| chr1 1 | 68843208 | 68843933 | 1,62 | -1,55 | 3,17 | 0,0037 | 0,0442 | Intron (uc009ysk.1/219931, intron 12 of 13) | 20578 | ENSG00000162341 | TPCN2 |
| chrl | 246333805 | 246334537 | 2,89 | -0,4 | 3,29 | 0,00371 | 0,0443 | Intron (uc001ibk.3/64754, intron 5 of 11) | -239680 | ENSG00000185420 | SMYD3 |
| chr7 | 10655494 | 10655857 | 1,36 | -1,55 | 2,91 | 0,00372 | 0,0443 | Distal Intergenic | 323956 | ENSG00000 189043 | NDUFA4 |
| chr1 1 | 317155 | 321242 | 5,33 | 2,31 | 3,03 | 0,00372 | 0,0443 | Promoter (<=1kb) | 0 | ENSG00000142089 | IFITM3 |
| chr3 | 176868077 | 176869925 | 4,83 | 1,79 | 3,05 | 0,00372 | 0,0443 | Intron (uc003fiw.4/79718, intron 1 of 15) | 44341 | ENSG00000177565 | TBL1XR1 |
| chr10 | 12454145 | 12455235 | 3,59 | 0,48 | 3,11 | 0,00372 | 0,0443 | Intron (uc001iln.3/57118, intron 1 of 9) | 62562 | ENSG00000183049 | CAMK1D |
| chr12 | 10707019 | 10708371 | 3,5 | 0,23 | 3,27 | 0,00372 | 0,0443 | Distal Intergenic | 44063 | NA | KLRA1P |
| chr6 | 45404726 | 45405203 | 1,88 | -1,55 | 3,44 | 0,00372 | 0,0443 | Intron (uc0 1dvx.2/860, intron 4 of 8) | 14812 | ENSG00000124813 | RUNX2 |
| chr5 | 163930966 | 163931649 | 2,29 | -0,65 | 2,94 | 0,00373 | 0,0443 | Intron (uc0031zn.3/uc0031zn.3, intron 2 of 2) | 986503 | ENSG0000003 8274 | MAT2B |
| chr4 | 155965381 | 155965866 | 1,52 | -1,55 | 3,07 | 0,00373 | 0,0444 | Distal Intergenic | -163915 | ENSG00000185149 | NPY2R |
| chr8 | 133929401 | 133930745 | 4,41 | 1,27 | 3,14 | 0,00373 | 0,0444 | Promoter (<=1kb) | -876 | ENSG00000042832 | TG |
| chr1 1 | 26733603 | 26734715 | 2,82 | -0,46 | 3,28 | 0,00373 | 0,0444 | Exon (uc001mra.2/159963,exon 2 of 15) | 8859 | ENSG00000148942 | SLC5A12 |
| chr2 | 69718607 | 69719673 | 2,95 | -0,02 | 2,97 | 0,00374 | 0,0444 | Intron (uc002sfp.2/22848, intron 17 of 21) | 27630 | ENSG00000207016 | SNORA36C |
| chr8 | 3221987 | 3222705 | 1,76 | -1,55 | 3,32 | 0,00374 | 0,0444 | Intron (uc011kwj.2/64478, intron 8 of 55) | 31198 | ENSG00000183117 | CSMD1 |
| chr3 | 43440297 | 43440745 | 1,52 | -1,55 | 3,07 | 0,00375 | 0,0445 | Intron (uc003cmv.3/55129, intron 12 of 12) | -46843 | NA | SNRK-AS1 |
| chr4 | 140523361 | 140524906 | 4,42 | 0,67 | 3,75 | 0,00375 | 0,0445 | Distal Intergenic | -45784 | ENSG00000145391 | SETD7 |
| chr6 | 126491729 | 126492638 | 2,72 | -0,45 | 3,18 | 0,00375 | 0,0446 | Distal Intergenic | -47967 | ENSG00000266721 | MIR5695 |
| chr4 | 41015401 | 41015760 | 1,25 | -1,55 | 2,8 | 0,00376 | 0,0446 | Promoter (<=1kb) | 655 | ENSG00000163697 | APBB2 |
| chr9 | 100231361 | 100231833 | 1,44 | -1,55 | 3 | 0,00376 | 0,0446 | Promoter (<=1kb) | 684 | ENSG00000 196116 | TDRD7 |
| chr4 | 26127683 | 26128091 | 1,53 | -1,55 | 3,08 | 0,00376 | 0,0446 | Distal Intergenic | -193241 | ENSG00000168214 | RBPJ |
| chr10 | 54898634 | 54899437 | 2,09 | -1,04 | 3,12 | 0,00376 | 0,0446 | Distal Intergenic | -367174 | ENSG00000165471 | MBL2 |
| chr14 | 66980109 | 66980456 | 1,2 | -1,55 | 2,75 | 0,00377 | 0,0447 | Intron (uc001xiw.3/10243, intron 1 of 10) | 5984 | ENSG00000 171723 | GPHN |
| chr10 | 52192195 | 52192757 | 1,36 | -1,55 | 2,92 | 0,00377 | 0,0447 | Intron (uc001jje.3/259230. intron 6 of 10) | -14255 | ENSG00000198964 | SGMS1 |
| chr5 | 120622065 | 120622630 | 1,65 | -1,38 | 3,04 | 0,00377 | 0,0447 | Distal Intergenic | -565020 | ENSG00000 181867 | FTMT |
| chr21 | 23593057 | 23593912 | 3,33 | 0,2 | 3,12 | 0,00377 | 0,0447 | Distal Intergenic | 122121 | ENSG00000184856 | LINC00308 |
| chr8 | 93443449 | 93444416 | 3,41 | 0,27 | 3,14 | 0,00377 | 0,0447 | Distal Intergenic | -327995 | ENSG00000079102 | RUNX1T1 |
| chr14 | 81158848 | 81159370 | 1,58 | -1,55 | 3,14 | 0,00378 | 0,0447 | Intron (uc010asz.3/145508, intron 8 of 14) | 143311 | ENSG00000100629 | CEP128 |
| chr15 | 38901428 | 38901749 | 1,45 | -1,4 | 2,85 | 0,00378 | 0,0448 | Distal Intergenic | -44421 | ENSG00000172575 | RASGRP1 |
| chr12 | 125733525 | 125734061 | 1,41 | -1,55 | 2,96 | 0,00379 | 0,0449 | Distal Intergenic | -77101 | ENSG00000139364 | TMEM132B |
| chr5 | 26635501 | 26635901 | 1,58 | -1,55 | 3,13 | 0,0038 | 0,0449 | Distal Intergenic | 255041 | ENSG00000113100 | CDH9 |
| chr17 | 46518256 | 46519485 | 2,69 | -0,06 | 2,75 | 0,0038 | 0,045 | Distal Intergenic | -10662 | ENSG00000141293 | SKAP1 |
| chr8 | 112674600 | 112675005 | 1,72 | -1,55 | 3,28 | 0,0038 | 0,045 | Distal Intergenic | 674955 | ENSG00000164796 | CSMD3 |
| chr3 | 138232109 | 138232533 | 1,35 | -1,55 | 2,9 | 0,00381 | 0,045 | Intron (uc003esl.3/80321, intron 11 of 17) | 78265 | ENSG00000114107 | CEP70 |
| chr15 | 91467868 | 91468474 | 2,02 | -1,26 | 3,29 | 0,00381 | 0,045 | Distal Intergenic | -4936 | ENSG00000140553 | UNC45A |
| chr7 | 114109322 | 114109812 | 1,36 | -1,55 | 2,91 | 0,00381 | 0,0451 | Intron (uc003vgt.2/93986, intron 5 of 11) | 42765 | ENSG00000128573 | FOXP2 |
| chr5 | 164337691 | 164338252 | 1,57 | -1,55 | 3,13 | 0,00382 | 0,0451 | Distal Intergenic | 1393228 | ENSG0000003 8274 | MAT2B |
| chr2 | 22113960 | 22114497 | 2,36 | -0,8 | 3,16 | 0,00382 | 0,0451 | Distal Intergenic | -180436 | ENSGO0000229621 | LINC01822 |
| chr5 | 127894947 | 127895481 | 1,59 | -1,55 | 3,15 | 0,00383 | 0,0452 | Distal Intergenic | -21031 | ENSG00000138829 | FBN2 |
| chr3 | 175061380 | 175062123 | 1,63 | -1,55 | 3,18 | 0,00383 | 0,0452 | Intron (uc003fit.3/254827, intron 5 of 13) | -25206 | ENSG00000264974 | MIR4789 |
| chr13 | 99440974 | 99441446 | 1,76 | -1,17 | 2,93 | 0,00384 | 0,0452 | Distal Intergenic | -36045 | ENSG00000088386 | SLC15A1 |
| chr18 | 5975195 | 5975975 | 3,22 | 0,19 | 3,03 | 0,00385 | 0,0453 | Intron (uc002kmv.4/91133, intron 14 of 16) | -83092 | ENSG00000206432 | TMEM200C |
| chr2 | 160001107 | 160002349 | 4,41 | 1,03 | 3,38 | 0,00385 | 0,0453 | Intron (uc010fol.1/85461, intron 5 of 11) | 8402 | ENSG000001 15183 | TANC1 |
| chr6 | 74564893 | 74565214 | 1,39 | -1,55 | 2,95 | 0,00385 | 0,0454 | Distal Intergenic | 159385 | ENSG00000156535 | CD109 |
| chr19 | 755800 | 756245 | 1,69 | -1,29 | 2,97 | 0,00386 | 0,0454 | Intron (uc0021po.3/126353, intron 1 of 4) | 4654 | ENSGO0000099812 | MISP |
| chr21 | 19940275 | 19941013 | 2,32 | -0,87 | 3,19 | 0,00386 | 0,0454 | Intron (uc010glf.2/uc010glf.2, intron 2 of 3) | -164305 | ENSG00000154646 | TMPRSS15 |
| chr3 | 152460275 | 152460888 | 1,55 | -1,31 | 2,86 | 0,00386 | 0,0455 | Distal Intergenic | -91848 | ENSG00000169860 | P2RY1 |
| chr1 1 | 38294956 | 38295351 | 1,57 | -1,55 | 3,13 | 0,00387 | 0,0455 | Distal Intergenic | -1675127 | ENSG00000175097 | RAG2 |
| chr13 | 50809604 | 50810439 | 3,23 | 0,04 | 3,19 | 0,00387 | 0,0455 | Intron (uc010adl.1/10301, intron 1 of 1) | 63450 | NA | ST13P4 |
| chr2 | 181427899 | 181428284 | 1,43 | -1,55 | 2,99 | 0,00388 | 0,0457 | Distal Intergenic | -416828 | ENSG00000170035 | UBE2E3 |
| chr2 | 144987213 | 144987965 | 1,58 | -1,55 | 3,13 | 0,00389 | 0,0457 | Intron (uc021vqf.1/79712, intron 2 of 9) | 6941 | ENSG00000 121964 | GTDC1 |
| chr6 | 53291291 | 53292605 | 3,35 | 0,15 | 3,2 | 0,00389 | 0,0457 | Distal Intergenic | -77314 | ENSG000000 12660 | ELOVL5 |
| chr20 | 57024563 | 57025817 | 3,28 | 0,04 | 3,25 | 0,0039 | 0,0458 | 3'UTR | 60388 | ENSG00000124164 | VAPB |
| chr1 | 120328565 | 120329207 | 1,71 | -1,31 | 3,03 | 0,00391 | 0,0459 | Distal Intergenic | -17010 | ENSG00000134240 | HMGCS2 |
| chr1 | 221951437 | 221952346 | 2,54 | -0,39 | 2,93 | 0,00392 | 0,0459 | Distal Intergenic | -35921 | ENSG00000143507 | DUSP10 |
| chr1 | 238436014 | 238436597 | 1,61 | -1,55 | 3,16 | 0,00392 | 0,0459 | Distal Intergenic | 212720 | ENSG00000215808 | LINC01139 |
| chr4 | 24858002 | 24858562 | 1,92 | -0,44 | 2,35 | 0,00392 | 0,046 | Intron (uc003grc.3/91050, intron 3 of 11) | -3180 | ENSG00000181982 | CCDC149 |
| chr13 | 100883970 | 100884268 | 1,07 | -1,55 | 2,62 | 0,00392 | 0,046 | Intron (uc001voo.3/5095, intron 7 of 23) | 142701 | ENSG00000175198 | PCCA |
| chr8 | 82009853 | 82010339 | 1,1 | -1,55 | 2,65 | 0,00392 | 0,046 | Intron (uc003vbz.3/55824, intron 1 of 8) | 13964 | ENSG00000076641 | PAG1 |
| chr7 | 18151827 | 18152409 | 1,48 | -1,55 | 3,03 | 0,00392 | 0,046 | Intron (uc011jya.2/9734, intron 1 of 12) | 25255 | ENSG00000048052 | HDAC9 |
| chr13 | 104874660 | 104875440 | 1,8 | -1,55 | 3,35 | 0,00392 | 0,046 | Distal Intergenic | -939812 | ENSG00000263741 | MIR548AS |
| chr2 | 113394413 | 113394753 | 2,04 | -0,7 | 2,74 | 0,00393 | 0,046 | Distal Intergenic | 7004 | NA | FLJ42351 |
| chr13 | 40273231 | 40273870 | 2,9 | 0,42 | 2,48 | 0,00394 | 0,0461 | Exon (uc001uxh.2/57511, exon 13 of 19) | -34959 | ENSG00000264171 | MIR4305 |
| chr14 | 87304719 | 87305135 | 1,69 | -1,26 | 2,94 | 0,00394 | 0,0462 | Distal Intergenic | -66987 | ENSG00000258804 | LINC01148 |
| chr1 1 | 37733558 | 37733955 | 1,73 | -1,23 | 2,96 | 0,00395 | 0,0462 | Distal Intergenic | -1113729 | ENSG00000175097 | RAG2 |
| chr2 | 136415656 | 136416191 | 1,62 | -1,55 | 3,17 | 0,00395 | 0,0463 | Intron (uc002tuo.3/23518, intron 17 of 25) | -6776 | ENSG00000207654 | MIR128-1 |
| chr2 | 9003478 | 9004083 | 1,92 | -1,1 | 3,01 | 0,00396 | 0,0463 | Intron (uc002qzg.1/129642, intron 10 of 12) | -25723 | ENSG00000134313 | KIDINS220 |
| chr7 | 106297570 | 106298051 | 1,84 | -1,55 | 3,39 | 0,00396 | 0,0463 | 3'UTR | 3583 | ENSG00000253276 | CCDC71L |
| chr1 1 | 76548536 | 76549293 | 1,7 | -0,87 | 2,56 | 0,00398 | 0,0465 | Distal Intergenic | -22624 | ENSG00000078124 | ACER3 |
| chr3 | 70145307 | 70145586 | 1,3 | -1,55 | 2,85 | 0,00398 | 0,0465 | Distal Intergenic | 159556 | ENSG00000187098 | MITF |
| chr5 | 124683809 | 124684743 | 2,54 | -0,8 | 3,35 | 0,00398 | 0,0465 | Distal Intergenic | -599309 | ENSG00000168916 | ZNF608 |
| chr7 | 127541032 | 127542059 | 2,56 | -0,63 | 3,19 | 0,00399 | 0,0465 | Intron (uc003vmi.3/27044, intron 13 of 23) | -95503 | ENSG00000279078 | SND1-IT1 |
| chr4 | 69435443 | 69435855 | 1,77 | -1,55 | 3,32 | 0,00399 | 0,0465 | Promoter (1-2kb) | -1198 | ENSG00000197888 | UGT2B17 |
| chr4 | 179879894 | 179880796 | 3,03 | 0,29 | 2,75 | 0,004 | 0,0466 | Distal Intergenic | 1229983 | ENSG00000231171 | LINC01098 |
| chr14 | 53999593 | 54000340 | 2,91 | 0,02 | 2,89 | 0,004 | 0,0466 | Distal Intergenic | -379547 | ENSG00000100523 | DDHD1 |
| chr10 | 120458159 | 120458618 | 1,29 | -1,55 | 2,84 | 0,004 | 0,0467 | Intron (uc010qst.1/143384, intron 5 of 9) | 56140 | ENSG00000151893 | CACUL1 |
| chr17 | 76875870 | 76876350 | 1,84 | -1,55 | 3,39 | 0,004 | 0,0467 | Intron (uc010wtv.2/7077,intron 1 of 4) | -5699 | ENSG00000035862 | TIMP2 |
| chr6 | 71972499 | 71973304 | 3,2 | -0,18 | 3,38 | 0,00401 | 0,0467 | Distal Intergenic | -25173 | ENSG00000 119900 | OGFRL1 |
| chr9 | 78726491 | 78727161 | 2,19 | -0,62 | 2,82 | 0,00401 | 0,0468 | Intron (uc004ajy.2/5125, intron 9 of 13) | -69153 | ENSG00000099139 | PCSK5 |
| chr16 | 72638051 | 72638364 | 1,32 | -1,55 | 2,88 | 0,00402 | 0,0468 | Distal Intergenic | -431702 | ENSG00000118557 | PMFBP1 |
| chr3 | 146071375 | 146072106 | 1,57 | -1,55 | 3,12 | 0,00402 | 0,0468 | Distal Intergenic | -102409 | ENSG00000114698 | PLSCR4 |
| chr6 | 108987911 | 108988603 | 1,74 | -0,97 | 2,71 | 0,00403 | 0,0469 | Intron (uc003psk.2/2309, intron 3 of 3) | 10362 | ENSG00000118689 | FOXO3 |
| chr12 | 59776397 | 59777183 | 2,56 | -0,56 | 3,12 | 0,00403 | 0,0469 | Distal Intergenic | -212638 | ENSG00000118596 | SLC16A7 |
| chr2 | 204558807 | 204559370 | 2,54 | -0,08 | 2,62 | 0,00404 | 0,047 | Distal Intergenic | -11828 | ENSG00000178562 | CD28 |
| chr7 | 29322360 | 29322850 | 1,63 | -1,17 | 2,8 | 0,00405 | 0,0471 | Intron (uc011jzs.2/1124, intron 2 of 13) | 85003 | ENSG00000106069 | CHN2 |
| chr18 | 26600192 | 26600979 | 2,49 | -0,86 | 3,35 | 0,00405 | 0,0471 | Distal Intergenic | -842747 | ENSG00000170558 | CDH2 |
| chr9 | 28887561 | 28888079 | 2,13 | -0,53 | 2,66 | 0,00406 | 0,0471 | Promoter (<=1kb) | 874 | ENSG00000215939 | MIR873 |
| chr5 | 72114437 | 72114907 | 1,74 | -0,92 | 2,66 | 0,00406 | 0,0471 | Promoter (2-3kb) | 2019 | ENSG00000083312 | TNPO1 |
| chr14 | 98978746 | 98978962 | 1,15 | -1,29 | 2,43 | 0,00406 | 0,0472 | Distal Intergenic | -198988 | ENSG00000176605 | C14orf177 |
| chrX | 147995618 | 147996066 | 1,3 | -1,55 | 2,86 | 0,00407 | 0,0472 | Intron (uc004fcp.3/2334, intron 9 of 20) | 195003 | ENSG00000155966 | AFF2 |
| chr3 | 1641912 | 1643116 | 3,35 | 0,01 | 3,34 | 0,00407 | 0,0472 | Distal Intergenic | -497434 | ENSG00000 144619 | CNTN4 |
| chr21 | 26685991 | 26686392 | 1,31 | -1,55 | 2,86 | 0,00408 | 0,0473 | Distal Intergenic | 117621 | ENSG00000185433 | LINC00158 |
| chr1 | 240245352 | 240245769 | 1,43 | -1,55 | 2,98 | 0,00408 | 0,0473 | Distal Intergenic | -9416 | ENSG00000155816 | FMN2 |
| chr3 | 176892140 | 176892924 | 1,5 | -1,55 | 3,06 | 0,00408 | 0,0473 | Intron (uc003fiw.4/79718, intron 1 of 15) | 21342 | ENSG00000177565 | TBL1XR1 |
| chr6 | 109242922 | 109243431 | 1,66 | -1,55 | 3,21 | 0,00408 | 0,0473 | Intron (uc003pss.4/84071, intron 10 of 17) | 73303 | ENSG00000 118690 | ARMC2 |
| chr13 | 109691486 | 109692279 | 2,66 | -0,61 | 3,27 | 0,00408 | 0,0473 | Intron (uc001vqt.1/23026, intron 23 of 34) | 127372 | ENSG00000236242 | MY016-AS1 |
| chr4 | 168309758 | 168310343 | 1,64 | -1,38 | 3,03 | 0,00409 | 0,0473 | Distal Intergenic | -154017 | ENSG00000196104 | SPOCK3 |
| chr4 | 47012650 | 47014776 | 4,13 | 0,53 | 3,6 | 0,00409 | 0,0474 | Distal Intergenic | -16226 | ENSG00000109158 | GABRA4 |
| chr5 | 4630285 | 4630684 | 1,94 | -1,31 | 3,25 | 0,0041 | 0,0474 | Distal Intergenic | -403788 | ENSG00000215231 | LINC01020 |
| chr2 | 192204753 | 192205240 | 1,93 | -0,87 | 2,79 | 0,00411 | 0,0475 | Intron (uc002usq.2/4430,intron4 of 28) | -40490 | ENSG00000128641 | MYO1B |
| chr5 | 104777208 | 104778232 | 3,02 | -0,4 | 3,41 | 0,00411 | 0,0476 | Distal Intergenic | 342033 | ENSG00000232159 | RAB9BP1 |
| chr9 | 95202518 | 95203310 | 2,68 | -0,26 | 2,94 | 0,00412 | 0,0476 | Intron (uc004arz.3/401541, intron 5 of 7) | -15682 | ENSG00000127083 | OMD |
| chr4 | 32561799 | 32562307 | 1,78 | -1,38 | 3,16 | 0,00412 | 0,0476 | Distal Intergenic | 1839762 | ENSG00000169851 | PCDH7 |
| chr16 | 6833038 | 6833385 | 1,28 | -1,55 | 2,83 | 0,00412 | 0,0477 | Intron (uc002cyr.1/54715, intron 3 of 11) | 9228 | ENSG00000078328 | RBFOX1 |
| chr14 | 22848827 | 22849182 | 1,84 | -0,68 | 2,52 | 0,00413 | 0,0477 | Exon (uc001wds.1/uc001wds.1. exon 3 of 3) | 208961 | ENSG00000129562 | DAD1 |
| chr3 | 40368608 | 40369206 | 1,57 | -1,55 | 3,12 | 0,00413 | 0,0477 | Distal Intergenic | -17419 | ENSG00000280739 | EIF1B-AS1 |
| chr3 | 154301437 | 154302252 | 2,28 | -0,87 | 3,14 | 0,00413 | 0,0477 | Distal Intergenic | -153933 | ENSG00000174948 | GPR149 |
| chr5 | 18893301 | 18894092 | 2,28 | -1,01 | 3,29 | 0,00413 | 0,0477 | Distal Intergenic | 992289 | ENSG00000145526 | CDH18 |
| chr3 | 173604548 | 173605285 | 1,76 | -1,55 | 3,31 | 0,00413 | 0,0477 | Intron (uc003fio.1/22871, intron 4 of 6) | 282479 | ENSG00000169760 | NLGN1 |
| chrX | 81381382 | 81381955 | 1,6 | -1,26 | 2,86 | 0,00414 | 0,0478 | Distal Intergenic | 923467 | ENSG00000131171 | SH3BGRL |
| chr21 | 19721761 | 19722078 | 1,4 | -1,55 | 2,95 | 0,00414 | 0,0478 | Intron (uc002vkw.3/5651, intron 10 of 24) | 53892 | ENSG00000154646 | TMPRSS15 |
| chr9 | 90918868 | 90919728 | 2,45 | -0,53 | 2,99 | 0,00414 | 0,0478 | Distal Intergenic | -83112 | ENSG00000106723 | SPIN1 |
| chr5 | 179186567 | 179187333 | 1,69 | -1,55 | 3,24 | 0,00414 | 0,0478 | Intron (uc003mkm.3/9794, intron 1 of 4) | 26716 | ENSG00000161021 | MAML1 |
| chr4 | 116596509 | 116597193 | 2,16 | -1,1 | 3,26 | 0,00414 | 0,0478 | Distal Intergenic | -561477 | ENSG00000138653 | NDST4 |
| chr12 | 59307166 | 59308063 | 1,76 | -1,55 | 3,31 | 0,00414 | 0,0478 | Exon (uc009zqh.3/121227, exon 2 of 19) | 5264 | ENSG00000139263 | LRIG3 |
| chr22 | 28693593 | 28694338 | 2,54 | 0,04 | 2,5 | 0,00415 | 0,0478 | Exon (uc003adp.4/23331, exon 4 of 23) | 304677 | ENSG00000235954 | TTC28-AS1 |
| chr4 | 45125086 | 45126036 | 4,16 | 1,42 | 2,74 | 0,00415 | 0,0478 | Distal Intergenic | -396435 | ENSG00000163281 | GNPDA2 |
| chr1 | 237865708 | 237866252 | 1,47 | -1,55 | 3,02 | 0,00415 | 0,0478 | Intron (uc001hyl.1/6262, intron 66 of 104) | -20176 | ENSG00000 198626 | RYR2 |
| chr4 | 87477651 | 87479817 | 3,93 | 0,93 | 3 | 0,00416 | 0,0479 | Intron (uc010ikh.1/5602, intron 1 of 6) | 35446 | ENSG00000109339 | MAPK10 |
| chr3 | 85089204 | 85089741 | 1,63 | -1,23 | 2,87 | 0,00417 | 0,0479 | Intron (uc003dqj.3/253559, intron 1 of 9) | 81071 | ENSG00000175161 | CADM2 |
| chr4 | 45188102 | 45188759 | 3,08 | 0,3 | 2,78 | 0,00417 | 0,048 | Distal Intergenic | -459451 | ENSG00000163281 | GNPDA2 |
| chr20 | 9373764 | 9374125 | 1,42 | -1,11 | 2,53 | 0,00418 | 0,048 | Intron (uc010gbw.1/5332, intron 16 of 31) | 22183 | ENSG00000101333 | PLCB4 |
| chr5 | 123264158 | 123264769 | 1,6 | -1,55 | 3,15 | 0,00418 | 0,0481 | Distal Intergenic | 383047 | ENSG00000151292 | CSNK1G3 |
| chr1 | 248645344 | 248645915 | 1,53 | -1,23 | 2,76 | 0,00419 | 0,0481 | Distal Intergenic | -5975 | ENSG00000203661 | OR2T5 |
| chr14 | 59509226 | 59509693 | 1,59 | -1,55 | 3,14 | 0,0042 | 0,0482 | Distal Intergenic | -145688 | ENSG00000100592 | DAAM1 |
| chr6 | 67964762 | 67965330 | 1,63 | -1,55 | 3,19 | 0,0042 | 0,0482 | Distal Intergenic | -1380302 | ENSG00000135298 | ADGRB3 |
| chr1 | 204660278 | 204661376 | 2,73 | -0,55 | 3,28 | 0,0042 | 0,0482 | Intron (uc001hbd.2/4194, intron 11 of 11) | -5681 | ENSG00000170382 | LRRN2 |
| chr8 | 26516259 | 26516804 | 1,44 | -1,55 | 2,99 | 0,00421 | 0,0483 | Distal Intergenic | 80338 | ENSG00000092964 | DPYSL2 |
| chr3 | 124864326 | 124865016 | 1,58 | -1,55 | 3,13 | 0,00421 | 0,0483 | Intron (uc003ehv.4/84561, intron 5 of 13) | 5380 | ENSG00000264986 | MIR5092 |
| chr2 | 146481413 | 146481912 | 1,57 | -1,55 | 3,13 | 0,00424 | 0,0486 | Distal Intergenic | -862713 | NA | PABPC1P2 |
| chr1 | 240995555 | 240996049 | 1,56 | -1,55 | 3,12 | 0,00425 | 0,0487 | Intron (uc010pyh.2/6000, intron 9 of 17) | 36103 | ENSG00000182901 | RGS7 |
| chr18 | 30009602 | 30010272 | 1,71 | -1,55 | 3,27 | 0,00425 | 0,0487 | Intron (uc002kxk.2/64762, intron 1 of 5) | 40175 | ENSG00000141441 | GAREM1 |
| chr4 | 94376397 | 94377133 | 2,99 | -0,16 | 3,15 | 0,00426 | 0,0487 | Exon (uc011cdt.2/2895, exon 11 of 16) | 251297 | ENSG00000152208 | GRID2 |
| chr4 | 45373581 | 45374590 | 3,23 | -0,01 | 3,23 | 0,00426 | 0,0487 | Distal Intergenic | -644930 | ENSG00000163281 | GNPDA2 |
| chr8 | 93088228 | 93091121 | 4,9 | 1,11 | 3,79 | 0,00426 | 0,0488 | 5'UTR | -13037 | ENSG00000079102 | RUNX1T1 |
| chr1 | 238397362 | 238398015 | 2,28 | -0,75 | 3,03 | 0,00428 | 0,0489 | Distal Intergenic | 251302 | ENSG00000215808 | LINC01139 |
| chr3 | 170067765 | 170068317 | 1,7 | -1,55 | 3,25 | 0,00428 | 0,0489 | Distal Intergenic | -7156 | ENSG00000136603 | SKIL |
| chr3 | 12436460 | 12437495 | 3,78 | 1,07 | 2,71 | 0,00429 | 0,049 | Intron (uc031rym.1/5468, intron 5 of 7) | 15257 | ENSG00000132170 | PPARG |
| chr4 | 143486712 | 143487477 | 2,55 | -0,4 | 2,96 | 0,00429 | 0,049 | Intron (uc003iiw.4/8821, intron 2 of 25) | -51264 | ENSG00000109452 | INPP4B |
| chr8 | 5674410 | 5675052 | 1,51 | -1,55 | 3,07 | 0,00429 | 0,049 | Distal Intergenic | 589017 | ENSG00000246089 | LOC100287015 |
| chr16 | 85049831 | 85050989 | 2,64 | -0,51 | 3,16 | 0,00429 | 0,049 | Distal Intergenic | -4690 | ENSG00000153786 | ZDHHC7 |
| chr6 | 111478244 | 111478817 | 1,57 | -1,55 | 3,12 | 0,0043 | 0,0491 | Intron (uc003pur.4/117247, intron 1 of 2) | -19598 | ENSG00000112394 | SLC16A10 |
| chr9 | 105235220 | 105235842 | 1,88 | -0,94 | 2,83 | 0,00431 | 0,0492 | Distal Intergenic | -521751 | ENSG00000155833 | CYLC2 |
| chr1 1 | 113797264 | 113797796 | 2 | -0,87 | 2,86 | 0,00431 | 0,0492 | Intron (uc001pok.3/9177, intron 2 of 8) | 17309 | ENSG00000149305 | HTR3B |
| chr6 | 76329199 | 76331616 | 5,13 | 2,14 | 2,99 | 0,00432 | 0,0492 | Exon (uc003pic.2/26054, exon 2 of 15) | -11681 | ENSG00000112701 | SENP6 |
| chr4 | 31749855 | 31750316 | 1,65 | -1,38 | 3,04 | 0,00432 | 0,0492 | Distal Intergenic | 1027818 | ENSG00000169851 | PCDH7 |
| chr18 | 77959610 | 77960150 | 1,55 | -1,55 | 3,1 | 0,00432 | 0,0492 | Intron (uc0021ny.3/84552, intron 2 of 2) | 45247 | ENSG00000178184 | PARD6G |
| chr10 | 83440375 | 83441508 | 3,23 | 0,01 | 3,22 | 0,00432 | 0,0492 | Distal Intergenic | -193562 | ENSG00000185737 | NRG3 |
| chr14 | 55196844 | 55197371 | 1,71 | -0,79 | 2,5 | 0,00434 | 0,0494 | Intron (uc001xbb.4/23034, intron 2 of 11) | -24135 | ENSG00000020577 | SAMD4A |
| chr18 | 41487156 | 41487798 | 1,46 | -1,55 | 3,02 | 0,00434 | 0,0494 | Distal Intergenic | -629541 | ENSG00000132872 | SYT4 |
| chr7 | 8787732 | 8788514 | 3,5 | 0,48 | 3,02 | 0,00434 | 0,0494 | Intron (uc003srv.3/30010, intron 2 of 2) | 314147 | ENSG00000122584 | NXPH1 |
| chr5 | 112017037 | 112017608 | 2,27 | -0,32 | 2,58 | 0,00435 | 0,0494 | Distal Intergenic | -25594 | ENSG00000134982 | APC |
| chr1 | 243508486 | 243509021 | 1,35 | -1,55 | 2,9 | 0,00435 | 0,0494 | Promoter (<=1kb) | -457 | ENSG00000265201 | MIR4677 |
| chr2 | 67422427 | 67423098 | 1,22 | -1,55 | 2,78 | 0,00435 | 0,0495 | Intron (uc002sdx.3/uc002sdx.3, intron 1 of 4) | 19353 | ENSG00000236780 | LINC01829 |
| chr13 | 44833044 | 44834560 | 4,12 | 1,3 | 2,82 | 0,00437 | 0,0496 | Distal Intergenic | -100686 | ENSG00000235285 | SMIM2-IT1 |
| chr2 | 85157591 | 85159283 | 4,49 | 1,35 | 3,14 | 0,00437 | 0,0496 | Distal Intergenic | 24828 | ENSG00000034510 | TMSB10 |
| chr18 | 26683516 | 26684113 | 1,73 | -1,55 | 3,28 | 0,00437 | 0,0497 | Distal Intergenic | -926071 | ENSG00000170558 | CDH2 |
| chr17 | 1737858 | 1738462 | 2,8 | 0,19 | 2,62 | 0,00438 | 0,0497 | Intron (uc002fto.2/6117, intron 1 of 16) | 4585 | ENSG00000132383 | RPA1 |
| chr7 | 28331966 | 28332600 | 2,08 | -0,88 | 2,96 | 0,00438 | 0,0497 | Distal Intergenic | -6340 | ENSG00000146592 | CREB5 |
| chr1 | 223566271 | 223567423 | 3,64 | 0,31 | 3,33 | 0,00438 | 0,0497 | Promoter (<=1kb) | 0 | ENSG00000178395 | CCDC185 |
| chr10 | 125306270 | 125306919 | 1,68 | -1,55 | 3,23 | 0,00439 | 0,0497 | Distal Intergenic | -118952 | ENSG00000 154478 | GPR26 |
| chr6 | 22471517 | 22472424 | 2,97 | -0,25 | 3,21 | 0,00439 | 0,0498 | Distal Intergenic | -97254 | ENSG00000 112273 | HDGFL1 |
| chr15 | 60849684 | 60850043 | 1,04 | -1,55 | 2,6 | 0,0044 | 0,0499 | Intron (uc002agt.4/6095, intron 1 of 9) | 34664 | ENSG00000069667 | RORA |
| chr21 | 16064390 | 16064810 | 1,26 | -1,55 | 2,82 | 0,0044 | 0,0499 | Distal Intergenic | -48962 | ENSG00000155307 | LOC388813 |
| chr7 | 8426518 | 8427889 | 3,46 | 0,08 | 3,38 | 0,0044 | 0,0499 | Distal Intergenic | -45696 | ENSG00000122584 | NXPH1 |
| chr4 | 84156512 | 84156976 | 1,46 | -1,55 | 3,01 | 0,00441 | 0,05 | Distal Intergenic | 43291 | ENSG00000173085 | COQ2 |

**Table 3: H3K4me3 differentially bound sites significantly enriched in the romidepsin-sensitive cell lines compared to the romidepsin-resistant cell lines**

| Seqnames | Start | End | Conc resistant | Conc sensitive | Fold | p.value | FDR | Annotation | Distance to TSS | ENSEMBL | SYMBOL |
|---|---|---|---|---|---|---|---|---|---|---|---|
| chr4 | 7754447 | 7759273 | -0,53 | 8,17 | -8,7 | 2,02E-25 | 1,19E-20 | Promoter (<=1kb) | 0 | ENSG00000272620 | AFAP1-AS1 |
| chr7 | 126307600 | 126309976 | -2,12 | 7,13 | -9,25 | 5,83E-15 | 1,72E-10 | Intron (uc003vlr.2/2918, intron 6 of 9) | 388262 | ENSG00000207692 | MIR592 |
| chrX | 37337335 | 37343103 | -1,21 | 7,47 | -8,68 | 1,64E-14 | 3,23E-10 | Distal Intergenic | -87719 | ENSG00000147036 | LANCL3 |
| chr8 | 15741287 | 15745557 | -2,12 | 7,23 | -9,35 | 4,03E-13 | 5,95E-09 | Distal Intergenic | 140240 | ENSG00000104723 | TUSC3 |
| chr2 | 6071833 | 6075960 | -2,12 | 7,24 | -9,36 | 5,40E-10 | 5,15E-06 | Promoter (<=1kb) | 0 | NA | SILC1 |
| chr19 | 54056562 | 54058776 | -2,12 | 6,56 | -8,68 | 2,01E-09 | 1,48E-05 | Promoter (<=1kb) | 0 | ENSG00000130844 | ZNF331 |
| chr19 | 29092558 | 29104070 | -2,12 | 8,68 | -10,8 | 3,54E-09 | 2,01E-05 | Intron (uc002nsa.2/uc002nsa.2, intron 5 of 9) | -351968 | ENSG00000267339 | LINC00906 |
| chr8 | 116459246 | 116465306 | -2,12 | 6,45 | -8,57 | 3,86E-09 | 2,01E-05 | Intron (uc011lhy.2/7227, intron 4 of 5) | 214928 | ENSG00000104447 | TRPS1 |
| chrX | 130879997 | 130882192 | -1,36 | 6,26 | -7,62 | 5,90E-09 | 2,68E-05 | Intron (uc004ewi.3/286467, intron 10 of 12) | 82479 | ENSG00000213468 | FIRRE |
| chr9 | 112259181 | 112261144 | -0,71 | 7,46 | -8,16 | 8,69E-09 | 3,42E-05 | Promoter (<=1kb) | 0 | ENSG00000070159 | PTPN3 |
| chr1 1 | 63858116 | 63860127 | -2,12 | 6,46 | -8,57 | 1,37E-08 | 4,50E-05 | Intron (uc001nyh.3/28992, intron 3 of 10) | -11235 | ENSG00000 126500 | FLRT1 |
| chrX | 130925875 | 130928159 | -2,12 | 5,84 | -7,96 | 2,59E-08 | 6,52E-05 | Exon (uc004ewj.1/286467, exon 4 of 11) | 36512 | ENSG00000213468 | FIRRE |
| chr5 | 25437416 | 25446845 | -2,12 | 7,35 | -9,47 | 3,17E-08 | 7,49E-05 | Distal Intergenic | -596724 | ENSG00000248908 | LINC02239 |
| chr3 | 165473289 | 165484606 | -2,12 | 7,33 | -9,44 | 4,25E-08 | 9,24E-05 | Distal Intergenic | 70647 | ENSG00000 114200 | BCHE |
| chr5 | 25961530 | 25968047 | -2,12 | 5,91 | -8,03 | 4,44E-08 | 9,24E-05 | Distal Intergenic | 922895 | ENSG00000113100 | CDH9 |
| chrX | 130896411 | 130898560 | -1,93 | 5,67 | -7,6 | 4,54E-08 | 9,24E-05 | Intron (uc004ewi.3/286467, intron 7 of 12) | 66111 | ENSG00000213468 | FIRRE |
| chr3 | 165502292 | 165506988 | -2,12 | 5,89 | -8,01 | 5,22E-08 | 0,000103 | Exon (uc003fem.4/590, exon 3 of 4) | 48265 | ENSG00000 114200 | BCHE |
| chr5 | 13832763 | 13838513 | -1,39 | 6,47 | -7,85 | 6,63E-08 | 0,000119 | Intron (uc003jfd.2/1767, intron 35 of 78) | -63511 | ENSG00000039139 | DNAH5 |
| chr7 | 88745217 | 88750521 | -0,65 | 6,36 | -7,01 | 6,88E-08 | 0,000119 | Intron (uc01 1khi.2/219578, intron 1 of 3) | -320186 | ENSG00000 164645 | TEX47 |
| chr3 | 9987240 | 9988757 | -2,12 | 5,66 | -7,77 | 8,02E-08 | 0,000132 | Promoter (<=1kb) | -331 | ENSG00000230082 | PRRT3-AS1 |
| chr13 | 90939081 | 90942861 | -2,12 | 6,1 | -8,21 | 9,65E-08 | 0,000149 | Distal Intergenic | 55645 | ENSG00000283783 | MIR622 |
| chr17 | 25957781 | 25959720 | -2,12 | 5,65 | -7,76 | 1,65E-07 | 0,000226 | Promoter (<=1kb) | 0 | ENSG00000168961 | LGALS9 |
| chrX | 130885811 | 130887926 | -2,12 | 5,26 | -7,37 | 1,70E-07 | 0,000228 | Intron (uc004ewi.3/286467, intron 9 of 12) | 76745 | ENSG00000213468 | FIRRE |
| chr2 | 191844177 | 191846411 | -2,12 | 5,78 | -7,9 | 1,81E-07 | 0,000237 | Exon (uc021vue.1/6772, exon 17 of 23) | 31993 | ENSG00000115415 | STAT1 |
| chr2 | 139536751 | 139538677 | -2,12 | 5,27 | -7,39 | 2,05E-07 | 0,000247 | Promoter (<=1kb) | 0 | ENSG00000144227 | NXPH2 |
| chr10 | 21065624 | 21068298 | -2,12 | 5,06 | -7,18 | 2,05E-07 | 0,000247 | Downstream (<1kb) | 118233 | ENSG00000078114 | NEBL |
| chr5 | 27247400 | 27251507 | -2,12 | 5,34 | -7,46 | 2,34E-07 | 0,000273 | Distal Intergenic | -208711 | ENSG00000113100 | CDH9 |
| chr14 | 47313401 | 47316043 | -2,12 | 5,19 | -7,31 | 2,36E-07 | 0,000273 | Exon (uc001wwh.4/161357, exon 5 of 6) | 35381 | ENSG00000139915 | MDGA2 |
| chrX | 42315147 | 42320577 | -1,62 | 6,15 | -7,77 | 2,63E-07 | 0,000293 | Distal Intergenic | 316909 | ENSG00000102055 | PPP1R2C |
| chr19 | 51713652 | 51715534 | 0,22 | 6,73 | -6,51 | 2,75E-07 | 3,00E-04 | Distal Intergenic | -12801 | ENSG00000105383 | CD33 |
| chr14 | 81788060 | 81791002 | -2,12 | 5,44 | -7,56 | 3,00E-07 | 0,000321 | Intron (uc010tvu.3/85439, intron 4 of 5) | -42898 | ENSG00000 140022 | STON2 |
| chr1 | 217248622 | 217252227 | -1,62 | 5,58 | -7,19 | 3,07E-07 | 0,000321 | Promoter (<=1kb) | 0 | ENSG00000 196482 | ESRRG |
| chr8 | 3818520 | 3823059 | -0,69 | 6,2 | -6,89 | 3,10E-07 | 0,000321 | Intron (uc022aqr.1/64478, intron 5 of 69) | -551393 | ENSG00000183117 | CSMD1 |
| chr13 | 20694256 | 20696282 | -2,12 | 5,45 | -7,56 | 3,26E-07 | 0,000327 | Distal Intergenic | 38901 | ENSG00000121743 | GJA3 |
| chr8 | 16353254 | 16356217 | -1,14 | 5,99 | -7,13 | 3,67E-07 | 0,000361 | Distal Intergenic | -302954 | ENSG00000038945 | MSR1 |
| chr8 | 5367445 | 5370456 | -2,12 | 5,18 | -7,29 | 3,99E-07 | 0,000382 | Distal Intergenic | -515117 | ENSG00000183117 | CSMD1 |
| chr10 | 19492248 | 19497837 | -2,12 | 5,5 | -7,62 | 4,02E-07 | 0,000382 | Distal Intergenic | 543935 | ENSG00000165997 | ARL5B |
| chr3 | 165527782 | 165531035 | -2,12 | 5,5 | -7,62 | 4,22E-07 | 0,000395 | Intron (uc003fem.4/590, intron 2 of 3) | 24218 | ENSG00000 114200 | BCHE |
| chr4 | 189139366 | 189141253 | -2,12 | 4,51 | -6,62 | 4,30E-07 | 0,000396 | Distal Intergenic | 74569 | ENSG00000184108 | TRIML1 |
| chr19 | 939812 | 942436 | -0,48 | 6,53 | -7,01 | 4,35E-07 | 0,000396 | Intron (uc002lql.3/1820, intron 3 of 8) | 13775 | ENSG00000116017 | ARID3A |
| chr18 | 62590578 | 62591754 | -2,12 | 4,9 | -7,01 | 4,45E-07 | 0,000398 | Distal Intergenic | -663288 | ENSG00000266952 | LINC01538 |
| chr19 | 51727770 | 51729790 | -0,01 | 6,87 | -6,88 | 5,02E-07 | 0,000419 | Promoter (<=1kb) | 0 | ENSG00000105383 | CD33 |
| chr15 | 79338203 | 79340317 | -2,12 | 4,67 | -6,79 | 5,06E-07 | 0,000419 | Exon (uc002bep.3/5923, exon 5 of 27) | 16624 | ENSG00000058335 | RASGRF1 |
| chr13 | 103821163 | 103822323 | -2,12 | 4,25 | -6,37 | 5.11E-07 | 0,000419 | Distal Intergenic | -101967 | ENSG00000125255 | SLC10A2 |
| chr22 | 39695281 | 39696723 | -1,39 | 4,51 | -5,89 | 5,53E-07 | 0,000448 | Distal Intergenic | 18395 | ENSG00000263764 | SNORD43 |
| chr5 | 20106834 | 20109945 | -2,12 | 5,24 | -7,35 | 5,65E-07 | 0,000451 | Intron (uc003jgc.3/1016, intron 2 of 14) | -118481 | ENSG00000 145526 | CDH18 |
| chr1 | 240567258 | 240571304 | -1,29 | 5,82 | -7,11 | 5,92E-07 | 0,000461 | Intron (uc010pyd.2/56776, intron 15 of 17) | -49490 | ENSG00000155816 | FMN2 |
| chr13 | 24650159 | 24651204 | 0,15 | 4,86 | -4,71 | 5,94E-07 | 0,000461 | Intron (uc001upd.3/221178, intron 3 of 14) | -83657 | ENSG00000182957 | SPATA13 |
| chr7 | 17657318 | 17658652 | -2,12 | 4,63 | -6,75 | 6,04E-07 | 0,000463 | Distal Intergenic | 202668 | ENSG00000071189 | SNX13 |
| chr7 | 83774895 | 83776877 | -2,12 | 4,67 | -6,79 | 6,45E-07 | 0,000482 | Intron (uc003uhz.3/10371, intron 1 of 16) | 47340 | ENSG00000075213 | SEMA3 A |
| chr7 | 12360025 | 12363638 | -2,12 | 5,27 | -7,39 | 6,70E-07 | 0,000485 | Distal Intergenic | 80214 | ENSG00000146530 | VWDE |
| chr5 | 25426892 | 25431619 | -2,12 | 5,24 | -7,35 | 6,73E-07 | 0,000485 | Distal Intergenic | -586200 | ENSG00000248908 | LINC02239 |
| chr8 | 112396719 | 112398030 | -1,44 | 4,52 | -5,97 | 6,96E-07 | 0,000489 | Distal Intergenic | 951930 | ENSG00000164796 | CSMD3 |
| chr2 | 105554627 | 105556887 | -1,83 | 4,51 | -6,34 | 7,10E-07 | 0,000493 | Distal Intergenic | 82658 | ENSG00000198914 | POU3F3 |
| chrX | 53308734 | 53311111 | 0,3 | 6,41 | -6,11 | 7,28E-07 | 0,000499 | Promoter (<=1kb) | 0 | ENSG00000 124313 | IQSEC2 |
| chr15 | 87666771 | 87668198 | -1,86 | 5,29 | -7,14 | 7,76E-07 | 0,000515 | Distal Intergenic | -451962 | ENSG00000259527 | LINC00052 |
| chr8 | 116942339 | 116943483 | -1,73 | 4,57 | -6,3 | 8,09E-07 | 0,000525 | Distal Intergenic | -261111 | ENSG00000104447 | TRPS1 |
| chr13 | 58891680 | 58894023 | 0,02 | 6,74 | -6,72 | 8,34E-07 | 0,000529 | Distal Intergenic | 650893 | ENSG00000 118946 | PCDH17 |
| chr2 | 103187896 | 103189696 | -2,12 | 4,52 | -6,64 | 8,71E-07 | 0,000547 | Distal Intergenic | -46470 | ENSG00000115616 | SLC9A2 |
| chr18 | 6370296 | 6371445 | -2,12 | 4,66 | -6,78 | 9,05E-07 | 0,000551 | Promoter (2-3kb) | -2614 | ENSG00000154655 | L3MB1L4 |
| chr1 | 190547041 | 190549472 | -2,12 | 5,05 | -7,16 | 9,86E-07 | 0,000582 | Distal Intergenic | -44548 | ENSG00000231175 | LINC0 1720 |
| chr13 | 77453071 | 77454455 | -0,72 | 4,28 | -5,01 | 1,14E-06 | 0,000649 | 3'UTR | 6085 | ENSG00000178695 | KCTD12 |
| chr8 | 116572384 | 116574279 | -2,12 | 4,37 | -6,49 | 1,27E-06 | 0,000688 | Intron (uc011lhy.2/7227, intron 4 of 5) | 105955 | ENSG00000104447 | TRPS1 |
| chr18 | 70304339 | 70305734 | -1,28 | 4,88 | -6,16 | 1,27E-06 | 0,000688 | Distal Intergenic | -92616 | ENSG00000141668 | CBLN2 |
| chr8 | 89241034 | 89246468 | 0,12 | 6,82 | -6,71 | 1,34E-06 | 0,000715 | Intron (uc003yeb.4/4325, intron 1 of 9) | 93249 | ENSG00000156103 | MMP16 |
| chr5 | 80475459 | 80476975 | -1,83 | 4,61 | -6,44 | 1,34E-06 | 0,000715 | Exon (uc003kha.2/5924, exon 18 of 27) | -24487 | ENSG00000199347 | RNU5E-1 |
| chr6 | 162391871 | 162394189 | -2,12 | 4,44 | -6,56 | 1,42E-06 | 0,000747 | Intron (uc003qtv.4/5071, intron 4 of 6) | -133818 | ENSG00000185345 | PRKN |
| chr4 | 94995558 | 94997894 | -2,12 | 4,9 | -7,02 | 1,55E-06 | 0,000811 | Distal Intergenic | -130865 | ENSG00000 163104 | SMARCAD1 |
| chr19 | 28891128 | 28893723 | -2,12 | 4,86 | -6,98 | 1,57E-06 | 0,000813 | Distal Intergenic | -562315 | ENSG00000267339 | LINC00906 |
| chr3 | 170956406 | 170959262 | -2,12 | 4,87 | -6,99 | 1,65E-06 | 0,000842 | Intron (uc003fhh.2/23043, intron 2 of 32) | -131858 | ENSG00000207963 | MIR569 |
| chr3 | 165468117 | 165470141 | -2,12 | 4,65 | -6,77 | 1,68E-06 | 0,000843 | Distal Intergenic | 85112 | ENSG00000 114200 | BCHE |
| chr1 1 | 26119353 | 26122199 | -2,12 | 4,82 | -6,94 | 1,69E-06 | 0,000843 | Distal Intergenic | -88630 | ENSG00000134343 | ANO3 |
| chr6 | 9140128 | 9141895 | -2,12 | 4,22 | -6,33 | 1,80E-06 | 0,000894 | Distal Intergenic | 487686 | NA | HULC |
| chr15 | 40360875 | 40362833 | -2,12 | 4,59 | -6,71 | 1,90E-06 | 0,000903 | Distal Intergenic | 29363 | ENSG00000248508 | SRP14-AS1 |
| chr22 | 23247040 | 23250444 | -2,12 | 4,68 | -6,8 | 1,93E-06 | 0,000913 | Exon (uc031rxg.1/uc03lrxg.1, exon 455 of 462) | 17080 | ENSG00000254709 | IGLL5 |
| chr20 | 12680347 | 12682138 | -2,12 | 4,91 | -7,02 | 1,99E-06 | 0,000933 | Distal Intergenic | -307489 | ENSG00000172296 | SPTLC3 |
| chr2 | 130075491 | 130077134 | -1,21 | 4,96 | -6,17 | 2,20E-06 | 0,00102 | Distal Intergenic | 614756 | ENSG00000214100 | PLAC9P1 |
| chr1 1 | 89989162 | 89991328 | -2,12 | 4,69 | -6,81 | 2,40E-06 | 0,00108 | Distal Intergenic | -32630 | ENSG00000110172 | CHORDC1 |
| chr8 | 89284531 | 89285818 | -2,12 | 4,29 | -6,41 | 2,53E-06 | 0,00113 | Intron (uc003yeb.4/4325, intron 1 of 9) | 53899 | ENSG00000156103 | MMP16 |
| chr2 | 21365332 | 21366873 | -2,12 | 4,2 | -6,32 | 2,69E-06 | 0,00119 | Distal Intergenic | -98387 | ENSG00000084674 | APOB |
| chr2 | 194047434 | 194049506 | -2,12 | 4,33 | -6,44 | 2,81E-06 | 0,00123 | Distal Intergenic | 432863 | ENSG00000227418 | PCGEM1 |
| chr7 | 5458095 | 5470245 | 9,88 | 11,24 | -1,36 | 2,83E-06 | 0,00123 | Promoter (<=1kb) | 0 | ENSG00000182095 | TNRC18 |
| chr4 | 149521560 | 149523388 | -2,12 | 4,3 | -6,42 | 2,92E-06 | 0,00126 | Distal Intergenic | -157888 | ENSG00000 151623 | NR3C2 |
| chr20 | 14343815 | 14344925 | -2,12 | 3,66 | -5,78 | 2,96E-06 | 0,00127 | Intron (uc002wot.3/14073 3, intron 3 of 16) | -25502 | ENSG00000 125848 | FLRT3 |
| chr6 | 164343993 | 164345707 | -2,12 | 4,52 | -6,64 | 2,99E-06 | 0,00127 | Distal Intergenic | 508318 | ENSG00000112531 | QKI |
| chr8 | 2069871 | 2072690 | -2,12 | 4,45 | -6,57 | 3,07E-06 | 0,00129 | Exon (uc003wpx.4/9172, exon 29 of 37) | 76713 | ENSG00000036448 | MYOM2 |
| chr4 | 189917320 | 189918742 | -2,12 | 3,62 | -5,74 | 3,26E-06 | 0,00136 | Distal Intergenic | 540588 | ENSG00000249378 | LINC01060 |
| chr1 1 | 128861269 | 128862147 | -2,12 | 3,5 | -5,62 | 3,28E-06 | 0,00136 | Intron (uc009zco.3/9743, intron 1 of 10) | 6216 | ENSG00000134909 | ARHGAP3 2 |
| chr2 | 21420109 | 21422851 | -2,12 | 4,6 | -6,72 | 3,34E-06 | 0,00137 | Distal Intergenic | -153164 | ENSG00000084674 | APOB |
| chr3 | 165464384 | 165467844 | -2,12 | 4,69 | -6,81 | 3,35E-06 | 0,00137 | Distal Intergenic | 87409 | ENSG00000 114200 | BCHE |
| chr16 | 10475656 | 10476630 | -1,73 | 3,27 | -5 | 3,64E-06 | 0,00147 | Distal Intergenic | -3282 | ENSG00000 166669 | ATF7IP2 |
| chr7 | 86272954 | 86274789 | -1,06 | 5,13 | -6,19 | 4,09E-06 | 0,00163 | Promoter (<=1kb) | 0 | ENSG00000198822 | GRM3 |
| chr7 | 14854748 | 14857050 | -2,12 | 4,46 | -6,57 | 4,28E-06 | 0,00166 | Intron (uc003ssz.3/1607, intron 1 of 24) | 24025 | ENSG00000136267 | DGKB |
| chr8 | 89274286 | 89275847 | -2,12 | 4,33 | -6,45 | 4,29E-06 | 0,00166 | Intron (uc003veb.4/4325, intron 1 of 9) | 63870 | ENSG00000156103 | MMP16 |
| chr1 | 17914352 | 17915505 | -2,12 | 3,9 | -6,02 | 4,30E-06 | 0,00166 | Promoter (<=1kb) | 0 | ENSG00000074964 | ARHGEF10L |
| chr3 | 185703526 | 185704640 | -2,12 | 4,1 | -6,22 | 4,44E-06 | 0,0017 | Distal Intergenic | 25768 | ENSG00000171658 | NMRAL2P |
| chr5 | 27037848 | 27039278 | -2,12 | 4,53 | -6,65 | 4,55E-06 | 0,00173 | Promoter (<=1kb) | 0 | ENSG00000113100 | CDH9 |
| chr13 | 71967398 | 71968727 | -1,28 | 4,25 | -5,53 | 4,64E-06 | 0,00176 | Distal Intergenic | 378125 | ENSG00000226846 | LINC00348 |
| chr1 1 | 133445682 | 133447032 | -1,68 | 4,6 | -6,28 | 4,80E-06 | 0,00178 | Distal Intergenic | -43279 | ENSG00000183715 | OPCML |
| chr7 | 77687406 | 77688401 | -1,29 | 4,4 | -5,69 | 4,82E-06 | 0,00178 | Intron (uc003ugx.3/9863, intron 21 of 21) | 149296 | ENSG00000006576 | PHTF2 |
| chr18 | 44811724 | 44814263 | -0,99 | 5,15 | -6,14 | 4,98E-06 | 0,00181 | 5'UTR | -36170 | ENSG00000215474 | SKOR2 |
| chr2 | 201020689 | 201021562 | -2,12 | 3,04 | -5,16 | 5,00E-06 | 0,00181 | Distal Intergenic | -149042 | ENSG00000196141 | SPATS2L |
| chr6 | 154567197 | 154568886 | -2,12 | 3,57 | -5,69 | 5,10E-06 | 0,00182 | 3'UTR | -32733 | ENSG00000074706 | IPCEF1 |
| chr4 | 136442707 | 136445397 | -2,12 | 4,31 | -6,43 | 5,11E-06 | 0,00182 | Distal Intergenic | -1E+06 | ENSG00000254535 | PABPC4L |
| chr3 | 194395765 | 194396734 | -2,12 | 3,66 | -5,78 | 5,16E-06 | 0,00182 | Promoter (2-3kb) | -2559 | ENSG00000041802 | LSG1 |
| chr5 | 147466622 | 147468866 | -2,12 | 4,4 | -6,52 | 5,35E-06 | 0,00188 | Exon (uc010jgq.1/11005, exon 6 of 12) | 23087 | ENSG00000133710 | SPINK5 |
| chr21 | 22523594 | 22526261 | -2,12 | 4,13 | -6,24 | 5,42E-06 | 0,00189 | Intron (uc002yld.2/4685, intron 1 of 17) | 4156 | ENSG00000154654 | NCAM2 |
| chr18 | 28464383 | 28465482 | -2,12 | 3,36 | -5,47 | 5,50E-06 | 0,00191 | Distal Intergenic | 157299 | ENSG00000134762 | DSC3 |
| chr2 | 67902541 | 67903160 | -1,59 | 3,05 | -4,64 | 5,63E-06 | 0,00193 | Distal Intergenic | 278099 | ENSG00000143971 | ETAA1 |
| chr8 | 55409703 | 55410695 | -2,12 | 3,33 | -5,44 | 5,69E-06 | 0,00194 | Distal Intergenic | 39208 | ENSG00000164736 | SOX17 |
| chr13 | 106810632 | 106812548 | -2,12 | 4,45 | -6,56 | 5,90E-06 | 0,002 | Distal Intergenic | -216363 | NA | LINC00460 |
| chr15 | 60987314 | 60988552 | -1,21 | 3,98 | -5,19 | 5,96E-06 | 0,00201 | Intron (uc002agx.3/6095, intron 1 of 10) | -67585 | ENSG00000069667 | RORA |
| chr9 | 77462764 | 77463851 | -2,12 | 3,61 | -5,73 | 6,14E-06 | 0,00203 | Intron (uc004ajk.1/140803. intron 3 of 38) | 38412 | ENSG00000119121 | TRPM6 |
| chr13 | 113496585 | 113497611 | -2,12 | 3,81 | -5,92 | 6,21E-06 | 0,00203 | Promoter (<=1kb) | 0 | ENSG00000068650 | ATP11A |
| chr18 | 35763233 | 35764276 | -2,12 | 3,55 | -5,67 | 6,24E-06 | 0,00203 | Distal Intergenic | 526135 | ENSG00000266530 | MIR4318 |
| chr3 | 165938043 | 165939047 | -2,12 | 4 | -6,11 | 6,27E-06 | 0,00203 | Distal Intergenic | -382790 | ENSG00000 114200 | BCHE |
| chr9 | 24470922 | 24471871 | -2,12 | 3,42 | -5,54 | 6,30E-06 | 0,00203 | Distal Intergenic | 73803 | ENSG00000205442 | IZUMO3 |
| chr5 | 21001387 | 21003588 | -2,12 | 4,26 | -6,38 | 6,31E-06 | 0,00203 | Distal Intergenic | -338354 | ENSG00000183666 | GUSBP1 |
| chr1 | 241307622 | 241308741 | -1,57 | 3,62 | -5,19 | 6,31E-06 | 0,00203 | Promoter (<=1kb) | 0 | ENSG00000182901 | RGS7 |
| chr12 | 131513041 | 131514155 | -2,12 | 3,46 | -5,58 | 6,56E-06 | 0,00207 | Intron (uc001uit.4/283383, intron 13 of 24) | -41243 | ENSG00000 111452 | ADGRD1 |
| chr16 | 73587154 | 73590576 | -2,12 | 4,33 | -6,45 | 6,96E-06 | 0,00219 | Distal Intergenic | 166450 | ENSG00000258779 | LINC01568 |
| chr10 | 21225802 | 21227320 | -2,12 | 3,33 | -5,45 | 7,05E-06 | 0,0022 | Intron (uc001iqk.3/10529, intron 4 of 6) | -39271 | ENSG00000078114 | NEBL |
| chr6 | 98204772 | 98205463 | -2,12 | 3,3 | -5,42 | 7,19E-06 | 0,00222 | Distal Intergenic | -266944 | ENSG00000238367 | MIR2113 |
| chr21 | 22754318 | 22756951 | -2,12 | 4,31 | -6,43 | 7,29E-06 | 0,00224 | Intron (uc002yld.2/4685, intron 9 of 17) | 96496 | NA | RNU6-67P |
| chr21 | 17958840 | 17961462 | 0,07 | 6,32 | -6,24 | 7,36E-06 | 0,00225 | Promoter (1-2kb) | -1095 | ENSG00000207863 | MIR125B2 |
| chr3 | 9056738 | 9057515 | -2,12 | 3,27 | -5,39 | 7,42E-06 | 0,00225 | Exon (uc003brf.2/9901, exon 15 of 22) | -51579 | ENSG00000070950 | RAD18 |
| chr6 | 164404276 | 164405846 | -2,12 | 3,89 | -6,01 | 7,44E-06 | 0,00225 | Distal Intergenic | 568601 | ENSG00000112531 | QKI |
| chr3 | 85575878 | 85576695 | -2,12 | 4,06 | -6,18 | 7,52E-06 | 0,00225 | Intron (uc003dqj.3/253559, intron 1 of 9) | 21669 | ENSG00000175161 | CADM2 |
| chr14 | 40868234 | 40870189 | -2,12 | 4,34 | -6,46 | 7,56E-06 | 0,00225 | Distal Intergenic | -966530 | ENSG00000165355 | FBXO33 |
| chr4 | 176198531 | 176199704 | -2,12 | 4,25 | -6,37 | 7,74E-06 | 0,0023 | Distal Intergenic | 359022 | ENSG00000 168594 | ADAM2 9 |
| chr5 | 14249405 | 14251935 | -2,12 | 4,08 | -6,2 | 8,01E-06 | 0,00236 | Intron (uc003jff.3/7204, intron 1 of 56) | -41186 | ENSG00000038382 | TRIO |
| chr3 | 175389765 | 175390591 | -2,12 | 3,7 | -5,82 | 8,09E-06 | 0,00236 | Intron (uc003fit.3/254827, intron 11 of 13) | 302436 | ENSG00000264974 | MIR4789 |
| chr9 | 129916206 | 129917980 | -2,12 | 4,18 | -6,29 | 8,35E-06 | 0,00243 | Intron (uc022bno.1/9649, intron 8 of 11) | -31162 | ENSG00000136859 | ANGPTL2 |
| chr3 | 30935556 | 30936645 | -0,18 | 5,14 | -5,32 | 8,42E-06 | 0,00244 | Promoter (<=1kb) | 0 | ENSG00000 144644 | GADL1 |
| chr12 | 106176550 | 106179246 | -1,83 | 4,03 | -5,86 | 8,54E-06 | 0,00246 | Distal Intergenic | 354565 | ENSG0000007 4590 | NUAK1 |
| chr3 | 107815456 | 107816208 | -1,25 | 3,77 | -5,02 | 8,63E-06 | 0,00247 | Distal Intergenic | -5521 | ENSG00000196776 | CD47 |
| chr6 | 162670084 | 162671487 | -1,91 | 4,17 | -6,08 | 9,08E-06 | 0,00255 | Intron (uc003qtv.4/5071, intron 1 of 6) | 12310 | ENSG00000185345 | PRKN |
| chr2 | 21414676 | 21415570 | -2,12 | 3,61 | -5,72 | 9,16E-06 | 0,00256 | Distal Intergenic | -147731 | ENSG00000084674 | APOB |
| chr2 | 137703544 | 137704943 | -2,12 | 3,45 | -5,57 | 9,37E-06 | 0,00261 | Intron (uc010zbj.1/80731, intron 3 of 8) | -43519 | ENSG00000144229 | THSD7B |
| chr2 | 194045734 | 194047355 | -2,12 | 4,17 | -6,28 | 9,40E-06 | 0,00261 | Distal Intergenic | 431163 | ENSG00000227418 | PCGEM1 |
| chr21 | 26736687 | 26737618 | -2,12 | 3,35 | -5,47 | 9,49E-06 | 0,00262 | Distal Intergenic | 66395 | ENSG00000185433 | LINC00158 |
| chr4 | 189129640 | 189131388 | -1,83 | 3,65 | -5,48 | 9,77E-06 | 0,00266 | Distal Intergenic | 64843 | ENSG00000184108 | TRIML1 |
| chrl 5 | 81108981 | 81109748 | -2,12 | 2,85 | -4,97 | 9,77E-06 | 0,00266 | Intron (uc002bfw.1/57214, intron 1 of 28) | 24666 | ENSG00000208003 | MIR549A |
| chr5 | 20370800 | 20372239 | -2,12 | 4,08 | -6,2 | 9,86E-06 | 0,00267 | Intron (uc003jgc.3/1016, intron 1 of 14) | 203743 | ENSG00000145526 | CDH18 |
| chr1 | 191421854 | 191422949 | -2,12 | 3,78 | -5,89 | 1,00E-05 | 0,00268 | Distal Intergenic | -704643 | ENSG00000150681 | RGS18 |
| chr5 | 127104 | 128809 | -1,14 | 4,22 | -5,36 | 1,03E-05 | 0,00273 | Distal Intergenic | -11564 | ENSG00000153404 | PLEKHG4B |
| chr8 | 2111797 | 2113192 | -2,12 | 4,01 | -6,13 | 1,04E-05 | 0,00274 | Distal Intergenic | 118639 | ENSG00000036448 | MYOM2 |
| chr8 | 2048094 | 2049892 | -2,12 | 3,99 | -6,11 | 1,10E-05 | 0,00285 | Exon (uc003wpx.4/9172, exon 20 of 37) | 54936 | ENSG00000036448 | MYOM2 |
| chr7 | 21409945 | 21410660 | -2,12 | 3,04 | -5,16 | 1,13E-05 | 0,00291 | Distal Intergenic | -57029 | ENSG00000105866 | SP4 |
| chr3 | 145615700 | 145617515 | -2,12 | 3,82 | -5,94 | 1,15E-05 | 0,00293 | Distal Intergenic | 187007 | ENSG00000152952 | PLOD2 |
| chr9 | 14317573 | 14319294 | -2,12 | 3,46 | -5,58 | 1,15E-05 | 0,00293 | Intron (uc022bdp.1/4781, intron 1 of 8) | -3528 | ENSG00000147862 | NFIB |
| chr2 | 207864624 | 207866738 | 0,89 | 6,25 | -5,36 | 1,16E-05 | 0,00293 | Distal Intergenic | 60346 | ENSG00000144410 | CPO |
| chr8 | 3810920 | 3818251 | 0,46 | 6,67 | -6,21 | 1,17E-05 | 0,00295 | Intron (uc022aqr.1/64478, intron 5 of 69) | -543793 | ENSG00000183117 | CSMD1 |
| chr18 | 60767244 | 60768232 | -2,12 | 4,13 | -6,25 | 1,21E-05 | 0,00301 | Distal Intergenic | 218381 | ENSG00000171791 | BCL2 |
| chr6 | 154679335 | 154680737 | -2,12 | 3,7 | -5,81 | 1,21E-05 | 0,00301 | Promoter (1-2kb) | -1435 | ENSG00000074706 | IPCEF1 |
| chr4 | 157119132 | 157120405 | -2,12 | 3,95 | -6,06 | 1,22E-05 | 0,00301 | Distal Intergenic | -244084 | ENSG00000256043 | CTSO |
| chr7 | 153536982 | 153538313 | -2,12 | 3,77 | -5,89 | 1,24E-05 | 0,00303 | Distal Intergenic | -46106 | ENSG00000130226 | DPP6 |
| chr1 | 4403970 | 4404777 | -2,12 | 3,08 | -5,2 | 1,25E-05 | 0,00305 | Distal Intergenic | -67334 | ENSG00000235054 | LINC01777 |
| chr5 | 25448183 | 25449901 | -2,12 | 3,98 | -6,09 | 1,31E-05 | 0,00314 | Distal Intergenic | -607491 | ENSG00000248908 | LINC02239 |
| chr1 | 18905069 | 18906103 | -2,12 | 3,46 | -5,58 | 1,33E-05 | 0,00316 | Distal Intergenic | -51397 | ENSG00000009709 | PAX7 |
| chr3 | 175758762 | 175759813 | -2,12 | 3,35 | -5,47 | 1,33E-05 | 0,00316 | Distal Intergenic | 671433 | ENSG00000264974 | MIR4789 |
| chr6 | 98566326 | 98568295 | -2,12 | 4,03 | -6,14 | 1,34E-05 | 0,00316 | Distal Intergenic | 93919 | ENSG00000238367 | MIR2113 |
| chr14 | 88526999 | 88527746 | -2,12 | 3,36 | -5,48 | 1,34E-05 | 0,00316 | Intron (uc001xvw.3/283587, intron 2 of 3) | 36105 | ENSG00000258867 | LINC01146 |
| chr4 | 189922768 | 189923969 | -1,28 | 3,89 | -5,17 | 1,34E-05 | 0,00316 | Distal Intergenic | 546036 | ENSG00000249378 | LINC01060 |
| chr8 | 57565778 | 57566902 | -1,93 | 3,73 | -5,66 | 1,35E-05 | 0,00316 | Distal Intergenic | -93396 | ENSG00000246430 | LINC00968 |
| chr2 | 166105139 | 166106257 | -2,12 | 3,58 | -5,7 | 1,39E-05 | 0,00324 | Intron (uc002udc.3/6326, intron 1 of 26) | 9227 | ENSG00000136531 | SCN2A |
| chr17 | 19748355 | 19749108 | -2,12 | 3,54 | -5,66 | 1,39E-05 | 0,00324 | Exon (uc002gwm.4/9706, exon 7 of 28) | 22131 | ENSG00000083290 | ULK2 |
| chr12 | 771985 | 773085 | -2,12 | 3,2 | -5,32 | 1,40E-05 | 0,00325 | Promoter (<=1kb) | 0 | ENSG00000171840 | NINJ2 |
| chr4 | 136446708 | 136448061 | -2,12 | 3,54 | -5,66 | 1,43E-05 | 0,00328 | Distal Intergenic | -1E+06 | ENSG00000254535 | PABPC4L |
| chr3 | 165570086 | 165572001 | -2,12 | 4,01 | -6,13 | 1,44E-05 | 0,00329 | Distal Intergenic | -14833 | ENSG00000114200 | BCHE |
| chr2 | 185660265 | 185661730 | -2,12 | 3,28 | -5,4 | 1,44E-05 | 0,00329 | Intron (uc002uph.3/91752, intron 1 of 3) | 197172 | ENSG00000170396 | ZNF804A |
| chr2 | 238498322 | 238500424 | -1,04 | 4,46 | -5,5 | 1,46E-05 | 0,0033 | Promoter (<=1kb) | 0 | ENSG00000124839 | RAB17 |
| chr1 1 | 128867896 | 128868553 | -2,12 | 2,93 | -5,05 | 1,46E-05 | 0,0033 | Promoter (<=1kb) | 0 | ENSG00000134909 | ARHGAP32 |
| chr3 | 176043575 | 176045192 | -2,12 | 3,7 | -5,82 | 1,51E-05 | 0,00337 | Distal Intergenic | 869074 | ENSG00000177565 | TBL1XR1 |
| chr14 | 68334446 | 68335710 | -2,12 | 3,78 | -5,9 | 1,55E-05 | 0,00342 | Intron (uc010aqq.3/5890, intron 5 of 6) | 47950 | ENSG00000182185 | RAD51B |
| chr4 | 149747309 | 149748394 | -1,48 | 3,43 | -4,91 | 1,55E-05 | 0,00342 | Distal Intergenic | -383637 | ENSG00000151623 | NR3C2 |
| chr10 | 19467344 | 19468132 | -2,12 | 3,25 | -5,37 | 1,56E-05 | 0,00342 | Distal Intergenic | 519031 | ENSG00000165997 | ARL5B |
| chr9 | 15810325 | 15811039 | -2,12 | 3,15 | -5,27 | 1,57E-05 | 0,00343 | Intron (uc003zmd.3/203238, intron 21 of 25) | 66054 | ENSG00000164989 | CCDC171 |
| chr8 | 4441338 | 4442310 | -2,12 | 3,47 | -5,59 | 1,58E-05 | 0,00345 | Intron (uc022aqr.1/64478, intron 2 of 69) | 410018 | ENSG00000183117 | CSMD1 |
| chr8 | 87533859 | 87536154 | -2,12 | 3,83 | -5,95 | 1,59E-05 | 0,00345 | Intron (uc003vdv.2/8895, intron 2 of 16) | 7203 | ENSG00000085719 | CPNE3 |
| chr8 | 116890278 | 116892160 | -1,39 | 4,42 | -5,81 | 1,60E-05 | 0,00345 | Distal Intergenic | -209050 | ENSG00000104447 | TRPS1 |
| chr8 | 15977560 | 15978345 | -2,12 | 3,84 | -5,95 | 1,61E-05 | 0,00345 | Exon (uc010lsu.3/4481, exon 9 of 10) | 65436 | ENSG00000038945 | MSR1 |
| chr1 1 | 2484119 | 2485243 | -1,77 | 3,87 | -5,64 | 1,61E-05 | 0,00345 | Promoter (1-2kb) | 1453 | ENSG00000053918 | KCNQ1 |
| chr1 | 191564981 | 191565587 | -2,12 | 3,18 | -5,3 | 1,66E-05 | 0,00354 | Distal Intergenic | -562005 | ENSG00000150681 | RGS18 |
| chr8 | 2400553 | 2401723 | -0,96 | 3,97 | -4,93 | 1,68E-05 | 0,00357 | Distal Intergenic | 407395 | ENSG00000036448 | MYOM2 |
| chr9 | 137257556 | 137258813 | -2,12 | 3,88 | -6 | 1,72E-05 | 0,00362 | Intron (uc004cfa.1/6256, intron 2 of 2) | -12444 | ENSG00000263897 | MIR4669 |
| chrX | 43036135 | 43038568 | -2,12 | 3,87 | -5,99 | 1,72E-05 | 0,00362 | Exon (uc004dfw.1/uc004dfw.1, exon 1 of 6) | -398649 | ENSG00000102055 | PPP1R2C |
| chr2 | 11514330 | 11515380 | -2,12 | 3,47 | -5,59 | 1,75E-05 | 0,00366 | Distal Intergenic | -18727 | ENSG00000224177 | LINC00570 |
| chr10 | 9335854 | 9336959 | -2,12 | 3,72 | -5,84 | 1,78E-05 | 0,0037 | Distal Intergenic | 1239187 | ENSG00000107485 | GATA3 |
| chr5 | 19044080 | 19045889 | -2,12 | 3,78 | -5,9 | 1,79E-05 | 0,0037 | Distal Intergenic | 840492 | ENSG00000145526 | CDH18 |
| chr3 | 165563621 | 165565002 | -2,12 | 3,91 | -6,03 | 1,80E-05 | 0,0037 | Distal Intergenic | -8368 | ENSG00000114200 | BCHE |
| chr1 | 13825021 | 13826603 | -2,12 | 3,41 | -5,53 | 1,82E-05 | 0,00372 | Intron (uc001avb.3/126755, intron 1 of 1) | 13639 | ENSG00000162494 | LRRC38 |
| chr9 | 4543898 | 4545030 | -2,12 | 3,35 | -5,47 | 1,85E-05 | 0,00377 | Exon (uc003zij.2/6505, exon 2 of 12) | 53471 | ENSG00000106688 | SLC1A1 |
| chr4 | 186654889 | 186655785 | -1,59 | 3,44 | -5,03 | 1,87E-05 | 0,00379 | Intron (uc003ivh.3/8470, intron 2 of 22) | 40735 | ENSG00000154556 | SORB S2 |
| chr3 | 175602967 | 175603977 | -1,62 | 3,78 | -5,39 | 1,88E-05 | 0,00379 | Distal Intergenic | 515638 | ENSG00000264974 | MIR4789 |
| chr4 | 188401796 | 188403211 | -1,06 | 3,83 | -4,9 | 1,89E-05 | 0,00381 | Intron (uc021xvl.1/339975, intron 1 of 4) | 23556 | NA | LOC339975 |
| chr22 | 17141188 | 17142246 | -2,12 | 2,83 | -4,94 | 1,96E-05 | 0,00395 | Intron (uc002zls.1/387590, intron 2 of 2) | 14184 | NA | ANKRD62P1-PARP4P3 |
| chr7 | 41187208 | 41189958 | 0,13 | 6,28 | -6,15 | 2,01E-05 | 0,00401 | Distal Intergenic | 354152 | ENSG00000175600 | SUGCT |
| chr7 | 144569246 | 144569966 | -2,12 | 2,96 | -5,08 | 2,03E-05 | 0,00402 | Distal Intergenic | -36100 | ENSG00000196511 | TPK1 |
| chr1 1 | 134230776 | 134232179 | -1,83 | 3,96 | -5,79 | 2,04E-05 | 0,00402 | Exon (uc009zdg.1/89944, exon 6 of 22) | 14492 | ENSG00000149328 | GLB1L2 |
| chr9 | 139585899 | 139586667 | -1,93 | 3,67 | -5,6 | 2,04E-05 | 0,00402 | Distal Intergenic | -3988 | ENSG00000169692 | AGPAT2 |
| chr7 | 80550337 | 80552061 | -2,12 | 3,86 | -5,97 | 2,06E-05 | 0,00405 | Promoter (<=1kb) | 0 | ENSG00000075223 | SEMA3 C |
| chr5 | 94403822 | 94405977 | -2,12 | 3,78 | -5,9 | 2,11E-05 | 0,00409 | Intron (uc003kkv.2/79772, intron 1 of 22) | 11568 | ENSG00000175471 | MCTP1 |
| chr4 | 135542853 | 135544644 | -1,39 | 3,7 | -5,09 | 2,11E-05 | 0,00409 | Distal Intergenic | -419950 | ENSG00000254535 | PABPC4L |
| chr8 | 116714481 | 116716412 | -2,12 | 3,85 | -5,97 | 2,12E-05 | 0,00409 | Distal Intergenic | -33253 | ENSG00000104447 | TRPS1 |
| chr6 | 50810341 | 50811924 | -2,12 | 3,4 | -5,52 | 2,18E-05 | 0,00416 | 3'UTR | 23902 | ENSG00000008196 | TFAP2B |
| chr3 | 165982701 | 165983784 | -1,46 | 3,56 | -5,02 | 2,19E-05 | 0,00416 | Distal Intergenic | -427448 | ENSG00000114200 | BCHE |
| chr2 | 155311408 | 155312708 | -2,12 | 3,64 | -5,75 | 2,22E-05 | 0,0042 | Promoter (1-2kb) | 1242 | ENSG00000224675 | LOC1001445 95 |
| chr10 | 21283904 | 21285597 | -1,83 | 3,75 | -5,58 | 2,26E-05 | 0,00426 | Intron (uc001iqk.3/10529, intron 3 of 6) | -97373 | ENSG00000078114 | NEBL |
| chr1 | 11039718 | 11041130 | -2,12 | 3,4 | -5,52 | 2,29E-05 | 0,0043 | Promoter (<=1kb) | 964 | ENSG00000175262 | Clorf127 |
| chr4 | 95916704 | 95919430 | -1,83 | 4,04 | -5,87 | 2,30E-05 | 0,00432 | Promoter (<=1kb) | 0 | ENSG00000138696 | BMPR1B |
| chr7 | 53478693 | 53479545 | -2,12 | 3,03 | -5,15 | 2,34E-05 | 0,00438 | Distal Intergenic | 375344 | ENSG00000221900 | POM121L12 |
| chr3 | 135811195 | 135811844 | -1,75 | 3,21 | -4,96 | 2,35E-05 | 0,00438 | Intron (uc003eqv.2/5523, intron 10 of 13) | 65521 | ENSG00000073711 | PPP2R3 A |
| chr6 | 162483307 | 162485168 | -2,12 | 3,79 | -5,91 | 2,40E-05 | 0,00442 | Intron (uc003qtv.4/5071, intron 2 of 6) | 198629 | ENSG00000185345 | PRKN |
| chr5 | 25073075 | 25074153 | -1,93 | 3,63 | -5,56 | 2,40E-05 | 0,00442 | Distal Intergenic | -232383 | ENSG00000248908 | LINC02239 |
| chr2 | 36189202 | 36190157 | -2,12 | 3,48 | -5,6 | 2,41E-05 | 0,00442 | Distal Intergenic | 392556 | NA | CRIM1-DT |
| chr5 | 18395584 | 18396574 | -2,12 | 3,65 | -5,77 | 2,44E-05 | 0,00446 | Distal Intergenic | -1E+06 | ENSG00000250822 | LINC02111 |
| chr2 | 189029319 | 189030226 | -2,12 | 3,64 | -5,75 | 2,45E-05 | 0,00447 | Distal Intergenic | -126170 | ENSG00000144366 | GULP1 |
| chr9 | 82472974 | 82473935 | -0,31 | 3,86 | -4,17 | 2,47E-05 | 0,00449 | Distal Intergenic | 283186 | ENSG00000106829 | 1LE4 |
| chr8 | 77577574 | 77578604 | -2,12 | 3,75 | -5,87 | 2,53E-05 | 0,00457 | Intron (uc003yas.4/100192378, intron 2 of 3) | -14911 | ENSG00000091656 | ZFHX4 |
| chr8 | 116434091 | 116436136 | -2,12 | 3,75 | -5,86 | 2,54E-05 | 0,00457 | Intron (uc011lhy.2/7227, intron 4 of 5) | 244098 | ENSG00000104447 | TRPS1 |
| chrX | 130844699 | 130845751 | -2,12 | 3,51 | -5,62 | 2,56E-05 | 0,0046 | Exon (uc004ewi.3/286467, exon 11 of 13) | 118920 | ENSG00000213468 | FIRRE |
| chr9 | 24262976 | 24264068 | -2,12 | 2,83 | -4,95 | 2,57E-05 | 0,0046 | Distal Intergenic | 281606 | ENSG00000205442 | IZUM03 |
| chr1 1 | 15673211 | 15673941 | -2,12 | 3,36 | -5,47 | 2,70E-05 | 0,00478 | Distal Intergenic | 502563 | ENSG00000188487 | INSC |
| chr8 | 111127532 | 111129006 | 0,02 | 5,27 | -5,25 | 2,70E-05 | 0,00478 | Distal Intergenic | -139456 | ENSG00000164794 | KCNV1 |
| chr12 | 88811081 | 88812820 | -2,12 | 3,76 | -5,87 | 2,71E-05 | 0,0048 | Distal Intergenic | 99824 | ENSG00000049130 | KITLG |
| chr5 | 138288333 | 138289866 | -1,86 | 3,83 | -5,69 | 2,74E-05 | 0,00483 | Intron (uc003ldo.3/64374, intron 8 of 10) | 77248 | ENSG00000044115 | CTNNA1 |
| chr7 | 83810657 | 83812030 | -2,12 | 3,51 | -5,63 | 2,80E-05 | 0,00492 | Intron (uc003uhz.3/10371, intron 1 of 16) | 12187 | ENSG00000075213 | SEMA3 A |
| chrl 5 | 69108328 | 69115464 | 10,46 | 11,32 | -0,86 | 2,88E-05 | 0,005 | Promoter (<=1kb) | 0 | ENSG00000140350 | ANP32A |
| chr20 | 50820151 | 50821110 | -2,12 | 2,95 | -5,07 | 3,02E-05 | 0,00517 | Distal Intergenic | -11627 | ENSG00000020256 | ZFP64 |
| chr6 | 6834527 | 6835391 | -1,66 | 3,32 | -4,98 | 3,02E-05 | 0,00517 | Distal Intergenic | 184679 | ENSG00000112799 | LY86 |
| chr5 | 22423634 | 22424831 | -2,12 | 3,14 | -5,26 | 3,03E-05 | 0,00517 | Intron (uc003jgk.2/1010, intron 2 of 14) | -209725 | ENSG00000154162 | CDH12 |
| chr3 | 173071775 | 173072641 | -2,12 | 3,13 | -5,25 | 3,06E-05 | 0,00518 | Distal Intergenic | -43603 | ENSG00000169760 | NLGN1 |
| chr3 | 70666337 | 70667030 | -2,12 | 2,96 | -5,08 | 3,06E-05 | 0,00518 | Distal Intergenic | 423653 | ENSG00000114861 | FOXP1 |
| chr9 | 24608281 | 24609485 | -2,12 | 3,62 | -5,74 | 3,11E-05 | 0,00522 | Distal Intergenic | -62607 | ENSG00000205442 | IZUMO3 |
| chr6 | 4774839 | 4783243 | 9,29 | 10,24 | -0,95 | 3,12E-05 | 0,00522 | Promoter (<=1kb) | 0 | ENSG00000153046 | CDYL |
| chr3 | 166222979 | 166223980 | -2,12 | 3,28 | -5,4 | 3,15E-05 | 0,00522 | Distal Intergenic | -667726 | ENSG00000114200 | BCHE |
| chr8 | 39659480 | 39660481 | -2,12 | 3,06 | -5,18 | 3,15E-05 | 0,00522 | Intron (uc003xnj.4/2515, intron 7 of 20) | 35327 | ENSG00000104755 | ADAM2 |
| chr21 | 20289263 | 20290436 | -2,12 | 3,55 | -5,66 | 3,18E-05 | 0,00523 | Distal Intergenic | -513293 | ENSG00000154646 | TMPRSS15 |
| chr4 | 97009652 | 97010906 | -2,12 | 3,5 | -5,61 | 3,24E-05 | 0,00531 | Distal Intergenic | 248413 | ENSG00000163114 | PDHA2 |
| chr3 | 165178237 | 165180196 | -2,12 | 3,64 | -5,76 | 3,31E-05 | 0,00538 | Distal Intergenic | -263768 | ENSG00000121871 | SLITRK3 |
| chr5 | 27927085 | 27928389 | -1,21 | 3,87 | -5,08 | 3,31E-05 | 0,00538 | Distal Intergenic | 454686 | ENSG00000250337 | PURPL |
| chr7 | 9977734 | 9979271 | -2,12 | 3,61 | -5,73 | 3,33E-05 | 0,00538 | Distal Intergenic | 303834 | NA | PER4 |
| chr15 | 36789514 | 36790888 | -2,12 | 3,47 | -5,59 | 3,33E-05 | 0,00538 | Distal Intergenic | -80924 | ENSG00000186073 | C15orf41 |
| chr10 | 19816627 | 19818210 | -2,12 | 3,46 | -5,58 | 3,34E-05 | 0,00538 | Intron (uc010qcq.1/uc010qcq.1, intron 3 of 6) | -287162 | ENSG00000120594 | PLXDC2 |
| chr18 | 59643033 | 59643705 | -2,12 | 2,59 | -4,7 | 3,37E-05 | 0,00538 | Distal Intergenic | -82729 | ENSG00000176641 | RNF152 |
| chr1 1 | 44104796 | 44105396 | -1,77 | 2,82 | -4,58 | 3,43E-05 | 0,00543 | 3'UTR | 7720 | ENSG00000151348 | EXT2 |
| chr5 | 24267947 | 24269374 | -2,12 | 3,72 | -5,84 | 3,48E-05 | 0,00547 | Distal Intergenic | 375711 | ENSG00000040731 | CDH10 |
| chr4 | 158430081 | 158430997 | -2,12 | 3,1 | -5,22 | 3,49E-05 | 0,00547 | Distal Intergenic | -62645 | ENSG00000234111 | LINC02433 |
| chr2 | 210593248 | 210594101 | -0,8 | 3,77 | -4,57 | 3,49E-05 | 0,00547 | Intron (uc002vdd.1/4133, intron 13 of 14) | -42616 | ENSG00000144406 | UNC80 |
| chr1 1 | 113113586 | 113114659 | 0,01 | 4,43 | -4,42 | 3,51E-05 | 0,00548 | Intron (uc001pnp.3/4684, intron 16 of 18) | -11940 | ENSG00000149294 | NCAM1 |
| chr5 | 93700428 | 93701219 | -2,12 | 2,98 | -5,1 | 3,65E-05 | 0,00564 | Intron (uc003kkn.3/285600, intron 4 of 4) | 30995 | ENSG00000185261 | KIAA0825 |
| chr2 | 163472259 | 163473068 | -2,12 | 2,87 | -4,98 | 3,72E-05 | 0,00573 | Intron (uc002uch.2/90134, intron 2 of 15) | 222189 | ENSG00000184611 | KCNH7 |
| chr7 | 127124682 | 127125533 | -0,86 | 4 | -4,86 | 3,75E-05 | 0,00576 | Distal Intergenic | -91915 | ENSG00000048405 | ZNF800 |
| chr5 | 25435951 | 25437278 | -2,12 | 3,49 | -5,61 | 3,76E-05 | 0,00576 | Distal Intergenic | -595259 | ENSG00000248908 | LINC02239 |
| chr1 | 159435095 | 159436148 | 0,25 | 4,59 | -4,34 | 3,77E-05 | 0,00576 | Intron (uc001fts.4/uc001fts.4, intron 1 of 3) | 25583 | ENSG00000196184 | OR10J1 |
| chr2 | 189045853 | 189046553 | -2,12 | 2,56 | -4,68 | 3,78E-05 | 0,00576 | Distal Intergenic | -109843 | ENSG00000144366 | GULP1 |
| chr2 | 210359271 | 210360209 | -2,12 | 3,06 | -5,18 | 3,85E-05 | 0,00585 | Intron (uc002vdc.1/4133, intron 1 of 7) | 70500 | ENSG00000078018 | MAP2 |
| chr10 | 120699155 | 120699749 | -1,86 | 2,82 | -4,67 | 3,86E-05 | 0,00585 | Distal Intergenic | -89479 | ENSG00000188613 | NANOS1 |
| chr20 | 48962359 | 48963325 | -2,12 | 2,96 | -5,08 | 3,87E-05 | 0,00585 | Distal Intergenic | 53102 | ENSG00000203999 | LINC01270 |
| chr17 | 68356827 | 68357784 | -2,12 | 3,17 | -5,29 | 3,93E-05 | 0,0059 | Distal Intergenic | 191151 | ENSG00000123700 | KCNJ2 |
| chr7 | 76140872 | 76142443 | -1,59 | 3,65 | -5,24 | 3,94E-05 | 0,0059 | Promoter (1-2kb) | 1127 | NA | NA |
| chr4 | 181377860 | 181378780 | -1,48 | 3,51 | -4,99 | 3,95E-05 | 0,00591 | Distal Intergenic | 701522 | ENSG00000248197 | LINC00290 |
| chr3 | 120397000 | 120398139 | -2,12 | 3,38 | -5,5 | 3,97E-05 | 0,00592 | Intron (uc003edw.3/3081, intron 1 of 13) | 3279 | ENSG00000113924 | HGD |
| chr13 | 110356883 | 110358253 | -1,93 | 3,16 | -5,09 | 3,99E-05 | 0,00592 | Distal Intergenic | 80661 | ENSG00000185950 | IRS2 |
| chr12 | 55051074 | 55052457 | -2,12 | 3,18 | -5,3 | 4,00E-05 | 0,00592 | Distal Intergenic | -8925 | ENSG00000161634 | DCD |
| chr1 1 | 78064708 | 78066644 | -1,48 | 4,24 | -5,72 | 4,06E-05 | 0,00598 | Intron (uc001ozh.3/9846, intron 1 of 9) | -11782 | ENSG00000033327 | GAB2 |
| chr5 | 19902043 | 19903095 | -2,12 | 3,4 | -5,52 | 4,06E-05 | 0,00598 | Intron (uc003jgd.3/1016, intron 2 of 12) | -15662 | ENSG00000145526 | CDH18 |
| chr2 | 81637676 | 81638726 | -1,93 | 3,29 | -5,22 | 4,12E-05 | 0,00601 | Distal Intergenic | 821498 | ENSG00000066032 | CTNNA2 |
| chr6 | 37483986 | 37484821 | -1,44 | 3,17 | -4,61 | 4,12E-05 | 0,00601 | Distal Intergenic | -16286 | ENSG00000198937 | CCDC167 |
| chr5 | 164937452 | 164938706 | -1,59 | 3,64 | -5,23 | 4,13E-05 | 0,00601 | Distal Intergenic | -2E+06 | ENSG00000145934 | TENM2 |
| chr10 | 3038925 | 3040203 | -1,39 | 3,32 | -4,71 | 4,15E-05 | 0,00602 | Distal Intergenic | -69509 | ENSG00000067057 | PFKP |
| chr6 | 54601565 | 54602375 | -0,62 | 3,9 | -4,52 | 4,17E-05 | 0,00603 | Distal Intergenic | -109194 | ENSG00000168143 | FAM83B |
| chr2 | 187684180 | 187685608 | -0,64 | 5,04 | -5,68 | 4,21E-05 | 0,00604 | Distal Intergenic | 28289 | ENSG00000163012 | ZSWIM2 |
| chr1 | 22690889 | 22692310 | -2,12 | 3,31 | -5,43 | 4,21E-05 | 0,00604 | Distal Intergenic | -86034 | ENSG00000184677 | ZBTB40 |
| chr1 | 166478619 | 166479493 | -1,15 | 3,25 | -4,4 | 4,22E-05 | 0,00604 | Distal Intergenic | -93660 | ENSG00000215834 | FM09P |
| chr1 1 | 47939252 | 47940610 | -2,12 | 3,15 | -5,27 | 4,24E-05 | 0,00605 | Distal Intergenic | -61500 | ENSG00000149177 | PTPRJ |
| chr10 | 61842367 | 61843464 | -1,29 | 3,4 | -4,68 | 4,24E-05 | 0,00605 | Promoter (<=1kb) | 0 | ENSG00000151150 | ANK3 |
| chr9 | 75567216 | 75568489 | -1,83 | 3,37 | -5,2 | 4,34E-05 | 0,00616 | Promoter (<=1kb) | 0 | ENSG00000165092 | ALDH1A1 |
| chr8 | 117957279 | 117958892 | -2,12 | 3,56 | -5,68 | 4,43E-05 | 0,00624 | Distal Intergenic | -3620 | ENSG00000164756 | SLC30A8 |
| chr3 | 166785007 | 166785689 | -2,12 | 2,88 | -5 | 4,43E-05 | 0,00624 | Distal Intergenic | 312396 | ENSG00000169064 | ZBBX |
| chr18 | 27606870 | 27607863 | -2,12 | 2,73 | -4,85 | 4,60E-05 | 0,00639 | Distal Intergenic | -271013 | ENSG00000283218 | MIR302F |
| chr2 | 21332238 | 21333588 | -2,12 | 3,37 | -5,49 | 4,61E-05 | 0,00639 | Distal Intergenic | -65293 | ENSG00000084674 | APOB |
| chr1 | 239679214 | 239680267 | -2,12 | 3,44 | -5,56 | 4,62E-05 | 0,00639 | Intron (uc001hyo.1/1131, intron 2 of 8) | -112106 | ENSG00000133019 | CHRM3 |
| chr4 | 87373131 | 87374523 | -1,59 | 3,95 | -5,54 | 4,62E-05 | 0,00639 | Promoter (<=1kb) | 0 | ENSG00000109339 | MAPK10 |
| chr4 | 87503112 | 87503834 | -1,77 | 3,12 | -4,89 | 4,68E-05 | 0,00646 | Intron (uc010ikh.1/5602, intron 1 of 6) | 11429 | ENSG00000109339 | MAPK10 |
| chr3 | 165531310 | 165532637 | -2,12 | 3,47 | -5,59 | 4,72E-05 | 0,00647 | Intron (uc003fem.4/590, intron 2 of 3) | 22616 | ENSG00000114200 | BCHE |
| chr2 | 228906312 | 228907043 | -2,12 | 3,1 | -5,22 | 4,74E-05 | 0,00647 | Intron (uc002vpp.2/80309, intron 3 of 10) | 139318 | ENSG00000153820 | SPHKAP |
| chr3 | 165538024 | 165539436 | -2,12 | 3,47 | -5,59 | 4,76E-05 | 0,00648 | Intron (uc003fem.4/590, intron 2 of 3) | 15817 | ENSG00000114200 | BCHE |
| chr4 | 181481206 | 181482407 | -2,12 | 3,32 | -5,43 | 4,86E-05 | 0,00656 | Distal Intergenic | 597895 | ENSG00000248197 | LINC00290 |
| chr12 | 100838894 | 100840122 | -2,12 | 3 | -5,12 | 4,86E-05 | 0,00656 | Distal Intergenic | -27429 | ENSG00000012504 | NR1H4 |
| chr15 | 75741686 | 75749348 | 10,48 | 11,29 | -0,81 | 4,86E-05 | 0,00656 | Promoter (<=1kb) | 0 | ENSG00000169375 | SIN3A |
| chr1 1 | 106030827 | 106031901 | -1,28 | 4,06 | -5,34 | 4,91E-05 | 0,0066 | Distal Intergenic | 69550 | ENSG00000149313 | AASDHPPT |
| chr21 | 22851623 | 22852976 | -2,12 | 3,33 | -5,45 | 5,01E-05 | 0,00672 | Intron (uc002yld.2/4685, intron 15 of 17) | 193801 | NA | RNU6-67P |
| chr9 | 25835576 | 25836503 | -2,12 | 3,13 | -5,25 | 5,04E-05 | 0,00674 | Distal Intergenic | -156720 | ENSG00000198680 | TUSC1 |
| chr9 | 103800583 | 103802123 | -2,12 | 3,48 | -5,6 | 5,05E-05 | 0,00674 | Intron (uc004bbb.3/54886, intron 1 of 7) | 9552 | ENSG00000148123 | PLPPR1 |
| chr8 | 52270306 | 52271613 | -2,12 | 3,42 | -5,54 | 5,06E-05 | 0,00674 | Intron (uc003xqt.4/137902, intron 3 of 4) | 50508 | ENSG00000147485 | PXDNL |
| chr4 | 182491640 | 182492368 | -1,93 | 2,85 | -4,78 | 5,08E-05 | 0,00676 | Distal Intergenic | -411338 | ENSG00000248197 | LINC00290 |
| chr3 | 165534473 | 165535761 | -2,12 | 3,42 | -5,53 | 5,15E-05 | 0,00682 | Intron (uc003fem.4/590, intron 2 of 3) | 19492 | ENSG00000114200 | BCHE |
| chr1 1 | 73172671 | 73173726 | -1,83 | 3,25 | -5,08 | 5,17E-05 | 0,00682 | Intron (uc001oty.1/23201, intron 2 of 7) | 68382 | ENSG00000054967 | RELT |
| chr1 | 191210908 | 191215460 | -0,09 | 5,95 | -6,04 | 5,19E-05 | 0,00682 | Distal Intergenic | 616888 | ENSG00000231175 | LINC0 1720 |
| chrl | 241051628 | 241052534 | -2,12 | 3,25 | -5,36 | 5,24E-05 | 0,00687 | Intron (uc010pyh.2/6000, intron 6 of 17) | -19476 | ENSG00000182901 | RGS7 |
| chr13 | 65453973 | 65455013 | -2,12 | 3,23 | -5,35 | 5,25E-05 | 0,00687 | Distal Intergenic | 1142405 | NA | OR7E156P |
| chr7 | 96988788 | 96989629 | -2,12 | 2,89 | -5,01 | 5,35E-05 | 0,00696 | Distal Intergenic | 242883 | ENSG00000196636 | SDHAF3 |
| chr4 | 150835840 | 150837622 | 1,18 | 6,19 | -5,01 | 5,37E-05 | 0,00696 | Distal Intergenic | -161804 | ENSG00000170390 | DCLK2 |
| chr3 | 110689601 | 110690449 | -0,86 | 4,14 | -5 | 5,53E-05 | 0,00712 | Distal Intergenic | 98357 | NA | NECTIN3 - AS1 |
| chr19 | 13716019 | 13717099 | -1,46 | 3,36 | -4,82 | 5,67E-05 | 0,00727 | Distal Intergenic | -98745 | ENSG00000141837 | CACNA1A |
| chr7 | 28725229 | 28725740 | -2,12 | 2,34 | -4,46 | 5,68E-05 | 0,00727 | Promoter (<=1kb) | 0 | ENSG00000146592 | CREB5 |
| chr6 | 28719136 | 28719802 | -2,12 | 3,06 | -5,18 | 5,92E-05 | 0,00754 | Distal Intergenic | 111652 | ENSG00000225595 | LINC01623 |
| chr12 | 9946629 | 9947746 | -1,39 | 3,28 | -4,67 | 5,95E-05 | 0,00755 | Distal Intergenic | -32331 | ENSG00000150045 | KLRF1 |
| chr5 | 36173167 | 36174917 | -0,83 | 4,77 | -5,59 | 5,99E-05 | 0,00759 | Intron (uc003jkc.2/6502, intron 7 of 9) | 21022 | ENSG00000145604 | SKP2 |
| chr4 | 16409190 | 16410344 | -2,12 | 2,64 | -4,76 | 6,00E-05 | 0,00759 | Distal Intergenic | 180904 | ENSG00000263327 | TAPT1-AS1 |
| chr5 | 19070146 | 19071576 | -2,12 | 3,29 | -5,41 | 6,04E-05 | 0,0076 | Distal Intergenic | 814805 | ENSG00000145526 | CDH18 |
| chr1 1 | 126523084 | 126526247 | -0,01 | 5,08 | -5,09 | 6,06E-05 | 0,00761 | Intron (uc009zcf.1/uc009zcf.1, intron 1 of 1) | 245955 | ENSG00000110080 | ST3 GAL4 |
| chr5 | 94422444 | 94423938 | -1,39 | 3,99 | -5,38 | 6,10E-05 | 0,00765 | Intron (uc003kkx.2/79772, intron 1 of 22) | -4899 | ENSG00000175471 | MCTP1 |
| chr4 | 168269844 | 168271060 | -1,14 | 4,18 | -5,32 | 6,11E-05 | 0,00765 | Distal Intergenic | -114103 | ENSG00000196104 | SPOCK3 |
| chr4 | 180384686 | 180385722 | -2,12 | 2,98 | -5,1 | 6,15E-05 | 0,00767 | Distal Intergenic | 1694580 | ENSG00000248197 | LINC00290 |
| chr5 | 147118210 | 147119319 | -2,12 | 3,4 | -5,52 | 6,16E-05 | 0,00768 | Intron (uc003loq.2/9832, intron 1 of 20) | 43092 | ENSG00000176049 | JAKMIP2 |
| chr7 | 14724725 | 14725494 | -2,12 | 3,07 | -5,19 | 6,19E-05 | 0,00768 | 3'UTR | 155581 | ENSG00000136267 | DGKB |
| chr18 | 68326253 | 68327293 | -2,12 | 2,94 | -5,05 | 6,22E-05 | 0,00768 | Distal Intergenic | 344826 | ENSG00000170677 | SOCS6 |
| chr3 | 23700155 | 23701281 | -0,89 | 3,63 | -4,52 | 6,22E-05 | 0,00768 | Distal Intergenic | -146103 | ENSG00000170142 | UBE2E1 |
| chr10 | 19483604 | 19485198 | -2,12 | 3,23 | -5,35 | 6,40E-05 | 0,00788 | Distal Intergenic | 535291 | ENSG00000165997 | ARL5B |
| chr1 | 241571662 | 241572494 | -0,9 | 3,76 | -4,66 | 6,41E-05 | 0,00788 | Distal Intergenic | -51184 | ENSG00000182901 | RGS7 |
| chr3 | 115342299 | 115343071 | -2,12 | 2,83 | -4,95 | 6,57E-05 | 0,00803 | Promoter (<=1kb) | 148 | ENSG00000172020 | GAP43 |
| chr2 | 104048698 | 104049362 | -2,12 | 2,66 | -4,77 | 6,59E-05 | 0,00803 | Distal Intergenic | 634473 | ENSG00000170417 | TMEM182 |
| chr4 | 86952998 | 86954697 | -2,12 | 3,19 | -5,3 | 6,67E-05 | 0,0081 | Intron (uc003hpn.3/5602, intron 3 of 5) | 65733 | ENSG00000109339 | MAPK10 |
| chr13 | 107222256 | 107223293 | -2,12 | 3,12 | -5,23 | 6,73E-05 | 0,00813 | Promoter (1-2kb) | -1742 | ENSG00000134884 | ARGLU1 |
| chr6 | 89060439 | 89061416 | -2,12 | 2,73 | -4,85 | 6,77E-05 | 0,00815 | Distal Intergenic | -184672 | ENSG00000118432 | CNR1 |
| chr1 1 | 104868085 | 104868697 | -2,12 | 2,9 | -5,02 | 6,80E-05 | 0,00816 | Exon (uc009yxh.2/838, exon 6 of 8) | 25198 | ENSG00000137757 | CASP5 |
| chr5 | 21751773 | 21752252 | -2,12 | 2,73 | -4,85 | 6,83E-05 | 0,00818 | 3'UTR | 292184 | ENSG00000183666 | GUSBP1 |
| chr3 | 165471829 | 165472838 | -2,12 | 2,8 | -4,92 | 6,93E-05 | 0,00827 | Distal Intergenic | 82415 | ENSG00000114200 | BCHE |
| chr4 | 107856033 | 107857155 | -1,86 | 2,82 | -4,67 | 7,07E-05 | 0,0084 | Intron (uc003hyi.3/27123, intron 1 of 3) | 100298 | ENSG00000155011 | DKK2 |
| chr1 | 176282646 | 176283834 | -1,21 | 4,03 | -5,24 | 7,18E-05 | 0,0085 | Distal Intergenic | -106276 | ENSG00000143207 | COP1 |
| chr4 | 167195155 | 167196038 | -1,05 | 3,69 | -4,73 | 7,23E-05 | 0,00852 | Distal Intergenic | 400745 | ENSG00000038295 | TLL1 |
| chr2 | 218474927 | 218476160 | -2,12 | 3,03 | -5,15 | 7,25E-05 | 0,00852 | Intron (uc002vgo.3/729582, intron 1 of 12) | -9375 | NA | DIRC3 |
| chr1 | 17902300 | 17903109 | -1,48 | 3,67 | -5,15 | 7,29E-05 | 0,00854 | Intron (uc031pli.1/55160, intron 1 of 1) | -3939 | ENSG00000074964 | ARHGEF10L |
| chr2 | 193952136 | 193959430 | 0,72 | 7,09 | -6,37 | 7,30E-05 | 0,00854 | Distal Intergenic | 337565 | ENSG00000227418 | PCGEM1 |
| chr20 | 15456317 | 15457125 | -1,86 | 2,76 | -4,62 | 7,30E-05 | 0,00854 | Intron (uc002wot.3/140733, intron 7 of 16) | -223376 | ENSG00000172264 | MACROD2 |
| chr7 | 18329379 | 18331846 | 0,05 | 5,46 | -5,41 | 7,48E-05 | 0,0087 | Promoter (<=1kb) | 0 | ENSG00000048052 | HDAC9 |
| chr5 | 33162096 | 33162809 | -2,12 | 2,51 | -4,63 | 7,51E-05 | 0,0087 | Distal Intergenic | 213047 | ENSG00000248279 | LINC02120 |
| chr10 | 118613639 | 118614518 | -2,12 | 3,13 | -5,25 | 7,61E-05 | 0,00879 | Intron (uc001lcw.3/387712, intron 1 of 6) | -3923 | ENSG00000165868 | HSPA12A |
| chr7 | 125455409 | 125456101 | -1,46 | 3,19 | -4,65 | 7,66E-05 | 0,00883 | Distal Intergenic | -885372 | ENSG00000128513 | POT1 |
| chr2 | 138738378 | 138739297 | -2,12 | 3,14 | -5,26 | 7,70E-05 | 0,00886 | 3'UTR | 16570 | ENSG00000150540 | HNMT |
| chr7 | 54956110 | 54956656 | -1,73 | 2,68 | -4,41 | 7,72E-05 | 0,00886 | Distal Intergenic | -129171 | ENSG00000132432 | SEC61G |
| chr1 | 7515055 | 7516079 | -1,83 | 3,29 | -5,12 | 7,74E-05 | 0,00886 | Intron (uc001aoi.3/23261, intron 5 of 22) | -215975 | ENSG00000171735 | CAMTA1 |
| chr10 | 9271462 | 9272153 | -2,12 | 2,46 | -4,58 | 7,74E-05 | 0,00886 | Distal Intergenic | 1174795 | ENSG00000107485 | GATA3 |
| chr1 1 | 134090849 | 134097277 | 9,69 | 10,51 | -0,82 | 7,76E-05 | 0,00886 | Promoter (<=1kb) | 0 | ENSG00000151502 | VPS26B |
| chr16 | 31434141 | 31434862 | -2,12 | 2,53 | -4,64 | 7,82E-05 | 0,00891 | Exon (uc002ebv.1/3681, exon 24 of 30) | 4887 | ENSG00000156885 | COX6A2 |
| chr7 | 15181459 | 15182907 | -2,12 | 3,19 | -5,31 | 7,96E-05 | 0,00902 | Distal Intergenic | -238909 | ENSG00000136267 | DGKB |
| chr20 | 41527149 | 41528246 | -2,12 | 3,19 | -5,31 | 7,96E-05 | 0,00902 | Intron (uc002xkg.3/11122, intron 1 of 30) | 290311 | ENSG00000196090 | PTPRT |
| chr5 | 20327090 | 20327762 | -2,12 | 2,72 | -4,84 | 8,03E-05 | 0,00906 | Intron (uc003jgc.3/1016, intron 1 of 14) | 248220 | ENSG00000145526 | CDH18 |
| chr5 | 94411632 | 94412653 | -2,12 | 3,2 | -5,31 | 8,06E-05 | 0,00906 | Intron (uc003kkv.2/79772, intron 1 of 22) | 4892 | ENSG00000175471 | MCTP1 |
| chr1 | 168789983 | 168790863 | -2,12 | 2,75 | -4,87 | 8,13E-05 | 0,0091 | Distal Intergenic | 33804 | ENSG00000225826 | LINC00626 |
| chr7 | 80537188 | 80538073 | -1,83 | 3,07 | -4,9 | 8,32E-05 | 0,00927 | Intron (uc003uhj.3/10512, intron 2 of 17) | 10594 | ENSG00000075223 | SEMA3 C |
| chr7 | 53286563 | 53287346 | -2,12 | 3,07 | -5,19 | 8,35E-05 | 0,00929 | Distal Intergenic | 183214 | ENSG00000221900 | POM121L12 |
| chr13 | 71963838 | 71965429 | -0,99 | 3,99 | -4,98 | 8,37E-05 | 0,00929 | Distal Intergenic | 374565 | ENSG00000226846 | LINC00348 |
| chr1 | 189173737 | 189174856 | -2,12 | 3,24 | -5,36 | 8,38E-05 | 0,00929 | Distal Intergenic | 1270086 | ENSG00000162670 | BRINP3 |
| chr18 | 67253444 | 67254892 | -2,12 | 3,12 | -5,23 | 8,61E-05 | 0,00949 | Intron (uc002lkl.3/220164, intron 2 of 7) | 185160 | ENSG00000206052 | DOK6 |
| chr2 | 41562470 | 41563244 | -1,86 | 2,69 | -4,55 | 8,62E-05 | 0,00949 | Distal Intergenic | -541451 | ENSG00000214691 | LINC0 1913 |
| chr8 | 116219228 | 116228721 | 1,13 | 8,2 | -7,07 | 8,63E-05 | 0,00949 | Distal Intergenic | 451513 | ENSG00000104447 | TRPS1 |
| chr17 | 68234756 | 68235711 | -2,12 | 3,11 | -5,23 | 8,66E-05 | 0,00949 | Distal Intergenic | 69080 | ENSG00000123700 | KCNJ2 |
| chr1 | 31359877 | 31360942 | -2,12 | 3,2 | -5,32 | 8,67E-05 | 0,00949 | Intron (uc001bse.2/9672, intron 1 of 4) | -8290 | ENSG00000162512 | SDC3 |
| chr4 | 183098107 | 183098812 | -2,12 | 2,4 | -4,52 | 8,69E-05 | 0,0095 | Intron (uc010irv.1/55714, intron 1 of 3) | 7661 | ENSG00000221227 | MIR1305 |
| chr21 | 47167158 | 47167944 | -2,12 | 2,68 | -4,8 | 8,71E-05 | 0,0095 | Intron (uc010gqb.3/54039, intron 2 of 13) | 88389 | ENSG00000205424 | LOC1001290 27 |
| chr1 1 | 117680874 | 117681782 | -2,12 | 2,53 | -4,65 | 8,84E-05 | 0,00962 | Intron (uc001pri.1/57453, intron 1 of 6) | 6458 | ENSG00000177103 | DSCAML1 |
| chr13 | 57900216 | 57901220 | -2,12 | 3 | -5,12 | 8,85E-05 | 0,00962 | Distal Intergenic | 158885 | ENSG00000234278 | PRR20E |
| chr3 | 36746025 | 36747138 | -2,12 | 3,03 | -5,15 | 8,88E-05 | 0,00963 | Distal Intergenic | 34214 | ENSG00000163673 | DCLK3 |
| chr22 | 35899044 | 35899976 | -1,39 | 3,2 | -4,59 | 8,95E-05 | 0,00964 | Distal Intergenic | -37376 | ENSG00000100302 | RASD2 |
| chr1 1 | 93063257 | 93064324 | -0,36 | 5,2 | -5,56 | 8,96E-05 | 0,00964 | Promoter (<=1kb) | 0 | ENSG00000165325 | DEUP1 |
| chr1 | 186237211 | 186237870 | -2,12 | 2,31 | -4,42 | 8,97E-05 | 0,00964 | Intron (uc021pgf.1/100302192, intron 1 of 1) | -27535 | ENSG00000116690 | PRG4 |
| chr5 | 25454731 | 25455676 | -2,12 | 3,06 | -5,18 | 9,03E-05 | 0,00969 | Distal Intergenic | -614039 | ENSG00000248908 | LINC02239 |
| chr9 | 125145570 | 125146889 | -1,59 | 3,29 | -4,88 | 9,05E-05 | 0,0097 | Exon (uc010mwb.2/5742, exon 8 of 11) | 7969 | ENSG00000095303 | PTGS1 |
| chr1 | 98685454 | 98686181 | -2,12 | 2,72 | -4,83 | 9,13E-05 | 0,00974 | Intron (uc031pnf.1/729987, intron 1 of 2) | 9187 | ENSG00000226053 | LINC01776 |
| chrX | 32384190 | 32385143 | -2,12 | 3,03 | -5,15 | 9,15E-05 | 0,00975 | Promoter (<=1kb) | -865 | ENSG00000198947 | DMD |
| chr7 | 3213267 | 3214409 | -1,57 | 3,52 | -5,09 | 9,18E-05 | 0,00977 | Exon (uc003smw.1/uc003smw.1, exon 1 of 4) | -126671 | ENSG00000146555 | SDK1 |
| chr4 | 160108854 | 160110680 | -1,39 | 3,86 | -5,24 | 9,23E-05 | 0,00979 | Distal Intergenic | -58808 | ENSG00000264105 | MIR3688-1 |
| chr1 | 242539240 | 242539767 | -2,12 | 2,25 | -4,37 | 9,23E-05 | 0,00979 | Intron (uc001hzl.4/200150, intron 1 of 9) | 73017 | ENSG00000180287 | PLD5 |
| chr1 | 238405209 | 238407466 | -0,83 | 4,57 | -5,4 | 9,40E-05 | 0,00993 | Distal Intergenic | 241851 | ENSG00000215808 | LINC01139 |
| chr4 | 136448278 | 136449254 | -2,12 | 2,68 | -4,79 | 9,46E-05 | 0,00998 | Distal Intergenic | -1E+06 | ENSG00000254535 | PABPC4L |
| chr3 | 150089209 | 150089794 | -2,12 | 2,71 | -4,83 | 9,50E-05 | 0,01 | Distal Intergenic | -36994 | ENSG00000196428 | TSC22D2 |
| chrX | 2835739 | 2836666 | -0,8 | 3,29 | -4,09 | 9,56E-05 | 0,01 | Exon (uc004cqv.3/414, exon 5 of 10) | 10750 | ENSG00000006756 | ARSD |
| chrX | 68795187 | 68796291 | -1,77 | 3,12 | -4,89 | 9,57E-05 | 0,01 | Distal Intergenic | -39620 | ENSG00000158813 | EDA |
| chr5 | 18588458 | 18589172 | -2,12 | 3,02 | -5,13 | 9,59E-05 | 0,01 | Distal Intergenic | -1E+06 | ENSG00000250822 | LINC02111 |
| chr21 | 20116968 | 20118006 | -2,12 | 3,08 | -5,19 | 9,64E-05 | 0,01 | Intron (uc002ykx.3/uc002ykx.3, intron 1 of 3) | -340998 | ENSG00000154646 | TMPRSS15 |
| chr6 | 123068024 | 123069253 | -2,12 | 2,78 | -4,89 | 9,68E-05 | 0,0101 | Distal Intergenic | 29111 | ENSG00000135549 | PKIB |
| chr1 1 | 101969299 | 101969812 | -1,93 | 2,23 | -4,16 | 9,74E-05 | 0,0101 | Distal Intergenic | -11380 | ENSG00000137693 | YAP1 |
| chrl 9 | 29119269 | 29119965 | -2,12 | 2,64 | -4,76 | 9,78E-05 | 0,0101 | Intron (uc002nsa.2/uc002nsa.2, intron 5 of 9) | -336073 | ENSG00000267339 | LINC00906 |
| chr4 | 167089647 | 167091010 | -0,9 | 3,79 | -4,7 | 9,82E-05 | 0,0101 | Distal Intergenic | 295237 | ENSG00000038295 | TLL1 |
| chr12 | 30473409 | 30474060 | -2,12 | 2,41 | -4,53 | 9,82E-05 | 0,0101 | Distal Intergenic | 342528 | ENSG00000133704 | IPO8 |
| chr9 | 79167808 | 79168599 | -2,12 | 2,6 | -4,71 | 9,91E-05 | 0,0102 | Distal Intergenic | 52259 | ENSG00000187210 | GCNT1 |
| chr14 | 23729746 | 23730338 | -1,64 | 2,61 | -4,24 | 9,91E-05 | 0,0102 | Distal Intergenic | 24971 | ENSG00000215271 | HOMEZ |
| chr13 | 110067639 | 110068917 | -0,05 | 4,18 | -4,23 | 9,92E-05 | 0,0102 | Distal Intergenic | -247988 | ENSG00000236242 | MYO16-AS1 |
| chr3 | 165336223 | 165337050 | -2,12 | 3,08 | -5,2 | 9,96E-05 | 0,0102 | Distal Intergenic | 218203 | ENSG00000114200 | BCHE |
| chr4 | 15650413 | 15652102 | -2,12 | 2,93 | -5,05 | 1,00E-04 | 0,0103 | Intron (uc003gob.2/26234, intron 1 of 11) | -4133 | ENSG00000118564 | FBXL5 |
| chr3 | 166319684 | 166320681 | -2,12 | 2,76 | -4,88 | 0,000101 | 0,0103 | Distal Intergenic | -764431 | ENSG00000114200 | BCHE |
| chr2 | 165300523 | 165301275 | -1,44 | 2,93 | -4,37 | 0,000101 | 0,0103 | Distal Intergenic | 123742 | ENSG00000115290 | GRB14 |
| chr13 | 106862595 | 106863880 | -1,86 | 3,42 | -5,28 | 0,000102 | 0,0104 | Distal Intergenic | -165031 | NA | LINC00460 |
| chr12 | 29537498 | 29538869 | -0,83 | 4,01 | -4,84 | 0,000102 | 0,0103 | Distal Intergenic | -3355 | ENSG00000087502 | ERGIC2 |
| chr5 | 94413710 | 94418200 | 0,37 | 6,97 | -6,6 | 0,000103 | 0,0104 | Promoter (<=1kb) | 0 | ENSG00000175471 | MCTP1 |
| chr4 | 179663669 | 179664350 | -2,12 | 2,96 | -5,08 | 0,000103 | 0,0104 | Distal Intergenic | 1013758 | ENSG00000231171 | LINC0 1098 |
| chr4 | 116702663 | 116703394 | -2,12 | 2,68 | -4,79 | 0,000103 | 0,0104 | Distal Intergenic | -517487 | ENSG00000284253 | MIR1973 |
| chr5 | 22314116 | 22315309 | -0,99 | 3,68 | -4,67 | 0,000104 | 0,0105 | Intron (uc003jgk.2/1010, intron 3 of 14) | -100207 | ENSG00000154162 | CDH12 |
| chr1 | 4659590 | 4666445 | 0,92 | 7,71 | -6,79 | 0,000106 | 0,0107 | Distal Intergenic | -48660 | ENSG00000196581 | AJAP1 |
| chr15 | 92944026 | 92944805 | -2,12 | 3,03 | -5,15 | 0,000106 | 0,0106 | Intron (uc002bra.3/8128, intron 1 of 5) | 6886 | ENSG00000140557 | ST8SIA2 |
| chr8 | 77712186 | 77712978 | -2,12 | 3,11 | -5,23 | 0,000108 | 0,0108 | Intron (uc003yau.2/79776, intron 4 of 10) | 95908 | ENSG00000091656 | ZFHX4 |
| chr17 | 54450267 | 54451189 | -2,12 | 2,59 | -4,7 | 0,000108 | 0,0108 | Intron (uc002iun.1/162282, intron 6 of 16) | 219431 | ENSG00000153930 | ANKFN1 |
| chr7 | 4455621 | 4456559 | 0,1 | 4,34 | -4,25 | 0,000108 | 0,0108 | Distal Intergenic | 188602 | ENSG00000146555 | SDK1 |
| chr9 | 74253171 | 74253857 | -1,73 | 2,47 | -4,2 | 0,000108 | 0,0108 | Distal Intergenic | 59300 | ENSG00000135048 | CEMIP2 |
| chr6 | 162531426 | 162532727 | -2,12 | 3,14 | -5,26 | 0,000109 | 0,0108 | Intron (uc003qtv.4/5071, intron 2 of 6) | 151070 | ENSG00000185345 | PRKN |
| chr2 | 21553398 | 21554326 | -2,12 | 3,11 | -5,23 | 0,000109 | 0,0108 | Distal Intergenic | -286453 | ENSG00000084674 | APOB |
| chr1 1 | 25347509 | 25348160 | -2,12 | 2,48 | -4,6 | 0,000109 | 0,0108 | Distal Intergenic | 828993 | ENSG00000187398 | LUZP2 |
| chr4 | 102265572 | 102270768 | 9,44 | 10,35 | -0,92 | 0,000109 | 0,0108 | Promoter (<=1kb) | 0 | ENSG00000138814 | PPP3 CA |
| chr7 | 100342174 | 100343283 | -1,12 | 3,93 | -5,05 | 0,00011 | 0,0108 | Intron (uc003uwj.3/7455, intron 7 of 48) | 10925 | ENSG00000146839 | ZAN |
| chr12 | 83584660 | 83585359 | -2,12 | 2,65 | -4,76 | 0,000111 | 0,0109 | Distal Intergenic | 503726 | ENSG00000179104 | TMTC2 |
| chr3 | 125421269 | 125421719 | -2,12 | 2,01 | -4,13 | 0,000111 | 0,0109 | Distal Intergenic | 87676 | ENSG00000221737 | MIR548I1 |
| chr14 | 96729141 | 96730129 | -0,93 | 3,63 | -4,56 | 0,000112 | 0,0109 | 5'UTR | 6594 | ENSG00000100739 | BDKRB1 |
| chr3 | 145997564 | 145998120 | -2,12 | 2,34 | -4,46 | 0,000112 | 0,0109 | Distal Intergenic | -28598 | ENSG00000114698 | PLSCR4 |
| chr7 | 14021816 | 14023260 | -2,12 | 2,95 | -5,06 | 0,000113 | 0,011 | Promoter (1-2kb) | 1885 | ENSG00000006468 | ETV1 |
| chr10 | 66201307 | 66202296 | -2,12 | 2,85 | -4,96 | 0,000113 | 0,011 | Distal Intergenic | -382989 | NA | ANXA2P3 |
| chr6 | 152701328 | 152701977 | -2,12 | 2,67 | -4,79 | 0,000114 | 0,011 | Promoter (<=1kb) | 520 | ENSG00000131018 | SYNE1 |
| chr2 | 42981378 | 42981810 | -2,12 | 2,52 | -4,64 | 0,000114 | 0,011 | 3'UTR | 9591 | ENSG00000162881 | OXER1 |
| chr5 | 18970054 | 18970877 | -2,12 | 2,97 | -5,09 | 0,000115 | 0,011 | Distal Intergenic | 915504 | ENSG00000145526 | CDH18 |
| chr10 | 33540852 | 33541684 | -2,12 | 2,66 | -4,77 | 0,000116 | 0,0111 | Intron (uc001iwv.4/8829, intron 6 of 15) | 82084 | ENSG00000099250 | NRP1 |
| chr6 | 12007032 | 12014319 | 9,96 | 10,83 | -0,87 | 0,000116 | 0,0111 | Promoter (<=1kb) | 0 | ENSG00000095951 | HIVEP1 |
| chr5 | 19270514 | 19271759 | -2,12 | 2,79 | -4,91 | 0,000117 | 0,0111 | Distal Intergenic | 614622 | ENSG00000145526 | CDH18 |
| chr4 | 153512146 | 153513175 | -2,12 | 3,37 | -5,49 | 0,000118 | 0,0112 | Distal Intergenic | 54730 | ENSG00000268471 | MIR4453HG |
| chr2 | 141260533 | 141261230 | -2,12 | 2,64 | -4,76 | 0,000119 | 0,0113 | Exon (uc002tvj.1/53353, exon 54 of 91) | 1605639 | NA | YY1P2 |
| chr9 | 112239353 | 112240468 | -2,12 | 2,98 | -5,1 | 0,00012 | 0,0113 | Intron (uc004bed.2/5774, intron 1 of 25) | -13622 | ENSG00000070159 | PTPN3 |
| chr5 | 20110009 | 20110978 | -2,12 | 2,85 | -4,97 | 0,00012 | 0,0113 | Intron (uc003jgc.3/1016, intron 2 of 14) | -121656 | ENSG00000145526 | CDH18 |
| chr18 | 33820168 | 33821268 | -2,12 | 2,5 | -4,62 | 0,000121 | 0,0113 | Intron (uc002kzq.4/55034, intron 9 of 14) | 52688 | ENSG00000075643 | MOCOS |
| chr1 | 237837965 | 237840206 | -0,82 | 4,41 | -5,23 | 0,000122 | 0,0114 | Promoter (<=1kb) | 0 | ENSG00000198626 | RYR2 |
| chr1 1 | 105569750 | 105570615 | -1,77 | 2,74 | -4,5 | 0,000122 | 0,0114 | Intron (uc001piu.1/2893, intron 3 of 10) | 88339 | ENSG00000152578 | GRIA4 |
| chr15 | 102137674 | 102140979 | 0,74 | 6,2 | -5,46 | 0,000123 | 0,0114 | Distal Intergenic | 49916 | ENSG00000184277 | TM2D3 |
| chr9 | 109876339 | 109877237 | -1,44 | 3,64 | -5,08 | 0,000123 | 0,0114 | Distal Intergenic | -27623 | ENSG00000221331 | MIR548Q |
| chr16 | 19872671 | 19873608 | -0,86 | 3,96 | -4,82 | 0,000123 | 0,0114 | Exon (uc021tef.1/51704, exon 2 of 3) | 10560 | ENSG00000167191 | GPRC5B |
| chr8 | 48947104 | 48948081 | -2,12 | 2,91 | -5,02 | 0,000124 | 0,0114 | Intron (uc003xqm.3/7336, intron 1 of 3) | 26109 | ENSG00000169139 | UBE2V2 |
| chr5 | 5651927 | 5652633 | -2,12 | 2,85 | -4,97 | 0,000124 | 0,0114 | Distal Intergenic | 229141 | ENSG00000164151 | ICE1 |
| chr4 | 181029195 | 181029929 | -2,12 | 2,69 | -4,8 | 0,000124 | 0,0114 | Distal Intergenic | 1050373 | ENSG00000248197 | LINC00290 |
| chr14 | 30118727 | 30119602 | -1,86 | 2,82 | -4,67 | 0,000125 | 0,0114 | Intron (uc021rrv.1/100616347, intron 1 of 1) | 55006 | ENSG00000265226 | MIR548AI |
| chr13 | 106839539 | 106840150 | -2,12 | 2,65 | -4,76 | 0,000126 | 0,0115 | Distal Intergenic | -188761 | NA | LINC00460 |
| chr7 | 115359758 | 115360303 | -2,12 | 2,55 | -4,67 | 0,000126 | 0,0115 | Distal Intergenic | 248064 | ENSG00000105967 | TFEC |
| chr8 | 62748768 | 62749625 | -2,12 | 3,03 | -5,15 | 0,000127 | 0,0115 | Distal Intergenic | 121421 | ENSG00000264408 | MIR4470 |
| chr5 | 20155547 | 20156471 | -1,75 | 2,91 | -4,66 | 0,000127 | 0,0115 | Intron (uc003jgc.3/1016, intron 2 of 14) | -167194 | ENSG00000145526 | CDH18 |
| chr1 1 | 104951539 | 104952248 | -2,12 | 2,66 | -4,78 | 0,000128 | 0,0116 | Distal Intergenic | 19910 | ENSG00000255221 | CARD17 |
| chr9 | 16887952 | 16888669 | -2,12 | 2,47 | -4,59 | 0,000128 | 0,0116 | Distal Intergenic | -17166 | ENSG00000173068 | BNC2 |
| chr10 | 58816194 | 58817245 | -1,68 | 3,3 | -4,98 | 0,000131 | 0,0118 | Distal Intergenic | 247074 | ENSG00000264747 | MIR3924 |
| chr4 | 137023888 | 137025244 | -1,59 | 3,39 | -4,98 | 0,000131 | 0,0118 | Distal Intergenic | 1428385 | ENSG00000189184 | PCDH18 |
| chr9 | 130727158 | 130728453 | -2,12 | 3,32 | -5,44 | 0,000132 | 0,0119 | Intron (uc004bsx.2/399665, intron 1 of 10) | -14165 | ENSG00000167106 | FAM102A |
| chrX | 124345524 | 124347340 | -0,64 | 4,44 | -5,08 | 0,000133 | 0,0119 | Distal Intergenic | -106629 | ENSG00000282815 | TEX13C |
| chr19 | 51248601 | 51249412 | -2,12 | 2,29 | -4,41 | 0,000133 | 0,0119 | Distal Intergenic | 21996 | ENSG00000105472 | CLEC11A |
| chr6 | 163611221 | 163611871 | -1,83 | 2,53 | -4,36 | 0,000134 | 0,012 | Intron (uc003qua.3/135138, intron 5 of 6) | 133634 | ENSG00000281692 | PACRG-AS1 |
| chr17 | 62031148 | 62032036 | -1,22 | 3,52 | -4,75 | 0,000135 | 0,012 | Intron (uc002jds.1/6329, intron 13 of 23) | 18242 | ENSG00000007314 | SCN4A |
| chr7 | 16484222 | 16484855 | -2,12 | 2,43 | -4,55 | 0,000135 | 0,012 | Distal Intergenic | 20627 | ENSG00000171243 | SOSTDC1 |
| chr8 | 112673317 | 112674222 | -2,12 | 2,9 | -5,01 | 0,000137 | 0,0121 | Distal Intergenic | 675738 | ENSG00000164796 | CSMD3 |
| chr6 | 35457817 | 35458320 | -1,64 | 2,82 | -4,46 | 0,000137 | 0,0121 | Intron (uc003oku.4/7005, intron 1 of 12) | 6541 | ENSG00000007866 | TEAD3 |
| chr4 | 111116168 | 111120981 | 9,27 | 10,18 | -0,92 | 0,000137 | 0,0122 | Promoter (<=1kb) | 0 | ENSG00000170522 | ELOVL6 |
| chr2 | 11261837 | 11262787 | -2,12 | 2,98 | -5,1 | 0,000138 | 0,0122 | Intron (uc021vdv.1/285150, intron 5 of 8) | 9515 | ENSG00000145063 | FLJ33534 |
| chr6 | 138225645 | 138226587 | -2,12 | 3,08 | -5,19 | 0,000139 | 0,0123 | Distal Intergenic | 33295 | ENSG00000118503 | TNFAIP3 |
| chr14 | 104902331 | 104903064 | -1,21 | 3,44 | -4,65 | 0,000139 | 0,0122 | Distal Intergenic | -142992 | ENSG00000184601 | C14orf180 |
| chr8 | 123982003 | 123983036 | -2,12 | 2,95 | -5,07 | 0,00014 | 0,0123 | Intron (uc003ypk.1/22882, intron 3 of 3) | 71627 | ENSG00000136986 | DERL1 |
| chr1 1 | 57078402 | 57079435 | -0,5 | 4,52 | -5,01 | 0,000141 | 0,0123 | Intron (uc001njr.3/85456, intron 4 of 10) | -8043 | ENSG00000149115 | TNKS1BP1 |
| chr4 | 179241675 | 179242781 | -1,75 | 3,02 | -4,77 | 0,000141 | 0,0123 | Distal Intergenic | 591764 | ENSG00000231171 | LINC01098 |
| chr5 | 27273969 | 27274700 | -2,12 | 2,88 | -5 | 0,000142 | 0,0124 | Distal Intergenic | -197699 | ENSG00000250337 | PURPL |
| chr1 | 237195014 | 237195784 | -2,12 | 2,6 | -4,72 | 0,000142 | 0,0124 | Distal Intergenic | -9918 | ENSG00000198626 | RYR2 |
| chr6 | 72565300 | 72565895 | -2,12 | 2,37 | -4,48 | 0,000143 | 0,0124 | Distal Intergenic | -30511 | ENSG00000079841 | RIMS1 |
| chr19 | 50435549 | 50436240 | -2,12 | 2,93 | -5,05 | 0,000144 | 0,0124 | Promoter (<=1kb) | -81 | ENSG00000283842 | MIR4751 |
| chr10 | 70821222 | 70822021 | -2,12 | 2,61 | -4,72 | 0,000144 | 0,0124 | Distal Intergenic | -25807 | ENSG00000122862 | SRGN |
| chr2 | 136995961 | 136996659 | -2,12 | 2,5 | -4,62 | 0,000144 | 0,0124 | Distal Intergenic | -120236 | ENSG00000121966 | CXCR4 |
| chr4 | 101453824 | 101454410 | -2,12 | 2,28 | -4,4 | 0,000144 | 0,0124 | Distal Intergenic | -14574 | ENSG00000164035 | EMCN |
| chr8 | 116999549 | 117000898 | -0,63 | 4,31 | -4,94 | 0,000145 | 0,0125 | Intron (uc031tcc.1/100859921, intron 7 of 13) | -318321 | ENSG00000104447 | TRPS1 |
| chr8 | 79770706 | 79772362 | -0,25 | 4,77 | -5,02 | 0,000147 | 0,0126 | Distal Intergenic | -52948 | ENSG00000104432 | IL7 |
| chr9 | 18718181 | 18718898 | -2,12 | 2,37 | -4,48 | 0,000147 | 0,0126 | Intron (uc003zne.4/92949, intron 14 of 28) | -107238 | ENSG00000178031 | ADAMTSL1 |
| chr15 | 75869258 | 75872526 | 9,08 | 9,86 | -0,78 | 0,000147 | 0,0126 | Promoter (<=1kb) | 0 | ENSG00000169410 | PTPN9 |
| chr7 | 46296157 | 46297239 | -2,12 | 2,65 | -4,76 | 0,000148 | 0,0127 | Distal Intergenic | -335286 | ENSG00000146674 | IGFBP3 |
| chr5 | 61106838 | 61107499 | -2,12 | 2,45 | -4,57 | 0,000149 | 0,0127 | Distal Intergenic | 173202 | ENSG00000178722 | C5orf64 |
| chr3 | 114172269 | 114173898 | -0,23 | 4,61 | -4,84 | 0,00015 | 0,0127 | Intron (uc003ebj.3/26137, intron 2 of 5) | -69780 | ENSG00000181722 | ZBTB20 |
| chr2 | 231440040 | 231440859 | -2,12 | 2,72 | -4,83 | 0,00015 | 0,0127 | Distal Intergenic | 71139 | ENSG00000067066 | SP100 |
| chr5 | 19796302 | 19797133 | -2,12 | 2,65 | -4,77 | 0,00015 | 0,0127 | Intron (uc021xwu.1/1016, intron 3 of 12) | 89248 | ENSG00000145526 | CDH18 |
| chr5 | 607094 | 607885 | -1,56 | 2,91 | -4,47 | 0,00015 | 0,0127 | Intron (uc031sin.1/100996325, intron 2 of 2) | 4440 | NA | LOC1009963 25 |
| chr6 | 131322049 | 131322903 | -1,73 | 2,99 | -4,72 | 0,000151 | 0,0127 | Promoter (<=1kb) | -142 | ENSG00000079819 | EPB41L2 |
| chr5 | 21405526 | 21406087 | -2,12 | 2,18 | -4,3 | 0,000151 | 0,0127 | Exon (uc011cnn.1/728411, exon 2 of 9) | -53502 | ENSG00000183666 | GUSBP1 |
| chr2 | 102281798 | 102282621 | -2,12 | 2,68 | -4,8 | 0,000153 | 0,0128 | Distal Intergenic | -31544 | ENSG00000071054 | MAP4K4 |
| chr3 | 165343477 | 165344166 | -2,12 | 3,02 | -5,14 | 0,000156 | 0,0131 | Distal Intergenic | 211087 | ENSG00000114200 | BCHE |
| chr1 | 213089739 | 213090504 | -2,12 | 2,74 | -4,86 | 0,000157 | 0,0131 | Distal Intergenic | -33383 | ENSG00000143494 | VASH2 |
| chr2 | 155562702 | 155563489 | -2,12 | 2,37 | -4,48 | 0,000157 | 0,0131 | Intron (uc021vrh.1/3760, intron 1 of 1) | 7609 | ENSG00000162989 | KCNJ3 |
| chrX | 82899297 | 82900067 | -2,12 | 2,54 | -4,65 | 0,000158 | 0,0132 | Distal Intergenic | 136028 | ENSG00000196767 | POU3F4 |
| chr8 | 13960579 | 13961358 | -1,19 | 3,17 | -4,36 | 0,000158 | 0,0131 | Intron (uc010lss.3/137868, intron 4 of 5) | 451077 | ENSG00000185053 | SGCZ |
| chr22 | 41797748 | 41798636 | -2,12 | 2,79 | -4,91 | 0,000159 | 0,0132 | Distal Intergenic | 19815 | ENSG00000167074 | TEF |
| chr12 | 48133349 | 48135791 | 3,25 | 7,29 | -4,04 | 0,000159 | 0,0132 | Promoter (<=1kb) | 284 | ENSG00000079337 | RAPGEF3 |
| chr2 | 197583641 | 197584467 | -1,59 | 3 | -4,59 | 0,00016 | 0,0133 | Promoter (<=1kb) | 0 | ENSG00000144395 | CCDC150 |
| chr17 | 75730713 | 75731541 | -2,12 | 2,39 | -4,51 | 0,00016 | 0,0133 | Distal Intergenic | 148628 | ENSG00000204283 | LINC01973 |
| chr16 | 19469939 | 19470463 | -2,12 | 2,36 | -4,48 | 0,00016 | 0,0132 | Promoter (2-3kb) | 2133 | ENSG00000103534 | TMC5 |
| chr6 | 164329392 | 164330238 | -2,12 | 2,34 | -4,46 | 0,000162 | 0,0134 | Distal Intergenic | 493717 | ENSG00000112531 | QKI |
| chr2 | 163568877 | 163569882 | -2,12 | 2,66 | -4,78 | 0,000163 | 0,0134 | Intron (uc002uch.2/90134, intron 2 of 15) | 125375 | ENSG00000184611 | KCNH7 |
| chr6 | 122370065 | 122370747 | -2,12 | 2,46 | -4,58 | 0,000163 | 0,0134 | Distal Intergenic | -349949 | ENSG00000025156 | HSF2 |
| chr3 | 165384162 | 165386292 | -0,67 | 4,36 | -5,03 | 0,000164 | 0,0134 | Distal Intergenic | 168961 | ENSG00000114200 | BCHE |
| chr6 | 121725736 | 121726580 | -2,12 | 2,44 | -4,56 | 0,000164 | 0,0134 | Distal Intergenic | -30165 | ENSG00000152661 | GJA1 |
| chr5 | 39740057 | 39740973 | -2,12 | 2,6 | -4,71 | 0,000168 | 0,0137 | Distal Intergenic | -314722 | ENSG00000153071 | DAB2 |
| chr9 | 78912002 | 78913282 | -1,03 | 3,58 | -4,61 | 0,000168 | 0,0137 | Intron (uc004akc.2/5125, intron 27 of 36) | 96162 | ENSG00000135002 | RFK |
| chr3 | 114619308 | 114620029 | -1,86 | 2,65 | -4,51 | 0,000168 | 0,0137 | Intron (uc003ebm.3/26137, intron 2 of 11) | -141184 | ENSG00000181722 | ZBTB20 |
| chr2 | 79396443 | 79397416 | -0,99 | 3,37 | -4,35 | 0,000168 | 0,0137 | Distal Intergenic | -9563 | ENSG00000172016 | REG3A |
| chr18 | 20109399 | 20110573 | -2,12 | 2,76 | -4,87 | 0,000169 | 0,0137 | Distal Intergenic | -111521 | ENSG00000212710 | CTAGE1 |
| chr9 | 84416064 | 84416669 | -2,12 | 2,56 | -4,68 | 0,00017 | 0,0137 | Distal Intergenic | -111683 | ENSG00000240632 | SPATA31D5P |
| chr16 | 73752790 | 73753345 | -2,12 | 2,38 | -4,5 | 0,00017 | 0,0137 | Distal Intergenic | 332086 | ENSG00000258779 | LINC01568 |
| chrl | 95762769 | 95764137 | -1,32 | 2,74 | -4,07 | 0,000171 | 0,0138 | Distal Intergenic | 63058 | ENSG00000122481 | RWDD3 |
| chr4 | 133268330 | 133268918 | -2,12 | 2,84 | -4,96 | 0,000172 | 0,0138 | Distal Intergenic | -801552 | ENSG00000138650 | PCDH1 0 |
| chr8 | 85160227 | 85161208 | -2,12 | 2,81 | -4,93 | 0,000174 | 0,014 | Intron (uc003ycq.4/138046,intron 1 of 9) | 63127 | ENSG00000184672 | RALYL |
| chr8 | 61812069 | 61812871 | -2,12 | 2,3 | -4,42 | 0,000174 | 0,014 | Distal Intergenic | 67436 | NA | LOC1001302 98 |
| chr2 | 6221296 | 6221972 | -1,24 | 2,94 | -4,18 | 0,000174 | 0,014 | Distal Intergenic | 99186 | NA | LOC400940 |
| chr5 | 147465446 | 147466380 | -2,12 | 2,79 | -4,91 | 0,000175 | 0,014 | Exon (uc010jgq.1/11005, exon 5 of 12) | 21911 | ENSG00000133710 | SPINK5 |
| chr1 | 216275777 | 216276427 | -2,12 | 2,75 | -4,87 | 0,000175 | 0,014 | Intron (uc001hku.1/7399, intron 21 of 71) | 320311 | ENSG00000042781 | USH2A |
| chr10 | 15311946 | 15312828 | -2,12 | 2,32 | -4,44 | 0,000175 | 0,014 | Intron (uc001iob.3/221061, intron 3 of 7) | 100230 | ENSG00000 148468 | FAM171A1 |
| chr10 | 1704872 | 1705274 | -1,64 | 2,06 | -3,7 | 0,000175 | 0,014 | Intron (uc009xhq.3/105, intron 1 of 9) | 74396 | ENSG00000185736 | ADARB2 |
| chr13 | 58368856 | 58369518 | -2,12 | 2,49 | -4,61 | 0,000176 | 0,0141 | Distal Intergenic | 128069 | ENSG00000 118946 | PCDH17 |
| chr1 | 219653361 | 219653998 | -2,12 | 2,23 | -4,35 | 0,000177 | 0,0141 | Distal Intergenic | 306169 | ENSG00000143353 | LYPLAL1 |
| chr5 | 164552165 | 164552860 | -2,12 | 2,55 | -4,66 | 0,000178 | 0,0141 | Distal Intergenic | 1607702 | ENSG00000038274 | MAT2B |
| chr7 | 123454447 | 123455012 | -2,12 | 2,64 | -4,76 | 0,000179 | 0,0141 | Promoter (<=1kb) | 254 | NA | HYAL6P |
| chr21 | 23998991 | 23999612 | -1,77 | 2,48 | -4,25 | 0,00018 | 0,0142 | Distal Intergenic | 528055 | ENSG00000 184856 | LINC00308 |
| chr12 | 37935086 | 37936092 | -2,12 | 2,39 | -4,51 | 0,000181 | 0,0142 | Distal Intergenic | -774465 | ENSG00000175548 | ALG10B |
| chr10 | 345496 | 348062 | 0,13 | 5,39 | -5,25 | 0,000182 | 0,0143 | Intron (uc001ifp.3/22982, intron 32 of 36) | 67845 | ENSG00000 151240 | DIP2C |
| chr13 | 57827490 | 57829495 | -0,39 | 4,13 | -4,52 | 0,000182 | 0,0143 | Distal Intergenic | 86159 | ENSG00000234278 | PRR20E |
| chr22 | 28111960 | 28115780 | 1,02 | 6,32 | -5,29 | 0,000183 | 0,0143 | Distal Intergenic | 72091 | ENSG00000169184 | MN1 |
| chr15 | 94013083 | 94013818 | -2,12 | 2,72 | -4,84 | 0,000183 | 0,0143 | Intron (uc002bsu.1/uc002bsu.1, intron 2 of 3) | -380640 | ENSG00000182175 | RGMA |
| chr9 | 84363613 | 84364427 | -2,12 | 2,73 | -4,85 | 0,000184 | 0,0143 | Intron (uc004amd.3/uc004amd.3, intron 2 of 3) | -60017 | ENSG00000196781 | TLE1 |
| chr7 | 83773771 | 83774487 | -2,12 | 2,45 | -4,57 | 0,000184 | 0,0143 | Intron (uc003uhz.3/10371, intron 1 of 16) | 49730 | ENSG00000075213 | SEMA3 A |
| chr5 | 116838514 | 116839208 | -2,12 | 2,37 | -4,49 | 0,000184 | 0,0143 | Intron (uc003kry.3/728342, intron 3 of 5) | 87306 | NA | LINC00992 |
| chr2 | 163279526 | 163280389 | -0,92 | 3,5 | -4,41 | 0,000186 | 0,0144 | 3'UTR | 78943 | ENSG00000115271 | GCA |
| chr1 | 238854613 | 238855322 | -2,12 | 2,52 | -4,63 | 0,000187 | 0,0145 | Distal Intergenic | -205296 | ENSG00000215808 | LINC01139 |
| chr4 | 179207774 | 179208370 | -2,12 | 2,76 | -4,88 | 0,000188 | 0,0145 | Distal Intergenic | 557863 | ENSG00000231171 | LINCO 1098 |
| chr10 | 127386273 | 127386947 | -2,12 | 2,59 | -4,71 | 0,000188 | 0,0145 | Intron (uc0011i1.3/283038, intron 2 of 4) | 14461 | ENSG00000228021 | LOC283038 |
| chr2 | 192756904 | 192757529 | -2,12 | 2,58 | -4,7 | 0,000188 | 0,0145 | Distal Intergenic | -44898 | ENSG00000168497 | CAVIN2 |
| chr8 | 116676433 | 116682248 | 8,53 | 9,78 | -1,25 | 0,000188 | 0,0145 | Promoter (<=1kb) | 0 | ENSG00000104447 | TRPS1 |
| chr5 | 5605230 | 5605628 | -2,12 | 1,96 | -4,07 | 0,000189 | 0,0146 | Distal Intergenic | 182444 | ENSG00000 164151 | ICE1 |
| chr15 | 72408681 | 72411967 | 9,1 | 9,88 | -0,79 | 0,000189 | 0,0146 | Promoter (<=1kb) | 0 | ENSG00000166192 | SENP8 |
| chr1 | 190818508 | 190819471 | -1,91 | 2,87 | -4,78 | 0,000191 | 0,0146 | Distal Intergenic | 224488 | ENSG00000231175 | LINC0 1720 |
| chr3 | 112593257 | 112593818 | -2,12 | 2,35 | -4,47 | 0,000191 | 0,0146 | Distal Intergenic | -28460 | ENSG00000206531 | CD200R1L |
| chr2 | 184894603 | 184895271 | -2,12 | 2,75 | -4,87 | 0,000192 | 0,0146 | Distal Intergenic | -567822 | ENSG00000170396 | ZNF804A |
| chr2 | 79102422 | 79103840 | 0,13 | 4,81 | -4,68 | 0,000192 | 0,0146 | Distal Intergenic | -148972 | ENSG00000143954 | REG3G |
| chr10 | 94958445 | 94959397 | -0,64 | 3,05 | -3,69 | 0,000192 | 0,0146 | Distal Intergenic | 124798 | ENSG00000095596 | CYP26A1 |
| chr3 | 66104414 | 66105150 | -2,12 | 2,5 | -4,62 | 0,000193 | 0,0147 | Distal Intergenic | -14135 | ENSG00000144741 | SLC25A26 |
| chr15 | 34948833 | 34949285 | -2,12 | 2,23 | -4,35 | 0,000193 | 0,0146 | Distal Intergenic | 97497 | ENSG00000159248 | GJD2 |
| chr5 | 18800326 | 18801082 | -2,12 | 2,53 | -4,65 | 0,000194 | 0,0147 | Distal Intergenic | 1085299 | ENSG00000 145526 | CDH18 |
| chr9 | 128788415 | 128788913 | -2,12 | 1,98 | -4,1 | 0,000194 | 0,0147 | Distal Intergenic | 110450 | ENSG00000167081 | PBX3 |
| chr2 | 12516102 | 12516720 | -2,12 | 2,29 | -4,41 | 0,000195 | 0,0148 | Intron (uc002rbu.1/uc002rbu.1, intron 4 of 5) | -340278 | ENSG00000071575 | TRIB2 |
| chr3 | 31255433 | 31256280 | -0,48 | 3,46 | -3,94 | 0,000196 | 0,0148 | Distal Intergenic | -318211 | ENSG00000163527 | STT3B |
| chr8 | 79651168 | 79652274 | -2,12 | 2,98 | -5,1 | 0,000197 | 0,0148 | Exon (uc003ybg.3/3574, exon 3 of 6) | -14559 | NA | LOC1012419 02 |
| chr9 | 25489756 | 25490882 | -0,77 | 3,79 | -4,56 | 0,000198 | 0,0149 | Distal Intergenic | 187974 | ENSG00000 198680 | TUSC1 |
| chr3 | 122043621 | 122044300 | -2,12 | 2,61 | -4,73 | 0,000199 | 0,0149 | Promoter (<=1kb) | 0 | ENSG00000121552 | CSTA |
| chr20 | 55403287 | 55403989 | -2,12 | 2,23 | -4,34 | 0,000199 | 0,0149 | Distal Intergenic | 197349 | ENSG00000087510 | TFAP2C |
| chr1 1 | 26134770 | 26135985 | -2,12 | 2,7 | -4,81 | 2,00E-04 | 0,015 | Distal Intergenic | -74844 | ENSG00000134343 | ANO3 |
| chr7 | 86122369 | 86122947 | -2,12 | 2,38 | -4,5 | 2,00E-04 | 0,015 | Distal Intergenic | -150283 | ENSG00000198822 | GRM3 |
| chr7 | 124698971 | 124699945 | -2,12 | 2,97 | -5,09 | 0,000201 | 0,015 | Intron (uc003vlp.3/uc003vlp.3, intron 3 of 7) | -128934 | ENSG00000 128513 | POT1 |
| chr7 | 115347390 | 115348297 | -0,9 | 3,56 | -4,46 | 0,000203 | 0,0151 | Distal Intergenic | 260070 | ENSG00000105967 | TFEC |
| chr6 | 153455488 | 153456365 | -1,77 | 2,48 | -4,24 | 0,000203 | 0,0151 | Distal Intergenic | -3099 | ENSG00000091844 | RGS17 |
| chr15 | 77710657 | 77715216 | 9,35 | 10,17 | -0,81 | 0,000203 | 0,0151 | Promoter (<=1kb) | 0 | ENSG00000173517 | PEAK1 |
| chr1 | 247534820 | 247535236 | -2,12 | 2,07 | -4,18 | 0,000204 | 0,0152 | Distal Intergenic | -39775 | ENSG00000162714 | ZNF496 |
| chr2 | 220796148 | 220797570 | -0,49 | 3,82 | -4,31 | 0,000206 | 0,0153 | Distal Intergenic | -24862 | ENSG00000266518 | MIR4268 |
| chr8 | 97461143 | 97461677 | -1,42 | 2,58 | -4,01 | 0,000207 | 0,0153 | Distal Intergenic | -44205 | ENSG00000169439 | SDC2 |
| chr2 | 128421691 | 128422250 | -2,12 | 2,36 | -4,48 | 0,00021 | 0,0156 | Promoter (<=1kb) | 0 | ENSG00000072163 | LIMS2 |
| chr3 | 165536084 | 165537032 | -2,12 | 2,71 | -4,83 | 0,000211 | 0,0156 | Intron (uc003fem.4/590, intron 2 of 3) | 18221 | ENSG00000 114200 | BCHE |
| chr6 | 169277780 | 169278749 | -2,12 | 2,53 | -4,64 | 0,000212 | 0,0156 | Distal Intergenic | 259858 | ENSG00000 112562 | SMOC2 |
| chr3 | 11179305 | 11179841 | -2,12 | 2,45 | -4,56 | 0,000213 | 0,0157 | Promoter (<=1kb) | 526 | ENSG00000196639 | HRH1 |
| chr7 | 142428644 | 142429198 | -0,69 | 3,23 | -3,91 | 0,000213 | 0,0157 | 5'UTR | -28121 | ENSG00000204983 | PRSS1 |
| chr12 | 74635496 | 74636172 | -2,12 | 2,68 | -4,8 | 0,000214 | 0,0157 | Intron (uc009zrx.3/100507377, intron 3 of 6) | 50239 | ENSG00000251138 | LOC1005073 77 |
| chr2 | 194770090 | 194770964 | -2,12 | 2,32 | -4,44 | 0,000215 | 0,0158 | Distal Intergenic | 1155519 | ENSG00000227418 | PCGEM1 |
| chr5 | 14075489 | 14076991 | -1,12 | 3,58 | -4,7 | 0,000217 | 0,0158 | Distal Intergenic | -66838 | ENSG00000038382 | TRIO |
| chr7 | 15481053 | 15481840 | -2,12 | 2,54 | -4,66 | 0,000217 | 0,0158 | Intron (uc003stb.1/392636, intron 3 of 12) | 119800 | ENSG00000187546 | AGMO |
| chr21 | 27637653 | 27638536 | -1,39 | 3,03 | -4,41 | 0,000217 | 0,0158 | Distal Intergenic | -94207 | ENSG00000142192 | APP |
| chr7 | 112739883 | 112740280 | -2,12 | 2,12 | -4,24 | 0,000217 | 0,0158 | Distal Intergenic | -12050 | ENSG00000 164604 | GPR85 |
| chr5 | 130823968 | 130824858 | -2,12 | 2,26 | -4,38 | 0,000218 | 0,0159 | Intron (uc003kvn.2/51735, intron 15 of 27) | 19042 | ENSG00000158987 | RAPGEF6 |
| chr8 | 20109917 | 20111068 | -2,12 | 2,55 | -4,66 | 0,000219 | 0,0159 | Promoter (1-2kb) | 1735 | ENSG00000061337 | LZTS1 |
| chr2 | 189386420 | 189387201 | -2,12 | 2,63 | -4,75 | 0,000221 | 0,016 | Intron (uc002uqc.4/51454, intron 4 of 6) | -47554 | ENSG00000144366 | GULP1 |
| chr9 | 136152952 | 136154397 | -1,01 | 3,89 | -4,9 | 0,000222 | 0,016 | Promoter (2-3kb) | -2322 | ENSG00000175164 | ABO |
| chr5 | 27274748 | 27275578 | -2,12 | 2,66 | -4,78 | 0,000222 | 0,016 | Distal Intergenic | -196821 | ENSG00000250337 | PURPL |
| chr3 | 13191218 | 13191676 | -1,77 | 2,12 | -3,89 | 0,000222 | 0,016 | Distal Intergenic | -76601 | ENSG00000144711 | IQSEC1 |
| chr6 | 15244547 | 15250480 | 9,98 | 10,79 | -0,82 | 0,000222 | 0,016 | Promoter (<=1kb) | 0 | ENSG00000008083 | JARID2 |
| chr19 | 47814337 | 47814756 | -2,12 | 2,41 | -4,53 | 0,000223 | 0,016 | Promoter (1-2kb) | 1233 | ENSG00000197405 | C5AR1 |
| chr1 | 178991033 | 178991590 | -2,12 | 2,34 | -4,46 | 0,000223 | 0,016 | Distal Intergenic | -3484 | ENSG00000116199 | FAM20B |
| chr21 | 38747837 | 38749046 | -0,99 | 3,49 | -4,48 | 0,000224 | 0,016 | Intron (uc002vwg.1/1859, intron 1 of 2) | 7978 | ENSG00000157540 | DYRK1A |
| chr17 | 4690111 | 4691151 | -2,12 | 2,51 | -4,63 | 0,000225 | 0,0161 | Promoter (<=1kb) | -382 | ENSG00000182853 | VMO1 |
| chr12 | 129429594 | 129430131 | -2,12 | 1,97 | -4,09 | 0,000226 | 0,0161 | Exon (uc001uhx.1/144423, exon 5 of 8) | 80913 | ENSG00000 151948 | GLT1D1 |
| chr4 | 105830037 | 105830718 | -2,12 | 2,68 | -4,8 | 0,000227 | 0,0161 | Distal Intergenic | -236314 | ENSG00000168769 | TET2 |
| chr16 | 19221990 | 19223024 | -1,62 | 3,06 | -4,68 | 0,000227 | 0,0162 | Intron (uc002dfw.3/51760, intron 5 of 7) | 38311 | ENSG00000103528 | SYT17 |
| chr15 | 64207562 | 64208786 | -2,12 | 2,74 | -4,86 | 0,000228 | 0,0162 | Intron (uc002amr.3/23604, intron 9 of 11) | -81415 | ENSG00000103657 | HERC1 |
| chr8 | 112316970 | 112318005 | 0,7 | 4,83 | -4,13 | 0,000228 | 0,0162 | Distal Intergenic | 1031955 | ENSG00000164796 | CSMD3 |
| chr21 | 22468840 | 22469540 | -2,12 | 2,72 | -4,84 | 0,00023 | 0,0162 | Intron (uc002yld.2/4685, intron 1 of 17) | -49898 | ENSG00000154654 | NCAM2 |
| chr6 | 120191340 | 120192141 | -2,12 | 2,42 | -4,53 | 0,00023 | 0,0162 | Distal Intergenic | -378873 | NA | LOC285762 |
| chr4 | 149351194 | 149352145 | -2,12 | 2,62 | -4,74 | 0,000231 | 0,0162 | Intron (uc003ilj.4/4306, intron 2 of 8) | 11527 | ENSG00000 151623 | NR3C2 |
| chr5 | 20535199 | 20536035 | -2,12 | 2,58 | -4,7 | 0,000231 | 0,0162 | Intron (uc003jgc.3/1016, intron 1 of 14) | 39947 | ENSG00000 145526 | CDH18 |
| chr16 | 18107482 | 18108544 | -1,83 | 2,76 | -4,6 | 0,000232 | 0,0162 | Distal Intergenic | 387584 | ENSG00000266291 | MIR3180-3 |
| chr10 | 4586544 | 4587769 | -2,12 | 2,56 | -4,68 | 0,000233 | 0,0162 | Distal Intergenic | -110579 | NA | LINC00705 |
| chr2 | 224418543 | 224419581 | -2,12 | 2,57 | -4,69 | 0,000234 | 0,0163 | Distal Intergenic | 47636 | ENSG00000 171951 | SCG2 |
| chr21 | 20124816 | 20125916 | -2,12 | 2,74 | -4,86 | 0,000235 | 0,0163 | Intron (uc002ykx.3/uc002ykx.3, intron 1 of 3) | -348846 | ENSG00000 154646 | TMPRSS15 |
| chr4 | 176056968 | 176057836 | -2,12 | 2,63 | -4,75 | 0,000235 | 0,0163 | Distal Intergenic | 217459 | ENSG00000 168594 | ADAM2 9 |
| chr21 | 20307131 | 20308022 | -2,12 | 2,51 | -4,62 | 0,000235 | 0,0163 | Distal Intergenic | -531161 | ENSG00000 154646 | TMPRSS15 |
| chr22 | 47588582 | 47589054 | -2,12 | 2,55 | -4,67 | 0,000236 | 0,0163 | Distal Intergenic | 418758 | ENSG00000054611 | TBC1D22A |
| chr9 | 127597201 | 127597929 | -2,12 | 2,55 | -4,67 | 0,000236 | 0,0163 | Distal Intergenic | -17767 | ENSG00000136918 | WDR3 8 |
| chr7 | 91084503 | 91086077 | 0,32 | 4,79 | -4,47 | 0,000236 | 0,0163 | Distal Intergenic | 190720 | ENSG00000157240 | FZD1 |
| chr12 | 10049538 | 10050394 | -2,12 | 2,6 | -4,72 | 0,000239 | 0,0165 | Downstream (<1kb) | 15450 | ENSG00000256797 | KLRF2 |
| chr20 | 36190476 | 36191159 | -1,83 | 2,51 | -4,34 | 0,000239 | 0,0165 | Distal Intergenic | -34143 | ENSG00000166619 | BLCAP |
| chr10 | 95059285 | 95060220 | -2,12 | 2,52 | -4,64 | 0,00024 | 0,0165 | Distal Intergenic | 80933 | ENSG00000138119 | MYOF |
| chr7 | 86176202 | 86177138 | -0,53 | 3,65 | -4,18 | 0,00024 | 0,0165 | Distal Intergenic | -96092 | ENSG00000198822 | GRM3 |
| chr2 | 139458213 | 139458907 | -1,59 | 2,74 | -4,34 | 0,000241 | 0,0166 | Intron (uc002tvi.3/11249, intron 1 of 1) | 78904 | ENSG00000144227 | NXPH2 |
| chr5 | 164627969 | 164629365 | -2,12 | 2,98 | -5,1 | 0,000243 | 0,0167 | Distal Intergenic | 1683506 | ENSG00000038274 | MAT2B |
| chr4 | 182624331 | 182625092 | -2,12 | 2,5 | -4,62 | 0,000245 | 0,0167 | Distal Intergenic | -440048 | ENSG00000218336 | TENM3 |
| chr8 | 57995320 | 57996478 | -0,54 | 4,05 | -4,59 | 0,000245 | 0,0167 | Distal Intergenic | -88890 | ENSG00000 104331 | IMPAD1 |
| chr2 | 102872636 | 102873234 | -2,12 | 1,97 | -4,09 | 0,000245 | 0,0167 | Distal Intergenic | -54728 | ENSG00000 115604 | IL18R1 |
| chr5 | 19320730 | 19321842 | -2,12 | 2,53 | -4,65 | 0,000247 | 0,0168 | Distal Intergenic | 564539 | ENSG00000 145526 | CDH18 |
| chr7 | 81022398 | 81023022 | -2,12 | 2,48 | -4,59 | 0,000248 | 0,0168 | Distal Intergenic | 376430 | ENSG00000019991 | HGF |
| chr6 | 20400664 | 20406203 | 9,8 | 10,63 | -0,83 | 0,000248 | 0,0168 | Promoter (<=1kb) | 0 | ENSG00000 112242 | E2F3 |
| chr9 | 18649578 | 18650663 | -1,64 | 2,71 | -4,34 | 0,000249 | 0,0168 | Intron (uc003znb.3/92949, intron 7 of 9) | 76274 | ENSG00000264638 | MIR3152 |
| chr2 | 230446699 | 230447292 | -1,44 | 2,53 | -3,98 | 0,00025 | 0,0169 | Intron (uc002vpv.3/92737, intron 4 of 12) | 4323 | ENSG00000187957 | DNER |
| chr21 | 19942473 | 19942952 | -2,12 | 2,5 | -4,62 | 0,000252 | 0,0169 | Exon (uc010glf.2/uc010glf.2, exon 2 of 4) | -166503 | ENSG00000 154646 | TMPRSS15 |
| chr4 | 179512476 | 179513196 | -2,12 | 2,39 | -4,5 | 0,000252 | 0,0169 | Distal Intergenic | 862565 | ENSG00000231171 | LINC01098 |
| chr8 | 115597390 | 115606712 | 7,39 | 9,12 | -1,73 | 0,000252 | 0,0169 | Distal Intergenic | 1073522 | ENSG00000104447 | TRPS1 |
| chr1 | 40851546 | 40852915 | -0,19 | 4,6 | -4,79 | 0,000254 | 0,017 | Intron (uc010ojh.2/64744, intron 1 of 7) | -6102 | ENSG00000084070 | SMAP2 |
| chr1 1 | 123429287 | 123429674 | -2,12 | 1,76 | -3,87 | 0,000254 | 0,017 | Promoter (1-2kb) | -1296 | ENSG00000023171 | GRAMD1B |
| chr4 | 186530053 | 186530697 | -2,12 | 2,53 | -4,64 | 0,000255 | 0,017 | Intron (uc011cku.2/8470, intron 12 of 14) | 47426 | ENSG00000154556 | SORB S2 |
| chrX | 133688068 | 133688988 | -0,99 | 3,96 | -4,95 | 0,000257 | 0,0171 | Intron (uc004exn.1/100506757, intron 1 of 2) | 4014 | NA | LINC00629 |
| chr3 | 85570250 | 85571676 | -0,52 | 4,19 | -4,71 | 0,000259 | 0,0172 | Intron (uc003dqj.3/253559, intron 1 of 9) | 16041 | ENSG00000175161 | CADM2 |
| chr5 | 50718239 | 50718686 | -2,12 | 2,31 | -4,43 | 0,000259 | 0,0172 | Distal Intergenic | -39073 | NA | LOC642366 |
| chr2 | 103198354 | 103198944 | -2,12 | 2,11 | -4,22 | 0,000259 | 0,0172 | Distal Intergenic | -37222 | ENSG00000115616 | SLC9A2 |
| chr1 1 | 94372001 | 94372384 | -2,12 | 2,03 | -4,14 | 0,000259 | 0,0172 | Distal Intergenic | 71527 | ENSG00000134627 | PIWIL4 |
| chr1 | 241016809 | 241017581 | -2,12 | 2,42 | -4,54 | 0,00026 | 0,0172 | Intron (uc010pyh.2/6000, intron 9 of 17) | 14571 | ENSG00000182901 | RGS7 |
| chr12 | 102318588 | 102318899 | -1,68 | 1,99 | -3,67 | 0,00026 | 0,0172 | Distal Intergenic | 47483 | ENSG00000136048 | DRAM1 |
| chr13 | 40889230 | 40890026 | -1,83 | 2,58 | -4,41 | 0,000261 | 0,0172 | Distal Intergenic | -94591 | ENSG00000215483 | LINC00548 |
| chr7 | 14550924 | 14551694 | -0,54 | 3,29 | -3,83 | 0,000261 | 0,0172 | Intron (uc003ssz.3/1607, intron 19 of 24) | 329381 | ENSG00000136267 | DGKB |
| chr5 | 65249584 | 65250152 | -2,12 | 2,57 | -4,69 | 0,000262 | 0,0172 | Intron (uc003jui.2/55914, intron 1 of 24) | -8183 | NA | LOC1003037 49 |
| chr9 | 24594501 | 24595102 | -2,12 | 2,28 | -4,39 | 0,000262 | 0,0172 | Distal Intergenic | -48827 | ENSG00000205442 | IZUMO3 |
| chr1 1 | 102662851 | 102663649 | -1,28 | 3,07 | -4,35 | 0,000262 | 0,0172 | Exon (uc001phi.2/4312,exon 7 of 10) | 5317 | ENSG00000 196611 | MMP1 |
| chr21 | 46331005 | 46331946 | -2,12 | 2,53 | -4,65 | 0,000264 | 0,0173 | Promoter (<=1kb) | -264 | ENSG00000 160255 | ITGB2 |
| chr21 | 27567038 | 27567824 | -2,12 | 2,49 | -4,61 | 0,000264 | 0,0173 | Distal Intergenic | -23592 | ENSG00000142192 | APP |
| chr10 | 3027516 | 3029048 | -0,2 | 3,83 | -4,03 | 0,000264 | 0,0173 | Distal Intergenic | -80664 | ENSG00000067057 | PFKP |
| chr1 1 | 23988214 | 23989299 | -1,86 | 2,86 | -4,71 | 0,000265 | 0,0173 | Distal Intergenic | -529217 | ENSG00000187398 | LUZP2 |
| chr5 | 98538264 | 98539282 | -2,12 | 2,51 | -4,62 | 0,000266 | 0,0173 | Distal Intergenic | 273426 | NA | LOC1002892 30 |
| chr18 | 27550949 | 27551488 | -2,12 | 1,98 | -4,09 | 0,000266 | 0,0173 | Distal Intergenic | -327388 | ENSG00000283218 | MIR302F |
| chr18 | 11839581 | 11840091 | -1,12 | 2,55 | -3,67 | 0,000267 | 0,0173 | Intron (uc002kqc.3/2774, intron 5 of 11) | -11298 | ENSG00000255112 | CHMP1B |
| chr1 1 | 117685678 | 117686872 | -0,03 | 4,5 | -4,52 | 0,000268 | 0,0174 | Promoter (1-2kb) | 1368 | ENSG00000177103 | DSCAML1 |
| chr4 | 180385759 | 180386696 | -1,28 | 2,88 | -4,16 | 0,000268 | 0,0174 | Distal Intergenic | 1693606 | ENSG00000248197 | LINC00290 |
| chr1 | 153282812 | 153283352 | -2,12 | 2,1 | -4,21 | 0,000272 | 0,0176 | Promoter (<=1kb) | 0 | ENSG00000159527 | PGLYRP3 |
| chr13 | 106490131 | 106491346 | -0,9 | 3,77 | -4,68 | 0,000273 | 0,0176 | Distal Intergenic | 130913 | NA | LINC00343 |
| chr15 | 89891920 | 89893042 | -1,05 | 3,69 | -4,73 | 0,000274 | 0,0176 | Distal Intergenic | -13894 | ENSG00000140521 | POLG |
| chr4 | 168648436 | 168649086 | -1,86 | 2,72 | -4,58 | 0,000275 | 0,0177 | Distal Intergenic | -364602 | ENSG00000109511 | ANXA10 |
| chr5 | 18465424 | 18466665 | 0,17 | 4,72 | -4,54 | 0,000275 | 0,0177 | Distal Intergenic | -1E+06 | ENSG00000250822 | LINC02111 |
| chr1 | 245343577 | 245344321 | -2,12 | 2,27 | -4,39 | 0,000275 | 0,0177 | Intron (uc010pyq.1/55083,intron 2 of 9) | 25290 | ENSG00000 162849 | KIF26B |
| chr19 | 23901745 | 23903049 | -1,39 | 3,34 | -4,73 | 0,000276 | 0,0177 | Distal Intergenic | -31728 | ENSG00000197372 | ZNF675 |
| chr4 | 176174382 | 176174781 | -2,12 | 2,39 | -4,51 | 0,000277 | 0,0177 | Distal Intergenic | 334873 | ENSG00000 168594 | ADAM2 9 |
| chr2 | 149566162 | 149566699 | -2,12 | 2,27 | -4,38 | 0,000277 | 0,0177 | Distal Intergenic | -66093 | ENSG00000 168280 | KIF5C |
| chr8 | 16777579 | 16778394 | -2,12 | 2,41 | -4,53 | 0,000278 | 0,0177 | Distal Intergenic | 81280 | ENSG00000078579 | FGF20 |
| chr4 | 18079239 | 18080136 | -2,12 | 2,36 | -4,47 | 0,00028 | 0,0178 | Distal Intergenic | -55756 | ENSG00000178177 | LCORL |
| chr7 | 145770970 | 145771667 | -2,12 | 2,54 | -4,65 | 0,000281 | 0,0179 | Distal Intergenic | -41786 | ENSG00000174469 | CNTNAP2 |
| chr2 | 36621907 | 36622442 | -2,12 | 2,18 | -4,3 | 0,000282 | 0,0179 | Intron (uc002rpd.3/51232, intron 1 of 16) | 38537 | ENSG00000150938 | CRIM1 |
| chr9 | 125357558 | 125358626 | -1,86 | 2,4 | -4,25 | 0,000283 | 0,018 | Distal Intergenic | -18391 | ENSG00000 165202 | OR1Q1 |
| chr4 | 166635867 | 166636320 | -2,12 | 2,08 | -4,2 | 0,000283 | 0,018 | Distal Intergenic | -158090 | ENSG00000038295 | TLL1 |
| chr5 | 2142101 | 2143083 | 0,54 | 4,09 | -3,55 | 0,000284 | 0,018 | Distal Intergenic | -254808 | ENSG00000113430 | IRX4 |
| chr14 | 81991837 | 81992809 | -2,12 | 2,66 | -4,78 | 0,000285 | 0,018 | Intron (uc010tvv.2/6400, intron 3 of 20) | 7396 | ENSG00000071537 | SEL1L |
| chr1 | 238802747 | 238803712 | -2,12 | 2,47 | -4,59 | 0,000287 | 0,0181 | Distal Intergenic | -153430 | ENSG00000215808 | LINC01139 |
| chr3 | 171671436 | 171672110 | -2,12 | 2,31 | -4,43 | 0,000287 | 0,0181 | Distal Intergenic | -85308 | ENSG00000075420 | FNDC3B |
| chr4 | 168861756 | 168862155 | -2,12 | 1,92 | -4,04 | 0,000292 | 0,0184 | Distal Intergenic | -151533 | ENSG00000109511 | ANXA10 |
| chr12 | 8994569 | 8995233 | -2,12 | 1,99 | -4,11 | 0,000293 | 0,0184 | Promoter (<=1kb) | -497 | ENSG00000166535 | A2ML1 |
| chr1 1 | 1580362 | 1580930 | -2,12 | 2,32 | -4,43 | 0,000295 | 0,0185 | Intron (uc0011tq.2/81532, intron 1 of 4) | 12220 | ENSG00000 184545 | DUSP8 |
| chr2 | 4780743 | 4782283 | -2,12 | 1,98 | -4,1 | 0,000295 | 0,0185 | Distal Intergenic | -76931 | ENSG00000231532 | LINC01249 |
| chr4 | 166924358 | 166924987 | -2,12 | 2,59 | -4,71 | 0,000296 | 0,0185 | Exon (uc021xud.1/7092, exon 6 of 10) | 129948 | ENSG00000038295 | TLL1 |
| chr1 | 99870492 | 99871631 | 1,3 | 4,74 | -3,44 | 0,000296 | 0,0185 | Distal Intergenic | 140644 | ENSG00000 117600 | PLPPR4 |
| chr18 | 27593616 | 27594209 | -2,12 | 2,21 | -4,33 | 0,000297 | 0,0186 | Distal Intergenic | -284667 | ENSG00000283218 | MIR302F |
| chr4 | 96013232 | 96014129 | -2,12 | 2,02 | -4,14 | 0,000297 | 0,0186 | Promoter (<=1kb) | 590 | ENSG00000138696 | BMPR1B |
| chr2 | 105944327 | 105947711 | 8,54 | 9,47 | -0,93 | 3,00E-04 | 0,0187 | Promoter (<=1kb) | 0 | ENSG00000135966 | TGFBRAP1 |
| chr20 | 7406475 | 7407759 | -2,12 | 2,52 | -4,64 | 0,000302 | 0,0188 | Distal Intergenic | 513334 | ENSG00000101323 | HAO1 |
| chr1 | 158969441 | 158970178 | -2,12 | 2,41 | -4,52 | 0,000302 | 0,0188 | Promoter (<=1kb) | 0 | ENSG00000163565 | IFI16 |
| chr1 1 | 8928711 | 8929542 | -1,39 | 2,79 | -4,18 | 0,000302 | 0,0188 | Promoter (2-3kb) | 2956 | ENSG00000 166444 | ST5 |
| chr10 | 131661749 | 131662576 | -2,12 | 2,71 | -4,83 | 0,000303 | 0,0188 | Intron (uc001lki.2/253738, intron 10 of 15) | -20111 | ENSG00000266676 | MIR4297 |
| chr10 | 4694804 | 4695104 | -2,12 | 1,73 | -3,85 | 0,000303 | 0,0188 | Intron (uc001ihe.4/100216001, intron 1 of 3) | -3244 | NA | LINC00705 |
| chr2 | 130199776 | 130201583 | 0,77 | 5,84 | -5,07 | 0,000305 | 0,0188 | Distal Intergenic | 490307 | ENSG00000214100 | PLAC9P1 |
| chr2 | 108011402 | 108012064 | -2,12 | 2,26 | -4,38 | 0,000305 | 0,0188 | Distal Intergenic | 430518 | ENSG00000230651 | RGPD4-AS1 |
| chr7 | 66828674 | 66829377 | -2,12 | 2,22 | -4,34 | 0,000305 | 0,0188 | Distal Intergenic | 61049 | ENSG00000106610 | STAG3L4 |
| chr3 | 189857044 | 189857967 | -2,12 | 2,4 | -4,52 | 0,000306 | 0,0188 | Distal Intergenic | -16818 | ENSG00000090530 | P3H2 |
| chr4 | 72682203 | 72682815 | -2,12 | 2,08 | -4,2 | 0,000307 | 0,0189 | Distal Intergenic | -10966 | ENSG00000145321 | GC |
| chr4 | 168543757 | 168544380 | -1,91 | 2,39 | -4,3 | 0,000309 | 0,019 | Distal Intergenic | -388016 | ENSG00000196104 | SPOCK3 |
| chr5 | 18893301 | 18894092 | -2,12 | 2,57 | -4,68 | 0,00031 | 0,019 | Distal Intergenic | 992289 | ENSG00000 145526 | CDH18 |
| chr1 | 20173674 | 20174370 | 0,03 | 3,69 | -3,65 | 0,00031 | 0,019 | Distal Intergenic | -31903 | ENSG00000178828 | RNF186 |
| chr7 | 51968527 | 51969482 | -2,12 | 2,64 | -4,75 | 0,000312 | 0,019 | Distal Intergenic | -584012 | ENSG00000106078 | COBL |
| chr9 | 125663390 | 125664485 | -2,12 | 2,57 | -4,69 | 0,000312 | 0,019 | Intron (uc004bnc.2/54542, intron 1 of 21) | -3001 | ENSG00000056586 | RC3H2 |
| chr3 | 54378808 | 54379395 | -1,93 | 2,23 | -4,16 | 0,000312 | 0,019 | Intron (uc003dhf.3/55799, intron 3 of 37) | 147410 | ENSG00000157445 | CACNA2D3 |
| chr3 | 165824005 | 165824802 | -1,21 | 3,21 | -4,41 | 0,000313 | 0,0191 | Distal Intergenic | -268752 | ENSG00000 114200 | BCHE |
| chr17 | 1057143 | 1057758 | -0,9 | 3,33 | -4,23 | 0,000314 | 0,0191 | Intron (uc002fsd.4/29, intron 1 of 22) | 25408 | ENSG00000159842 | ABR |
| chr7 | 109236579 | 109237429 | -1,91 | 2,3 | -4,21 | 0,000314 | 0,0191 | Distal Intergenic | 362841 | ENSG00000237064 | EIF3IP1 |
| chr13 | 104630670 | 104631483 | -2,12 | 2,25 | -4,37 | 0,000316 | 0,0192 | Distal Intergenic | -695822 | ENSG00000263741 | MIR548AS |
| chr6 | 29651906 | 29652272 | -1,83 | 1,71 | -3,54 | 0,000317 | 0,0192 | Distal Intergenic | -6975 | ENSG00000204644 | ZFP57 |
| chr1 | 69987603 | 69988140 | -1,16 | 2,99 | -4,15 | 0,000321 | 0,0194 | Distal Intergenic | -44728 | ENSG00000033122 | LRRC7 |
| chr5 | 87958748 | 87959559 | -2,12 | 2,44 | -4,56 | 0,000322 | 0,0194 | Intron (uc011cua.1/645323, intron 3 of 4) | 3198 | ENSG00000284447 | MIR9-2 |
| chr7 | 139913853 | 139914741 | -1,64 | 2,37 | -4,01 | 0,000322 | 0,0194 | Distal Intergenic | 36792 | ENSG00000260231 | KDM7A-DT |
| chr7 | 87935669 | 87936567 | 0,66 | 4,37 | -3,72 | 0,000322 | 0,0194 | Promoter (<=1kb) | 0 | ENSG00000127954 | STEAP4 |
| chr20 | 49608983 | 49609603 | -2,12 | 2,02 | -4,14 | 0,000323 | 0,0194 | Distal Intergenic | 30014 | ENSG00000026559 | KCNG1 |
| chr2 | 41540190 | 41541032 | -1,77 | 2,57 | -4,33 | 0,000325 | 0,0196 | Distal Intergenic | -563663 | ENSG00000214691 | LINC0 1913 |
| chr3 | 50637908 | 50639209 | -0,52 | 4,12 | -4,64 | 0,000327 | 0,0196 | Distal Intergenic | 10053 | ENSG00000114737 | CISH |
| chr15 | 25038841 | 25040470 | -0,54 | 4,24 | -4,78 | 0,000328 | 0,0196 | Distal Intergenic | -28324 | ENSG00000128739 | SNRPN |
| chr19 | 20615371 | 20616106 | -2,12 | 2,61 | -4,73 | 0,000329 | 0,0196 | Distal Intergenic | -7600 | NA | ZNF826P |
| chr13 | 32240188 | 32240858 | -1,86 | 2,29 | -4,15 | 0,000329 | 0,0197 | Distal Intergenic | -72821 | ENSG00000133105 | RXFP2 |
| chr3 | 161235275 | 161236146 | -2,12 | 2,3 | -4,41 | 0,00033 | 0,0197 | Distal Intergenic | 20679 | ENSG00000182447 | OTOL1 |
| chr18 | 64403839 | 64404459 | -2,12 | 1,92 | -4,04 | 0,000331 | 0,0197 | Distal Intergenic | -132464 | ENSG00000071991 | CDH19 |
| chr13 | 85424542 | 85425443 | -2,12 | 2,54 | -4,66 | 0,000334 | 0,0198 | Distal Intergenic | -512295 | ENSG00000226317 | LINC00351 |
| chr3 | 25492922 | 25493951 | -2,12 | 2,27 | -4,39 | 0,000334 | 0,0198 | Intron(uc011awl.2/5915,intron 1 of 7) | 23088 | ENSG00000077092 | RARB |
| chr5 | 151042959 | 151043531 | -2,12 | 2,03 | -4,15 | 0,000334 | 0,0198 | 3'UTR | 10704 | ENSG00000113140 | SPARC |
| chr12 | 63370609 | 63371717 | -1,28 | 2,95 | -4,23 | 0,000335 | 0,0198 | Distal Intergenic | -41944 | ENSG00000111110 | PPM1H |
| chr7 | 14916818 | 14918605 | -0,9 | 3,9 | -4,8 | 0,000336 | 0,0198 | Intron (uc011jxt.2/1607, intron 1 of 23) | 23945 | ENSG00000136267 | DGKB |
| chr7 | 101427807 | 101428474 | -1,28 | 2,83 | -4,1 | 0,000336 | 0,0198 | Distal Intergenic | -30710 | ENSG00000257923 | CUX1 |
| chr16 | 49602012 | 49602663 | -0,94 | 2,92 | -3,86 | 0,000336 | 0,0198 | Intron (uc010vgn.3/23090, intron 3 of 5) | 95473 | ENSG00000102935 | ZNF423 |
| chr18 | 6362597 | 6364151 | -0,6 | 4,31 | -4,9 | 0,000339 | 0,02 | Intron (uc002kmz.4/91133, intron 1 of 19) | 3531 | ENSG00000154655 | L3MBTL4 |
| chr15 | 100535523 | 100536283 | -1,59 | 2,9 | -4,49 | 0,00034 | 0,02 | Intron (uc002bvv.1/170691, intron 19 of 21) | -19047 | ENSG00000140470 | ADAMTS17 |
| chr1 1 | 82253730 | 82254359 | -2,12 | 1,94 | -4,06 | 0,00034 | 0,02 | Distal Intergenic | 190547 | ENSG00000182103 | FAM181B |
| chr4 | 97519493 | 97520125 | -2,12 | 2,38 | -4,5 | 0,000341 | 0,02 | Distal Intergenic | 758254 | ENSG00000 163114 | PDHA2 |
| chr7 | 14144791 | 14145314 | -2,12 | 2,25 | -4,37 | 0,000341 | 0,02 | Distal Intergenic | -113741 | ENSG00000006468 | ETV1 |
| chr2 | 429630 | 430209 | -1,48 | 2,22 | -3,7 | 0,000341 | 0,02 | Distal Intergenic | -141322 | ENSG00000189292 | ALKAL2 |
| chr3 | 105706384 | 105707152 | -1,05 | 2,92 | -3,97 | 0,000342 | 0,02 | Distal Intergenic | -118118 | ENSG00000 114423 | CBLB |
| chr13 | 71693713 | 71694078 | -1,91 | 1,98 | -3,89 | 0,000342 | 0,02 | Intron (uc031qmd.1/100885781, intron 1 of 3) | 104440 | ENSG00000226846 | LINC00348 |
| chr9 | 917540 | 918230 | -1,01 | 2,75 | -3,76 | 0,000342 | 0,02 | Intron (uc003zgv.3/1761, intron 4 of 4) | -58738 | ENSG00000064218 | DMRT3 |
| chr3 | 30793088 | 30793877 | -2,12 | 2,23 | -4,35 | 0,000344 | 0,0201 | Intron (uc003cep.2/339896, intron 14 of 14) | 142276 | ENSG00000 144644 | GADL1 |
| chr3 | 105656865 | 105657404 | -2,12 | 2,01 | -4,13 | 0,000345 | 0,0201 | Distal Intergenic | -68599 | ENSG00000 114423 | CBLB |
| chr1 | 196296701 | 196297343 | -2,12 | 2,65 | -4,77 | 0,000346 | 0,0201 | Intron (uc009wyt.1/343450, intron 12 of 20) | 14036 | ENSG00000162687 | KCNT2 |
| chr1 | 194909156 | 194909743 | -2,12 | 2,55 | -4,67 | 0,000346 | 0,0201 | Distal Intergenic | 1401636 | ENSG00000162687 | KCNT2 |
| chr4 | 179820506 | 179821377 | -2,12 | 2,51 | -4,63 | 0,000347 | 0,0201 | Distal Intergenic | 1170595 | ENSG00000231171 | LINC01098 |
| chr12 | 75702102 | 75703174 | -1,43 | 2,99 | -4,42 | 0,000348 | 0,0201 | Intron (uc001sxi.4/84698, intron 3 of 11) | 8779 | ENSG00000180881 | CAPS2 |
| chr12 | 20974752 | 20975538 | -2,12 | 1,9 | -4,02 | 0,000348 | 0,0201 | Promoter (<=1kb) | 0 | ENSG00000 111700 | SLCO1B3 |
| chr1 | 74470566 | 74471509 | -0,97 | 3,32 | -4,3 | 0,000349 | 0,0201 | Distal Intergenic | 192362 | ENSG00000 162620 | LRRIQ3 |
| chr19 | 52645939 | 52646533 | -2,12 | 1,86 | -3,97 | 0,000349 | 0,0201 | Promoter (2-3kb) | -2748 | ENSG00000204611 | ZNF616 |
| chr6 | 14149218 | 14149830 | -2,12 | 2,11 | -4,23 | 0,000352 | 0,0203 | Distal Intergenic | 31353 | ENSG00000112149 | CD83 |
| chr3 | 114973325 | 114973947 | -1,54 | 2,56 | -4,1 | 0,000353 | 0,0203 | Distal Intergenic | -107198 | ENSG00000181722 | ZBTB20 |
| chr13 | 104547019 | 104548229 | -2,12 | 2,63 | -4,75 | 0,000355 | 0,0204 | Distal Intergenic | -612171 | ENSG00000263741 | MIR548AS |
| chr20 | 18242053 | 18242637 | -1,28 | 2,5 | -3,78 | 0,000357 | 0,0205 | Distal Intergenic | -26484 | ENSG00000 125846 | ZNF133 |
| chr1 1 | 117400280 | 117401522 | -0,99 | 3,61 | -4,6 | 0,000359 | 0,0205 | Intron (uc001prh.1/57453, intron 4 of 32) | 144967 | ENSG00000110274 | CEP164 |
| chr15 | 91781878 | 91782953 | -0,4 | 3,7 | -4,1 | 0,000359 | 0,0205 | Intron (uc002bqt.3/9899, intron 2 of 8) | 138696 | ENSG00000 185518 | SV2B |
| chr17 | 26649286 | 26649687 | -2,12 | 1,84 | -3,95 | 0,00036 | 0,0206 | Intron (uc002hat.3/27346, intron 1 of 2) | 3165 | ENSG00000109084 | TMEM97 |
| chr13 | 36365957 | 36366618 | -2,12 | 2,32 | -4,43 | 0,000362 | 0,0206 | Intron (uc001uvc.3/100302239, intron 1 of 1) | 63380 | ENSG00000133083 | DCLK1 |
| chr12 | 7649049 | 7649572 | -2,12 | 1,92 | -4,04 | 0,000362 | 0,0207 | Exon (uc001qsz.3/9332, exon 5 of 17) | 6842 | ENSG00000177575 | CD163 |
| chr19 | 14906323 | 14906775 | -1,27 | 2,51 | -3,78 | 0,000365 | 0,0208 | Distal Intergenic | 4173 | ENSG00000127530 | OR7C1 |
| chr9 | 29779239 | 29780524 | -0,64 | 3,93 | -4,57 | 0,000368 | 0,0209 | Distal Intergenic | -566241 | ENSG00000174482 | LINGO2 |
| chr20 | 23161143 | 23161757 | -2,12 | 2,29 | -4,41 | 0,000368 | 0,0209 | Distal Intergenic | -47870 | ENSG00000233746 | LINC00656 |
| chr2 | 183953851 | 183954892 | -0,36 | 4,65 | -5,01 | 0,00037 | 0,021 | Intron (uc002upd.3/142679, intron 3 of 3) | 10564 | ENSG00000 162999 | DUSP19 |
| chr4 | 167723556 | 167724580 | -2,12 | 2,61 | -4,72 | 0,000371 | 0,021 | Intron (uc021xuf.1/50859, intron 6 of 10) | 430744 | ENSG00000196104 | SPOCK3 |
| chr14 | 62026059 | 62026851 | -2,12 | 2,43 | -4,55 | 0,000373 | 0,0211 | Distal Intergenic | -10407 | ENSG00000232774 | FLJ22447 |
| chr19 | 1939730 | 1944239 | 9,73 | 10,52 | -0,8 | 0,000375 | 0,0212 | Promoter (<=1kb) | 0 | ENSG00000133275 | CSNK1G2 |
| chr8 | 13675131 | 13676235 | -2,12 | 2,59 | -4,71 | 0,000376 | 0,0212 | Distal Intergenic | 250779 | ENSG00000164743 | C8orf48 |
| chr9 | 105133327 | 105134436 | -0,9 | 3,99 | -4,9 | 0,000378 | 0,0213 | Distal Intergenic | -623157 | ENSG00000155833 | CYLC2 |
| chr9 | 21576390 | 21577240 | -1,73 | 2,53 | -4,26 | 0,000378 | 0,0213 | Distal Intergenic | -16693 | NA | MIR31HG |
| chr5 | 26598010 | 26598850 | -1,59 | 3,05 | -4,64 | 0,000379 | 0,0213 | Distal Intergenic | 292092 | ENSG00000113100 | CDH9 |
| chr8 | 114409582 | 114410312 | -1,59 | 2,88 | -4,47 | 0,000379 | 0,0213 | Intron (uc003ynu.3/114788, intron 1 of 70) | -20200 | ENSG00000164796 | CSMD3 |
| chr1 1 | 62169081 | 62170085 | -2,12 | 2,72 | -4,83 | 0,000381 | 0,0213 | Distal Intergenic | -16422 | ENSG00000149021 | SCGB1A1 |
| chr9 | 82186651 | 82190326 | 8,88 | 9,73 | -0,86 | 0,000381 | 0,0213 | Promoter (<=1kb) | 0 | ENSG00000 106829 | TLE4 |
| chr9 | 78423770 | 78424355 | -1,33 | 2,22 | -3,55 | 0,000383 | 0,0214 | Distal Intergenic | -81205 | ENSG00000099139 | PCSK5 |
| chr5 | 25451230 | 25451844 | -2,12 | 2,29 | -4,41 | 0,000384 | 0,0214 | Distal Intergenic | -610538 | ENSG00000248908 | LINC02239 |
| chr4 | 92196641 | 92197183 | -2,12 | 2 | -4,12 | 0,000384 | 0,0214 | Intron (uc003hsv.4/401145, intron 10 of 10) | 625970 | ENSG00000184305 | CCSER1 |
| chr1 1 | 101741767 | 101742510 | -2,12 | 2,22 | -4,34 | 0,000385 | 0,0214 | Distal Intergenic | -43236 | ENSG00000110318 | CEP126 |
| chr9 | 26219783 | 26220475 | -1,04 | 3,13 | -4,17 | 0,000385 | 0,0214 | Distal Intergenic | 153110 | ENSG00000276759 | LOC1005064 22 |
| chr1 | 13831371 | 13831915 | -1,75 | 2,26 | -4,01 | 0,000385 | 0,0214 | Intron (uc001avb.3/126755, intron 1 of 1) | 8327 | ENSG00000 162494 | LRRC38 |
| chr21 | 18039921 | 18040671 | -2,12 | 2,18 | -4,3 | 0,000386 | 0,0214 | Distal Intergenic | 77364 | ENSG00000207863 | MIR125B2 |
| chr15 | 93591673 | 93592665 | -0,21 | 3,94 | -4,15 | 0,000386 | 0,0214 | Intron (uc002bsq.2/56963, intron 2 of 2) | 23724 | ENSG00000182175 | RGMA |
| chr4 | 96724525 | 96726159 | -0,65 | 4,09 | -4,74 | 0,000387 | 0,0214 | Distal Intergenic | -35080 | ENSG00000 163114 | PDHA2 |
| chr8 | 3191655 | 3192615 | -2,12 | 2,49 | -4,61 | 0,000389 | 0,0214 | Intron (uco11kwj.2/64478, intron 11 of 55) | 61288 | ENSG00000183117 | CSMD1 |
| chr1 | 233803895 | 233804755 | -2,12 | 2,42 | -4,54 | 0,00039 | 0,0214 | Intron (uc010pxo.1/3775, intron 2 of 2) | 38542 | ENSG00000135750 | KCNK1 |
| chr4 | 105345750 | 105346569 | -0,9 | 3,47 | -4,38 | 0,000391 | 0,0215 | Distal Intergenic | 69489 | ENSG00000168772 | CXXC4 |
| chr5 | 19461178 | 19462086 | -0,99 | 3,51 | -4,5 | 0,000393 | 0,0215 | Distal Intergenic | 424295 | ENSG00000 145526 | CDH18 |
| chrX | 118896485 | 118897538 | -1,46 | 2,8 | -4,26 | 0,000393 | 0,0215 | Distal Intergenic | 3909 | ENSG00000187808 | SOWAHD |
| chr3 | 107240720 | 107245443 | 9,56 | 10,38 | -0,82 | 0,000393 | 0,0215 | Promoter (<=1kb) | 0 | ENSG00000114439 | BBX |
| chr5 | 18841548 | 18842110 | -2,12 | 2,28 | -4,4 | 0,000394 | 0,0215 | Distal Intergenic | 1044271 | ENSG00000 145526 | CDH18 |
| chr5 | 26674793 | 26675476 | -2,12 | 2,26 | -4,38 | 0,000395 | 0,0215 | Distal Intergenic | 215466 | ENSG00000113100 | CDH9 |
| chr6 | 4884835 | 4885455 | -2,12 | 2,15 | -4,27 | 0,000395 | 0,0215 | Intron (uc003mwi.3/9425, intron 3 of 8) | -4771 | ENSG00000153046 | CDYL |
| chr1 | 191144744 | 191145617 | -2,12 | 2,57 | -4,68 | 0,000396 | 0,0216 | Distal Intergenic | 550724 | ENSG00000231175 | LINC0 1720 |
| chr15 | 72673939 | 72674726 | -2,12 | 2,07 | -4,19 | 0,000397 | 0,0216 | Distal Intergenic | -5419 | ENSG00000 140488 | CELF6 |
| chr2 | 156527500 | 156527922 | -2,12 | 1,99 | -4,11 | 0,000398 | 0,0216 | Distal Intergenic | 656604 | ENSG00000153234 | NR4A2 |
| chr14 | 34407267 | 34408068 | -0,69 | 2,79 | -3,48 | 0,000399 | 0,0217 | Intron (uc001wry.3/112399, intron 1 of 4) | 11964 | ENSG00000129521 | EGLN3 |
| chr19 | 56850430 | 56851731 | -2,12 | 2,1 | -4,21 | 4,00E-04 | 0,0217 | Intron (uc002qmr.3/79149, intron 2 of 6) | -24136 | ENSG00000 131848 | ZSCAN5A |
| chr4 | 189476117 | 189476904 | -0,34 | 3,49 | -3,83 | 4,00E-04 | 0,0217 | Intron (uc021xvn.1/401164.intron 4 of 4) | 99385 | ENSG00000249378 | LINC01060 |
| chr15 | 60617015 | 60617575 | -2,12 | 2,06 | -4,18 | 0,000401 | 0,0217 | Distal Intergenic | 72610 | ENSG00000182718 | ANXA2 |
| chr3 | 164983665 | 164984384 | -2,12 | 2,4 | -4,51 | 0,000402 | 0,0217 | Distal Intergenic | -69196 | ENSG00000121871 | SLITRK3 |
| chr12 | 20307153 | 20307915 | -2,12 | 2,35 | -4,47 | 0,000402 | 0,0217 | Distal Intergenic | 139534 | ENSG00000256287 | LINC02398 |
| chr15 | 86422372 | 86423567 | -1,12 | 3,19 | -4,31 | 0,000402 | 0,0217 | Distal Intergenic | -84183 | ENSG00000183655 | KLHL25 |
| chr2 | 141845998 | 141846380 | -2,12 | 1,82 | -3,94 | 0,000403 | 0,0217 | Intron (uc002tvj.1/53353, intron 7 of 90) | 1042890 | ENSG00000168702 | LRP1B |
| chr10 | 91087252 | 91090813 | 0,36 | 5,23 | -4,88 | 0,000404 | 0,0217 | Promoter (<=1kb) | 0 | ENSG00000 119917 | IFIT3 |
| chr1 | 207483274 | 207483636 | -2,12 | 1,59 | -3,71 | 0,000406 | 0,0218 | Distal Intergenic | -11181 | ENSG00000196352 | CD55 |
| chr5 | 123548834 | 123549587 | -1,93 | 2,17 | -4,1 | 0,000408 | 0,0219 | Distal Intergenic | 430767 | ENSG00000168916 | ZNF608 |
| chr8 | 80272193 | 80272752 | -2,12 | 2,48 | -4,6 | 0,000409 | 0,0219 | Distal Intergenic | -250297 | ENSG00000104435 | STMN2 |
| chr4 | 171087636 | 171088357 | -1,22 | 2,32 | -3,53 | 0,000409 | 0,0219 | Distal Intergenic | -76264 | ENSG00000109576 | AADAT |
| chr3 | 165532997 | 165533561 | -2,12 | 2,38 | -4,5 | 0,00041 | 0,0219 | Intron (uc003fem.4/590, intron 2 of 3) | 21692 | ENSG00000 114200 | BCHE |
| chr5 | 28368272 | 28369089 | -2,12 | 2,33 | -4,45 | 0,000411 | 0,0219 | Distal Intergenic | -557888 | NA | LSP1P3 |
| chr1 | 238953911 | 238954698 | -0,74 | 3,46 | -4,2 | 0,000412 | 0,022 | Distal Intergenic | -304594 | ENSG00000215808 | LINC01139 |
| chr19 | 17292604 | 17293249 | -2,12 | 2,15 | -4,26 | 0,000413 | 0,022 | Intron (uc002nfi.3/4650, intron 15 of 39) | -15678 | ENSG00000099331 | MY09B |
| chr6 | 71791669 | 71792325 | -0,86 | 3,02 | -3,87 | 0,000416 | 0,0221 | Distal Intergenic | -124881 | ENSG00000112309 | B3 GAT2 |
| chr18 | 3988426 | 3989178 | -1,62 | 2,6 | -4,22 | 0,000417 | 0,0221 | Intron (uc03lrhf.1/101410534, intron 1 of 4) | 26073 | ENSG00000263878 | DLGAP1-AS4 |
| chr8 | 25836241 | 25837064 | -1,39 | 2,68 | -4,06 | 0,000417 | 0,0221 | Intron (uc003xek.3/5520, intron 7 of 10) | 65576 | ENSG00000221818 | EBF2 |
| chr13 | 113801014 | 113801359 | -1,83 | 1,99 | -3,82 | 0,000419 | 0,0222 | Intron (uc010agq.1/2159, intron 6 of 6) | -11609 | ENSG00000 126231 | PROZ |
| chr4 | 183512286 | 183514912 | -0,63 | 4,2 | -4,83 | 0,00042 | 0,0222 | Intron (uc021xux.1/55714, intron 1 of 2) | -86489 | ENSG00000218336 | TENM3 |
| chr7 | 86203126 | 86203541 | -2,12 | 1,83 | -3,95 | 0,000422 | 0,0222 | Distal Intergenic | -69689 | ENSG00000198822 | GRM3 |
| chr13 | 71883534 | 71884657 | -0,02 | 3,8 | -3,82 | 0,000422 | 0,0222 | Distal Intergenic | 294261 | ENSG00000226846 | LINC00348 |
| chr12 | 22884109 | 22884959 | -2,12 | 2,49 | -4,61 | 0,000423 | 0,0222 | Distal Intergenic | 106033 | ENSG00000139163 | ETNK1 |
| chr1 | 100069194 | 100069813 | -0,19 | 3,15 | -3,34 | 0,000424 | 0,0223 | Distal Intergenic | -41618 | ENSG00000099260 | PALMD |
| chr4 | 99847779 | 99851991 | 8,81 | 9,63 | -0,82 | 0,000424 | 0,0222 | Promoter (<=1kb) | 0 | ENSG00000 151247 | EIF4E |
| chr4 | 108721055 | 108721478 | -0,8 | 2,41 | -3,2 | 0,000426 | 0,0223 | Distal Intergenic | -24243 | ENSG00000164023 | SGMS2 |
| chr5 | 26761263 | 26761756 | -1,86 | 2,29 | -4,14 | 0,000428 | 0,0224 | Distal Intergenic | 129186 | ENSG00000113100 | CDH9 |
| chr1 | 238951075 | 238952044 | -2,12 | 2,35 | -4,47 | 0,000429 | 0,0224 | Distal Intergenic | -301758 | ENSG00000215808 | LINC01139 |
| chr16 | 69216940 | 69217454 | -2,12 | 2,13 | -4,25 | 0,000429 | 0,0224 | Distal Intergenic | -3596 | ENSG00000168807 | SNIB2 |
| chr21 | 19904854 | 19905664 | -2,12 | 2,43 | -4,55 | 0,000432 | 0,0225 | Distal Intergenic | -128884 | ENSG00000 154646 | TMPRSS15 |
| chr19 | 17291469 | 17292412 | -0,95 | 3,65 | -4,6 | 0,000433 | 0,0225 | Exon (uc002nfi.3/4650, exon 15 of 40) | -16515 | ENSG00000099331 | MYO9B |
| chr2 | 23626720 | 23627696 | -2,12 | 2,23 | -4,35 | 0,000433 | 0,0225 | Intron (uc010ykg.2/114818, intron 1 of 13) | 18422 | ENSG00000 119771 | KLHL29 |
| chr3 | 163180362 | 163180991 | -2,12 | 2,06 | -4,18 | 0,000434 | 0,0225 | Distal Intergenic | -159273 | ENSG00000241369 | LINC01192 |
| chr10 | 11589162 | 11589853 | -2,12 | 2,63 | -4,75 | 0,000436 | 0,0226 | Intron (uc001ikt.3/9712, intron 2 of 14) | -14888 | ENSG00000148429 | USP6NL |
| chr5 | 3181788 | 3182433 | -2,12 | 2,15 | -4,26 | 0,000436 | 0,0226 | Distal Intergenic | 353775 | ENSG00000248118 | LINC01019 |
| chr4 | 84814674 | 84815570 | -0,44 | 3,67 | -4,11 | 0,000436 | 0,0226 | Intron (uc010ikd.2/uc010ikd.2, intron 4 of 5) | 357204 | ENSG00000138678 | GPAT3 |
| chr5 | 28512788 | 28513361 | -2,12 | 2,16 | -4,28 | 0,000437 | 0,0226 | Distal Intergenic | -413616 | NA | LSP1P3 |
| chr15 | 92690246 | 92690906 | -1,64 | 2,84 | -4,47 | 0,000438 | 0,0227 | Exon (uc002bqx.2/28232, exon 8 of 10) | -246234 | ENSG00000140557 | ST8SIA2 |
| chr7 | 83368413 | 83369000 | -2,12 | 2,14 | -4,26 | 0,000441 | 0,0228 | Distal Intergenic | -89934 | ENSG00000170381 | SEMA3E |
| chr1 | 71065963 | 71067179 | -0,51 | 3,64 | -4,16 | 0,000441 | 0,0228 | Distal Intergenic | 189062 | ENSG00000 116761 | CTH |
| chr21 | 40510434 | 40511085 | -2,12 | 2,1 | -4,22 | 0,000442 | 0,0228 | Distal Intergenic | 44355 | ENSG00000183527 | PSMG1 |
| chr9 | 126025319 | 126032261 | 8,86 | 9,88 | -1,01 | 0,000442 | 0,0228 | Promoter (<=1kb) | 0 | ENSG00000 165209 | STRBP |
| chr9 | 132357297 | 132357932 | -1,39 | 2,66 | -4,04 | 0,000443 | 0,0228 | Distal Intergenic | 25123 | ENSG00000179058 | C9orf50 |
| chr18 | 13381775 | 13382911 | 0,69 | 4,34 | -3,65 | 0,000443 | 0,0228 | Intron (uc002ksb.3/753, intron 2 of 5) | -4429 | ENSG00000168675 | LDLRAD4 |
| chr1 | 192445888 | 192446472 | -2,12 | 2,14 | -4,26 | 0,000444 | 0,0228 | Distal Intergenic | -98385 | ENSG00000090104 | RGS1 |
| chr1 | 241072595 | 241074301 | -0,31 | 3,8 | -4,12 | 0,000444 | 0,0228 | Intron (uc010pyh.2/6000, intron 6 of 17) | -40443 | ENSG00000182901 | RGS7 |
| chr2 | 164452673 | 164453728 | -2,12 | 2,5 | -4,62 | 0,000445 | 0,0228 | Distal Intergenic | 138785 | ENSG00000182263 | FIGN |
| chr4 | 103421140 | 103426410 | 8,98 | 9,8 | -0,82 | 0,000445 | 0,0228 | Promoter (<=1kb) | 0 | ENSG00000109320 | NFKB1 |
| chr2 | 102439949 | 102440669 | -2,12 | 2,1 | -4,21 | 0,000446 | 0,0228 | Promoter (1-2kb) | -1112 | ENSG00000071054 | MAP4K4 |
| chr7 | 15105275 | 15106214 | -0,77 | 3,56 | -4,33 | 0,000447 | 0,0228 | Distal Intergenic | -162725 | ENSG00000136267 | DGKB |
| chr12 | 65948291 | 65949619 | -0,77 | 3,44 | -4,21 | 0,000448 | 0,0229 | Intron (uc010ssu.2/uc010ssu.2, intron 2 of 2) | -267818 | ENSG00000 149948 | HMGA2 |
| chr4 | 136446047 | 136446565 | -2,12 | 2,12 | -4,24 | 0,000449 | 0,0229 | Distal Intergenic | -1E+06 | ENSG00000254535 | PABPC4L |
| chr4 | 41081178 | 41082034 | -2,12 | 2,43 | -4,54 | 0,000451 | 0,0229 | Intron (uc003gv1.3/323, intron 3 of 17) | -64763 | ENSG00000163697 | APBB2 |
| chr1 1 | 129061645 | 129062521 | -1,36 | 2,9 | -4,26 | 0,000451 | 0,0229 | Promoter (<=1kb) | 0 | ENSG00000134909 | ARHGAP3 2 |
| chr4 | 129579797 | 129580330 | -1,59 | 2,52 | -4,11 | 0,000451 | 0,0229 | Distal Intergenic | -150449 | ENSG00000077684 | JADE1 |
| chr8 | 61590090 | 61594459 | 9,69 | 10,54 | -0,85 | 0,000451 | 0,0229 | Promoter (<=1kb) | 0 | ENSG00000171316 | CHD7 |
| chr9 | 25752320 | 25752861 | -2,12 | 2,25 | -4,37 | 0,000453 | 0,023 | Distal Intergenic | -73464 | ENSG00000 198680 | TUSC1 |
| chr9 | 80302565 | 80303404 | 0,21 | 4,06 | -3,85 | 0,000453 | 0,023 | Distal Intergenic | -39333 | ENSG00000156049 | GNA14 |
| chr6 | 128891329 | 128892179 | -2,12 | 2,53 | -4,65 | 0,000454 | 0,023 | Distal Intergenic | -49459 | ENSG00000 152894 | PTPRK |
| chr14 | 103726859 | 103727273 | -1,75 | 1,64 | -3,39 | 0,000454 | 0,023 | Distal Intergenic | -71494 | ENSG00000251533 | LINC00605 |
| chr8 | 53335921 | 53336484 | -1,86 | 2,14 | -4 | 0,000455 | 0,023 | Distal Intergenic | -13482 | ENSG00000147488 | ST18 |
| chr4 | 31026287 | 31027122 | -2,12 | 2,48 | -4,6 | 0,000456 | 0,023 | Intron (uc011bxx.2/5099, intron 2 of 2) | 304250 | ENSG00000 169851 | PCDH7 |
| chr4 | 25554311 | 25555003 | -2,12 | 2,45 | -4,57 | 0,000456 | 0,023 | Distal Intergenic | -102432 | ENSG00000157765 | SLC34A2 |
| chr4 | 113065468 | 113069415 | 7,98 | 9,09 | -1,12 | 0,000456 | 0,023 | Promoter (<=1kb) | 0 | ENSG00000174749 | FAM241A |
| chr4 | 106066626 | 106070386 | 8,79 | 9,64 | -0,85 | 0,000456 | 0,023 | Promoter (<=1kb) | 0 | ENSG00000168769 | TET2 |
| chr8 | 18645359 | 18646235 | -1,17 | 3,04 | -4,21 | 0,000457 | 0,023 | Intron (uc003wvv.3/23362, intron 5 of 12) | 20170 | ENSG00000156011 | PSD3 |
| chr1 | 220891328 | 220892790 | -0,49 | 3,78 | -4,26 | 0,000458 | 0,023 | Distal Intergenic | 27700 | ENSG00000162817 | C1orf115 |
| chr13 | 23566215 | 23566719 | -1,12 | 2,23 | -3,35 | 0,000458 | 0,023 | Distal Intergenic | -93895 | NA | BASP1P1 |
| chr4 | 53191141 | 53192100 | -1,91 | 2,07 | -3,98 | 0,000459 | 0,023 | Distal Intergenic | 273548 | ENSG00000163071 | SPATA18 |
| chr2 | 121936127 | 121936608 | -1,62 | 2,04 | -3,65 | 0,000459 | 0,023 | Distal Intergenic | 55162 | ENSG00000115112 | TFCP2L1 |
| chr8 | 112392362 | 112392990 | -2,12 | 2,62 | -4,74 | 0,000461 | 0,0231 | Distal Intergenic | 956970 | ENSG00000164796 | CSMD3 |
| chr10 | 21607117 | 21607826 | -1,39 | 2,75 | -4,14 | 0,000462 | 0,0231 | Distal Intergenic | 143886 | ENSG00000231920 | NEBL-AS1 |
| chr19 | 55070701 | 55071149 | -2,12 | 1,82 | -3,94 | 0,000466 | 0,0233 | Distal Intergenic | -13333 | ENSG00000239998 | LILRA2 |
| chr1 1 | 25985472 | 25985905 | -2,12 | 1,99 | -4,11 | 0,000467 | 0,0233 | Distal Intergenic | -224924 | ENSG00000134343 | ANO3 |
| chr20 | 11506670 | 11507242 | -2,12 | 1,97 | -4,09 | 0,000467 | 0,0233 | Distal Intergenic | -252639 | NA | LOC339593 |
| chr6 | 101763858 | 101764771 | -2,12 | 2,22 | -4,34 | 0,000468 | 0,0233 | Distal Intergenic | -81898 | ENSG00000164418 | GRIK2 |
| chr12 | 74963772 | 74964266 | -1,05 | 2,8 | -3,85 | 0,00047 | 0,0234 | Distal Intergenic | 32221 | ENSG00000253719 | ATXN7L3B |
| chr15 | 72765224 | 72769842 | 9,46 | 10,16 | -0,7 | 0,00047 | 0,0234 | Promoter (<=1kb) | 0 | ENSG00000166233 | ARIH1 |
| chr20 | 2761151 | 2761763 | -0,93 | 2,66 | -3,59 | 0,000474 | 0,0235 | Distal Intergenic | 19529 | ENSG00000088882 | CPXM1 |
| chr4 | 179217047 | 179218597 | -1,57 | 2,82 | -4,39 | 0,000476 | 0,0236 | Distal Intergenic | 567136 | ENSG00000231171 | LINC01098 |
| chr6 | 138818406 | 138821090 | 0,21 | 5,23 | -5,02 | 0,000477 | 0,0236 | Promoter (<=1kb) | 0 | ENSG00000135540 | NHSL1 |
| chr2 | 204514363 | 204515074 | -2,12 | 2,39 | -4,51 | 0,000478 | 0,0237 | Distal Intergenic | -56124 | ENSG00000178562 | CD28 |
| chr10 | 29115023 | 29115614 | -2,12 | 2,09 | -4,21 | 0,000479 | 0,0237 | Distal Intergenic | -30044 | ENSG00000235824 | LINC00837 |
| chr19 | 16471541 | 16472394 | -2,12 | 2,37 | -4,49 | 0,00048 | 0,0237 | 3'UTR | 35890 | ENSG00000127528 | KLF2 |
| chr4 | 117279965 | 117280574 | -2,12 | 2,1 | -4,22 | 0,00048 | 0,0237 | Distal Intergenic | 59084 | ENSG00000284253 | MIR1973 |
| chr2 | 166150443 | 166151887 | 0,4 | 4,97 | -4,58 | 0,000483 | 0,0239 | Promoter (<=1kb) | 102 | ENSG00000136531 | SCN2A |
| chr21 | 20247400 | 20248678 | -0,16 | 3,49 | -3,65 | 0,000484 | 0,0239 | Distal Intergenic | -471430 | ENSG00000 154646 | TMPRSS15 |
| chr2 | 140920375 | 140921132 | -2,12 | 2,15 | -4,27 | 0,000485 | 0,0239 | Distal Intergenic | 1265481 | NA | YY1P2 |
| chr3 | 14088221 | 14088754 | -2,12 | 2,24 | -4,35 | 0,000486 | 0,0239 | Intron (uc011avd.2/348825, intron 1 of 2) | 29320 | ENSG00000180438 | TPRXL |
| chr2 | 35793385 | 35794587 | -0,75 | 3,37 | -4,12 | 0,000486 | 0,0239 | Distal Intergenic | 788126 | NA | CRIM1-DT |
| chr3 | 51787493 | 51789462 | -0,09 | 4,43 | -4,52 | 0,00049 | 0,024 | Distal Intergenic | 23741 | ENSG00000214686 | IQCF6 |
| chr7 | 53493708 | 53494785 | -0,2 | 3,77 | -3,97 | 0,00049 | 0,024 | Distal Intergenic | 384839 | ENSG00000205628 | LINC01446 |
| chr13 | 71497577 | 71499309 | 1,15 | 5,51 | -4,36 | 0,000491 | 0,0241 | Distal Intergenic | -89964 | ENSG00000226846 | LINC00348 |
| chr7 | 3067631 | 3068086 | -2,12 | 1,98 | -4,1 | 0,000492 | 0,0241 | Intron (uc003smv.3/84433, intron 1 of 24) | 15423 | ENSG00000 198286 | CARD11 |
| chr12 | 9370635 | 9371209 | -2,12 | 1,8 | -3,91 | 0,000492 | 0,0241 | Distal Intergenic | -9669 | ENSG00000126838 | PZP |
| chr16 | 8573250 | 8573731 | -1,73 | 2,16 | -3,89 | 0,000493 | 0,0241 | Distal Intergenic | 48495 | ENSG00000232258 | 4 |
| chr9 | 73943194 | 73943550 | -2,12 | 1,74 | -3,86 | 0,000494 | 0,0241 | Intron (uc004aii.3/80036, intron 1 of 5) | 118270 | ENSG00000083067 | TRPM3 |
| chr7 | 150691619 | 150692570 | -2,12 | 2,38 | -4,5 | 0,000495 | 0,0241 | Promoter (<=1kb) | 778 | ENSG00000164867 | NOS3 |
| chr4 | 44881414 | 44882447 | -2,12 | 2,07 | -4,19 | 0,000495 | 0,0241 | Distal Intergenic | -152763 | ENSG00000163281 | GNPDA2 |
| chr6 | 72352107 | 72352710 | -2,12 | 2,06 | -4,18 | 0,000496 | 0,0242 | Distal Intergenic | -221659 | ENSG00000233237 | LINC00472 |
| chr14 | 100285839 | 100286305 | -2,12 | 1,9 | -4,02 | 0,000496 | 0,0242 | Intron (uc010avt.1/2009, intron 1 of 10) | 26094 | ENSG00000066629 | EML1 |
| chr1 | 228672857 | 228679356 | 9,45 | 10,26 | -0,81 | 0,000498 | 0,0242 | Promoter (<=1kb) | 0 | ENSG00000168159 | RNF187 |
| chr20 | 34688621 | 34689346 | -1,62 | 2,6 | -4,21 | 0,000502 | 0,0243 | Intron (uc002xeu.3/2036, intron 1 of 19) | 7989 | ENSG00000088367 | EPB41L1 |
| chr12 | 76577244 | 76578072 | -1,83 | 2,4 | -4,23 | 0,000503 | 0,0243 | Distal Intergenic | -98506 | ENSG00000187109 | NAP1L1 |
| chr8 | 12422908 | 12423498 | -2,12 | 2,04 | -4,16 | 0,000503 | 0,0243 | Exon (uc01 1kxp.1/100506990, exon 6 of 6) | 84570 | NA | LOC1005069 90 |
| chr4 | 127090879 | 127091369 | -0,67 | 2,66 | -3,33 | 0,000503 | 0,0243 | Distal Intergenic | 662465 | ENSG00000283550 | MIR2054 |
| chr2 | 230715254 | 230715804 | -1,93 | 2,56 | -4,49 | 0,000507 | 0,0244 | Intron (uc021vxw.1/9320, intron 2 of 39) | 29040 | ENSG00000153827 | TRIP12 |
| chr12 | 4417152 | 4417679 | -1,62 | 2,8 | -4,42 | 0,000508 | 0,0245 | Distal Intergenic | -12680 | ENSG00000078237 | TIGAR |
| chr2 | 23597759 | 23598375 | -1,86 | 2,13 | -3,99 | 0,000508 | 0,0245 | Distal Intergenic | -9923 | ENSG00000 119771 | KLHL29 |
| chr13 | 25671601 | 25672505 | -1,57 | 2,48 | -4,05 | 0,000509 | 0,0245 | Promoter (1-2kb) | 1325 | ENSG00000 151846 | PABPC3 |
| chr5 | 125544556 | 125545222 | -2,12 | 2,22 | -4,34 | 0,000512 | 0,0246 | Distal Intergenic | -150566 | ENSG00000155324 | GRAMD2B |
| chr4 | 169412972 | 169413723 | -2,12 | 2,33 | -4,44 | 0,000513 | 0,0246 | Distal Intergenic | -4494 | ENSG00000129116 | PALLD |
| chr7 | 16991227 | 16991948 | -1,29 | 2,9 | -4,19 | 0,000514 | 0,0246 | Distal Intergenic | -69614 | ENSG00000173467 | AGR3 |
| chr8 | 111477993 | 111478646 | -2,12 | 2,41 | -4,52 | 0,000515 | 0,0246 | Distal Intergenic | -489917 | ENSG00000164794 | KCNV1 |
| chr4 | 181471134 | 181471622 | -2,12 | 2,01 | -4,13 | 0,000517 | 0,0246 | Distal Intergenic | 608680 | ENSG00000248197 | LINC00290 |
| chr3 | 192158133 | 192158702 | -2,12 | 1,79 | -3,91 | 0,000519 | 0,0247 | Intron (uc003fsy.3/2257, intron 2 of 5) | -31295 | ENSG00000114279 | FGF12 |
| chr2 | 163678222 | 163678732 | -1,59 | 2,67 | -4,26 | 0,000523 | 0,0248 | Intron (uc002uch.2/90134, intron 2 of 15) | 16525 | ENSG00000 184611 | KCNH7 |
| chr6 | 12288087 | 12288530 | -2,12 | 1,76 | -3,87 | 0,000523 | 0,0248 | Promoter (1-2kb) | -1999 | ENSG00000078401 | EDN1 |
| chr1 | 89872900 | 89873818 | -1,83 | 2,62 | -4,45 | 0,000527 | 0,025 | Promoter (<=1kb) | 0 | ENSG00000225492 | GBP1P1 |
| chr5 | 21282438 | 21284052 | -0,77 | 3,65 | -4,42 | 0,00053 | 0,0251 | Distal Intergenic | -57890 | ENSG00000183666 | GUSBP1 |
| chr8 | 47930303 | 47931273 | -2,12 | 1,88 | -4 | 0,00053 | 0,0251 | Distal Intergenic | -169657 | NA | LOC1002878 46 |
| chr1 | 240352846 | 240353217 | -2,12 | 2,14 | -4,26 | 0,000533 | 0,0252 | Intron (uc010pyd.2/56776, intron 4 of 17) | -55343 | ENSG00000155816 | FMN2 |
| chr13 | 71847248 | 71848136 | -2,12 | 2,3 | -4,42 | 0,000535 | 0,0252 | Distal Intergenic | 257975 | ENSG00000226846 | LINC00348 |
| chr18 | 41268849 | 41269209 | -2,12 | 1,5 | -3,62 | 0,000535 | 0,0253 | Distal Intergenic | -411234 | ENSG00000132872 | SYT4 |
| chr5 | 27640239 | 27640923 | -1,64 | 2,93 | -4,57 | 0,000539 | 0,0254 | Distal Intergenic | 167840 | ENSG00000250337 | PURPL |
| chr2 | 232273238 | 232273738 | -2,12 | 1,94 | -4,06 | 0,000539 | 0,0254 | Distal Intergenic | 12903 | ENSG00000 156966 | B3 GNT7 |
| chr2 | 211568845 | 211569125 | -2,12 | 1,56 | -3,67 | 0,000541 | 0,0254 | Distal Intergenic | 86550 | ENSG00000280837 | CPS1-IT1 |
| chr13 | 24721621 | 24722290 | -1,83 | 1,98 | -3,81 | 0,000542 | 0,0254 | Intron (uc001upd.3/221178, intron 3 of 14) | -12571 | ENSG00000182957 | SPATA13 |
| chr7 | 73876902 | 73878096 | -1,14 | 3,06 | -4,21 | 0,000549 | 0,0257 | Intron (uc003uap.3/9569, intron 1 of 26) | 8782 | ENSG00000006704 | GTF2IRD1 |
| chr8 | 125381243 | 125385971 | 9,48 | 10,2 | -0,72 | 0,000549 | 0,0257 | Promoter (<=1kb) | 0 | ENSG00000164983 | TMEM65 |
| chr8 | 116543562 | 116544231 | -2,12 | 2,36 | -4,48 | 0,000551 | 0,0258 | Intron (uc011lhy.2/7227, intron 4 of 5) | 136003 | ENSG00000104447 | TRPS1 |
| chr7 | 54773337 | 54773891 | -2,12 | 2,03 | -4,14 | 0,000551 | 0,0258 | Distal Intergenic | 53048 | ENSG00000132432 | SEC61G |
| chr7 | 119659833 | 119660503 | -2,12 | 2,17 | -4,29 | 0,000554 | 0,0258 | Distal Intergenic | -253219 | ENSG00000184408 | KCND2 |
| chr9 | 24015669 | 24016467 | -0,77 | 3,36 | -4,13 | 0,000554 | 0,0258 | Distal Intergenic | -189606 | ENSG00000107105 | ELAVL2 |
| chr3 | 82627835 | 82628718 | -2,12 | 2,13 | -4,25 | 0,000555 | 0,0259 | Distal Intergenic | -816885 | ENSG00000114480 | GBE1 |
| chr8 | 2556929 | 2560589 | 0,61 | 5,57 | -4,96 | 0,000558 | 0,0259 | Intron (uc003wqb.1/uc003wqb.1, intron 2 of 2) | 484898 | ENSG00000183117 | CSMD1 |
| chr8 | 92785022 | 92785976 | -1,28 | 3,11 | -4,39 | 0,000558 | 0,0259 | Distal Intergenic | 218141 | ENSG00000079102 | RUNX1T1 |
| chr2 | 236208748 | 236209782 | -2,12 | 2,26 | -4,38 | 0,000558 | 0,0259 | Distal Intergenic | -192951 | ENSG00000157985 | AGAP1 |
| chr3 | 166146462 | 166147124 | -2,12 | 2,34 | -4,46 | 0,000559 | 0,0259 | Distal Intergenic | -591209 | ENSG00000114200 | BCHE |
| chr20 | 2689676 | 2690672 | -0,74 | 3,34 | -4,07 | 0,00056 | 0,026 | Exon (uc002wgs.4/57593, exon 7 of 18) | 16152 | ENSG00000088881 | EBF4 |
| chr10 | 990411 | 990772 | -2,12 | 1,79 | -3,9 | 0,000561 | 0,026 | Distal Intergenic | -12766 | ENSG00000107929 | LARP4B |
| chr20 | 16550080 | 16550922 | -2,12 | 2,29 | -4,4 | 0,000562 | 0,026 | Intron (uc002wpg.2/55614, intron 1 of 25) | 3157 | ENSG00000089177 | KIF16B |
| chr5 | 27943527 | 27944031 | -2,12 | 1,99 | -4,11 | 0,000562 | 0,026 | Distal Intergenic | 471128 | ENSG00000250337 | PURPL |
| chr7 | 144545746 | 144546379 | -2,12 | 2,03 | -4,15 | 0,000563 | 0,026 | Distal Intergenic | -12600 | ENSG00000196511 | TPK1 |
| chr1 1 | 37498601 | 37499152 | -2,12 | 2 | -4,12 | 0,000569 | 0,0262 | Distal Intergenic | -878772 | ENSG00000175097 | RAG2 |
| chr18 | 61874535 | 61876145 | 0,87 | 5,41 | -4,54 | 0,000574 | 0,0264 | Intron (uc002Ljx.3/284294, intron 2 of 9) | 51145 | ENSG00000266952 | LINC01538 |
| chr1 | 220854736 | 220855189 | -1,52 | 2,57 | -4,09 | 0,000574 | 0,0264 | Distal Intergenic | -8439 | ENSG00000162817 | C1orf115 |
| chr2 | 189813332 | 189814628 | -0,54 | 3,92 | -4,46 | 0,000575 | 0,0264 | Distal Intergenic | -24471 | ENSG00000168542 | COL3 A1 |
| chr9 | 8822448 | 8823143 | -2,12 | 2,05 | -4,17 | 0,000575 | 0,0264 | Intron (uc003zkk.3/5789, intron 11 of 45) | -88502 | ENSG00000153707 | PTPRD |
| chr2 | 40332603 | 40333395 | -2,12 | 2 | -4,12 | 0,000576 | 0,0264 | Intron (uc002rrw.3/100128590, intron 3 of 4) | 187829 | ENSG00000227028 | SLC8A1-AS1 |
| chr19 | 56692879 | 56693476 | -2,12 | 1,85 | -3,97 | 0,000576 | 0,0264 | Intron (uc002qmo.1/85569, intron 3 of 5) | 5490 | ENSG00000197487 | GALP |
| chr1 | 154291072 | 154291535 | -1,64 | 1,74 | -3,38 | 0,000576 | 0,0264 | Promoter (2-3kb) | -2057 | ENSG00000143595 | AQP10 |
| chrX | 29801112 | 29801612 | -1,12 | 2,2 | -3,33 | 0,000576 | 0,0264 | Intron (uc004dby.2/11141, intron 6 of 10) | -432063 | ENSG00000099399 | MAGEB2 |
| chr3 | 6089460 | 6089917 | -1,44 | 2,2 | -3,64 | 0,000579 | 0,0265 | Distal Intergenic | -797520 | ENSG00000265180 | MIR4790 |
| chr1 | 117907918 | 117914867 | 9,29 | 10,13 | -0,85 | 0,000579 | 0,0265 | Promoter (<=1kb) | 0 | ENSG00000198162 | MAN1A2 |
| chr4 | 179006873 | 179007560 | -2,12 | 2,12 | -4,24 | 0,00058 | 0,0265 | Distal Intergenic | 356962 | ENSG00000231171 | LINC01098 |
| chr10 | 1650975 | 1651473 | -1,48 | 2,24 | -3,72 | 0,000581 | 0,0265 | Intron (uc009xhq.3/105, intron 1 of 9) | 82150 | ENSG00000205696 | ADARB2-AS1 |
| chr4 | 149788593 | 149789964 | 0,45 | 4,93 | -4,48 | 0,000582 | 0,0265 | Distal Intergenic | -424921 | ENSG00000151623 | NR3C2 |
| chr3 | 194800416 | 194802149 | -0,66 | 4,01 | -4,67 | 0,000583 | 0,0266 | Intron (uc003fuk.3/152002, intron 1 of 1) | 40775 | ENSG00000173950 | XXYLT1 |
| chr4 | 189749569 | 189750375 | -2,12 | 2,07 | -4,19 | 0,000584 | 0,0266 | Distal Intergenic | 372837 | ENSG00000249378 | LINC01060 |
| chr1 | 190816409 | 190816877 | -1,91 | 2,15 | -4,06 | 0,000584 | 0,0266 | Distal Intergenic | 222389 | ENSG00000231175 | LINC0 1720 |
| chr18 | 75639270 | 75640071 | -1,19 | 2,7 | -3,88 | 0,000585 | 0,0266 | Distal Intergenic | 677262 | ENSG00000166573 | GALR1 |
| chr8 | 23591854 | 23592175 | -1,83 | 1,72 | -3,55 | 0,000589 | 0,0267 | Distal Intergenic | -27743 | ENSG00000180053 | NKX2-6 |
| chr5 | 25434917 | 25435536 | -2,12 | 2,07 | -4,19 | 0,00059 | 0,0268 | Distal Intergenic | -594225 | ENSG00000248908 | LINC02239 |
| chr4 | 128980780 | 128984962 | 9,09 | 9,9 | -0,81 | 0,000591 | 0,0268 | Promoter (<=1kb) | 0 | ENSG00000138709 | LARP1B |
| chr7 | 82135889 | 82136572 | -2,12 | 2,31 | -4,43 | 0,000595 | 0,0269 | Distal Intergenic | -62858 | ENSG00000153956 | CACNA2D1 |
| chr4 | 31624767 | 31625252 | -2,12 | 2,07 | -4,18 | 0,000595 | 0,0269 | Distal Intergenic | 902730 | ENSG00000169851 | PCDH7 |
| chr4 | 133737501 | 133740129 | 1,11 | 6,04 | -4,93 | 0,000598 | 0,0269 | Distal Intergenic | -330341 | ENSG00000138650 | PCDH10 |
| chr21 | 21816161 | 21816608 | -2,12 | 2,23 | -4,34 | 0,000598 | 0,0269 | Distal Intergenic | 358818 | ENSG00000224924 | LINC00320 |
| chr13 | 107150632 | 107151793 | -0,9 | 2,93 | -3,84 | 0,000598 | 0,0269 | Intron (uc001vqi.3/1948, intron 2 of 4) | 35595 | ENSG00000125266 | EFNB2 |
| chr21 | 19855408 | 19855741 | -2,12 | 1,57 | -3,69 | 0,000598 | 0,0269 | Distal Intergenic | -79438 | ENSG00000154646 | TMPRSS15 |
| chr10 | 98923468 | 98924219 | -2,12 | 2,06 | -4,18 | 0,000599 | 0,0269 | Intron (uc001kmw.2/6585, intron 2 of 36) | 21464 | ENSG00000187122 | SLIT1 |
| chr1 1 | 64917566 | 64918766 | -0,65 | 3,45 | -4,11 | 0,000599 | 0,0269 | Distal Intergenic | -15563 | ENSG00000162298 | SYVN1 |
| chr5 | 26237236 | 26237946 | -1,64 | 2,68 | -4,32 | 6,00E-04 | 0,0269 | Distal Intergenic | 652996 | ENSG00000113100 | CDH9 |
| chr7 | 51906280 | 51907592 | 0,2 | 4,24 | -4,04 | 6,00E-04 | 0,0269 | Distal Intergenic | -521765 | ENSG00000106078 | COBL |
| chr1 | 78956450 | 78962968 | 2,95 | 7,11 | -4,16 | 0,000603 | 0,027 | Promoter (<=1kb) | 0 | ENSG00000122420 | PTGFR |
| chrX | 2886240 | 2886631 | -2,12 | 1,62 | -3,73 | 0,000603 | 0,027 | Promoter (<=1kb) | 0 | ENSG00000157399 | ARSE |
| chr15 | 74906047 | 74910664 | 9,58 | 10,26 | -0,68 | 0,000604 | 0,027 | Promoter (<=1kb) | 0 | ENSG00000179335 | CLK3 |
| chr3 | 165899574 | 165899993 | -2,12 | 1,86 | -3,97 | 0,000606 | 0,0271 | Distal Intergenic | -344321 | ENSG00000114200 | BCHE |
| chr5 | 18480190 | 18480768 | -2,12 | 1,96 | -4,07 | 0,000611 | 0,0273 | Distal Intergenic | -1E+06 | ENSG00000250822 | LINC02111 |
| chr2 | 183794953 | 183795918 | -2,12 | 2,3 | -4,41 | 0,000612 | 0,0273 | Exon (uc002upb.4/10787, exon 28 of 32) | -63455 | ENSG00000162998 | FRZB |
| chr8 | 14056737 | 14057401 | -2,12 | 2,3 | -4,42 | 0,000613 | 0,0273 | Intron (uc010lss.3/137868, intron 2 of 5) | 355034 | ENSG00000185053 | SGCZ |
| chr10 | 3695096 | 3695487 | -1,86 | 1,75 | -3,6 | 0,000614 | 0,0273 | Intron (uc001igy.1/uc001igy.1 intron 1 of 1) | 131986 | ENSG00000067082 | KLF6 |
| chr6 | 9254310 | 9254912 | -2,12 | 1,96 | -4,08 | 0,000615 | 0,0274 | Distal Intergenic | 601868 | NA | HULC |
| chr2 | 223932930 | 223933597 | -2,12 | 2,07 | -4,18 | 0,000621 | 0,0276 | Distal Intergenic | 16282 | ENSG00000152049 | KCNE4 |
| chr12 | 47617288 | 47617816 | -1,64 | 2,52 | -4,15 | 0,000621 | 0,0276 | Intron (uc001rpn.3/91523, intron 3 of 3) | -7062 | ENSG00000247774 | PCED1B-AS1 |
| chr1 | 227867246 | 227868257 | -0,83 | 3,56 | -4,39 | 0,000622 | 0,0276 | Intron (uc009xeu.2/339500, intron 4 of 4) | 17151 | NA | ZNF847P |
| chr7 | 76757397 | 76758301 | -2,12 | 1,63 | -3,75 | 0,000622 | 0,0276 | Intron (uc003uga.3/57639, intron 1 of 18) | 5463 | ENSG00000135205 | CCDC146 |
| chr9 | 39154438 | 39155212 | -0,71 | 2,73 | -3,44 | 0,000622 | 0,0276 | Intron (uc004abi.3/79937, intron 9 of 23) | 133088 | ENSG00000106714 | CNTNAP3 |
| chr2 | 189515217 | 189515930 | -2,12 | 2,03 | -4,15 | 0,000623 | 0,0276 | Distal Intergenic | 80462 | ENSG00000144366 | GULP1 |
| chr12 | 10578373 | 10578810 | -2,12 | 1,73 | -3,85 | 0,000624 | 0,0276 | Intron (uc001qyh.3/3823, intron 3 of 6) | -5179 | ENSG00000205810 | KLRC3 |
| chr4 | 99914593 | 99918837 | 8,54 | 9,4 | -0,86 | 0,000624 | 0,0276 | Promoter (<=1kb) | 0 | ENSG00000164024 | METAP1 |
| chr8 | 87834080 | 87835695 | -0,13 | 4,55 | -4,67 | 0,000625 | 0,0276 | Distal Intergenic | -42981 | ENSG00000176571 | CNBD1 |
| chr13 | 59040634 | 59041463 | -1,01 | 2,93 | -3,94 | 0,000625 | 0,0276 | Distal Intergenic | 799847 | ENSG00000118946 | PCDH17 |
| chr1 1 | 101756291 | 101756945 | -1,22 | 2,82 | -4,04 | 0,000626 | 0,0276 | Distal Intergenic | -28801 | ENSG00000110318 | CEP126 |
| chr19 | 49912576 | 49913064 | -2,12 | 1,89 | -4,01 | 0,000626 | 0,0276 | Exon (uc002pnm.2/147872, exon 15 of 20) | 13634 | ENSG00000142538 | PTH2 |
| chr7 | 86004574 | 86005457 | -1,57 | 2,44 | -4 | 0,000627 | 0,0276 | Distal Intergenic | -267773 | ENSG00000198822 | GRM3 |
| chr3 | 164987482 | 164988282 | -2,12 | 2,45 | -4,56 | 0,000628 | 0,0276 | Distal Intergenic | -73013 | ENSG00000121871 | SLITRK3 |
| chr17 | 12750502 | 12751083 | -2,12 | 1,63 | -3,75 | 0,000628 | 0,0276 | Intron (uc002gnr.4/9912, intron 1 of 20) | 57673 | ENSG00000006740 | ARHGAP44 |
| chr2 | 141183713 | 141187264 | 0,58 | 5,28 | -4,7 | 0,000629 | 0,0276 | Intron (uc002tvj.1/53353, intron 66 of 90) | 1528819 | NA | YY1P2 |
| chr17 | 29697676 | 29698738 | -2,12 | 2,37 | -4,48 | 0,000631 | 0,0277 | Intron (uc002hgg.3/4763, intron 57 of 57) | -19904 | ENSG00000131242 | RAB11FIP4 |
| chr18 | 74290885 | 74291210 | -2,12 | 1,68 | -3,8 | 0,000632 | 0,0277 | Distal Intergenic | 50273 | ENSG00000266256 | LINC00908 |
| chr9 | 92270610 | 92271418 | -1,04 | 3,26 | -4,3 | 0,000634 | 0,0277 | Intron (uc004aqs.2/100129066, intron 1 of 5) | 15912 | ENSG00000237372 | UNQ6494 |
| chr5 | 154547716 | 154548924 | -0,99 | 3,01 | -4 | 0,000634 | 0,0277 | Distal Intergenic | 154456 | ENSG00000226650 | KIF4B |
| chr20 | 55892370 | 55892800 | -1,83 | 1,78 | -3,61 | 0,000634 | 0,0277 | Distal Intergenic | 3847 | ENSG00000266666 | MIR4325 |
| chr5 | 18539143 | 18540127 | -2,12 | 2,05 | -4,16 | 0,000636 | 0,0278 | Distal Intergenic | -1E+06 | ENSG00000250822 | LINC02111 |
| chr2 | 105829877 | 105831156 | -1,02 | 2,29 | -3,31 | 0,000637 | 0,0278 | Distal Intergenic | -27044 | ENSG00000135973 | GPR45 |
| chr1 | 43078601 | 43079458 | -0,2 | 3,94 | -4,15 | 0,000638 | 0,0278 | Intron (uc001chm.2/728621, intron 17 of 22) | -44590 | ENSG00000171960 | PPIH |
| chr1 1 | 73307598 | 73310709 | 8,64 | 9,62 | -0,98 | 0,000639 | 0,0279 | Promoter (<=1kb) | 0 | ENSG00000054965 | FAM168A |
| chr9 | 124553197 | 124553584 | -1,59 | 1,97 | -3,56 | 0,000641 | 0,0279 | Distal Intergenic | 22486 | ENSG00000136848 | DAB2IP |
| chr21 | 20201758 | 20202191 | -2,12 | 1,81 | -3,93 | 0,000642 | 0,0279 | Distal Intergenic | -425788 | ENSG00000154646 | TMPRSS15 |
| chr7 | 46568870 | 46569665 | -1,12 | 2,48 | -3,6 | 0,000643 | 0,028 | Distal Intergenic | -607999 | ENSG00000146674 | IGFBP3 |
| chr4 | 166531872 | 166532439 | -1,83 | 1,9 | -3,73 | 0,000644 | 0,028 | Distal Intergenic | 224478 | ENSG00000207559 | MIR578 |
| chr3 | 125451261 | 125451934 | -1,91 | 1,62 | -3,53 | 0,000646 | 0,028 | Distal Intergenic | 57461 | ENSG00000221737 | MIR548I1 |
| chr1 | 211160908 | 211161676 | -2,12 | 1,75 | -3,87 | 0,000647 | 0,028 | Intron (uc001hib.2/3756, intron 6 of 10) | 145781 | ENSG00000143473 | KCNH1 |
| chr2 | 104147714 | 104148423 | -1,09 | 2,58 | -3,67 | 0,000647 | 0,028 | Distal Intergenic | 733489 | ENSG00000170417 | TMEM182 |
| chr18 | 13641588 | 13642110 | -2,12 | 2,26 | -4,38 | 0,00065 | 0,0281 | Promoter (<=1kb) | 0 | ENSG00000168675 | LDLRAD4 |
| chr19 | 3451142 | 3451622 | -2,12 | 1,78 | -3,9 | 0,00065 | 0,0281 | Intron (uc002lxo.3/4782, intron 7 of 10) | 28918 | ENSG00000095932 | SMIM24 |
| chr4 | 135751099 | 135751424 | -2,12 | 1,48 | -3,6 | 0,00065 | 0,0281 | Distal Intergenic | -628196 | ENSG00000254535 | PABPC4L |
| chr15 | 66584639 | 66588641 | 8,81 | 9,59 | -0,79 | 0,000651 | 0,0281 | Promoter (<=1kb) | 0 | ENSG00000166938 | DIS3L |
| chr14 | 88354585 | 88355470 | 0,08 | 3,88 | -3,81 | 0,000655 | 0,0282 | Intron (uc010tvw.1/2581, intron 13 of 13) | 91522 | ENSG00000054983 | GALC |
| chr7 | 14236445 | 14237316 | -0,54 | 3,17 | -3,71 | 0,000658 | 0,0283 | Intron (uc003ssz.3/1607, intron 22 of 24) | -205395 | ENSG00000006468 | ETV1 |
| chr5 | 124059848 | 124061037 | -0,65 | 3,67 | -4,32 | 0,00066 | 0,0284 | Intron (uc003ktq.1/57507, intron 1 of 8) | 19828 | ENSG00000168916 | ZNF608 |
| chr13 | 107355385 | 107355993 | -1,59 | 2,42 | -4,01 | 0,000662 | 0,0284 | Distal Intergenic | 49157 | ENSG00000230156 | LINC00443 |
| chr15 | 20076127 | 20077480 | -0,45 | 4,19 | -4,64 | 0,000663 | 0,0284 | Distal Intergenic | -410517 | ENSG00000259156 | CHEK2P2 |
| chr19 | 35953709 | 35954086 | -1,77 | 1,61 | -3,37 | 0,000664 | 0,0285 | Distal Intergenic | 13092 | ENSG00000126262 | FFAR2 |
| chr1 1 | 106321218 | 106321888 | -1,83 | 2,41 | -4,24 | 0,000666 | 0,0285 | Distal Intergenic | 359941 | ENSG00000149313 | AASDHPPT |
| chr15 | 76134400 | 76139386 | 9,41 | 10,13 | -0,72 | 0,000666 | 0,0285 | Promoter (<=1kb) | 0 | ENSG00000140367 | UBE2Q2 |
| chr15 | 53881003 | 53881828 | -2,12 | 2,04 | -4,16 | 0,000668 | 0,0285 | Intron (uc002acj.2/256764, intron 18 of 19) | -47703 | ENSG00000166415 | WDR7 2 |
| chr6 | 12426906 | 12427520 | -1,75 | 2,22 | -3,97 | 0,000668 | 0,0285 | Distal Intergenic | 136377 | ENSG00000078401 | EDN1 |
| chr14 | 35811001 | 35811479 | -1,77 | 2,45 | -4,21 | 0,000671 | 0,0286 | Distal Intergenic | 49344 | ENSG00000100902 | PSMA6 |
| chr19 | 4693775 | 4694225 | -2,12 | 2,14 | -4,25 | 0,000676 | 0,0288 | Intron (uc002mba.3/91039, intron 13 of 21) | 14481 | ENSG00000205790 | DPP9-AS1 |
| chr7 | 77669416 | 77670046 | -2,12 | 2,15 | -4,26 | 0,000679 | 0,0288 | Intron (uc003ugx.3/9863, intron 21 of 21) | 131306 | ENSG00000006576 | PHTF2 |
| chr4 | 162266955 | 162267600 | -2,12 | 1,97 | -4,08 | 0,000679 | 0,0288 | Distal Intergenic | 817586 | ENSG00000168843 | FSTL5 |
| chr1 1 | 44617606 | 44619458 | 0,61 | 5,13 | -4,52 | 0,00068 | 0,0288 | Intron (uc001myc.3/3732, intron 3 of 9) | 30465 | ENSG00000085117 | CD82 |
| chr1 1 | 78060911 | 78062080 | -1,39 | 2,86 | -4,25 | 0,00068 | 0,0288 | Intron (uc001ozh.3/9846, intron 1 of 9) | -7985 | ENSG00000033327 | GAB2 |
| chr4 | 137603314 | 137609581 | 1,11 | 5,83 | -4,71 | 0,000681 | 0,0288 | Distal Intergenic | 844048 | ENSG00000189184 | PCDH18 |
| chr8 | 78230993 | 78231625 | -1,52 | 2,6 | -4,12 | 0,000681 | 0,0288 | Distal Intergenic | -317713 | ENSG00000164751 | PEX2 |
| chr1 | 7295344 | 7295872 | -2,12 | 1,86 | -3,98 | 0,000681 | 0,0288 | Intron (uc001aoi.3/23261, intron 4 of 22) | -436182 | ENSG00000171735 | CAMTA1 |
| chr8 | 112674600 | 112675005 | -2,12 | 2,03 | -4,15 | 0,000682 | 0,0288 | Distal Intergenic | 674955 | ENSG00000164796 | CSMD3 |
| chr12 | 129488142 | 129488891 | -0,52 | 2,92 | -3,44 | 0,000682 | 0,0288 | Distal Intergenic | 81654 | ENSG00000151952 | TMEM132D |
| chr4 | 131601216 | 131601912 | -1,39 | 3,01 | -4,4 | 0,000686 | 0,029 | Distal Intergenic | 1583934 | ENSG00000151470 | C4orf33 |
| chr4 | 31774064 | 31774778 | -2,12 | 2,23 | -4,35 | 0,000692 | 0,0292 | Distal Intergenic | 1052027 | ENSG00000169851 | PCDH7 |
| chr12 | 54404558 | 54405698 | -0,46 | 3,59 | -4,05 | 0,000693 | 0,0292 | Promoter (1-2kb) | 1668 | ENSG00000037965 | HOXC8 |
| chr13 | 31406661 | 31407535 | -1,21 | 2,78 | -3,98 | 0,000697 | 0,0293 | Distal Intergenic | 29318 | ENSG00000237879 | LINC00398 |
| chr3 | 174259631 | 174259987 | -2,12 | 1,65 | -3,77 | 0,000705 | 0,0296 | Intron (uc003fir.3/uc003fir.3, intron 2 of 3) | -317124 | ENSG00000177694 | NAALADL2 |
| chr1 | 211255904 | 211256568 | -1,38 | 2,31 | -3,69 | 0,000705 | 0,0295 | Exon (uc001hib.2/3756, exon 5 of 11) | 50889 | ENSG00000143473 | KCNH1 |
| chr1 | 65531264 | 65535142 | 8,97 | 9,93 | -0,96 | 0,000709 | 0,0297 | Distal Intergenic | -7073 | ENSG00000199135 | MIR101-1 |
| chr12 | 18449802 | 18450168 | -0,9 | 2,52 | -3,42 | 0,00071 | 0,0297 | Intron (uc001rdt.3/5288, intron 5 of 31) | 14864 | ENSG00000139144 | PIK3 C2G |
| chr12 | 96789381 | 96796780 | 9,63 | 10,36 | -0,73 | 0,000711 | 0,0297 | Promoter (<=1kb) | 0 | ENSG00000059758 | CDK17 |
| chr21 | 19737914 | 19738482 | -1,48 | 2,6 | -4,09 | 0,000714 | 0,0298 | Intron (uc002vkw.3/5651, intron 6 of 24) | 37488 | ENSG00000154646 | TMPRSS15 |
| chr5 | 19792028 | 19792618 | -2,12 | 2,21 | -4,33 | 0,000715 | 0,0298 | Intron (uc021xwu.1/1016, intron 3 of 12) | 93763 | ENSG00000145526 | CDH18 |
| chr3 | 163181246 | 163181590 | -2,12 | 1,82 | -3,94 | 0,000719 | 0,03 | Distal Intergenic | -160157 | ENSG00000241369 | LINC01192 |
| chr12 | 10095556 | 10096477 | -0,77 | 3,43 | -4,2 | 0,000721 | 0,03 | Distal Intergenic | -7438 | ENSG00000172322 | CLEC12A |
| chr9 | 30347233 | 30350423 | 2,19 | 6,69 | -4,51 | 0,000722 | 0,0301 | Distal Intergenic | 58029 | NA | LINC01242 |
| chr15 | 68518819 | 68523927 | 9,44 | 10,11 | -0,68 | 0,000724 | 0,0301 | Promoter (<=1kb) | 0 | ENSG00000128973 | CLN6 |
| chr4 | 82157384 | 82158719 | -1,06 | 3,01 | -4,07 | 0,000726 | 0,0301 | Distal Intergenic | -21166 | ENSG00000138669 | PRKG2 |
| chr10 | 36434073 | 36434811 | -1,22 | 2,79 | -4 | 0,000726 | 0,0301 | Distal Intergenic | -503711 | ENSG00000177283 | FZD8 |
| chr5 | 111326841 | 111328244 | 0,57 | 4,5 | -3,93 | 0,000727 | 0,0301 | Intron (uc031skr.1/100873948, intron 3 of 3) | -14213 | ENSG00000134986 | NREP |
| chr6 | 122417967 | 122418578 | -2,12 | 1,89 | -4,01 | 0,000729 | 0,0302 | Distal Intergenic | -302118 | ENSG00000025156 | HSF2 |
| chr12 | 66122809 | 66123354 | -2,12 | 1,85 | -3,97 | 0,000729 | 0,0302 | Distal Intergenic | -94083 | ENSG00000149948 | HMGA2 |
| chr13 | 98381858 | 98383547 | 0,82 | 4,68 | -3,86 | 0,000729 | 0,0302 | Distal Intergenic | -222382 | ENSG00000065150 | IPO5 |
| chr2 | 189439505 | 189440113 | -2,12 | 2,1 | -4,22 | 0,00073 | 0,0302 | Intron (uc002uqd.3/51454, intron 9 of 12) | 4750 | ENSG00000144366 | GULP1 |
| chr6 | 8182443 | 8183190 | -0,95 | 3,03 | -3,98 | 0,000731 | 0,0302 | Distal Intergenic | -79615 | ENSG00000124802 | EEF1E1 |
| chr2 | 141367965 | 141368545 | -2,12 | 2,19 | -4,31 | 0,000732 | 0,0302 | Intron (uc002tvj.1/53353, intron 41 of 90) | 1520725 | ENSG00000168702 | LRP1B |
| chr5 | 106037990 | 106038830 | -2,12 | 2,19 | -4,31 | 0,000732 | 0,0302 | Distal Intergenic | 967766 | ENSG00000184349 | EFNA5 |
| chr6 | 68272452 | 68273072 | -2,12 | 1,91 | -4,02 | 0,000732 | 0,0302 | Distal Intergenic | -1E+06 | ENSG00000135298 | ADGRB3 |
| chr15 | 21926431 | 21936246 | 1,86 | 7,73 | -5,87 | 0,000734 | 0,0302 | Exon (uc010tzj.1/646214, exon 1 of 1) | 4493 | NA | LOC646214 |
| chr8 | 116458137 | 116458970 | -2,12 | 1,88 | -3,99 | 0,000735 | 0,0302 | Intron (uc011lhv.2/7227, intron 4 of 5) | 221264 | ENSG00000104447 | TRPS1 |
| chr10 | 18522645 | 18523904 | -0,72 | 3,37 | -4,09 | 0,000736 | 0,0302 | Intron (uc001ipr.2/783, intron 2 of 13) | -25680 | ENSG00000165995 | CACNB2 |
| chr4 | 95941602 | 95942041 | -2,12 | 1,62 | -3,74 | 0,000736 | 0,0303 | Intron (uc003htm.4/658, intron 3 of 12) | 24219 | ENSG00000138696 | BMPR1B |
| chr6 | 141497574 | 141498136 | -2,12 | 2,01 | -4,13 | 0,000739 | 0,0303 | Distal Intergenic | 492623 | ENSG00000264390 | MIR4465 |
| chr8 | 18708771 | 18709515 | -2,12 | 1,86 | -3,98 | 0,00074 | 0,0304 | Intron (uc003wza.3/23362, intron 4 of 15) | -42366 | ENSG00000156011 | PSD3 |
| chr12 | 19565488 | 19566398 | 0,37 | 3,91 | -3,54 | 0,000743 | 0,0304 | Distal Intergenic | -26210 | ENSG00000139154 | AEBP2 |
| chr1 | 196416162 | 196416903 | -2,12 | 2,19 | -4,3 | 0,000745 | 0,0304 | Intron (uc009wvt.1/343450, intron 2 of 20) | 20013 | ENSG00000162687 | KCNT2 |
| chr2 | 43369774 | 43370217 | -2,12 | 1,72 | -3,84 | 0,000745 | 0,0304 | Distal Intergenic | 83528 | ENSG00000152518 | ZFP36L2 |
| chr3 | 114864157 | 114867885 | 8,91 | 9,73 | -0,82 | 0,000745 | 0,0304 | Promoter (<=1kb) | 0 | ENSG00000181722 | ZBTB20 |
| chr4 | 167647723 | 167648564 | -2,12 | 2,18 | -4,29 | 0,000748 | 0,0305 | Distal Intergenic | 506760 | ENSG00000196104 | SPOCK3 |
| chr5 | 98542828 | 98543862 | -0,86 | 3,39 | -4,25 | 0,000752 | 0,0307 | Distal Intergenic | 277990 | NA | LOC1002892 30 |
| chr2 | 67551586 | 67552008 | -2,12 | 1,68 | -3,8 | 0,000752 | 0,0307 | Distal Intergenic | -72434 | ENSG00000143971 | ETAA1 |
| chr3 | 173400734 | 173401336 | -2,12 | 1,68 | -3,8 | 0,000753 | 0,0307 | Intron (uc003fio.1/22871, intron 3 of 6) | 78665 | ENSG00000169760 | NLGN1 |
| chr6 | 39614402 | 39615088 | -1,64 | 2,26 | -3,89 | 0,000754 | 0,0307 | Intron (uc003oot.2/221458, intron 3 of 22) | -6869 | ENSG00000164627 | KIF6 |
| chr4 | 72903093 | 72903743 | -2,12 | 1,82 | -3,94 | 0,000755 | 0,0307 | Promoter (1-2kb) | -1106 | ENSG00000056291 | NPFFR2 |
| chr4 | 167055669 | 167056070 | -1,41 | 2,08 | -3,49 | 0,000758 | 0,0307 | Distal Intergenic | 261259 | ENSG00000038295 | TLL1 |
| chr20 | 14812253 | 14812989 | -2,12 | 2,33 | -4,45 | 0,000762 | 0,0308 | Intron (uc002wot.3/140733, intron 5 of 16) | 97172 | ENSG00000235914 | MACROD2 - AS1 |
| chr9 | 15054824 | 15056069 | 0,12 | 4,34 | -4,22 | 0,000763 | 0,0308 | Intron (uc003zln.1/uc003zln.1, intron 3 of 9) | 61499 | NA | LOC389705 |
| chr1 | 89354387 | 89358907 | 8,11 | 9,05 | -0,94 | 0,000763 | 0,0308 | Promoter (<=1kb) | 0 | ENSG00000137947 | GTF2B |
| chr4 | 117271291 | 117271852 | -2,12 | 1,92 | -4,03 | 0,000767 | 0,031 | Distal Intergenic | 50410 | ENSG00000284253 | MIR1973 |
| chr8 | 3221987 | 3222705 | -2,12 | 2,07 | -4,19 | 0,000768 | 0,031 | Intron (uc011kwj.2/64478, intron 8 of 55) | 31198 | ENSG00000183117 | CSMD1 |
| chr10 | 19042294 | 19042759 | -2,12 | 2 | -4,11 | 0,000768 | 0,031 | Distal Intergenic | 93981 | ENSG00000165997 | ARL5B |
| chr5 | 19865307 | 19866914 | -0,17 | 4,47 | -4,64 | 0,000773 | 0,0311 | Intron (uc021xwu.1/1016, intron 2 of 12) | 19467 | ENSG00000145526 | CDH18 |
| chr4 | 32604246 | 32604755 | -2,12 | 1,96 | -4,08 | 0,000775 | 0,0312 | Distal Intergenic | 1882209 | ENSG00000169851 | PCDH7 |
| chr17 | 20369345 | 20371311 | -1,36 | 2,38 | -3,74 | 0,000775 | 0,0312 | Promoter (<=1kb) | 0 | ENSG00000170298 | LGALS9B |
| chr16 | 10803228 | 10804240 | -1,28 | 2,93 | -4,2 | 0,000776 | 0,0312 | Distal Intergenic | -14426 | ENSG00000153060 | TEKT5 |
| chr3 | 69493888 | 69494611 | 0,21 | 4,08 | -3,88 | 0,000777 | 0,0312 | Intron (uc003dnw.2/23150, intron 1 of 4) | -58458 | ENSG00000114541 | FRMD4B |
| chr15 | 47388142 | 47388802 | -2,12 | 2,16 | -4,27 | 0,000778 | 0,0312 | Distal Intergenic | -87601 | ENSG00000137872 | SEMA6D |
| chr2 | 6921022 | 6921884 | -1,73 | 2,35 | -4,08 | 0,000778 | 0,0312 | Distal Intergenic | -10580 | ENSG00000205837 | LINC00487 |
| chr8 | 40617516 | 40618122 | -2,12 | 1,63 | -3,75 | 0,000782 | 0,0313 | Intron (uc003xnr.3/79698, intron 3 of 6) | 137221 | ENSG00000165061 | ZMAT4 |
| chr3 | 165090027 | 165090733 | -2,12 | 2,29 | -4,41 | 0,000783 | 0,0313 | Distal Intergenic | -175558 | ENSG00000121871 | SLITRK3 |
| chr4 | 167797087 | 167797614 | -2,12 | 1,93 | -4,05 | 0,000784 | 0,0314 | Intron (uc021xuf.1/50859, intron 6 of 10) | 357710 | ENSG00000196104 | SPOCK3 |
| chr17 | 13415468 | 13415895 | -1,83 | 1,93 | -3,76 | 0,000786 | 0,0314 | Intron (uc002gob.1/9955, intron 1 of 1) | 89349 | ENSG00000153976 | HS3ST3A1 |
| chr1 | 91176331 | 91176940 | -1,39 | 2,65 | -4,04 | 0,000787 | 0,0314 | Downstream (<1kb) | 5854 | ENSG00000143032 | BARHL2 |
| chr15 | 66677842 | 66684092 | 9,27 | 9,89 | -0,63 | 0,000789 | 0,0315 | Promoter (<=1kb) | 0 | ENSG00000169032 | MAP2K1 |
| chr1 | 12614195 | 12614608 | -1,46 | 1,93 | -3,39 | 0,00079 | 0,0315 | Distal Intergenic | 41498 | ENSG00000162496 | DHRS3 |
| chr8 | 112292376 | 112296269 | 0,77 | 5,76 | -5 | 0,000791 | 0,0315 | Distal Intergenic | 1053691 | ENSG00000164796 | CSMD3 |
| chr2 | 220126844 | 220127375 | -2,12 | 1,74 | -3,86 | 0,000791 | 0,0315 | Intron (uc002vku.3/80086, intron 1 of 3) | -7514 | ENSG00000127824 | TUBA4A |
| chr20 | 54319349 | 54320053 | -2,12 | 2,28 | -4,4 | 0,000792 | 0,0315 | Distal Intergenic | 260475 | ENSG00000054803 | CBLN4 |
| chr7 | 14686785 | 14688163 | -0,27 | 3,97 | -4,24 | 0,000793 | 0,0315 | Intron (uc003ssz.3/1607, intron 12 of 24) | 192912 | ENSG00000136267 | DGKB |
| chr15 | 100012797 | 100013893 | -0,75 | 3,76 | -4,51 | 0,000796 | 0,0316 | Distal Intergenic | -92240 | ENSG00000068305 | MEF2A |
| chr7 | 126844792 | 126845660 | 0,28 | 4,17 | -3,89 | 0,000797 | 0,0316 | Intron (uc003vlr.2/2918, intron 1 of 9) | 37909 | ENSG00000179603 | GRM8 |
| chr4 | 179333224 | 179334021 | -2,12 | 2,32 | -4,44 | 0,000799 | 0,0316 | Distal Intergenic | 683313 | ENSG00000231171 | LINC01098 |
| chr1 | 146649530 | 146649988 | -2,12 | 2,18 | -4,3 | 0,000799 | 0,0316 | Promoter (<=1kb) | 100 | NA | PDIA3P1 |
| chr9 | 133033097 | 133033663 | -1,21 | 2,35 | -3,56 | 0,000802 | 0,0317 | Distal Intergenic | 70225 | ENSG00000107130 | NCS1 |
| chr13 | 105077303 | 105078225 | -0,99 | 3,08 | -4,06 | 0,000806 | 0,0318 | Distal Intergenic | -1E+06 | ENSG00000182346 | DAOA |
| chr8 | 23380749 | 23381301 | -1,83 | 1,93 | -3,76 | 0,000811 | 0,032 | Distal Intergenic | -5062 | ENSG00000147454 | SLC25A37 |
| chr4 | 31670397 | 31671109 | -2,12 | 2,12 | -4,24 | 0,000812 | 0,032 | Distal Intergenic | 948360 | ENSG00000169851 | PCDH7 |
| chr10 | 28460125 | 28460701 | -2,12 | 1,8 | -3,92 | 0,000812 | 0,032 | Intron (uc009xkz.1/143098, intron 2 of 15) | 66963 | ENSG00000150054 | MPP7 |
| chr22 | 47589209 | 47589687 | -2,12 | 1,86 | -3,97 | 0,000815 | 0,0321 | Distal Intergenic | 419385 | ENSG00000054611 | TBC1D22A |
| chr13 | 85697207 | 85698321 | -1,11 | 2,71 | -3,82 | 0,000819 | 0,0322 | Distal Intergenic | -239417 | ENSG00000226317 | LINC00351 |
| chr4 | 181315461 | 181316260 | -1,48 | 2,63 | -4,12 | 0,00082 | 0,0322 | Distal Intergenic | 764042 | ENSG00000248197 | LINC00290 |
| chr1 1 | 90994457 | 90994954 | -2,12 | 1,84 | -3,96 | 0,000821 | 0,0322 | Distal Intergenic | -705432 | ENSG00000266703 | MIR4490 |
| chr1 1 | 112665929 | 112666552 | -1,21 | 2,6 | -3,8 | 0,000821 | 0,0322 | Distal Intergenic | -165417 | ENSG00000149294 | NCAM1 |
| chr2 | 18139108 | 18139709 | -2,12 | 2,27 | -4,38 | 0,000823 | 0,0323 | Distal Intergenic | 79163 | ENSG00000170745 | KCNS3 |
| chr19 | 41667550 | 41667932 | -2,12 | 1,59 | -3,7 | 0,000823 | 0,0323 | Distal Intergenic | -31183 | ENSG00000167600 | CYP2S1 |
| chr3 | 85463719 | 85464519 | -2,12 | 2,12 | -4,24 | 0,000828 | 0,0324 | Intron (uc003dqj.3/253559, intron 1 of 9) | 28859 | ENSG00000264084 | MIR5688 |
| chr2 | 35223080 | 35223721 | -2,12 | 1,82 | -3,94 | 0,000829 | 0,0324 | Distal Intergenic | -1E+06 | ENSG00000239649 | MYADML |
| chr8 | 25840567 | 25841377 | 0,88 | 4,46 | -3,58 | 0,000835 | 0,0326 | Intron (uc003xek.3/5520, intron 7 of 10) | 61263 | ENSG00000221818 | EBF2 |
| chr2 | 481255 | 481950 | -0,49 | 3,47 | -3,96 | 0,000836 | 0,0326 | Distal Intergenic | -192947 | ENSG00000189292 | ALKAL2 |
| chr2 | 107204008 | 107204594 | -2,12 | 1,82 | -3,93 | 0,000836 | 0,0326 | Distal Intergenic | -119207 | ENSG00000153165 | RGPD3 |
| chr20 | 21588764 | 21589215 | -2,12 | 1,8 | -3,92 | 0,000837 | 0,0326 | Distal Intergenic | -94100 | ENSG00000125820 | NKX2-2 |
| chr13 | 104874660 | 104875440 | -2,12 | 2,1 | -4,22 | 0,000839 | 0,0327 | Distal Intergenic | -939812 | ENSG00000263741 | MIR548AS |
| chr7 | 125359576 | 125360378 | -2,12 | 2,1 | -4,22 | 0,000839 | 0,0327 | Distal Intergenic | -789539 | ENSG00000128513 | POT1 |
| chr5 | 6788958 | 6789733 | -0,86 | 3,07 | -3,94 | 0,000847 | 0,0329 | Distal Intergenic | 38301 | ENSG00000283420 | MIR4278 |
| chr1 | 206272760 | 206273906 | -0,14 | 3,85 | -3,99 | 0,000848 | 0,0329 | Intron (uc001hdt.2/440712, intron 1 of 5) | 14330 | ENSG00000263961 | RHEX |
| chr12 | 120814063 | 120815542 | -1,04 | 3,22 | -4,26 | 0,000852 | 0,0331 | Distal Intergenic | -7080 | ENSG00000135097 | MSI1 |
| chr12 | 124604057 | 124604794 | -2,12 | 2,06 | -4,17 | 0,000853 | 0,0331 | Intron (uc021rfy.1/100533183, intron 2 of 4) | 146295 | ENSG00000179195 | ZNF664 |
| chr1 | 207032383 | 207032799 | -2,12 | 1,89 | -4,01 | 0,000856 | 0,0332 | Distal Intergenic | -6355 | ENSG00000162891 | IL20 |
| chr6 | 18415241 | 18416064 | -0,86 | 3,1 | -3,96 | 0,000857 | 0,0332 | Intron (uc003ncs.3/255488, intron 2 of 7) | 27660 | ENSG00000137393 | RNF144B |
| chr5 | 23306968 | 23307445 | -1,93 | 2,05 | -3,98 | 0,00086 | 0,0333 | Distal Intergenic | -200279 | ENSG00000164256 | PRDM9 |
| chr1 | 247450715 | 247450999 | -2,12 | 1,49 | -3,61 | 0,000861 | 0,0333 | Distal Intergenic | 22759 | ENSG00000162714 | ZNF496 |
| chr1 1 | 82324163 | 82324608 | -2,12 | 1,61 | -3,73 | 0,000863 | 0,0333 | Distal Intergenic | 120298 | ENSG00000182103 | FAM181B |
| chr6 | 9505134 | 9505693 | -1,83 | 1,9 | -3,73 | 0,000864 | 0,0333 | Distal Intergenic | 852692 | NA | HULC |
| chr12 | 110231998 | 110233156 | -1,11 | 2,8 | -3,91 | 0,000865 | 0,0333 | Exon (uc001tpg.2/59341, exon 8 of 16) | 19445 | ENSG00000111199 | TRPV4 |
| chr2 | 141297291 | 141297762 | -2,12 | 1,75 | -3,87 | 0,000865 | 0,0333 | Intron (uc002tvj.1/53353, intron 45 of 90) | 1591508 | ENSG00000168702 | LRP1B |
| chr4 | 188493168 | 188494109 | -0,9 | 2,88 | -3,78 | 0,000865 | 0,0333 | Distal Intergenic | -66401 | NA | LOC339975 |
| chr19 | 2454604 | 2457754 | 9,03 | 9,9 | -0,88 | 0,000866 | 0,0333 | Promoter (<=1kb) | 0 | ENSG00000176619 | LMNB2 |
| chr13 | 95481419 | 95482297 | -0,48 | 3,24 | -3,72 | 0,000869 | 0,0334 | Intron (uc001vmc.3/uc001vmc.3, intron 2 of 4) | -117030 | ENSG00000125285 | SOX21 |
| chrX | 146854505 | 146855104 | -1,83 | 2,07 | -3,9 | 0,000872 | 0,0335 | Distal Intergenic | -138365 | ENSG00000102081 | FMR1 |
| chr17 | 21427338 | 21427809 | -2,12 | 1,68 | -3,79 | 0,000873 | 0,0335 | Distal Intergenic | 27132 | ENSG00000212719 | LINC02693 |
| chr1 1 | 26118216 | 26118976 | -2,12 | 2,08 | -4,2 | 0,000874 | 0,0335 | Distal Intergenic | -91853 | ENSG00000134343 | ANO3 |
| chr1 | 240245352 | 240245769 | -2,12 | 1,66 | -3,77 | 0,000875 | 0,0335 | Distal Intergenic | -9416 | ENSG00000155816 | FMN2 |
| chr8 | 12573082 | 12573467 | -2,12 | 1,34 | -3,46 | 0,000879 | 0,0336 | Distal Intergenic | -11279 | ENSG00000283523 | MIR3926-2 |
| chr8 | 57014267 | 57014843 | -2,12 | 1,63 | -3,75 | 0,00088 | 0,0336 | Distal Intergenic | 11698 | ENSG00000172680 | MOS |
| chr3 | 183056323 | 183056836 | -1,86 | 2,08 | -3,93 | 0,000882 | 0,0337 | Exon (uc003fli.1/23101, exon 5 of 30) | -27444 | ENSG00000053524 | MCF2L2 |
| chr8 | 15794763 | 15795514 | -1,77 | 2,37 | -4,14 | 0,000884 | 0,0337 | Distal Intergenic | 193716 | ENSG00000104723 | TUSC3 |
| chr13 | 82421320 | 82421829 | -2,12 | 1,93 | -4,04 | 0,000884 | 0,0337 | Distal Intergenic | -2E+06 | ENSG00000136158 | SPRY2 |
| chr5 | 17013250 | 17013767 | -1,59 | 1,97 | -3,57 | 0,000886 | 0,0337 | Distal Intergenic | -76865 | ENSG00000145555 | MYO10 |
| chr4 | 1719046 | 1726657 | 9,19 | 10,24 | -1,05 | 0,000889 | 0,0338 | Promoter (<=1kb) | 0 | ENSG00000168936 | TMEM129 |
| chr15 | 75992511 | 75993007 | -2,12 | 1,7 | -3,81 | 0,00089 | 0,0338 | Intron (uc002baw.3/1464, intron 1 of 9) | 12182 | ENSG00000173546 | CSPG4 |
| chr3 | 159978134 | 159978752 | -2,12 | 1,99 | -4,11 | 0,000891 | 0,0338 | Intron (uc003fda.3/57560, intron 17 of 18) | 34711 | ENSG00000180044 | C3orf80 |
| chr9 | 107010511 | 107011135 | -1,21 | 2,62 | -3,82 | 0,000891 | 0,0338 | Distal Intergenic | 120944 | ENSG00000136824 | SMC2 |
| chr4 | 35106337 | 35111646 | 1,25 | 6,94 | -5,69 | 0,000892 | 0,0338 | Distal Intergenic | 963811 | ENSG0000004 7365 | ARAP2 |
| chr1 1 | 106455315 | 106455965 | -2,12 | 2,07 | -4,18 | 0,000895 | 0,0339 | Distal Intergenic | 433206 | ENSG00000152402 | GUCY1A2 |
| chr2 | 181844049 | 181850999 | 9,1 | 9,91 | -0,82 | 0,000895 | 0,0339 | Promoter (<=1kb) | 0 | ENSG00000170035 | UBE2E3 |
| chr9 | 107472762 | 107473509 | -2,12 | 1,98 | -4,1 | 0,000896 | 0,0339 | Distal Intergenic | 16059 | ENSG00000179055 | OR13D1 |
| chr15 | 66461545 | 66461892 | -0,6 | 2,86 | -3,47 | 0,000898 | 0,0339 | Intron (uc002apl.2/84465, intron 1 of 20) | 84183 | ENSG00000157890 | MEGF11 |
| chr18 | 4008740 | 4009209 | -1,19 | 2,09 | -3,27 | 0,000898 | 0,0339 | Intron (uc031rhf.1/101410534, intron 4) of 4) | 46387 | ENSG00000263878 | DLGAP1-AS4 |
| chr4 | 178717225 | 178717877 | -2,12 | 1,88 | -4 | 0,000901 | 0,0339 | Intron (uc010int.3/285501, intron 2 of 5) | 67314 | ENSG00000231171 | LINC01098 |
| chr1 1 | 92608433 | 92608673 | -2,12 | 1,37 | -3,49 | 0,000901 | 0,0339 | Intron (uc001pdj.4/120114, intron 21 of 24) | 34705 | ENSG00000165323 | FAT3 |
| chr15 | 75197253 | 75200216 | 8,78 | 9,52 | -0,73 | 0,000901 | 0,0339 | Promoter (<=1kb) | 0 | ENSG00000178761 | FAM219B |
| chr8 | 63847350 | 63847810 | -2,12 | 1,92 | -4,04 | 0,000902 | 0,0339 | Intron (uc010lyq.1/286183, intron 5 of 5) | -42610 | ENSG00000274956 | NKAIN3 -IT1 |
| chr12 | 124055564 | 124056338 | -0,9 | 2,99 | -3,89 | 0,000902 | 0,0339 | Distal Intergenic | -12738 | ENSG00000086598 | TMED2 |
| chr15 | 63062110 | 63062618 | -1,35 | 2,26 | -3,61 | 0,000905 | 0,034 | Intron (uc002alb.4/83660, intron 38 of 55) | 11321 | ENSG00000171914 | TLN2 |
| chr1 | 235520703 | 235521325 | -0,77 | 2,52 | -3,29 | 0,000906 | 0,034 | Distal Intergenic | -9403 | ENSG00000284770 | TBCE |
| chr16 | 29350240 | 29350856 | -2,12 | 2,03 | -4,15 | 0,000907 | 0,034 | Intron (uc031qv1.1/440352, intron 6 of 10) | 10366 | NA | SNX29P2 |
| chr7 | 50599232 | 50599718 | -1,46 | 2,45 | -3,91 | 0,000907 | 0,034 | Promoter (<=1kb) | 0 | ENSG00000226122 | DDC-AS1 |
| chr8 | 60187222 | 60188084 | -1,77 | 2,23 | -3,99 | 0,000914 | 0,0342 | Distal Intergenic | -155455 | ENSG00000198846 | TOX |
| chr1 | 234891328 | 234891683 | -2,12 | 1,76 | -3,88 | 0,000914 | 0,0342 | Distal Intergenic | 31539 | ENSG00000227630 | LINC01132 |
| chr1 1 | 70487643 | 70488359 | -1,59 | 1,75 | -3,34 | 0,000915 | 0,0342 | Intron (uc009ysn.1/uc009ysn.1, intron 1 of 1) | 10444 | ENSG00000226627 | SHANK2-AS1 |
| chr15 | 61349219 | 61349973 | -2,12 | 1,95 | -4,07 | 0,000916 | 0,0342 | Intron (uc002agx.3/6095, intron 1 of 10) | 171529 | ENSG00000069667 | RORA |
| chr4 | 8052096 | 8053513 | 2,13 | 5,35 | -3,23 | 0,000919 | 0,0343 | Intron (uc003gkl.3/84448, intron 3 of 14) | 20200 | ENSG00000163995 | ABLIM2 |
| chr4 | 86877710 | 86878129 | -2,12 | 1,71 | -3,82 | 0,00092 | 0,0343 | Intron (uc003hpj.3/83478, intron 5 of 5) | 26284 | ENSG00000138639 | ARHGAP24 |
| chr13 | 86936121 | 86936757 | -2,12 | 2,05 | -4,16 | 0,000923 | 0,0344 | Distal Intergenic | -562638 | ENSG00000184564 | SLITRK6 |
| chr12 | 64432836 | 64433771 | -1,44 | 2,53 | -3,98 | 0,000926 | 0,0344 | Intron (uc001srt.3/57522, intron 4 of 9) | 55250 | ENSG00000196935 | SRGAP1 |
| chr7 | 107292162 | 107292890 | -1,93 | 1,84 | -3,77 | 0,000928 | 0,0344 | Distal Intergenic | -8190 | ENSG00000091137 | SLC26A4 |
| chr2 | 101356005 | 101356910 | 0,01 | 3,94 | -3,93 | 0,000931 | 0,0345 | Distal Intergenic | -79703 | ENSG00000170485 | NPAS2 |
| chr19 | 23592413 | 23592766 | -2,12 | 1,77 | -3,88 | 0,000931 | 0,0345 | Intron (uc002nrh.1/uc002nrh.1, intron 1 of 3) | -14144 | ENSG00000167232 | ZNF91 |
| chr15 | 78899941 | 78900779 | -2,12 | 1,46 | -3,58 | 0,000934 | 0,0345 | Intron (uc002bea.3/1136, intron 4 of 7) | -10867 | ENSG00000080644 | CHRNA3 |
| chr8 | 110977055 | 110977496 | -2,12 | 1,85 | -3,97 | 0,000935 | 0,0345 | Downstream (1-2kb) | 9925 | ENSG00000164794 | KCNV1 |
| chr6 | 167125976 | 167126772 | -2,12 | 1,97 | -4,09 | 0,000936 | 0,0345 | Intron (uc003qvc.1/6196, intron 2 of 21) | -85250 | ENSG00000071242 | RPS6KA2 |
| chr8 | 74818504 | 74819222 | -0,65 | 3,02 | -3,67 | 0,000936 | 0,0345 | Distal Intergenic | -27359 | ENSG00000104343 | UBE2W |
| chr16 | 6838589 | 6839124 | -1,64 | 2,07 | -3,71 | 0,000939 | 0,0346 | Intron (uc002cvr.1/54715, intron 3 of 11) | 14779 | ENSG00000078328 | RBFOX1 |
| chr2 | 160469155 | 160473875 | 8,39 | 9,42 | -1,03 | 0,000943 | 0,0347 | Promoter (<=1kb) | 0 | ENSG00000123636 | BAZ2B |
| chr15 | 72518282 | 72525203 | 9,88 | 10,51 | -0,63 | 0,000943 | 0,0347 | Promoter (<=1kb) | 0 | ENSG00000067225 | PKM |
| chr15 | 67812117 | 67816733 | 9,08 | 9,78 | -0,7 | 0,000944 | 0,0347 | Promoter (<=1kb) | 0 | ENSG00000259673 | IQCH-AS1 |
| chr2 | 147992337 | 147992758 | -2,12 | 1,54 | -3,65 | 0,000947 | 0,0347 | Distal Intergenic | -609328 | ENSG00000121989 | ACVR2A |
| chr4 | 2536545 | 2540509 | 9,22 | 10,05 | -0,84 | 0,000947 | 0,0347 | Distal Intergenic | -57319 | ENSG00000125386 | FAM193A |
| chr8 | 62747301 | 62747575 | -2,12 | 1,71 | -3,83 | 0,000948 | 0,0347 | Distal Intergenic | 119954 | ENSG00000264408 | MIR4470 |
| chr6 | 80044391 | 80044906 | -2,12 | 2,02 | -4,14 | 0,00095 | 0,0347 | Distal Intergenic | -99936 | ENSG00000118418 | HMGN3 |
| chr13 | 74962898 | 74963781 | -0,51 | 3,58 | -4,08 | 0,00095 | 0,0347 | Distal Intergenic | -29529 | ENSG00000226240 | LINC00381 |
| chr13 | 22794591 | 22795133 | -2,12 | 1,77 | -3,89 | 0,00095 | 0,0347 | Intron (uc001uoi.3/uc001uoi.3, intron 1 of 1) | -342292 | ENSG00000226722 | LINC00424 |
| chr6 | 134260866 | 134261441 | -0,32 | 3,11 | -3,43 | 0,00095 | 0,0347 | Distal Intergenic | -11867 | ENSG00000028839 | TBPL1 |
| chr6 | 7725656 | 7731771 | 9,52 | 10,46 | -0,94 | 0,000951 | 0,0347 | Promoter (<=1kb) | 0 | ENSG00000153162 | BMP6 |
| chr6 | 102241278 | 102242340 | -2,12 | 1,62 | -3,74 | 0,000952 | 0,0347 | Intron (uc003pqn.3/2898, intron 6 of 10) | 394417 | ENSG00000164418 | GRIK2 |
| chr2 | 156402929 | 156404354 | -0,36 | 3,97 | -4,33 | 0,000956 | 0,0349 | Distal Intergenic | 780172 | ENSG00000153234 | NR4A2 |
| chr8 | 8432944 | 8433236 | -2,12 | 1,25 | -3,36 | 0,000958 | 0,0349 | Distal Intergenic | -126430 | ENSG00000253958 | CLDN23 |
| chr18 | 71680039 | 71680512 | -1,44 | 2,04 | -3,49 | 0,000959 | 0,0349 | Distal Intergenic | 86791 | ENSG00000141665 | FBXO15 |
| chr2 | 45005526 | 45005981 | -1,28 | 2,72 | -4 | 0,00096 | 0,0349 | Distal Intergenic | -163056 | ENSG00000138083 | SIX3 |
| chr7 | 14417226 | 14417627 | -2,12 | 1,63 | -3,74 | 0,00096 | 0,0349 | Intron (uc003ssz.3/1607, intron 20 of 24) | -386176 | ENSG00000006468 | ETV1 |
| chr9 | 30471634 | 30472327 | -2,12 | 2,09 | -4,21 | 0,000961 | 0,035 | Distal Intergenic | -63182 | NA | LINC01242 |
| chr1 | 212808796 | 212810794 | 1,48 | 5,28 | -3,8 | 0,000968 | 0,0352 | Distal Intergenic | 11007 | ENSG00000162771 | FAM71A |
| chr1 | 237905647 | 237908192 | -0,17 | 4,17 | -4,34 | 0,00097 | 0,0352 | Exon (uc001hyl.1/6262, exon 80 of 105) | 15260 | ENSG00000198626 | RYR2 |
| chr9 | 29397597 | 29398068 | -1,86 | 2,1 | -3,96 | 0,000971 | 0,0352 | Distal Intergenic | -184599 | ENSG00000174482 | LINGO2 |
| chr10 | 26190771 | 26191718 | -0,95 | 2,87 | -3,83 | 0,000974 | 0,0352 | Distal Intergenic | -31284 | ENSG00000095777 | MYO3A |
| chr10 | 91918246 | 91919347 | -0,97 | 2,33 | -3,3 | 0,000976 | 0,0353 | Distal Intergenic | 328996 | ENSG00000232229 | LINC00865 |
| chr2 | 164704782 | 164705337 | -2,12 | 2 | -4,12 | 0,000979 | 0,0354 | Distal Intergenic | -112269 | ENSG00000182263 | FIGN |
| chr15 | 100680806 | 100681619 | -0,86 | 3,26 | -4,12 | 0,000982 | 0,0354 | Intron (uc002byy.1/170691, intron 10 of 21) | -164330 | ENSG00000140470 | ADAMTS17 |
| chr15 | 64751929 | 64754801 | 8,59 | 9,41 | -0,82 | 0,000984 | 0,0354 | 5'UTR | -36818 | ENSG00000180357 | ZNF609 |
| chr4 | 121568734 | 121569894 | -2,12 | 1,88 | -4 | 0,000986 | 0,0354 | Distal Intergenic | 274119 | ENSG00000138738 | PRDM5 |
| chr5 | 25456007 | 25456491 | -2,12 | 1,82 | -3,94 | 0,000986 | 0,0354 | Distal Intergenic | -615315 | ENSG00000248908 | LINC02239 |
| chr1 | 245641626 | 245642129 | -2,12 | 1,75 | -3,87 | 0,000986 | 0,0354 | Intron (uc010pyq.1/55083, intron 4 of 9) | -32175 | ENSG00000162849 | KIF26B |
| chr7 | 3161009 | 3162101 | -2,12 | 1,57 | -3,68 | 0,000986 | 0,0354 | Distal Intergenic | -77500 | ENSG00000198286 | CARD11 |
| chr7 | 3175059 | 3175759 | -1,77 | 1,9 | -3,66 | 0,000988 | 0,0355 | Distal Intergenic | -91550 | ENSG00000198286 | CARD11 |
| chr6 | 153603411 | 153603944 | -2,12 | 1,17 | -3,29 | 0,000989 | 0,0355 | Distal Intergenic | -151022 | ENSG00000091844 | RGS17 |
| chr4 | 179654395 | 179655223 | -1,42 | 2,72 | -4,14 | 0,000991 | 0,0355 | Distal Intergenic | 1004484 | ENSG00000231171 | LINC01098 |
| chr12 | 63908088 | 63908546 | -2,12 | 1,81 | -3,93 | 0,000992 | 0,0355 | Distal Intergenic | 69292 | ENSG00000177990 | DPY19L2 |
| chr1 | 8357330 | 8357948 | -2,12 | 1,7 | -3,82 | 0,000993 | 0,0355 | Distal Intergenic | -26442 | ENSG00000162426 | SLC45A1 |
| chr8 | 115541579 | 115545392 | -0,01 | 4,6 | -4,61 | 0,000995 | 0,0356 | Distal Intergenic | -1E+06 | ENSG00000164796 | CSMD3 |
| chr10 | 93328800 | 93329167 | -1,91 | 1,64 | -3,55 | 0,000995 | 0,0356 | Intron (uc010qnl.2/100188947, intron 1 of 4) | 42050 | NA | HECTD2-AS1 |
| chr1 | 104646875 | 104647645 | -0,53 | 3,13 | -3,66 | 0,000997 | 0,0356 | Distal Intergenic | 31230 | ENSG00000215869 | LOC1001291 38 |
| chr4 | 113151120 | 113155331 | 8,65 | 9,42 | -0,77 | 0,000998 | 0,0356 | Promoter (<=1kb) | 0 | ENSG00000138660 | AP1AR |
| chr2 | 9904210 | 9904934 | -2,12 | 1,92 | -4,04 | 0,001 | 0,0357 | Distal Intergenic | -78637 | ENSG00000115750 | TAF1B |
| chr16 | 67318985 | 67319471 | -2,12 | 1,79 | -3,91 | 0,001 | 0,0357 | Exon (uc002eso.4/25894, exon 12 of 21) | 5493 | ENSG00000196155 | PLEKHG4 |
| chr5 | 90139192 | 90139756 | -2,12 | 1,75 | -3,87 | 0,001 | 0,0357 | Intron (uc003kjt.4/84059, intron 78 of 89) | -55323 | ENSG00000164199 | ADGRV1 |
| chr4 | 166849111 | 166849557 | -2,12 | 1,58 | -3,69 | 0,001 | 0,0357 | Intron (uc021xud.1/7092, intron 1 of 9) | 54701 | ENSG00000038295 | TLL1 |
| chr19 | 51684346 | 51686943 | 1,18 | 5,66 | -4,48 | 0,00101 | 0,0359 | Exon (uc031rmj.1/uc031rmj.1, exon 1 of 5) | 13761 | ENSG00000171101 | SIGLEC17P |
| chr1 1 | 99553131 | 99553693 | -2,12 | 1,98 | -4,1 | 0,00101 | 0,0359 | Intron (uc009ywv.2/53942, intron 3 of 15) | 126255 | ENSG00000149972 | CNTN5 |
| chr21 | 22879756 | 22880191 | -2,12 | 1,77 | -3,89 | 0,00101 | 0,0358 | Intron (uc002vld.2/4685, intron 15 of 17) | 221934 | NA | RNU6-67P |
| chr20 | 20823366 | 20823960 | -2,12 | 1,72 | -3,84 | 0,00101 | 0,0359 | Distal Intergenic | -130100 | ENSG00000188559 | RALGAPA2 |
| chr6 | 53892239 | 53892833 | -1,75 | 1,99 | -3,74 | 0,00101 | 0,0358 | Intron (uc003pcf.2/90523, intron 1 of 11) | 8525 | ENSG00000146147 | MLIP |
| chr8 | 108593790 | 108594175 | -2,12 | 1,62 | -3,74 | 0,00101 | 0,0358 | Distal Intergenic | -83536 | ENSG00000154188 | ANGPT1 |
| chr4 | 46065790 | 46068590 | 1,28 | 6,14 | -4,87 | 0,00102 | 0,036 | Exon (uc003gxb.3/2565, exon 4 of 9) | 57492 | ENSG00000163285 | GABRG1 |
| chr6 | 68741539 | 68742323 | -2,12 | 1,98 | -4,1 | 0,00102 | 0,036 | Distal Intergenic | -603309 | ENSG00000135298 | ADGRB3 |
| chr4 | 176058164 | 176058563 | -2,12 | 1,94 | -4,06 | 0,00102 | 0,036 | Distal Intergenic | 218655 | ENSG00000168594 | ADAM2 9 |
| chr4 | 31220579 | 31221275 | -1,83 | 2,12 | -3,95 | 0,00102 | 0,0359 | Distal Intergenic | 498542 | ENSG00000169851 | PCDH7 |
| chr8 | 85771031 | 85771819 | -0,54 | 3,24 | -3,78 | 0,00102 | 0,0359 | Intron (uc003ycq.4/138046, intron 6 of 9) | 152869 | ENSG00000184672 | RALYL |
| chr7 | 17571139 | 17572262 | -0,32 | 3,44 | -3,76 | 0,00102 | 0,0359 | Distal Intergenic | 232863 | ENSG00000106546 | AHR |
| chr5 | 20223241 | 20223620 | -2,12 | 1,59 | -3,7 | 0,00102 | 0,036 | Intron (uc003jgc.3/1016, intron 2 of 14) | -234888 | ENSG00000145526 | CDH18 |
| chr1 1 | 71157636 | 71162025 | 9,16 | 10,04 | -0,89 | 0,00102 | 0,036 | Promoter (<=1kb) | 0 | ENSG00000172893 | DHCR7 |
| chr15 | 76601894 | 76604749 | 8,3 | 9,05 | -0,75 | 0,00102 | 0,036 | Promoter (<=1kb) | 0 | ENSG00000140374 | ETFA |
| chr5 | 26852741 | 26853533 | -1,83 | 2,41 | -4,24 | 0,00103 | 0,0361 | Distal Intergenic | 37409 | ENSG00000113100 | CDH9 |
| chr10 | 44812202 | 44812705 | -2,12 | 1,78 | -3,9 | 0,00103 | 0,0361 | Distal Intergenic | 24004 | ENSG00000277288 | C10orf142 |
| chrX | 73571539 | 73571984 | -1,44 | 2,06 | -3,51 | 0,00103 | 0,0362 | Distal Intergenic | 47514 | ENSG00000187969 | ZCCHC13 |
| chr5 | 131353342 | 131353801 | -0,93 | 2,53 | -3,46 | 0,00103 | 0,0362 | Distal Intergenic | -5472 | ENSG00000164398 | ACSL6 |
| chr4 | 1239589 | 1244898 | 9,2 | 10,28 | -1,09 | 0,00103 | 0,0362 | Promoter (<=1kb) | 0 | ENSG00000159692 | CTBP1 |
| chr4 | 100008225 | 100011738 | 8,45 | 9,31 | -0,86 | 0,00103 | 0,0361 | Promoter (<=1kb) | 0 | ENSG00000197894 | ADH5 |
| chr5 | 24563123 | 24563850 | -1,59 | 2,44 | -4,03 | 0,00104 | 0,0363 | Intron (uc003jgr.2/1008, intron 2 of 11) | 81235 | ENSG00000040731 | CDH10 |
| chr5 | 138058422 | 138059070 | -2,12 | 1,91 | -4,03 | 0,00104 | 0,0364 | Distal Intergenic | -30037 | ENSG00000044115 | CTNNA1 |
| chr20 | 23426897 | 23427383 | -2,12 | 1,76 | -3,88 | 0,00104 | 0,0362 | Distal Intergenic | 6099 | ENSG00000125831 | CST11 |
| chr20 | 53955836 | 53956413 | -0,27 | 3,34 | -3,61 | 0,00104 | 0,0362 | Distal Intergenic | 624115 | ENSG00000054803 | CBLN4 |
| chr3 | 57735135 | 57735618 | -1,33 | 2,24 | -3,57 | 0,00104 | 0,0363 | Distal Intergenic | -7556 | ENSG00000163681 | SLMAP |
| chr1 | 218311137 | 218311796 | -2,12 | 1,96 | -4,08 | 0,00105 | 0,0366 | Distal Intergenic | -146833 | ENSG00000067533 | RRP15 |
| chr1 | 197738702 | 197739671 | -1,86 | 2,15 | -4,01 | 0,00105 | 0,0365 | Intron (uc010ppf.2/163486, intron 2 of 19) | 4952 | ENSG00000213047 | DENND1B |
| chr7 | 106018123 | 106018805 | -0,75 | 3,08 | -3,83 | 0,00105 | 0,0366 | Distal Intergenic | -92485 | ENSG00000105835 | NAMPT |
| chr3 | 14431025 | 14431802 | -2,12 | 1,7 | -3,82 | 0,00105 | 0,0365 | Distal Intergenic | -12274 | ENSG00000131389 | SLC6A6 |
| chr18 | 3493535 | 3494283 | -1,01 | 2,78 | -3,79 | 0,00105 | 0,0365 | Downstream (1-2kb) | 38123 | ENSG00000177426 | TGIF1 |
| chr3 | 9463764 | 9465336 | -0,95 | 3,31 | -4,27 | 0,00106 | 0,0366 | Intron (uc003brs.1/55209, intron 1 of 19) | -6175 | ENSG00000168137 | SETD5 |
| chr1 | 194842016 | 194842591 | -2,12 | 1,96 | -4,08 | 0,00106 | 0,0366 | Distal Intergenic | 1468788 | ENSG00000162687 | KCNT2 |
| chr12 | 88916235 | 88917421 | 0,25 | 4,17 | -3,92 | 0,00106 | 0,0366 | Intron (uc001tav.3/4254, intron 3 of 9) | -3591 | ENSG00000049130 | KITLG |
| chr3 | 161237692 | 161238080 | -2,12 | 1,71 | -3,83 | 0,00106 | 0,0366 | Distal Intergenic | 23096 | ENSG00000182447 | OTOL1 |
| chr18 | 74816952 | 74817524 | -1,75 | 1,94 | -3,68 | 0,00106 | 0,0366 | 5'UTR | 27250 | ENSG00000197971 | MBP |
| chr3 | 152333309 | 152333666 | -2,12 | 1,49 | -3,6 | 0,00106 | 0,0366 | Distal Intergenic | 200579 | ENSG00000152601 | MBNL1 |
| chr5 | 28397967 | 28398385 | -2,12 | 1,34 | -3,46 | 0,00106 | 0,0366 | Distal Intergenic | -528592 | NA | LSP1P3 |
| chr2 | 183840327 | 183841054 | -2,12 | 2,1 | -4,22 | 0,00107 | 0,0368 | Intron (uc002upb.4/10787, intron 16 of 31) | 62532 | ENSG00000061676 | NCKAP1 |
| chr15 | 54265794 | 54266182 | -2,12 | 1,78 | -3,9 | 0,00107 | 0,0367 | Distal Intergenic | -38919 | ENSG00000137766 | UNC13C |
| chr5 | 20075438 | 20075799 | -2,12 | 1,63 | -3,74 | 0,00107 | 0,0368 | Intron (uc003jgc.3/1016, intron 2 of 14) | -87085 | ENSG00000145526 | CDH18 |
| chr15 | 21925834 | 21926370 | -1,64 | 2,07 | -3,7 | 0,00107 | 0,0367 | Distal Intergenic | 14369 | NA | LOC646214 |
| chr5 | 166398814 | 166400005 | -2,12 | 1,27 | -3,39 | 0,00107 | 0,0367 | Distal Intergenic | -311838 | ENSG00000145934 | TENM2 |
| chr19 | 679185 | 681697 | 8,31 | 9,27 | -0,97 | 0,00107 | 0,0367 | Promoter (<=1kb) | 0 | ENSG00000070404 | FSTL3 |
| chr19 | 5975949 | 5980327 | 9,36 | 10,06 | -0,7 | 0,00107 | 0,0367 | Promoter (<=1kb) | 0 | ENSG00000266983 | LOC1001285 68 |
| chr2 | 229423915 | 229424658 | -1,86 | 2,33 | -4,19 | 0,00108 | 0,037 | Distal Intergenic | -377554 | ENSG00000153820 | SPHKAP |
| chr12 | 25103419 | 25104147 | -2,12 | 1,94 | -4,06 | 0,00108 | 0,037 | Promoter (1-2kb) | -1026 | ENSG00000060982 | BCAT1 |
| chr2 | 22113960 | 22114497 | -1,48 | 2,54 | -4,02 | 0,00108 | 0,0371 | Distal Intergenic | -180436 | ENSG00000229621 | LINC01822 |
| chr10 | 21821672 | 21825180 | 8,99 | 9,86 | -0,87 | 0,00108 | 0,037 | Promoter (<=1kb) | 0 | ENSG00000078403 | MLLT10 |
| chr6 | 7106730 | 7111677 | 9,75 | 10,59 | -0,84 | 0,00108 | 0,0371 | Promoter (<=1kb) | 0 | ENSG00000124782 | RREB1 |
| chr2 | 136871868 | 136877555 | 9,5 | 10,31 | -0,81 | 0,00108 | 0,0371 | Promoter (<=1kb) | 0 | ENSG00000121966 | CXCR4 |
| chr4 | 155965381 | 155965866 | -2,12 | 1,68 | -3,8 | 0,00109 | 0,0372 | Distal Intergenic | -163915 | ENSG00000185149 | NPY2R |
| chr13 | 63163766 | 63164423 | -1,22 | 2,54 | -3,76 | 0,00109 | 0,0371 | Distal Intergenic | -1E+06 | NA | OR7E156P |
| chr12 | 8404624 | 8405149 | -2,12 | 1,62 | -3,74 | 0,00109 | 0,0372 | Distal Intergenic | -9082 | NA | FAM86FP |
| chr12 | 66356652 | 66357037 | -2,12 | 1,55 | -3,67 | 0,00109 | 0,0372 | Exon (uc001ssx.3/8091, exon 5 of 5) | 124353 | ENSG00000149948 | HMGA2 |
| chr2 | 168524568 | 168525517 | -1,21 | 2,31 | -3,52 | 0,00109 | 0,0371 | Distal Intergenic | -149665 | ENSG00000172318 | B3GALT1 |
| chr14 | 88675607 | 88675895 | -2,12 | 1,3 | -3,41 | 0,00109 | 0,0372 | Intron (uc001xwm.3/54207, intron 4 of 6) | 61360 | ENSG00000100433 | KCNK10 |
| chr1 | 97186358 | 97189711 | 8,32 | 9,26 | -0,95 | 0,00109 | 0,0372 | Promoter (<=1kb) | 0 | ENSG00000117569 | PTBP2 |
| chr4 | 123842398 | 123846022 | 8,77 | 9,53 | -0,76 | 0,00109 | 0,0371 | Promoter (<=1kb) | 0 | ENSG00000170917 | NUDT6 |
| chr15 | 56311235 | 56312818 | -0,25 | 4,18 | -4,43 | 0,0011 | 0,0373 | Distal Intergenic | -25400 | ENSG00000069869 | NEDD4 |
| chr4 | 189029203 | 189029909 | -1,83 | 2,28 | -4,11 | 0,0011 | 0,0372 | Promoter (<=1kb) | 513 | ENSG00000179046 | TRIML2 |
| chr4 | 106064171 | 106064987 | -0,84 | 3,24 | -4,08 | 0,0011 | 0,0373 | Promoter (2-3kb) | -2045 | ENSG00000168769 | TET2 |
| chr1 1 | 120821786 | 120822263 | -1,44 | 2,39 | -3,84 | 0,0011 | 0,0372 | Intron (uc001pxn.2/2900, intron 14 of 20) | -72540 | ENSG00000154114 | TBCEL |
| chr4 | 136445508 | 136445947 | -2,12 | 1,67 | -3,79 | 0,0011 | 0,0373 | Distal Intergenic | -1E+06 | ENSG00000254535 | PABPC4L |
| chr12 | 31182380 | 31183290 | -1,41 | 2,35 | -3,76 | 0,0011 | 0,0373 | Intron (uc001qq.2/100506660, intron 2 of 2) | -43489 | ENSG00000013573 | DDX11 |
| chr12 | 15650564 | 15651330 | -0,93 | 2,82 | -3,75 | 0,0011 | 0,0373 | Intron (uc001rcu.2/5800, intron 3 of 8) | -47948 | ENSG00000 151490 | PTPRO |
| chr9 | 136357394 | 136358029 | -1,21 | 2,5 | -3,71 | 0,0011 | 0,0372 | Distal Intergenic | -13118 | ENSG00000160326 | SLC2A6 |
| chr7 | 114426249 | 114426937 | -0,54 | 3,17 | -3,71 | 0,0011 | 0,0373 | Distal Intergenic | 128158 | ENSG00000128573 | FOXP2 |
| chr10 | 19835767 | 19836436 | -2,12 | 2,08 | -4,19 | 0,00111 | 0,0375 | Intron (uc010qcq.1/uc010qcq.1, intron 4 of 6) | -268936 | ENSG00000120594 | PLXDC2 |
| chrX | 82763954 | 82764579 | -2,12 | 1,81 | -3,93 | 0,00111 | 0,0375 | Promoter (<=1kb) | 685 | ENSG00000196767 | POU3F4 |
| chr21 | 20598672 | 20599353 | -1,06 | 2,57 | -3,63 | 0,00111 | 0,0375 | Distal Intergenic | -822702 | ENSG00000 154646 | TMPRSS15 |
| chr2 | 240729071 | 240729657 | -1,41 | 2,15 | -3,56 | 0,00111 | 0,0375 | Distal Intergenic | 44517 | NA | LOC150935 |
| chr14 | 77294331 | 77294994 | -0,11 | 3,27 | -3,38 | 0,00111 | 0,0375 | Promoter (1-2kb) | 1604 | ENSG00000 100565 | LRRC74A |
| chr3 | 154794093 | 154794559 | -2,12 | 1,92 | -4,04 | 0,00112 | 0,0377 | Promoter (2-3kb) | -2877 | ENSG00000 196549 | MME |
| chr12 | 113137354 | 113138262 | -0,53 | 3,23 | -3,75 | 0,00112 | 0,0377 | Intron (uc010syl.2/22895, intron 1 of 21) | -91287 | ENSGO0000089169 | RPH3A |
| chr17 | 81102871 | 81103972 | -0,53 | 3,21 | -3,74 | 0,00112 | 0,0377 | Distal Intergenic | 60922 | ENSG00000176845 | METRNL |
| chr7 | 11672838 | 11673243 | -2,12 | 1,55 | -3,66 | 0,00112 | 0,0377 | Intron (uc021zzn.1/221981, intron 2 of 26) | 198581 | ENSG00000005108 | THSD7A |
| chr1 | 185643368 | 185643708 | -1,83 | 1,65 | -3,48 | 0,00112 | 0,0377 | Distal Intergenic | -59975 | ENSG00000143341 | HMCN1 |
| chr19 | 6891167 | 6891662 | -1,21 | 2,23 | -3,44 | 0,00112 | 0,0377 | Intron (uc002mfw.4/2015, intron 2 of 20) | 3607 | ENSG00000174837 | ADGRE1 |
| chr19 | 29010729 | 29011419 | -2,12 | 2,06 | -4,17 | 0,00113 | 0,0379 | Intron (uc002nsa.2/uc002nsa.2, intron 5 of 9) | -444619 | ENSG00000267339 | LINC00906 |
| chr5 | 19069646 | 19070134 | -2,12 | 1,92 | -4,04 | 0,00113 | 0,0378 | Distal Intergenic | 816247 | ENSG00000 145526 | CDH18 |
| chr1 | 73836777 | 73837459 | -0,2 | 3,61 | -3,81 | 0,00113 | 0,0378 | Distal Intergenic | 826412 | ENSG00000 162620 | LRRIQ3 |
| chr19 | 8197822 | 8198285 | -2,12 | 1,65 | -3,76 | 0,00113 | 0,0378 | Exon (uc002mjf.3/84467, exon 13 of 63) | 14096 | ENSG00000 142449 | FBN3 |
| chr1 | 175221844 | 175222324 | -1,83 | 1,88 | -3,71 | 0,00113 | 0,0378 | Distal Intergenic | -59615 | ENSG00000235750 | KIAA0040 |
| chr1 | 214360677 | 214361011 | -2,12 | 1,52 | -3,63 | 0,00113 | 0,0379 | Distal Intergenic | -93554 | ENSG00000143499 | SMYD2 |
| chr19 | 55497110 | 55498136 | 0,21 | 3,76 | -3,55 | 0,00113 | 0,0379 | Exon (uc021vbq.1/55655, exon 8 of 13) | 19399 | ENSG00000022556 | NLRP2 |
| chr2 | 141592522 | 141593312 | -1,44 | 2,39 | -3,84 | 0,00114 | 0,0381 | Intron (uc002tvj.1/53353, intron 31 of 90) | 1295958 | ENSG00000168702 | LRP1B |
| chr6 | 27043769 | 27044112 | -2,12 | 1,5 | -3,62 | 0,00114 | 0,0381 | Distal Intergenic | -55684 | NA | LOC1002707 46 |
| chr2 | 223397055 | 223397425 | -2,12 | 1,48 | -3,6 | 0,00114 | 0,0381 | Intron (uc010zlo.2/130367, intron 4 of 4) | 107733 | ENSG00000163082 | SGPP2 |
| chr1 1 | 117100788 | 117105025 | 9,49 | 10,26 | -0,78 | 0,00114 | 0,038 | Promoter (<=1kb) | 0 | ENSG00000167257 | RNF214 |
| chr14 | 24992803 | 24993172 | -2,12 | 1,81 | -3,93 | 0,00115 | 0,0383 | Distal Intergenic | -15332 | ENSGO0000092009 | CMA1 |
| chr2 | 168779460 | 168780572 | -1,09 | 2,81 | -3,91 | 0,00115 | 0,0383 | Distal Intergenic | 104278 | ENSG00000 172318 | B3GALT1 |
| chr18 | 7746793 | 7747581 | -1,31 | 2,48 | -3,79 | 0,00115 | 0,0383 | Intron (uc002knn.4/5797, intron 1 of 30) | 179479 | ENSG00000173482 | PTPRM |
| chr8 | 58008995 | 58009335 | -2,12 | 1,57 | -3,69 | 0,00115 | 0,0383 | Distal Intergenic | -102565 | ENSG00000 104331 | IMPAD1 |
| chr2 | 210027999 | 210028614 | -2,12 | 1,34 | -3,46 | 0,00115 | 0,0383 | Distal Intergenic | -260157 | ENSG00000078018 | MAP2 |
| chr2 | 45265606 | 45266245 | -1,06 | 2,13 | -3,19 | 0,00115 | 0,0383 | Distal Intergenic | -29064 | ENSG00000170577 | SIX2 |
| chr4 | 127888085 | 127888690 | -2,12 | 2,09 | -4,21 | 0,00116 | 0,0385 | Distal Intergenic | -665397 | ENSG00000 164066 | INTU |
| chr14 | 65115979 | 65116772 | -1,21 | 2,54 | -3,75 | 0,00116 | 0,0386 | Distal Intergenic | -54421 | ENSG00000126822 | PLEKHG3 |
| chr10 | 63138341 | 63139132 | -1,22 | 2,36 | -3,58 | 0,00116 | 0,0385 | Distal Intergenic | 35739 | ENSG00000196932 | TMEM26 |
| chr15 | 53851280 | 53851561 | -2,12 | 1,28 | -3,4 | 0,00116 | 0,0385 | Intron (uc002acj.2/256764, intron 18 of 19) | -17980 | ENSG00000166415 | WDR72 |
| chr1 1 | 112329586 | 112330366 | 0,57 | 3,77 | -3,2 | 0,00116 | 0,0384 | Distal Intergenic | -198003 | ENSG00000188771 | PLET1 |
| chr8 | 22216241 | 22216687 | -2,12 | 1,39 | -3,51 | 0,00117 | 0,0387 | Distal Intergenic | 5929 | ENSG00000197181 | PIWIL2 |
| chr4 | 110353724 | 110357446 | 8,69 | 9,49 | -0,8 | 0,00117 | 0,0387 | Promoter (<=1kb) | 0 | ENSG00000138802 | SEC24B |
| chr6 | 18293868 | 18294242 | -2,12 | 1,45 | -3,56 | 0,00118 | 0,0389 | Distal Intergenic | -29069 | ENSG00000124795 | DEK |
| chr1 | 179197191 | 179200073 | 8,79 | 9,64 | -0,85 | 0,00118 | 0,039 | Promoter (<=1kb) | 0 | ENSG00000143322 | ABL2 |
| chr21 | 22699299 | 22699841 | -2,12 | 2,04 | -4,16 | 0,00119 | 0,039 | Intron (uc002yld.2/4685, intron 6 of 17) | 41477 | NA | RNU6-67P |
| chr4 | 105831636 | 105832075 | -2,12 | 2,04 | -4,16 | 0,00119 | 0,039 | Distal Intergenic | -234957 | ENSG00000168769 | TET2 |
| chr3 | 85501098 | 85501574 | -2,12 | 1,89 | -4,01 | 0,00119 | 0,0392 | Intron (uc003dqj.3/253559, intron 1 of 9) | -52635 | ENSG00000175161 | CADM2 |
| chr12 | 100813221 | 100813740 | -2,12 | 1,62 | -3,74 | 0,00119 | 0,0392 | Exon (uc009ztx.3/246213, exon 11 of 11) | -53811 | ENSG000000 12504 | NR1H4 |
| chr1 1 | 96438050 | 96438766 | -1,21 | 2,5 | -3,71 | 0,00119 | 0,0391 | Distal Intergenic | -198009 | NA | JRKL-AS1 |
| chr9 | 140523386 | 140523884 | -2,12 | 2,08 | -4,2 | 0,0012 | 0,0393 | Intron (uc004coa.3/79813, intron 1 of 15) | 9942 | ENSG00000181090 | EHMT1 |
| chr2 | 5813222 | 5814252 | 0,99 | 5,15 | -4,16 | 0,0012 | 0,0392 | Distal Intergenic | -18547 | ENSG00000176887 | SOX11 |
| chr1 | 214483944 | 214484615 | -1,01 | 2,94 | -3,95 | 0,0012 | 0,0393 | Intron (uc021piw.1/56950, intron 1 of 7) | 29379 | ENSG00000143499 | SMYD2 |
| chr1 | 4587544 | 4588275 | -2,12 | 1,82 | -3,93 | 0,0012 | 0,0393 | Distal Intergenic | 115433 | ENSG00000235054 | LINC01777 |
| chr7 | 14416519 | 14417063 | -2,12 | 1,76 | -3,88 | 0,0012 | 0,0393 | Intron (uc003ssz.3/1607, intron 20 of 24) | -385469 | ENSG00000006468 | ETV1 |
| chr19 | 254056 | 254555 | -1,44 | 2,02 | -3,47 | 0,0012 | 0,0394 | Distal Intergenic | 36614 | ENSG00000141934 | PLPP2 |
| chr1 | 190471874 | 190472499 | -2,12 | 1,88 | -4 | 0,00121 | 0,0395 | Distal Intergenic | -25115 | ENSG00000162670 | BRINP3 |
| chrX | 7810716 | 7812250 | -1,04 | 2,83 | -3,87 | 0,00121 | 0,0394 | Promoter (<=1kb) | 413 | ENSG00000182583 | VCX |
| chr2 | 43793449 | 43794037 | -2,12 | 1,73 | -3,85 | 0,00121 | 0,0395 | 5'UTR | 3612 | ENSG00000 115970 | THADA |
| chr5 | 13809916 | 13810791 | -0,19 | 3,6 | -3,79 | 0,00121 | 0,0395 | Exon (uc003jfd.2/1767, exon 45 of 79) | -40664 | ENSG00000039139 | DNAH5 |
| chr6 | 166259695 | 166260418 | -0,6 | 3,09 | -3,7 | 0,00121 | 0,0394 | Intron (uc003qup.1/uc003qup.1, intron 1 of 1) | -140621 | ENSG00000281832 | LINC00602 |
| chr5 | 16666227 | 16667199 | -0,77 | 3,47 | -4,23 | 0,00122 | 0,0397 | 3'UTR | 15031 | ENSG00000145555 | MYO10 |
| chr3 | 164975991 | 164976573 | -2,12 | 2,02 | -4,14 | 0,00122 | 0,0397 | Distal Intergenic | -61522 | ENSG00000121871 | SLITRK3 |
| chr7 | 122870163 | 122871091 | -0,34 | 3,67 | -4,02 | 0,00122 | 0,0396 | Distal Intergenic | -30138 | ENSGO0000081800 | SLC13A1 |
| chr14 | 59509226 | 59509693 | -2,12 | 1,8 | -3,91 | 0,00122 | 0,0396 | Distal Intergenic | -145688 | ENSG00000 100592 | DAAM1 |
| chr17 | 3438640 | 3439489 | 0,19 | 4,02 | -3,83 | 0,00122 | 0,0397 | Promoter (<=1kb) | 0 | ENSG00000167723 | TRPV3 |
| chrX | 143715865 | 143716494 | -2,12 | 1,49 | -3,6 | 0,00122 | 0,0396 | Distal Intergenic | -612613 | ENSG00000203923 | SPANXN1 |
| chr12 | 12711915 | 12716716 | 9,23 | 10,02 | -0,79 | 0,00122 | 0,0396 | Promoter (<=1kb) | 0 | ENSG00000 111266 | DUSP16 |
| chr5 | 6029621 | 6030257 | -1,75 | 1,9 | -3,65 | 0,00123 | 0,0399 | Distal Intergenic | 307148 | ENSG00000250490 | LINC02145 |
| chrX | 141352730 | 141353189 | -2,12 | 1,51 | -3,63 | 0,00123 | 0,0398 | Distal Intergenic | -59654 | ENSG0000004677 4 | MAGEC2 |
| chr18 | 44237382 | 44237642 | -2,12 | 1,24 | -3,36 | 0,00123 | 0,0398 | Promoter (<=1kb) | -386 | ENSG00000167210 | LOXHD1 |
| chr1 | 25192997 | 25193656 | -0,05 | 3,09 | -3,15 | 0,00123 | 0,0398 | Distal Intergenic | 63114 | ENSG00000020633 | RUNX3 |
| chrX | 53077253 | 53078599 | -0,33 | 3,94 | -4,27 | 0,00124 | 0,0401 | Promoter (<=1kb) | 0 | ENSG00000184194 | GPR173 |
| chr3 | 166219875 | 166220599 | -2,12 | 2,05 | -4,17 | 0,00124 | 0,0401 | Distal Intergenic | -664622 | ENSG00000 114200 | BCHE |
| chr17 | 79533465 | 79533948 | -2,12 | 1,74 | -3,86 | 0,00124 | 0,04 | Promoter (<=1kb) | 0 | ENSG00000 182446 | NPLOC4 |
| chr5 | 22249411 | 22249860 | -2,12 | 1,72 | -3,84 | 0,00124 | 0,04 | Intron (uc003jgk.2/1010, intron 3 of 14) | -35502 | ENSG00000154162 | CDH12 |
| chr15 | 66081265 | 66086523 | 9,31 | 10,06 | -0,75 | 0,00124 | 0,04 | Promoter (<=1kb) | 0 | ENSG00000174485 | DENND4A |
| chr9 | 139267123 | 139267625 | -2,12 | 1,95 | -4,07 | 0,00125 | 0,0402 | Promoter (<=1kb) | 508 | ENSG00000187796 | CARD9 |
| chr7 | 54772845 | 54773281 | -2,12 | 1,5 | -3,62 | 0,00125 | 0,0402 | Distal Intergenic | 53658 | ENSG00000132432 | SEC61G |
| chr9 | 73483537 | 73484366 | -0,17 | 3,36 | -3,53 | 0,00125 | 0,0402 | Promoter (<=1kb) | 0 | ENSG00000083067 | TRPM3 |
| chr13 | 77020171 | 77020655 | -2,12 | 1,72 | -3,84 | 0,00126 | 0,0404 | Distal Intergenic | 439885 | ENSG00000178695 | KCTD12 |
| chr9 | 131006768 | 131007740 | 1,05 | 4,85 | -3,8 | 0,00126 | 0,0404 | Promoter (<=1kb) | 0 | ENSG00000207581 | MIR199B |
| chr3 | 105722877 | 105723507 | -0,32 | 3,24 | -3,56 | 0,00126 | 0,0404 | Distal Intergenic | -134611 | ENSG00000 114423 | CBLB |
| chrX | 152086385 | 152087292 | -0,83 | 2,92 | -3,76 | 0,00127 | 0,0407 | Promoter (<=1kb) | 0 | ENSG00000 147394 | ZNF185 |
| chr5 | 20056743 | 20057102 | -2,12 | 1,58 | -3,69 | 0,00127 | 0,0407 | Intron (uc003jgc.3/1016, intron 2 of 14) | -68390 | ENSG00000 145526 | CDH18 |
| chr1 | 89530089 | 89531487 | 0,94 | 4,58 | -3,64 | 0,00127 | 0,0407 | Promoter (<=1kb) | 0 | ENSG00000 117228 | GBP1 |
| chr5 | 5271251 | 5272007 | -0,19 | 3,33 | -3,53 | 0,00127 | 0,0407 | Intron (uc003jdk.1/170690, intron 18 of 19) | 36124 | ENSG00000145536 | ADAMTS16 |
| chr6 | 147981385 | 147983307 | 0,53 | 4,82 | -4,29 | 0,00128 | 0,041 | Distal Intergenic | 151557 | ENSG00000203727 | SAMD5 |
| chr16 | 65731890 | 65732589 | -2,12 | 1,83 | -3,95 | 0,00128 | 0,0409 | Distal Intergenic | -121687 | ENSG00000261742 | LINC00922 |
| chr2 | 163828646 | 163829284 | -2,12 | 1,74 | -3,86 | 0,00128 | 0,041 | Distal Intergenic | -133389 | ENSG00000 184611 | KCNH7 |
| chr2 | 10466438 | 10467187 | -2,12 | 1,73 | -3,84 | 0,00128 | 0,041 | Promoter (1-2kb) | 1965 | ENSG00000115756 | HPCAL1 |
| chr5 | 21257528 | 21257963 | -2,12 | 1,57 | -3,69 | 0,00128 | 0,041 | Distal Intergenic | -83979 | ENSG00000183666 | GUSBP1 |
| chr15 | 68345734 | 68349702 | 9,04 | 9,81 | -0,77 | 0,00128 | 0,041 | Promoter (<=1kb) | 0 | ENSG00000033800 | PIAS1 |
| chr15 | 67832700 | 67836546 | 8,96 | 9,65 | -0,69 | 0,00128 | 0,041 | Promoter (<=1kb) | 0 | ENSG00000137764 | MAP2K5 |
| chr18 | 24281414 | 24283760 | 1,94 | 6,23 | -4,29 | 0,00129 | 0,041 | Promoter (<=1kb) | 0 | ENSG00000265369 | PCAT18 |
| chr5 | 164283544 | 164284235 | -0,54 | 3,53 | -4,08 | 0,00129 | 0,0411 | Distal Intergenic | 1339081 | ENSG00000038274 | MAT2B |
| chr8 | 39135509 | 39135914 | -1,93 | 1,49 | -3,42 | 0,00129 | 0,0411 | Intron (uc003xmt.4/203102,intron22 of 24) | -36268 | ENSG00000196115 | ADAM5 |
| chr1 | 248820986 | 248822045 | -2,12 | 1,29 | -3,41 | 0,00129 | 0,0411 | Distal Intergenic | -6801 | ENSG00000187701 | OR2T27 |
| chr14 | 22080365 | 22081028 | -1,21 | 2,51 | -3,71 | 0,0013 | 0,0413 | Distal Intergenic | 21970 | ENSG00000255582 | OR10G2 |
| chr1 | 238632555 | 238633004 | -2,12 | 1,49 | -3,61 | 0,0013 | 0,0413 | Distal Intergenic | 16313 | ENSG00000215808 | LINC01139 |
| chr3 | 180504697 | 180504958 | -2,12 | 1,36 | -3,48 | 0,0013 | 0,0413 | Intron (uc003fko.3/uc003fko.3, intron 3 of 13) | -49035 | ENSG00000284862 | CCDC39 |
| chr4 | 138522534 | 138522958 | -1,73 | 1,74 | -3,46 | 0,0013 | 0,0413 | Distal Intergenic | -68886 | ENSG00000189184 | PCDH18 |
| chr9 | 7477869 | 7478414 | -2,12 | 1,3 | -3,41 | 0,0013 | 0,0413 | Distal Intergenic | 321353 | ENSG00000137038 | DMAC1 |
| chr4 | 69435443 | 69435855 | -2,12 | 1,98 | -4,1 | 0,00131 | 0,0415 | Promoter (1-2kb) | -1198 | ENSG00000197888 | UGT2B17 |
| chr1 | 75430357 | 75430736 | -2,12 | 1,41 | -3,53 | 0,00131 | 0,0415 | Distal Intergenic | -163383 | ENSG00000 162624 | LHX8 |
| chr1 | 114470265 | 114474864 | 8,83 | 9,66 | -0,82 | 0,00131 | 0,0415 | Promoter (<=1kb) | 0 | ENSG00000163349 | HIPK1 |
| chr1 1 | 185421 | 186436 | -1,77 | 2,2 | -3,97 | 0,00132 | 0,0418 | Distal Intergenic | -6644 | ENSG00000188076 | SCGB1C1 |
| chr21 | 22273973 | 22274708 | -1,77 | 2,11 | -3,88 | 0,00132 | 0,0417 | Distal Intergenic | -95925 | ENSG00000154654 | NCAM2 |
| chr3 | 166145466 | 166145866 | -1,75 | 2,12 | -3,87 | 0,00132 | 0,0417 | Distal Intergenic | -590213 | ENSG00000 114200 | BCHE |
| chr18 | 77959610 | 77960150 | -1,91 | 1,84 | -3,75 | 0,00133 | 0,042 | Intron (uc0021ny.3/84552, intron 2 of 2) | 45247 | ENSG00000178184 | PARD6G |
| chr3 | 23846723 | 23855724 | 10,07 | 10,92 | -0,85 | 0,00133 | 0,042 | Promoter (<=1kb) | 0 | ENSG00000170142 | UBE2E1 |
| chr9 | 101684969 | 101685545 | -0,77 | 2,48 | -3,25 | 0,00134 | 0,0421 | Distal Intergenic | -20450 | ENSG00000204291 | COL15A1 |
| chr12 | 96335543 | 96338163 | 8,81 | 9,6 | -0,8 | 0,00134 | 0,0422 | Promoter (<=1kb) | 0 | ENSG00000165972 | CCDC38 |
| chr19 | 6422439 | 6426429 | 9,01 | 9,81 | -0,79 | 0,00134 | 0,042 | Promoter (<=1kb) | 0 | ENSG00000088247 | KHSRP |
| chr15 | 74986746 | 74989779 | 8,68 | 9,37 | -0,69 | 0,00134 | 0,0421 | Promoter (<=1kb) | 0 | ENSG00000179151 | EDC3 |
| chr1 | 68512382 | 68513254 | -0,16 | 4,06 | -4,22 | 0,00135 | 0,0424 | 5'UTR | 3227 | ENSG00000162595 | DIRAS3 |
| chr7 | 11671989 | 11672612 | -2,12 | 1,81 | -3,93 | 0,00135 | 0,0423 | Intron (uc021zzn.l/221981, intron 2 of 26) | 199212 | ENSG00000005108 | THSD7 A |
| chr8 | 115524438 | 115525796 | -0,17 | 3,48 | -3,65 | 0,00135 | 0,0423 | Distal Intergenic | -1E+06 | ENSG00000164796 | CSMD3 |
| chr19 | 1859945 | 1865812 | 9,99 | 10,76 | -0,77 | 0,00135 | 0,0422 | Promoter (<=1kb) | 0 | ENSG00000129911 | KLF16 |
| chr8 | 112136436 | 112136790 | -2,12 | 1,81 | -3,93 | 0,00136 | 0,0424 | Distal Intergenic | -1E+06 | ENSG00000164794 | KCNV1 |
| chr4 | 55905747 | 55906394 | -2,12 | 1,71 | -3,82 | 0,00136 | 0,0424 | Distal Intergenic | 85368 | ENSG00000 128052 | KDR |
| chr2 | 175400966 | 175402017 | -0,32 | 3,37 | -3,69 | 0,00136 | 0,0424 | Distal Intergenic | -49150 | ENSG00000163328 | GPR155 |
| chr1 | 8284993 | 8285416 | -2,12 | 1,37 | -3,48 | 0,00136 | 0,0424 | Distal Intergenic | -98974 | ENSG00000 162426 | SLC45A1 |
| chr5 | 104663281 | 104663915 | -2,12 | 1,74 | -3,86 | 0,00137 | 0,0427 | Distal Intergenic | 228106 | ENSG00000232159 | RAB9BP1 |
| chr19 | 755800 | 756245 | -1,75 | 1,9 | -3,64 | 0,00137 | 0,0427 | Intron (uc0021po.3/126353, intron 1 of 4) | 4654 | ENSGO0000099812 | MISP |
| chr1 | 222790317 | 222794547 | 8,59 | 9,47 | -0,87 | 0,00137 | 0,0426 | Promoter (<=1kb) | 0 | ENSG00000154305 | MIA3 |
| chr1 1 | 32334233 | 32334681 | -1,66 | 1,92 | -3,58 | 0,00138 | 0,0429 | Distal Intergenic | 117682 | ENSG00000184937 | WT1 |
| chr5 | 163931050 | 163931622 | -1,64 | 1,92 | -3,56 | 0,00138 | 0,0428 | Intron (uc003lzn.3/uc003lzn.3, intron 2 of 2) | 986587 | ENSG00000038274 | MAT2B |
| chr8 | 83279534 | 83279923 | -2,12 | 1,36 | -3,48 | 0,00138 | 0,0429 | Distal Intergenic | -525013 | ENSG00000104497 | SNX16 |
| chr6 | 6096185 | 6097128 | -0,5 | 3,65 | -4,15 | 0,00139 | 0,0432 | Distal Intergenic | -88552 | ENSG00000124785 | NRN1 |
| chrX | 34405970 | 34406457 | -1,34 | 2,23 | -3,57 | 0,00139 | 0,0432 | Distal Intergenic | -255523 | ENSG00000 185448 | FAM47A |
| chr3 | 173278865 | 173279353 | -2,12 | 1,79 | -3,9 | 0,0014 | 0,0434 | Intron (uc003fio.1/22871, intron 2 of 6) | -23124 | ENSG00000169760 | NLGN1 |
| chr4 | 4137102 | 4137646 | -2,12 | 1,41 | -3,53 | 0,0014 | 0,0434 | Distal Intergenic | 90975 | ENSG00000163982 | OTOP1 |
| chr12 | 18449319 | 18449747 | -0,73 | 2,59 | -3,32 | 0,0014 | 0,0433 | Intron (uc001rdt.3/5288, intron 5 of 31) | 14381 | ENSG00000139144 | PIK3C2G |
| chr16 | 2334226 | 2334799 | -2,12 | 1,72 | -3,84 | 0,00141 | 0,0436 | Exon (uc002cpv.1/21, exon 24 of 33) | 9605 | ENSG00000264004 | MIR4717 |
| chr17 | 40446604 | 40447030 | -2,12 | 1,57 | -3,69 | 0,00141 | 0,0435 | Intron (uc002hzj.2/6776, intron 5 of 19) | 6530 | ENSG00000126561 | STAT5A |
| chr3 | 110158507 | 110159901 | -0,36 | 3,24 | -3,6 | 0,00141 | 0,0434 | Distal Intergenic | 628905 | NA | NECTIN3 - AS1 |
| chr7 | 141400441 | 141402808 | 8,61 | 9,48 | -0,87 | 0,00141 | 0,0434 | Promoter (<=1kb) | 0 | ENSG00000257093 | KIAA1147 |
| chr2 | 8202280 | 8202878 | 0,44 | 3,79 | -3,34 | 0,00142 | 0,0437 | Intron (uc010eww.2/339789,intron 7 of 8) | -85303 | ENSG00000235665 | LINC00298 |
| chr8 | 85162482 | 85163761 | -0,23 | 4,04 | -4,26 | 0,00143 | 0,0439 | Intron (uc003vcq.4/13 8046,intron 1 of 9) | 65382 | ENSG00000184672 | RALYL |
| chr4 | 175755514 | 175757717 | 0,27 | 4,46 | -4,19 | 0,00143 | 0,0439 | Exon (uc003iua.1/uc003iua.1, exon 2 of 3) | -5049 | ENSG00000 145451 | GLRA3 |
| chr1 | 33704129 | 33705055 | 0,13 | 3,96 | -3,82 | 0,00143 | 0,0439 | Distal Intergenic | -17119 | ENSG00000 160094 | ZNF362 |
| chr3 | 86400819 | 86401439 | -2,12 | 1,41 | -3,53 | 0,00143 | 0,0439 | Distal Intergenic | -523619 | ENSG00000241648 | CADM2-AS2 |
| chr12 | 9282162 | 9282496 | -2,12 | 1,2 | -3,31 | 0,00143 | 0,0439 | Distal Intergenic | -13604 | ENSG00000175899 | A2M |
| chr1 1 | 77529120 | 77533268 | 9,49 | 10,3 | -0,82 | 0,00143 | 0,0439 | Promoter (<=1kb) | 0 | ENSG00000087884 | AAMDC |
| chr16 | 24739870 | 24743298 | 8,85 | 9,65 | -0,79 | 0,00143 | 0,0439 | Promoter (<=1kb) | 0 | ENSG00000090905 | TNRC6A |
| chr1 | 40777470 | 40778651 | 0,24 | 4,4 | -4,16 | 0,00144 | 0,0442 | 5'UTR | 4330 | ENSG00000049089 | COL9A2 |
| chr16 | 2523159 | 2526410 | 9,38 | 10,22 | -0,84 | 0,00144 | 0,0441 | Promoter (<=1kb) | 0 | ENSG00000 162065 | TBC1D24 |
| chr1 1 | 38300007 | 38300708 | -1,14 | 2,91 | -4,05 | 0,00145 | 0,0443 | Distal Intergenic | -2E+06 | ENSG00000175097 | RAG2 |
| chr2 | 62702100 | 62702986 | -0,54 | 3,41 | -3,95 | 0,00145 | 0,0443 | Distal Intergenic | 30618 | ENSGO0000186889 | TMEM17 |
| chr5 | 125170985 | 125171463 | -1,83 | 1,83 | -3,66 | 0,00145 | 0,0443 | Distal Intergenic | -524325 | ENSG00000155324 | GRAMD2B |
| chr5 | 17097144 | 17097664 | -1,73 | 1,68 | -3,41 | 0,00145 | 0,0443 | Distal Intergenic | -119268 | ENSG00000176788 | BASP1 |
| chr9 | 38669296 | 38669656 | -2,12 | 1,23 | -3,34 | 0,00145 | 0,0443 | Distal Intergenic | 48211 | NA | FAM201A |
| chr6 | 32375828 | 32376217 | -1,83 | 1,45 | -3,28 | 0,00145 | 0,0443 | Promoter (<=1kb) | -928 | ENSG00000204290 | BTNL2 |
| chr4 | 56410175 | 56414393 | 9,26 | 10,04 | -0,78 | 0,00145 | 0,0443 | Promoter (<=1kb) | 0 | ENSG00000134852 | CLOCK |
| chr1 | 200707049 | 200711479 | 9,08 | 9,84 | -0,75 | 0,00145 | 0,0443 | Promoter (<=1kb) | 0 | ENSG00000 118200 | CAMSAP2 |
| chr7 | 153545087 | 153545757 | -1,48 | 2,5 | -3,98 | 0,00146 | 0,0443 | Distal Intergenic | -38662 | ENSG00000130226 | DPP6 |
| chr1 | 195489436 | 195490062 | -1,75 | 2,01 | -3,77 | 0,00146 | 0,0443 | Distal Intergenic | 821317 | ENSG00000162687 | KCNT2 |
| chr3 | 34131303 | 34132142 | -2,12 | 1,27 | -3,38 | 0,00146 | 0,0445 | Distal Intergenic | 261173 | ENSG00000170248 | PDCD6IP |
| chr19 | 18919978 | 18920786 | -0,9 | 3,02 | -3,93 | 0,00147 | 0,0445 | Distal Intergenic | -17864 | ENSG00000 105664 | COMP |
| chr2 | 225543893 | 225544457 | -2,12 | 1,76 | -3,88 | 0,00147 | 0,0446 | Distal Intergenic | -93779 | ENSG00000036257 | CUL3 |
| chr4 | 122115558 | 122115917 | -2,12 | 1,41 | -3,53 | 0,00147 | 0,0447 | Intron (uc011cgj.2/79931, intron 2 of 12) | 21865 | ENSG00000050730 | TNIP3 |
| chr9 | 34922449 | 34922929 | -1,52 | 1,76 | -3,29 | 0,00147 | 0,0447 | Distal Intergenic | -34592 | ENSG00000122733 | PHF24 |
| chr2 | 211215469 | 211216084 | -0,25 | 2,75 | -2,99 | 0,00147 | 0,0446 | Distal Intergenic | -35574 | ENSG00000168530 | MYL1 |
| chr2 | 172013421 | 172019102 | 9,54 | 10,28 | -0,75 | 0,00147 | 0,0446 | Promoter (<=1kb) | 0 | ENSG00000198586 | TLK1 |
| chr12 | 74876943 | 74877759 | -1,64 | 2,21 | -3,85 | 0,00148 | 0,0448 | Distal Intergenic | -53792 | ENSG00000253719 | ATXN7L3B |
| chr10 | 103746038 | 103746398 | -2,12 | 1,67 | -3,79 | 0,00148 | 0,0447 | Intron (uc009xwx.1/79591, intron2 of 7) | 5397 | ENSGO0000120029 | ARMH3 |
| chr5 | 23459104 | 23459587 | -1,86 | 1,77 | -3,63 | 0,00148 | 0,0447 | Distal Intergenic | -48137 | ENSG00000 164256 | PRDM9 |
| chr7 | 123038122 | 123038608 | -2,12 | 1,51 | -3,63 | 0,00148 | 0,0448 | Distal Intergenic | 63062 | ENSG00000164675 | IQUB |
| chr1 | 99729678 | 99730665 | 0,69 | 4,15 | -3,46 | 0,00148 | 0,0447 | Promoter (<=1kb) | 0 | ENSG00000 117600 | PLPPR4 |
| chr1 | 31361001 | 31361318 | -1,41 | 1,92 | -3,32 | 0,00148 | 0,0448 | Intron (uc001bse.2/9672, intron 1 of 4) | -9414 | ENSG00000 162512 | SDC3 |
| chr2 | 141366590 | 141367010 | -2,12 | 1,75 | -3,87 | 0,00149 | 0,045 | Intron (uc002tvj.1/53353, intron 41 of 90) | 1522260 | ENSG00000168702 | LRP1B |
| chr7 | 110231279 | 110231819 | -2,12 | 1,74 | -3,86 | 0,00149 | 0,045 | Distal Intergenic | -499243 | ENSG00000 173114 | LRRN3 |
| chr3 | 166278267 | 166278550 | -2,12 | 1,42 | -3,54 | 0,00149 | 0,0449 | Distal Intergenic | -723014 | ENSG00000 114200 | BCHE |
| chr8 | 4440278 | 4440641 | -2,12 | 1,39 | -3,51 | 0,00149 | 0,045 | Intron (uc022aqr.1/64478, intron 2 of 69) | 411687 | ENSG00000183117 | CSMD1 |
| chr4 | 181933922 | 181934900 | -0,9 | 3,13 | -4,03 | 0,0015 | 0,0451 | Distal Intergenic | 145402 | ENSG00000248197 | LINC00290 |
| chr10 | 67251905 | 67252368 | -2,12 | 1,72 | -3,84 | 0,0015 | 0,0451 | Distal Intergenic | 666620 | NA | ANXA2P3 |
| chr8 | 127939245 | 127939770 | -1,57 | 2,14 | -3,71 | 0,0015 | 0,045 | Distal Intergenic | -85629 | ENSG00000253438 | PCAT1 |
| chr2 | 152748251 | 152748689 | -1,77 | 1,92 | -3,69 | 0,0015 | 0,0451 | Intron (uc002txv.3/785, intron 1 of 12) | -63242 | ENSG00000162980 | ARL5A |
| chr1 | 161676975 | 161677331 | -2,12 | 1,54 | -3,66 | 0,0015 | 0,0451 | Promoter (<=1kb) | 213 | ENSG00000132185 | FCRLA |
| chr12 | 95779118 | 95779637 | -0,55 | 2,72 | -3,27 | 0,0015 | 0,045 | Distal Intergenic | 75419 | ENSG00000265917 | MIR3685 |
| chr6 | 141836383 | 141836873 | -2,12 | 1,75 | -3,87 | 0,00151 | 0,0453 | Distal Intergenic | 573063 | ENSG00000135577 | NMBR |
| chr8 | 41593727 | 41594540 | -0,9 | 2,8 | -3,7 | 0,00151 | 0,0453 | Intron (uc010lxa.1/157848. intron 1 of 2) | -29968 | ENSG00000029534 | ANK1 |
| chr12 | 105368238 | 105368484 | -2,12 | 1,35 | -3,47 | 0,00151 | 0,0452 | Distal Intergenic | -11614 | ENSG00000 151131 | C12orf45 |
| chr9 | 128507428 | 128516735 | 9,54 | 10,38 | -0,84 | 0,00151 | 0,0452 | Promoter (<=1kb) | 0 | ENSG00000167081 | PBX3 |
| chr15 | 73923064 | 73926799 | 8,66 | 9,44 | -0,79 | 0,00151 | 0,0453 | Promoter (<=1kb) | 0 | ENSG00000156642 | NPTN |
| chr1 | 240995555 | 240996049 | -2,12 | 1,74 | -3,85 | 0,00152 | 0,0455 | Intron (uc010pyh.2/6000, intron 9 of 17) | 36103 | ENSG00000182901 | RGS7 |
| chr1 1 | 32383083 | 32383384 | -2,12 | 1,3 | -3,41 | 0,00152 | 0,0455 | Distal Intergenic | 68979 | ENSG00000184937 | WT1 |
| chr16 | 4103680 | 4104052 | -1,39 | 1,86 | -3,25 | 0,00152 | 0,0454 | Intron (uc002cvx.3/115, intron 2 of 10) | 62134 | ENSG00000162104 | ADCY9 |
| chr19 | 2162697 | 2167070 | 9,52 | 10,3 | -0,78 | 0,00152 | 0,0454 | Promoter (<=1kb) | 0 | ENSG00000 104885 | DOT1L |
| chr1 | 1772384 | 1774502 | 0,5 | 4,65 | -4,15 | 0,00153 | 0,0457 | Intron (uc001aif.3/2782, intron 1 of 11) | 48024 | ENSG00000078369 | GNB1 |
| chrX | 116922378 | 116923824 | -0,71 | 3,33 | -4,04 | 0,00153 | 0,0456 | Distal Intergenic | 158081 | ENSG00000003096 | KLHL13 |
| chr8 | 117043782 | 117044465 | -2,12 | 1,74 | -3,86 | 0,00153 | 0,0457 | Intron (uc031tcc.1/100859921, intron 7 of 13) | 292832 | ENSG00000249917 | LINC00536 |
| chr16 | 73764623 | 73765119 | -2,12 | 1,73 | -3,85 | 0,00153 | 0,0456 | Distal Intergenic | 343919 | ENSG00000258779 | LINC01568 |
| chr1 | 117509517 | 117510027 | -2,12 | 1,58 | -3,69 | 0,00153 | 0,0456 | Exon (uc001egv.1/5738,exon 6 of 9) | -34345 | ENSG00000134256 | CD101 |
| chr10 | 179509 | 183301 | 9,26 | 10 | -0,74 | 0,00153 | 0,0457 | Promoter (<=1kb) | 0 | ENSG00000015171 | ZMYND11 |
| chr4 | 137077414 | 137078410 | -1,21 | 2,88 | -4,09 | 0,00154 | 0,0459 | Distal Intergenic | 1375219 | ENSG00000189184 | PCDH18 |
| chr1 | 194370255 | 194370783 | -2,12 | 1,74 | -3,86 | 0,00154 | 0,0459 | Distal Intergenic | -1E+06 | ENSG00000162630 | B3 GALT2 |
| chr9 | 24479399 | 24479961 | -1,91 | 1,85 | -3,76 | 0,00154 | 0,0459 | Distal Intergenic | 65713 | ENSG00000205442 | IZUMO3 |
| chr12 | 77456032 | 77460539 | 8,76 | 9,51 | -0,76 | 0,00154 | 0,0459 | Promoter (<=1kb) | 0 | ENSG00000165891 | E2F7 |
| chr6 | 16756649 | 16765069 | 9,97 | 10,68 | -0,71 | 0,00154 | 0,0459 | Promoter (<=1kb) | 0 | ENSG00000124788 | ATXN1 |
| chr3 | 107806984 | 107810890 | 9,05 | 9,74 | -0,69 | 0,00155 | 0,0459 | Promoter (<=1kb) | 0 | ENSG00000196776 | CD47 |
| chr2 | 232555925 | 232556326 | -1,86 | 1,83 | -3,69 | 0,00156 | 0,0463 | Distal Intergenic | -16909 | ENSG00000 187514 | PTMA |
| chr12 | 84283806 | 84284381 | -1,44 | 2,21 | -3,65 | 0,00156 | 0,0463 | Distal Intergenic | 1022227 | ENSG00000072041 | SLC6 A15 |
| chr4 | 100865439 | 100872982 | 9,63 | 10,35 | -0,72 | 0,00156 | 0,0461 | Promoter (<=1kb) | 0 | ENSG00000 164031 | DNAJB14 |
| chr1 | 229759646 | 229763838 | 9,54 | 10,24 | -0,7 | 0,00156 | 0,0463 | Promoter (<=1kb) | 0 | ENSG00000135801 | TAF5L |
| chr10 | 8177983 | 8180997 | 1,87 | 5,71 | -3,84 | 0,00157 | 0,0465 | Distal Intergenic | 81316 | ENSG00000107485 | GATA3 |
| chr6 | 19556961 | 19557692 | -1,62 | 2,04 | -3,66 | 0,00157 | 0,0465 | Distal Intergenic | -279909 | ENSG00000172201 | ID4 |
| chr19 | 1405556 | 1412065 | 9,69 | 10,48 | -0,79 | 0,00157 | 0,0463 | Promoter (<=1kb) | 0 | ENSG00000071626 | DAZAP1 |
| chr8 | 89256413 | 89256679 | -2,12 | 1,85 | -3,97 | 0,00158 | 0,0466 | Intron (uc003yeb.4/4325, intron 1 of 9) | 83038 | ENSG00000156103 | MMP16 |
| chr8 | 39441947 | 39442775 | -1,05 | 2,85 | -3,9 | 0,00158 | 0,0466 | Promoter (<=1kb) | 0 | ENSG00000168619 | ADAM18 |
| chr15 | 76772912 | 76773487 | -2,12 | 1,52 | -3,64 | 0,00158 | 0,0466 | Intron (uc010bkr.3/49855, intron 8 of 14) | -125920 | ENSG00000140386 | SCAPER |
| chr10 | 61455731 | 61456426 | -2,12 | 1,52 | -3,64 | 0,00158 | 0,0466 | Intron (uc010qig.1/220963. intron 1 of 5) | 13223 | ENSG00000 165449 | SLC16A9 |
| chr18 | 74823758 | 74824443 | -1,59 | 2,03 | -3,62 | 0,00158 | 0,0466 | Intron (uc010xfd.2/4155, intron 1 of 8) | 20331 | ENSG00000197971 | MBP |
| chr17 | 42274990 | 42278976 | 9,44 | 10,29 | -0,85 | 0,00158 | 0,0466 | Promoter (<=1kb) | 0 | ENSG00000087152 | ATXN7L3 |
| chr17 | 42293313 | 42300389 | 10,35 | 11,08 | -0,74 | 0,00158 | 0,0466 | Promoter (<=1kb) | 0 | ENSG00000 108312 | UBTF |
| chr19 | 1590006 | 1593904 | 9,47 | 10,21 | -0,74 | 0,00158 | 0,0466 | Promoter (<=1kb) | 0 | ENSG00000071655 | MBD3 |
| chr7 | 126697896 | 126698456 | -0,99 | 2,88 | -3,87 | 0,00159 | 0,0466 | Promoter (<=1kb) | 0 | ENSG00000207692 | MIR592 |
| chr5 | 28614677 | 28615282 | -2,12 | 1,71 | -3,83 | 0,00159 | 0,0466 | Distal Intergenic | -311695 | NA | LSP1P3 |
| chr15 | 45104314 | 45104754 | -1,64 | 1,8 | -3,44 | 0,00159 | 0,0467 | Distal Intergenic | 75774 | ENSG00000185880 | TRIM69 |
| chr20 | 21748101 | 21748784 | -2,12 | 1,54 | -3,66 | 0,00161 | 0,0471 | Distal Intergenic | 61240 | ENSG00000125813 | PAX1 |
| chr4 | 163630821 | 163631556 | -1,39 | 2,24 | -3,62 | 0,00161 | 0,0472 | Distal Intergenic | 456517 | ENSG00000145414 | NAF1 |
| chr8 | 4813049 | 4813696 | -2,12 | 1,69 | -3,81 | 0,00162 | 0,0474 | Intron (uc022aqr.1/64478, intron 1 of 69) | 38632 | ENSG00000183117 | CSMD1 |
| chr15 | 54991018 | 54991612 | -2,12 | 1,6 | -3,72 | 0,00162 | 0,0474 | Distal Intergenic | 89478 | ENSG00000137766 | UNC13C |
| chr10 | 72091874 | 72092200 | -2,12 | 1,22 | -3,33 | 0,00162 | 0,0474 | Intron (uc031pvo.1/55222, intron 2 of 3) | 44335 | ENSG00000172731 | LRRC20 |
| chr5 | 36460157 | 36460520 | -1,83 | 1,43 | -3,26 | 0,00162 | 0,0475 | Distal Intergenic | -145937 | ENSG00000079215 | SLC1 A3 |
| chr1 | 167187905 | 167192686 | 9,85 | 10,52 | -0,67 | 0,00162 | 0,0475 | Promoter (<=1kb) | 0 | ENSG00000 143190 | POU2F1 |
| chr3 | 165541540 | 165541850 | -2,12 | 1,74 | -3,85 | 0,00163 | 0,0476 | Intron (uc003fem.4/590, intron 2 of 3) | 13403 | ENSG00000 114200 | BCHE |
| chr12 | 64480690 | 64481030 | -2,12 | 1,39 | -3,51 | 0,00163 | 0,0476 | Exon (uc010ssp.1/57522. exon 11 of 22) | 103104 | ENSG00000196935 | SRGAP1 |
| chr12 | 39861367 | 39861907 | -0,62 | 2,54 | -3,16 | 0,00163 | 0,0476 | Distal Intergenic | -24175 | ENSG00000139116 | KIF21A |
| chr6 | 129372882 | 129373798 | -0,65 | 3,23 | -3,89 | 0,00164 | 0,0477 | Intron (uc003qbn.3/3908, intron 2 of 63) | 168596 | ENSG00000 196569 | LAMA2 |
| chr6 | 155782765 | 155783098 | -2,12 | 1,39 | -3,51 | 0,00164 | 0,0477 | Distal Intergenic | -5728 | ENSG00000074771 | NOX3 |
| chr17 | 29754851 | 29755408 | -1,41 | 2,08 | -3,49 | 0,00164 | 0,0477 | Intron (uc002hgn.1/84440, intron 1 of 14) | 36209 | ENSG00000 131242 | RAB11FIP4 |
| chr6 | 72153618 | 72154050 | -1,75 | 1,7 | -3,45 | 0,00164 | 0,0478 | Distal Intergenic | -23170 | ENSG00000233237 | LINC00472 |
| chr9 | 41018039 | 41018705 | -2,12 | 1,19 | -3,3 | 0,00164 | 0,0478 | Distal Intergenic | -181624 | ENSG00000274349 | ZNF658 |
| chr19 | 2048481 | 2052395 | 9,01 | 9,86 | -0,85 | 0,00164 | 0,0478 | Promoter (<=1kb) | 0 | ENSG00000099875 | MKNK2 |
| chr8 | 89480821 | 89481474 | -0,91 | 2,97 | -3,88 | 0,00165 | 0,0479 | Distal Intergenic | -141104 | ENSG00000156103 | MMP16 |
| chr18 | 18960875 | 18961372 | -1,83 | 1,94 | -3,77 | 0,00165 | 0,048 | Intron (uc002ktf.1/80000, intron 2 of 14) | -69612 | ENSG00000141449 | GREB1L |
| chr9 | 82239077 | 82239759 | -2,12 | 1,64 | -3,76 | 0,00165 | 0,0478 | Intron (uc004alc.3/7091, intron 5 of 19) | 49289 | ENSG00000 106829 | TLE4 |
| chr5 | 23133872 | 23134813 | -0,86 | 2,82 | -3,68 | 0,00165 | 0,048 | Distal Intergenic | -280141 | ENSG00000154162 | CDH12 |
| chr6 | 44092648 | 44096633 | 9,13 | 9,99 | -0,86 | 0,00165 | 0,0479 | Promoter (<=1kb) | 0 | ENSG00000180992 | MRPL14 |
| chr10 | 974465 | 979070 | 9,3 | 10,1 | -0,8 | 0,00165 | 0,0479 | Promoter (<=1kb) | 0 | ENSG00000107929 | LARP4B |
| chr7 | 121776707 | 121778294 | 0,11 | 4,26 | -4,15 | 0,00166 | 0,048 | Promoter (2-3kb) | -2831 | ENSG00000008311 | AASS |
| chr1 1 | 132533514 | 132533953 | -2,12 | 1,67 | -3,79 | 0,00166 | 0,048 | Intron (uc001qgs.3/4978. intron 1 of 6) | 279084 | ENSG00000183715 | OPCML |
| chr22 | 31074496 | 31075742 | 0,29 | 3,65 | -3,36 | 0,00166 | 0,0481 | Distal Intergenic | -10624 | ENSG00000167065 | DUSP18 |
| chr5 | 60623172 | 60632084 | 9,95 | 10,86 | -0,9 | 0,00166 | 0,048 | Promoter (<=1kb) | 0 | ENSG00000130449 | ZSWIM6 |
| chr4 | 167959139 | 167959738 | -1,77 | 2,12 | -3,89 | 0,00167 | 0,0482 | Intron (uc021xuf.1/50859, intron 3 of 10) | 195586 | ENSG00000196104 | SPOCK3 |
| chr9 | 15627626 | 15629071 | 1,26 | 4,86 | -3,6 | 0,00167 | 0,0483 | Intron (uc003zmc.2/203238,intron7 of 7) | 63650 | ENSG00000 164989 | CCDC171 |
| chr1 1 | 25155751 | 25156061 | -2,12 | 1,47 | -3,59 | 0,00167 | 0,0482 | Distal Intergenic | 637235 | ENSG00000187398 | LUZP2 |
| chr17 | 11041073 | 11041982 | -0,93 | 2,64 | -3,57 | 0,00167 | 0,0482 | Distal Intergenic | -102758 | ENSG00000188803 | SHISA6 |
| chr6 | 68030335 | 68030669 | -2,12 | 1,44 | -3,56 | 0,00167 | 0,0482 | Distal Intergenic | -1E+06 | ENSG00000135298 | ADGRB3 |
| chr8 | 107795904 | 107796411 | -1,64 | 1,78 | -3,42 | 0,00167 | 0,0482 | Distal Intergenic | -13432 | ENSG00000174429 | ABRA |
| chr8 | 20674159 | 20674672 | -2,12 | 1,66 | -3,78 | 0,00168 | 0,0483 | Distal Intergenic | -156825 | ENSG00000253199 | LINC02153 |
| chr7 | 148469840 | 148470431 | -2,12 | 1,55 | -3,67 | 0,00168 | 0,0484 | Intron (uc010lpg.3/8454, intron 9 of 21) | -9522 | ENSG00000055130 | CUL1 |
| chr8 | 127394934 | 127395537 | -0,54 | 3 | -3,54 | 0,00169 | 0,0487 | Distal Intergenic | 175174 | ENSG00000168672 | LRATD2 |
| chr4 | 178834813 | 178835065 | -2,12 | 1,27 | -3,39 | 0,00169 | 0,0487 | Intron (uc010int.3/285501. intron 2 of 5) | 184902 | ENSG00000231171 | LINC01098 |
| chr2 | 176432332 | 176432674 | -1,21 | 2,02 | -3,22 | 0,00169 | 0,0486 | Distal Intergenic | -385842 | ENSG00000 154518 | ATP5MC3 |
| chr2 | 78624096 | 78624588 | -2,12 | 1,59 | -3,71 | 0,0017 | 0,0487 | Distal Intergenic | -441944 | NA | SNAR-H |
| chr2 | 129659214 | 129660042 | -1,05 | 2,6 | -3,64 | 0,0017 | 0,0489 | Distal Intergenic | -583043 | ENSG00000136720 | HS6ST1 |
| chr10 | 59061123 | 59061567 | -1,91 | 1,68 | -3,59 | 0,0017 | 0,0489 | Promoter (2-3kb) | 2752 | ENSG00000264747 | MIR3924 |
| chr21 | 22111157 | 22112775 | -0,17 | 3,78 | -3,95 | 0,00171 | 0,0489 | Downstream (2-3kb) | 62651 | ENSG00000224924 | LINC00320 |
| chr3 | 164969056 | 164969424 | -2,12 | 1,83 | -3,94 | 0,00171 | 0,0489 | Distal Intergenic | -54587 | ENSG00000121871 | SLITRK3 |
| chr5 | 18906600 | 18907217 | -2,12 | 1,66 | -3,78 | 0,00171 | 0,049 | Distal Intergenic | 979164 | ENSG00000 145526 | CDH18 |
| chr13 | 104640844 | 104641112 | -2,12 | 1,36 | -3,48 | 0,00171 | 0,0489 | Distal Intergenic | -705996 | ENSG00000263741 | MIR548AS |
| chr4 | 41211379 | 41217433 | 7,77 | 9,04 | -1,27 | 0,00171 | 0,0489 | Promoter (<=1kb) | 0 | ENSG00000163697 | APBB2 |
| chr2 | 1543936 | 1544581 | -1,64 | 2,01 | -3,65 | 0,00172 | 0,049 | Exon (uc002qwr.3/7173, exon 16 of 17) | 55568 | ENSG00000 115705 | TPO |
| chr1 1 | 43321703 | 43321995 | -2,12 | 1,26 | -3,38 | 0,00172 | 0,049 | Distal Intergenic | -11510 | ENSG00000166181 | API5 |
| chr5 | 139248431 | 139249542 | -0,14 | 3,78 | -3,92 | 0,00173 | 0,0492 | Intron (uc0031ev.2/9542, intron 4 of 10) | 73025 | ENSG00000 146005 | PSD2 |
| chr17 | 34325029 | 34325657 | -1,29 | 2,43 | -3,72 | 0,00173 | 0,0492 | Exon (uc010wcs.2/348249, exon 3 of 8) | 3427 | NA | CCL15-CCL14 |
| chr9 | 22461600 | 22461990 | -2,12 | 1,36 | -3,48 | 0,00173 | 0,0493 | Distal Intergenic | 14760 | ENSG00000176399 | DMRTA1 |
| chr19 | 50423531 | 50435290 | 8,95 | 9,93 | -0,97 | 0,00173 | 0,0492 | Promoter (<=1kb) | 0 | ENSG00000169136 | ATF5 |
| chr10 | 11651098 | 11654747 | 8,83 | 9,57 | -0,75 | 0,00174 | 0,0493 | Promoter (<=1kb) | 0 | ENSG00000148429 | USP6NL |
| chr20 | 53411585 | 53411958 | -1,62 | 2,11 | -3,73 | 0,00175 | 0,0497 | Distal Intergenic | 319319 | ENSG00000101134 | DOK5 |
| chr1 | 183786560 | 183786965 | -2,12 | 1,54 | -3,65 | 0,00175 | 0,0496 | Intron (uc010pof.1/23179, intron 2 of 11) | 12285 | ENSG00000143344 | RGL1 |
| chr15 | 62411308 | 62411805 | -2,12 | 1,44 | -3,56 | 0,00175 | 0,0496 | Distal Intergenic | 45677 | ENSG00000205502 | C2CD4B |
| chr6 | 154268740 | 154269196 | -1,17 | 2,3 | -3,47 | 0,00175 | 0,0496 | Distal Intergenic | -62435 | ENSG00000112038 | OPRM1 |
| chr3 | 152856598 | 152857039 | -2,12 | 1,29 | -3,41 | 0,00175 | 0,0497 | Distal Intergenic | -22990 | ENSG00000181467 | RAP2B |
| chr8 | 89330640 | 89331033 | -2,12 | 1,86 | -3,97 | 0,00176 | 0,0498 | Intron (uc003veb.4/4325, intron 1 of 9) | 8684 | ENSG00000156103 | MMP16 |
| chr4 | 188732737 | 188733234 | -2,12 | 1,62 | -3,74 | 0,00176 | 0,0498 | Distal Intergenic | -183691 | ENSG00000179059 | ZFP42 |
| chr3 | 30091391 | 30092013 | -1,44 | 2,18 | -3,62 | 0,00176 | 0,0497 | Distal Intergenic | -555981 | ENSG00000 163513 | TGFBR2 |
| chr7 | 16401116 | 16401781 | -1,44 | 2,17 | -3,62 | 0,00176 | 0,0499 | Intron (uc010ktx.2/729920, intron 3 of 9) | 59166 | ENSG00000214960 | CRPPA |
| chr10 | 34629006 | 34629381 | -2,12 | 1,28 | -3,4 | 0,00176 | 0,0497 | Intron (uc010qej.2/56288. intron 16 of 24) | 43801 | ENSG00000 148498 | PARD3 |
| chr1 1 | 113244703 | 113245036 | -1,44 | 1,72 | -3,16 | 0,00176 | 0,0498 | Distal Intergenic | -13477 | ENSG00000170209 | ANKK1 |
| chr1 | 192603851 | 192604511 | -2,12 | 1,62 | -3,74 | 0,00177 | 0,0499 | Promoter (<=1kb) | -757 | ENSG00000127074 | RGS13 |
| chr16 | 10532754 | 10533646 | -1,18 | 2,42 | -3,6 | 0,00177 | 0,0499 | Intron (uc002czu.3/80063, intron 5 of 11) | 10029 | ENSG00000 166669 | ATF7IP2 |
| chr5 | 91841391 | 91842154 | 0,1 | 3,5 | -3,4 | 0,00177 | 0,0499 | Distal Intergenic | 1057015 | ENSG00000237187 | NR2F1-AS1 |
| chr19 | 530416 | 534187 | 9,63 | 10,41 | -0,78 | 0,00177 | 0,0499 | Promoter (<=1kb) | 0 | ENSG00000099804 | CDC34 |
| chr1 1 | 117048550 | 117051471 | 8,61 | 9,39 | -0,78 | 0,00177 | 0,0499 | Promoter (<=1kb) | 0 | ENSG00000149577 | SIDT2 |
| chr4 | 129730068 | 129735341 | 9,64 | 10,41 | -0,77 | 0,00177 | 0,0499 | Promoter (<=1kb) | 0 | ENSG00000077684 | JADE1 |
| chr1 | 223241176 | 223241690 | -1,04 | 2,21 | -3,26 | 0,00178 | 0,0499 | Distal Intergenic | 66516 | ENSG00000187554 | ILR5 |

**Table 4: H3K4me3 differentially bound sites significantly enriched in the romidepsin-resistant cell lines compared to the romidepsin-sensitive cell lines**

| Seqnames | Start | End | Conc resistant | Conc sensitive | Fold | p.value | FDR | Annotation | Distance to TSS | ENSEMBL | SYMBOL |
|---|---|---|---|---|---|---|---|---|---|---|---|
| chr17 | 80692361 | 80694349 | 6,93 | -1,1 | 8,03 | 4,95E-10 | 5,15E-06 | Promoter (<=1kb) | 0 | ENSG00000 167363 | FN3K |
| chr8 | 134068476 | 134074283 | 7,39 | -1,1 | 8,49 | 6,11E-10 | 5,15E-06 | Promoter (<=1kb) | 0 | ENSG00000155926 | SLA |
| chr3 | 94653251 | 94660376 | 7,08 | -1,1 | 8,18 | 3,96E-09 | 2,01E-05 | Promoter (<=1kb) | 0 | ENSG00000239589 | LINC00879 |
| chr15 | 83315723 | 83317281 | 6,63 | -0,73 | 7,35 | 4,09E-09 | 2,01E-05 | Promoter (<=1kb) | 0 | ENSG00000214575 | CPEB1 |
| chr5 | 169692189 | 169695715 | 6,04 | -1,1 | 7,14 | 7,80E-09 | 3,29E-05 | Promoter (<=1kb) | 0 | ENSG00000043462 | LCP2 |
| chr2 | 99123862 | 99126542 | 5,95 | -1,1 | 7,05 | 1,12E-08 | 4,14E-05 | Intron (uc010yvj.1/3631, intron 1 of 25) | 62541 | ENSG00000040933 | INPP4 A |
| chr2 | 37557903 | 37566892 | 6,68 | -1,1 | 7,78 | 1,33E-08 | 4,50E-05 | Distal Intergenic | -4861 | ENSG00000115828 | QPCT |
| chr2 | 176956749 | 176958665 | 6,14 | -0,79 | 6,92 | 1,49E-08 | 4,64E-05 | Promoter (<=1kb) | 0 | ENSG00000128714 | HOXD13 |
| chr5 | 76144873 | 76148431 | 6,36 | -0,93 | 7,28 | 1,66E-08 | 4,91E-05 | Promoter (<=1kb) | 0 | ENSG00000171643 | S100Z |
| chr8 | 66712172 | 66713738 | 4.55 | -1.1 | 5.65 | 1.90E-08 | 5.33E-05 | Intron (uc003xvq.3/5150. intron 1 of 12) | -10843 | ENSG00000205268 | PDE7A |
| chr7 | 141290063 | 141293645 | 6,08 | -1,1 | 7,18 | 2,04E-08 | 5,49E-05 | Exon (uc003vwh.2/55750, exon 3 of 4) | 38985 | ENSG00000006530 | AGK |
| chr8 | 61834757 | 61836788 | 5,81 | -0,52 | 6,33 | 2,65E-0 8 | 6,52E-05 | Distal Intergenic | 43519 | NA | LOC100130298 |
| chr5 | 88191139 | 88201837 | 6,41 | -1,1 | 7,51 | 3,33E-0 8 | 7,56E-05 | Promoter (<=1kb) | 0 | ENSG00000081189 | MEF2C |
| chr2 | 95962476 | 95964164 | 5,87 | -1,1 | 6,97 | 5,56E-08 | 0,000106 | Promoter (<=1kb) | 0 | ENSG00000115041 | KCNIP3 |
| chr7 | 47538984 | 47543207 | 6.36 | -1.1 | 7.46 | 6.01E-08 | 0.000111 | Intron (uc003tnw.3/647S9. intron 3 of 30) | 35992 | ENSG00000136205 | TNS3 |
| chr5 | 180045568 | 180047100 | 5,4 | -1,1 | 6,49 | 7,12E-08 | 0,00012 | 3'UTR | 9327 | ENSG00000037280 | FLT4 |
| chr1 | 10310410 | 10314376 | 5,76 | -1,1 | 6,86 | 8,77E-08 | 0,00014 | Intron (uc009vmt.3/23095, intron 2 of 10) | 18102 | ENSG00000054523 | KIF1B |
| chr6 | 93431462 | 93434032 | 6,28 | -1,1 | 7,38 | 9,84E-08 | 0,000149 | Distal Intergenic | 695268 | ENSG00000135333 | EPHA7 |
| chr1 | 14923992 | 14926606 | 6,58 | 0,49 | 6,09 | 1,02E-07 | 0,000151 | Promoter (<=1kb) | 0 | ENSG00000189337 | KAZN |
| chrX | 99891142 | 99892358 | 5,45 | -0,17 | 5,63 | 1,09E-07 | 0,000156 | Promoter (<=1kb) | 0 | ENSG00000000003 | TSPAN6 |
| chr2 | 158369370 | 158372610 | 6,09 | -1,1 | 7,19 | 1,11E-07 | 0,000156 | Distal Intergenic | -23897 | ENSG00000115165 | CYTIP |
| chr2 | 46791776 | 46794351 | 5,16 | -1,1 | 6,26 | 1,95E-07 | 0,000247 | Intron (uc002rva.3/23433, intron 2 of 4) | 21909 | ENSG00000 119729 | RHOQ |
| chr19 | 19651233 | 19652750 | 5,65 | -0,76 | 6,4 | 2.03E-07 | 0,000247 | Promoter (2-3kb) | 2159 | ENSG00000160161 | CILP2 |
| chr5 | 178367321 | 178369204 | 6,45 | -0,43 | 6,88 | 2,53E-07 | 0,000287 | Promoter (<=1kb) | 0 | ENSG00000 178187 | ZNF454 |
| chr3 | 101518292 | 101522783 | 5,54 | -1,1 | 6,64 | 3,26E-07 | 0,000327 | Exon (uc003dvn.3/91775, exon 5 of 8) | 20006 | ENSG00000144815 | NXPE3 |
| chr9 | 140174768 | 140176054 | 5,28 | -1,1 | 6,38 | 4,54E-07 | 4.00E-04 | Promoter (2-3kb) | 2488 | ENSG00000 198113 | TOR4A |
| chr6 | 56256372 | 56259415 | 6,98 | 0,36 | 6,63 | 4,65E-07 | 0,000403 | Promoter (<=1kb) | 0 | ENSG00000124749 | COL21A1 |
| chr4 | 9496647 | 9500679 | 5,58 | -0,81 | 6,39 | 4,97E-07 | 0,000419 | Distal Intergenic | 50387 | ENSG00000186146 | DEFB131A |
| chr3 | 185017510 | 185018948 | 5,49 | -1,1 | 6,59 | 6.43E-07 | 0,000482 | Intron (uc0 10hyf.3/9175. intron 2 of 14) | 16781 | ENSG00000073803 | MAP3K13 |
| chr2 | 37526132 | 37533171 | 5,44 | -1,1 | 6,53 | 6.73E-07 | 0,000485 | Intron (uc002rqd.3/23683, intron 1 of 17) | 11051 | ENSG00000115825 | PRKD3 |
| chr19 | 7615623 | 7618476 | 4,9 | -1,1 | 5,99 | 6,94E-07 | 0,000489 | Exon (uc002mgq.2/10908, exon 20 of 35) | 15034 | ENSG00000032444 | PNPLA6 |
| chr3 | 43338025 | 43340084 | 4,67 | -0,65 | 5,32 | 7,36E-07 | 5,00E-04 | Intron (uc003cms.4/54861, intron 1 of6) | -4506 | ENSG00000 163788 | SNRK |
| chr6 | 74163394 | 74166365 | 5,5 | -1,1 | 6,59 | 7,77E-07 | 0,000515 | Promoter (1-2kb) | -1351 | ENSG00000164430 | CGAS |
| chr10 | 135148264 | 135150710 | 6.58 | 0.73 | 5.85 | 7.87E-07 | 0.000516 | Promoter (<=1kb) | 0 | ENSG00000 130643 | CALY |
| chr5 | 170736431 | 170740075 | 7,64 | 1,63 | 6,01 | 8,22E-07 | 0,000528 | Promoter (<=1kb) | 143 | ENSG00000164438 | TLX3 |
| chr3 | 9594592 | 9596115 | 6,59 | 1,03 | 5,56 | 9,01E-07 | 0,000551 | Promoter (<=1kb) | 0 | ENSG00000156959 | LHFPL4 |
| chr8 | 62659278 | 62661718 | 5,32 | -1,1 | 6,42 | 9,01E-07 | 0,000551 | Distal Intergenic | 31931 | ENSG00000264408 | MIR4470 |
| chr2 | 80703217 | 80704157 | 3,44 | -1,1 | 4,54 | 9,26E-07 | 0,000554 | Intron (uc010yse.2/1496, intron 13 of 21) | -112021 | ENSG00000066032 | CTNNA2 |
| chr6 | 24357618 | 24358920 | 4.83 | -1 | 5.82 | 9.30E-07 | 0.000554 | Promoter (<=1kb) | 0 | ENSG00000146038 | DCDC2 |
| chr9 | 95499252 | 95501044 | 4,53 | -1,1 | 5,63 | 1,01E-06 | 0,000593 | Intron (uc004aso.1/23299, intron 1 of 7) | 26039 | ENSG00000185963 | BICD2 |
| chr4 | 1164738 | 1166607 | 5.14 | -1.1 | 6.24 | 1.05E-06 | 0.000611 | Promoter (<=1kb) | 0 | ENSG00000159674 | SPON2 |
| chr14 | 70133445 | 70135137 | 5,04 | -1,1 | 6,14 | 1,12E-06 | 0,000644 | Intron (uc001xlk.3/9766, intron 2 of 5) | 55135 | ENSG00000100647 | SUSD6 |
| chr20 | 31956674 | 31959286 | 5,07 | -1,1 | 6,17 | 1,19E-06 | 0,000664 | Promoter (1-2kb) | 1669 | ENSG00000101391 | CDK5RAP1 |
| chr14 | 61746962 | 61748522 | 5,2 | -1,1 | 6,29 | 1,19E-06 | 0,000664 | Promoter (<=1kb) | 8 | ENSG00000182107 | TMEM30B |
| chr1 | 44330778 | 44332350 | 5,19 | -1,1 | 6,29 | 1,25E-06 | 0,000688 | Intron (uc009vwu.1/6487, intron 7 of 7) | -66642 | ENSG00000 117407 | ARTN |
| chr19 | 15661557 | 15662573 | 5,38 | 0.17 | 5,21 | 1.61E-06 | 0.000824 | 3'UTR | 42221 | ENSG00000171954 | CYP4F22 |
| chr8 | 81068178 | 81071121 | 5,09 | -1,1 | 6,19 | 1,82E-06 | 0,000897 | Intron (uc010lzs.1/7163, intron 1 of 7) | 12224 | ENSG00000076554 | TPD52 |
| chr5 | 140419342 | 140420871 | 6,08 | -0,1 | 6,18 | 1,84E-06 | 0,000898 | Distal Intergenic | -10108 | ENSG00000171815 | PCDHB1 |
| chr11 | 91957597 | 91959462 | 5,19 | -1 | 6,19 | 1,88E-06 | 0,000903 | Distal Intergenic | -125800 | ENSG00000165323 | FAT3 |
| chr15 | 71145557 | 71147190 | 6,89 | 0,91 | 5,98 | 1,90E-06 | 0,000903 | Promoter (<=1kb) | 0 | ENSG00000137821 | LRRC49 |
| chr1 | 244481544 | 244483676 | 4,38 | -0,76 | 5,14 | 2,16E-06 | 0,001 | Distal Intergenic | -32261 | ENSG00000 173728 | C1orf100 |
| chr10 | 11250430 | 11252746 | 4,28 | -1,1 | 5,38 | 2,32E-06 | 0,00106 | Intron (uc010qbi.2/10659, intron 2 of 7) | 42981 | ENSG00000048740 | CELF2 |
| chr6 | 73739194 | 73740870 | 3,87 | -1,1 | 4,97 | 2,34E-06 | 0,00106 | Intron (uc003pgj.4/56479, intron 2 of 8) | -61718 | ENSG00000266180 | MIR4282 |
| chr19 | 49539103 | 49540958 | 4,59 | -1,1 | 5,69 | 2,62E-06 | 0,00116 | Promoter (<=1kb) | 0 | NA | SNAR-G1 |
| chr4 | 40182139 | 40183715 | 4,98 | -1,1 | 6,08 | 3,59E-06 | 0,00146 | Distal Intergenic | -14812 | ENSG00000168421 | RHOH |
| chr5 | 137001359 | 137003265 | 4,6 | -0,93 | 5,53 | 3,76E-06 | 0,00151 | Intron (uc011cyc.2/26249, intron 1 of 8) | 5351 | ENSG00000146021 | KLHL3 |
| chr10 | 830156 | 831444 | 4,43 | -0,57 | 5 | 4,21E-0 6 | 0,00166 | Distal Intergenic | -94548 | ENSG00000151240 | DIP2C |
| chr2 | 160918160 | 160919561 | 4,8 | -1 | 5,8 | 4,30E-06 | 0,00166 | Promoter (<=1kb) | 0 | ENSG00000153246 | PLA2R1 |
| chr5 | 172660521 | 172662661 | 7,46 | 2,06 | 5,4 | 4,70E-06 | 0,00177 | Promoter (<=1kb) | 0 | ENSG00000 183072 | NKX2-5 |
| chr21 | 32715746 | 32716918 | 4,68 | -0,95 | 5,62 | 4,80E-06 | 0,00178 | Intron (uc002yow.1/7074, intron 2 of 28) | -66522 | ENSG00000156299 | TIAM1 |
| chr3 | 158031309 | 158033269 | 4,82 | -1,1 | 5,92 | 4,99E-06 | 0,00181 | Intron (uc011bou.2/51319, intron 4 of6) | 203468 | ENSG00000174891 | RSRC1 |
| chr17 | 78901541 | 78903362 | 4,81 | -1,1 | 5,91 | 5,13E-06 | 0,00182 | Intron (uc002jyt.1/57521, intron 24 of33) | -19874 | ENSG00000 141564 | RPTOR |
| chr5 | 112598128 | 112599147 | 3,29 | -1,1 | 4,39 | 5,57E-06 | 0,00192 | Intron (uc003kqj.4/4163, intron 1 of 16) | 31465 | ENSG00000171444 | MCC |
| chr22 | 40592550 | 40593921 | 4,08 | -1,1 | 5,18 | 6.23E-06 | 0,00203 | Intron (uc003aym.3/23112, intron 4 of 23) | 18621 | ENSG00000100354 | TNRC6B |
| chr22 | 29426316 | 29427408 | 4.68 | -1.1 | 5.78 | 6.40E-06 | 0.00205 | Promoter (<=1kb) | 56 | ENSG00000 177993 | ZNRF3-AS1 |
| chr20 | 25563768 | 25566904 | 7,44 | 1,03 | 6,41 | 6,44E-06 | 0,00206 | Promoter (<=1kb) | 0 | ENSG00000101004 | NINL |
| chr8 | 105675994 | 105678203 | 4,84 | -1,1 | 5,93 | 6,52E-06 | 0,00207 | Exon (uc022azo.1/uc022azo.1, exon 1 of 1) | -74742 | ENSG00000147650 | LRP12 |
| chr4 | 184718040 | 184720341 | 6,78 | 0,67 | 6,1 | 7,07E-06 | 0,0022 | Distal Intergenic | -106168 | ENSG00000173320 | STOX2 |
| chr3 | 43632545 | 43634274 | 4,68 | -0,93 | 5,61 | 7,50E-06 | 0,00225 | Intron (uc003cmv3/55129, intron 4 of 12) | 29286 | ENSG00000160746 | ANO10 |
| chr9 | 108090421 | 108091974 | 4.73 | -0.78 | 5,51 | 8.07E-06 | 0.00236 | Intron (uc004bcn.3/23446. intron 3 of 15) | -25783 | ENSG00000070214 | SLC44A1 |
| chr7 | 44892498 | 44893905 | 4,6 | -1,1 | 5,7 | 8,65E-06 | 0,00247 | Distal Intergenic | -4773 | ENSG00000105968 | H2AFV |
| chr19 | 15371022 | 15372194 | 3,52 | -1,1 | 4,62 | 8,70E-06 | 0,00247 | Intron (uc002nar.3/23476, intron 7 of 19) | 19068 | ENSG00000141867 | BRD4 |
| chr5 | 173115649 | 173117611 | 4,73 | -1,1 | 5,83 | 8,92E-06 | 0,00252 | Distal Intergenic | -71983 | ENSG00000145919 | BOD1 |
| chr6 | 26299101 | 26300418 | 4,67 | -1,1 | 5,77 | 9,78E-06 | 0,00266 | Exon (uc021ymv.1/uc021ymv.1, exon 1 of 1) | -13374 | ENSG00000158406 | HIST1H4H |
| chr1 | 28352310 | 28354777 | 4,95 | -0,84 | 5,79 | 9,95E-06 | 0,00268 | Exon (uc001bpi.2/2140, exon 7 of 18) | 60371 | ENSG00000158161 | EYA3 |
| chr12 | 109214416 | 109216491 | 4,59 | -1,1 | 5,69 | 1,00E-05 | 0,00268 | Intron (uc001tnm.3/54434, intron 3 of 14) | -3264 | ENSG00000084112 | SSH1 |
| chr10 | 46095713 | 46096794 | 5,02 | -0,73 | 5,75 | 1,02E-05 | 0,00272 | Distal Intergenic | -5359 | ENSG00000165406 | MARCH8 |
| chr19 | 40722797 | 40724352 | 5,42 | 0,28 | 5,13 | 1,07E-05 | 0,00281 | Promoter (<=1kb) | 0 | ENSG00000174521 | TTC9B |
| chr8 | 105572208 | 105574017 | 4,02 | -1,1 | 5,12 | 1,10E-05 | 0,00285 | Intron (uc003yma.3/29967, intron 1 of 6) | 27235 | ENSG00000147650 | LRP12 |
| chr19 | 21768147 | 21770020 | 5,11 | -0,68 | 5,79 | 1,10E-05 | 0,00285 | Distal Intergenic | 79710 | ENSG00000197013 | ZNF429 |
| chr2 | 38821557 | 38823203 | 4,04 | -0,72 | 4,76 | 1,11E-05 | 0,00286 | Intron (uc021vgb.1/92906, intron 2 of 13) | 6975 | ENSG00000143889 | HNRNPLL |
| chr1 | 174817438 | 174819411 | 4.43 | -1.1 | 5,52 | 1,19E-05 | 0.00299 | Intron (uc001gjx.3/9910, intron 19 of20) | -24113 | ENSG00000152061 | RABGAP1L |
| chr16 | 179960 | 181750 | 4,9 | -0,76 | 5,66 | 1,19E-05 | 0,00299 | 5'UTR | 6583 | ENSG00000103148 | NPRL3 |
| chr17 | 38308874 | 38310907 | 4,54 | -1,1 | 5,64 | 1,23E-05 | 0,00302 | Intron (uc010cws.1/22794, intron 3 of 7) | 12367 | ENSG00000108349 | CASC3 |
| chr19 | 16657733 | 16659428 | 4,53 | -1,1 | 5,63 | 1,29E-05 | 0,00312 | Intron (uc002nee.3/9441, intron 6 of 11) | -4470 | ENSG00000085872 | CHERP |
| chr18 | 76733213 | 76734780 | 4,48 | -0,81 | 5,29 | 1,30E-05 | 0,00314 | Distal Intergenic | -5495 | ENSG00000256463 | SALL3 |
| chr7 | 66215265 | 66217034 | 4,49 | -1.1 | 5.59 | 1.30E-05 | 0.00314 | Intron (uc003tvf.3/27342. intron 3 of 12) | -3842 | ENSG00000154710 | RABGEF1 |
| chr16 | 3138620 | 3140793 | 5,8 | 0,29 | 5,51 | 1,43E-05 | 0,00328 | Promoter (2-3kb) | 2068 | ENSG00000130182 | ZSCAN10 |
| chr3 | 112388800 | 112391025 | 4,47 | -1,1 | 5,56 | 1,48E-05 | 0,00334 | Distal Intergenic | -28823 | ENSG00000091986 | CCDC80 |
| chr12 | 19635456 | 19636622 | 3,84 | -1,1 | 4,94 | 1,49E-05 | 0,00334 | Intron (uc001ree.2/121536, intron 3 of7) | 41941 | ENSG00000139154 | AEBP2 |
| chr7 | 139207506 | 139208671 | 3,95 | -1,1 | 5,04 | 1,52E-05 | 0,00339 | Promoter (<=1kb) | -3 | ENSG00000236279 | CLEC2L |
| chr10 | 125833797 | 125836156 | 4,25 | -0,79 | 5,04 | 1,55E-05 | 0,00342 | Intron (uc001lhm.4/51363, intron 1 of 7) | 15784 | ENSG00000182022 | CHST15 |
| chr22 | 17724075 | 17725362 | 4,07 | -0,65 | 4,72 | 1,61E-05 | 0,00345 | Distal Intergenic | -21337 | ENSG00000093072 | ADA2 |
| chr19 | 36048532 | 36050659 | 6,18 | 1,17 | 5,02 | 1,70E-05 | 0,0036 | Promoter (<=1kb) | 0 | ENSG00000105675 | ATP4 A |
| chr16 | 88005281 | 88006969 | 4,39 | -1,1 | 5,48 | 1,77E-05 | 0,00369 | Promoter (1-2kb) | 1657 | ENSG00000172530 | BANP |
| chrX | 25024679 | 25025819 | 3,83 | -1,1 | 4,93 | 1,79E-05 | 0,0037 | Exon (uc004dbp.4/170302, exon 4 of 5) | 8246 | ENSG00000004848 | ARX |
| chr15 | 59169988 | 59175425 | 6,49 | 0,6 | 5,9 | 1,83E-05 | 0,00374 | 3'UTR | 11356 | ENSG00000137776 | SLTM |
| chr11 | 115644363 | 115647077 | 4,79 | -0,53 | 5,32 | 1,99E-05 | 0,00398 | Distal Intergenic | -13018 | NA | LINC00900 |
| chr1 | 10115536 | 10117084 | 4,35 | -1,1 | 5,45 | 2,07E-05 | 0,00405 | Intron (uc001aqr.4/10277, intron 1 of 26) | 22495 | ENSG00000 130939 | UBE4B |
| chr1 | 180095445 | 180096609 | 4,01 | -1,1 | 5,11 | 2,09E-05 | 0,00409 | Distal Intergenic | -27359 | ENSG00000116260 | QSOX1 |
| chr2 | 37582926 | 37584400 | 3,62 | -1,1 | 4,72 | 2,15E-05 | 0,00414 | Intron (uc002rqg.3/25797, intron 2 of 6) | 11173 | ENSG00000115828 | QPCT |
| chr3 | 80213954 | 80215538 | 3,88 | -1,1 | 4,98 | 2,15E-05 | 0,00414 | Distal Intergenic | -396895 | ENSG00000169855 | ROBO1 |
| chr18 | 55764851 | 55765971 | 3,97 | -1,1 | 5,07 | 2,18E-0 5 | 0,00416 | Intron (uc0021gx.3/23327, intron 1 of 29) | 50393 | ENSG00000049759 | NEDD4L |
| chr1 | 151591884 | 151593873 | 4,35 | -1,1 | 5,45 | 2,22E-05 | 0,0042 | Intron (uc031ppc.1/81609, intron 1 of 11) | 7222 | ENSG00000143376 | SNX27 |
| chr1 | 49195619 | 49196721 | 3,61 | -0,95 | 4,56 | 2,37E-05 | 0,0044 | Intron (uc001cru.2/84871, intron 6 of 13) | 45826 | ENSG00000 162373 | BEND5 |
| chr2 | 132201035 | 132203882 | 6.59 | 0.54 | 6.04 | 2,38E-05 | 0.0044 | Promoter (<=1kb) | 0 | NA | NOC2LP2 |
| chr19 | 58553681 | 58554743 | 3,88 | -1,1 | 4,97 | 2,48E-05 | 0,0045 | Intron (uc002qrc. 1/284312, intron 4 of 5) | 8247 | ENSG00000152467 | ZSCAN1 |
| chr8 | 897007 | 898763 | 4,52 | -0,72 | 5,24 | 2,58E-05 | 0,00461 | Intron (uc003wpj.2/619343, intron 3 of 3) | 63809 | NA | NA |
| chrY | 21702797 | 21704789 | 4,1 | -1,1 | 5,2 | 2,84E-05 | 0,00496 | Distal Intergenic | -24447 | ENSG00000 131002 | TXLNGY |
| chr6 | 96704841 | 96708444 | 6,56 | 0,77 | 5,79 | 2,84E-05 | 0,00496 | Distal Intergenic | 240996 | ENSG00000 172461 | FUT9 |
| chr20 | 57428901 | 57431115 | 4.4 | -0,25 | 4.64 | 2,87E-05 | 0.005 | Promoter (<=1kb) | 865 | ENSG00000087460 | GNAS |
| chr10 | 131329255 | 131332631 | 4,21 | -1,1 | 5,31 | 2,91E-0 5 | 0,00503 | Intron (uc001lkh.2/4255, intron 1 of 4) | 63801 | ENSG00000170430 | MGMT |
| chr4 | 186264709 | 186265850 | 4,03 | -1,1 | 5,13 | 2,92E-05 | 0,00503 | Intron (uc003ixh.3/83891, intron 12 of 18) | 22822 | ENSG00000109762 | SNX25 |
| chr2 | 143165462 | 143167703 | 4,14 | -1,1 | 5,24 | 3,02E-0 5 | 0,00517 | Distal Intergenic | -276192 | ENSG00000168702 | LRP1B |
| chrX | 19567412 | 19568532 | 4,43 | -0,81 | 5,24 | 3,04E-05 | 0,00517 | 5'UTR | 19073 | ENSG00000147010 | SH3KBP1 |
| chr2 | 30787934 | 30789290 | 4,27 | -1,1 | 5,36 | 3,09E-05 | 0,00521 | Intron (uc002rnj.3/253558, intron 5 of 6) | 117797 | ENSG00000 172954 | LCLAT1 |
| chr2 | 39630452 | 39631791 | 3,8 | -1,1 | 4,9 | 3,12E-05 | 0,00522 | Intron (uc002rro.4/8491, intron 1 of 33) | -31987 | ENSG00000231312 | MAP4K3-DT |
| chr19 | 49935324 | 49936343 | 4,25 | -0,56 | 4,82 | 3,13E-05 | 0,00522 | Promoter (1-2kb) | 1603 | ENSG00000104888 | SLC17A7 |
| chr18 | 14999598 | 15001933 | 5,2 | 0,01 | 5,19 | 3,15E-05 | 0,00522 | Distal Intergenic | 162450 | ENSG00000 180777 | ANKRD3 0B |
| chr9 | 96901375 | 96903503 | 4,24 | -1,1 | 5,34 | 3,17E-05 | 0,00523 | Distal Intergenic | -34736 | ENSG00000199165 | MIRLET7A1 |
| chr16 | 87635483 | 87637084 | 4,08 | -0,69 | 4,77 | 3,30E-05 | 0,00538 | Promoter (<=1kb) | 0 | ENSG00000154118 | JPH3 |
| chr21 | 15905869 | 15907513 | 4,01 | -1,1 | 5,11 | 3,35E-0 5 | 0,00538 | Intron (uc002yju.2/64092, intron 1 of 7) | 11168 | ENSG00000155307 | SAMSN1 |
| chr16 | 20084616 | 20085499 | 3.82 | -1.1 | 4.92 | 3.36E-05 | 0.00538 | Promoter (<=1kb) | 0 | ENSG00000180269 | GPR139 |
| chr1 | 20692943 | 20694841 | 4,17 | -1,1 | 5,27 | 3,41E-0 5 | 0,00543 | Intron (uc009vpu.2/339505,intron 2 of 3) | 33684 | ENSG00000158816 | VWA5B1 |
| chr7 | 121024576 | 121026682 | 4,19 | -1,1 | 5,29 | 3,41E-0 5 | 0,00543 | Intron (uc003vjx.3/10447, intron 1 of 9) | 9740 | ENSG00000196937 | FAM3C |
| chr4 | 2571966 | 2573376 | 4,22 | -1,1 | 5,32 | 3,43E-0 5 | 0,00543 | Distal Intergenic | -24452 | ENSG00000125386 | FAM193A |
| chr14 | 104037110 | 104038553 | 4,19 | -1,1 | 5,29 | 3,47E-05 | 0,00547 | Exon (uc0 10tyc2/84334, exon 2 of 5) | 7811 | ENSG00000256053 | COA8 |
| chr10 | 70395240 | 70397030 | 4.1 | -1.1 | 5.2 | 3.55E-05 | 0.00554 | Intron (uc001jok.4/80312. intron 3 of 11) | -35622 | ENSG00000138336 | TET1 |
| chr20 | 31959686 | 31961260 | 3,58 | -1,1 | 4,68 | 3,59E-05 | 0,00557 | Promoter (<=1kb) | 0 | ENSG00000101391 | CDK5RAP1 |
| chr19 | 8340455 | 8344821 | 4,18 | -1,1 | 5,28 | 3,60E-05 | 0,00558 | Distal Intergenic | 28419 | ENSG00000167775 | CD320 |
| chr15 | 23625132 | 23626618 | 3,78 | -1,1 | 4,88 | 3,87E-05 | 0,00585 | Distal Intergenic | 25237 | ENSG00000261739 | GOLGA8 S |
| chr16 | 88966986 | 88969708 | 5,04 | -0,04 | 5,08 | 3,92E-05 | 0,0059 | 5'UTR | -19421 | ENSG00000129993 | CBFA2T3 |
| chr6 | 170589224 | 170590212 | 4,03 | -1,1 | 5,12 | 4,00E-05 | 0,00592 | Downstream (1-2kb) | 9485 | ENSG00000198719 | DLL1 |
| chr8 | 17136638 | 17138650 | 4,1 | -1,1 | 5,2 | 4,12E-05 | 0,00601 | Exon (uc003wxj.3/137492, exon 7 of 12) | 4353 | ENSG00000155975 | VPS37A |
| chr14 | 77200587 | 77202351 | 5,08 | -0,29 | 5,36 | 4,12E-05 | 0,00601 | Distal Intergenic | -25884 | ENSG00000071246 | VASH1 |
| chr9 | 125709505 | 125711073 | 4,17 | -1,1 | 5,27 | 4,18E-0 5 | 0,00603 | Intron (uc004bnl.4/23637, intron 1 of 27) | 6206 | ENSG00000011454 | RABGAP1 |
| chr3 | 185164904 | 185166411 | 4,14 | -1,1 | 5,24 | 4,28E-05 | 0,00609 | 5'UTR | 50434 | ENSG00000163900 | TMEM41A |
| chr18 | 803347 | 804199 | 3,37 | -0,9 | 4,27 | 4,36E-05 | 0,00618 | Intron (uc002kky.3/7525, intron 1 of 11) | 8128 | ENSG00000176105 | YES1 |
| chr5 | 776401 | 778738 | 7,09 | 1,67 | 5,42 | 4,50E-05 | 0,00633 | Distal Intergenic | 45188 | ENSG00000188818 | ZDHHC11 |
| chr10 | 70804818 | 70806073 | 3,68 | -1,1 | 4,78 | 4,52E-05 | 0,00634 | Distal Intergenic | -41755 | ENSG00000122862 | SRGN |
| chr6 | 43118770 | 43120065 | 4,02 | -1,1 | 5,11 | 4,58E-05 | 0,00639 | Intron (uc003oub.2/5754, intron 17 of 19) | 18611 | ENSG00000112655 | PTK7 |
| chr16 | 4793099 | 4794309 | 4,46 | -0,65 | 5,11 | 4,60E-05 | 0,00639 | Exon (uc002cxn.3/146562, exon 5 of 10) | -8721 | ENSG00000168096 | ANKS3 |
| chr20 | 32429011 | 32429946 | 3,33 | -1,1 | 4,43 | 4,69E-05 | 0,00646 | Intron (uc002xaa.3/128866, intron 1 of 4) | 29901 | ENSG00000101421 | CHMP4B |
| chr7 | 22829606 | 22830770 | 4,11 | -0,93 | 5,05 | 4,73E-0 5 | 0,00647 | Distal Intergenic | 31701 | ENSG00000196683 | TOMM7 |
| chr15 | 65359515 | 65360682 | 4,61 | -0,57 | 5,18 | 4,74E-05 | 0,00647 | Promoter (<=1kb) | 0 | ENSG00000103710 | RASL12 |
| chr12 | 69180681 | 69181928 | 3,62 | -0,98 | 4,6 | 4,84E-05 | 0,00656 | Distal Intergenic | 17346 | NA | LOC100130075 |
| chr22 | 40057879 | 40059155 | 4,29 | -0.66 | 4.95 | 5,16E-05 | 0.00682 | Exon (uc003ayc.3/8911, exon 17 of 37) | 63723 | ENSG00000100346 | CACNA1 I |
| chr3 | 42200720 | 42201938 | 3,87 | -0,65 | 4,53 | 5,17E-05 | 0,00682 | Promoter (<=1kb) | 0 | ENSG00000182606 | TRAK1 |
| chr11 | 19433336 | 19434679 | 4,19 | -0,57 | 4,76 | 5,30E-05 | 0,00692 | Intron (uc001mpp.3/89797, intron 1 of 37) | 61065 | ENSG00000166833 | NAV2 |
| chr1 | 1885928 | 1887521 | 4,98 | -0,12 | 5,11 | 5,35E-0 5 | 0,00696 | 3'UTR | 34893 | ENSG00000142609 | CFAP74 |
| chr1 | 107900766 | 107902831 | 4,98 | 0,32 | 4,66 | 5,37E-05 | 0,00696 | Intron (uc001dvc.4/22854, intron 3 of 5) | -34945 | ENSG00000162631 | NTNG1 |
| chr20 | 47502502 | 47505172 | 5.73 | 0.26 | 5.47 | 5.53E-05 | 0.00712 | Distal Intergenic | -33103 | ENSG00000124198 | ARFGEF2 |
| chr6 | 67804224 | 67806229 | 4,62 | -0,36 | 4,98 | 5,62E-0 5 | 0,00723 | Distal Intergenic | 1306452 | NA | SLC25A51P1 |
| chr19 | 39159684 | 39161269 | 3,34 | -1,1 | 4,44 | 5,84E-05 | 0,00746 | Intron (uc010egc.2/81, intron 1 of 11) | 21417 | ENSG00000 130402 | ACTN4 |
| chr3 | 185016535 | 185017404 | 3,89 | -1,1 | 4,99 | 5,94E-05 | 0,00755 | Intron (uc0 10hyf.3/9175, intron 2 of 14) | 15806 | ENSG00000073803 | MAP3K13 |
| chr15 | 42268785 | 42270336 | 4,7 | -0,52 | 5,22 | 6,03E-0 5 | 0,0076 | Distal Intergenic | -4030 | ENSG00000103966 | EHD4 |
| chr17 | 29396844 | 29397868 | 3,88 | -1,1 | 4,97 | 6,20E-05 | 0,00768 | Distal Intergenic | 23575 | ENSG00000265444 | MIR4733 |
| chr22 | 40390418 | 40391269 | 3,9 | -0,98 | 4,88 | 6,42E-05 | 0,00788 | Promoter (<=1kb) | 0 | ENSG00000133477 | FAM83F |
| chr6 | 53690082 | 53691058 | 3,53 | -1,1 | 4,63 | 6,48E-05 | 0,00794 | Intron (uc003pcd.1/55227, intron 1 of 13) | 30548 | ENSG00000137269 | LRRC1 |
| chr4 | 2402977 | 2406883 | 5,54 | 0,73 | 4,82 | 6,64E-05 | 0,00808 | Intron (uc003gex.2/57732, intron 1 of 12) | 13487 | ENSG00000159733 | ZFYVE28 |
| chr3 | 118674009 | 118676547 | 3,8 | -1,1 | 4,9 | 6,70E-05 | 0,00812 | Intron (uc003ebw.3/152404, intron 1 of 6) | 12086 | ENSG00000239877 | IGSF11-AS1 |
| chrY | 21977878 | 21978880 | 3,75 | -1,1 | 4,85 | 6,73E-0 5 | 0,00813 | Distal Intergenic | -71053 | ENSG00000012817 | KDM5D |
| chr2 | 11414820 | 11416195 | 3,62 | -1,1 | 4,72 | 6,79E-05 | 0,00816 | Intron (uc002rbd.1/9475, intron 3 of 32) | 68516 | ENSG00000 134318 | ROCK2 |
| chr5 | 112425742 | 112427472 | 4.19 | -0.84 | 5.03 | 6.90E-05 | 0.00825 | Intron (uc003kqj.4/4163. intron 6 of 16) | 113335 | ENSG00000172795 | DCP2 |
| chr6 | 57072442 | 57073554 | 3,83 | -1,1 | 4,92 | 7,01E-0 5 | 0,00834 | Exon (uc003pds.3/51715, exon 3 of7) | 12703 | ENSG00000 112210 | RAB23 |
| chr6 | 151814809 | 151816332 | 4,7 | -0,15 | 4,86 | 7,16E-05 | 0,00848 | Promoter (<=1kb) | 0 | ENSG00000120262 | CCDC170 |
| chr11 | 55652500 | 55654522 | 3,13 | -1,1 | 4,22 | 7,24E-05 | 0,00852 | Promoter (<=1kb) | 0 | ENSG00000124900 | TRIM51 |
| chr17 | 41195689 | 41196695 | 3,8 | -1,1 | 4,9 | 7,48E-05 | 0,0087 | 3'UTR | 18431 | ENSG00000108830 | RND2 |
| chr9 | 18056648 | 18057609 | 2.97 | -0.52 | 3.48 | 7.50E-05 | 0.0087 | Distal Intergenic | 320947 | ENSG00000107295 | SH3 GL2 |
| chr22 | 50063449 | 50065163 | 5,62 | 0,67 | 4,94 | 7,54E-05 | 0,00873 | Distal Intergenic | -12259 | ENSG00000188511 | C22orf34 |
| chr6 | 90030889 | 90032021 | 3,53 | -1,1 | 4,62 | 7,91E-0 5 | 0,009 | Distal Intergenic | -5871 | ENSG00000111886 | GABRR2 |
| chr4 | 83327856 | 83329204 | 3,39 | -1,1 | 4,49 | 8,00E-05 | 0,00905 | Distal Intergenic | 22174 | ENSG00000152795 | HNRNPDL |
| chr17 | 76741003 | 76741557 | 2,94 | -1,1 | 4,04 | 8,04E-05 | 0,00906 | Intron (uc002jvw.3/9267, intron 1 of 12) | -21171 | ENSG00000108669 | CYTH1 |
| chr10 | 126101958 | 126103228 | 3,44 | -1,1 | 4,53 | 8,13E-05 | 0,0091 | Intron (uc001lhp.3/4942, intron 1 of 9) | 4317 | ENSG00000065154 | OAT |
| chr15 | 99481633 | 99482502 | 3,1 | -1,1 | 4,2 | 8,14E-05 | 0,0091 | Exon (uc002bul.3/3480, exon 18 of 21) | 13879 | ENSG00000140443 | IGF1R |
| chr12 | 31723409 | 31725037 | 3,46 | -1,1 | 4,56 | 8,18E-05 | 0,00913 | Intron (uc001ikh.1/160518, intron 1 of22) | -17820 | ENSG00000255867 | DENND5B-ASI |
| chr7 | 127333705 | 127335491 | 3,66 | -1,1 | 4,76 | 8,50E-05 | 0,0094 | Exon (uc003vmi.3/27044, exon 3 of 24) | -23159 | ENSG00000 197157 | SND1 |
| chr9 | 136651410 | 136652906 | 4,05 | -0,24 | 4,29 | 8,89E-05 | 0,00963 | Promoter (2-3kb) | 2443 | ENSG00000160293 | VAV2 |
| chr11 | 77011902 | 77012906 | 3,48 | -1,1 | 4,58 | 8,94E-05 | 0,00964 | Distal Intergenic | -13439 | ENSG00000178795 | GDPD4 |
| chr9 | 109796932 | 109797975 | 3,1 | -1,1 | 4,2 | 9,I0E-05 | 0,00973 | Intron (uc022bli.1/100313841, intron 1 of 1) | 50741 | ENSG000002213 31 | MIR548Q |
| chr2 | 197081453 | 197082543 | 3,07 | -1,1 | 4,17 | 9,30E-05 | 0,00984 | Exon (uc002utl.1/57520, exon 25 of 27) | -40226 | ENSG000000813 20 | STK17B |
| chr1 | 161731668 | 161732557 | 3,82 | -1,1 | 4,92 | 9,55E-0 5 | 0,01 | Distal Intergenic | -3477 | ENSG00000118217 | ATF6 |
| chr5 | 50159307 | 50160001 | 2,62 | -1,1 | 3,72 | 9,65E-05 | 0,01 | Distal Intergenic | 196535 | ENSG00000151883 | PARP8 |
| chr16 | 4161555 | 4164293 | 5,45 | 0,47 | 4,98 | 9,97E-05 | 0,0102 | Promoter (1-2kb) | 1893 | ENSG00000162104 | ADCY9 |
| chr7 | 64290790 | 64292344 | 4,31 | -0,08 | 4,39 | 0,000104 | 0,0104 | 5'UTR | 36024 | ENSG00000197008 | ZNF138 |
| chr1 | 149902122 | 149903767 | 5,47 | 0,31 | 5,15 | 0,000108 | 0,0108 | Promoter (1-2kb) | -1978 | ENSG00000143368 | SF3B4 |
| chr21 | 44399860 | 44401045 | 3,64 | -1,1 | 4,74 | 0,000111 | 0,0109 | Intron (uc002zcp.1/5316, intron 1 of 9) | 5217 | ENSG00000160199 | PKNOX1 |
| chr12 | 125271134 | 125271966 | 3.64 | -1.1 | 4.73 | 0.000 112 | 0.0109 | Exon (uc001ugm.4/949, exon 10 of 13) | 76553 | ENSG00000073060 | SCARB1 |
| chr11 | 71362554 | 71365012 | 3,69 | -1,1 | 4,79 | 0,000 112 | 0,0109 | Distal Intergenic | -68633 | ENSG00000204571 | KRTAP5-11 |
| chr22 | 38039021 | 38040625 | 3,49 | -1,1 | 4,59 | 0,000113 | 0,011 | Promoter (2-3kb) | 2628 | ENSG00000 100092 | SH3BP1 |
| chr11 | 3886574 | 3888188 | 4,16 | 0,04 | 4,12 | 0,000 114 | 0,011 | Intron (uc001lyv.2/6786, intron 1 of 11) | 9282 | ENSG00000284525 | MIR4687 |
| chr1 | 110008661 | 110010219 | 6,35 | 1,28 | 5,07 | 0,000 114 | 0,011 | Promoter (<=1kb) | 0 | ENSG00000143028 | SYPL2 |
| chr3 | 32857191 | 32861764 | 7.9 | 2.5 | 5.4 | 0.000114 | 0,011 | Promoter (<=1kb) | 0 | ENSG00000206557 | TRIM71 |
| chr10 | 93732937 | 93734088 | 3,65 | -1,1 | 4,75 | 0,000115 | 0,011 | Intron (uc001khr.3/9044, intron 14 of 37) | 6543 | ENSG00000095564 | BTAF1 |
| chr16 | 72311780 | 72312545 | 3,27 | -1,1 | 4,37 | 0,000 116 | 0,0111 | Distal Intergenic | -105431 | ENSG00000118557 | PMFBP1 |
| chr7 | 96650361 | 96652478 | 5,08 | -0,04 | 5,12 | 0,000116 | 0,0111 | Promoter (1-2kb) | 1665 | ENSG00000105880 | DLX5 |
| chr3 | 74558944 | 74560325 | 3,01 | -1,1 | 4,11 | 0,000117 | 0,0111 | Intron (uc003dpm.1/5067, intron 1 of 21) | 10018 | ENSG00000113805 | CNTN3 |
| chr13 | 24191399 | 24192030 | 2,63 | -1,1 | 3,73 | 0,000118 | 0,0112 | Intron (uc001uot.3/55504, intron 4 of 9) | 37900 | ENSG00000127863 | TNFRSF19 |
| chr9 | 116224499 | 116227675 | 6,34 | 0,92 | 5,42 | 0,00012 | 0,0113 | Promoter (<=1kb) | 0 | ENSG00000138835 | RGS3 |
| chr20 | 21272294 | 21273745 | 4,35 | -0,51 | 4,86 | 0,000122 | 0,0114 | Distal Intergenic | -10197 | ENSG00000088930 | XRN2 |
| chr5 | 101833089 | 101835031 | 3,63 | -1,1 | 4,73 | 0,000123 | 0,0114 | Promoter (<=1kb) | 0 | ENSG00000205359 | SLCO6A1 |
| chr13 | 115024255 | 115035599 | 9,34 | 3,56 | 5,79 | 0,000123 | 0,0114 | Exon (uc001vuk.1/8881, exon 14 of 19) | -11460 | ENSG00000169062 | UPF3A |
| chr11 | 120110573 | 120111320 | 3,72 | -0,93 | 4,65 | 0,000124 | 0,0114 | Promoter (<=1kb) | 0 | ENSG00000137709 | POU2F3 |
| chr16 | 68340348 | 68341334 | 2,91 | -0,93 | 3,84 | 0,000125 | 0,0114 | Intron (uc002evw.2/84138, intron 2 of 4) | 3534 | ENSG00000103061 | SLC7A6OS |
| chr17 | 43690677 | 43692524 | 3,39 | -1.1 | 4.48 | 0.000125 | 0.0114 | Distal Intergenic | -5186 | ENSG00000120088 | CRHR1 |
| chr10 | 126420316 | 126422469 | 5,22 | 0,18 | 5,04 | 0,000125 | 0,0114 | Intron (uc001lhu.1/9679, intron 5 of 8) | 10461 | ENSG00000189319 | FAM53B |
| chr18 | 11302 | 11813 | 2,45 | -1,1 | 3,54 | 0,000126 | 0,0115 | Distal Intergenic | -97252 | ENSG00000263006 | ROCK1P1 |
| chr1 | 157466578 | 157467646 | 3 | -1,1 | 4,1 | 0,000126 | 0,0115 | Distal Intergenic | 54664 | ENSG00000143297 | FCRL5 |
| chr19 | 3808232 | 3809219 | 3,53 | -1,1 | 4,63 | 0,000129 | 0,0117 | Exon (uc002lyw.2/23217, exon 17 of 19) | -6422 | ENSG00000007264 | MATK |
| chr4 | 141489586 | 141490663 | 5.03 | 0.2 | 4.83 | 0.00013 | 0.0117 | Promoter (<=1kb) | 0 | ENSG00000109424 | UCP1 |
| chr2 | 85583577 | 85584695 | 3,56 | -1,1 | 4,66 | 0,000134 | 0,012 | Promoter (<=1kb) | 0 | ENSG00000115459 | ELMOD3 |
| chr17 | 26997339 | 26998390 | 3,54 | -1,1 | 4,64 | 0,000138 | 0,0122 | Intron (uc002hby.3/6830, intron 1 of 36) | 8037 | ENSG00000 109111 | SUPT6H |
| chr11 | 66686842 | 66688088 | 4,02 | -0,4 | 4,42 | 0,00014 | 0,0123 | Intron (uc001ojo.1/5091. intron 3 of22) | -11502 | ENSG00000173599 | PC |
| chr17 | 79448035 | 79449016 | 4,46 | -0,43 | 4,89 | 0,00014 | 0,0123 | Distal Intergenic | -29821 | ENSG00000266189 | MIR3186 |
| chr6 | 83851046 | 83852290 | 3,56 | -1,1 | 4,66 | 0,000144 | 0,0124 | Exon (uc003pjs.1/23033, exon 24 of 39) | 4154 | ENSG00000083097 | DOP1A |
| chr16 | 68309320 | 68310658 | 3,13 | -1,1 | 4,23 | 0,000147 | 0,0126 | Promoter (<=1kb) | 726 | ENSG00000103064 | SLC7 A6 |
| chr11 | 130451675 | 130452251 | 2,54 | -0,64 | 3,18 | 0,000148 | 0,0127 | Distal Intergenic | -90600 | ENSG00000 175728 | C11orf44 |
| chr19 | 22805336 | 22806580 | 4,98 | 0,6 | 4,38 | 0,000148 | 0,0127 | Intron (uc002nqv.3/uc002nqv.3, intron 1 of 1 | -10546 | ENSG00000229676 | ZNF492 |
| chr4 | 178378831 | 178380290 | 3,52 | -1,1 | 4,61 | 0,00015 | 0,0127 | Intron (uc003iux.1/uc003iux.1, intron 2 of 5 | -15174 | ENSG00000038002 | AGA |
| chr19 | 13965145 | 13966351 | 3,56 | -1,1 | 4,65 | 0,000152 | 0,0128 | Distal Intergenic | -17672 | NA | LOC284454 |
| chr15 | 40695435 | 40696254 | 3,59 | -1,1 | 4,68 | 0,000156 | 0,0131 | Promoter (1-2kb) | -1432 | ENSG00000 128928 | IVD |
| chr22 | 17587157 | 17588243 | 3,6 | -1,1 | 4,69 | 0,000156 | 0,0131 | Intron (uc002zly.4/23765, intron 11 of 12) | 13970 | ENSG00000183307 | TMEM121B |
| chr2 | 242048584 | 242049595 | 3,5 | -1,1 | 4,6 | 0,00016 | 0,0133 | Intron (uc0 10zol.2/23178, intron 12 of 14) | -6837 | ENSG00000122085 | MTERF4 |
| chr7 | 69552870 | 69553920 | 3,47 | -1,1 | 4,56 | 0,000164 | 0,0134 | Intron (uc003tvv.4/26053, intron 2 of 4) | 488965 | ENSG00000158321 | AUTS2 |
| chr1 | 174840703 | 174842225 | 3,04 | -1,1 | 4,14 | 0,000166 | 0,0136 | Promoter (1-2kb) | -1299 | ENSG00000152061 | RABGAP1L |
| chr16 | 22368103 | 22369793 | 3,88 | -0,31 | 4,19 | 0,000168 | 0,0137 | Intron (uc002dkn.3/1039, intron 2 of 4) | 16145 | ENSG00000140743 | CDR2 |
| chr12 | 112781855 | 112783172 | 3,85 | -0,53 | 4,38 | 0,00017 | 0,0137 | Intron (uc021reb.1/283450, intron 1 of 75) | 36724 | ENSG00000 173064 | HECTD4 |
| chr12 | 50644901 | 50646351 | 3,52 | -1,1 | 4,62 | 0,000177 | 0,0141 | Intron (uc001rwj.4/51474, intron 1 of 10) | -16906 | ENSG00000221604 | MIR1293 |
| chr12 | 46272549 | 46273560 | 3.43 | -1.1 | 4.53 | 0.000178 | 0.0141 | Intron (uc001ror.3/196528. intron 16 of 19) | 28730 | ENSG00000189079 | ARID2 |
| chr12 | 22377680 | 22378983 | 3,83 | -0,87 | 4,71 | 0,000178 | 0,0141 | Intron (uc001rfo.4/6489, intron 4 of 4) | 108665 | ENSG00000111728 | ST8SIA1 |
| chr5 | 179480535 | 179481826 | 4,58 | -0,2 | 4,78 | 0,000178 | 0,0141 | Intron (uc003mll.1/55819, intron 1 of 8) | 17283 | ENSG00000 113269 | RNF130 |
| chr12 | 133010205 | 133010898 | 2,72 | -0,29 | 3,01 | 0,000179 | 0,0141 | Distal Intergenic | -56259 | ENSG00000 112787 | FBRSL1 |
| chr1 | 154474271 | 154475629 | 4,71 | 0 | 4,71 | 0,000181 | 0,0142 | Promoter (<=1kb) | 0 | ENSG00000163239 | TDRD10 |
| chr7 | 155754195 | 155754996 | 2.77 | -1.1 | 3.87 | 0.000187 | 0.0145 | Distal Intergenic | -149228 | ENSG00000164690 | SHH |
| chr14 | 106518289 | 106519015 | 2,95 | -0,96 | 3,91 | 0,00019 | 0,0146 | Exon (uc031qqx.1/uc031qqx.1, exon 2523 o | -79931 | ENSG00000271968 | ADAM6 |
| chr11 | 63718290 | 63720509 | 4,88 | -0,04 | 4,91 | 0,00019 | 0,0146 | Promoter (<=1kb) | 0 | ENSG00000110583 | NAA40 |
| chr12 | 32486970 | 32489755 | 5,03 | 0,15 | 4,88 | 0,000191 | 0,0146 | Promoter (<=1kb) | 0 | ENSG00000151746 | BICD1 |
| chr9 | 93702894 | 93704866 | 3,93 | -0,73 | 4,66 | 0,000196 | 0,0148 | Distal Intergenic | 96754 | ENSG00000165025 | SYK |
| chr2 | 212388832 | 212389819 | 3,43 | -1,1 | 4,53 | 0,000205 | 0,0152 | Intron (uc002veg.1/2066, intron 20 of 27) | 901265 | ENSG00000221782 | MIR548F2 |
| chr19 | 58565249 | 58567198 | 5,59 | 1,5 | 4,1 | 0,000215 | 0,0158 | 3'UTR | -3409 | ENSG00000176293 | ZNF135 |
| chr10 | 33218064 | 33219276 | 3,4 | -1,1 | 4,49 | 0,000216 | 0,0158 | Exon (uc001iwr.4/3688, exon 3 of 16) | 5210 | ENSG00000150093 | ITGB1 |
| chr1 | 228943035 | 228943857 | 2,83 | -1,1 | 3,93 | 0,000219 | 0,0159 | Distal Intergenic | 72211 | ENSG00000116574 | RHOU |
| chr17 | 41128353 | 41129264 | 3,68 | -0,84 | 4,52 | 0,00022 | 0,016 | Promoter (2-3kb) | 2756 | ENSG00000108825 | PTGES3L-AARSE D1 |
| chr15 | 40482645 | 40483794 | 3,34 | -1,1 | 4,44 | 0,000221 | 0,016 | Intron (uc001zkx.4/701, intron 8 of 22) | 13967 | ENSG00000156970 | BUB1B |
| chr7 | 99738292 | 99739552 | 3,85 | -0,79 | 4,64 | 0,000221 | 0,016 | Distal Intergenic | -6978 | ENSG00000188186 | LAMTOR4 |
| chr6 | 108620453 | 108621634 | 3.34 | -1.1 | 4.44 | 0.000223 | 0.016 | Intron (uc003psj.3/246269, intron 1 of 12) | 4355 | ENSG00000135537 | AFG1L |
| chr4 | 125878937 | 125880293 | 3,4 | -1,1 | 4,49 | 0,000224 | 0,016 | Distal Intergenic | -245050 | ENSG00000151458 | ANKRD5 0 |
| chr9 | 124115438 | 124116854 | 3,36 | -1,1 | 4,46 | 0,000227 | 0,0161 | Exon (uc004blh.4/2040, exon 4 of 7) | 15728 | ENSG00000148175 | STOM |
| chr8 | 145613671 | 145614590 | 3,4 | -1,1 | 4,49 | 0,00023 | 0,0162 | Promoter (2-3kb) | -2505 | ENSG00000173137 | ADCK5 |
| chr3 | 75557481 | 75558747 | 3,29 | -0,79 | 4,08 | 0,000232 | 0,0162 | Distal Intergenic | -73215 | ENSG00000244026 | FAM86DP |
| chr9 | 100615261 | 100616311 | 3.55 | -0.57 | 4.12 | 0.000232 | 0.0162 | Promoter (<=1kb) | 0 | ENSG00000178919 | FOXE1 |
| chr12 | 112808802 | 112809981 | 3,36 | -1,1 | 4,45 | 0,000232 | 0,0162 | Intron (uc021reb.1/283450, intron 1 of 75) | 9915 | ENSG00000173064 | HECTD4 |
| chr12 | 120687410 | 120688923 | 3,37 | -1,1 | 4,47 | 0,000232 | 0,0162 | Promoter (<=1kb) | 0 | ENSG00000089159 | PXN |
| chr15 | 49558237 | 49559351 | 3,02 | -1,1 | 4,12 | 0,000233 | 0,0162 | Intron (uc001zxi.1/2585, intron 5 of 9) | 95794 | ENSG00000156958 | GALK2 |
| chr14 | 63905840 | 63907116 | 3,03 | -1,1 | 4,12 | 0,000233 | 0,0162 | Intron (uc0 10tsf.1/5529, intron 2 of 12) | 67839 | ENSG00000154001 | PPP2R5E |
| chr7 | 97848801 | 97850177 | 3,07 | -1,1 | 4,16 | 0,000235 | 0,0163 | Intron (uc003upg.4/25851, intron 23 of 25) | 7179 | ENSG00000180535 | BHLHA15 |
| chr16 | 2713229 | 2714827 | 3,3 | -1,1 | 4,39 | 0,000243 | 0,0167 | Exon (uc010bsr.2/100507321, exon 3 of 3) | 8613 | NA | ERVK13-1 |
| chr6 | 1378832 | 1380476 | 5,26 | 0,48 | 4,79 | 0,000243 | 0,0167 | Distal Intergenic | -9593 | ENSG00000137273 | FOXF2 |
| chr10 | 21869919 | 21871110 | 3,29 | -1,1 | 4,39 | 0,000246 | 0,0167 | Intron (uc001iqs.3/8028, intron 3 of 23) | -34931 | ENSG00000078403 | MLLT10 |
| chr1 | 25531626 | 25533364 | 4,38 | -0,16 | 4,54 | 0,000246 | 0,0167 | Distal Intergenic | 25649 | ENSG00000 117614 | SYF2 |
| chr17 | 17917331 | 17918053 | 3,13 | -1,1 | 4,23 | 0,00025 | 0,0169 | Intron (uc021trj.1/83450, intron 13 of 14) | 24427 | ENSG00000171953 | ATPAF2 |
| chr3 | 133392942 | 133393857 | 3,25 | -1,1 | 4,35 | 0,000251 | 0,0169 | Distal Intergenic | -12205 | ENSG00000163781 | TOPBP1 |
| chr20 | 37356701 | 37358387 | 4,57 | 0,13 | 4,44 | 0,000251 | 0,0169 | 3'UTR | 3596 | ENSG00000101438 | SLC32A1 |
| chr10 | 135091652 | 135092381 | 3,1 | -0,48 | 3,59 | 0,000252 | 0,0169 | Promoter (1-2kb) | -1245 | ENSG00000151651 | ADAM8 |
| chr12 | 66660701 | 66662526 | 4,45 | 0 | 4,45 | 0,000252 | 0,0169 | Distal Intergenic | -33809 | ENSG00000127311 | HELB |
| chr7 | 44755591 | 44756521 | 3,27 | -1,1 | 4,37 | 0,000253 | 0,017 | Distal Intergenic | -31659 | ENSG00000122515 | ZMIZ2 |
| chr1 | 220186380 | 220187983 | 3,3 | -1,1 | 4,39 | 0,000254 | 0,017 | Intron (uc021pjd.1/26828, intron 1 of 1) | 32017 | ENSG00000136628 | EPRS |
| chr1 | 165896301 | 165897489 | 4,87 | 0,3 | 4,57 | 0,000258 | 0,0171 | Distal Intergenic | 19143 | ENSG00000283936 | MIR3658 |
| chr1 | 15898144 | 15899230 | 3,31 | -1,1 | 4,41 | 0,000259 | 0,0172 | 3'UTR | 12123 | ENSG00000116138 | DNAJC16 |
| chr5 | 157076448 | 157080003 | 6.35 | 1.55 | 4.8 | 0.000263 | 0.0173 | Promoter (<=1kb) | 0 | ENSG00000039600 | SOX30 |
| chr19 | 2961795 | 2962647 | 3,07 | -1,1 | 4,17 | 0,000264 | 0,0173 | Distal Intergenic | -14889 | ENSG00000104953 | TLE6 |
| chr15 | 63790949 | 63791784 | 3,26 | -1,1 | 4,36 | 0,000268 | 0,0174 | Distal Intergenic | -4926 | ENSG00000140455 | USP3 |
| chr3 | 195881544 | 195882514 | 3,78 | -0,48 | 4,26 | 0,000271 | 0,0175 | Intron (uc011bts.2/401109, intron 2 of 2) | 11918 | ENSG00000224652 | LINC00885 |
| chr3 | 12684776 | 12687355 | 4,26 | -0,03 | 4,29 | 0,000272 | 0,0176 | Intron (uc003bxf.4/5894, intron 1 of 16) | 18345 | ENSG00000132155 | RAF1 |
| chr19 | 53547185 | 53548718 | 3.26 | -1.1 | 4.36 | 0.000278 | 0.0177 | Promoter (<=1kb) | 0 | ENSG00000268964 | ERVV-2 |
| chr14 | 91867708 | 91871171 | 5,29 | 0,7 | 4,58 | 0,000278 | 0,0177 | Intron (uc010aty.3/440193, intron 3 of 29) | 12011 | ENSG00000015133 | CCDC88C |
| chr4 | 175255712 | 175257754 | 3,3 | -1,1 | 4,4 | 0,00028 | 0,0178 | Distal Intergenic | -50310 | ENSG00000 164117 | FBXO8 |
| chr7 | 75141283 | 75142922 | 2,95 | -1,1 | 4,04 | 0,000284 | 0,018 | Exon (uc003udq.3/5387, exon 6 of 8) | 14531 | ENSG00000127957 | PMS2P3 |
| chr11 | 393782 | 395620 | 4,76 | 0,24 | 4,52 | 0,000291 | 0,0184 | Promoter (<=1kb) | 0 | ENSG00000184363 | PKP3 |
| chr10 | 21923744 | 21925335 | 3,32 | -1,1 | 4,41 | 0,000296 | 0,0185 | Intron (uc001iqs.3/8028, intron 8 of 23) | 17703 | ENSG00000078403 | MLLT10 |
| chr3 | 98243925 | 98245855 | 4,62 | 0,57 | 4,04 | 3.00E-04 | 0,0187 | Promoter (2-3kb) | -2015 | ENSG00000080822 | CLDND1 |
| chr1 | 229569683 | 229570580 | 2,79 | -1,1 | 3,89 | 0,000305 | 0,0188 | Promoter (<=1kb) | 0 | ENSG00000143632 | ACTA1 |
| chr2 | 202060047 | 202061080 | 3,09 | -0,65 | 3,74 | 0,000308 | 0,0189 | Exon (uc002uxi.1/843, exon 5 of 6) | 12426 | ENSG00000003400 | CASP10 |
| chr8 | 70946336 | 70947590 | 3,77 | -0,68 | 4,45 | 0,000308 | 0,0189 | Distal Intergenic | 35972 | ENSG00000147596 | PRDM14 |
| chr7 | 47989824 | 47992099 | 4,68 | 0,25 | 4,44 | 0,00031 | 0,019 | Promoter (1-2kb) | -1753 | ENSG00000158683 | PKD1L1 |
| chr1 | 213211219 | 213212321 | 3,18 | -1,1 | 4,28 | 0,000312 | 0,019 | Distal Intergenic | -12267 | ENSG00000136643 | RPS6KC1 |
| chr3 | 185060416 | 185061200 | 3.07 | -1.1 | 4.16 | 0.000314 | 0.0191 | Intron (uc0 10hyf.3/9175, intron 2 of 14) | 13733 | ENSG00000073803 | MAP3K13 |
| chr2 | 224773120 | 224774426 | 3,21 | -1,1 | 4,3 | 0,000322 | 0,0194 | Intron (uc002vnq.3/57590, intron 3 of 11) | 35626 | ENSG00000085449 | WDFY1 |
| chr7 | 108886380 | 108886998 | 2,81 | -0,9 | 3,71 | 0,000328 | 0,0196 | Distal Intergenic | -361743 | ENSG00000205174 | C7orf66 |
| chr1 | 89883244 | 89884652 | 2,85 | -1,1 | 3,94 | 0,000328 | 0,0196 | Intron (uc009wcv.1/400759, intron 2 of 4) | 10006 | ENSG00000225492 | GBP1P1 |
| chr10 | 72043294 | 72044247 | 4,66 | 0,16 | 4,5 | 0,000328 | 0,0196 | Promoter (<=1kb) | 0 | ENSG00000148734 | NPFFR1 |
| chr3 | 97747827 | 97748399 | 2.44 | -0.93 | 3.38 | 0.000332 | 0.0198 | Intron (uc021xbo.1/200959. intron 2 of 9) | 5749 | ENSG00000183185 | GABRR3 |
| chr7 | 138577747 | 138578769 | 3,15 | -1,1 | 4,25 | 0,000332 | 0,0198 | Intron (uc011kqi.2/57670, intron 2 of 11) | 5196 | ENSG00000122778 | KIAA1549 |
| chr16 | 596621 | 597828 | 2,94 | -1,1 | 4,04 | 0,000334 | 0,0198 | 5'UTR | 3344 | ENSG00000266235 | MIR3176 |
| chr19 | 53724890 | 53726423 | 3,22 | -1,1 | 4,31 | 0,000339 | 0,02 | Distal Intergenic | 8689 | NA | ZNF818P |
| chr5 | 139633098 | 139634211 | 3,15 | -0,65 | 3,8 | 0,000344 | 0,0201 | Intron (uc010jfi.3/84418, intron 2 of3) | 48478 | ENSG00000113068 | PFDN1 |
| chr18 | 1036731 | 1037507 | 2,92 | -0,95 | 3,87 | 0,000345 | 0,0201 | Distal Intergenic | 131534 | ENSG00000141433 | ADCYAP1 |
| chr22 | 50232047 | 50232891 | 3,21 | -0,81 | 4,01 | 0,000345 | 0,0201 | Distal Intergenic | -10851 | ENSG00000100425 | BRD1 |
| chr6 | 16124457 | 16125351 | 2,97 | -0,65 | 3,62 | 0,000347 | 0,0201 | Distal Intergenic | -3966 | ENSG00000007944 | MYLIP |
| chr20 | 32361715 | 32362800 | 3,14 | -1,1 | 4,24 | 0,000347 | 0,0201 | Intron (uc002wzx.3/84905, intron 10 of 14) | -12094 | ENSG00000131061 | ZNF341 |
| chr12 | 59202931 | 59203814 | 3,04 | -1,1 | 4,14 | 0,000353 | 0,0203 | Distal Intergenic | 109513 | ENSG00000139263 | LRIG3 |
| chr15 | 89148013 | 89148715 | 2,71 | -0,84 | 3,55 | 0,000371 | 0,021 | Promoter (2-3kb) | -2623 | ENSG00000221630 | MIR1179 |
| chr6 | 169795934 | 169797228 | 3,38 | -0,93 | 4,31 | 0,000371 | 0,021 | Distal Intergenic | -141797 | ENSG00000186340 | THBS2 |
| chr16 | 80040175 | 80041010 | 2,81 | -1,1 | 3,91 | 0,000372 | 0,0211 | Distal Intergenic | -405553 | ENSG00000 178573 | MAF |
| chr10 | 102984920 | 102985634 | 3,24 | -0,4 | 3,64 | 0,000379 | 0,0213 | Downstream (1-2kb) | 3083 | ENSG00000138136 | LBX1 |
| chr17 | 1901238 | 1902259 | 3,97 | -0,43 | 4,4 | 0,00038 | 0,0213 | Intron (uc002ftp.3/146760, intron 1 of 1) | 25919 | ENSG00000185924 | RTN4RL1 |
| chr5 | 176625977 | 176626694 | 3,08 | -0,95 | 4,03 | 0,000382 | 0,0214 | Intron (uc003mfp.2/64324, intron 3 of 3) | 63889 | ENSG00000165671 | NSD1 |
| chr9 | 38020212 | 38021319 | 3,13 | -1,1 | 4,23 | 0,000382 | 0,0214 | Intron (uc004aax.3/6461, intron 1 of 5) | 47891 | ENSG00000107338 | SHB |
| chr14 | 93389384 | 93389984 | 2,65 | -0,93 | 3,58 | 0,000384 | 0,0214 | Promoter (<=1kb) | 0 | ENSG00000100604 | CHGA |
| chr18 | 38655160 | 38656102 | 2,82 | -1,1 | 3,92 | 0,000384 | 0,0214 | Distal Intergenic | 444459 | ENSG00000267313 | KC6 |
| chr5 | 78842500 | 78843500 | 3.07 | -1.1 | 4.17 | 0.000387 | 0.0214 | Distal Intergenic | -32841 | ENSG00000152413 | HOMER1 |
| chr14 | 91857938 | 91867546 | 8,63 | 6,91 | 1,72 | 0,000388 | 0,0214 | Intron (uc010aty.3/440193, intron 3 of 29) | 15636 | ENSG00000015133 | CCDC88C |
| chr2 | 45240385 | 45241727 | 5,42 | 0,83 | 4,59 | 0,000388 | 0,0214 | Distal Intergenic | -3843 | ENSG00000170577 | SIX2 |
| chr12 | 30860661 | 30861895 | 3,21 | -1,1 | 4,31 | 0,000389 | 0,0214 | Downstream (<1kb) | -11732 | ENSG00000133704 | IPO8 |
| chr10 | 32539008 | 32540256 | 2,71 | -1,1 | 3,81 | 0,00039 | 0,0214 | Distal Intergenic | 95890 | ENSG00000120616 | EPC1 |
| chr6 | 44113049 | 44113763 | 2.76 | -1.1 | 3.86 | 0.000392 | 0.0215 | Intron (uc003owq.1/55362, intron 10 of 14) | 5604 | ENSG00000137216 | TMEM63B |
| chr18 | 9517622 | 9518454 | 3,09 | -1,1 | 4,19 | 0,000395 | 0,0215 | Exon (uc021ugv.1/uc021ugv.1, exon 1 of 1) | 42092 | ENSG00000017797 | RALBP1 |
| chr17 | 40142821 | 40144076 | 3,13 | -1,1 | 4,23 | 0,000399 | 0,0217 | Intron (uc010wgb.2/7266, intron 5 of 13) | 24062 | ENSG00000173786 | CNP |
| chr9 | 116135123 | 116136653 | 4,62 | 0,63 | 3,98 | 0,000405 | 0,0218 | Promoter (1-2kb) | 1688 | ENSG00000119431 | HDHD3 |
| chr12 | 130823559 | 130825014 | 3,03 | -1,1 | 4,13 | 0,000405 | 0,0218 | Promoter (1-2kb) | 1126 | ENSG00000125207 | PIWIL1 |
| chr12 | 175693 | 176436 | 2,46 | -0,78 | 3,24 | 0,000407 | 0,0218 | Promoter (<=1kb) | 0 | ENSG00000120645 | IQSEC3 |
| chr2 | 113381854 | 113382897 | 2,98 | -1,1 | 4,07 | 0,000407 | 0,0218 | Distal Intergenic | 18860 | NA | FLJ42351 |
| chr1 | 184570511 | 184571666 | 3,08 | -1,1 | 4,18 | 0,000409 | 0,0219 | Intron (uc001gqv.1/81563, intron 5 of 5) | 152375 | ENSG00000116406 | EDEM3 |
| chr17 | 1266311 | 1267966 | 3,73 | -0,17 | 3,9 | 0,000414 | 0,022 | Intron (uc002fsj.3/7531, intron 2 of 5) | 35590 | ENSG00000108953 | YWHAE |
| chr5 | 153856932 | 153858225 | 2,94 | -1,1 | 4,04 | 0,000417 | 0,0221 | Promoter (<=1kb) | 0 | ENSG00000113196 | HAND1 |
| chr2 | 48089753 | 48090988 | 3,16 | -1,1 | 4,25 | 0,000418 | 0,0221 | Intron (uc010fbl.3/80204, intron 1 of 22) | 24870 | ENSG00000138081 | FBXO11 |
| chr10 | 104059537 | 104060666 | 3,07 | -1,1 | 4,16 | 0,000422 | 0,0222 | Intron (uc001kuw.3/8729, intron 3 of 9) | 54282 | ENSG00000107862 | GBF1 |
| chr19 | 52034601 | 52035644 | 3.84 | -0.18 | 4.02 | 0.000423 | 0.0222 | Promoter (<=1kb) | 0 | ENSG00000105492 | SIGLEC6 |
| chr1 | 36321970 | 36322828 | 3,54 | -0,65 | 4,19 | 0,000423 | 0,0222 | 3'UTR | -12581 | ENSG00000092847 | AGO1 |
| chr16 | 71950197 | 71951191 | 3,17 | -0,64 | 3,81 | 0,000424 | 0,0223 | Exon (uc010cgh.2/9798, exon 4 of 11) | -5186 | ENSG00000182149 | IST1 |
| chr4 | 12748307 | 12751107 | 4,66 | 0,12 | 4,54 | 0,000432 | 0,0225 | Distal Intergenic | 734882 | ENSG00000157869 | RAB28 |
| chr1 | 193460642 | 193461413 | 2,74 | -1,1 | 3,84 | 0,000433 | 0,0225 | Distal Intergenic | -304899 | ENSG00000162630 | B3GALT2 |
| chr7 | 90364137 | 90365642 | 3.83 | -0.52 | 4.34 | 0.000436 | 0.0226 | Intron (uc003ukt.1/5218, intron 6 of7) | 24961 | ENSG00000058091 | CDK14 |
| chr4 | 169432465 | 169435735 | 6,02 | 1,12 | 4,91 | 0,000445 | 0,0228 | 5'UTR | 14248 | ENSG00000129116 | PALLD |
| chr5 | 180525773 | 180526501 | 3 | -1,1 | 4,1 | 0,000446 | 0,0228 | Distal Intergenic | 25803 | ENSG00000185372 | OR2V1 |
| chr7 | 25007600 | 25008380 | 2,87 | -1,1 | 3,97 | 0,000452 | 0,0229 | Intron (uc003sxf3/26031, intron 1 of22) | 11380 | ENSG00000070882 | OSBPL3 |
| chr16 | 30563138 | 30564340 | 3,01 | -1,1 | 4,11 | 0,000466 | 0,0233 | Downstream (<1kb) | 5302 | ENSG00000169951 | ZNF764 |
| chr1 | 151189935 | 151190913 | 3 | -1,1 | 4,1 | 0,000468 | 0,0233 | Intron (uc021oyo.1/8394, intron 1 of 13) | -15760 | ENSG00000143398 | PIP5K1A |
| chr14 | 91850641 | 91851802 | 2,97 | -1,1 | 4,07 | 0,000472 | 0,0234 | Intron (uc010aty.3/440193, intron 3 of 29) | 31380 | ENSG00000015133 | CCDC88C |
| chr19 | 45811105 | 45812301 | 4,21 | -0,2 | 4,41 | 0,000486 | 0,0239 | Exon (uc002pbd.4/115 8, exon 6 of 8) | 13934 | ENSG00000104879 | CKM |
| chr17 | 64395768 | 64396645 | 2,51 | -0,84 | 3,35 | 0,000489 | 0,024 | Intron (uc002jfo.1/5578, intron 2 of 14) | 96842 | ENSG00000154229 | PRKCA |
| chr17 | 80263724 | 80264429 | 3,2 | -0,93 | 4,13 | 0,000498 | 0,0242 | Distal Intergenic | 11051 | ENSG00000173762 | CD7 |
| chr8 | 126343110 | 126344344 | 3,6 | -0,64 | 4,24 | 0,000499 | 0,0242 | Intron (uc003yrw.2/286053, intron 5 of 7) | -98219 | ENSG00000173334 | TRIB1 |
| chr18 | 59514262 | 59515090 | 2,54 | -0,93 | 3,48 | 5,00E-04 | 0,0242 | Intron (uc0021ih.1/220441, intron 1 of 1) | 45214 | ENSG00000176641 | RNF152 |
| chr17 | 59481345 | 59483206 | 5,49 | 1,33 | 4,16 | 0,000502 | 0,0243 | 3'UTR | 4088 | ENSG00000121068 | TBX2 |
| chr3 | 185444348 | 185445505 | 3,68 | -0,62 | 4,3 | 0,000508 | 0,0245 | Intron (uc003fps.4/646600, intron 5 of 7) | 13308 | ENSG00000163915 | IGF2BP2-AS1 |
| chr10 | 35645158 | 35645630 | 2,77 | -1,1 | 3,87 | 0,000512 | 0,0246 | Intron (uc001iyu.4/219771, intron 3 of 11) | 19356 | ENSG00000108100 | CCNY |
| chr18 | 51737351 | 51738179 | 2,2 | -1,1 | 3,3 | 0,000513 | 0,0246 | Intron (uc0021fg.2/8932, intron 1 of 6) | 10603 | ENSG00000207233 | SNORA37 |
| chr4 | 14864339 | 14864998 | 2,95 | -1,1 | 4,05 | 0,000514 | 0,0246 | Intron (uc003gne.3/uc003gne.3, intron 1 of 6 | 131131 | ENSG00000247624 | CPEB2-DT |
| chr17 | 27955393 | 27956509 | 2,97 | -1,1 | 4,07 | 0,000514 | 0,0246 | 3'UTR | -6952 | ENSG00000167549 | CORO6 |
| chr19 | 22990004 | 22990748 | 3,66 | -0,58 | 4,23 | 0,000514 | 0,0246 | Distal Intergenic | -23031 | ENSG00000213973 | ZNF99 |
| chr21 | 46437809 | 46439167 | 4.83 | 0.68 | 4.15 | 0.000515 | 0.0246 | Distal Intersenic | -13167 | ENSG00000275874 | PICSAR |
| chr5 | 167618691 | 167619529 | 2,63 | -1,1 | 3,72 | 0,000519 | 0,0247 | Intron (uc010jjd.3/57451, intron 14 of28) | 66836 | ENSG00000145934 | TENM2 |
| chr9 | 134594898 | 134596277 | 3,95 | 0,41 | 3,54 | 0,000522 | 0,0248 | Intron (uc022bot.1/2889, intron 1 of 23) | -9669 | ENSG00000107263 | RAPGEF1 |
| chr17 | 48981639 | 48982715 | 2,72 | -1,1 | 3,82 | 0,000526 | 0,025 | Distal Intergenic | -36300 | ENSG00000141232 | TOB1 |
| chr3 | 47125019 | 47125937 | 2,95 | -1,1 | 4,04 | 0,000528 | 0,025 | Promoter (<=1kb) | 0 | ENSG00000181555 | SETD2 |
| chr16 | 3744012 | 3747044 | 6.1 | 1.07 | 5.03 | 0.000537 | 0.0253 | Intron (uc002cvt.4/10131. intron 1 of 17) | -15707 | ENSG00000126602 | TRAP1 |
| chr10 | 102585842 | 102591021 | 8,97 | 7,29 | 1,68 | 0,00054 | 0,0254 | 3'UTR | 77233 | ENSG00000075891 | PAX2 |
| chr4 | 5891675 | 5892838 | 4,18 | 0,18 | 4 | 0,000541 | 0,0254 | Promoter (<=1kb) | 0 | ENSG00000072832 | CRMP1 |
| chr11 | 111842878 | 111844170 | 3,08 | -0,79 | 3,86 | 0,000542 | 0,0254 | Intron (uc001pmj.4/85458, intron 4 of 5) | -3863 | ENSG00000150764 | DIXDC1 |
| chr14 | 21114498 | 21115123 | 2,78 | -1,1 | 3,88 | 0,000547 | 0,0256 | Distal Intergenic | -4648 | ENSG00000181803 | OR6S1 |
| chr9 | 71597949 | 71598903 | 2,79 | -1 | 3,79 | 0,000555 | 0,0258 | Intron (uc004agu.4/8395, intron 14 of 15) | 30136 | ENSG00000165059 | PRKACG |
| chr1 | 169877973 | 169880876 | 5,88 | 1,03 | 4,84 | 0,000561 | 0,026 | Distal Intergenic | -14873 | ENSG00000000457 | SCYL3 |
| chr1 | 146549690 | 146552793 | 5,72 | 1,37 | 4,36 | 0,000566 | 0,0261 | Exon (uc021ovj.1/uc021ovj.1, exon 1 of 1) | 33135 | NA | NBPF13P |
| chr11 | 111814662 | 111815506 | 2,81 | -1,1 | 3,9 | 0,000567 | 0,0261 | Intron (uc001pmj.4/85458, intron 2 of 5) | 6735 | ENSG00000150764 | DIXDC1 |
| chr17 | 48980351 | 48981581 | 3,43 | -0,46 | 3,89 | 0,000578 | 0,0265 | Distal Intergenic | -35012 | ENSG00000141232 | TOB1 |
| chr12 | 775538 | 776748 | 4,36 | -0,02 | 4,37 | 0,000596 | 0,0269 | Promoter (2-3kb) | -2783 | ENSG00000171840 | NINJ2 |
| chr10 | 15919882 | 15920708 | 3,87 | -0,42 | 4,29 | 0,000597 | 0,0269 | Distal Intergenic | -17363 | ENSG00000148481 | MINDY3 |
| chr18 | 145984 | 148035 | 5.02 | 0.42 | 4.6 | 0.000599 | 0.0269 | Distal Intergenic | -10448 | ENSG00000101557 | USP14 |
| chr3 | 196005549 | 196009090 | 5,92 | 1,15 | 4,78 | 0,000599 | 0,0269 | Intron (uc003fwg.3/5130, intron 2 of9) | 5494 | ENSG00000161217 | PCYT1A |
| chr16 | 90113310 | 90114252 | 3 | -0,93 | 3,94 | 0,000606 | 0,0271 | Promoter (<=1kb) | 0 | ENSG00000222019 | URAHP |
| chr8 | 61858942 | 61860533 | 3,52 | -0,53 | 4,05 | 0,000612 | 0,0273 | Distal Intergenic | 19774 | NA | LOC100130298 |
| chr7 | 91195866 | 91197213 | 3,49 | -0,65 | 4,14 | 0,000622 | 0,0276 | Distal Intergenic | 302083 | ENSG00000157240 | FZD1 |
| chr5 | 170743141 | 170744176 | 2.84 | -1.1 | 3.94 | 0.000623 | 0.0276 | Distal Intergenic | 6853 | ENSG00000164438 | TLX3 |
| chr1 | 160683653 | 160684696 | 2,35 | -1,1 | 3,45 | 0,00064 | 0,0279 | Promoter (2-3kb) | -2012 | ENSG00000117091 | CD48 |
| chr3 | 121532181 | 121533487 | 3,1 | -0,93 | 4,04 | 0,000646 | 0,028 | Intron (uc003eek.2/9657, intron 5 of 11) | 20439 | ENSG00000173226 | IQCB1 |
| chr5 | 176023427 | 176024609 | 3,37 | -0,65 | 4,02 | 0,00065 | 0,0281 | 3'UTR | 12522 | ENSG00000 169258 | GPRIN1 |
| chr2 | 54584537 | 54585414 | 3,42 | -0,57 | 3,98 | 0,000658 | 0,0283 | Intron (uc002rxs.1/129852, intron 2 of 3) | 26466 | ENSG00000 177994 | C2orf73 |
| chr7 | 74585655 | 74588535 | 3,45 | -0,63 | 4,08 | 0,000662 | 0,0284 | Promoter (<=1kb) | 0 | ENSG00000165178 | NCF1C |
| chr6 | 45630930 | 45631999 | 4,06 | -0,29 | 4,34 | 0,000662 | 0,0284 | Distal Intergenic | 241016 | ENSG00000124813 | RUNX2 |
| chr1 | 247274053 | 247275914 | 6,97 | 2,3 | 4,67 | 0,000665 | 0,0285 | Promoter (<=1kb) | 0 | ENSG00000221953 | C1orf229 |
| chr3 | 6902300 | 6903786 | 3,77 | 0,5 | 3,27 | 0,000666 | 0,0285 | Promoter (<=1kb) | 0 | ENSG00000196277 | GRM7 |
| chr11 | 73741474 | 73742166 | 3,35 | -0,65 | 3,99 | 0,000667 | 0,0285 | Intron (uc001out.3/26005, intron 11 of 12) | -21192 | ENSG00000175564 | UCP3 |
| chr11 | 120374333 | 120374961 | 2,71 | -1,1 | 3,8 | 0,000677 | 0,0288 | Distal Intergenic | -7507 | ENSG00000149403 | GRIK4 |
| chr3 | 169384311 | 169384830 | 2,74 | -0,81 | 3,55 | 0,000679 | 0,0288 | Promoter (2-3kb) | -2748 | ENSG00000085276 | MECOM |
| chr2 | 64706060 | 64706564 | 2,42 | -1,1 | 3,52 | 0,000686 | 0,029 | Distal Intergenic | 24733 | ENSG00000119862 | LGALSL |
| chr8 | 103827416 | 103828729 | 5,04 | 1,49 | 3,55 | 0,000689 | 0,029 | Distal Intergenic | 47668 | ENSG00000155096 | AZIN1 |
| chr4 | 90174169 | 90175172 | 2,85 | -1,1 | 3,95 | 0,000693 | 0,0292 | Intron (uc003hsm.1/285513, intron 1 of 1) | 53989 | ENSG00000185477 | GPRIN3 |
| chr17 | 46175191 | 46176095 | 2,96 | -0,98 | 3,94 | 7.00E-04 | 0,0294 | Promoter (2-3kb) | 2465 | ENSG00000108468 | CBX1 |
| chr5 | 68670243 | 68671455 | 4,86 | 0,67 | 4,19 | 7.00E-04 | 0,0294 | Promoter (2-3kb) | 2962 | ENSG00000152942 | RAD17 |
| chr18 | 23974138 | 23974870 | 2,48 | -1,1 | 3,58 | 0,000705 | 0,0295 | Distal Intergenic | 81985 | ENSG00000134504 | KCTD1 |
| chr19 | 19648890 | 19649562 | 2,75 | -1,1 | 3,85 | 0,000715 | 0,0298 | Promoter (<=1kb) | 0 | ENSG00000160161 | CILP2 |
| chr6 | 25298463 | 25299504 | 3.34 | -0.59 | 3.93 | 0.000724 | 0.0301 | Intron (uc031smx.1/55604, intron 2 of8) | 18807 | ENSG00000079691 | CARMIL1 |
| chr6 | 37285327 | 37286077 | 2,48 | -1,1 | 3,57 | 0,000732 | 0,0302 | Intron (uc003onn.3/55633, intron 12 of 12) | 11677 | ENSG00000065491 | TBC1D22B |
| chr1 | 54232126 | 54233261 | 3,27 | -0,76 | 4,03 | 0,000735 | 0,0302 | 3'UTR | -32249 | ENSG00000174332 | GLIS1 |
| chr22 | 45898178 | 45901537 | 6,82 | 2,53 | 4,29 | 0,000737 | 0,0303 | Promoter (<=1kb) | 0 | ENSG00000077942 | FBLN1 |
| chr5 | 150591327 | 150591759 | 1,91 | -1,1 | 3 | 0,000745 | 0,0304 | Intron (uc0031tq.3/26112, intron 1 of 8) | -6291 | ENSG00000198624 | CCDC69 |
| chr16 | 75808980 | 75809666 | 2.42 | -1.1 | 3.51 | 0.000754 | 0.0307 | Distal Intergenic | -126439 | ENSG00000065427 | KARS |
| chr2 | 233219660 | 233220872 | 3,96 | -0,13 | 4,09 | 0,000755 | 0,0307 | Distal Intergenic | -22476 | ENSG00000163283 | ALPP |
| chr3 | 99307802 | 99308501 | 2,25 | -1,1 | 3,35 | 0,000757 | 0,0307 | Intron (uc021xbq.11100313938, intron 1 of 1 | -48939 | ENSG00000144810 | COL8A1 |
| chr12 | 45650536 | 45651574 | 3,05 | -0,96 | 4,02 | 0,000758 | 0,0307 | Intron (uc031qha.1/196527, intron 1 of 17) | -34892 | ENSG00000177119 | ANO6 |
| chr13 | 90535895 | 90536793 | 3,26 | -0,7 | 3,96 | 0,000759 | 0,0307 | Distal Intergenic | 235178 | ENSG00000261446 | LINC00559 |
| chr15 | 85331456 | 85332234 | 2,96 | -0,4 | 3,36 | 0,000761 | 0,0308 | Intron (uc002bld.3/9640, intron 4 of 10) | 10042 | ENSG00000166716 | ZNF592 |
| chr10 | 134217960 | 134219279 | 3,98 | -0,26 | 4,24 | 0,000773 | 0,0311 | Exon (uc001111.4/170394, exon 2 of 3) | 7258 | ENSG00000171813 | PWWP2B |
| chr12 | 94256 | 94777 | 2,92 | -0,93 | 3,86 | 0,000777 | 0,0312 | Distal Intergenic | 5999 | ENSG00000226210 | WASH8P |
| chr3 | 10644428 | 10645711 | 4,34 | 0,65 | 3,69 | 0,000787 | 0,0314 | Intron (uc003bvw.3/491, intron 2 of 21) | -97160 | ENSG00000157087 | ATP2B2 |
| chr5 | 131484830 | 131485830 | 2,98 | -0,63 | 3,61 | 0,000794 | 0,0315 | Distal Intergenic | 75345 | ENSG00000164400 | CSF2 |
| chr5 | 169688946 | 169689856 | 2,7 | -1,1 | 3,79 | 0,000794 | 0,0315 | Exon (uc011des.1/3937, exon 4 of 13) | 4408 | ENSG00000043462 | LCP2 |
| chr2 | 9736193 | 9738140 | 4,36 | -0,04 | 4,4 | 0,000802 | 0,0317 | Intron (uc002qzx.3/10971, intron 2 of 5) | 32966 | ENSG00000134308 | YWHAQ |
| chr12 | 105076210 | 105077270 | 3.88 | -0,29 | 4.17 | 0.000812 | 0.032 | Intron (uc001tky.3/50515, intron 2 of 2) | 90799 | ENSG00000264295 | MIR3922 |
| chr1 | 157486473 | 157491665 | 7,71 | 2,32 | 5,39 | 0,000815 | 0,0321 | Exon (uc001fqu.3/83416, exon 11 of 17) | 30645 | ENSG00000143297 | FCRL5 |
| chr5 | 37362869 | 37363616 | 2,76 | -1,1 | 3,86 | 0,000817 | 0,0321 | Intron (uc003jkt.1/9631, intron 3 of 34) | 7271 | ENSG00000113569 | NUP155 |
| chr4 | 71539146 | 71539954 | 2,2 | -1,1 | 3,3 | 0,000836 | 0,0326 | Distal Intergenic | -6798 | ENSG00000132465 | JCHAIN |
| chr2 | 9757535 | 9759208 | 4,1 | -0,21 | 4,31 | 0,000842 | 0,0328 | Intron (uc002qzx.3/10971, intron 2 of 5) | 11898 | ENSG00000134308 | YWHAQ |
| chr1 | 120636179 | 120637163 | 2,29 | -1.1 | 3,39 | 0.000843 | 0.0328 | Distal Intergenic | -23862 | ENSG00000134250 | NOTCH2 |
| chr20 | 35934323 | 35934861 | 2,32 | -1,1 | 3,41 | 0,000865 | 0,0333 | Intron (uc002xgu.3/63905, intron 3 of 3) | 16272 | ENSG00000101363 | MANBAL |
| chr15 | 56299080 | 56300222 | 4,29 | 0,37 | 3,92 | 0,00087 | 0,0334 | Distal Intergenic | -13245 | ENSG00000069869 | NEDD4 |
| chr9 | 140033061 | 140033892 | 2,63 | -1,1 | 3,73 | 0,000871 | 0,0334 | Promoter (<=1kb) | 0 | ENSG00000176884 | GRIN1 |
| chr15 | 43376390 | 43377913 | 4,17 | -0,05 | 4,22 | 0,000871 | 0,0334 | Intron (uc001zqq.3/197131, intron 2 of 46) | 20373 | ENSG00000159459 | UBR1 |
| chr4 | 60383958 | 60385110 | 4,13 | 0,21 | 3,91 | 0,000873 | 0,0335 | Distal Intergenic | -1681864 | ENSG00000150471 | ADGRL3 |
| chr19 | 53773130 | 53774405 | 2,74 | -1,1 | 3,83 | 0,000883 | 0,0337 | Promoter (2-3kb) | -2212 | ENSG00000228567 | VN1R4 |
| chrX | 102947705 | 102948443 | 2,7 | -1,1 | 3,8 | 0,000886 | 0,0337 | Distal Intergenic | -4619 | ENSG00000123562 | MORF4L2 |
| chr19 | 5953376 | 5956161 | 4,76 | 0,45 | 4,3 | 0,000891 | 0,0338 | Intron (uc002mdw.3/8498, intron 2 of 16) | -18903 | ENSG00000031823 | RANBP3 |
| chr5 | 138970400 | 138974428 | 5,54 | 1,23 | 4,32 | 0,000892 | 0,0338 | Intron (uc0031eq.3/7322, intron 2 of 7) | 29649 | ENSG00000131508 | UBE2D2 |
| chr19 | 10883798 | 10884803 | 3,22 | -0,78 | 4 | 0,000893 | 0,0338 | Intron (uc010dxk2/1785, intron 4 of 13) | -6127 | ENSG00000265879 | MIR4748 |
| chr18 | 47929516 | 47930708 | 3,58 | -0,22 | 3,8 | 0,000896 | 0,0339 | Distal Intergenic | 28124 | ENSG00000154839 | SKA1 |
| chr11 | 76750540 | 76751438 | 2,97 | -0,55 | 3,52 | 0,000898 | 0,0339 | Exon (uc021qnp.1/192134, exon 2 of 2) | 5105 | ENSG00000198488 | B3GNT6 |
| chr17 | 1274517 | 1276813 | 4,6 | 0,45 | 4,15 | 0,000898 | 0,0339 | Intron (uc002fsj.3/7531, intron 1 of 5) | 26743 | ENSG00000108953 | YWHAE |
| chr7 | 55611532 | 55612436 | 2,64 | -1,1 | 3,73 | 0,000906 | 0,034 | Intron (uc010kzi.3/81552, intron 1 of4) | -5179 | ENSG00000154978 | VOPP1 |
| chr6 | 76138841 | 76139364 | 2,18 | -1,1 | 3,28 | 0,000917 | 0,0342 | Intron (uc003phy.1/27145, intron 1 of6) | 64132 | ENSG00000118407 | FILIP 1 |
| chr6 | 33832275 | 33833311 | 3,2 | -0,79 | 3,98 | 0,000922 | 0,0344 | Distal Intergenic | 27216 | ENSG00000249346 | LINC01016 |
| chr21 | 34807320 | 34807795 | 2,42 | -1,1 | 3,51 | 0,000923 | 0,0344 | Intron (uc002yrp.4/3460, intron 6 of 6) | 32118 | ENSG00000159128 | IFNGR2 |
| chr2 | 202305947 | 202306807 | 2,69 | -1,1 | 3,78 | 0,000925 | 0,0344 | Intron (uc002uyb.4/66008, intron 1 of 15) | 9512 | ENSG00000115993 | TRAK2 |
| chr7 | 25086553 | 25089330 | 5.63 | 0.98 | 4.65 | 0.000926 | 0.0344 | Distal Intergenic | -66793 | ENSG00000070882 | OSBPL3 |
| chr4 | 71436610 | 71437401 | 2,72 | -0,3 | 3,02 | 0,000932 | 0,0345 | Distal Intergenic | -20574 | ENSG00000178522 | AMBN |
| chr10 | 14569543 | 14570577 | 2,87 | -0,72 | 3,6 | 0,000935 | 0,0345 | Intron (uc001imx.1/83641, intron 2 of 3) | 20050 | ENSG00000065809 | FAM107B |
| chr11 | 119101631 | 119102212 | 2,69 | -1,1 | 3,78 | 0,000935 | 0,0345 | Intron (uc001pwe.4/867, intron 1 of 15) | 24645 | ENSG00000110395 | CBL |
| chr7 | 47802793 | 47803801 | 3,85 | -0,05 | 3,9 | 0,000935 | 0,0345 | Promoter (1-2kb) | 1719 | ENSG00000146666 | LINC00525 |
| chr5 | 31721086 | 31721617 | 2,21 | -1.1 | 3,31 | 0.000939 | 0.0346 | Intron (uc003jh1.3/23037, intron 1 of 24) | -77379 | ENSG00000133401 | PDZD2 |
| chr15 | 45098715 | 45099665 | 2,75 | -1,1 | 3,85 | 0,000941 | 0,0346 | Distal Intergenic | 70175 | ENSG00000185880 | TRIM69 |
| chr1 | 25422019 | 25423433 | 3,36 | -0,65 | 4,01 | 0,000942 | 0,0347 | Distal Intergenic | -130407 | ENSG00000020633 | RUNX3 |
| chr18 | 45438904 | 45439887 | 3,23 | -0,53 | 3,76 | 0,00095 | 0,0347 | Intron (uc0021cy.4/4087, intron 1 of 10) | 17083 | ENSG00000175387 | SMAD2 |
| chr14 | 70104344 | 70105150 | 2,08 | -1,1 | 3,18 | 0,000967 | 0,0351 | Intron (uc001xlk3/9766, intron 1 of 5) | 26034 | ENSG00000100647 | SUSD6 |
| chr3 | 57239883 | 57241017 | 3,67 | -0,42 | 4,08 | 0,000969 | 0,0352 | Distal Intergenic | -5603 | ENSG00000163666 | HESX1 |
| chr1 | 230270809 | 230271602 | 2,61 | -1,1 | 3,71 | 0,000973 | 0,0352 | Intron (uc010pvy.1/2590, intron 1 of 15) | 67853 | ENSG00000143641 | GALNT2 |
| chr1 | 242081798 | 242082475 | 2,65 | -1,1 | 3,75 | 0,000973 | 0,0352 | Distal Intergenic | 69765 | ENSG00000174371 | EXO1 |
| chr2 | 233215507 | 233217387 | 4,95 | 0,73 | 4,23 | 0,000973 | 0,0352 | Distal Intergenic | -25961 | ENSG00000163283 | ALPP |
| chr10 | 121376515 | 121379462 | 6,58 | 2,28 | 4,3 | 0,000977 | 0,0353 | Distal Intergenic | -19974 | ENSG00000151923 | TIAL1 |
| chr15 | 70281919 | 70282262 | 2,04 | -1,1 | 3,13 | 0,00098 | 0,0354 | Distal Intergenic | 84684 | ENSG00000140332 | TLE3 |
| chr17 | 77682177 | 77682643 | 2,22 | -1 | 3,22 | 0,000985 | 0,0354 | Promoter (1-2kb) | -1119 | ENSG00000266665 | MIR4739 |
| chr4 | 10015034 | 10015575 | 2.53 | -0.93 | 3.46 | 0.000986 | 0.0354 | Intron (uc003gmc.3/56606, intron 2 of 11) | 7539 | ENSG00000109667 | SLC2A9 |
| chr5 | 15656072 | 15656813 | 2,65 | -1,1 | 3,75 | 0,000991 | 0,0355 | Intron (uc003jfn.1/23194, intron 2 of 3) | 155767 | ENSG00000183580 | FBXL7 |
| chr22 | 46285353 | 46286251 | 3,2 | -0,64 | 3,84 | 0,001 | 0,0357 | Distal Intergenic | 81993 | ENSG00000188064 | WNT7B |
| chr3 | 177526601 | 177527193 | 3,22 | -0,6 | 3,82 | 0,00101 | 0,0357 | Distal Intergenic | 366892 | NA | LINC00578 |
| chr1 | 2165347 | 2166203 | 2,45 | -1,1 | 3,55 | 0,00102 | 0,0359 | Intron (uc001aja.4/6497, intron 1 of 6) | 5213 | ENSG00000157933 | SKI |
| chr6 | 42597209 | 42598303 | 2.85 | -0.93 | 3.79 | 0.00102 | 0.0359 | Intron (uc011dur.2/23304, intron 11 of 46) | 12354 | ENSG00000024048 | UBR2 |
| chr19 | 54006519 | 54008633 | 4,27 | 0,43 | 3,84 | 0,00102 | 0,0359 | Exon (uc010eqq.1/126017, exon 3 of 3) | -15544 | ENSG00000130844 | ZNF331 |
| chr6 | 42879207 | 42880000 | 3,8 | -0,18 | 3,97 | 0,00102 | 0,0359 | Distal Intergenic | -3727 | ENSG00000171611 | PTCRA |
| chr10 | 74435926 | 74437097 | 3,92 | -0,22 | 4,14 | 0,00102 | 0,0359 | Distal Intergenic | -14792 | ENSG00000156026 | MCU |
| chr10 | 1781113 | 1782074 | 3,03 | -0,65 | 3,67 | 0,00103 | 0,0361 | Promoter (1-2kb) | -1443 | ENSG00000185736 | ADARB2 |
| chr7 | 97680261 | 97681053 | 3,56 | 0,19 | 3,37 | 0,00104 | 0,0362 | Distal Intergenic | -55144 | ENSG00000164715 | LMTK2 |
| chr6 | 161419505 | 161420361 | 2,61 | -1,1 | 3,71 | 0,00104 | 0,0362 | Intron (uc0 10kkc2/4216, intron 1 of 22) | 6683 | ENSG00000085511 | MAP3K4 |
| chr19 | 17434316 | 17434941 | 2,64 | -1,1 | 3,74 | 0,00105 | 0,0365 | 3'UTR | 10697 | ENSG00000074855 | ANO8 |
| chr3 | 27503844 | 27507316 | 6,21 | 1,49 | 4,72 | 0,00105 | 0,0365 | Intron (uc011aww.3/9497, intron 1 of25) | -5599 | ENSG00000033867 | SLC4A7 |
| chr20 | 62959002 | 62960046 | 2,92 | -0,46 | 3,38 | 0,00106 | 0,0366 | Distal Intergenic | 37264 | NA | LINC00266-1 |
| chr19 | 34143215 | 34143815 | 2,79 | -0,98 | 3,77 | 0,00106 | 0,0366 | Intron (uc002nus.4/64377, intron 1 of 4) | 30354 | ENSG00000124302 | CHST8 |
| chr15 | 90577013 | 90578395 | 3,04 | -0,77 | 3,82 | 0,00106 | 0,0366 | Intron (uc002boy.2/374655, intron 1 of 4) | 32261 | ENSG00000140548 | ZNF710 |
| chrX | 144902732 | 144903815 | 3,54 | -0,37 | 3,91 | 0,00106 | 0,0366 | Promoter (<=1kb) | 0 | ENSG00000185985 | SLIIRK2 |
| chr6 | 137818427 | 137819496 | 3,88 | -0,11 | 3,99 | 0,00106 | 0,0366 | Promoter (2-3kb) | -2896 | ENSG00000177468 | OLIG3 |
| chr17 | 75363191 | 75363710 | 2,64 | -0,1 | 2,75 | 0,00108 | 0,037 | Intron (uc002jts.4/10801, intron 2 of 11) | -5562 | ENSG00000184640 | SEPTIN9 |
| chr2 | 219737844 | 219738554 | 2,88 | -0,65 | 3,54 | 0,0011 | 0,0373 | Exon (uc002vjc.1/7475, exon 4 of 4) | -6701 | ENSG00000135925 | WNT10A |
| chr6 | 16725021 | 16725820 | 2,55 | -1,1 | 3,65 | 0,0011 | 0,0372 | Intron (uc003nbt.3/6310, intron 3 of 8) | 24504 | ENSG00000124788 | ATXN1 |
| chr19 | 16743078 | 16743640 | 2,6 | -1,1 | 3,7 | 0,0011 | 0,0373 | Distal Intergenic | -4063 | ENSG00000105085 | MED26 |
| chr3 | 179367060 | 179368095 | 4,16 | 0,41 | 3,75 | 0,00112 | 0,0377 | Promoter (2-3kb) | -2838 | ENSG00000058056 | USP13 |
| chr11 | 36196327 | 36197523 | 3.64 | -0,31 | 3.96 | 0,00114 | 0.0381 | Intron (uc001mwk.1/143458, intron 4 of 5) | 76450 | ENSG00000179241 | LDLRAD3 |
| chr19 | 21796912 | 21797981 | 3,49 | -0,2 | 3,7 | 0,00115 | 0,0382 | Distal Intergenic | 108475 | ENSG00000197013 | ZNF429 |
| chr2 | 18286236 | 18286730 | 2,13 | -1,1 | 3,23 | 0,00117 | 0,0386 | Distal Intergenic | 226291 | ENSG00000170745 | KCNS3 |
| chr20 | 32434821 | 32435578 | 2,44 | -1,1 | 3,54 | 0,00117 | 0,0388 | Intron (uc002xaa.3/128866, intron 1 of 4) | 35711 | ENSG00000101421 | CHMP4B |
| chr5 | 166887192 | 166888731 | 4,03 | 0,11 | 3,92 | 0,00117 | 0,0387 | Intron (uc021yhi.1/57451, intron 2 of 3) | 175349 | ENSG00000145934 | TENM2 |
| chr3 | 177463691 | 177464517 | 2.58 | -1.1 | 3.68 | 0.00118 | 0,0389 | Intron (uc031sch.1/100505566, intron 3 of 3 | 303982 | NA | LINC00578 |
| chr1 | 49255558 | 49256450 | 2,97 | -0,62 | 3,59 | 0,00119 | 0,0391 | Intron (uc001cru.2/84871, intron 6 of 13) | -13011 | ENSG00000162373 | BEND5 |
| chr16 | 81047799 | 81048341 | 2,56 | -1,1 | 3,66 | 0,00119 | 0,0392 | Intron (uc002ffw.4/55839, intron 3 of 6) | -7297 | ENSG00000103121 | CMC2 |
| chr2 | 3610362 | 3613004 | 5,07 | 0,67 | 4,4 | 0,0012 | 0,0392 | Distal Intergenic | 4386 | ENSG00000234171 | RNASEH1-AS1 |
| chr18 | 56761526 | 56762502 | 2,74 | -0,98 | 3,72 | 0,00121 | 0,0394 | Distal Intergenic | -44623 | ENSG00000166562 | SEC11C |
| chr17 | 61732173 | 61732887 | 2,61 | -1,1 | 3,71 | 0,00122 | 0,0397 | Intron (uc002jbe.3/4215, intron 6 of 17) | 32372 | ENSG00000198909 | MAP3K3 |
| chr6 | 56732296 | 56732901 | 2,54 | -1,1 | 3,64 | 0,00123 | 0,0398 | Intron (uc021zba.2/667, intron 3 of 96) | -15582 | ENSG00000151914 | DST |
| chr2 | 164204552 | 164205312 | 3 | -0,71 | 3,71 | 0,00123 | 0,0398 | Distal Intergenic | 387201 | ENSG00000182263 | FIGN |
| chr5 | 15824155 | 15825057 | 3,55 | -0,06 | 3,61 | 0,00126 | 0,0404 | Intron (uc003jfn.1/23194, intron 2 of 3) | 266169 | ENSG00000183654 | MARCH11 |
| chr5 | 74937625 | 74939089 | 2,8 | -0,9 | 3,7 | 0,00127 | 0,0407 | Intron (uc010izt.4/728780, intron 1 of6) | 30324 | ENSG00000189045 | ANKDD1B |
| chr21 | 42043281 | 42044437 | 2,7 | -0,98 | 3,68 | 0,00128 | 0,041 | Intron (uc002yyq.1/1826, intron 3 of 32) | -40588 | ENSG00000233756 | DSCAM-IT1 |
| chr3 | 180635896 | 180636764 | 3,07 | -0,19 | 3,26 | 0,00129 | 0,0411 | Promoter (2-3kb) | 2649 | ENSG00000 114416 | FXR1 |
| chr3 | 172007074 | 172007871 | 2.48 | -1,1 | 3.58 | 0.00129 | 0.0411 | Intron (uc003fhy.3/64778, intron 7 of 25) | 155813 | ENSG00000075420 | FNDC3B |
| chr17 | 57353860 | 57355378 | 4,44 | 0,52 | 3,93 | 0,00129 | 0,0411 | Distal Intergenic | -53675 | ENSG00000175155 | YPEL2 |
| chr7 | 95347902 | 95348407 | 2,44 | -1,1 | 3,54 | 0,0013 | 0,0413 | Distal Intergenic | -53411 | ENSG00000158560 | DYNC1I1 |
| chr19 | 48676617 | 48678087 | 4,28 | 0,22 | 4,06 | 0,00131 | 0,0414 | Intron (uc002pic.3/uc002pic.3, intron 2 of 4) | -3057 | ENSG00000105486 | LIG1 |
| chr17 | 37948849 | 37949784 | 2,24 | -1,1 | 3,34 | 0,00132 | 0,0416 | 5'UTR | -14371 | ENSG00000161405 | IKZF3 |
| chr5 | 72677268 | 72678229 | 2.47 | -1,1 | 3.57 | 0.00133 | 0.042 | Distal Intergenic | -116021 | ENSG00000145741 | BTF3 |
| chr18 | 20943410 | 20944145 | 2,59 | -1,1 | 3,69 | 0,00134 | 0,0421 | Intron (uc010xaq.2/85019, intron 11 of 15) | -32187 | ENSG00000 134490 | TMEM241 |
| chr7 | 148251353 | 148253541 | 5,11 | 0,95 | 4,16 | 0,00134 | 0,0421 | Distal Intergenic | -34116 | ENSG00000170279 | C7orf33 |
| chr1 | 17051120 | 17051933 | 2,55 | -1,1 | 3,65 | 0,00135 | 0,0423 | Distal Intergenic | -4468 | ENSG00000268869 | ESPNP |
| chr13 | 101261483 | 101263237 | 3,88 | -0,16 | 4,05 | 0,00135 | 0,0423 | Intron (uc001vot.3/84899, intron 17 of 18) | -20437 | ENSG00000 134864 | GGACT |
| chr1 | 52336886 | 52338919 | 5,09 | 1,03 | 4,06 | 0,00135 | 0,0422 | Intron (uc001cte.3/4898, intron 1 of 32) | 4793 | ENSG00000078618 | NRDC |
| chrX | 9555078 | 9555914 | 2,47 | -1,1 | 3,57 | 0,00136 | 0,0426 | Intron (uc004csq.4/6907, intron 3 of 17) | -95517 | ENSG00000101849 | TBL1X |
| chr19 | 52049496 | 52049938 | 1,86 | -1,1 | 2,96 | 0,00137 | 0,0427 | Distal Intergenic | -14386 | ENSG00000105492 | SIGLEC6 |
| chr18 | 47840371 | 47840857 | 2,01 | -1,1 | 3,11 | 0,00137 | 0,0427 | Distal Intergenic | -25679 | ENSG00000154832 | CXXC1 |
| chr22 | 46262650 | 46263467 | 2,37 | -1,1 | 3,47 | 0,00137 | 0,0427 | Distal Intergenic | 104777 | ENSG00000 188064 | WNT7B |
| chr1 | 35810504 | 35811765 | 4,84 | 0,51 | 4,33 | 0,00137 | 0,0426 | Intron (uc001byt.3/9202, intron 2 of 29) | -12761 | ENSG00000146463 | ZMYM4 |
| chr17 | 40144206 | 40144888 | 2,49 | -1,1 | 3,59 | 0,0014 | 0,0434 | Intron (uc010wgb.2/7266, intron 5 of 13) | 24295 | ENSG00000168259 | DNAJC7 |
| chr3 | 95463588 | 95464324 | 2,44 | -1,1 | 3,53 | 0,00141 | 0,0434 | Distal Intergenic | -61551 | ENSG00000244681 | MTHFD2P1 |
| chr10 | 134271106 | 134271901 | 2,64 | -0,33 | 2,98 | 0,00142 | 0,0438 | Distal Intergenic | 12392 | NA | C10orf91 |
| chr4 | 146693050 | 146695942 | 5,74 | 1,9 | 3,84 | 0,00142 | 0,0439 | 5'UTR | 48735 | ENSG00000151612 | ZNF827 |
| chr8 | 134067793 | 134068401 | 2,55 | -1,1 | 3,65 | 0,00143 | 0,0439 | Intron (uc003ytw.3/7038, intron 41 of 47) | 4202 | ENSG00000155926 | SLA |
| chr6 | 117591330 | 117592349 | 2,49 | -0,98 | 3,47 | 0,00144 | 0,044 | Exon (uc003pxn.3/245806, exon 3 of 4) | 4609 | ENSG00000170162 | VGLL2 |
| chr5 | 79854347 | 79856133 | 4,5 | 0,44 | 4,05 | 0,00145 | 0,0443 | 5'UTR | 8808 | ENSG00000189127 | ANKRD3 4 B |
| chr3 | 30672671 | 30673529 | 2,47 | -1,1 | 3,56 | 0,00146 | 0,0444 | Intron (uc021wut.1/7048, intron 1 of 3) | 24677 | ENSG00000 163513 | TGFBR2 |
| chr1 | 50884735 | 50885674 | 4.1 | 0.28 | 3.82 | 0.00146 | 0.0443 | Promoter (1-2kb) | 1630 | ENSG00000142700 | DMRTA2 |
| chr11 | 36627022 | 36628213 | 4,28 | 0,67 | 3,61 | 0,00148 | 0,0448 | Intron (uc001mwy.2/119710, intron 1 of 5) | -7193 | ENSG00000175097 | RAG2 |
| chr15 | 99936355 | 99937118 | 2,56 | -1,1 | 3,66 | 0,00148 | 0,0448 | Distal Intergenic | 138625 | ENSG00000259706 | HSP90B2P |
| chr9 | 123329964 | 123331076 | 3,88 | 0,15 | 3,73 | 0,00149 | 0,045 | 5'UTR | 11372 | ENSG00000 136861 | CDK5RAP2 |
| chr7 | 50464950 | 50465474 | 2,43 | -0,8 | 3,23 | 0,00155 | 0,046 | Intron (uc003tow.4/10320, intron 7 of 7) | 50208 | ENSG00000132436 | FIGNL1 |
| chr1 | 157369953 | 157371010 | 2.47 | -1,1 | 3.57 | 0,00155 | 0.046 | Distal Intergenic | 151300 | ENSG00000143297 | FCRL5 |
| chr12 | 53937409 | 53938618 | 3,43 | -0,18 | 3,61 | 0,00158 | 0,0466 | Intron (uc010sok.1/11016, intron 3 of 12) | 29641 | ENSG00000257550 | LOC100652999 |
| chr8 | 101313911 | 101315947 | 4,14 | 0,21 | 3,93 | 0,00158 | 0,0466 | Promoter (<=1kb) | 0 | ENSG00000034677 | RNF19A |
| chrX | 64391140 | 64392068 | 2,82 | -0,79 | 3,6 | 0,00159 | 0,0466 | Distal Intergenic | -136516 | ENSG00000126970 | ZC4H2 |
| chr5 | 553900 | 555019 | 3,78 | 0,05 | 3,74 | 0,00159 | 0,0467 | Distal Intergenic | -17903 | ENSG00000264233 | MIR4456 |
| chr7 | 92411804 | 92415885 | 5,36 | 1,19 | 4,16 | 0,00159 | 0,0466 | Intron (uc011khw.2/1021, intron 2 of7) | 47346 | ENSG00000105810 | CDK6 |
| chr6 | 158692424 | 158692746 | 1,75 | -1 | 2,75 | 0,00163 | 0,0476 | Distal Intergenic | -40946 | ENSG00000130338 | TULP4 |
| chr8 | 101309965 | 101313860 | 5,5 | 1,71 | 3,78 | 0,00166 | 0,048 | Promoter (1-2kb) | 1627 | ENSG00000034677 | RNF19A |
| chr8 | 127167812 | 127168833 | 2,3 | -1,1 | 3,4 | 0,00168 | 0,0485 | Distal Intergenic | -204371 | ENSG00000245164 | LINC0086 1 |
| chr2 | 47979511 | 47980727 | 4,11 | 0,24 | 3,86 | 0,00168 | 0,0485 | Distal Intergenic | -29494 | ENSG00000116062 | MSH6 |
| chr18 | 72338884 | 72340123 | 3,45 | -0,42 | 3,87 | 0,00169 | 0,0486 | Promoter (2-3kb) | -2800 | ENSG00000215421 | ZNF407 |
| chr9 | 140355717 | 140357179 | 5,57 | 1,35 | 4,22 | 0,00169 | 0,0486 | Promoter (1-2kb) | -1931 | ENSG00000165802 | NSMF |
| chr20 | 51831394 | 51832508 | 3.81 | 0.55 | 3.27 | 0.00171 | 0.0489 | Intron (uc002xwo.3/128553, intron 1 of 2) | 29572 | ENSG00000182463 | TSHZ2 |
| chr15 | 52850243 | 52851276 | 3,52 | -0,21 | 3,74 | 0,00171 | 0,0489 | Intron (uc002acd.1/10776, intron 1 of 2) | 9937 | ENSG00000128989 | ARPP19 |
| chr1 | 33457002 | 33458941 | 5,61 | 1,46 | 4,15 | 0,00171 | 0,0489 | Intron (uc001bwn.3/uc001bwn.3, intron 1 of | -26716 | ENSG00000116514 | RNF19B |
| chr3 | 121711394 | 121712611 | 3,18 | -0,52 | 3,7 | 0,00172 | 0,0492 | Exon (uc003eeq.3/286676, exon 5 of 6) | 13537 | ENSG00000145103 | ILDR1 |
| chr12 | 3600110 | 3601024 | 4,06 | 0,44 | 3,62 | 0,00173 | 0,0493 | Promoter (<=1kb) | 0 | ENSG00000 111218 | PRMT8 |
| chr2 | 225467210 | 225468012 | 3.41 | -0,39 | 3.8 | 0.00173 | 0.0493 | Distal Intergenic | -17096 | ENSG00000036257 | CUL3 |
| chr15 | 60864682 | 60868681 | 4,59 | 0,51 | 4,08 | 0,00173 | 0,0492 | Intron (uc002agt.4/6095, intron 1 of 9) | 16026 | ENSG00000069667 | RORA |
| chr2 | 25966553 | 25967336 | 2,33 | -1,1 | 3,43 | 0,00174 | 0,0493 | Exon (uc002rgs.2/55252, exon 12 of 12) | 35842 | ENSG00000143970 | ASXL2 |
| chr1 | 184334562 | 184335896 | 3,53 | -0,32 | 3,84 | 0,00177 | 0,0499 | Distal Intergenic | -20254 | ENSG00000116667 | C1 orf21 |
| chr15 | 85570221 | 85570910 | 2,41 | -1,1 | 3,5 | 0,00178 | 0,0499 | Intron (uc021stv.1/5151, intron 1 of 21) | -34818 | ENSG00000073417 | PDE8A |

## Claims

1. A method for the *in vitro* prognosis of the likelihood of an individual having multiple myeloma to be sensitive to a therapeutic treatment, comprising the following steps
a) Measuring, in a biological sample of the individual, the level of expression of at least five genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 ZNF5188B and/or disclosed in Table 1, Table 2, Table 3 or Table 4 said gene being associated to at least a modified histone selected from H3K4me1, H3K4me3, H3K9me3, H3K27ac and H3K27me3, to obtain an individual value
b) Comparing the individual value obtained in a) with a reference value,
c) Classifying the individual as sensitive to the treatment if the individual value is modified when compared with the reference value.

2. A method according to claim 1, comprising the following steps
a) Measuring in a biological sample, the level of expression of at least five genes selected from the group consisting of BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ,
b) Calculating a risk score from the expression level obtained in step a)
c) Comparing the risk score of the individual to a reference value, and if the risk score is higher than the reference value, classifying the individual as sensitive to the treatment by a drug which is selected from Bromodomain and Extra-Terminal motif (BET) inhibitors.

3. The method according to claim 2, wherein in step b), the at least 5 genes are selected from HK2, HNRNPC, HSPA9, ILF3, LDHB, MDH1, MYBPC2, NCL, NUDC, PARP1, PDIA6, PRPS1, RPL13A, RPL27A, SF3B2, SLC25A39, SMARCA4, STC2, TNPO2, TPR and YWHAQ, in particular from MYBPC2, NUDC, RPL27A, SF3BS, SLC25A39, TNPO2, TPR and YWHAQ.

4. The method according to any of claims 2 or 3, wherein in step c) the individual is classified as sensitive to a BET inhibitor selected from OTX015 (NCT01713582), CPI-0610 (NCT02157636), GSK525762 (NCT01943851), and RO6870810 (NCT03068351.

5. A method according to claim 1 comprising the following steps
a) Measuring in a biological sample, the level of expression of at least five genes selected from the group consisting of ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B,
b) Calculating a risk score from the expression level obtained in step a)
c) Comparing the risk score of the individual to a reference value, and if the risk score is higher than the reference value, classifying the individual as sensitive to the treatment by a drug modifying the level of H3K9me3 and H3K27me3.

6. The method according to claim 5, wherein in step c) the individual is classified as sensitive to a treatment by a drug selected from SUV39H1 inhibitors, SUV39H2 inhibitors and Polycomb repressive complex (PRC) 2 inhibitors.

7. The method according to claim 6 or 7, wherein the at least 5 genes in step a) are selected from ARHGEF5, DEF8, GRID2IP, HDAC9, HSPA1L, KDM4C, P4HA3, PAG1, SEMA6A, SFMBT2, THEMIS2, ZFP2 and ZNF518B.

8. The method according to any of claims 5 to 7 wherein in step c) the individual is classified as sensitive to a treatment by a PRC2 inhibitor selected from EZH2 inhibitor selected in a group comprising CPI-169, EI-1, EPZ-005687, EPZ-6438 (Tazemetostat), GSK-126, GSK-343, GSK-503, DZNep, UNC-1999 and EED inhibitor such asMAK683, or to a treatment by the SUV39H1 inhibitor which is chaetocin.

9. A method for the *in vitro* prognosis of the likelihood of a individual having multiple myeloma to be sensitive to a therapeutic treatment comprising the following steps
a) Measuring in a biological sample the presence and the level of H3K4me3 in at least five genes selected from genes listed in table 1 and/or table 2,
b) Classifying the individual having a significant level of H3K4me3 of at least five genes selected from the genes listed in table 1 and/or having no significant level of H3K4me3 of at least five genes selected from the genes listed in table 2 as sensitive to a drug which is an IMiD.

10. A method according to claim 1 comprising the following steps
a) Measuring in a biological sample the presence and the level of H3K4me3 in at least least five genes selected from the genes listed in table 3 and /or listed in Table 4,
b) Classifying the individual having a significant level of H3K4me3 of at least five genes selected from the genes listed in table 3 and/or having no significant level of H3K4me3 of at least five genes selected from the genes listed in table 4 as sensitive to a drug selected from histone desacetylase inhibitors (HDACi) such as romidepsin.

11. Kit for performing the method according to at least one of the preceding claims, comprising means for measuring the expression level of at least 5 genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR, YWHAQ, CUL4B, ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B.

12. Kit for performing a method according to any of claims 10 or 11 comprising antibodies directed to H3K4me3 and means for measuring the expression level of at least five genes selected from the genes listed in Table 1, in Table 2, in Table 3 or in Table 4.

13. A method for the in vitro prognosis of the outcome of an individual having multiple myeloma comprising the following steps :
a) Measuring, in a biological sample of the individual, the level of expression of at least five genes selected from BSG (CD147), HK2, HNRNPC, HSPA9, IL10, ILF3, LDHB, MDH1, MYBPC2, NCL, NUDC, PARP1, PDIA6, PRPS1, RPL8, RPL13A, RPL27A, RPL35, SF3B2, SLC7A5, SLC25A39, SMARCA4, SPN (CD43), STC2, THY1 (CD90), TNPO2, TPR and YWHAQ,
b) Calculating a risk score from the expression level obtained in step a)
c) Comparing the risk score of the individual to a reference value, and if the risk score is higher than the reference value, classifying the individual as having a bad prognosis.

14. A method for the in vitro prognosis of the outcome of an individual having multiple myeloma comprising the following steps :
a) Measuring, in a biological sample of the individual, the level of expression of at least five genes selected from ARHGEF5, BIVM, DEF8, GRID2IP, HDAC9, HSPA1L, KDM4C, NLRP2, P4HA3, PAG1, PM20D1, RMND5A, SEMA6A, SFMBT2, THEMIS2, TPRKB, ZFP2 and ZNF5188B,
b) Calculating a risk score from the expression level obtained in step a)
c) Comparing the risk score of the individual to a reference value, and if the risk score is higher than the reference value, classifying the individual as having a bad prognosis.
